# FASCICULE DE BREVET EUROPEEN

(11) **EP 2 702 407 B1**
(45) Date de publication et mention de la délivrance du brevet: **06.06.2018**
(21) Numéro de dépôt: 12722687.6
(22) Date de dépôt: 20.04.2012
(51) Int. Cl.: G01N 33/569

(54) **PROCEDE DE DETECTION D'AU MOINS UN MECANISME DE RESISTANCE AUX CARBAPENEMES PAR SPECTROMETRIE DE MASSE**
VERFAHREN ZUR ERFASSUNG VON MINDESTENS EINEM CARBAPENEM-RESISTENZMECHANISMUS MITTELS MASSENSPEKTROMETRIE
METHOD FOR DETECTING AT LEAST ONE CARBAPENEM RESISTANCE MECHANISM BY MEANS OF MASS SPECTROMETRY

(30) Priorité: 21.04.2011 US 201161477915 P
(43) Date de publication de la demande: 05.03.2014
(73) Titulaire: Biomerieux, Inc, Durham, NC 27712 (US)
(72) Inventeur: CHARRETIER, Yannick, F-69690 Courzieu (FR); CHARRIER, Jean-Philippe, F-69160 Tassin La Demi-Lune (FR); FRANCESCHI, Christine, F-01800 Meximieux (FR); ZAMBARDI, Gilles, F-38300 Chezeneuve (FR); CECCHINI, Tiphaine, F-69290 Saint-Genis les Ollières (FR); DEGOUT-CHARMETTE, Elodie, F-01600 Toussieux (FR)
(74) Mandataire: Sprugnoli, Claude Louis Victor
(86) Numéro de dépôt international: PCT/EP2012/057323
(87) Numéro de publication internationale: WO 2012/143535

(56) Documents cités:
- WO-A1-2008/145763
- WO-A1-2008/145763
- WO-A2-2011/045544
- WO-A2-2011/045544
- WO-A2-2011/045544
- S. GROBNER ET AL: "Emergence of carbapenem-non-susceptible extended-spectrum -lactamase-producing Klebsiella pneumoniae isolates at the university hospital of Tubingen, Germany", JOURNAL OF MEDICAL MICROBIOLOGY, vol. 58, no. 7, 1 juillet 2009 (2009-07-01), pages 912-922, XP055035479, ISSN: 0022-2615, DOI: 10.1099/jmm.0.005850-0
- N. A. LEDEBOER ET AL: "Molecular Detection of Resistance Determinants", JOURNAL OF CLINICAL MICROBIOLOGY, vol. 49, no. 9 Supplement, 1 septembre 2011 (2011-09-01), pages S20-S24, XP055173120, ISSN: 0095-1137, DOI: 10.1128/JCM.00771-11
- LIN Y C ET AL: "Differences in carbapenem resistance genes among Acinetobacterbaumannii, Acinetobacter genospecies 3 and Acinetobacter genospecies 13TU in Taiwan", INTERNATIONAL JOURNAL OF ANTIMICROBIAL AGENTS, ELSEVIER SCIENCE, AMSTERDAM, NL, vol. 35, no. 5, 1 mai 2010 (2010-05-01), pages 439-443, XP026971170, ISSN: 0924-8579 [extrait le 2010-01-27]
- S. GROBNER ET AL: "Emergence of carbapenem-non-susceptible extended-spectrum -lactamase-producing Klebsiella pneumoniae isolates at the university hospital of Tubingen, Germany", JOURNAL OF MEDICAL MICROBIOLOGY, vol. 58, no. 7, 1 juillet 2009 (2009-07-01), pages 912-922, XP055035479, ISSN: 0022-2615, DOI: 10.1099/jmm.0.005850-0
- LIN Y C ET AL: "Differences in carbapenem resistance genes among Acinetobacterbaumannii, Acinetobacter genospecies 3 and Acinetobacter genospecies 13TU in Taiwan", INTERNATIONAL JOURNAL OF ANTIMICROBIAL AGENTS, ELSEVIER SCIENCE, AMSTERDAM, NL, vol. 35, no. 5, 1 mai 2010 (2010-05-01), pages 439-443, XP026971170, ISSN: 0924-8579 [extrait le 2010-01-27]
- S. GROBNER ET AL: "Emergence of carbapenem-non-susceptible extended-spectrum -lactamase-producing Klebsiella pneumoniae isolates at the university hospital of Tubingen, Germany", JOURNAL OF MEDICAL MICROBIOLOGY, vol. 58, no. 7, 1 July 2009 (2009-07-01), pages 912-922, XP055035479, ISSN: 0022-2615, DOI: 10.1099/jmm.0.005850-0
- N. A. LEDEBOER ET AL: "Molecular Detection of Resistance Determinants", JOURNAL OF CLINICAL MICROBIOLOGY, vol. 49, no. 9 Supplement, 1 September 2011 (2011-09-01), pages S20-S24, XP055173120, ISSN: 0095-1137, DOI: 10.1128/JCM.00771-11
- LIN Y C ET AL: "Differences in carbapenem resistance genes among Acinetobacterbaumannii, Acinetobacter genospecies 3 and Acinetobacter genospecies 13TU in Taiwan", INTERNATIONAL JOURNAL OF ANTIMICROBIAL AGENTS, ELSEVIER SCIENCE, AMSTERDAM, NL, vol. 35, no. 5, 1 May 2010 (2010-05-01), pages 439-443, XP026971170, ISSN: 0924-8579 [retrieved on 2010-01-27]

### SEQUENCE LISTING

<110> bioMérieux Inc.
<120> Procédé de détection d'au moins un mécanisme de résistance aux carbapénèmes par spectrométrie de masse
<130> CARBA MS DK585
<150> US61477915
   <151> 2011-04-21
<160> 1109
<210> 1
   <211> 255
   <212> PRT
   <213> RESISTANCE GENE
<400> 1
   Met Glu Leu Pro Asn Ile Met His Pro Val Ala Lys Leu Ser Thr Ala
   1 5 10 15
   Leu Ala Ala Ala Leu Met Leu Ser Gly Cys Met Pro Gly Glu Ile Arg
   20 25 30
   Pro Thr Ile Gly Gln Gln Met Glu Thr Gly Asp Gln Arg Phe Gly Asp
   35 40 45
   Leu Val Phe Arg Gln Leu Ala Pro Asn Val Trp Gln His Thr Ser Tyr
   50 55 60
   Leu Asp Met Pro Gly Phe Gly Ala Val Ala Ser Asn Gly Leu Ile Val
   65 70 75 80
   Arg Asp Gly Gly Arg Val Leu Val Val Asp Thr Ala Trp Thr Asp Asp
   85 90 95
   Gln Thr Ala Gln Ile Leu Asn Trp Ile Lys Gln Glu Ile Asn Leu Pro
   100 105 110
   Val Ala Leu Ala Val Val Thr His Ala His Gln Asp Lys Met Gly Gly
   115 120 125
   Met Asp Ala Leu His Ala Ala Gly Ile Ala Thr Tyr Ala Asn Ala Leu
   130 135 140
   Ser Asn Gln Leu Ala Pro Gln Glu Gly Met Val Ala Ala Gln His Ser
   145 150 155 160
   Leu Thr Phe Ala Ala Asn Gly Trp Val Glu Pro Ala Thr Ala Pro Asn
   165 170 175
   Phe Gly Pro Leu Lys Val Phe Tyr Pro Gly Pro Gly His Thr Ser Asp
   180 185 190
   Asn Ile Thr Val Gly Ile Asp Gly Thr Asp Ile Ala Phe Gly Gly Cys
   195 200 205
   Leu Ile Lys Asp Ser Lys Ala Lys Ser Leu Gly Asn Leu Gly Asp Ala
   210 215 220
   Asp Thr Glu His Tyr Ala Ala Ser Ala Arg Ala Phe Gly Ala Ala Phe
   225 230 235 240
   Pro Lys Ala Ser Met Ile Val Met Ser His Ser Ala Pro Asp Ser
   245 250 255
<210> 2
   <211> 8
   <212> PRT
   <213> RESISTANCE GENE
<400> 2
   Ala Ala Ile Thr His Thr Ala Arg
   1 5
<210> 3
   <211> 8
   <212> PRT
   <213> RESISTANCE GENE
<400> 3
   Ala Phe Gly Ala Ala Phe Pro Lys
   1 5
<210> 4
   <211> 14
   <212> PRT
   <213> RESISTANCE GENE
<400> 4
   Ala Ser Met Ile Val Met Ser His Ser Ala Pro Asp Ser Arg
    1               5                   10
<210> 5
   <211> 7
   <212> PRT
   <213> RESISTANCE GENE
<400> 5
   Phe Gly Asp Leu Val Phe Arg
    1               5
<210> 6
   <211> 12
   <212> PRT
   <213> RESISTANCE GENE
<400> 6
   Met Glu Leu Pro Asn Ile Met His Pro Val Ala Lys
    1               5                   10
<210> 7
   <211> 19
   <212> PRT
   <213> RESISTANCE GENE
<400> 7
   Gln Glu Ile Asn Leu Pro Val Ala Leu Ala Val Val Thr His Ala His
    1               5                   10                  15
   Gln Asp Lys
<210> 8
   <211> 18
   <212> PRT
   <213> RESISTANCE GENE
<400> 8
   Ser Leu Gly Asn Leu Gly Asp Ala Asp Thr Glu His Tyr Ala Ala Ser
    1               5                   10                  15
   Ala Arg
<210> 9
   <211> 21
   <212> PRT
   <213> RESISTANCE GENE
<400> 9
   Val Leu Val Val Asp Thr Ala Trp Thr Asp Asp Gln Thr Ala Gln Ile
    1               5                   10                  15
   Leu Asn Trp Ile Lys
                20
<210> 10
   <211> 293
   <212> PRT
   <213> RESISTANCE GENE
<400> 10
   Met Ser Leu Tyr Arg Arg Leu Val Leu Leu Ser Cys Leu Ser Trp Pro
   1               5                   10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 11
   <211> 293
   <212> PRT
   <213> RESISTANCE GENE
<400> 11
   Met Ser Leu Tyr Arg Arg Leu Val Leu Leu Ser Cys Leu Ser Trp Pro
   1               5                   10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
   50 55 60
   Phe Pro Leu Cys Ser Ser Phe Lys Gly Phe Leu Ala Ala Ala Val Leu
   65 70 75 80
   Ala Arg Ser Gln Gln Gln Ala Gly Leu Leu Asp Thr Pro Ile Arg Tyr
   85 90 95
   Gly Lys Asn Ala Leu Val Pro Trp Ser Pro Ile Ser Glu Lys Tyr Leu
   100 105 110
   Thr Thr Gly Met Thr Val Ala Glu Leu Ser Ala Ala Ala Val Gln Tyr
   115 120 125
   Ser Asp Asn Ala Ala Ala Asn Leu Leu Leu Lys Glu Leu Gly Gly Pro
   130 135 140
   Ala Gly Leu Thr Ala Phe Met Arg Ser Ile Gly Asp Thr Thr Phe Arg
   145 150 155 160
   Leu Asp Arg Trp Glu Leu Glu Leu Asn Ser Ala Ile Pro Gly Asp Ala
   165 170 175
   Arg Asp Thr Ser Ser Pro Arg Ala Val Thr Glu Ser Leu Gln Lys Leu
   180 185 190
   Thr Leu Gly Ser Ala Leu Ala Ala Pro Gln Arg Gln Gln Phe Val Asp
   195 200 205
   Trp Leu Lys Gly Asn Thr Thr Gly Asn His Arg Ile Arg Ala Ala Val
   210 215 220
   Pro Ala Asp Trp Ala Val Gly Asp Lys Thr Gly Thr Cys Gly Val Tyr
   225 230 235 240
   Gly Thr Ala Asn Asp Tyr Ala Val Val Trp Pro Thr Gly Arg Ala Pro
   245 250 255
   Ile Val Leu Ala Val Tyr Thr Arg Ala Pro Asn Lys Asp Asp Lys Tyr
   260 265 270
   Ser Glu Ala Val Ile Ala Ala Ala Ala Arg Leu Ala Leu Glu Gly Leu
   275 280 285
   Gly Val Asn Gly Gln
   290
<210> 12
   <211> 293
   <212> PRT
   <213> RESISTANCE GENE
<400> 12
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Arg Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Gly Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 13
   <211> 293
   <212> PRT
   <213> RESISTANCE GENE
<400> 13
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Arg Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 14
   <211> 293
   <212> PRT
   <213> RESISTANCE GENE
<400> 14
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Gly Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 15
   <211> 293
   <212> PRT
   <213> RESISTANCE GENE
<400> 15
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Ile Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 16
   <211> 293
   <212> PRT
   <213> RESISTANCE GENE
<400> 16
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Gly Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 17
   <211> 289
   <212> PRT
   <213> RESISTANCE GENE
<400> 17
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Ala Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly
<210> 18
   <211> 293
   <212> PRT
   <213> RESISTANCE GENE
<400> 18
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
               50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Arg Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 19
   <211> 293
   <212> PRT
   <213> RESISTANCE GENE
<400> 19
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Leu Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 20
   <211> 12
   <212> PRT
   <213> RESISTANCE GENE
<400> 20
   Ala Ala Val Pro Ala Asp Trp Ala Val Gly Asp Lys
    1               5                  10
<210> 21
   <211> 10
   <212> PRT
   <213> RESISTANCE GENE
<400> 21
   Ala Pro Ile Val Leu Ala Val Tyr Thr Arg
    1               5                  10
<210> 22
   <211> 8
   <212> PRT
   <213> RESISTANCE GENE
<400> 22
   Ala Val Thr Glu Ser Leu Gln Lys
   1 5
<210> 23
   <211> 13
   <212> PRT
   <213> RESISTANCE GENE
<400> 23
   Glu Leu Gly Gly Pro Ala Gly Leu Thr Ala Phe Met Arg
   1 5 10
<210> 24
   <211> 8
   <212> PRT
   <213> RESISTANCE GENE
<400> 24
   Phe Pro Leu Cys Ser Ser Phe Lys
   1 5
<210> 25
   <211> 10
   <212> PRT
   <213> RESISTANCE GENE
<400> 25
   Gly Phe Leu Ala Ala Ala Val Leu Ala Arg
   1 5 10
<210> 26
   <211> 8
   <212> PRT
   <213> RESISTANCE GENE
<400> 26
   Gly Asn Thr Thr Gly Asn His Arg
   1 5
<210> 27
   <211> 11
   <212> PRT
   <213> RESISTANCE GENE
<400> 27
   Leu Ala Leu Glu Gly Leu Gly Val Asn Gly Gln
   1 5 10
<210> 28
   <211> 12
   <212> PRT
   <213> RESISTANCE GENE
<400> 28
   Leu Thr Leu Gly Ser Ala Leu Ala Ala Pro Gln Arg
   1 5 10
<210> 29
   <211> 12
   <212> PRT
   <213> RESISTANCE GENE
<400> 29
   Asn Ala Leu Val Pro Trp Ser Pro Ile Ser Glu Lys
   1 5 10
<210> 30
   <211> 8
   <212> PRT
   <213> RESISTANCE GENE
<400> 30
   Gln Gln Phe Val Asp Trp Leu Lys
   1 5
<210> 31
   <211> 8
   <212> PRT
   <213> RESISTANCE GENE
<400> 31
   Ser Ile Gly Asp Thr Thr Phe Arg
   1 5
<210> 32
   <211> 13
   <212> PRT
   <213> RESISTANCE GENE
<400> 32
   Ser Gln Gln Gln Ala Gly Leu Leu Asp Thr Pro Ile Arg
   1 5 10
<210> 33
   <211> 14
   <212> PRT
   <213> RESISTANCE GENE
<400> 33
   Trp Glu Leu Glu Leu Asn Ser Ala Ile Pro Gly Asp Ala Arg
    1               5                  10
<210> 34
   <211> 287
   <212> PRT
   <213> RESISTANCE GENE
<400> 34
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 35
   <211> 287
   <212> PRT
   <213> RESISTANCE GENE
<400> 35
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 36
   <211> 287
   <212> PRT
   <213> RESISTANCE GENE
<400> 36
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Leu Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 37
   <211> 287
   <212> PRT
   <213> RESISTANCE GENE
<400> 37
   Met Arg Phe Ile His Ala Leu Leu Leu Ala Gly Ile Ala His Ser Ala
     1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Asn Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 38
   <211> 287
   <212> PRT
   <213> RESISTANCE GENE
<400> 38
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 39
   <211> 287
   <212> PRT
   <213> RESISTANCE GENE
<400> 39
   Met Arg Phe Ile His Ala Leu Leu Leu Ala Gly Ile Ala His Ser Ala
   1               5                   10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 40
   <211> 287
   <212> PRT
   <213> RESISTANCE GENE
<400> 40
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Ser Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 41
   <211> 287
   <212> PRT
   <213> RESISTANCE GENE
<400> 41
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
                20                  25                  30
   Arg Glu Lys Ala Ala Gln Ile Gly Val Ala Ile Val Asp Pro Gln Gly
            35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Ala Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 42
   <211> 287
   <212> PRT
   <213> RESISTANCE GENE
<400> 42
   Met Arg Phe Ile His Ala Leu Leu Leu Ala Gly Thr Ala His Ser Ala
    1               5                  10                  15
   Tyr Ala Ser Glu Lys Leu Thr Phe Lys Thr Asp Leu Glu Lys Leu Glu
                20                  25                  30
   Arg Glu Lys Ala Ala Gln Ile Gly Val Ala Ile Val Asp Pro Gln Gly
            35                  40                  45
   Glu Ile Val Ala Gly His Arg Thr Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Cys Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 43
   <211> 287
   <212> PRT
   <213> RESISTANCE GENE
<400> 43
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Asn Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 44
   <211> 287
   <212> PRT
   <213> RESISTANCE GENE
<400> 44
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Ser Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 45
   <211> 287
   <212> PRT
   <213> RESISTANCE GENE
<400> 45
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                           85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Ala Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 46
   <211> 287
   <212> PRT
   <213> RESISTANCE GENE
<400> 46
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Glu Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 47
   <211> 287
   <212> PRT
   <213> RESISTANCE GENE
<400> 47
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Ala Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 48
   <211> 287
   <212> PRT
   <213> RESISTANCE GENE
<400> 48
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Thr Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 49
   <211> 287
   <212> PRT
   <213> RESISTANCE GENE
<400> 49
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Thr Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Lys Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Ser Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Thr Cys Ala Asn Gly Gly Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 50
   <211> 287
   <212> PRT
   <213> RESISTANCE GENE
<400> 50
   Met Arg Phe Ile His Ala Leu Leu Leu Ala Gly Ile Ala His Ser Ala
    1               5                  10                  15
   Tyr Ala Ser Glu Lys Leu Thr Phe Lys Thr Asp Leu Glu Lys Leu Glu
               20                  25                  30
   Arg Glu Lys Ala Ala Gln Ile Gly Val Ala Ile Val Asp Pro Gln Gly
           35                  40                  45
   Glu Ile Val Ala Gly His Arg Met Ala Gln Arg Phe Ala Met Cys Ser
       50                  55                  60
   Thr Phe Lys Phe Pro Leu Ala Ala Leu Val Phe Glu Arg Ile Asp Ser
   65                  70                  75                  80
   Gly Thr Glu Arg Gly Asp Arg Lys Leu Ser Tyr Gly Pro Asp Met Ile
                   85                  90                  95
   Val Glu Trp Ser Pro Ala Thr Glu Arg Phe Leu Ala Ser Gly His Met
               100                 105                 110
   Thr Val Leu Glu Ala Ala Gln Ala Ala Val Gln Leu Ser Asp Asn Gly
           115                 120                 125
   Ala Thr Asn Leu Leu Leu Arg Glu Ile Gly Gly Pro Ala Ala Met Thr
       130                 135                 140
   Gln Tyr Phe Arg Lys Ile Gly Asp Ser Val Ser Arg Leu Asp Arg Lys
   145                 150                 155                 160
   Glu Pro Glu Met Gly Asp Asn Thr Pro Gly Asp Leu Arg Asp Thr Thr
                   165                 170                 175
   Thr Pro Ile Ala Met Ala Arg Thr Val Ala Lys Val Leu Tyr Gly Gly
               180                 185                 190
   Ala Leu Thr Ser Thr Ser Thr His Thr Ile Glu Arg Trp Leu Ile Gly
           195                 200                 205
   Asn Gln Thr Gly Asp Ala Thr Leu Arg Ala Gly Phe Pro Lys Asp Trp
       210                 215                 220
   Val Val Gly Glu Lys Thr Gly Ala Cys Ala Asn Gly Ala Arg Asn Asp
   225                 230                 235                 240
   Ile Gly Phe Phe Lys Ala Gln Glu Arg Asp Tyr Ala Val Ala Val Tyr
                   245                 250                 255
   Thr Thr Ala Pro Lys Leu Ser Ala Val Glu Arg Asp Glu Leu Val Ala
               260                 265                 270
   Ser Val Gly Gln Val Ile Thr Gln Leu Ile Leu Ser Thr Asp Lys
           275                 280                 285
<210> 51
   <211> 20
   <212> PRT
   <213> RESISTANCE GENE
<400> 51
   Ala Ala Glu Ile Gly Val Ala Ile Val Asp Pro Gln Gly Glu Ile Val
    1               5                  10                  15
   Ala Gly His Arg
               20
<210> 52
   <211> 20
   <212> PRT
   <213> RESISTANCE GENE
<400> 52
   Ala Ala Gln Ile Gly Val Ala Ile Val Asp Pro Gln Gly Glu Ile Val
    1               5                  10                  15
   Ala Gly His Arg
               20
<210> 53
   <211> 5
   <212> PRT
   <213> RESISTANCE GENE
<400> 53
   Ala Gly Phe Pro Lys
   1 5
<210> 54
   <211> 10
   <212> PRT
   <213> RESISTANCE GENE
<400> 54
   Asp Thr Thr Thr Pro Ile Ala Met Ala Arg
   1 5 10
<210> 55
   <211> 7
   <212> PRT
   <213> RESISTANCE GENE
<400> 55
   Asp Trp Val Val Gly Glu Lys
   1 5
<210> 56
   <211> 12
   <212> PRT
   <213> RESISTANCE GENE
<400> 56
   Asp Tyr Ala Val Ala Val Tyr Thr Thr Ala Pro Lys
   1 5 10
<210> 57
   <211> 13
   <212> PRT
   <213> RESISTANCE GENE
<400> 57
   Glu Ile Gly Gly Pro Ala Ala Met Thr Gln Tyr Phe Arg
   1 5 10
<210> 58
   <211> 13
   <212> PRT
   <213> RESISTANCE GENE
<400> 58
   Glu Pro Glu Met Gly Asp Asn Thr Pro Gly Asp Leu Arg
   1 5 10
<210> 59
   <211> 13
   <212> PRT
   <213> RESISTANCE GENE
<400> 59
   Glu Pro Glu Met Asn Asp Asn Thr Pro Gly Asp Leu Arg
   1 5 10
<210> 60
   <211> 13
   <212> PRT
   <213> RESISTANCE GENE
<400> 60
   Glu Pro Glu Met Ser Asp Asn Thr Pro Gly Asp Leu Arg
   1 5 10
<210> 61
   <211> 13
   <212> PRT
   <213> RESISTANCE GENE
<400> 61
   Glu Ser Glu Met Ser Asp Asn Thr Pro Gly Asp Leu Arg
   1 5 10
<210> 62
   <211> 8
   <212> PRT
   <213> RESISTANCE GENE
<400> 62
   Phe Ala Met Cys Ser Thr Phe Lys
   1 5
<210> 63
   <211> 19
   <212> PRT
   <213> RESISTANCE GENE
<400> 63
   Phe Ile His Ala Leu Leu Leu Ala Gly Ile Ala His Ser Ala Tyr Ala
   1 5 10 15
   Ser Glu Lys
<210> 64
   <211> 19
   <212> PRT
   <213> RESISTANCE GENE
<400> 64
   Phe Ile His Ala Leu Leu Leu Ala Gly Thr Ala His Ser Ala Tyr Ala
    1               5                  10                  15
   Ser Glu Lys
<210> 65
   <211> 10
   <212> PRT
   <213> RESISTANCE GENE
<400> 65
   Phe Pro Leu Ala Ala Leu Val Phe Glu Arg
    1               5                  10
<210> 66
   <211> 7
   <212> PRT
   <213> RESISTANCE GENE
<400> 66
   Ile Asp Ser Gly Thr Glu Arg
    1               5
<210> 67
   <211> 7
   <212> PRT
   <213> RESISTANCE GENE
<400> 67
   Ile Gly Asp Ser Val Ser Arg
    1               5
<210> 68
   <211> 6
   <212> PRT
   <213> RESISTANCE GENE
<400> 68
   Leu Ser Ala Val Glu Arg
    1               5
<210> 69
   <211> 17
   <212> PRT
   <213> RESISTANCE GENE
<400> 69
   Leu Ser Tyr Gly Pro Asp Met Ile Val Glu Trp Ser Pro Ala Thr Glu
   1 5 10 15
   Arg
<210> 70
   <211> 10
   <212> PRT
   <213> RESISTANCE GENE
<400> 70
   Leu Ser Tyr Gly Pro Asp Met Ile Val Lys
   1 5 10
<210> 71
   <211> 7
   <212> PRT
   <213> RESISTANCE GENE
<400> 71
   Asn Asp Ile Gly Phe Phe Lys
   1 5
<210> 72
   <211> 5
   <212> PRT
   <213> RESISTANCE GENE
<400> 72
   Thr Asp Leu Glu Lys
   1 5
<210> 73
   <211> 9
   <212> PRT
   <213> RESISTANCE GENE
<400> 73
   Thr Gly Ala Cys Ala Asn Gly Ala Arg
   1 5
<210> 74
   <211> 9
   <212> PRT
   <213> RESISTANCE GENE
<400> 74
   Thr Gly Thr Cys Ala Asn Gly Ala Arg
   1 5
<210> 75
   <211> 9
   <212> PRT
   <213> RESISTANCE GENE
<400> 75
   Thr Gly Thr Cys Ala Asn Gly Gly Arg
   1 5
<210> 76
   <211> 9
   <212> PRT
   <213> RESISTANCE GENE
<400> 76
   Thr Gly Thr Cys Ala Asn Gly Ser Arg
   1 5
<210> 77
   <211> 17
   <212> PRT
   <213> RESISTANCE GENE
<400> 77
   Val Leu Tyr Gly Gly Ala Leu Thr Ser Thr Ser Thr His Thr Ile Glu
   1 5 10 15
   Arg
<210> 78
   <211> 13
   <212> PRT
   <213> RESISTANCE GENE
<400> 78
   Trp Leu Ile Gly Asn Gln Thr Gly Asp Ala Thr Leu Arg
   1 5 10
<210> 79
   <211> 7
   <212> PRT
   <213> RESISTANCE GENE
<400> 79
   Trp Ser Pro Ala Thr Glu Arg
   1 5
<210> 80
   <211> 246
   <212> PRT
   <213> RESISTANCE GENE
<400> 80
   Met Ser Lys Leu Ser Val Phe Phe Ile Phe Leu Phe Cys Ser Ile Ala
    1               5                  10                  15
   Thr Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Ser His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 81
   <211> 246
   <212> PRT
   <213> RESISTANCE GENE
<400> 81
   Met Lys Lys Leu Ser Val Phe Phe Met Phe Leu Phe Cys Ser Ile Ala
    1               5                  10                  15
   Ala Ser Gly Glu Ala Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Thr Asp Ala Tyr Leu
       50                  55                  60
   Ile Asp Thr Pro Phe Thr Ala Lys Asp Thr Glu Lys Leu Val Thr Trp
   65 70 75 80
   Phe Val Glu Arg Gly Tyr Lys Ile Lys Gly Ser Ile Ser Ser His Phe
   85 90 95
   His Ser Asp Ser Thr Gly Gly Ile Glu Trp Leu Asn Ser Gln Ser Ile
   100 105 110
   Pro Thr Tyr Ala Ser Glu Leu Thr Asn Glu Leu Leu Lys Lys Asp Gly
   115 120 125
   Lys Val Gln Ala Lys Asn Ser Phe Ser Gly Ala Ser Tyr Trp Leu Val
   130 135 140
   Lys Lys Lys Ile Glu Ile Phe Tyr Pro Gly Pro Gly His Thr Pro Asp
   145 150 155 160
   Asn Val Val Val Trp Leu Pro Glu His Arg Val Leu Phe Gly Gly Cys
   165 170 175
   Phe Val Lys Pro Tyr Gly Leu Gly Asn Leu Gly Asp Ala Asn Leu Glu
   180 185 190
   Ala Trp Pro Lys Ser Ala Lys Leu Leu Val Ser Lys Tyr Gly Lys Ala
   195 200 205
   Lys Leu Val Val Pro Ser His Ser Glu Val Gly Asp Ala Ser Leu Leu
   210 215 220
   Lys Arg Thr Leu Glu Gln Ala Val Lys Gly Leu Asn Glu Ser Lys Lys
   225 230 235 240
   Leu Ser Lys Pro Ser Asn
   245
<210> 82
   <211> 246
   <212> PRT
   <213> RESISTANCE GENE
<400> 82
   Met Ser Lys Leu Ser Val Phe Phe Ile Phe Leu Phe Cys Ser Ile Ala
    1               5                  10                  15
   Thr Ala Ala Glu Pro Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asp Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Gly Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Leu Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Leu Glu
               180                 185                 190
   Ala Trp Pro Lys Ser Ala Lys Leu Leu Ile Ser Lys Tyr Gly Lys Ala
           195                 200                 205
   Lys Leu Val Val Pro Ser His Ser Glu Ala Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Leu Ser Asn
                   245
<210> 83
   <211> 246
   <212> PRT
   <213> RESISTANCE GENE
<400> 83
   Met Lys Lys Leu Phe Val Leu Cys Val Cys Phe Leu Cys Ser Ile Thr
    1               5                  10                  15
   Ala Ala Gly Ala Ala Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
   65                  70                  75                  80
   Phe Val Glu Arg Gly Tyr Lys Ile Lys Gly Thr Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Glu Ile Gly Asp Ala Ser Leu Leu
       210                 215                 220
   Lys Arg Thr Trp Glu Gln Ala Val Lys Gly Leu Asn Glu Ser Lys Lys
   225                 230                 235                 240
   Pro Ser Gln Pro Ser Asn
                   245
<210> 84
   <211> 246
   <212> PRT
   <213> RESISTANCE GENE
<400> 84
   Met Ser Lys Leu Phe Val Phe Phe Met Phe Leu Phe Cys Ser Ile Thr
    1               5                  10                  15
   Ala Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Thr Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Ala Ser Tyr Trp Leu Val
       130                 135                 140
   Lys Lys Lys Ile Glu Val Phe Tyr Pro Gly Pro Gly His Thr Pro Asp
   145                 150                 155                 160
   Asn Val Val Val Trp Leu Pro Glu Asn Arg Val Leu Phe Gly Gly Cys
                   165                 170                 175
   Phe Val Lys Pro Tyr Gly Leu Gly Asn Leu Gly Asp Ala Asn Val Glu
               180                 185                 190
   Ala Trp Pro Lys Ser Ala Lys Leu Leu Met Ser Lys Tyr Gly Lys Ala
           195                 200                 205
   Lys Leu Val Val Pro Ser His Ser Glu Val Gly Asp Ala Ser Leu Leu
       210                 215                 220
   Lys Arg Thr Leu Glu Gln Ala Val Lys Gly Leu Asn Glu Ser Lys Lys
   225                 230                 235                 240
   Pro Ser Lys Pro Ser Asn
                   245
<210> 85
   <211> 245
   <212> PRT
   <213> RESISTANCE GENE
<400> 85
   Met Ser Lys Leu Phe Val Phe Phe Met Phe Leu Phe Cys Ser Ile Thr
    1               5                  10                  15
   Ala Ala Gly Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Ile Pro Lys His Gly Leu Val Val Leu Val Asn Thr Asp Ala Tyr Leu
       50                  55                  60
   Ile Asp Thr Pro Phe Thr Ala Lys Asp Thr Glu Asn Leu Val Asn Trp
    65                  70                  75                  80
   Phe Val Glu Arg Gly Tyr Arg Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Tyr Ser Phe Ser Gly Val Ser Tyr Trp Leu Val
       130                 135                 140
   Lys Lys Lys Ile Glu Val Phe Tyr Pro Gly Pro Gly His Ala Pro Asp
   145                 150                 155                 160
   Asn Val Val Val Trp Leu Pro Glu Asn Arg Val Leu Phe Gly Gly Cys
                   165                 170                 175
   Phe Val Lys Pro Tyr Gly Leu Gly Asn Leu Gly Asp Ala Asn Leu Glu
               180                 185                 190
   Ala Trp Pro Lys Ser Ala Lys Leu Leu Met Ser Lys Tyr Ser Lys Ala
           195                 200                 205
   Lys Leu Val Val Pro Ser His Ser Asp Ile Gly Asp Ser Ser Leu Leu
       210                 215                 220
   Lys Leu Thr Trp Glu Gln Thr Val Lys Gly Phe Asn Glu Ser Lys Lys
   225                 230                 235                 240
   Ser Thr Thr Ala His
                   245
<210> 86
   <211> 246
   <212> PRT
   <213> RESISTANCE GENE
<400> 86
   Met Lys Lys Leu Phe Val Leu Cys Val Phe Phe Phe Cys Asn Ile Ala
    1               5                  10                  15
   Val Ala Glu Glu Ser Leu Pro Asp Leu Lys Ile Glu Lys Leu Glu Glu
                20                  25                  30
   Gly Val Tyr Val His Thr Ser Phe Glu Glu Val Lys Gly Trp Ser Val
           35                  40                  45
   Val Thr Lys His Gly Leu Val Val Leu Val Lys Asn Asp Ala Tyr Leu
       50                  55                  60
   Ile Asp Thr Pro Ile Thr Ala Lys Asp Thr Glu Lys Leu Val Asn Trp
   65                  70                  75                  80
   Phe Val Glu Arg Gly Tyr Lys Ile Lys Gly Ser Ile Ser Thr His Phe
                   85                  90                  95
   His Gly Asp Ser Thr Ala Gly Ile Glu Trp Leu Asn Ser Gln Ser Ile
               100                 105                 110
   Pro Thr Tyr Ala Ser Glu Leu Thr Asn Glu Leu Leu Lys Lys Asp Asn
           115                 120                 125
   Lys Val Gln Ala Lys His Ser Phe Asn Gly Val Ser Tyr Ser Leu Ile
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Tyr Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Asp Ile Gly Asp Val Ser Leu Leu
       210                 215                 220
   Lys Arg Thr Trp Glu Gln Ala Val Lys Gly Leu Asn Glu Ser Lys Lys
   225                 230                 235                 240
   Ser Ser Gln Pro Ser Asp
                   245
<210> 87
   <211> 246
   <212> PRT
   <213> RESISTANCE GENE
<400> 87
   Met Asn Lys Leu Ser Val Phe Phe Met Phe Met Phe Cys Ser Ile Thr
    1               5                  10                  15
   Ala Ala Gly Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Thr Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Gly Ser Tyr Trp Leu Val
       130                 135                 140
   Asn Asn Lys Ile Glu Val Phe Tyr Pro Gly Pro Gly His Thr Pro Asp
   145                 150                 155                 160
   Asn Val Val Val Trp Leu Pro Glu Asn Arg Val Leu Phe Gly Gly Cys
                   165                 170                 175
   Phe Val Lys Pro Tyr Gly Leu Gly Asn Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Glu Thr Gly Asn Ala Ser Leu Leu
       210                 215                 220
   Lys Leu Thr Trp Glu Gln Ala Val Lys Gly Leu Lys Glu Ser Lys Lys
   225                 230                 235                 240
   Pro Ser Leu Pro Ser Asn
                   245
<210> 88
   <211> 246
   <212> PRT
   <213> RESISTANCE GENE
<400> 88
   Met Lys Lys Leu Phe Val Leu Cys Val Phe Phe Leu Cys Asn Ile Ala
    1               5                  10                  15
   Ala Ala Asp Asp Ser Leu Pro Asp Leu Lys Ile Glu Lys Leu Glu Lys
               20                  25                  30
   Gly Val Tyr Val His Thr Ser Phe Glu Glu Val Lys Gly Trp Gly Val
           35                  40                  45
   Val Thr Lys His Gly Leu Val Val Leu Val Lys Asn Asp Ala Tyr Leu
       50                  55                  60
   Ile Asp Thr Pro Ile Thr Ala Lys Asp Thr Glu Lys Leu Val Asn Trp
   65                  70                  75                  80
   Phe Ile Glu His Gly Tyr Arg Ile Lys Gly Ser Ile Ser Thr His Phe
                   85                  90                  95
   His Gly Asp Ser Thr Ala Gly Ile Glu Trp Leu Asn Ser Gln Ser Ile
               100                 105                 110
   Ser Thr Tyr Ala Ser Glu Leu Thr Asn Glu Leu Leu Lys Lys Asp Asn
           115                 120                 125
   Lys Val Gln Ala Thr Asn Ser Phe Ser Gly Val Ser Tyr Ser Leu Ile
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Glu Ile Gly Asn Ala Ser Leu Leu
       210                 215                 220
   Gln Arg Thr Trp Glu Gln Ala Val Lys Gly Leu Asn Glu Ser Lys Lys
   225                 230                 235                 240
   Pro Leu Gln Pro Ser Ser
                   245
<210> 89
   <211> 246
   <212> PRT
   <213> RESISTANCE GENE
<400> 89
   Met Lys Lys Leu Phe Val Leu Cys Val Phe Leu Phe Cys Ser Ile Thr
    1               5                  10                  15
   Ala Ala Gly Glu Ser Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Ile Leu Val Asn Thr Asp Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                           85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Asp Leu Leu Lys Gln Asn Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Tyr Gly Leu Gly Asn Leu Asp Asp Ala Asn Val Val
               180                 185                 190
   Ala Trp Pro His Ser Ala Glu Ile Leu Met Ser Arg Tyr Gly Asn Ala
           195                 200                 205
   Lys Leu Val Val Pro Ser His Ser Asp Ile Gly Asp Ala Ser Leu Leu
       210                 215                 220
   Lys Leu Thr Trp Glu Gln Ala Val Lys Gly Leu Lys Glu Ser Lys Lys
   225                 230                 235                 240
   Pro Ser Glu Pro Ser Asn
                   245
<210> 90
   <211> 246
   <212> PRT
   <213> RESISTANCE GENE
<400> 90
   Met Ser Lys Leu Ser Val Phe Phe Ile Phe Leu Phe Cys Ser Ile Ala
   1               5                   10                  15
   Thr Ala Ala Glu Ser Leu Pro Asp Leu Lys Ile Glu Lys Leu Asp Glu
               20                  25                  30
   Gly Val Tyr Val His Thr Ser Phe Lys Glu Val Asn Gly Trp Gly Val
           35                  40                  45
   Val Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Ser His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 91
   <211> 246
   <212> PRT
   <213> RESISTANCE GENE
<400> 91
   Met Lys Lys Leu Phe Val Leu Cys Val Cys Phe Leu Cys Ser Ile Thr
    1               5                  10                  15
   Ala Ala Gly Ala Ala Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
    65                 70                  75                  80
   Phe Val Glu Arg Gly Tyr Lys Ile Lys Gly Thr Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Val Lys Ala
           195                 200                 205
   Lys Leu Val Val Ser Ser His Ser Glu Ile Gly Asp Ala Ser Leu Leu
       210                 215                 220
   Lys Arg Thr Trp Glu Gln Ala Val Lys Gly Leu Asn Glu Ser Lys Lys
   225                 230                 235                 240
   Pro Ser Gln Pro Ser Asn
                   245
<210> 92
   <211> 246
   <212> PRT
   <213> RESISTANCE GENE
<400> 92
   Met Lys Lys Leu Phe Val Leu Cys Val Cys Phe Leu Cys Ser Ile Thr
   1               5                   10                  15
   Ala Ala Gly Ala Ala Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
   65                  70                  75                  80
   Phe Val Glu Arg Gly Tyr Lys Ile Lys Gly Thr Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Glu Ile Gly Asp Ala Ser Leu Leu
       210                 215                 220
   Lys Arg Thr Trp Glu Gln Ala Val Lys Gly Leu Asn Glu Ser Arg Lys
   225                 230                 235                 240
   Pro Ser Gln Pro Ser Asn
                   245
<210> 93
   <211> 246
   <212> PRT
   <213> RESISTANCE GENE
<400> 93
   Met Ser Lys Leu Ser Val Phe Phe Ile Phe Leu Phe Cys Ser Ile Ala
   1               5                   10                  15
   Thr Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Ser Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Gly His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 94
   <211> 246
   <212> PRT
   <213> RESISTANCE GENE
<400> 94
   Met Ser Lys Leu Ser Val Phe Phe Ile Phe Leu Phe Cys Ser Ile Ala
   1               5                   10                  15
   Thr Ala Ala Glu Pro Leu Pro Asp Leu Lys Ile Glu Lys Leu Asp Glu
               20                  25                  30
   Gly Val Tyr Val His Thr Ser Phe Glu Glu Val Asn Gly Trp Gly Val
               35                  40                  45
   Phe Pro Lys His Gly Leu Val Val Leu Val Asp Ala Glu Ala Tyr Leu
           50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Gly Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Leu Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Leu Glu
               180                 185                 190
   Ala Trp Pro Lys Ser Ala Lys Leu Leu Ile Ser Lys Tyr Gly Lys Ala
           195                 200                 205
   Lys Leu Val Val Pro Ser His Ser Glu Ala Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Leu Ser Asn
                   245
<210> 95
   <211> 246
   <212> PRT
   <213> RESISTANCE GENE
<400> 95
   Met Lys Lys Leu Phe Val Leu Cys Val Phe Val Phe Cys Ser Ile Thr
    1               5                   10                  15
   Val Ala Gly Glu Thr Leu Pro Asn Leu Arg Val Glu Lys Leu Glu Glu
               20                  25                  30
   Gly Val Tyr Val His Thr Ser Tyr Glu Glu Val Lys Gly Trp Gly Val
           35                  40                  45
   Val Thr Lys His Gly Leu Val Val Leu Ile Gly Ala Asp Ala Tyr Leu
       50                  55                  60
   Ile Asp Thr Pro Phe Thr Ala Lys Asp Thr Glu Lys Leu Val Asn Trp
   65                  70                  75                  80
   Phe Val Glu Arg Gly Tyr Lys Ile Lys Gly Thr Val Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Asp Gly Val Ser Tyr Trp Leu Ala
       130                 135                 140
   Lys Asp Lys Ile Glu Val Phe Tyr Pro Gly Pro Gly His Thr Gln Asp
   145                 150                 155                 160
   Asn Val Val Val Trp Leu Pro Glu Lys Glu Ile Leu Phe Gly Gly Cys
                   165                 170                 175
   Phe Val Lys Pro His Gly Leu Gly Asn Leu Gly Asp Ala Asn Leu Glu
               180                 185                 190
   Ala Trp Pro Glu Ser Ala Lys Ile Leu Met Glu Lys Tyr Gly Lys Ala
           195                 200                 205
   Lys Leu Val Val Ser Gly His Ser Glu Thr Gly Asp Ala Thr His Leu
       210                 215                 220
   Lys Arg Thr Trp Glu Gln Ala Val Lys Gly Leu Lys Glu Ser Lys Lys
   225                 230                 235                 240
   Thr Leu Gln Pro Ser Asn
                   245
<210> 96
   <211> 246
   <212> PRT
   <213> RESISTANCE GENE
<400> 96
   Met Ser Lys Leu Ser Val Phe Phe Ile Phe Leu Phe Cys Ser Ile Ala
    1               5                  10                  15
   Thr Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Gly Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Gly His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 97
   <211> 246
   <212> PRT
   <213> RESISTANCE GENE
<400> 97
   Met Ser Lys Leu Ser Val Phe Phe Ile Phe Leu Phe Cys Ser Ile Ala
    1               5                  10                  15
   Thr Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Val Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Gly His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 98
   <211> 246
   <212> PRT
   <213> RESISTANCE GENE
<400> 98
   Met Ser Lys Leu Ser Val Phe Phe Ile Phe Leu Phe Cys Ser Ile Ala
     1               5                  10                  15
   Thr Ala Ala Glu Ser Leu Pro Asp Leu Lys Ile Glu Lys Leu Asp Glu
               20                  25                  30
   Gly Val Tyr Val His Thr Ser Phe Glu Glu Val Asn Gly Trp Gly Val
           35                  40                  45
   Phe Pro Lys His Gly Leu Val Val Leu Val Asn Ala Glu Ala Tyr Leu
       50                  55                  60
   Ile Asp Thr Pro Phe Thr Ala Lys Asp Thr Glu Lys Leu Val Thr Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Arg Ser Ile
               100                 105                 110
   Pro Thr Tyr Ala Ser Glu Leu Thr Asn Glu Leu Leu Lys Lys Asp Gly
           115                 120                 125
   Lys Val Gln Ala Thr Asn Ser Phe Ser Gly Val Asn Tyr Trp Leu Val
       130                 135                 140
   Lys Asn Lys Ile Glu Val Phe Tyr Pro Gly Pro Gly His Thr Pro Asp
   145                 150                 155                 160
   Asn Val Val Val Trp Leu Pro Glu Arg Lys Ile Leu Phe Gly Gly Cys
                   165                 170                 175
   Phe Ile Lys Pro Tyr Gly Leu Gly Asn Leu Gly Asp Ala Asn Ile Glu
               180                 185                 190
   Ala Trp Pro Lys Ser Ala Lys Leu Leu Lys Ser Lys Tyr Gly Lys Ala
           195                 200                 205
   Lys Leu Val Val Pro Ser His Ser Glu Val Gly Asp Ala Ser Leu Leu
       210                 215                 220
   Lys Leu Thr Leu Glu Gln Ala Val Lys Gly Leu Asn Glu Ser Lys Lys
   225                 230                 235                 240
   Pro Ser Lys Pro Ser Asn
                   245
<210> 99
   <211> 245
   <212> PRT
   <213> RESISTANCE GENE
<400> 99
   Met Lys Lys Leu Phe Val Leu Cys Ile Phe Leu Phe Cys Ser Ile Thr
   1               5                   10                  15
   Ala Ala Gly Ala Ser Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Val Leu Val Asn Thr Asp Ala Tyr Leu
       50                  55                  60
   Ile Asp Thr Pro Phe Thr Ala Lys Asp Thr Glu Lys Leu Val Asn Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Val Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Lys Asn Lys Ile Leu Phe Gly Gly Cys
                   165                 170                 175
   Phe Val Lys Pro Tyr Gly Leu Gly Asn Leu Asp Asp Ala Asn Val Glu
               180                 185                 190
   Ala Trp Pro His Ser Ala Glu Lys Leu Ile Ser Lys Tyr Gly Asn Ala
           195                 200                 205
   Lys Leu Val Val Pro Ser His Ser Asp Ile Gly Asp Ala Ser Leu Leu
       210                 215                 220
   Lys Leu Thr Trp Glu Gln Ala Val Lys Gly Leu Asn Glu Ser Lys Lys
   225                 230                 235                 240
   Ser Asn Thr Val His
                   245
<210> 100
   <211> 246
   <212> PRT
   <213> RESISTANCE GENE
<400> 100
   Met Lys Lys Leu Phe Val Leu Cys Val Cys Phe Leu Cys Ser Ile Thr
     1               5                  10                  15
   Ala Ala Gly Ala Ala Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Phe Ser Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
   65                  70                  75                  80
   Phe Val Glu Arg Gly Tyr Lys Ile Lys Gly Thr Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Val Lys Ala
           195                 200                 205
   Lys Leu Val Val Ser Ser His Ser Glu Ile Gly Asp Ala Ser Leu Leu
       210                 215                 220
   Lys Arg Thr Trp Glu Gln Ala Val Lys Gly Leu Asn Glu Ser Lys Lys
   225                 230                 235                 240
   Pro Ser Gln Pro Ser Asn
                   245
<210> 101
   <211> 245
   <212> PRT
   <213> RESISTANCE GENE
<400> 101
   Met Lys Lys Leu Phe Val Leu Cys Ile Phe Leu Phe Cys Ser Ile Thr
    1               5                  10                  15
   Ala Ala Gly Ala Ser Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Ala Ser Lys His Gly Leu Val Val Leu Val Asn Thr Asp Ala Tyr Leu
       50                  55                  60
   Ile Asp Thr Pro Phe Thr Ala Lys Asp Thr Glu Lys Leu Val Asn Trp
   65                  70                  75                  80
   Phe Val Glu Arg Gly Tyr Lys Ile Lys Gly Ser Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Val Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Lys Asn Lys Ile Leu Phe Gly Gly Cys
                   165                 170                 175
   Phe Val Lys Pro Tyr Gly Leu Gly Asn Leu Asp Asp Ala Asn Val Glu
               180                 185                 190
   Ala Trp Pro His Ser Ala Glu Lys Leu Ile Ser Lys Tyr Gly Asn Ala
           195                 200                 205
   Lys Leu Val Val Pro Ser His Ser Asp Ile Gly Asp Ala Ser Leu Leu
       210                 215                 220
   Lys Leu Thr Trp Glu Gln Ala Val Lys Gly Leu Asn Glu Ser Lys Lys
   225                 230                 235                 240
   Ser Asn Thr Val His
                   245
<210> 102
   <211> 246
   <212> PRT
   <213> RESISTANCE GENE
<400> 102
   Met Lys Lys Leu Phe Val Leu Cys Val Cys Phe Leu Cys Ser Ile Thr
    1               5                  10                  15
   Ala Ala Gly Ala Arg Leu Pro Asp Leu Lys Ile Glu Lys Leu Glu Glu
               20                  25                  30
   Gly Val Tyr Val His Thr Ser Phe Glu Glu Val Asn Gly Trp Gly Val
           35                  40                  45
   Val Ser Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
    65                 70                  75                  80
   Phe Val Glu Arg Gly Tyr Lys Ile Lys Gly Thr Ile Ser Ser His Phe
                    85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asp Gly
           115                 120                 125
   Lys Val Gln Ala Lys Asn Ser Phe Ser Gly Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Asp Gly Leu Gly Asn Leu Gly Asp Ala Asn Leu Glu
               180                 185                 190
   Ala Trp Pro Lys Ser Ala Lys Ile Leu Met Ser Lys Tyr Val Lys Ala
           195                 200                 205
   Lys Leu Val Val Ser Ser His Ser Glu Ile Gly Asp Ala Ser Leu Leu
       210                 215                 220
   Lys Arg Thr Trp Glu Gln Ala Val Lys Gly Leu Asn Glu Ser Lys Lys
   225                 230                 235                 240
   Pro Ser Gln Pro Ser Asn
                   245
<210> 103
   <211> 246
   <212> PRT
   <213> RESISTANCE GENE
<400> 103
   Met Lys Lys Leu Phe Val Leu Cys Ile Phe Leu Phe Leu Ser Ile Thr
    1               5                  10                  15
   Ala Ser Gly Glu Val Leu Pro Asp Leu Lys Ile Glu Lys Leu Glu Glu
               20                  25                  30
   Gly Val Tyr Leu His Thr Ser Phe Glu Glu Val Ser Gly Trp Gly Val
           35                  40                  45
   Val Thr Lys His Gly Leu Val Val Leu Val Asn Asn Asp Ala Tyr Leu
       50                  55                  60
   Ile Asp Thr Pro Phe Thr Asn Lys Asp Thr Glu Lys Leu Val Ala Trp
   65                  70                  75                  80
   Phe Val Gly Arg Gly Phe Thr Ile Lys Gly Ser Val Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Asn Gly
           115                 120                 125
   Lys Val Gln Ala Thr Asn Ser Phe Ser Gly Val Ser Tyr Trp Leu Val
       130                 135                 140
   Lys Asn Lys Ile Glu Ile Phe Tyr Pro Gly Pro Gly His Thr Gln Asp
   145                 150                 155                 160
   Asn Val Val Val Trp Leu Pro Glu Asn Lys Ile Leu Phe Gly Gly Cys
                   165                 170                 175
   Phe Val Lys Pro Asp Gly Leu Gly Asn Leu Asp Asp Ala Asn Leu Lys
               180                 185                 190
   Ala Trp Pro Lys Ser Ala Lys Ile Leu Met Ser Lys Tyr Gly Lys Ala
           195                 200                 205
   Lys Leu Val Val Ser Gly His Ser Glu Ile Gly Asn Ala Ser Leu Leu
       210                 215                 220
   Lys Leu Thr Trp Glu Gln Ala Val Lys Gly Leu Lys Glu Ser Lys Lys
   225                 230                 235                 240
   Pro Leu Leu Pro Ser Asn
                   245
<210> 104
   <211> 246
   <212> PRT
   <213> RESISTANCE GENE
<400> 104
   Met Lys Lys Leu Phe Val Leu Cys Val Cys Phe Phe Cys Ser Ile Thr
    1               5                  10                  15
   Ala Ala Gly Ala Ala Leu Pro Asp Leu Lys Ile Glu Lys Leu Glu Glu
               20                  25                  30
   Gly Val Phe Val His Thr Ser Phe Glu Glu Val Asn Gly Trp Gly Val
           35                  40                  45
   Val Thr Lys His Gly Leu Val Val Leu Val Asn Thr Asp Ala Tyr Leu
       50                  55                  60
   Ile Asp Thr Pro Phe Thr Ala Thr Asp Thr Glu Lys Leu Val Asn Trp
   65                  70                  75                  80
   Phe Val Glu Arg Gly Tyr Glu Ile Lys Gly Thr Ile Ser Ser His Phe
                   85                  90                  95
   His Ser Asp Ser Thr Gly Gly Ile Glu Trp Leu Asn Ser Gln Ser Ile
               100                 105                 110
   Pro Thr Tyr Ala Ser Glu Leu Thr Asn Glu Leu Leu Lys Lys Ser Gly
           115                 120                 125
   Lys Val Gln Ala Lys Tyr Ser Phe Ser Glu Val Ser Tyr Trp Leu Val
       130                 135                 140
   Lys Asn Lys Ile Glu Val Phe Tyr Pro Gly Pro Gly His Thr Gln Asp
   145                 150                 155                 160
   Asn Leu Val Val Trp Leu Pro Glu Ser Lys Ile Leu Phe Gly Gly Cys
                   165                 170                 175
   Phe Ile Lys Pro His Gly Leu Gly Asn Leu Gly Asp Ala Asn Leu Glu
               180                 185                 190
   Ala Trp Pro Lys Ser Ala Lys Ile Leu Met Ser Lys Tyr Gly Lys Ala
           195                 200                 205
   Lys Leu Val Val Ser Ser His Ser Glu Lys Gly Asp Ala Ser Leu Met
       210                 215                 220
   Lys Arg Thr Trp Glu Gln Ala Leu Lys Gly Leu Lys Glu Ser Lys Lys
   225                 230                 235                 240
   Thr Ser Ser Pro Ser Asn
                   245
<210> 105
   <211> 246
   <212> PRT
   <213> RESISTANCE GENE
<400> 105
   Met Lys Lys Leu Phe Val Leu Cys Ile Phe Leu Phe Cys Ser Ile Thr
    1               5                  10                  15
   Ala Ala Gly Glu Ser Leu Pro Asp Leu Lys Ile Glu Lys Leu Glu Asp
               20                  25                  30
   Gly Val Tyr Val His Thr Ser Phe Glu Glu Val Asn Gly Trp Gly Val
           35                  40                  45
   Val Thr Lys His Gly Leu Val Phe Leu Val Asn Thr Asp Ala Tyr Leu
       50                  55                  60
   Ile Asp Thr Pro Phe Ala Ala Lys Asp Thr Glu Lys Leu Val Asn Trp
   65                  70                  75                  80
   Phe Val Glu Arg Gly Tyr Lys Ile Lys Gly Ser Ile Ser Ser His Phe
                   85                  90                  95
   His Ser Asp Ser Ser Gly Gly Ile Glu Trp Leu Asn Ser Gln Ser Ile
               100                 105                 110
   Pro Thr Tyr Ala Ser Glu Leu Thr Asn Glu Leu Leu Lys Lys Asn Gly
           115                 120                 125
   Lys Val Gln Ala Lys Asn Ser Phe Ser Gly Val Ser Tyr Trp Leu Leu
       130                 135                 140
   Lys Asn Lys Ile Glu Ile Phe Tyr Pro Gly Pro Gly His Thr Gln Asp
   145                 150                 155                 160
   Asn Val Val Val Trp Leu Pro Glu Lys Lys Ile Leu Phe Gly Gly Cys
                   165                 170                 175
   Phe Val Lys Pro Tyr Gly Leu Gly Asn Leu Asp Asp Ala Asn Val Glu
               180                 185                 190
   Ala Trp Pro His Ser Ala Glu Ile Leu Met Ser Arg Tyr Gly Asn Ala
           195                 200                 205
   Lys Leu Val Val Pro Ser His Ser Asp Val Gly Asp Ala Ser Leu Leu
       210                 215                 220
   Lys Leu Thr Trp Glu Gln Ala Val Lys Gly Leu Lys Glu Ser Lys Lys
   225                 230                 235                 240
   Pro Ser Gln Pro Ser Asn
                   245
<210> 106
   <211> 12
   <212> PRT
   <213> RESISTANCE GENE
<400> 106
   Asp Thr Glu Asn Leu Val Asn Trp Phe Val Glu Arg
    1               5                  10
<210> 107
   <211> 10
   <212> PRT
   <213> RESISTANCE GENE
<400> 107
   Glu Ile Leu Phe Gly Gly Cys Phe Val Lys
    1               5                  10
<210> 108
   <211> 10
   <212> PRT
   <213> RESISTANCE GENE
<400> 108
   Glu Val Asn Gly Trp Gly Val Val Pro Lys
    1               5                  10
<210> 109
   <211> 7
   <212> PRT
   <213> RESISTANCE GENE
<400> 109
   Gly Asp Ala Ser Leu Met Lys
    1               5
<210> 110
   <211> 6
   <212> PRT
   <213> RESISTANCE GENE
<400> 110
   Gly Phe Asn Glu Ser Lys
   1 5
<210> 111
   <211> 5
   <212> PRT
   <213> RESISTANCE GENE
<400> 111
   Gly Phe Thr Ile Lys
   1 5
<210> 112
   <211> 6
   <212> PRT
   <213> RESISTANCE GENE
<400> 112
   Gly Leu Asn Glu Ser Lys
   1 5
<210> 113
   <211> 6
   <212> PRT
   <213> RESISTANCE GENE
<400> 113
   Gly Leu Asn Glu Ser Arg
   1 5
<210> 114
   <211> 21
   <212> PRT
   <213> RESISTANCE GENE
<400> 114
   Gly Ser Ile Ser Ser His Phe His Ser Asp Ser Thr Gly Gly Ile Glu
   1 5 10 15
   Trp Leu Asn Ser Arg
   20
<210> 115
   <211> 21
   <212> PRT
   <213> RESISTANCE GENE
<400> 115
   Gly Ser Ile Ser Ser His Phe His Ser Asp Ser Thr Gly Gly Ile Gly
   1 5 10 15
   Trp Leu Asn Ser Arg
   20
<210> 116
   <211> 12
   <212> PRT
   <213> RESISTANCE GENE
<400> 116
   Gly Val Tyr Val His Thr Ser Phe Glu Glu Val Lys
   1 5 10
<210> 117
   <211> 7
   <212> PRT
   <213> RESISTANCE GENE
<400> 117
   Gly Trp Gly Val Val Thr Lys
   1 5
<210> 118
   <211> 7
   <212> PRT
   <213> RESISTANCE GENE
<400> 118
   Gly Trp Ser Val Val Thr Lys
   1 5
<210> 119
   <211> 5
   <212> PRT
   <213> RESISTANCE GENE
<400> 119
   Gly Tyr Glu Ile Lys
   1 5
<210> 120
   <211> 21
   <212> PRT
   <213> RESISTANCE GENE
<400> 120
   His Gly Leu Val Phe Leu Val Asn Thr Asp Ala Tyr Leu Ile Asp Thr
   1 5 10 15
   Pro Phe Ala Ala Lys
   20
<210> 121
   <211> 21
   <212> PRT
   <213> RESISTANCE GENE
<400> 121
   His Gly Leu Val Ile Leu Val Asn Thr Asp Ala Tyr Leu Ile Asp Thr
   1 5 10 15
   Pro Phe Thr Ala Lys
   20
<210> 122
   <211> 21
   <212> PRT
   <213> RESISTANCE GENE
<400> 122
   His Gly Leu Val Val Leu Ile Gly Ala Asp Ala Tyr Leu Ile Asp Thr
   1 5 10 15
   Pro Phe Thr Ala Lys
   20
<210> 123
   <211> 21
   <212> PRT
   <213> RESISTANCE GENE
<400> 123
   His Gly Leu Val Val Leu Val Asp Ala Glu Ala Tyr Leu Ile Asp Thr
   1 5 10 15
   Pro Phe Thr Ala Lys
   20
<210> 124
   <211> 8
   <212> PRT
   <213> RESISTANCE GENE
<400> 124
   His Gly Leu Val Val Leu Val Lys
   1 5
<210> 125
   <211> 21
   <212> PRT
   <213> RESISTANCE GENE
<400> 125
   His Gly Leu Val Val Leu Val Asn Ala Glu Ala Tyr Leu Ile Asp Thr
     1               5                  10                  15
   Pro Phe Thr Ala Lys
               20
<210> 126
   <211> 21
   <212> PRT
   <213> RESISTANCE GENE
<400> 126
   His Gly Leu Val Val Leu Val Asn Asn Asp Ala Tyr Leu Ile Asp Thr
     1               5                  10                  15
   Pro Phe Thr Asn Lys
               20
<210> 127
   <211> 21
   <212> PRT
   <213> RESISTANCE GENE
<400> 127
   His Gly Leu Val Val Leu Val Asn Thr Asp Ala Tyr Leu Ile Asp Thr
     1               5                  10                  15
   Pro Phe Thr Ala Lys
               20
<210> 128
   <211> 21
   <212> PRT
   <213> RESISTANCE GENE
<400> 128
   His Gly Leu Val Val Leu Val Asn Thr Glu Ala Tyr Leu Ile Asp Thr
     1               5                  10                  15
   Pro Phe Thr Ala Lys
               20
<210> 129
   <211> 12
   <212> PRT
   <213> RESISTANCE GENE
<400> 129
   His Ser Phe Asn Gly Val Ser Tyr Ser Leu Ile Lys
     1               5                  10
<210> 130
   <211> 21
   <212> PRT
   <213> RESISTANCE GENE
<400> 130
   Ile Glu Val Phe Tyr Pro Gly Pro Gly His Thr Gln Asp Asn Val Val
   1 5 10 15
   Val Trp Leu Pro Lys
   20
<210> 131
   <211> 9
   <212> PRT
   <213> RESISTANCE GENE
<400> 131
   Ile Leu Phe Gly Gly Cys Phe Ile Lys
   1 5
<210> 132
   <211> 9
   <212> PRT
   <213> RESISTANCE GENE
<400> 132
   Ile Leu Phe Gly Gly Cys Phe Val Lys
   1 5
<210> 133
   <211> 5
   <212> PRT
   <213> RESISTANCE GENE
<400> 133
   Ile Leu Met Glu Lys
   1 5
<210> 134
   <211> 5
   <212> PRT
   <213> RESISTANCE GENE
<400> 134
   Ile Leu Met Ser Lys
   1 5
<210> 135
   <211> 12
   <212> PRT
   <213> RESISTANCE GENE
<400> 135
   Leu Asp Glu Gly Val Tyr Val His Thr Ser Phe Lys
   1 5 10
<210> 136
   <211> 15
   <212> PRT
   <213> RESISTANCE GENE
<400> 136
   Leu Glu Glu Gly Val Tyr Val His Thr Ser Phe Glu Glu Val Lys
   1 5 10 15
<210> 137
   <211> 15
   <212> PRT
   <213> RESISTANCE GENE
<400> 137
   Leu Glu Glu Gly Val Tyr Val His Thr Ser Tyr Glu Glu Val Lys
   1 5 10 15
<210> 138
   <211> 18
   <212> PRT
   <213> RESISTANCE GENE
<400> 138
   Leu Phe Val Leu Cys Val Cys Phe Leu Cys Ser Ile Thr Ala Ala Gly
   1 5 10 15
   Ala Arg
<210> 139
   <211> 5
   <212> PRT
   <213> RESISTANCE GENE
<400> 139
   Leu Leu Ile Ser Lys
   1 5
<210> 140
   <211> 5
   <212> PRT
   <213> RESISTANCE GENE
<400> 140
   Leu Leu Met Ser Lys
   1 5
<210> 141
   <211> 5
   <212> PRT
   <213> RESISTANCE GENE
<400> 141
   Leu Leu Val Ser Lys
   1 5
<210> 142
   <211> 5
   <212> PRT
   <213> RESISTANCE GENE
<400> 142
   Leu Pro Asp Leu Lys
   1 5
<210> 143
   <211> 8
   <212> PRT
   <213> RESISTANCE GENE
<400> 143
   Leu Thr Leu Glu Gln Ala Val Lys
   1 5
<210> 144
   <211> 8
   <212> PRT
   <213> RESISTANCE GENE
<400> 144
   Leu Thr Trp Glu Gln Ala Val Lys
   1 5
<210> 145
   <211> 8
   <212> PRT
   <213> RESISTANCE GENE
<400> 145
   Leu Thr Trp Glu Gln Thr Val Lys
   1 5
<210> 146
   <211> 8
   <212> PRT
   <213> RESISTANCE GENE
<400> 146
   Leu Val Ala Trp Phe Val Gly Arg
   1 5
<210> 147
   <211> 11
   <212> PRT
   <213> RESISTANCE GENE
<400> 147
   Leu Val Asn Trp Phe Ile Glu His Gly Tyr Arg
   1 5 10
<210> 148
   <211> 8
   <212> PRT
   <213> RESISTANCE GENE
<400> 148
   Leu Val Asn Trp Phe Val Glu Arg
   1 5
<210> 149
   <211> 8
   <212> PRT
   <213> RESISTANCE GENE
<400> 149
   Leu Val Thr Trp Phe Val Glu Arg
   1 5
<210> 150
   <211> 16
   <212> PRT
   <213> RESISTANCE GENE
<400> 150
   Leu Val Val Pro Gly His Ser Glu Val Gly Asp Ala Ser Leu Leu Lys
   1 5 10 15
<210> 151
   <211> 16
   <212> PRT
   <213> RESISTANCE GENE
<400> 151
   Leu Val Val Pro Ser His Ser Asp Ile Gly Asp Ala Ser Leu Leu Lys
   1 5 10 15
<210> 152
   <211> 16
   <212> PRT
   <213> RESISTANCE GENE
<400> 152
   Leu Val Val Pro Ser His Ser Asp Ile Gly Asp Ser Ser Leu Leu Lys
   1 5 10 15
<210> 153
   <211> 16
   <212> PRT
   <213> RESISTANCE GENE
<400> 153
   Leu Val Val Pro Ser His Ser Asp Val Gly Asp Ala Ser Leu Leu Lys
   1 5 10 15
<210> 154
   <211> 16
   <212> PRT
   <213> RESISTANCE GENE
<400> 154
   Leu Val Val Pro Ser His Ser Glu Ala Gly Asp Ala Ser Leu Leu Lys
   1 5 10 15
<210> 155
   <211> 16
   <212> PRT
   <213> RESISTANCE GENE
<400> 155
   Leu Val Val Pro Ser His Ser Glu Val Gly Asp Ala Ser Leu Leu Lys
   1 5 10 15
<210> 156
   <211> 16
   <212> PRT
   <213> RESISTANCE GENE
<400> 156
   Leu Val Val Ser Gly His Ser Glu Ile Gly Asn Ala Ser Leu Leu Lys
   1 5 10 15
<210> 157
   <211> 16
   <212> PRT
   <213> RESISTANCE GENE
<400> 157
   Leu Val Val Ser Gly His Ser Glu Thr Gly Asp Ala Thr His Leu Lys
   1 5 10 15
<210> 158
   <211> 16
   <212> PRT
   <213> RESISTANCE GENE
<400> 158
   Leu Val Val Ser Ser His Ser Asp Ile Gly Asp Val Ser Leu Leu Lys
   1 5 10 15
<210> 159
   <211> 16
   <212> PRT
   <213> RESISTANCE GENE
<400> 159
   Leu Val Val Ser Ser His Ser Glu Ile Gly Asp Ala Ser Leu Leu Lys
   1 5 10 15
<210> 160
   <211> 17
   <212> PRT
   <213> RESISTANCE GENE
<400> 160
   Leu Val Val Ser Ser His Ser Glu Ile Gly Asn Ala Ser Leu Leu Gln
   1 5 10 15
   Arg
<210> 161
   <211> 9
   <212> PRT
   <213> RESISTANCE GENE
<400> 161
   Leu Val Val Ser Ser His Ser Glu Lys
   1 5
<210> 162
   <211> 16
   <212> PRT
   <213> RESISTANCE GENE
<400> 162
   Leu Val Val Ser Ser His Ser Glu Thr Gly Asn Ala Ser Leu Leu Lys
   1 5 10 15
<210> 163
   <211> 13
   <212> PRT
   <213> RESISTANCE GENE
<400> 163
   Asn Asp Ala Tyr Leu Ile Asp Thr Pro Ile Thr Ala Lys
   1 5 10
<210> 164
   <211> 12
   <212> PRT
   <213> RESISTANCE GENE
<400> 164
   Asn Ser Phe Asp Gly Val Ser Tyr Trp Leu Ala Lys
   1 5 10
<210> 165
   <211> 12
   <212> PRT
   <213> RESISTANCE GENE
<400> 165
   Asn Ser Phe Gly Gly Val Asn Tyr Trp Leu Val Lys
   1 5 10
<210> 166
   <211> 12
   <212> PRT
   <213> RESISTANCE GENE
<400> 166
   Asn Ser Phe Ser Gly Ala Ser Tyr Trp Leu Val Lys
   1 5 10
<210> 167
   <211> 14
   <212> PRT
   <213> RESISTANCE GENE
<400> 167
   Asn Ser Phe Ser Gly Gly Ser Tyr Trp Leu Val Asn Asn Lys
   1 5 10
<210> 168
   <211> 12
   <212> PRT
   <213> RESISTANCE GENE
<400> 168
   Asn Ser Phe Ser Gly Val Ser Tyr Trp Leu Leu Lys
   1 5 10
<210> 169
   <211> 12
   <212> PRT
   <213> RESISTANCE GENE
<400> 169
   Asn Ser Phe Ser Gly Val Ser Tyr Trp Leu Val Lys
   1 5 10
<210> 170
   <211> 15
   <212> PRT
   <213> RESISTANCE GENE
<400> 170
   Ser Ile Pro Thr Tyr Ala Ser Glu Leu Thr Asn Glu Leu Leu Lys
   1               5                   10                  15
<210> 171
   <211> 7
   <212> PRT
   <213> RESISTANCE GENE
<400> 171
   Thr Leu Glu Gln Ala Val Lys
   1               5
<210> 172
   <211> 7
   <212> PRT
   <213> RESISTANCE GENE
<400> 172
   Thr Trp Glu Gln Ala Leu Lys
   1               5
<210> 173
   <211> 7
   <212> PRT
   <213> RESISTANCE GENE
<400> 173
   Thr Trp Glu Gln Ala Val Lys
   1               5
<210> 174
   <211> 9
   <212> PRT
   <213> RESISTANCE GENE
<400> 174
   Val Leu Phe Gly Gly Cys Phe Val Lys
   1               5
<210> 175
   <211> 16
   <212> PRT
   <213> RESISTANCE GENE
<400> 175
   Val Gln Ala Thr Asn Ser Phe Ser Gly Val Asn Tyr Trp Leu Val Lys
   1               5                   10                  15
<210> 176
   <211> 16
   <212> PRT
   <213> RESISTANCE GENE
<400> 176
   Val Gln Ala Thr Asn Ser Phe Ser Gly Val Ser Tyr Ser Leu Ile Lys
   1 5 10 15
<210> 177
   <211> 16
   <212> PRT
   <213> RESISTANCE GENE
<400> 177
   Val Gln Ala Thr Asn Ser Phe Ser Gly Val Ser Tyr Trp Leu Val Lys
   1 5 10 15
<210> 178
   <211> 5
   <212> PRT
   <213> RESISTANCE GENE
<400> 178
   Tyr Gly Asn Ala Lys
   1 5
<210> 179
   <211> 12
   <212> PRT
   <213> RESISTANCE GENE
<400> 179
   Tyr Ser Phe Ser Glu Val Ser Tyr Trp Leu Val Lys
   1 5 10
<210> 180
   <211> 12
   <212> PRT
   <213> RESISTANCE GENE
<400> 180
   Tyr Ser Phe Ser Gly Val Ser Tyr Trp Leu Val Lys
   1 5 10
<210> 181
   <211> 239
   <212> PRT
   <213> RESISTANCE GENE
<400> 181
   Met Lys Lys Ser Ile Arg Phe Phe Ile Val Ser Ile Leu Leu Ser Pro
    1               5                  10                  15
   Phe Ala Ser Ala Gln Val Lys Asp Phe Val Ile Glu Pro Pro Ile Lys
               20                  25                  30
   Asn Asn Leu His Ile Tyr Lys Thr Phe Gly Val Phe Gly Gly Lys Glu
           35                  40                  45
   Tyr Ser Ala Asn Ser Met Tyr Leu Val Thr Lys Lys Gly Val Val Leu
       50                  55                  60
   Phe Asp Val Pro Trp Glu Lys Ile Gln Tyr Gln Ser Leu Met Asp Thr
    65                  70                  75                  80
   Ile Lys Lys Arg His Asn Leu Pro Val Val Ala Val Phe Ala Thr His
                    85                  90                  95
   Ser His Asp Asp Arg Ala Gly Asp Leu Ser Phe Phe Asn Asn Lys Gly
               100                 105                 110
   Ile Lys Thr Tyr Ala Thr Ala Lys Thr Asn Glu Phe Leu Lys Lys Asp
           115                 120                 125
   Gly Lys Ala Thr Ser Thr Glu Ile Ile Lys Thr Gly Lys Pro Tyr Arg
       130                 135                 140
   Ile Gly Gly Glu Glu Phe Val Val Asp Phe Leu Gly Glu Gly His Thr
   145                 150                 155                 160
   Ala Asp Asn Val Val Val Trp Phe Pro Lys Tyr Asn Val Leu Asp Gly
                   165                 170                 175
   Gly Cys Leu Val Lys Ser Asn Ser Ala Thr Asp Leu Gly Tyr Ile Lys
               180                 185                 190
   Glu Ala Asn Val Glu Gln Trp Pro Lys Thr Ile Asn Lys Leu Lys Ala
           195                 200                 205
   Lys Tyr Ser Lys Ala Thr Leu Ile Ile Pro Gly His Asp Glu Trp Lys
       210                 215                 220
   Gly Gly Gly His Val Glu His Thr Leu Glu Leu Leu Asn Lys Lys
   225                 230                 235
<210> 182
   <211> 243
   <212> PRT
   <213> RESISTANCE GENE
<400> 182
   Met Lys Lys Ser Ile Gln Leu Leu Met Met Ser Met Phe Leu Ser Pro
    1               5                  10                  15
   Leu Ile Asn Ala Gln Val Lys Asp Phe Val Ile Glu Pro Pro Val Lys
               20                  25                  30
   Pro Asn Leu Tyr Leu Tyr Lys Ser Phe Gly Val Phe Gly Gly Lys Glu
           35                  40                  45
   Tyr Ser Ala Asn Ala Val Tyr Leu Thr Thr Lys Lys Gly Val Val Leu
       50                  55                  60
   Phe Asp Val Pro Trp Gln Lys Glu Gln Tyr Gln Thr Leu Met Asp Thr
   65                  70                  75                  80
   Ile Gln Lys Arg His His Leu Pro Val Ile Ala Val Phe Ala Thr His
                   85                  90                  95
   Ser His Asp Asp Arg Ala Gly Asp Leu Ser Phe Tyr Asn Gln Lys Gly
               100                 105                 110
   Ile Lys Thr Tyr Ala Thr Ala Lys Thr Asn Glu Leu Leu Lys Lys Asp
           115                 120                 125
   Gly Lys Ala Thr Ser Thr Glu Ile Ile Lys Thr Gly Lys Pro Tyr Lys
       130                 135                 140
   Ile Gly Gly Glu Glu Phe Met Val Asp Phe Leu Gly Glu Gly His Thr
   145                 150                 155                 160
   Val Asp Asn Val Val Val Trp Phe Pro Lys Tyr Lys Val Leu Asp Gly
                   165                 170                 175
   Gly Cys Leu Val Lys Ser Arg Thr Ala Thr Asp Leu Gly Tyr Thr Gly
               180                 185                 190
   Glu Ala Asn Val Lys Gln Trp Pro Glu Thr Met Arg Lys Leu Lys Thr
           195                 200                 205
   Lys Tyr Ala Gln Ala Thr Leu Val Ile Pro Gly His Asp Glu Trp Lys
       210                 215                 220
   Gly Gly Gly His Val Gln His Thr Leu Asp Leu Leu Asp Lys Asn Lys
   225                 230                 235                 240
   Lys Pro Glu
<210> 183
   <211> 243
   <212> PRT
   <213> RESISTANCE GENE
<400> 183
   Met Lys Lys Ser Ile Gln Leu Leu Met Met Ser Met Phe Leu Ser Pro
    1               5                  10                  15
   Leu Ile Asn Ala Gln Val Lys Asp Phe Val Ile Glu Pro Pro Val Lys
               20                  25                  30
   Pro Asn Leu Tyr Leu Tyr Lys Ser Phe Gly Val Phe Gly Gly Lys Glu
           35                  40                  45
   Tyr Ser Ala Asn Ala Val Tyr Leu Thr Thr Lys Lys Gly Val Val Leu
       50                  55                  60
   Phe Asp Val Pro Trp Gln Lys Glu Gln Tyr Gln Thr Leu Met Asp Thr
   65                  70                  75                  80
   Ile Gln Lys Arg His His Leu Pro Val Ile Ala Val Phe Ala Thr His
                   85                  90                  95
   Ser His Asp Asp Arg Ala Gly Asp Leu Ser Phe Tyr Asn Gln Lys Gly
               100                 105                 110
   Ile Lys Thr Tyr Ala Thr Ala Lys Thr Asn Glu Leu Leu Lys Lys Asp
           115                 120                 125
   Gly Lys Ala Thr Ser Thr Glu Ile Ile Lys Thr Gly Lys Pro Tyr Lys
       130                 135                 140
   Ile Gly Gly Glu Glu Phe Met Val Asp Phe Leu Gly Glu Gly His Thr
   145                 150                 155                 160
   Val Asp Asn Val Val Val Trp Phe Pro Lys Tyr Lys Val Leu Asp Gly
                   165                 170                 175
   Gly Cys Leu Val Lys Ser Arg Thr Ala Thr Asp Leu Gly Tyr Thr Gly
               180                 185                 190
   Glu Ala Asn Val Lys Gln Trp Pro Glu Thr Met Arg Lys Leu Lys Thr
           195                 200                 205
   Lys Tyr Ala Gln Ala Thr Leu Val Ile Pro Gly His Glu Glu Trp Lys
       210                 215                 220
   Gly Gly Gly His Val Gln His Thr Leu Asp Leu Leu Asp Lys Asn Lys
   225                 230                 235                 240
   Lys Pro Glu
<210> 184
   <211> 239
   <212> PRT
   <213> RESISTANCE GENE
<400> 184
   Met Lys Lys Arg Ile Gln Phe Phe Met Val Ser Met Met Leu Ser Ser
    1               5                  10                  15
   Leu Phe Ser Ala Gln Val Lys Asp Phe Val Ile Glu Pro Pro Ile Lys
               20                  25                  30
   Lys Asn Leu His Ile Tyr Lys Thr Phe Gly Val Phe Gly Gly Lys Glu
           35                  40                  45
   Tyr Ser Ala Asn Ser Val Tyr Leu Val Thr Gln Lys Gly Val Val Leu
       50                  55                  60
   Phe Asp Val Pro Trp Glu Lys Val Gln Tyr Gln Ser Leu Met Asp Thr
   65                  70                  75                  80
   Ile Gln Lys Arg His Asn Leu Pro Val Ile Ala Val Phe Ala Thr His
                   85                  90                  95
   Ser His Asp Asp Arg Ala Gly Asp Leu Ser Phe Phe Asn Asn Lys Gly
               100                 105                 110
   Ile Lys Thr Tyr Ala Thr Ser Lys Thr Asn Glu Phe Leu Lys Lys Asp
           115                 120                 125
   Gly Lys Ala Thr Ser Thr Glu Ile Ile Lys Thr Gly Lys Pro Tyr Arg
       130                 135                 140
   Ile Gly Gly Glu Glu Phe Val Val Asp Phe Leu Gly Glu Gly His Thr
   145                 150                 155                 160
   Ala Asp Asn Val Val Val Trp Phe Pro Lys Tyr Asn Val Leu Asp Gly
                   165                 170                 175
   Gly Cys Leu Val Lys Ser Lys Ala Ala Thr Asp Leu Gly Tyr Ile Lys
               180                 185                 190
   Glu Ala Asn Val Glu Gln Trp Pro Lys Thr Ile Asn Lys Leu Lys Ser
           195                 200                 205
   Lys Tyr Ser Lys Ala Ser Leu Val Ile Pro Gly His Asp Glu Trp Lys
       210                 215                 220
   Gly Gly Gly His Val Lys His Thr Leu Glu Leu Leu Asn Lys Lys
   225                 230                 235
<210> 185
   <211> 242
   <212> PRT
   <213> RESISTANCE GENE
<400> 185
   Met Arg Lys Asn Val Arg Ile Phe Thr Val Leu Ser Leu Phe Leu Ile
    1               5                  10                  15
   Asn Phe Phe Asn Ala Gln Ala Arg Asp Phe Val Ile Glu Gln Pro Phe
               20                  25                  30
   Gly Lys Gln Leu Tyr Leu Tyr Lys Thr Phe Gly Val Phe Asp Gly Lys
           35                  40                  45
   Glu Tyr Ser Thr Asn Ala Leu Tyr Leu Val Thr Lys Lys Gly Val Val
       50                  55                  60
   Leu Phe Asp Val Pro Trp Gln Lys Thr Gln Tyr Gln Ser Leu Met Asp
   65                  70                  75                  80
   Thr Ile Lys Lys Arg His Asn Leu Pro Val Ile Ala Val Phe Ala Thr
                   85                  90                  95
   His Ser His Ser Asp Arg Ala Gly Asp Leu Ser Phe Tyr Asn Lys Lys
               100                 105                 110
   Gly Ile Pro Thr Tyr Ala Thr Ala Lys Thr Asn Glu Leu Leu Lys Lys
           115                 120                 125
   Glu Gly Lys Ala Thr Ser Ser Lys Leu Thr Lys Ile Gly Lys Lys Tyr
       130                 135                 140
   Lys Ile Gly Gly Glu Glu Phe Thr Val Asp Phe Leu Gly Glu Gly His
   145                 150                 155                 160
   Thr Ala Asp Asn Val Val Val Trp Phe Pro Lys Tyr Asn Val Leu Asp
                   165                 170                 175
   Gly Gly Cys Leu Val Lys Ser Ser Ala Ala Val Asp Leu Gly Tyr Thr
               180                 185                 190
   Gly Glu Ala Asn Val Glu Gln Trp Pro Ala Thr Met Lys Lys Leu Gln
           195                 200                 205
   Ala Lys Tyr Pro Ser Thr Ala Lys Val Ile Pro Gly His Asp Glu Trp
       210                 215                 220
   Lys Gly Asn Asp His Val Lys His Thr Leu Glu Leu Leu Asp Gln Gln
   225                 230                 235                 240
   Lys Gln
<210> 186
   <211> 239
   <212> PRT
   <213> RESISTANCE GENE
<400> 186
   Met Lys Lys Arg Ile Gln Phe Phe Met Val Ser Met Met Leu Ala Pro
    1               5                  10                  15
   Met Phe Asn Ala Gln Val Lys Asp Phe Val Ile Glu Pro Pro Ile Lys
               20                  25                  30
   Asn Asn Leu His Ile Tyr Lys Thr Phe Gly Val Phe Gly Gly Lys Glu
           35                  40                  45
   Tyr Ser Ala Asn Ser Val Tyr Leu Val Thr Lys Lys Gly Val Val Leu
       50                  55                  60
   Phe Asp Val Pro Trp Glu Lys Ala Gln Tyr Gln Ser Leu Met Asp Thr
   65                  70                  75                  80
   Ile Lys Lys Arg His Asn Leu Pro Val Ile Ala Val Phe Ala Thr His
                   85                  90                  95
   Ser His Asp Asp Arg Ala Gly Asp Leu Ser Phe Phe Asn Asn Lys Gly
               100                 105                 110
   Ile Lys Thr Tyr Ala Thr Ser Lys Thr Asn Glu Phe Leu Lys Lys Asp
           115                 120                 125
   Gly Lys Ala Thr Ser Thr Glu Ile Ile Lys Thr Gly Lys Pro Tyr Arg
       130                 135                 140
   Ile Gly Gly Glu Glu Phe Thr Val Asp Phe Leu Gly Glu Gly His Thr
   145                 150                 155                 160
   Ala Asp Asn Val Val Val Trp Phe Pro Lys Tyr Asn Val Leu Asp Gly
                   165                 170                 175
   Gly Cys Leu Val Lys Ser Asn Ser Ala Thr Asp Leu Gly Tyr Ile Lys
               180                 185                 190
   Glu Ala Asn Val Glu Gln Trp Pro Ile Thr Ile Asp Lys Leu Lys Ala
           195                 200                 205
   Lys Tyr Ser Lys Ala Thr Leu Ile Ile Pro Gly His Asp Asp Trp Lys
       210                 215                 220
   Gly Gly Gly His Val Glu His Thr Leu Glu Leu Leu Asn Lys Lys
   225                 230                 235
<210> 187
   <211> 239
   <212> PRT
   <213> RESISTANCE GENE
<400> 187
   Met Lys Arg Arg Ile Gln Phe Phe Met Val Ser Met Met Leu Thr Pro
    1               5                  10                  15
   Leu Phe Ser Ala Gln Val Lys Asp Phe Val Ile Glu Pro Pro Ile Lys
               20                  25                  30
   Lys Asn Leu Tyr Ile Tyr Lys Thr Phe Gly Val Phe Gly Gly Lys Glu
           35                  40                  45
   Tyr Ser Ala Asn Ser Val Tyr Leu Val Thr Lys Thr Gly Val Val Leu
       50                  55                  60
   Phe Asp Val Pro Trp Glu Lys Ala Gln Tyr Gln Ser Leu Met Asp Thr
   65                  70                  75                  80
   Ile Lys Lys Arg His Asn Leu Pro Val Val Ala Val Phe Ala Thr His
                   85                  90                  95
   Ser His Asp Asp Arg Ala Gly Asp Leu Ser Phe Phe Asn Asn Lys Gly
               100                 105                 110
   Ile Lys Thr Tyr Ala Thr Pro Lys Thr Asn Gln Phe Leu Lys Arg Asp
           115                 120                 125
   Gly Lys Ala Thr Ser Thr Glu Leu Ile Lys Pro Gly Lys Pro Tyr Arg
       130                 135                 140
   Phe Gly Gly Glu Glu Phe Val Val Asp Phe Leu Gly Glu Gly His Thr
   145                 150                 155                 160
   Ala Asp Asn Val Val Val Trp Phe Pro Lys Tyr Lys Val Leu Asp Gly
                   165                 170                 175
   Gly Cys Leu Val Lys Ser Asn Ser Ala Thr Asp Leu Gly Tyr Ile Lys
               180                 185                 190
   Glu Ala Asn Leu Glu Gln Trp Pro Lys Thr Met His Lys Leu Lys Thr
           195                 200                 205
   Lys Tyr Ser Glu Ala Val Leu Ile Ile Pro Gly His Asp Glu Trp Lys
       210                 215                 220
   Gly Gly Gly His Val Glu His Thr Leu Glu Leu Leu Asp Lys Lys
   225                 230                 235
<210> 188
   <211> 9
   <212> PRT
   <213> RESISTANCE GENE
<400> 188
   Ala Ala Thr Asp Leu Gly Tyr Ile Lys
    1               5
<210> 189
   <211> 10
   <212> PRT
   <213> RESISTANCE GENE
<400> 189
   Ala Gly Asp Leu Ser Phe Phe Asn Asn Lys
    1               5                  10
<210> 190
   <211> 9
   <212> PRT
   <213> RESISTANCE GENE
<400> 190
   Ala Gly Asp Leu Ser Phe Tyr Asn Lys
   1 5
<210> 191
   <211> 10
   <212> PRT
   <213> RESISTANCE GENE
<400> 191
   Ala Gly Asp Leu Ser Phe Tyr Asn Gln Lys
   1 5 10
<210> 192
   <211> 11
   <212> PRT
   <213> RESISTANCE GENE
<400> 192
   Ala Gln Tyr Gln Ser Leu Met Asp Thr Ile Lys
   1 5 10
<210> 193
   <211> 12
   <212> PRT
   <213> RESISTANCE GENE
<400> 193
   Ala Ser Leu Val Ile Pro Gly His Asp Glu Trp Lys
   1 5 10
<210> 194
   <211> 12
   <212> PRT
   <213> RESISTANCE GENE
<400> 194
   Ala Thr Leu Ile Ile Pro Gly His Asp Asp Trp Lys
   1 5 10
<210> 195
   <211> 12
   <212> PRT
   <213> RESISTANCE GENE
<400> 195
   Ala Thr Leu Ile Ile Pro Gly His Asp Glu Trp Lys
   1 5 10
<210> 196
   <211> 5
   <212> PRT
   <213> RESISTANCE GENE
<400> 196
   Ala Thr Ser Ser Lys
   1 5
<210> 197
   <211> 8
   <212> PRT
   <213> RESISTANCE GENE
<400> 197
   Ala Thr Ser Thr Glu Ile Ile Lys
   1 5
<210> 198
   <211> 8
   <212> PRT
   <213> RESISTANCE GENE
<400> 198
   Ala Thr Ser Thr Glu Leu Ile Lys
   1 5
<210> 199
   <211> 11
   <212> PRT
   <213> RESISTANCE GENE
<400> 199
   Ala Thr Ser Thr Glu Leu Ile Lys Pro Gly Lys
   1 5 10
<210> 200
   <211> 14
   <212> PRT
   <213> RESISTANCE GENE
<400> 200
   Ala Thr Ser Thr Glu Leu Ile Lys Pro Gly Lys Pro Tyr Arg
   1 5 10
<210> 201
   <211> 9
   <212> PRT
   <213> RESISTANCE GENE
<400> 201
   Asp Phe Val Ile Glu Pro Pro Ile Lys
   1 5
<210> 202
   <211> 9
   <212> PRT
   <213> RESISTANCE GENE
<400> 202
   Asp Phe Val Ile Glu Pro Pro Val Lys
   1 5
<210> 203
   <211> 16
   <212> PRT
   <213> RESISTANCE GENE
<400> 203
   Asp Phe Val Ile Glu Pro Pro Val Lys Pro Asn Leu Tyr Leu Tyr Lys
   1 5 10 15
<210> 204
   <211> 10
   <212> PRT
   <213> RESISTANCE GENE
<400> 204
   Asp Phe Val Ile Glu Gln Pro Phe Gly Lys
   1 5 10
<210> 205
   <211> 9
   <212> PRT
   <213> RESISTANCE GENE
<400> 205
   Glu Ala Asn Leu Glu Gln Trp Pro Lys
   1 5
<210> 206
   <211> 13
   <212> PRT
   <213> RESISTANCE GENE
<400> 206
   Glu Ala Asn Val Glu Gln Trp Pro Ile Thr Ile Asp Lys
   1 5 10
<210> 207
   <211> 9
   <212> PRT
   <213> RESISTANCE GENE
<400> 207
   Glu Ala Asn Val Glu Gln Trp Pro Lys
   1 5
<210> 208
   <211> 12
   <212> PRT
   <213> RESISTANCE GENE
<400> 208
   Glu Gln Tyr Gln Thr Leu Met Asp Thr Ile Gln Lys
   1 5 10
<210> 209
   <211> 12
   <212> PRT
   <213> RESISTANCE GENE
<400> 209
   Glu Tyr Ser Ala Asn Ala Val Tyr Leu Thr Thr Lys
   1 5 10
<210> 210
   <211> 12
   <212> PRT
   <213> RESISTANCE GENE
<400> 210
   Glu Tyr Ser Ala Asn Ser Met Tyr Leu Val Thr Lys
   1 5 10
<210> 211
   <211> 12
   <212> PRT
   <213> RESISTANCE GENE
<400> 211
   Glu Tyr Ser Ala Asn Ser Val Tyr Leu Val Thr Lys
   1 5 10
<210> 212
   <211> 13
   <212> PRT
   <213> RESISTANCE GENE
<400> 212
   Glu Tyr Ser Ala Asn Ser Val Tyr Leu Val Thr Gln Lys
   1 5 10
<210> 213
   <211> 12
   <212> PRT
   <213> RESISTANCE GENE
<400> 213
   Glu Tyr Ser Thr Asn Ala Leu Tyr Leu Val Thr Lys
   1 5 10
<210> 214
   <211> 17
   <212> PRT
   <213> RESISTANCE GENE
<400> 214
   Phe Phe Ile Val Ser Ile Leu Leu Ser Pro Phe Ala Ser Ala Gln Val
    1               5                   10                  15
   Lys
<210> 215
   <211> 14
   <212> PRT
   <213> RESISTANCE GENE
<400> 215
   Gly Gly Gly His Val Glu His Thr Leu Glu Leu Leu Asp Lys
    1               5                   10
<210> 216
   <211> 14
   <212> PRT
   <213> RESISTANCE GENE
<400> 216
   Gly Gly Gly His Val Glu His Thr Leu Glu Leu Leu Asn Lys
    1               5                   10
<210> 217
   <211> 6
   <212> PRT
   <213> RESISTANCE GENE
<400> 217
   Gly Gly Gly His Val Lys
    1               5
<210> 218
   <211> 14
   <212> PRT
   <213> RESISTANCE GENE
<400> 218
   Gly Gly Gly His Val Gln His Thr Leu Asp Leu Leu Asp Lys
    1               5                   10
<210> 219
   <211> 9
   <212> PRT
   <213> RESISTANCE GENE
<400> 219
   Gly Ile Pro Thr Tyr Ala Thr Ala Lys
   1 5
<210> 220
   <211> 6
   <212> PRT
   <213> RESISTANCE GENE
<400> 220
   Gly Asn Asp His Val Lys
   1 5
<210> 221
   <211> 11
   <212> PRT
   <213> RESISTANCE GENE
<400> 221
   Gly Val Val Leu Phe Asp Val Pro Trp Glu Lys
   1 5 10
<210> 222
   <211> 11
   <212> PRT
   <213> RESISTANCE GENE
<400> 222
   Gly Val Val Leu Phe Asp Val Pro Trp Gln Lys
   1 5 10
<210> 223
   <211> 17
   <212> PRT
   <213> RESISTANCE GENE
<400> 223
   His His Leu Pro Val Ile Ala Val Phe Ala Thr His Ser His Asp Asp
   1 5 10 15
   Arg
<210> 224
   <211> 17
   <212> PRT
   <213> RESISTANCE GENE
<400> 224
   His Asn Leu Pro Val Ile Ala Val Phe Ala Thr His Ser His Asp Asp
   1 5 10 15
   Arg
<210> 225
   <211> 17
   <212> PRT
   <213> RESISTANCE GENE
<400> 225
   His Asn Leu Pro Val Ile Ala Val Phe Ala Thr His Ser His Ser Asp
    1               5                  10                  15
   Arg
<210> 226
   <211> 17
   <212> PRT
   <213> RESISTANCE GENE
<400> 226
   His Asn Leu Pro Val Val Ala Val Phe Ala Thr His Ser His Asp Asp
    1               5                  10                  15
   Arg
<210> 227
   <211> 10
   <212> PRT
   <213> RESISTANCE GENE
<400> 227
   His Thr Leu Glu Leu Leu Asp Gln Gln Lys
    1               5                  10
<210> 228
   <211> 8
   <212> PRT
   <213> RESISTANCE GENE
<400> 228
   His Thr Leu Glu Leu Leu Asn Lys
    1               5
<210> 229
   <211> 18
   <212> PRT
   <213> RESISTANCE GENE
<400> 229
   Ile Phe Thr Val Leu Ser Leu Phe Leu Ile Asn Phe Phe Asn Ala Gln
    1               5                  10                  15
   Ala Arg
<210> 230
   <211> 19
   <212> PRT
   <213> RESISTANCE GENE
<400> 230
   Ile Gln Phe Phe Met Val Ser Met Met Leu Ala Pro Met Phe Asn Ala
   1               5                   10                  15
   Gln Val Lys
<210> 231
   <211> 19
   <212> PRT
   <213> RESISTANCE GENE
<400> 231
   Ile Gln Phe Phe Met Val Ser Met Met Leu Ser Ser Leu Phe Ser Ala
   1               5                   10                  15
   Gln Val Lys
<210> 232
   <211> 19
   <212> PRT
   <213> RESISTANCE GENE
<400> 232
   Ile Gln Phe Phe Met Val Ser Met Met Leu Thr Pro Leu Phe Ser Ala
   1               5                   10                  15
   Gln Val Lys
<210> 233
   <211> 11
   <212> PRT
   <213> RESISTANCE GENE
<400> 233
   Ile Gln Tyr Gln Ser Leu Met Asp Thr Ile Lys
   1               5                   10
<210> 234
   <211> 6
   <212> PRT
   <213> RESISTANCE GENE
<400> 234
   Asn Leu His Ile Tyr Lys
   1               5
<210> 235
   <211> 6
   <212> PRT
   <213> RESISTANCE GENE
<400> 235
   Asn Leu Tyr Ile Tyr Lys
   1 5
<210> 236
   <211> 7
   <212> PRT
   <213> RESISTANCE GENE
<400> 236
   Asn Asn Leu His Ile Tyr Lys
   1 5
<210> 237
   <211> 6
   <212> PRT
   <213> RESISTANCE GENE
<400> 237
   Gln Leu Tyr Leu Tyr Lys
   1 5
<210> 238
   <211> 7
   <212> PRT
   <213> RESISTANCE GENE
<400> 238
   Gln Trp Pro Glu Thr Met Arg
   1 5
<210> 239
   <211> 8
   <212> PRT
   <213> RESISTANCE GENE
<400> 239
   Ser Phe Gly Val Phe Gly Gly Lys
   1 5
<210> 240
   <211> 20
   <212> PRT
   <213> RESISTANCE GENE
<400> 240
   Ser Ile Gln Leu Leu Met Met Ser Met Phe Leu Ser Pro Leu Ile Asn
   1 5 10 15
   Ala Gln Val Lys
   20
<210> 241
   <211> 11
   <212> PRT
   <213> RESISTANCE GENE
<400> 241
   Ser Asn Ser Ala Thr Asp Leu Gly Tyr Ile Lys
   1 5 10
<210> 242
   <211> 14
   <212> PRT
   <213> RESISTANCE GENE
<400> 242
   Thr Ala Thr Asp Leu Gly Tyr Thr Gly Glu Ala Asn Val Lys
   1 5 10
<210> 243
   <211> 8
   <212> PRT
   <213> RESISTANCE GENE
<400> 243
   Thr Phe Gly Val Phe Asp Gly Lys
   1 5
<210> 244
   <211> 8
   <212> PRT
   <213> RESISTANCE GENE
<400> 244
   Thr Phe Gly Val Phe Gly Gly Lys
   1 5
<210> 245
   <211> 6
   <212> PRT
   <213> RESISTANCE GENE
<400> 245
   Thr Gly Lys Pro Tyr Lys
   1 5
<210> 246
   <211> 6
   <212> PRT
   <213> RESISTANCE GENE
<400> 246
   Thr Gly Lys Pro Tyr Arg
   1 5
<210> 247
   <211> 12
   <212> PRT
   <213> RESISTANCE GENE
<400> 247
   Thr Gly Val Val Leu Phe Asp Val Pro Trp Glu Lys
   1 5 10
<210> 248
   <211> 6
   <212> PRT
   <213> RESISTANCE GENE
<400> 248
   Thr Asn Glu Phe Leu Lys
   1 5
<210> 249
   <211> 6
   <212> PRT
   <213> RESISTANCE GENE
<400> 249
   Thr Asn Glu Leu Leu Lys
   1 5
<210> 250
   <211> 6
   <212> PRT
   <213> RESISTANCE GENE
<400> 250
   Thr Asn Gln Phe Leu Lys
   1 5
<210> 251
   <211> 11
   <212> PRT
   <213> RESISTANCE GENE
<400> 251
   Thr Gln Tyr Gln Ser Leu Met Asp Thr Ile Lys
   1 5 10
<210> 252
   <211> 6
   <212> PRT
   <213> RESISTANCE GENE
<400> 252
   Thr Tyr Ala Thr Ala Lys
   1 5
<210> 253
   <211> 6
   <212> PRT
   <213> RESISTANCE GENE
<400> 253
   Thr Tyr Ala Thr Pro Lys
   1 5
<210> 254
   <211> 6
   <212> PRT
   <213> RESISTANCE GENE
<400> 254
   Thr Tyr Ala Thr Ser Lys
   1 5
<210> 255
   <211> 9
   <212> PRT
   <213> RESISTANCE GENE
<400> 255
   Val Ile Pro Gly His Asp Glu Trp Lys
   1 5
<210> 256
   <211> 9
   <212> PRT
   <213> RESISTANCE GENE
<400> 256
   Val Leu Asp Gly Gly Cys Leu Val Lys
   1 5
<210> 257
   <211> 12
   <212> PRT
   <213> RESISTANCE GENE
<400> 257
   Val Gln Tyr Gln Ser Leu Met Asp Thr Ile Gln Lys
   1 5 10
<210> 258
   <211> 15
   <212> PRT
   <213> RESISTANCE GENE
<400> 258
   Tyr Ala Gln Ala Thr Leu Val Ile Pro Gly His Asp Glu Trp Lys
   1 5 10 15
<210> 259
   <211> 15
   <212> PRT
   <213> RESISTANCE GENE
<400> 259
   Tyr Ala Gln Ala Thr Leu Val Ile Pro Gly His Glu Glu Trp Lys
   1 5 10 15
<210> 260
   <211> 11
   <212> PRT
   <213> RESISTANCE GENE
<400> 260
   Tyr Asn Val Leu Asp Gly Gly Cys Leu Val Lys
   1 5 10
<210> 261
   <211> 6
   <212> PRT
   <213> RESISTANCE GENE
<400> 261
   Tyr Pro Ser Thr Ala Lys
   1 5
<210> 262
   <211> 15
   <212> PRT
   <213> RESISTANCE GENE
<400> 262
   Tyr Ser Glu Ala Val Leu Ile Ile Pro Gly His Asp Glu Trp Lys
   1 5 10 15
<210> 263
   <211> 294
   <212> PRT
   <213> RESISTANCE GENE
<400> 263
   Met Ser Asn Lys Val Asn Phe Lys Thr Ala Ser Phe Leu Phe Ser Val
    1               5                  10                  15
   Cys Leu Ala Leu Ser Ala Phe Asn Ala His Ala Asn Lys Ser Asp Ala
               20                  25                  30
   Ala Ala Lys Gln Ile Lys Lys Leu Glu Glu Asp Phe Asp Gly Arg Ile
           35                  40                  45
   Gly Val Phe Ala Ile Asp Thr Gly Ser Gly Asn Thr Phe Gly Tyr Arg
       50                  55                  60
   Ser Asp Glu Arg Phe Pro Leu Cys Ser Ser Phe Lys Gly Phe Leu Ala
   65                  70                  75                  80
   Ala Ala Val Leu Glu Arg Val Gln Gln Lys Lys Leu Asp Ile Asn Gln
                   85                  90                  95
   Lys Val Lys Tyr Glu Ser Arg Asp Leu Glu Tyr His Ser Pro Ile Thr
               100                 105                 110
   Thr Lys Tyr Lys Gly Ser Gly Met Thr Leu Gly Asp Met Ala Ser Ala
           115                 120                 125
   Ala Leu Gln Tyr Ser Asp Asn Gly Ala Thr Asn Ile Ile Met Glu Arg
       130                 135                 140
   Phe Leu Gly Gly Pro Glu Gly Met Thr Lys Phe Met Arg Ser Ile Gly
   145                 150                 155                 160
   Asp Asn Glu Phe Arg Leu Asp Arg Trp Glu Leu Glu Leu Asn Thr Ala
                   165                 170                 175
   Ile Pro Gly Asp Lys Arg Asp Thr Ser Thr Pro Lys Ala Val Ala Asn
               180                 185                 190
   Ser Leu Asn Lys Leu Ala Leu Gly Asn Val Leu Asn Ala Lys Glu Lys
           195                 200                 205
   Ala Ile Tyr Gln Asn Trp Leu Lys Gly Asn Thr Thr Gly Asp Ala Arg
       210                 215                 220
   Ile Arg Ala Ser Val Pro Ala Asp Trp Val Val Gly Asp Lys Thr Gly
   225                 230                 235                 240
   Ser Cys Gly Ala Tyr Gly Thr Ala Asn Asp Tyr Ala Val Ile Trp Pro
                   245                 250                 255
   Lys Asn Arg Ala Pro Leu Ile Val Ser Ile Tyr Thr Thr Arg Lys Ser
               260                 265                 270
   Lys Asp Asp Lys His Ser Asp Lys Thr Ile Ala Glu Ala Ser Arg Ile
           275                 280                 285
   Ala Ile Gln Ala Ile Asp
       290
<210> 264
   <211> 294
   <212> PRT
   <213> RESISTANCE GENE
<400> 264
   Met Ser Asn Lys Val Asn Phe Lys Thr Ala Ser Phe Leu Phe Ser Val
    1               5                  10                  15
   Cys Leu Ala Leu Ser Ala Phe Asn Ala His Ala Asn Lys Ser Asp Ala
               20                  25                  30
   Ala Ala Lys Gln Ile Lys Lys Leu Glu Glu Asp Phe Asp Gly Arg Ile
           35                  40                  45
   Gly Val Phe Ala Ile Asp Thr Gly Ser Gly Asn Thr Phe Gly Tyr Arg
       50                  55                  60
   Ser Asp Glu Arg Phe Pro Leu Cys Ser Ser Phe Lys Gly Phe Leu Ala
   65                  70                  75                  80
   Ala Ala Val Leu Glu Arg Val Gln Gln Lys Lys Leu Asp Ile Asn Gln
                   85                  90                  95
   Lys Val Lys Tyr Glu Ser Arg Asp Leu Glu Tyr Tyr Ser Pro Ile Thr
               100                 105                 110
   Thr Lys Tyr Lys Gly Ser Gly Met Thr Leu Gly Asp Met Ala Ser Ala
           115                 120                 125
   Ala Leu Gln Tyr Ser Asp Asn Gly Ala Thr Asn Ile Ile Met Glu Arg
       130                 135                 140
   Phe Leu Gly Gly Pro Glu Gly Met Thr Lys Phe Met Arg Ser Ile Gly
   145                 150                 155                 160
   Asp Asn Glu Phe Arg Leu Asp Arg Trp Glu Leu Glu Leu Asn Thr Ala
                   165                 170                 175
   Ile Pro Gly Asp Lys Arg Asp Thr Ser Thr Pro Lys Ala Val Ala Asn
               180                 185                 190
   Ser Leu Asn Lys Leu Ala Leu Gly Asn Val Leu Asn Ala Lys Val Lys
           195                 200                 205
   Ala Ile Tyr Gln Asn Trp Leu Lys Gly Asn Thr Thr Gly Asp Ala Arg
       210                 215                 220
   Ile Arg Ala Ser Val Pro Ala Asp Trp Val Val Gly Asp Lys Thr Gly
   225                 230                 235                 240
   Ser Cys Gly Ala Tyr Gly Thr Ala Asn Asp Tyr Ala Val Ile Trp Pro
                   245                 250                 255
   Lys Asn Arg Ala Pro Leu Ile Val Ser Ile Tyr Thr Thr Arg Lys Ser
               260                 265                 270
   Lys Asp Asp Lys His Ser Asp Lys Thr Ile Ala Glu Ala Ser Arg Ile
           275                 280                 285
   Ala Ile Gln Ala Ile Asp
       290
<210> 265
   <211> 294
   <212> PRT
   <213> RESISTANCE GENE
<400> 265
   Met Ser Asn Lys Val Asn Phe Lys Thr Ala Ser Phe Leu Phe Ser Val
   1               5                   10                  15
   Cys Leu Ala Leu Ser Ala Phe Asn Ala His Ala Asn Lys Ser Asp Ala
               20                  25                  30
   Ala Ala Lys Gln Ile Lys Lys Leu Glu Glu Asp Phe Asp Gly Arg Ile
           35                  40                  45
   Gly Val Phe Ala Ile Asp Thr Gly Ser Gly Asn Thr Phe Gly Tyr Arg
       50                  55                  60
   Ser Asp Glu Arg Phe Pro Leu Cys Ser Ser Phe Lys Gly Phe Leu Ala
   65                  70                  75                  80
   Ala Ala Val Leu Glu Arg Val Gln Gln Lys Lys Leu Asp Ile Asn Gln
                   85                  90                  95
   Lys Val Lys Tyr Glu Ser Arg Asp Leu Glu Tyr His Ser Pro Ile Thr
               100                 105                 110
   Thr Lys Tyr Lys Gly Ser Gly Met Thr Leu Gly Asp Met Ala Ser Ala
           115                 120                 125
   Ala Leu Gln Tyr Ser Asp Asn Gly Ala Thr Asn Ile Ile Met Glu Arg
       130                 135                 140
   Phe Leu Gly Gly Pro Glu Gly Met Thr Lys Phe Met Arg Ser Ile Gly
   145                 150                 155                 160
   Asp Asn Glu Phe Arg Leu Asp Arg Trp Glu Leu Glu Leu Asn Thr Ala
                   165                 170                 175
   Ile Pro Gly Asp Lys Arg Asp Thr Ser Thr Pro Lys Ala Val Ala Asn
               180                 185                 190
   Ser Leu Asn Lys Leu Ala Leu Gly Asn Val Leu Asn Ala Lys Val Lys
           195                 200                 205
   Ala Ile Tyr Gln Asn Trp Leu Lys Gly Asn Thr Thr Gly Asp Ala Arg
       210                 215                 220
   Ile Arg Ala Ser Val Pro Ala Asp Trp Val Val Gly Asp Lys Thr Gly
   225                 230                 235                 240
   Ser Cys Gly Ala Ile Gly Thr Ala Asn Asp Tyr Ala Val Ile Trp Pro
                   245                 250                 255
   Lys Asn Arg Ala Pro Leu Ile Val Ser Ile Tyr Thr Thr Arg Lys Ser
               260                 265                 270
   Lys Asp Asp Lys His Ser Asp Lys Thr Ile Ala Glu Ala Ser Arg Ile
           275                 280                 285
   Ala Ile Gln Ala Ile Asp
       290
<210> 266
   <211> 8
   <212> PRT
   <213> RESISTANCE GENE
<400> 266
   Ala Ile Tyr Gln Asn Trp Leu Lys
   1               5
<210> 267
   <211> 11
   <212> PRT
   <213> RESISTANCE GENE
<400> 267
   Ala Pro Leu Ile Val Ser Ile Tyr Thr Thr Arg
   1               5                   10
<210> 268
   <211> 12
   <212> PRT
   <213> RESISTANCE GENE
<400> 268
   Ala Ser Val Pro Ala Asp Trp Val Val Gly Asp Lys
   1 5 10
<210> 269
   <211> 8
   <212> PRT
   <213> RESISTANCE GENE
<400> 269
   Ala Val Ala Asn Ser Leu Asn Lys
   1 5
<210> 270
   <211> 11
   <212> PRT
   <213> RESISTANCE GENE
<400> 270
   Asp Leu Glu Tyr His Ser Pro Ile Thr Thr Lys
   1 5 10
<210> 271
   <211> 11
   <212> PRT
   <213> RESISTANCE GENE
<400> 271
   Asp Leu Glu Tyr Tyr Ser Pro Ile Thr Thr Lys
   1 5 10
<210> 272
   <211> 6
   <212> PRT
   <213> RESISTANCE GENE
<400> 272
   Asp Thr Ser Thr Pro Lys
   1 5
<210> 273
   <211> 10
   <212> PRT
   <213> RESISTANCE GENE
<400> 273
   Phe Leu Gly Gly Pro Glu Gly Met Thr Lys
   1 5 10
<210> 274
   <211> 10
   <212> PRT
   <213> RESISTANCE GENE
<400> 274
   Gly Phe Leu Ala Ala Ala Val Leu Glu Arg
   1 5 10
<210> 275
   <211> 8
   <212> PRT
   <213> RESISTANCE GENE
<400> 275
   Gly Asn Thr Thr Gly Asp Ala Arg
   1 5
<210> 276
   <211> 17
   <212> PRT
   <213> RESISTANCE GENE
<400> 276
   Ile Gly Val Phe Ala Ile Asp Thr Gly Ser Gly Asn Thr Phe Gly Tyr
   1 5 10 15
   Arg
<210> 277
   <211> 10
   <212> PRT
   <213> RESISTANCE GENE
<400> 277
   Leu Ala Leu Gly Asn Val Leu Asn Ala Lys
   1 5 10
<210> 278
   <211> 6
   <212> PRT
   <213> RESISTANCE GENE
<400> 278
   Leu Asp Ile Asn Gln Lys
   1 5
<210> 279
   <211> 8
   <212> PRT
   <213> RESISTANCE GENE
<400> 279
   Leu Glu Glu Asp Phe Asp Gly Arg
   1 5
<210> 280
   <211> 6
   <212> PRT
   <213> RESISTANCE GENE
<400> 280
   Ser Asp Ala Ala Ala Lys
   1 5
<210> 281
   <211> 8
   <212> PRT
   <213> RESISTANCE GENE
<400> 281
   Ser Ile Gly Asp Asn Glu Phe Arg
   1 5
<210> 282
   <211> 21
   <212> PRT
   <213> RESISTANCE GENE
<400> 282
   Thr Ala Ser Phe Leu Phe Ser Val Cys Leu Ala Leu Ser Ala Phe Asn
   1 5 10 15
   Ala His Ala Asn Lys
   20
<210> 283
   <211> 19
   <212> PRT
   <213> RESISTANCE GENE
<400> 283
   Thr Gly Ser Cys Gly Ala Ile Gly Thr Ala Asn Asp Tyr Ala Val Ile
   1 5 10 15
   Trp Pro Lys
<210> 284
   <211> 19
   <212> PRT
   <213> RESISTANCE GENE
<400> 284
   Thr Gly Ser Cys Gly Ala Tyr Gly Thr Ala Asn Asp Tyr Ala Val Ile
   1 5 10 15
   Trp Pro Lys
<210> 285
   <211> 7
   <212> PRT
   <213> RESISTANCE GENE
<400> 285
   Thr Ile Ala Glu Ala Ser Arg
   1 5
<210> 286
   <211> 13
   <212> PRT
   <213> RESISTANCE GENE
<400> 286
   Trp Glu Leu Glu Leu Asn Thr Ala Ile Pro Gly Asp Lys
   1 5 10
<210> 287
   <211> 8
   <212> PRT
   <213> RESISTANCE GENE
<400> 287
   Phe Pro Leu Cys Ser Ser Phe Lys
   1 5
<210> 288
   <211> 266
   <212> PRT
   <213> RESISTANCE GENE
<400> 288
   Met Phe Lys Leu Leu Ser Lys Leu Leu Val Tyr Leu Thr Ala Ser Ile
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 289
   <211> 266
   <212> PRT
   <213> RESISTANCE GENE
<400> 289
   Met Phe Lys Leu Leu Ser Lys Leu Leu Val Tyr Leu Thr Ala Ser Ile
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Lys Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Ser Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 290
   <211> 266
   <212> PRT
   <213> RESISTANCE GENE
<400> 290
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val His Glu Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 291
   <211> 266
   <212> PRT
   <213> RESISTANCE GENE
<400> 291
   Met Leu Lys Val Ile Ser Ser Leu Leu Val Tyr Met Thr Ala Ser Val
    1               5                   10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Lys
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val Leu Ala Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Thr Ala His Lys Asn Arg Ser Val Ala Glu
               260                 265
<210> 292
   <211> 266
   <212> PRT
   <213> RESISTANCE GENE
<400> 292
   Met Phe Lys Leu Leu Ser Lys Leu Leu Val Tyr Leu Thr Ala Ser Ile
    1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Arg Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Ser Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 293
   <211> 266
   <212> PRT
   <213> RESISTANCE GENE
<400> 293
   Met Phe Lys Leu Leu Ser Lys Leu Leu Val Tyr Leu Thr Ala Ser Ile
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Ala Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 294
   <211> 266
   <212> PRT
   <213> RESISTANCE GENE
<400> 294
   Met Phe Lys Leu Leu Ser Lys Leu Leu Val Tyr Leu Thr Ala Ser Ile
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Ile Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 295
   <211> 266
   <212> PRT
   <213> RESISTANCE GENE
<400> 295
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Tyr Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 296
   <211> 266
   <212> PRT
   <213> RESISTANCE GENE
<400> 296
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Ser Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 297
   <211> 266
   <212> PRT
   <213> RESISTANCE GENE
<400> 297
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Ser Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val His Glu Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 298
   <211> 266
   <212> PRT
   <213> RESISTANCE GENE
<400> 298
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val His Glu Leu Ser Ser Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 299
   <211> 266
   <212> PRT
   <213> RESISTANCE GENE
<400> 299
   Met Leu Lys Val Ile Ser Ser Leu Leu Phe Tyr Met Thr Ala Ser Leu
   1               5                   10                  15
   Met Ala Val Ala Ser Pro Leu Ala His Ser Gly Glu Ser Arg Gly Glu
               20                  25                  30
   Tyr Pro Thr Val Ser Glu Ile Pro Val Gly Glu Val Arg Leu Tyr Gln
           35                  40                  45
   Ile Asp Asp Gly Val Trp Ser His Ile Ala Thr His Thr Phe Asp Gly
       50                  55                  60
   Val Val Tyr Pro Ser Asn Gly Leu Ile Val Arg Asp Gly Asp Glu Leu
   65                  70                  75                  80
   Leu Leu Ile Asp Thr Ala Trp Gly Thr Lys Asn Thr Val Ala Leu Leu
                   85                  90                  95
   Ala Glu Ile Glu Lys Gln Ile Gly Leu Pro Val Thr Arg Ser Val Ser
               100                 105                 110
   Thr His Phe His Asp Asp Arg Val Gly Gly Val Asp Ala Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Val Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val Leu Glu Leu Ser Arg Thr Ser Ala
           195                 200                 205
   Gly Asn Val Ala Asp Ala Asp Leu Ala Glu Trp Pro Gly Ser Val Glu
       210                 215                 220
   Arg Ile Gln Gln His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Thr Asn Arg Ser Val Ala Glu
               260                 265
<210> 300
   <211> 266
   <212> PRT
   <213> RESISTANCE GENE
<400> 300
   Met Phe Lys Leu Leu Ser Lys Leu Leu Val Tyr Leu Thr Ala Ser Met
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 301
   <211> 266
   <212> PRT
   <213> RESISTANCE GENE
<400> 301
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Phe Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 302
   <211> 266
   <212> PRT
   <213> RESISTANCE GENE
<400> 302
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Leu His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 303
   <211> 266
   <212> PRT
   <213> RESISTANCE GENE
<400> 303
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Ser Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 304
   <211> 266
   <212> PRT
   <213> RESISTANCE GENE
<400> 304
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Lys
               180                 185                 190
   Val Leu Tyr Gly Gly Cys Ala Val His Glu Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 305
   <211> 266
   <212> PRT
   <213> RESISTANCE GENE
<400> 305
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Ser
               180                 185                 190
   Val Leu Tyr Gly Gly Cys Ala Ile Tyr Glu Leu Ser Leu Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 306
   <211> 266
   <212> PRT
   <213> RESISTANCE GENE
<400> 306
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Lys
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val Leu Ala Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                   245                 250                 255
   Lys Ala His Thr Asn Arg Ser Val Val Glu
               260                 265
<210> 307
   <211> 266
   <212> PRT
   <213> RESISTANCE GENE
<400> 307
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ser Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val His Glu Leu Ser Ser Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 308
   <211> 266
   <212> PRT
   <213> RESISTANCE GENE
<400> 308
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val Leu Glu Leu Ser Arg Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 309
   <211> 266
   <212> PRT
   <213> RESISTANCE GENE
<400> 309
   Met Phe Lys Leu Leu Ser Lys Leu Leu Val Tyr Leu Thr Ala Ser Ile
    1               5                  10                  15
   Met Ala Ile Ala Ser Pro Leu Ala Phe Ser Val Asp Ser Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
                   100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Val Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                       165                 170                 175
   Ala Ala His Ser Thr Asp Asn Leu Val Val Tyr Val Pro Ser Ala Ser
                   180                 185                 190
   Val Leu Tyr Gly Gly Cys Ala Ile Tyr Glu Leu Ser Arg Thr Ser Ala
           195                 200                 205
   Gly Asn Val Ala Asp Ala Asp Leu Ala Glu Trp Pro Thr Ser Ile Glu
       210                 215                 220
   Arg Ile Gln Gln His Tyr Pro Glu Ala Gln Phe Val Ile Pro Gly His
   225                 230                 235                 240
   Gly Leu Pro Gly Gly Leu Asp Leu Leu Lys His Thr Thr Asn Val Val
                       245                 250                 255
   Lys Ala His Thr Asn Arg Ser Val Val Glu
                   260                 265
<210> 310
   <211> 266
   <212> PRT
   <213> RESISTANCE GENE
<400> 310
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val His Glu Leu Ser Ser Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 311
   <211> 265
   <212> PRT
   <213> RESISTANCE GENE
<400> 311
   Met Phe Gln Ile Arg Ser Phe Leu Val Gly Ile Ser Ala Phe Val Met
   1               5                   10                  15
   Ala Val Leu Gly Ser Ala Ala Tyr Ser Ala Gln Pro Gly Gly Glu Tyr
               20                  25                  30
   Pro Thr Val Asp Asp Ile Pro Val Gly Glu Val Arg Leu Tyr Lys Ile
           35                  40                  45
   Gly Asp Gly Val Trp Ser His Ile Ala Thr Gln Lys Leu Gly Asp Thr
       50                  55                  60
   Val Tyr Ser Ser Asn Gly Leu Ile Val Arg Asp Ala Asp Glu Leu Leu
   65                  70                  75                  80
   Leu Ile Asp Thr Ala Trp Gly Ala Lys Asn Thr Val Ala Leu Leu Ala
                   85                  90                  95
   Glu Ile Glu Lys Gln Ile Gly Leu Pro Val Thr Arg Ser Ile Ser Thr
               100                 105                 110
   His Phe His Asp Asp Arg Val Gly Gly Val Asp Val Leu Arg Ala Ala
           115                 120                 125
   Gly Val Ala Thr Tyr Thr Ser Pro Leu Thr Arg Gln Leu Ala Glu Ala
       130                 135                 140
   Ala Gly Asn Glu Val Pro Ala His Ser Leu Lys Ala Leu Ser Ser Ser
   145                 150                 155                 160
   Gly Asp Val Val Arg Phe Gly Pro Val Glu Val Phe Tyr Pro Gly Ala
                   165                 170                 175
   Ala His Ser Gly Asp Asn Leu Val Val Tyr Val Pro Ala Val Arg Val
               180                 185                 190
   Leu Phe Gly Gly Cys Ala Val His Glu Ala Ser Arg Glu Ser Ala Gly
           195                 200                 205
   Asn Val Ala Asp Ala Asn Leu Ala Glu Trp Pro Ala Thr Ile Lys Arg
       210                 215                 220
   Ile Gln Gln Arg Tyr Pro Glu Ala Glu Val Val Ile Pro Gly His Gly
   225                 230                 235                 240
   Leu Pro Gly Gly Leu Glu Leu Leu Gln His Thr Thr Asn Val Val Lys
                   245                 250                 255
   Thr His Lys Val Arg Pro Val Ala Glu
               260                 265
<210> 312
   <211> 262
   <212> PRT
   <213> RESISTANCE GENE
<400> 312
   Met Phe Lys Leu Leu Ser Lys Leu Leu Val Tyr Leu Thr Ala Ser Ile
   1               5                   10                  15
   Met Ala Ile Ala Ser Pro Leu Ala Phe Ser Val Asp Ser Ser Gly Glu
               20                  25                  30
   Tyr Pro Thr Val Ser Glu Ile Pro Val Gly Glu Val Arg Leu Tyr Gln
           35                  40                  45
   Ile Ala Asp Gly Val Trp Ser His Ile Ala Thr Arg Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Asn
       130                 135                 140
   Glu Ile Pro Thr His Ser Leu Glu Gly Leu Ser Ser Ser Gly Asp Ala
   145                 150                 155                 160
   Val Arg Phe Gly Pro Val Glu Leu Phe Tyr Pro Gly Ala Ala His Ser
                   165                 170                 175
   Thr Asp Asn Leu Val Val Tyr Val Pro Ser Ala Ser Val Leu Tyr Gly
               180                 185                 190
   Gly Cys Ala Ile Tyr Glu Leu Ser Arg Thr Ser Ala Gly Asn Val Ala
           195                 200                 205
   Asp Ala Asp Leu Ala Glu Trp Pro Thr Ser Ile Glu Arg Ile Gln Gln
       210                 215                 220
   His Tyr Pro Glu Ala Gln Phe Val Ile Pro Gly His Gly Leu Pro Gly
   225                 230                 235                 240
   Gly Leu Asp Leu Leu Lys His Thr Thr Asn Val Val Lys Ala His Thr
                   245                 250                 255
   Asn Arg Ser Val Val Glu
               260
<210> 313
   <211> 266
   <212> PRT
   <213> RESISTANCE GENE
<400> 313
   Met Leu Lys Val Ile Ser Ser Leu Leu Val Tyr Met Thr Ala Ser Val
    1               5                  10                  15
   Met Ala Val Ala Ser Pro Leu Ala His Ser Gly Glu Pro Ser Gly Glu
               20                  25                  30
   Tyr Pro Thr Val Asn Glu Ile Pro Val Gly Glu Val Arg Leu Tyr Gln
           35                  40                  45
   Ile Ala Asp Gly Val Trp Ser His Ile Ala Thr Gln Ser Phe Asp Gly
       50                  55                  60
   Ala Val Tyr Pro Ser Asn Gly Leu Ile Val Arg Asp Gly Asp Glu Leu
   65                  70                  75                  80
   Leu Leu Ile Asp Thr Ala Trp Gly Ala Lys Asn Thr Ala Ala Leu Leu
                   85                  90                  95
   Ala Glu Ile Glu Lys Gln Ile Gly Leu Pro Val Thr Arg Ala Val Ser
               100                 105                 110
   Thr His Phe His Asp Asp Arg Val Gly Gly Val Asp Val Leu Arg Ala
           115                 120                 125
   Ala Gly Val Ala Thr Tyr Ala Ser Pro Ser Thr Arg Arg Leu Ala Glu
       130                 135                 140
   Ala Glu Gly Asn Glu Ile Pro Thr His Ser Leu Glu Gly Leu Ser Ser
   145                 150                 155                 160
   Ser Gly Asp Ala Val Arg Phe Gly Pro Val Glu Leu Phe Tyr Pro Gly
                   165                 170                 175
   Ala Ala His Ser Thr Asp Asn Leu Val Val Tyr Val Pro Ser Ala Asn
               180                 185                 190
   Val Leu Tyr Gly Gly Cys Ala Val Leu Glu Leu Ser Ser Thr Ser Ala
           195                 200                 205
   Gly Asn Val Ala Asp Ala Asp Leu Ala Glu Trp Pro Thr Ser Val Glu
       210                 215                 220
   Arg Ile Gln Lys His Tyr Pro Glu Ala Glu Val Val Ile Pro Gly His
   225                 230                 235                 240
   Gly Leu Pro Gly Gly Leu Asp Leu Leu Gln His Thr Ala Asn Val Val
                   245                 250                 255
   Lys Ala His Lys Asn Arg Ser Val Ala Glu
               260                 265
<210> 314
   <211> 13
   <212> PRT
   <213> RESISTANCE GENE
<400> 314
   Ala Ala Gly Val Ala Thr Tyr Ala Ser Pro Ser Ala Arg
   1 5 10
<210> 315
   <211> 13
   <212> PRT
   <213> RESISTANCE GENE
<400> 315
   Ala Ala Gly Val Ala Thr Tyr Ala Ser Pro Ser Ile Arg
   1 5 10
<210> 316
   <211> 13
   <212> PRT
   <213> RESISTANCE GENE
<400> 316
   Ala Ala Gly Val Ala Thr Tyr Ala Ser Pro Ser Thr Arg
   1 5 10
<210> 317
   <211> 13
   <212> PRT
   <213> RESISTANCE GENE
<400> 317
   Ala Ala Gly Val Ala Thr Tyr Thr Ser Pro Leu Thr Arg
   1 5 10
<210> 318
   <211> 12
   <212> PRT
   <213> RESISTANCE GENE
<400> 318
   Ala Gly Val Ala Thr Tyr Ala Ser Pro Ser Thr Arg
   1 5 10
<210> 319
   <211> 5
   <212> PRT
   <213> RESISTANCE GENE
<400> 319
   Ala His Thr Asn Arg
   1 5
<210> 320
   <211> 10
   <212> PRT
   <213> RESISTANCE GENE
<400> 320
   Ala Leu Ser Ser Ser Gly Asp Val Val Arg
   1 5 10
<210> 321
   <211> 10
   <212> PRT
   <213> RESISTANCE GENE
<400> 321
   Ala Val Ser Thr His Phe His Asp Asp Arg
   1 5 10
<210> 322
   <211> 15
   <212> PRT
   <213> RESISTANCE GENE
<400> 322
   Asp Ala Asp Glu Leu Leu Leu Ile Asp Thr Ala Trp Gly Ala Lys
   1 5 10 15
<210> 323
   <211> 15
   <212> PRT
   <213> RESISTANCE GENE
<400> 323
   Asp Gly Asp Glu Leu Leu Leu Ile Asp Thr Ala Trp Gly Ala Lys
   1 5 10 15
<210> 324
   <211> 15
   <212> PRT
   <213> RESISTANCE GENE
<400> 324
   Asp Gly Asp Glu Leu Leu Leu Ile Asp Thr Ala Trp Gly Thr Lys
   1 5 10 15
<210> 325
   <211> 19
   <212> PRT
   <213> RESISTANCE GENE
<400> 325
   Glu Ser Ala Gly Asn Val Ala Asp Ala Asn Leu Ala Glu Trp Pro Ala
   1 5 10 15
   Thr Ile Lys
<210> 326
   <211> 15
   <212> PRT
   <213> RESISTANCE GENE
<400> 326
   Gly Glu Tyr Pro Thr Val Ser Glu Ile Pro Val Gly Glu Val Arg
   1 5 10 15
<210> 327
   <211> 7
   <212> PRT
   <213> RESISTANCE GENE
<400> 327
   His Thr Thr Asn Val Val Lys
   1 5
<210> 328
   <211> 13
   <212> PRT
   <213> RESISTANCE GENE
<400> 328
   Ile Gly Asp Gly Val Trp Ser His Ile Ala Thr Gln Lys
   1 5 10
<210> 329
   <211> 21
   <212> PRT
   <213> RESISTANCE GENE
<400> 329
   Leu Ala Asn Glu Ile Pro Thr His Ser Leu Glu Gly Leu Ser Ser Ser
   1 5 10 15
   Gly Asp Ala Val Arg
   20
<210> 330
   <211> 14
   <212> PRT
   <213> RESISTANCE GENE
<400> 330
   Leu Gly Asp Thr Val Tyr Ser Ser Asn Gly Leu Ile Val Arg
   1 5 10
<210> 331
   <211> 15
   <212> PRT
   <213> RESISTANCE GENE
<400> 331
   Leu Tyr Gln Ile Ala Asp Gly Val Trp Ser His Ile Ala Thr Lys
    1               5                   10                  15
<210> 332
   <211> 15
   <212> PRT
   <213> RESISTANCE GENE
<400> 332
   Leu Tyr Gln Ile Ala Asp Gly Val Trp Ser His Ile Ala Thr Arg
    1               5                   10                  15
<210> 333
   <211> 11
   <212> PRT
   <213> RESISTANCE GENE
<400> 333
   Asn Thr Ala Ala Leu Leu Ala Glu Ile Glu Lys
    1               5                   10
<210> 334
   <211> 11
   <212> PRT
   <213> RESISTANCE GENE
<400> 334
   Asn Thr Val Ala Leu Leu Ala Glu Ile Glu Lys
    1               5                   10
<210> 335
   <211> 8
   <212> PRT
   <213> RESISTANCE GENE
<400> 335
   Gln Ile Gly Leu Pro Val Thr Arg
    1               5
<210> 336
   <211> 16
   <212> PRT
   <213> RESISTANCE GENE
<400> 336
   Gln Leu Ala Glu Ala Ala Gly Asn Glu Val Pro Ala His Ser Leu Lys
   1 5 10 15
<210> 337
   <211> 15
   <212> PRT
   <213> RESISTANCE GENE
<400> 337
   Ser Phe Asp Gly Ala Val Tyr Pro Ser Asn Gly Leu Ile Val Arg
   1 5 10 15
<210> 338
   <211> 10
   <212> PRT
   <213> RESISTANCE GENE
<400> 338
   Ser Ile Ser Thr His Phe His Asp Asp Arg
   1 5 10
<210> 339
   <211> 10
   <212> PRT
   <213> RESISTANCE GENE
<400> 339
   Ser Val Ser Thr His Phe His Asp Asp Arg
   1 5 10
<210> 340
   <211> 20
   <212> PRT
   <213> RESISTANCE GENE
<400> 340
   Thr Ser Ala Gly Asn Val Ala Asp Ala Asp Leu Ala Glu Trp Pro Gly
   1 5 10 15
   Ser Val Glu Arg
   20
<210> 341
   <211> 20
   <212> PRT
   <213> RESISTANCE GENE
<400> 341
   Thr Ser Ala Gly Asn Val Ala Asp Ala Asp Leu Ala Glu Trp Pro Thr
   1 5 10 15
   Ser Ile Glu Arg
   20
<210> 342
   <211> 20
   <212> PRT
   <213> RESISTANCE GENE
<400> 342
   Thr Ser Ala Gly Asn Val Ala Asp Ala Asp Leu Ala Glu Trp Pro Thr
    1              5                  10                  15
   Ser Val Glu Arg
               20
<210> 343
   <211> 8
   <212> PRT
   <213> RESISTANCE GENE
<400> 343
   Val Gly Gly Val Asp Ala Leu Arg
    1              5
<210> 344
   <211> 8
   <212> PRT
   <213> RESISTANCE GENE
<400> 344
   Val Gly Gly Val Asp Val Leu Arg
    1              5
<210> 345
   <211> 13
   <212> PRT
   <213> RESISTANCE GENE
<400> 345
   Val Leu Phe Gly Gly Cys Ala Val His Glu Ala Ser Arg
    1              5                  10
<210> 346
   <211> 13
   <212> PRT
   <213> RESISTANCE GENE
<400> 346
   Val Leu Tyr Gly Gly Cys Ala Val His Glu Leu Ser Arg
    1              5                  10
<210> 347
   <211> 264
   <212> PRT
   <213> RESISTANCE GENE
<400> 347
   Met Ser Arg Leu Leu Leu Ser Gly Leu Leu Ala Thr Gly Leu Leu Cys
    1               5                  10                  15
   Ala Val Pro Ala Ser Ala Ala Ser Gly Cys Phe Leu Tyr Ala Asp Gly
               20                  25                  30
   Asn Gly Gln Thr Leu Ser Ser Glu Gly Asp Cys Ser Ser Gln Leu Pro
           35                  40                  45
   Pro Ala Ser Thr Phe Lys Ile Pro Leu Ala Leu Met Gly Tyr Asp Ser
       50                  55                  60
   Gly Phe Leu Val Asn Glu Glu His Pro Ala Leu Pro Tyr Lys Pro Ser
   65                  70                  75                  80
   Tyr Asp Gly Trp Leu Pro Ala Trp Arg Glu Thr Thr Thr Pro Arg Arg
                   85                  90                  95
   Trp Glu Thr Tyr Ser Val Val Trp Phe Ser Gln Gln Ile Thr Glu Trp
               100                 105                 110
   Leu Gly Met Glu Arg Phe Gln Gln Tyr Val Asp Arg Phe Asp Tyr Gly
           115                 120                 125
   Asn Arg Asp Leu Ser Gly Asn Pro Gly Lys His Asp Gly Leu Thr Gln
       130                 135                 140
   Ala Trp Leu Ser Ser Ser Leu Ala Ile Ser Pro Glu Glu Gln Ala Arg
   145                 150                 155                 160
   Phe Leu Gly Lys Met Val Ser Gly Lys Leu Pro Val Ser Ala Gln Thr
                   165                 170                 175
   Leu Gln Tyr Thr Ala Asn Ile Leu Lys Val Ser Glu Val Glu Gly Trp
               180                 185                 190
   Gln Ile His Gly Lys Thr Gly Met Gly Tyr Pro Lys Lys Leu Asp Gly
           195                 200                 205
   Ser Leu Asn Arg Asp Gln Gln Ile Gly Trp Phe Val Gly Trp Ala Ser
       210                 215                 220
   Lys Pro Gly Lys Gln Leu Ile Phe Val His Thr Val Val Gln Lys Pro
   225                 230                 235                 240
   Gly Lys Gln Phe Ala Ser Ile Lys Ala Lys Glu Glu Val Leu Ala Ala
                   245                 250                 255
   Leu Pro Ala Gln Leu Lys Lys Leu
               260
<210> 348
   <211> 257
   <212> PRT
   <213> RESISTANCE GENE
<400> 348
   Ile Ala Cys Leu Ser Ser Thr Ala Leu Ala Gly Ser Ile Thr Glu Asn
    1               5                  10                  15
   Thr Ser Trp Asn Lys Glu Phe Ser Ala Glu Ala Val Asn Gly Val Phe
               20                  25                  30
   Val Leu Cys Lys Ser Ser Ser Lys Ser Cys Ala Thr Asn Asp Leu Ala
           35                  40                  45
   Arg Ala Ser Lys Glu Tyr Leu Pro Ala Ser Thr Phe Lys Ile Pro Asn
       50                  55                  60
   Ala Ile Ile Gly Leu Glu Thr Gly Val Ile Lys Asn Glu His Gln Val
   65                  70                  75                  80
   Phe Lys Trp Asp Gly Lys Pro Arg Ala Met Lys Gln Trp Glu Arg Asp
                   85                  90                  95
   Leu Thr Leu Arg Gly Ala Ile Gln Val Ser Ala Val Pro Val Phe Gln
               100                 105                 110
   Gln Ile Ala Arg Glu Val Gly Glu Val Arg Met Gln Lys Tyr Leu Lys
           115                 120                 125
   Lys Phe Ser Tyr Gly Asn Gln Asn Ile Ser Gly Gly Ile Asp Lys Phe
       130                 135                 140
   Trp Leu Glu Asp Gln Leu Arg Ile Ser Ala Val Asn Gln Val Glu Phe
   145                 150                 155                 160
   Leu Glu Ser Leu Tyr Leu Asn Lys Leu Ser Ala Ser Lys Glu Asn Gln
                   165                 170                 175
   Leu Ile Val Lys Glu Ala Leu Val Thr Glu Ala Ala Pro Glu Tyr Leu
               180                 185                 190
   Val His Ser Lys Thr Gly Phe Ser Gly Val Gly Thr Glu Ser Asn Pro
           195                 200                 205
   Gly Val Ala Trp Trp Val Gly Trp Val Glu Lys Glu Thr Glu Val Tyr
       210                 215                 220
   Phe Phe Ala Phe Asn Met Asp Ile Asp Asn Glu Ser Lys Leu Pro Leu
   225                 230                 235                 240
   Arg Lys Ser Ile Pro Thr Lys Ile Met Glu Ser Glu Gly Ile Ile Gly
                   245                 250                 255
   Gly
<210> 349
   <211> 274
   <212> PRT
   <213> RESISTANCE GENE
<400> 349
   Met Lys Lys Ile Leu Leu Leu His Met Leu Val Phe Val Ser Ala Thr
   1               5                   10                  15
   Leu Pro Ile Ser Ser Val Ala Ser Asp Glu Val Glu Thr Leu Lys Cys
               20                  25                  30
   Thr Ile Ile Ala Asp Ala Ile Thr Gly Asn Thr Leu Tyr Glu Thr Gly
           35                  40                  45
   Glu Cys Ala Arg Arg Val Ser Pro Cys Ser Ser Phe Lys Leu Pro Leu
       50                  55                  60
   Ala Ile Met Gly Phe Asp Ser Gly Ile Leu Gln Ser Pro Lys Ser Pro
   65                  70                  75                  80
   Thr Trp Glu Leu Lys Pro Glu Tyr Asn Pro Ser Pro Arg Asp Arg Thr
                   85                  90                  95
   Tyr Lys Gln Val Tyr Pro Ala Leu Trp Gln Ser Asp Ser Val Val Trp
               100                 105                 110
   Phe Ser Gln Gln Leu Thr Ser Arg Leu Gly Val Asp Arg Phe Thr Glu
           115                 120                 125
   Tyr Val Lys Lys Phe Glu Tyr Gly Asn Gln Asp Val Ser Gly Asp Ser
       130                 135                 140
   Gly Lys His Asn Gly Leu Thr Gln Ser Trp Leu Met Ser Ser Leu Thr
   145                 150                 155                 160
   Ile Ser Pro Lys Glu Gln Ile Gln Phe Leu Leu Arg Phe Val Ala His
                   165                 170                 175
   Lys Leu Pro Val Ser Glu Ala Ala Tyr Asp Met Ala Tyr Ala Thr Ile
               180                 185                 190
   Pro Gln Tyr Gln Ala Ala Glu Gly Trp Ala Val His Gly Lys Ser Gly
           195                 200                 205
   Ser Gly Trp Leu Arg Asp Asn Asn Gly Lys Ile Asn Glu Ser Arg Pro
       210                 215                 220
   Gln Gly Trp Phe Val Gly Trp Ala Glu Lys Asn Gly Arg Gln Val Val
   225                 230                 235                 240
   Phe Ala Arg Leu Glu Ile Gly Lys Glu Lys Ser Asp Ile Pro Gly Gly
                   245                 250                 255
   Ser Lys Ala Arg Glu Asp Ile Leu Val Glu Leu Pro Val Leu Met Gly
               260                 265                 270
   Asn Lys
<210> 350
   <211> 275
   <212> PRT
   <213> RESISTANCE GENE
<400> 350
   Met Ala Ile Arg Ile Phe Ala Ile Leu Phe Ser Ile Phe Ser Leu Ala
   1               5                   10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
   50 55 60
   Lys Lys Arg Tyr Ser Pro Ala Ser Thr Phe Lys Ile Pro His Thr Leu
   65 70 75 80
   Phe Ala Leu Asp Ala Gly Ala Val Arg Asp Glu Phe Gln Ile Phe Arg
   85 90 95
   Trp Asp Gly Val Asn Arg Gly Phe Ala Gly His Asn Gln Asp Gln Asp
   100 105 110
   Leu Arg Ser Ala Met Arg Asn Ser Thr Val Trp Val Tyr Glu Leu Phe
   115 120 125
   Ala Lys Glu Ile Gly Asp Asp Lys Ala Arg Arg Tyr Leu Lys Lys Ile
   130 135 140
   Asp Tyr Gly Asn Ala Gly Pro Ser Thr Ser Asn Gly Asp Tyr Trp Ile
   145 150 155 160
   Glu Gly Ser Leu Ala Ile Ser Ala Gln Glu Gln Ile Ala Phe Leu Arg
   165 170 175
   Lys Leu Tyr Arg Asn Glu Leu Pro Phe Arg Val Glu His Gln Arg Leu
   180 185 190
   Val Lys Asp Leu Met Ile Val Glu Ala Gly Arg Asn Trp Ile Leu Arg
   195 200 205
   Ala Lys Thr Gly Trp Glu Gly Arg Met Gly Trp Trp Val Gly Trp Val
   210 215 220
   Glu Trp Pro Thr Gly Ser Val Phe Phe Ala Leu Asn Ile Asp Thr Pro
   225 230 235 240
   Asn Arg Met Asp Asp Leu Phe Lys Arg Glu Ala Ile Val Arg Ala Ile
   245 250 255
   Leu Arg Ser Ile Glu Ala Leu Pro Pro Asn Pro Ala Val Asn Ser Asp
   260 265 270
   Ala Ala Arg
   275
<210> 351
   <211> 275
   <212> PRT
   <213> RESISTANCE GENE
<400> 351
   Met Gln Arg Ser Leu Ser Met Ser Gly Lys Arg His Phe Ile Phe Ala
   1               5                   10                  15
   Val Ser Phe Val Ile Ser Thr Val Cys Leu Thr Phe Ser Pro Ala Asn
               20                  25                  30
   Ala Ala Gln Lys Leu Ser Cys Thr Leu Val Ile Asp Glu Ala Ser Gly
           35                  40                  45
   Asp Leu Leu His Arg Glu Gly Ser Cys Asp Lys Ala Phe Ala Pro Met
       50                  55                  60
   Ser Thr Phe Lys Leu Pro Leu Ala Ile Met Gly Tyr Asp Ala Asp Ile
   65                  70                  75                  80
   Leu Leu Asp Ala Thr Thr Pro Arg Trp Asp Tyr Lys Pro Glu Phe Asn
                   85                  90                  95
   Gly Tyr Lys Ser Gln Gln Lys Pro Thr Asp Pro Thr Ile Trp Leu Lys
               100                 105                 110
   Asp Ser Ile Val Trp Tyr Ser Gln Glu Leu Thr Arg Arg Leu Gly Glu
           115                 120                 125
   Ser Arg Phe Ser Asp Tyr Val Gln Arg Phe Asp Tyr Gly Asn Lys Asp
       130                 135                 140
   Val Ser Gly Asp Pro Gly Lys His Asn Gly Leu Thr His Ala Trp Leu
   145                 150                 155                 160
   Ala Ser Ser Leu Lys Ile Ser Pro Glu Glu Gln Val Arg Phe Leu Arg
                   165                 170                 175
   Arg Phe Leu Arg Gly Glu Leu Pro Val Ser Glu Asp Ala Leu Glu Met
               180                 185                 190
   Thr Lys Ala Val Val Pro His Phe Glu Ala Gly Asp Trp Asp Val Gln
           195                 200                 205
   Gly Lys Thr Gly Thr Gly Ser Leu Ser Asp Ala Lys Gly Gly Lys Ala
       210                 215                 220
   Pro Ile Gly Trp Phe Ile Gly Trp Ala Thr Arg Asp Asp Arg Arg Val
   225                 230                 235                 240
   Val Phe Ala Arg Leu Thr Val Gly Ala Arg Lys Gly Glu Gln Pro Ala
                   245                 250                 255
   Gly Pro Ala Ala Arg Asp Glu Phe Leu Asn Thr Leu Pro Ala Leu Ser
               260                 265                 270
   Glu Asn Phe
           275
<210> 352
   <211> 266
   <212> PRT
   <213> RESISTANCE GENE
<400> 352
   Met Lys Thr Phe Ala Ala Tyr Val Ile Ile Ala Cys Leu Ser Ser Thr
    1               5                  10                  15
   Ala Leu Ala Gly Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asp Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Thr Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Tyr Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Glu Thr Glu Val Tyr Phe Phe Ala Phe Asn Met Asp
   225                 230                 235                 240
   Ile Asp Asn Glu Ser Lys Leu Pro Leu Arg Lys Ser Ile Pro Thr Lys
                   245                 250                 255
   Ile Met Glu Ser Glu Gly Ile Ile Gly Gly
               260                 265
<210> 353
   <211> 257
   <212> PRT
   <213> RESISTANCE GENE
<400> 353
   Ile Ala Cys Leu Ser Ser Thr Ala Leu Ala Gly Ser Ile Thr Glu Asn
    1               5                  10                  15
   Thr Ser Trp Asn Lys Glu Phe Ser Ala Glu Ala Val Asn Gly Val Phe
               20                  25                  30
   Val Leu Cys Lys Ser Ser Ser Lys Ser Cys Ala Thr Asn Asp Leu Ala
                   35                  40                  45
   Arg Ala Ser Lys Glu Tyr Leu Pro Ala Ser Thr Phe Lys Ile Pro Asn
       50                  55                  60
   Ala Ile Ile Gly Leu Glu Thr Gly Val Ile Lys Asn Glu His Gln Val
   65                  70                  75                  80
   Phe Lys Trp Asp Gly Lys Pro Arg Ala Met Lys Gln Trp Glu Arg Asp
                   85                  90                  95
   Leu Thr Leu Arg Gly Ala Ile Gln Val Ser Ala Val Pro Val Phe Gln
               100                 105                 110
   Gln Ile Thr Arg Glu Val Gly Glu Val Arg Met Gln Lys Tyr Leu Lys
           115                 120                 125
   Lys Phe Ser Tyr Gly Asn Gln Asn Ile Ser Gly Gly Ile Asp Lys Phe
       130                 135                 140
   Trp Leu Glu Asp Gln Leu Arg Ile Ser Ala Val Asn Gln Val Glu Phe
   145                 150                 155                 160
   Leu Glu Ser Leu Tyr Leu Asn Lys Leu Ser Ala Ser Lys Glu Asn Gln
                   165                 170                 175
   Leu Ile Val Lys Glu Ala Leu Val Thr Glu Ala Ala Pro Glu Tyr Leu
               180                 185                 190
   Val His Ser Lys Thr Gly Phe Ser Gly Val Gly Thr Glu Ser Asn Pro
           195                 200                 205
   Gly Val Ala Trp Trp Val Gly Trp Val Glu Lys Glu Thr Glu Val Tyr
       210                 215                 220
   Phe Phe Ala Phe Asn Met Asp Ile Asp Asn Glu Ser Lys Leu Pro Leu
   225                 230                 235                 240
   Arg Lys Ser Ile Pro Thr Lys Ile Met Glu Ser Glu Gly Ile Ile Gly
                   245                 250                 255
   Gly
<210> 354
   <211> 257
   <212> PRT
   <213> RESISTANCE GENE
<400> 354
   Ile Ala Cys Leu Ser Ser Thr Ala Leu Ala Gly Ser Ile Thr Glu Asn
    1              5                   10                  15
   Thr Ser Trp Asn Lys Glu Phe Ser Ala Glu Ala Val Asn Gly Val Phe
               20                  25                  30
   Val Leu Cys Lys Ser Ser Ser Lys Ser Cys Ala Thr Asn Asp Leu Ala
           35                  40                  45
   Arg Ala Ser Lys Glu Tyr Leu Pro Ala Ser Thr Phe Lys Ile Pro Ser
       50                  55                  60
   Ala Ile Ile Gly Leu Glu Thr Gly Val Ile Lys Asn Glu His Gln Val
   65                  70                  75                  80
   Phe Lys Trp Asp Gly Lys Pro Arg Ala Met Lys Gln Trp Glu Arg Asp
                   85                  90                  95
   Leu Thr Leu Arg Gly Ala Ile Gln Val Ser Ala Val Pro Val Phe Gln
               100                 105                 110
   Gln Ile Ala Arg Glu Val Gly Glu Val Arg Met Gln Lys Tyr Leu Lys
           115                 120                 125
   Lys Phe Ser Tyr Gly Asn Gln Asn Ile Ser Gly Gly Ile Asp Lys Phe
       130                 135                 140
   Trp Leu Glu Gly Gln Leu Arg Ile Ser Ala Val Asn Gln Val Glu Phe
   145                 150                 155                 160
   Leu Glu Ser Leu Tyr Leu Asn Lys Leu Ser Ala Ser Lys Glu Asn Gln
                   165                 170                 175
   Leu Ile Val Lys Glu Ala Leu Val Thr Glu Ala Ala Pro Glu Tyr Leu
               180                 185                 190
   Val His Ser Lys Thr Gly Phe Ser Gly Val Gly Thr Glu Ser Asn Pro
           195                 200                 205
   Gly Val Ala Trp Trp Val Gly Trp Val Glu Lys Glu Thr Glu Val Tyr
       210                 215                 220
   Phe Phe Ala Phe Asn Met Asp Ile Asp Asn Glu Ser Lys Leu Pro Leu
   225                 230                 235                 240
   Arg Lys Ser Ile Pro Thr Lys Ile Met Glu Ser Glu Gly Ile Ile Gly
                   245                 250                 255
   Gly
<210> 355
   <211> 266
   <212> PRT
   <213> RESISTANCE GENE
<400> 355
   Met Ile Ile Arg Phe Leu Ala Leu Leu Phe Ser Ala Val Val Leu Val
    1               5                  10                  15
   Ser Leu Gly His Ala Gln Glu Lys Thr His Glu Ser Ser Asn Trp Gly
               20                  25                  30
   Lys Tyr Phe Ser Asp Phe Asn Ala Lys Gly Thr Ile Val Val Val Asp
           35                  40                  45
   Glu Arg Thr Asn Gly Asn Ser Thr Ser Val Tyr Asn Glu Ser Arg Ala
       50                  55                  60
   Gln Gln Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe His Val Phe Arg
                   85                  90                  95
   Trp Asp Gly Ala Lys Arg Ser Phe Ala Gly His Asn Gln Asp Gln Asn
               100                 105                 110
   Leu Arg Ser Ala Met Arg Asn Ser Thr Val Trp Val Tyr Gln Leu Phe
           115                 120                 125
   Ala Lys Glu Ile Gly Glu Asn Lys Ala Arg Ser Tyr Leu Glu Lys Leu
       130                 135                 140
   Asn Tyr Gly Asn Ala Asp Pro Ser Thr Lys Ser Gly Asp Tyr Trp Ile
   145                 150                 155                 160
   Asp Gly Asn Leu Ala Ile Ser Ala Asn Glu Gln Ile Ser Ile Leu Lys
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Lys Arg Asp Trp Ile Leu Arg
           195                 200                 205
   Ala Lys Thr Gly Trp Asp Gly Gln Met Gly Trp Trp Val Gly Trp Val
       210                 215                 220
   Glu Trp Pro Thr Gly Pro Val Phe Phe Ala Leu Asn Ile Asp Thr Pro
   225                 230                 235                 240
   Asn Arg Met Glu Asp Leu His Lys Arg Glu Ala Ile Ala Arg Ala Ile
                   245                 250                 255
   Leu Gln Ser Val Asn Ala Leu Pro Pro Asn
               260                 265
<210> 356
   <211> 275
   <212> PRT
   <213> RESISTANCE GENE
<400> 356
   Met Ala Ile Arg Ile Phe Ala Ile Leu Phe Ser Thr Phe Val Phe Gly
    1               5                  10                  15
   Thr Phe Ala His Ala Gln Glu Gly Met Arg Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Thr Asp Arg Val Ile Leu Val Phe Asp Gln Val Arg Ser
       50                  55                  60
   Glu Lys Arg Tyr Ser Pro Ala Ser Thr Phe Lys Ile Pro His Thr Leu
    65                  70                  75                  80
   Phe Ala Leu Asp Ala Gly Ala Ala Arg Asp Glu Phe Gln Val Phe Arg
                    85                  90                  95
   Trp Asp Gly Ile Lys Arg Ser Phe Ala Ala His Asn Gln Asp Gln Asp
               100                 105                 110
   Leu Arg Ser Ala Met Arg Asn Ser Thr Val Trp Ile Tyr Glu Leu Phe
           115                 120                 125
   Ala Lys Glu Ile Gly Glu Asp Lys Ala Arg Arg Tyr Leu Lys Gln Ile
       130                 135                 140
   Asp Tyr Gly Asn Ala Asp Pro Ser Thr Ser Asn Gly Asp Tyr Trp Ile
   145                 150                 155                 160
   Asp Gly Asn Leu Ala Ile Ala Ala Gln Glu Gln Ile Ala Phe Leu Arg
                   165                 170                 175
   Lys Leu Tyr His Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Ile Gly Trp Trp Val Gly Trp Val
       210                 215                 220
   Glu Trp Pro Thr Gly Pro Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 357
   <211> 266
   <212> PRT
   <213> RESISTANCE GENE
<400> 357
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Ser Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 358
   <211> 266
   <212> PRT
   <213> RESISTANCE GENE
<400> 358
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Asp Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 359
   <211> 266
   <212> PRT
   <213> RESISTANCE GENE
<400> 359
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
    65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Gly Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 360
   <211> 276
   <212> PRT
   <213> RESISTANCE GENE
<400> 360
   Met Lys Asn Thr Ile His Ile Asn Phe Ala Ile Phe Leu Ile Ile Ala
    1               5                  10                  15
   Asn Ile Ile Tyr Ser Ser Ala Ser Ala Ser Thr Asp Ile Ser Thr Val
               20                  25                  30
   Ala Ser Pro Leu Phe Glu Gly Thr Glu Gly Cys Phe Leu Leu Tyr Asp
           35                  40                  45
   Ala Ser Thr Asn Ala Glu Ile Ala Gln Phe Asn Lys Ala Lys Cys Ala
       50                  55                  60
   Thr Gln Met Ala Pro Asp Ser Thr Phe Lys Ile Ala Leu Ser Leu Met
   65                  70                  75                  80
   Ala Phe Asp Ala Glu Ile Ile Asp Gln Lys Thr Ile Phe Lys Trp Asp
                   85                  90                  95
   Lys Thr Pro Lys Gly Met Glu Ile Trp Asn Ser Asn His Thr Pro Lys
               100                 105                 110
   Thr Trp Met Gln Phe Ser Val Val Trp Val Ser Gln Glu Ile Thr Gln
           115                 120                 125
   Lys Ile Gly Leu Asn Lys Ile Lys Asn Tyr Leu Lys Asp Phe Asp Tyr
       130                 135                 140
   Gly Asn Gln Asp Phe Ser Gly Asp Lys Glu Arg Asn Asn Gly Leu Thr
   145                 150                 155                 160
   Glu Ala Trp Leu Glu Ser Ser Leu Lys Ile Ser Pro Glu Glu Gln Ile
                   165                 170                 175
   Gln Phe Leu Arg Lys Ile Ile Asn His Asn Leu Pro Val Lys Asn Ser
               180                 185                 190
   Ala Ile Glu Asn Thr Ile Glu Asn Met Tyr Leu Gln Asp Leu Asp Asn
           195                 200                 205
   Ser Thr Lys Leu Tyr Gly Lys Thr Gly Ala Gly Phe Thr Ala Asn Arg
       210                 215                 220
   Thr Leu Gln Asn Gly Trp Phe Glu Gly Phe Ile Ile Ser Lys Ser Gly
   225                 230                 235                 240
   His Lys Tyr Val Phe Val Ser Ala Leu Thr Gly Asn Leu Gly Ser Asn
                   245                 250                 255
   Leu Thr Ser Ser Ile Lys Ala Lys Lys Asn Ala Ile Thr Ile Leu Asn
               260                 265                 270
   Thr Leu Asn Leu
           275
<210> 361
   <211> 256
   <212> PRT
   <213> RESISTANCE GENE
<400> 361
   Ala Asn Ile Ile Tyr Ser Ser Ala Ser Ala Ser Thr Asp Ile Ser Thr
    1               5                  10                  15
   Val Ala Ser Pro Leu Phe Glu Gly Thr Glu Gly Cys Phe Leu Leu Tyr
               20                  25                  30
   Asp Val Ser Thr Asn Ala Glu Ile Ala Gln Phe Asn Lys Ala Lys Cys
           35                  40                  45
   Ala Thr Gln Met Ala Pro Asp Ser Thr Phe Lys Ile Ala Leu Ser Leu
       50                  55                  60
   Met Ala Phe Asp Ala Glu Ile Ile Asp Gln Lys Thr Ile Phe Lys Trp
   65                  70                  75                  80
   Asp Lys Thr Pro Lys Gly Met Glu Ile Trp Asn Ser Asn His Thr Pro
                   85                  90                  95
   Lys Thr Trp Met Gln Phe Ser Val Val Trp Val Ser Gln Glu Ile Thr
               100                 105                 110
   Gln Lys Ile Gly Leu Asn Lys Ile Lys Asn Tyr Leu Lys Asp Phe Asp
           115                 120                 125
   Tyr Gly Asn Gln Asp Phe Ser Gly Asp Lys Glu Arg Asn Asn Gly Leu
       130                 135                 140
   Thr Glu Ala Trp Leu Glu Ser Ser Leu Lys Ile Ser Pro Glu Glu Gln
   145                 150                 155                 160
   Ile Gln Phe Leu Arg Lys Ile Ile Asn His Asn Leu Pro Val Lys Asn
                   165                 170                 175
   Ser Ala Ile Glu Asn Thr Ile Glu Asn Met Tyr Leu Gln Asp Leu Glu
               180                 185                 190
   Asn Ser Thr Lys Leu Tyr Gly Lys Thr Gly Ala Gly Phe Thr Ala Asn
           195                 200                 205
   Arg Thr Leu Gln Asn Gly Trp Phe Glu Gly Phe Ile Ile Ser Lys Ser
       210                 215                 220
   Gly His Lys Tyr Val Phe Val Ser Ala Leu Thr Gly Asn Leu Gly Ser
   225                 230                 235                 240
   Asn Leu Thr Ser Ser Ile Lys Ala Lys Lys Asn Ala Ile Thr Ile Leu
                   245                 250                 255
<210> 362
   <211> 246
   <212> PRT
   <213> RESISTANCE GENE
<400> 362
   Ile Phe Ser Leu Ala Thr Phe Ala His Ala Gln Glu Gly Thr Leu Glu
    1               5                  10                  15
   Arg Ser Asp Trp Arg Lys Phe Phe Ser Glu Phe Gln Ala Lys Gly Thr
               20                  25                  30
   Ile Val Val Ala Asp Glu Arg Gln Ala Asp Arg Ala Met Leu Val Phe
           35                  40                  45
   Asp Pro Val Arg Ser Lys Lys Arg Tyr Ser Pro Ala Ser Thr Phe Lys
       50                  55                  60
   Ile Pro His Thr Leu Phe Ala Leu Asp Ala Gly Ala Val Arg Asp Glu
   65                  70                  75                  80
   Phe Gln Ile Phe Arg Trp Asp Gly Val Asn Arg Gly Phe Ala Gly His
                   85                  90                  95
   Asn Gln Asp Gln Asp Leu Arg Ser Ala Met Arg Asn Ser Thr Val Trp
   100 105 110
   Val Tyr Glu Leu Phe Ala Lys Glu Ile Gly Asp Asp Lys Ala Arg Arg
   115 120 125
   Tyr Leu Lys Lys Ile Asp Tyr Gly Asn Ala Tyr Pro Ser Thr Ser Asn
   130 135 140
   Gly Asp Tyr Trp Ile Glu Gly Ser Leu Ala Ile Ser Ala Gln Glu Gln
   145 150 155 160
   Ile Ala Phe Leu Arg Lys Leu Tyr Arg Asn Glu Leu Pro Phe Arg Val
   165 170 175
   Glu His Gln Arg Leu Val Lys Asp Leu Met Ile Val Glu Ala Gly Arg
   180 185 190
   Asn Trp Ile Leu Arg Ala Lys Thr Gly Trp Glu Gly Arg Met Gly Trp
   195 200 205
   Trp Val Gly Trp Val Glu Trp Pro Thr Gly Ser Val Phe Phe Ala Leu
   210 215 220
   Asn Ile Asp Thr Pro Asn Arg Met Asp Asp Leu Phe Lys Arg Glu Ala
   225 230 235 240
   Ile Val Arg Ala Ile Leu
   245
<210> 363
   <211> 266
   <212> PRT
   <213> RESISTANCE GENE
<400> 363
   Met Ile Ile Arg Phe Leu Ala Leu Leu Phe Ser Ala Val Val Leu Val
    1              5                   10                  15
   Ser Leu Gly His Ala Gln Asp Lys Thr His Glu Ser Ser Asn Trp Gly
               20                  25                  30
   Lys Tyr Phe Ser Asp Phe Asn Ala Lys Gly Thr Ile Val Val Val Asp
           35                  40                  45
   Glu Arg Thr Asn Gly Asn Ser Thr Ser Val Tyr Asn Glu Ser Arg Ala
       50                  55                  60
   Gln Gln Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe His Val Phe Arg
                   85                  90                  95
   Trp Asp Gly Ala Lys Arg Ser Phe Ala Gly His Asn Gln Asp Gln Asn
               100                 105                 110
   Leu Arg Ser Ala Met Arg Asn Ser Thr Val Trp Val Tyr Gln Leu Phe
           115                 120                 125
   Ala Lys Glu Ile Gly Glu Asn Lys Ala Arg Ser Tyr Leu Glu Lys Leu
       130                 135                 140
   Asn Tyr Gly Asn Ala Asp Pro Ser Thr Lys Ser Gly Asp Tyr Trp Ile
   145                 150                 155                 160
   Asp Gly Asn Leu Ala Ile Ser Ala Asn Glu Gln Ile Ser Ile Leu Lys
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Lys Arg Asp Trp Ile Leu Arg
           195                 200                 205
   Ala Lys Thr Gly Trp Asp Gly Gln Met Gly Trp Trp Val Gly Trp Val
       210                 215                 220
   Glu Trp Pro Thr Gly Pro Val Phe Phe Ala Leu Asn Ile Asp Thr Pro
   225                 230                 235                 240
   Asn Arg Met Glu Asp Leu His Lys Arg Glu Ala Ile Ala Arg Ala Ile
                   245                 250                 255
   Leu Gln Ser Val Asn Ala Leu Pro Pro Asn
               260                 265
<210> 364
   <211> 275
   <212> PRT
   <213> RESISTANCE GENE
<400> 364
   Met Lys Lys Phe Ile Leu Pro Ile Phe Ser Ile Ser Ile Leu Val Ser
    1               5                  10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Thr Tyr
               100                 105                 110
   Pro Met Trp Glu Lys Asp Met Thr Leu Gly Glu Ala Met Ala Leu Ser
           115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Glu Lys Met Leu Leu Ile Lys
           195                 200                 205
   Glu Val Asn Gly Ser Lys Ile Tyr Ala Lys Ser Gly Trp Gly Met Gly
       210                 215                 220
   Val Thr Pro Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Ser Gly Ser Ile Arg Asn Glu Ile Thr Tyr Lys Leu Leu Glu Asn Leu
               260                 265                 270
   Gly Ile Ile
           275
<210> 365
   <211> 275
   <212> PRT
   <213> RESISTANCE GENE
<400> 365
   Met Lys Lys Phe Ile Leu Pro Ile Phe Ser Ile Ser Ile Leu Val Ser
    1               5                  10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Thr Tyr
               100                 105                 110
   Pro Met Trp Glu Lys Asp Met Thr Leu Gly Glu Ala Met Ala Leu Ser
           115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Gly
       210                 215                 220
   Val Thr Pro Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Thr Gly Ser Ile Arg Asn Glu Ile Thr Tyr Lys Ser Leu Glu Asn Leu
               260                 265                 270
   Gly Ile Ile
           275
<210> 366
   <211> 273
   <212> PRT
   <213> RESISTANCE GENE
<400> 366
   Met Asn Lys Tyr Phe Thr Cys Tyr Val Val Ala Ser Leu Phe Leu Ser
     1               5                  10                  15
   Gly Cys Thr Val Gln His Asn Leu Ile Asn Glu Thr Pro Ser Gln Ile
                20                  25                  30
   Val Gln Gly His Asn Gln Val Ile His Gln Tyr Phe Asp Glu Lys Asn
            35                  40                  45
   Thr Ser Gly Val Leu Val Ile Gln Thr Asp Lys Lys Ile Asn Leu Tyr
        50                  55                  60
   Gly Asn Ala Leu Ser Arg Ala Asn Thr Glu Tyr Val Pro Ala Ser Thr
    65                  70                  75                  80
   Phe Lys Met Leu Asn Ala Leu Ile Gly Leu Glu Asn Gln Lys Ala Asp
                    85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Lys Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 367
   <211> 257
   <212> PRT
   <213> RESISTANCE GENE
<400> 367
   Met Ala Ile Arg Ile Phe Ala Ile Leu Phe Ser Ile Phe Ser Leu Ala
   1               5                   10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
       50                  55                  60
   Lys Lys Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe Gln Ile Phe Arg
                   85                  90                  95
   Trp Asp Gly Val Asn Arg Gly Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Glu Ile Gly Asp Asp Lys Ala Arg Arg Tyr Leu Lys Lys Ile
       130                 135                 140
   Asp Tyr Gly Asn Ala Asp Pro Ser Thr Ser Asn Gly Asp Tyr Cys Ile
   145                 150                 155                 160
   Glu Gly Ser Leu Ala Ile Ser Ala Gln Glu Gln Ile Ala Phe Leu Arg
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Ser Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu
<210> 368
   <211> 266
   <212> PRT
   <213> RESISTANCE GENE
<400> 368
   Met Lys Thr Phe Ala Ala Tyr Val Ile Thr Ala Cys Leu Ser Ser Thr
   1               5                   10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 369
   <211> 266
   <212> PRT
   <213> RESISTANCE GENE
<400> 369
   Met Ala Ile Arg Phe Leu Thr Ile Leu Leu Ser Thr Phe Phe Leu Thr
   1 5 10 15
   Ser Phe Val His Ala Gln Glu His Val Leu Glu Arg Ser Asp Trp Lys
   20 25 30
   Lys Phe Phe Ser Asp Leu Arg Ala Glu Gly Ala Ile Val Ile Ser Asp
   35 40 45
   Glu Arg Gln Ala Glu His Ala Leu Leu Val Phe Gly Gln Glu Arg Ala
   50 55 60
   Ala Lys Arg Tyr Ser Pro Ala Ser Thr Phe Lys Leu Pro His Thr Leu
   65 70 75 80
   Phe Ala Leu Asp Ala Asp Ala Val Arg Asp Glu Phe Gln Val Phe Arg
   85 90 95
   Trp Asp Gly Val Lys Arg Ser Phe Ala Gly His Asn Gln Asp Gln Asp
   100 105 110
   Leu Arg Ser Ala Met Arg Asn Ser Ala Val Trp Val Tyr Glu Leu Phe
   115 120 125
   Ala Lys Glu Ile Gly Lys Asp Lys Ala Arg His Tyr Leu Lys Gln Ile
   130 135 140
   Asp Tyr Gly Asn Ala Asp Pro Ser Thr Ile Lys Gly Asp Tyr Trp Ile
   145 150 155 160
   Asp Gly Asn Leu Glu Ile Ser Ala His Glu Gln Ile Ser Phe Leu Arg
   165 170 175
   Lys Leu Tyr Arg Asn Gln Leu Pro Phe Gln Val Glu His Gln Arg Leu
   180 185 190
   Val Lys Asp Leu Met Ile Thr Glu Ala Gly Arg Asn Trp Ile Leu Arg
   195 200 205
   Ala Lys Thr Gly Trp Glu Gly Arg Phe Gly Trp Trp Val Gly Trp Val
   210 215 220
   Glu Trp Pro Thr Gly Pro Val Phe Phe Ala Leu Asn Ile Asp Thr Pro
   225 230 235 240
   Asn Arg Thr Asp Asp Leu Phe Lys Arg Glu Ala Ile Ala Arg Ala Ile
   245 250 255
   Leu Arg Ser Ile Asp Ala Leu Pro Pro Asn
   260 265
<210> 370
   <211> 275
   <212> PRT
   <213> RESISTANCE GENE
<400> 370
   Met Ala Ile Arg Ile Phe Ala Ile Leu Phe Ser Ile Phe Ser Leu Ala
    1               5                  10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
       50                  55                  60
   Lys Lys Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe Gln Ile Phe Arg
                   85                  90                  95
   Trp Asp Gly Val Asn Arg Gly Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Glu Ile Gly Asp Asp Lys Ala Arg Arg Tyr Leu Lys Lys Ile
       130                 135                 140
   Asp Tyr Gly Asn Ala Asp Pro Ser Thr Ser Asn Gly Asp Tyr Trp Ile
   145                 150                 155                 160
   Glu Gly Ser Ile Ala Ile Ser Ala Gln Glu Gln Ile Ala Phe Leu Arg
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Ser Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 371
   <211> 275
   <212> PRT
   <213> RESISTANCE GENE
<400> 371
   Met Lys Lys Phe Ile Leu Pro Ile Phe Ser Ile Ser Ile Leu Val Ser
    1               5                  10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Thr Tyr
               100                 105                 110
   Pro Met Trp Glu Lys Asp Met Thr Leu Gly Glu Ala Met Ala Leu Ser
           115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Gly
       210                 215                 220
   Val Thr Pro Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Ser Gly Ser Ile Arg Asn Glu Ile Thr Tyr Lys Ser Leu Glu Asn Leu
               260                 265                 270
   Gly Ile Ile
           275
<210> 372
   <211> 266
   <212> PRT
   <213> RESISTANCE GENE
<400> 372
   Met Ala Ile Arg Phe Leu Thr Ile Leu Leu Ser Thr Phe Phe Leu Thr
   1               5                   10                  15
   Ser Phe Val His Ala Gln Glu His Val Leu Glu Arg Ser Asp Trp Lys
               20                  25                  30
   Lys Phe Phe Ser Asp Leu Arg Ala Glu Gly Ala Ile Val Ile Ser Asp
           35                  40                  45
   Glu Arg Gln Ala Glu His Ala Leu Leu Val Phe Gly Gln Glu Arg Ala
       50                  55                  60
   Ala Lys Arg Tyr Ser Pro Ala Ser Thr Phe Lys Leu Pro His Thr Leu
   65                  70                  75                  80
   Phe Ala Leu Asp Ala Asp Ala Val Arg Asp Glu Phe Gln Val Phe Arg
                   85                  90                  95
   Trp Asp Gly Val Lys Arg Ser Phe Ala Gly His Asn Gln Asp Gln Asp
               100                 105                 110
   Leu Arg Ser Ala Met Arg Asn Ser Ala Val Trp Val Tyr Glu Leu Phe
           115                 120                 125
   Ala Lys Glu Ile Gly Glu Asp Lys Ala Arg Arg Tyr Leu Lys Gln Ile
       130                 135                 140
   Asp Tyr Gly Asn Ala Asp Pro Ser Thr Ile Lys Gly Asp Tyr Trp Ile
   145                 150                 155                 160
   Asp Gly Asn Leu Glu Ile Ser Ala His Glu Gln Ile Ser Phe Leu Arg
                   165                 170                 175
   Lys Leu Tyr Arg Asn Gln Leu Pro Phe Gln Val Glu His Gln Arg Leu
               180                 185                 190
   Val Lys Asp Leu Met Ile Thr Glu Ala Gly Arg Asn Trp Ile Leu Arg
           195                 200                 205
   Ala Lys Thr Gly Trp Glu Gly Arg Phe Gly Trp Trp Val Gly Trp Val
       210                 215                 220
   Glu Trp Pro Thr Gly Pro Val Phe Phe Ala Leu Asn Ile Asp Thr Pro
   225                 230                 235                 240
   Asn Arg Thr Asp Asp Leu Phe Lys Arg Glu Ala Ile Ala Arg Ala Ile
                   245                 250                 255
   Leu Arg Ser Ile Asp Ala Leu Pro Pro Asn
               260                 265
<210> 373
   <211> 266
   <212> PRT
   <213> RESISTANCE GENE
<400> 373
   Met Ala Ile Arg Phe Phe Thr Ile Leu Leu Ser Thr Phe Phe Leu Thr
   1               5                   10                  15
   Ser Phe Val Tyr Ala Gln Glu His Val Val Ile Arg Ser Asp Trp Lys
               20                  25                  30
   Lys Phe Phe Ser Asp Leu Gln Ala Glu Gly Ala Ile Val Ile Ala Asp
           35                  40                  45
   Glu Arg Gln Ala Lys His Thr Leu Ser Val Phe Asp Gln Glu Arg Ala
       50                  55                  60
   Ala Lys Arg Tyr Ser Pro Ala Ser Thr Phe Lys Ile Pro His Thr Leu
   65                  70                  75                  80
   Phe Ala Leu Asp Ala Asp Ala Val Arg Asp Glu Phe Gln Val Phe Arg
                   85                  90                  95
   Trp Asp Gly Val Asn Arg Ser Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Asp Ile Gly Glu Asp Lys Ala Arg Arg Tyr Leu Lys Gln Ile
       130                 135                 140
   Asp Tyr Gly Asn Val Asp Pro Ser Thr Ile Lys Gly Asp Tyr Trp Ile
   145                 150                 155                 160
   Asp Gly Asn Leu Lys Ile Ser Ala His Glu Gln Ile Leu Phe Leu Arg
                   165                 170                 175
   Lys Leu Tyr Arg Asn Gln Leu Pro Phe Lys Val Glu His Gln Arg Leu
               180                 185                 190
   Val Lys Asp Leu Met Ile Thr Glu Ala Gly Arg Ser Trp Ile Leu Arg
           195                 200                 205
   Ala Lys Thr Gly Trp Glu Gly Arg Phe Gly Trp Trp Val Gly Trp Ile
       210                 215                 220
   Glu Trp Pro Thr Gly Pro Val Phe Phe Ala Leu Asn Ile Asp Thr Pro
   225                 230                 235                 240
   Asn Arg Thr Asp Asp Leu Phe Lys Arg Glu Ala Ile Ala Arg Ala Ile
                   245                 250                 255
   Leu Arg Ser Ile Asp Ala Leu Pro Pro Asn
               260                 265
<210> 374
   <211> 274
   <212> PRT
   <213> RESISTANCE GENE
<400> 374
   Met Asn Lys Tyr Phe Thr Cys Tyr Val Val Ala Ser Leu Phe Leu Ser
   1               5                   10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Glu Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Ala Met Asp Ile
       210                 215                 220
   Lys Pro Gln Val Gly Trp Leu Thr Gly Trp Val Glu Gln Pro Asp Gly
   225                 230                 235                 240
   Lys Ile Val Ala Phe Ala Leu Asn Met Glu Met Arg Ser Glu Met Pro
                   245                 250                 255
   Ala Ser Ile Arg Asn Glu Leu Leu Met Lys Ser Leu Lys Gln Leu Asn
               260                 265                 270
   Ile Ile
<210> 375
   <211> 275
   <212> PRT
   <213> RESISTANCE GENE
<400> 375
   Met Ala Ile Gln Ile Phe Ala Ile Leu Phe Ser Thr Phe Val Leu Ala
   1 5 10 15
   Thr Phe Ala His Ala Gln Asp Gly Thr Leu Glu Arg Ser Asp Trp Gly
   20 25 30
   Lys Phe Phe Ser Asp Phe Gln Ala Lys Gly Thr Ile Val Val Ala Asp
   35 40 45
   Glu Arg Gln Ala Asp His Ala Ile Leu Val Phe Asp Gln Ala Arg Ser
   50 55 60
   Met Lys Arg Tyr Ser Pro Ala Ser Thr Phe Lys Ile Pro His Thr Leu
   65 70 75 80
   Phe Ala Leu Asp Ala Gly Ala Val Arg Asp Glu Phe Gln Ile Phe Arg
   85 90 95
   Trp Asp Gly Val Lys Arg Ser Phe Ala Gly His Asn Lys Asp Gln Asp
   100 105 110
   Leu Arg Ser Ala Met Arg Asn Ser Thr Val Trp Val Tyr Glu Leu Phe
   115 120 125
   Ala Lys Glu Ile Gly Asp Gly Lys Ala Arg Arg Tyr Leu Lys Gln Ile
   130 135 140
   Gly Tyr Gly Asn Ala Asp Pro Ser Thr Ser His Gly Asp Tyr Trp Ile
   145 150 155 160
   Glu Gly Ser Leu Ala Ile Ser Ala Gln Glu Gln Ile Ala Phe Leu Arg
   165 170 175
   Lys Leu Tyr Gln Asn Asp Leu Pro Phe Arg Val Glu His Gln Arg Leu
   180 185 190
   Val Lys Asp Leu Met Ile Val Glu Ala Gly Arg Asn Trp Ile Leu Arg
   195 200 205
   Ala Lys Thr Gly Trp Glu Gly Ser Met Gly Trp Trp Val Gly Trp Val
   210 215 220
   Glu Trp Pro Thr Gly Pro Val Phe Phe Ala Leu Asn Ile Asp Thr Pro
   225 230 235 240
   Asn Arg Met Asp Asp Leu Phe Lys Arg Glu Ala Ile Ala Arg Ala Ile
   245 250 255
   Leu Leu Ser Ile Glu Ala Leu Pro Pro Asn Pro Ala Val His Ser Asp
   260 265 270
   Ala Ala Arg
   275
<210> 376
   <211> 276
   <212> PRT
   <213> RESISTANCE GENE
<400> 376
   Met Lys Asn Thr Ile His Ile Asn Phe Ala Ile Phe Leu Ile Ile Ala
    1               5                  10                  15
   Asn Ile Ile Tyr Ser Ser Ala Ser Ala Ser Thr Asp Ile Ser Thr Val
               20                  25                  30
   Ala Ser Gln Leu Phe Glu Gly Thr Glu Gly Cys Phe Leu Leu Tyr Asp
           35                  40                  45
   Ala Ser Thr Asn Ala Glu Ile Ala Gln Phe Asn Lys Ala Lys Cys Ala
       50                  55                  60
   Ala Gln Met Ala Pro Asp Ser Thr Phe Lys Ile Ala Leu Ser Leu Met
    65                 70                  75                  80
   Ala Phe Asp Ala Glu Ile Ile Asp Gln Lys Thr Ile Phe Lys Trp Asp
                   85                  90                  95
   Lys Ile Pro Lys Gly Met Glu Ile Trp Asn Ser Asn His Thr Pro Lys
               100                 105                 110
   Thr Trp Met Gln Phe Ser Val Val Trp Val Ser Gln Glu Ile Thr Gln
           115                 120                 125
   Lys Ile Gly Leu Asn Lys Ile Lys Asn Tyr Leu Lys Asp Phe Asp Tyr
       130                 135                 140
   Gly Asn Gln Asp Phe Ser Gly Asp Lys Glu Arg Asn Asn Gly Leu Thr
   145                 150                 155                 160
   Glu Ala Trp Leu Glu Ser Ser Leu Lys Ile Ser Pro Glu Glu Gln Ile
                   165                 170                 175
   Gln Phe Leu Arg Lys Ile Ile Asn His Asn Leu Pro Val Arg Asn Ser
               180                 185                 190
   Ala Ile Glu Asn Thr Ile Asp Asn Met Tyr Leu Gln Asp Leu Glu Asn
           195                 200                 205
   Ser Thr Lys Leu Tyr Gly Lys Thr Gly Ala Gly Phe Thr Ala Asn Arg
       210                 215                 220
   Thr Leu Gln Asn Gly Trp Phe Glu Gly Phe Ile Ile Ser Lys Ser Gly
   225                 230                 235                 240
   His Lys Tyr Val Phe Val Ser Ala Leu Thr Gly Ser Leu Gly Ser Asn
                   245                 250                 255
   Leu Thr Ser Ser Ile Lys Ala Lys Lys Asn Ala Ile Thr Ile Leu Asn
               260                 265                 270
   Thr Leu Asn Leu
           275
<210> 377
   <211> 254
   <212> PRT
   <213> RESISTANCE GENE
<400> 377
   Met Leu Leu Phe Met Phe Ser Ile Ile Ser Phe Gly Asn Glu Asn Gln
    1               5                  10                  15
   Phe Met Lys Glu Ile Phe Glu Arg Lys Gly Leu Asn Gly Thr Phe Val
               20                  25                  30
   Val Tyr Asp Leu Lys Asn Asp Lys Ile Asp Tyr Tyr Asn Leu Asp Arg
           35                  40                  45
   Ala Asn Glu Arg Phe Tyr Pro Ala Ser Ser Phe Lys Ile Phe Asn Thr
       50                  55                  60
   Leu Ile Gly Leu Glu Asn Gly Ile Val Lys Asn Val Asp Glu Met Phe
    65                 70                  75                  80
   Tyr Tyr Tyr Asp Gly Ser Lys Val Phe Leu Asp Ser Trp Ala Lys Asp
                   85                  90                  95
   Ser Asn Leu Arg Tyr Ala Ile Lys Val Ser Gln Val Pro Ala Tyr Lys
                   100                 105                 110
   Lys Leu Ala Arg Glu Leu Gly Lys Glu Arg Met Gln Glu Gly Leu Asn
               115                 120                 125
   Lys Leu Asn Tyr Gly Asn Lys Glu Ile Gly Ser Glu Ile Asp Lys Phe
       130                 135                 140
   Trp Leu Glu Gly Pro Leu Lys Ile Ser Ala Met Glu Gln Val Lys Leu
   145                 150                 155                 160
   Leu Asn Leu Leu Ser Gln Ser Lys Leu Pro Phe Lys Leu Glu Asn Gln
                   165                 170                 175
   Glu Gln Val Lys Asp Ile Thr Ile Leu Glu Lys Lys Asp Asp Phe Ile
               180                 185                 190
   Leu His Gly Lys Thr Gly Trp Ala Thr Asp Asn Ile Val Val Pro Ile
           195                 200                 205
   Gly Trp Phe Val Gly Trp Ile Glu Thr Ser Asp Asn Ile Tyr Ser Phe
       210                 215                 220
   Ala Ile Asn Leu Asp Ile Ser Asp Ser Lys Phe Leu Pro Lys Arg Glu
   225                 230                 235                 240
   Glu Ile Val Arg Glu Tyr Phe Lys Asn Ile Asn Val Ile Lys
                   245                 250
<210> 378
   <211> 265
   <212> PRT
   <213> RESISTANCE GENE
<400> 378
   Met Arg Val Leu Ala Leu Ser Ala Val Phe Leu Val Ala Ser Ile Ile
   1               5                   10                  15
   Gly Met Pro Ala Val Ala Lys Glu Trp Gln Glu Asn Lys Ser Trp Asn
               20                  25                  30
   Ala His Phe Thr Glu His Lys Ser Gln Gly Val Val Val Leu Trp Asn
           35                  40                  45
   Glu Asn Lys Gln Gln Gly Phe Thr Asn Asn Leu Lys Arg Ala Asn Gln
       50                  55                  60
   Ala Phe Leu Pro Ala Ser Thr Phe Lys Ile Pro Asn Ser Leu Ile Ala
   65                  70                  75                  80
   Leu Asp Leu Gly Val Val Lys Asp Glu His Gln Val Phe Lys Trp Asp
                   85                  90                  95
   Gly Gln Thr Arg Asp Ile Ala Thr Trp Asn Arg Asp His Asn Leu Ile
               100                 105                 110
   Thr Ala Met Lys Tyr Ser Val Val Pro Val Tyr Gln Glu Phe Ala Arg
           115                 120                 125
   Gln Ile Gly Glu Ala Arg Met Ser Lys Met Leu His Ala Phe Asp Tyr
       130                 135                 140
   Gly Asn Glu Asp Ile Ser Gly Asn Val Asp Ser Phe Trp Leu Asp Gly
   145                 150                 155                 160
   Gly Ile Arg Ile Ser Ala Thr Glu Gln Ile Ser Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Gly Asp Tyr Ile Ile Arg Ala Lys
           195                 200                 205
   Thr Gly Tyr Ser Thr Arg Ile Glu Pro Lys Ile Gly Trp Trp Val Gly
       210                 215                 220
   Trp Val Glu Leu Asp Asp Asn Val Trp Phe Phe Ala Met Asn Met Asp
   225                 230                 235                 240
   Met Pro Thr Ser Asp Gly Leu Gly Leu Arg Gln Ala Ile Thr Lys Glu
                   245                 250                 255
   Val Leu Lys Gln Glu Lys Ile Ile Pro
               260                 265
<210> 379
   <211> 271
   <212> PRT
   <213> RESISTANCE GENE
<400> 379
   Met Leu Ser Arg Tyr Ser Lys Thr Leu Ala Phe Ala Val Val Ala Cys
    1               5                  10                  15
   Thr Leu Ala Ile Ser Thr Ala Thr Ala His Ala Glu Leu Val Val Arg
               20                  25                  30
   Asn Asp Leu Lys Arg Val Phe Asp Asp Ala Gly Val Ser Gly Thr Phe
           35                  40                  45
   Val Leu Met Asp Ile Thr Ala Asp Arg Thr Tyr Val Val Asp Pro Ala
       50                  55                  60
   Arg Ala Ala Arg Ser Ile His Pro Ala Ser Thr Phe Lys Ile Pro Asn
    65                 70                  75                  80
   Ser Leu Ile Ala Phe Asp Thr Gly Ala Val Arg Asp Asp Gln Glu Val
                   85                  90                  95
   Leu Pro Tyr Gly Gly Lys Pro Gln Pro Tyr Glu Gln Trp Glu His Asp
               100                 105                 110
   Met Ala Leu Pro Glu Ala Ile Arg Leu Ser Ala Val Pro Ile Tyr Gln
           115                 120                 125
   Glu Val Ala Arg Arg Val Gly Phe Glu Arg Met Gln Ala Tyr Val Asp
       130                 135                 140
   Ala Phe Asp Tyr Gly Asn Arg Gln Leu Gly Ser Ala Ile Asp Gln Phe
   145                 150                 155                 160
   Trp Leu Arg Gly Pro Leu Glu Ile Ser Ala Phe Glu Glu Ala Arg Phe
                   165                 170                 175
   Thr Ser Arg Met Ala Leu Lys Gln Leu Pro Val Lys Pro Arg Thr Trp
               180                 185                 190
   Asp Met Val Gln Arg Met Leu Leu Ile Glu Gln Gln Gly Asp Ala Ala
           195                 200                 205
   Leu Tyr Ala Lys Thr Gly Val Ala Thr Glu Tyr Gln Pro Glu Ile Gly
       210                 215                 220
   Trp Trp Ala Gly Trp Val Glu Arg Ala Gly His Val Tyr Ala Phe Ala
   225                 230                 235                 240
   Leu Asn Ile Asp Met Pro Arg Glu Gly Asp Met Ala Lys Arg Ile Pro
                   245                 250                 255
   Leu Gly Lys Gln Leu Met Arg Ala Leu Glu Val Trp Pro Ala Pro
               260                 265                 270
<210> 380
   <211> 262
   <212> PRT
   <213> RESISTANCE GENE
<400> 380
   Met Arg Pro Leu Leu Phe Ser Ala Leu Leu Leu Leu Ser Gly His Thr
    1               5                  10                  15
   Gln Ala Ser Glu Trp Asn Asp Ser Gln Ala Val Asp Lys Leu Phe Gly
               20                  25                  30
   Ala Ala Gly Val Lys Gly Thr Phe Val Leu Tyr Asp Val Gln Arg Gln
           35                  40                  45
   Arg Tyr Val Gly His Asp Arg Glu Arg Ala Glu Thr Arg Phe Val Pro
       50                  55                  60
   Ala Ser Thr Tyr Lys Val Ala Asn Ser Leu Ile Gly Leu Ser Thr Gly
   65                  70                  75                  80
   Ala Val Arg Ser Ala Asp Glu Val Leu Pro Tyr Gly Gly Lys Pro Gln
                   85                  90                  95
   Arg Phe Lys Ala Trp Glu His Asp Met Ser Leu Arg Asp Ala Ile Lys
               100                 105                 110
   Ala Ser Asn Val Pro Val Tyr Gln Glu Leu Ala Arg Arg Ile Gly Leu
           115                 120                 125
   Glu Arg Met Arg Ala Asn Val Ser Arg Leu Gly Tyr Gly Asn Ala Glu
       130                 135                 140
   Ile Gly Gln Val Val Asp Asn Phe Trp Leu Val Gly Pro Leu Lys Ile
   145                 150                 155                 160
   Ser Ala Met Glu Gln Thr Arg Phe Leu Leu Arg Leu Ala Gln Gly Glu
                   165                 170                 175
   Leu Pro Phe Pro Ala Pro Val Gln Ser Thr Val Arg Ala Met Thr Leu
               180                 185                 190
   Leu Glu Ser Gly Pro Gly Trp Glu Leu His Gly Lys Thr Gly Trp Cys
           195                 200                 205
   Phe Asp Cys Thr Pro Glu Leu Gly Trp Trp Val Gly Trp Val Lys Arg
       210                 215                 220
   Asn Glu Arg Leu Tyr Gly Phe Ala Leu Asn Ile Asp Met Pro Gly Gly
   225                 230                 235                 240
   Glu Ala Asp Ile Gly Lys Arg Val Glu Leu Gly Lys Ala Ser Leu Lys
                   245                 250                 255
   Ala Leu Gly Ile Leu Pro
               260
<210> 381
   <211> 289
   <212> PRT
   <213> RESISTANCE GENE
<400> 381
   Met Asn Lys Gly Leu His Arg Lys Arg Leu Ser Lys Arg Leu Leu Leu
   1               5                   10                  15
   Pro Met Leu Leu Cys Leu Leu Ala Gln Gln Thr Gln Ala Val Ala Ala
               20                  25                  30
   Glu Gln Thr Lys Val Ser Asp Val Cys Ser Glu Val Thr Ala Glu Gly
           35                  40                  45
   Trp Gln Glu Val Arg Arg Trp Asp Lys Leu Phe Glu Ser Ala Gly Val
       50                  55                  60
   Lys Gly Ser Leu Leu Leu Trp Asp Gln Lys Arg Ser Leu Gly Leu Ser
   65                  70                  75                  80
   Asn Asn Leu Ser Arg Ala Ala Glu Gly Phe Ile Pro Ala Ser Thr Phe
                   85                  90                  95
   Lys Leu Pro Ser Ser Leu Ile Ala Leu Glu Thr Gly Ala Val Arg Asp
               100                 105                 110
   Glu Thr Ser Arg Phe Ser Trp Asp Gly Lys Val Arg Glu Ile Ala Val
           115                 120                 125
   Trp Asn Arg Asp Gln Ser Phe Arg Thr Ala Met Lys Tyr Ser Val Val
       130                 135                 140
   Pro Val Tyr Gln Gln Leu Ala Arg Glu Ile Gly Pro Lys Val Met Ala
   145                 150                 155                 160
   Ala Met Val Arg Gln Leu Glu Tyr Gly Asn Gln Asp Ile Gly Gly Gln
                   165                 170                 175
   Ala Asp Ser Phe Trp Leu Asp Gly Gln Leu Arg Ile Thr Ala Phe Gln
               180                 185                 190
   Gln Val Asp Phe Leu Arg Gln Leu His Asp Asn Lys Leu Pro Val Ser
           195                 200                 205
   Glu Arg Ser Gln Arg Ile Val Lys Gln Met Met Leu Thr Glu Ala Ser
       210                 215                 220
   Thr Asp Tyr Ile Ile Arg Ala Lys Thr Gly Tyr Gly Val Arg Arg Thr
   225                 230                 235                 240
   Pro Ala Ile Gly Trp Trp Val Gly Trp Leu Glu Leu Asp Asp Asn Thr
                   245                 250                 255
   Val Tyr Phe Ala Val Asn Leu Asp Leu Ala Ser Ala Ser Gln Leu Pro
               260                 265                 270
   Leu Arg Gln Gln Leu Val Lys Gln Val Leu Lys Gln Glu Gln Leu Leu
           275                 280                 285
   Pro
<210> 382
   <211> 285
   <212> PRT
   <213> RESISTANCE GENE
<400> 382
   Met Asn Thr Ile Ile Ser Arg Arg Trp Arg Ala Gly Leu Trp Arg Arg
   1 5 10 15
   Leu Val Gly Ala Val Val Leu Pro Ala Thr Leu Ala Ala Thr Pro Ala
   20 25 30
   Ala Tyr Ala Ala Asp Val Pro Lys Ala Ala Leu Gly Arg Ile Thr Glu
   35 40 45
   Arg Ala Asp Trp Gly Lys Leu Phe Ala Ala Glu Gly Val Lys Gly Thr
   50 55 60
   Ile Val Val Leu Asp Ala Arg Thr Gln Thr Tyr Gln Ala Tyr Asp Ala
   65 70 75 80
   Ala Arg Ala Glu Lys Arg Met Ser Pro Ala Ser Thr Tyr Lys Ile Phe
   85 90 95
   Asn Ser Leu Leu Ala Leu Asp Ser Gly Ala Leu Asp Asn Glu Arg Ala
   100 105 110
   Ile Ile Pro Trp Asp Gly Lys Pro Arg Arg Ile Lys Asn Trp Asn Ala
   115 120 125
   Ala Met Asp Leu Arg Thr Ala Phe Arg Val Ser Cys Leu Pro Cys Tyr
   130 135 140
   Gln Val Val Ser His Lys Ile Gly Arg Arg Tyr Ala Gln Ala Lys Leu
   145 150 155 160
   Asn Glu Val Gly Tyr Gly Asn Arg Thr Ile Gly Gly Ala Pro Asp Ala
   165 170 175
   Tyr Trp Val Asp Asp Ser Leu Gln Ile Ser Ala Arg Glu Gln Val Asp
   180 185 190
   Phe Val Gln Arg Leu Ala Arg Gly Thr Leu Pro Phe Ser Ala Arg Ser
   195 200 205
   Gln Asp Ile Val Arg Gln Met Ser Ile Val Glu Ala Thr Pro Asp Tyr
   210 215 220
   Val Leu His Gly Lys Thr Gly Trp Phe Val Asp Lys Lys Pro Asp Ile
   225 230 235 240
   Gly Trp Trp Val Gly Trp Ile Glu Arg Asp Gly Asn Ile Thr Ser Val
   245 250 255
   Ala Ile Asn Ile Asp Met Leu Ser Glu Ala Asp Ala Pro Lys Arg Ala
   260 265 270
   Arg Ile Val Lys Ala Val Leu Lys Asp Leu Lys Leu Ile
   275 280 285
<210> 383
   <211> 266
   <212> PRT
   <213> RESISTANCE GENE
<400> 383
   Met Lys Thr Phe Ala Ala Tyr Val Ile Thr Ala Cys Leu Ser Ser Thr
   1 5 10 15
   Ala Leu Ala Ser Ser Ile Thr Glu Asn Thr Phe Trp Asn Lys Glu Phe
   20 25 30
   Ser Ala Glu Ala Val Asn Gly Val Phe Val Leu Cys Lys Ser Ser Ser
   35 40 45
   Lys Ser Cys Ala Thr Asn Asn Leu Ala Arg Ala Ser Lys Glu Tyr Leu
   50 55 60
   Pro Ala Ser Thr Phe Lys Ile Pro Asn Ala Ile Ile Gly Leu Glu Thr
   65 70 75 80
   Gly Val Ile Lys Asn Glu His Gln Ile Phe Lys Trp Asp Gly Lys Pro
   85 90 95
   Arg Ala Met Lys Gln Trp Glu Arg Asp Leu Ser Leu Arg Gly Ala Ile
   100 105 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Ala Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 384
   <211> 265
   <212> PRT
   <213> RESISTANCE GENE
<400> 384
   Met Arg Val Leu Ala Leu Ser Ala Val Leu Val Val Ala Ser Ile Val
    1               5                  10                  15
   Gly Met Pro Ala Met Ala Asn Glu Trp Gln Glu Lys Pro Ser Trp Asn
               20                  25                  30
   Thr His Phe Ser Glu His Lys Ala Gln Gly Val Ile Val Leu Trp Asn
           35                  40                  45
   Glu Asn Lys Gln Gln Gly Phe Thr Asn Asn Leu Lys Arg Ala Asn Gln
       50                  55                  60
   Ala Phe Leu Pro Ala Ser Thr Phe Lys Ile Pro Asn Ser Leu Ile Ala
    65                 70                  75                  80
   Leu Asp Leu Gly Val Val Lys Asp Glu His Gln Val Phe Lys Trp Asp
                   85                  90                  95
   Gly Gln Thr Arg Asp Ile Ala Ala Trp Asn Arg Asp His Asp Leu Ile
               100                 105                 110
   Thr Ala Met Lys Tyr Ser Val Val Pro Val Tyr Gln Glu Phe Ala Arg
           115                 120                 125
   Gln Ile Gly Gln Ala Arg Met Ser Lys Met Leu His Ala Phe Asp Tyr
       130                 135                 140
   Gly Asn Glu Asp Ile Ser Gly Asn Leu Asp Ser Phe Trp Leu Asp Gly
   145                 150                 155                 160
   Gly Ile Arg Ile Ser Ala Thr Glu Gln Val Ala Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Ser Asp Tyr Ile Ile Arg Ala Lys
           195                 200                 205
   Thr Gly Tyr Ser Thr Arg Ile Glu Pro Gln Ile Gly Trp Trp Val Gly
       210                 215                 220
   Trp Val Glu Leu Asp Asp Asn Val Trp Phe Phe Ala Met Asn Met Asp
   225                 230                 235                 240
   Met Pro Thr Ala Asp Gly Leu Gly Leu Arg Gln Ala Ile Thr Lys Glu
                   245                 250                 255
   Val Leu Lys Gln Glu Lys Ile Ile Pro
               260                 265
<210> 385
   <211> 257
   <212> PRT
   <213> RESISTANCE GENE
<400> 385
   Met Lys Lys Ile Thr Leu Phe Leu Leu Phe Leu Asn Leu Val Phe Gly
    1               5                  10                  15
   Gln Asp Lys Ile Leu Asn Asn Trp Phe Lys Glu Tyr Asn Thr Ser Gly
               20                  25                  30
   Thr Phe Val Phe Tyr Asp Gly Lys Thr Trp Ala Ser Asn Asp Phe Ser
           35                  40                  45
   Arg Ala Met Glu Thr Phe Ser Pro Ala Ser Thr Phe Lys Ile Phe Asn
       50                  55                  60
   Ala Leu Ile Ala Leu Asp Ser Gly Val Ile Lys Thr Lys Lys Glu Ile
   65                  70                  75                  80
   Phe Tyr His Tyr Arg Gly Glu Lys Val Phe Leu Ser Ser Trp Ala Gln
                   85                  90                  95
   Asp Met Asn Leu Ser Ser Ala Ile Lys Tyr Ser Asn Val Leu Ala Phe
               100                 105                 110
   Lys Glu Val Ala Arg Arg Ile Gly Ile Lys Thr Met Gln Glu Tyr Leu
           115                 120                 125
   Asn Lys Leu His Tyr Gly Asn Ala Lys Ile Ser Lys Ile Asp Thr Phe
       130                 135                 140
   Trp Leu Asp Asn Ser Leu Lys Ile Ser Ala Lys Glu Gln Ala Ile Leu
   145                 150                 155                 160
   Leu Phe Arg Leu Ser Gln Asn Ser Leu Pro Phe Ser Gln Glu Ala Met
                   165                 170                 175
   Asn Ser Val Lys Glu Met Ile Tyr Leu Lys Asn Met Glu Asn Leu Glu
               180                 185                 190
   Leu Phe Gly Lys Thr Gly Phe Asn Asp Gly Gln Lys Ile Ala Trp Ile
           195                 200                 205
   Val Gly Phe Val Tyr Leu Lys Asp Glu Asn Lys Tyr Lys Ala Phe Ala
       210                 215                 220
   Leu Asn Leu Asp Ile Asp Lys Phe Glu Asp Leu Tyr Lys Arg Glu Lys
   225                 230                 235                 240
   Ile Leu Glu Lys Tyr Leu Asp Glu Leu Val Lys Lys Val Lys Asn Asp
                   245                 250                 255
   Gly
<210> 386
   <211> 268
   <212> PRT
   <213> RESISTANCE GENE
<400> 386
   Met Ser Lys Lys Asn Phe Ile Leu Ile Phe Ile Phe Val Ile Leu Ile
    1               5                  10                  15
   Ser Cys Lys Asn Thr Glu Lys Ile Ser Asn Glu Thr Thr Leu Ile Asp
               20                  25                  30
   Asn Ile Phe Thr Asn Ser Asn Ala Glu Gly Thr Leu Val Ile Tyr Asn
           35                  40                  45
   Leu Asn Asp Asp Lys Tyr Ile Ile His Asn Lys Glu Arg Ala Glu Gln
       50                  55                  60
   Arg Phe Tyr Pro Ala Ser Thr Phe Lys Ile Tyr Asn Ser Leu Ile Gly
   65                  70                  75                  80
   Leu Asn Glu Lys Ala Val Lys Asp Val Asp Glu Val Phe Tyr Lys Leu
                   85                  90                  95
   Met Ala Lys Ser Phe Leu Glu Ser Trp Ala Lys Asp Ser Asn Leu Arg
               100                 105                 110
   Tyr Ala Ile Lys Asn Ser Gln Val Pro Ala Tyr Lys Glu Leu Ala Arg
           115                 120                 125
   Arg Ile Gly Ile Lys Lys Met Lys Glu Asn Ile Glu Lys Leu Asp Phe
       130                 135                 140
   Gly Asn Lys Ser Ile Gly Asp Ser Val Asp Thr Phe Trp Leu Glu Gly
   145                 150                 155                 160
   Pro Leu Glu Ile Ser Ala Met Glu Gln Val Lys Leu Leu Thr Lys Leu
                   165                 170                 175
   Ala Gln Asn Glu Leu Gln Tyr Pro Ile Glu Ile Gln Lys Ala Ile Ser
               180                 185                 190
   Asp Ile Thr Ile Thr Arg Ala Asn Leu His Ile Thr Leu His Gly Lys
           195                 200                 205
   Thr Gly Leu Ala Asp Ser Lys Asn Met Thr Thr Glu Pro Ile Gly Trp
       210                 215                 220
   Phe Val Gly Trp Leu Glu Glu Asn Asp Asn Ile Tyr Val Phe Ala Leu
   225                 230                 235                 240
   Asn Ile Asp Asn Ile Asn Ser Asp Asp Leu Ala Lys Arg Ile Asn Ile
                   245                 250                 255
   Val Lys Glu Ser Leu Lys Ala Leu Asn Leu Leu Lys
               260                 265
<210> 387
   <211> 273
   <212> PRT
   <213> RESISTANCE GENE
<400> 387
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
    65                 70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Lys Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 388
   <211> 274
   <212> PRT
   <213> RESISTANCE GENE
<400> 388
   Met Asn Ile Gln Ala Leu Leu Leu Ile Thr Ser Ala Ile Phe Ile Ser
    1               5                  10                  15
   Ala Cys Ser Pro Tyr Ile Val Thr Ala Asn Pro Asn Tyr Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Ile Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Ser
                   245                 250                 255
   Ser Ser Val Arg Lys Glu Ile Thr Tyr Arg Gly Leu Glu Gln Leu Gly
               260                 265                 270
   Ile Leu
<210> 389
   <211> 274
   <212> PRT
   <213> RESISTANCE GENE
<400> 389
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Gly Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 390
   <211> 274
   <212> PRT
   <213> RESISTANCE GENE
<400> 390
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 391
   <211> 274
   <212> PRT
   <213> RESISTANCE GENE
<400> 391
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 392
   <211> 274
   <212> PRT
   <213> RESISTANCE GENE
<400> 392
   Met Asn Ile Lys Ala Leu Leu Leu Ile Thr Ser Ala Ile Phe Ile Ser
   1               5                   10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Glu Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 393
   <211> 274
   <212> PRT
   <213> RESISTANCE GENE
<400> 393
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Asp Lys Ala Glu Lys Ile Lys Asn Leu Phe Asn Glu Ala
           35                  40                  45
   His Thr Thr Gly Val Leu Val Ile His Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Glu Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 394
   <211> 274
   <212> PRT
   <213> RESISTANCE GENE
<400> 394
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Lys Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asn Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln His Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 395
   <211> 274
   <212> PRT
   <213> RESISTANCE GENE
<400> 395
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Thr
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 396
   <211> 274
   <212> PRT
   <213> RESISTANCE GENE
<400> 396
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Ala Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Thr Pro
                   245                 250                 255
   Ser Ser Val Arg Lys Glu Ile Thr Tyr Lys Ser Leu Glu Gln Leu Gly
               260                 265                 270
   Ile Leu
<210> 397
   <211> 274
   <212> PRT
   <213> RESISTANCE GENE
<400> 397
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Pro Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 398
   <211> 274
   <212> PRT
   <213> RESISTANCE GENE
<400> 398
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Lys Leu Gly
               260                 265                 270
   Ile Leu
<210> 399
   <211> 274
   <212> PRT
   <213> RESISTANCE GENE
<400> 399
   Met Asn Ile Lys Thr Leu Leu Leu Ile Thr Ser Thr Ile Phe Ile Ser
    1               5                  10                  15
   Ala Cys Ser Pro Tyr Ile Val Thr Ala Asn Pro Asn His Ser Thr Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Ile Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Ile Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Leu Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 400
   <211> 274
   <212> PRT
   <213> RESISTANCE GENE
<400> 400
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Ser Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Ala Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 401
   <211> 274
   <212> PRT
   <213> RESISTANCE GENE
<400> 401
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
               35                  40                  45
   His Thr Thr Gly Val Leu Val Ile Gln Gln Gly Gln Thr Gln Gln Ser
           50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Leu Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 402
   <211> 274
   <212> PRT
   <213> RESISTANCE GENE
<400> 402
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Asp Lys Ala Glu Lys Ile Lys Asn Leu Phe Asn Glu Ala
           35                  40                  45
   His Thr Thr Gly Val Leu Val Ile His Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Glu Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Ser Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 403
   <211> 274
   <212> PRT
   <213> RESISTANCE GENE
<400> 403
   Met Asn Ile Lys Thr Leu Leu Leu Ile Thr Ser Thr Ile Phe Ile Ser
     1               5                  10                  15
   Ala Cys Ser Pro Tyr Ile Val Thr Ala Asn Pro Asn His Ser Thr Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Ile Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Ile Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Leu Lys Ala Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 404
   <211> 274
   <212> PRT
   <213> RESISTANCE GENE
<400> 404
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
               20                  25                  30
   Lys Ser Asp Lys Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 405
   <211> 274
   <212> PRT
   <213> RESISTANCE GENE
<400> 405
   Met Asn Ile Lys Thr Leu Leu Leu Ile Thr Ser Ala Ile Phe Ile Ser
     1               5                  10                  15
   Ala Cys Ser His Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Thr Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 406
   <211> 274
   <212> PRT
   <213> RESISTANCE GENE
<400> 406
   Met Asn Ile Lys Ala Leu Leu Leu Ile Thr Ser Ala Ile Phe Ile Ser
     1               5                  10                  15
   Ala Cys Ser Pro Tyr Ile Val Ser Ala Asn Pro Asn His Ser Ala Ser
                20                  25                  30
   Lys Ser Asp Glu Lys Ala Glu Lys Ile Lys Asn Leu Phe Asn Glu Ala
            35                  40                  45
   His Thr Thr Gly Val Leu Val Ile Gln Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 407
   <211> 274
   <212> PRT
   <213> RESISTANCE GENE
<400> 407
   Met Asn Ile Lys Ala Leu Leu Leu Ile Thr Ser Thr Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Glu Lys Ala Glu Lys Ile Lys Asn Leu Phe Asn Glu Ala
            35                  40                  45
   His Thr Thr Gly Val Leu Val Ile Gln Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Ser Leu Glu His His Lys Ala
                    85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 408
   <211> 274
   <212> PRT
   <213> RESISTANCE GENE
<400> 408
   Met Asn Ile Gln Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro His Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 409
   <211> 274
   <212> PRT
   <213> RESISTANCE GENE
<400> 409
   Met Asn Ile Lys Ala Leu Phe Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 410
   <211> 280
   <212> PRT
   <213> RESISTANCE GENE
<400> 410
   Met Lys Leu Leu Lys Ile Leu Ser Leu Val Cys Leu Ser Ile Ser Ile
   1 5 10 15
   Gly Ala Cys Ala Glu His Ser Met Ser Arg Ala Lys Thr Ser Thr Ile
   20 25 30
   Pro Gln Val Asn Asn Ser Ile Ile Asp Gln Asn Val Gln Ala Leu Phe
   35 40 45
   Asn Glu Ile Ser Ala Asp Ala Val Phe Val Thr Tyr Asp Gly Gln Asn
   50 55 60
   Ile Lys Lys Tyr Gly Thr His Leu Asp Arg Ala Lys Thr Ala Tyr Ile
   65 70 75 80
   Pro Ala Ser Thr Phe Lys Ile Ala Asn Ala Leu Ile Gly Leu Glu Asn
   85 90 95
   His Lys Ala Thr Ser Thr Glu Ile Phe Lys Trp Asp Gly Lys Pro Arg
   100 105 110
   Phe Phe Lys Ala Trp Asp Lys Asp Phe Thr Leu Gly Glu Ala Met Gln
   115 120 125
   Ala Ser Thr Val Pro Val Tyr Gln Glu Leu Ala Arg Arg Ile Gly Pro
   130 135 140
   Ser Leu Met Gln Ser Glu Leu Gln Arg Ile Gly Tyr Gly Asn Met Gln
   145 150 155 160
   Met Gly Thr Glu Val Asp Gln Phe Trp Leu Lys Gly Pro Leu Thr Ile
   165 170 175
   Thr Pro Ile Gln Glu Val Lys Phe Val Tyr Asp Leu Ala Gln Gly Gln
   180 185 190
   Leu Pro Phe Lys Pro Glu Val Gln Gln Gln Val Lys Glu Met Leu Tyr
   195 200 205
   Val Glu Arg Arg Gly Glu Asn Arg Leu Tyr Ala Lys Ser Gly Trp Gly
   210 215 220
   Met Ala Val Asp Pro Gln Val Gly Trp Tyr Val Gly Phe Val Glu Lys
   225 230 235 240
   Ala Asp Gly Gln Val Val Ala Phe Ala Leu Asn Met Gln Met Lys Ala
   245 250 255
   Gly Asp Asp Ile Ala Leu Arg Lys Gln Leu Ser Leu Asp Val Leu Asp
   260 265 270
   Lys Leu Gly Val Phe His Tyr Leu
   275 280
<210> 411
   <211> 274
   <212> PRT
   <213> RESISTANCE GENE
<400> 411
   Met Asn Ile Lys Ala Leu Leu Leu Ile Thr Ser Thr Ile Phe Ile Ser
   1 5 10 15
   Ala Cys Ser Pro Tyr Ile Val Thr Ala Asn Pro Asn His Ser Thr Ser
   20 25 30
   Lys Ser Asp Glu Lys Ala Glu Lys Ile Lys Asn Leu Phe Asn Glu Ala
   35 40 45
   His Thr Thr Gly Val Leu Val Ile Gln Gln Gly Gln Thr Gln Gln Ser
   50 55 60
   Tyr Gly Asn Asp Leu Ala Arg Ala Ser Thr Glu Tyr Val Pro Ala Ser
   65 70 75 80
   Thr Phe Lys Met Leu Asn Ala Leu Ile Gly Leu Glu His His Lys Ala
   85 90 95
   Thr Thr Thr Glu Ile Phe Lys Trp Asp Gly Gln Lys Arg Leu Phe Pro
   100 105 110
   Glu Trp Glu Lys Asp Met Thr Leu Gly Asp Ala Met Lys Ala Ser Ala
   115 120 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Leu Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 412
   <211> 274
   <212> PRT
   <213> RESISTANCE GENE
<400> 412
   Met Asn Ile Lys Thr Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Glu Lys Ala Glu Lys Ile Lys Asn Leu Phe Asn Glu Val
            35                  40                  45
   His Thr Thr Gly Val Leu Val Ile Arg Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Met Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 413
   <211> 280
   <212> PRT
   <213> RESISTANCE GENE
<400> 413
   Met Lys Leu Leu Lys Ile Leu Ser Leu Val Cys Leu Ser Ile Ser Ile
   1               5                   10                  15
   Gly Ala Cys Ala Glu His Ser Met Ser Arg Ala Lys Thr Ser Thr Ile
               20                  25                  30
   Pro Gln Val Asn Asn Ser Ile Ile Asp Gln Asn Val Gln Ala Leu Phe
           35                  40                  45
   Asn Glu Ile Ser Gly Asp Ala Val Phe Val Thr Tyr Asp Gly Gln Asn
       50                  55                  60
   Ile Lys Lys Tyr Gly Thr His Leu Asp Arg Ala Lys Thr Ala Tyr Ile
   65                  70                  75                  80
   Pro Ala Ser Thr Phe Lys Ile Ala Asn Ala Leu Ile Gly Leu Glu Asn
                   85                  90                  95
   His Lys Ala Thr Ser Thr Glu Ile Phe Lys Trp Asp Gly Lys Pro Arg
               100                 105                 110
   Phe Phe Lys Ala Trp Asp Lys Asp Phe Thr Leu Gly Glu Ala Met Gln
           115                 120                 125
   Ala Ser Thr Val Pro Val Tyr Gln Glu Leu Ala Arg Arg Ile Gly Pro
       130                 135                 140
   Ser Leu Met Gln Ser Glu Leu Gln Arg Ile Gly Tyr Gly Asn Met Gln
   145                 150                 155                 160
   Ile Gly Thr Glu Val Asp Gln Phe Trp Leu Lys Gly Pro Leu Thr Ile
                   165                 170                 175
   Thr Pro Ile Gln Glu Val Lys Phe Val Tyr Asp Leu Ala Gln Gly Gln
               180                 185                 190
   Leu Pro Phe Lys Pro Glu Val Gln Gln Gln Val Lys Glu Met Leu Tyr
           195                 200                 205
   Val Glu Arg Arg Gly Glu Asn Arg Leu Tyr Ala Lys Ser Gly Trp Gly
       210                 215                 220
   Met Ala Val Asp Pro Gln Val Gly Trp Tyr Val Gly Phe Val Glu Lys
   225                 230                 235                 240
   Ala Asp Gly Gln Val Val Ala Phe Ala Leu Asn Met Gln Met Lys Ala
                   245                 250                 255
   Gly Asp Asp Ile Ala Leu Arg Lys Gln Leu Ser Leu Asp Val Leu Asp
               260                 265                 270
   Lys Leu Gly Val Phe His Tyr Leu
           275                 280
<210> 414
   <211> 275
   <212> PRT
   <213> RESISTANCE GENE
<400> 414
   Met Lys Lys Phe Ile Leu Pro Ile Phe Ser Ile Ser Ile Leu Val Ser
   1               5                   10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Thr Tyr
                   100                 105                 110
   Pro Met Trp Glu Lys Asp Met Thr Leu Gly Glu Ala Met Ala Leu Ser
               115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Asp
       210                 215                 220
   Val Thr Pro Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Ser Gly Ser Ile Arg Asn Glu Ile Thr Tyr Lys Ser Leu Glu Asn Leu
               260                 265                 270
   Gly Ile Ile
           275
<210> 415
   <211> 275
   <212> PRT
   <213> RESISTANCE GENE
<400> 415
   Met Ala Ile Arg Ile Phe Ala Ile Leu Phe Ser Ile Phe Ser Leu Ala
   1 5 10 15
   Thr Phe Ala His Ala Gln Glu Gly Thr Leu Glu Arg Ser Asp Trp Arg
   20 25 30
   Lys Phe Phe Ser Glu Phe Gln Ala Lys Gly Thr Ile Val Val Ala Asp
   35 40 45
   Glu Arg Gln Ala Asp Arg Ala Met Leu Val Phe Asp Pro Val Arg Ser
   50 55 60
   Lys Lys Arg Tyr Ser Pro Ala Ser Thr Phe Lys Ile Pro His Thr Leu
   65 70 75 80
   Phe Ala Leu Asp Ala Gly Ala Val Arg Asp Glu Phe Gln Ile Phe Arg
   85 90 95
   Trp Asp Gly Val Asn Arg Gly Phe Ala Gly His Asn Gln Asp Gln Asp
   100 105 110
   Leu Arg Ser Ala Met Arg Asn Ser Thr Val Trp Val Tyr Glu Leu Phe
   115 120 125
   Ala Lys Glu Ile Gly Asp Asp Lys Ala Arg Arg Tyr Leu Lys Lys Ile
   130 135 140
   Asp Tyr Gly Asn Ala Asp Pro Ser Thr Ser Asn Gly Asp Tyr Trp Ile
   145 150 155 160
   Glu Ser Ser Leu Ala Ile Ser Ala Gln Glu Gln Ile Ala Phe Leu Arg
   165 170 175
   Lys Leu Tyr Arg Asn Glu Leu Pro Phe Arg Val Glu His Gln Arg Leu
   180 185 190
   Val Lys Asp Leu Met Ile Val Glu Ala Gly Arg Asn Trp Ile Leu Arg
   195 200 205
   Ala Lys Thr Gly Trp Glu Gly Arg Met Gly Trp Trp Val Gly Trp Val
   210 215 220
   Glu Trp Pro Thr Gly Ser Val Phe Phe Ala Leu Asn Ile Asp Thr Pro
   225 230 235 240
   Asn Arg Met Asp Asp Leu Phe Lys Arg Glu Ala Ile Val Arg Ala Ile
   245 250 255
   Leu Arg Ser Ile Glu Ala Leu Pro Pro Asn Pro Ala Val Asn Ser Asp
   260 265 270
   Ala Ala Arg
   275
<210> 416
   <211> 274
   <212> PRT
   <213> RESISTANCE GENE
<400> 416
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Glu Lys Ala Glu Lys Ile Lys Asn Leu Phe Asn Glu Ala
            35                  40                  45
   His Thr Thr Gly Val Leu Val Ile Gln Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Lys Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 417
   <211> 274
   <212> PRT
   <213> RESISTANCE GENE
<400> 417
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Val Lys Ala Glu Lys Ile Lys Asn Leu Phe Asn Glu Ala
            35                  40                  45
   His Thr Thr Gly Val Leu Val Ile Gln Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Leu Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 418
   <211> 274
   <212> PRT
   <213> RESISTANCE GENE
<400> 418
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Lys Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asn Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 419
   <211> 274
   <212> PRT
   <213> RESISTANCE GENE
<400> 419
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Asp Lys Ala Glu Lys Ile Lys Asn Leu Phe Asn Glu Ala
           35                  40                  45
   His Thr Thr Gly Val Leu Val Ile His Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Glu Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 420
   <211> 274
   <212> PRT
   <213> RESISTANCE GENE
<400> 420
   Met Asn Ile Lys Ala Leu Leu Leu Ile Thr Ser Ala Ile Phe Ile Ser
   1               5                   10                  15
   Ala Cys Ser Pro Tyr Ile Val Thr Thr Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Thr Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 421
   <211> 274
   <212> PRT
   <213> RESISTANCE GENE
<400> 421
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Gln Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 422
   <211> 274
   <212> PRT
   <213> RESISTANCE GENE
<400> 422
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Asp Lys Ala Glu Lys Ile Lys Asn Leu Phe Asn Glu Ala
            35                  40                  45
   His Thr Thr Gly Val Leu Val Ile His Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Glu Lys Arg Leu Phe Pro
               100                 105                 110
   Glu Trp Glu Lys Asn Met Thr Leu Gly Asp Ala Met Lys Ala Ser Ala
           115                 120                 125
   Leu Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 423
   <211> 274
   <212> PRT
   <213> RESISTANCE GENE
<400> 423
   Met Asn Ile Lys Thr Leu Leu Leu Ile Thr Ser Ala Ile Phe Ile Ser
     1               5                  10                  15
   Ala Cys Ser Pro Tyr Ile Val Ser Ala Asn Pro Asn His Ser Ala Ser
                20                  25                  30
   Lys Ser Asp Glu Lys Ala Glu Lys Ile Lys Asn Leu Phe Asn Glu Ala
               35                  40                  45
   His Thr Thr Gly Val Leu Val Ile Gln Gln Gly Gln Thr Gln Gln Ser
           50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Met Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 424
   <211> 274
   <212> PRT
   <213> RESISTANCE GENE
<400> 424
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Asp Lys Ala Glu Lys Ile Lys Asn Leu Phe Asn Glu Ala
            35                  40                  45
   His Thr Thr Gly Val Leu Val Ile His Gln Gly Gln Thr Gln Gln Ser
        50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Glu Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Pro Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 425
   <211> 274
   <212> PRT
   <213> RESISTANCE GENE
<400> 425
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Gly Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 426
   <211> 274
   <212> PRT
   <213> RESISTANCE GENE
<400> 426
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Leu Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 427
   <211> 274
   <212> PRT
   <213> RESISTANCE GENE
<400> 427
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 428
   <211> 274
   <212> PRT
   <213> RESISTANCE GENE
<400> 428
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Thr
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 429
   <211> 269
   <212> PRT
   <213> RESISTANCE GENE
<400> 429
   Met Ser Lys Lys Asn Phe Ile Leu Ile Phe Ile Phe Val Ile Leu Ile
   1               5                   10                  15
   Ser Cys Lys Asn Thr Glu Lys Ile Ser Asn Glu Thr Thr Leu Ile Asp
               20                  25                  30
   Asn Ile Phe Thr Asn Ser Asn Ala Glu Gly Thr Leu Val Ile Tyr Asn
           35                  40                  45
   Leu Asn Asp Asp Lys Tyr Ile Ile His Asn Lys Glu Arg Ala Glu Gln
       50                  55                  60
   Arg Phe Tyr Pro Ala Ser Thr Phe Lys Ile Tyr Asn Ser Leu Ile Gly
   65                  70                  75                  80
   Leu Asn Glu Lys Ala Val Lys Asp Val Asp Glu Val Phe Tyr Lys Tyr
                   85                  90                  95
   Asn Gly Glu Lys Val Phe Leu Glu Ser Trp Ala Lys Asp Ser Asn Leu
               100                 105                 110
   Arg Tyr Ala Ile Lys Asn Ser Gln Val Pro Ala Tyr Lys Glu Leu Ala
           115                 120                 125
   Arg Arg Ile Gly Leu Lys Lys Met Lys Glu Asn Ile Glu Lys Leu Asp
       130                 135                 140
   Phe Gly Asn Lys Ser Ile Gly Asp Ser Val Asp Thr Phe Trp Leu Glu
   145                 150                 155                 160
   Gly Pro Leu Glu Ile Ser Ala Met Glu Gln Val Lys Leu Leu Thr Lys
                   165                 170                 175
   Leu Ala Gln Asn Glu Leu Pro Tyr Pro Ile Glu Ile Gln Lys Ala Val
               180                 185                 190
   Ser Asp Ile Thr Ile Leu Glu Gln Thr Tyr Asn Tyr Thr Leu His Gly
           195                 200                 205
   Lys Thr Gly Leu Ala Asp Ser Lys Asn Met Thr Thr Glu Pro Ile Gly
       210                 215                 220
   Trp Phe Val Gly Trp Leu Glu Glu Asn Asp Asn Ile Tyr Val Phe Ala
   225                 230                 235                 240
   Leu Asn Ile Asp Asn Ile Asn Ser Asp Asp Leu Ala Lys Arg Ile Asn
                   245                 250                 255
   Ile Val Lys Glu Ser Leu Lys Ala Leu Asn Leu Leu Lys
               260                 265
<210> 430
   <211> 269
   <212> PRT
   <213> RESISTANCE GENE
<400> 430
   Met Ser Lys Lys Asn Phe Ile Leu Ile Phe Ile Phe Val Ile Leu Thr
   1               5                   10                  15
   Ser Cys Lys Asn Thr Glu Lys Ile Ser Asn Glu Thr Thr Leu Ile Asp
               20                  25                  30
   Asn Ile Phe Thr Asn Ser Asn Ala Glu Gly Thr Leu Val Ile Tyr Asn
           35                  40                  45
   Leu Asn Asp Asp Lys Tyr Ile Ile His Asn Lys Glu Arg Ala Glu Gln
       50                  55                  60
   Arg Phe Tyr Pro Ala Ser Thr Phe Lys Ile Tyr Asn Ser Leu Ile Gly
   65                  70                  75                  80
   Leu Asn Glu Lys Ala Val Lys Asp Val Asp Glu Val Phe Tyr Lys Tyr
                   85                  90                  95
   Asn Gly Glu Lys Val Phe Leu Glu Ser Trp Ala Lys Asp Ser Asn Leu
               100                 105                 110
   Arg Tyr Ala Ile Lys Asn Ser Gln Val Pro Ala Tyr Lys Glu Leu Ala
           115                 120                 125
   Arg Arg Ile Gly Leu Lys Lys Met Lys Glu Asn Ile Glu Lys Leu Asp
       130                 135                 140
   Phe Gly Asn Lys Ser Ile Gly Asp Ser Val Asp Thr Phe Trp Leu Glu
   145                 150                 155                 160
   Gly Pro Leu Glu Ile Ser Ala Met Glu Gln Ile Lys Leu Leu Thr Lys
                   165                 170                 175
   Leu Ala Gln Asn Glu Leu Pro Tyr Pro Ile Glu Ile Gln Lys Ala Val
               180                 185                 190
   Ser Asp Ile Thr Ile Leu Glu Gln Thr Tyr Asn Tyr Thr Leu His Gly
           195                 200                 205
   Lys Thr Gly Leu Ala Asp Ser Lys Asn Met Thr Thr Glu Pro Ile Gly
       210                 215                 220
   Trp Phe Val Gly Trp Leu Glu Glu Asn Asp Asn Ile Tyr Val Phe Ala
   225                 230                 235                 240
   Leu Asn Ile Asp Asn Ile Asn Ser Asp Asp Leu Ala Lys Arg Ile Asn
                   245                 250                 255
   Ile Val Lys Glu Ser Leu Lys Ala Leu Asn Leu Leu Lys
               260                 265
<210> 431
   <211> 262
   <212> PRT
   <213> RESISTANCE GENE
<400> 431
   Leu Leu Ile Thr Ser Ala Ile Phe Ile Ser Ala Cys Ser Pro Tyr Ile
     1               5                  10                  15
   Val Ser Ala Asn Pro Asn His Ser Ala Ser Lys Ser Asp Asp Lys Ala
               20                  25                  30
   Glu Lys Ile Lys Asn Leu Phe Asn Glu Ala His Thr Thr Gly Val Leu
           35                  40                  45
   Val Ile Gln Gln Gly Gln Thr Gln Gln Ser Tyr Gly Asn Asp Leu Ala
       50                  55                  60
   Arg Ala Ser Thr Glu Tyr Val Pro Ala Ser Thr Phe Lys Met Leu Asn
    65                  70                  75                  80
   Ala Leu Ile Gly Leu Glu His His Lys Ala Thr Thr Thr Glu Val Phe
                   85                  90                  95
   Lys Trp Asp Gly Gln Lys Arg Leu Phe Pro Glu Trp Glu Lys Asn Met
               100                 105                 110
   Thr Leu Gly Asp Ala Met Lys Ala Ser Ala Ile Pro Val Tyr Gln Asp
           115                 120                 125
   Leu Ala Arg Arg Ile Gly Leu Glu Leu Met Ser Asn Glu Val Lys Arg
       130                 135                 140
   Val Gly Tyr Gly Asn Ala Asp Ile Gly Thr Gln Val Asp Asn Phe Trp
   145                 150                 155                 160
   Leu Val Gly Pro Leu Lys Ile Thr Pro Gln Gln Glu Ala Gln Phe Ala
                   165                 170                 175
   Tyr Lys Leu Ala Asn Lys Thr Leu Pro Phe Ser Gln Lys Val Gln Asp
               180                 185                 190
   Glu Val Gln Ser Met Leu Phe Ile Glu Glu Lys Asn Gly Asn Lys Ile
           195                 200                 205
   Tyr Ala Lys Ser Gly Trp Gly Trp Asp Val Asp Pro Gln Val Gly Trp
       210                 215                 220
   Leu Thr Gly Trp Val Val Gln Pro Gln Gly Asn Ile Val Ala Phe Ser
   225                 230                 235                 240
   Leu Asn Leu Glu Met Lys Lys Gly Ile Pro Ser Ser Val Arg Lys Glu
                   245                 250                 255
   Ile Thr Tyr Lys Ser Leu
               260
<210> 432
   <211> 262
   <212> PRT
   <213> RESISTANCE GENE
<400> 432
   Leu Leu Ile Thr Ser Ala Ile Phe Ile Ser Ala Cys Ser Pro Tyr Ile
   1               5                   10                  15
   Val Ser Ala Asn Pro Asn His Ser Ala Ser Lys Ser Asp Glu Lys Ala
               20                  25                  30
   Glu Lys Ile Lys Asn Leu Phe Asn Glu Ala His Thr Thr Gly Val Leu
           35                  40                  45
   Val Ile Gln Gln Gly Gln Thr Gln Gln Ser Tyr Gly Asn Asp Leu Ala
       50                  55                  60
   Arg Ala Ser Thr Glu Tyr Val Pro Ala Ser Thr Phe Lys Met Leu Asn
   65                  70                  75                  80
   Ala Leu Ile Gly Leu Glu His His Lys Ala Thr Thr Thr Glu Val Phe
                   85                  90                  95
   Lys Trp Asp Gly Gln Lys Arg Leu Phe Pro Glu Trp Glu Lys Asn Met
               100                 105                 110
   Thr Leu Gly Asp Ala Met Lys Ala Ser Ala Ile Pro Val Tyr Gln Asp
           115                 120                 125
   Leu Ala Arg Arg Ile Gly Leu Glu Leu Met Ser Asn Glu Val Lys Arg
       130                 135                 140
   Val Gly Tyr Gly Asn Ala Asp Ile Gly Thr Gln Val Asp Asn Phe Trp
   145                 150                 155                 160
   Leu Val Gly Pro Leu Lys Ile Thr Pro Gln Gln Glu Ala Gln Phe Ala
                   165                 170                 175
   Tyr Lys Leu Ala Asn Lys Thr Leu Pro Phe Ser Gln Lys Val Gln Asp
               180                 185                 190
   Glu Val Gln Ser Met Leu Phe Ile Glu Glu Lys Asn Gly Asn Lys Ile
           195                 200                 205
   Tyr Ala Lys Ser Gly Trp Gly Trp Asp Val Asn Pro Gln Val Gly Trp
       210                 215                 220
   Leu Thr Glu Trp Val Val Gln Pro Gln Gly Asn Ile Val Ala Phe Ser
   225                 230                 235                 240
   Leu Asn Leu Glu Met Lys Lys Gly Ile Pro Ser Ser Val Arg Lys Glu
                   245                 250                 255
   Ile Thr Tyr Lys Ser Leu
               260
<210> 433
   <211> 275
   <212> PRT
   <213> RESISTANCE GENE
<400> 433
   Met Thr Val Arg Arg Leu Ser Cys Ala Leu Gly Ala Ala Leu Ser Leu
    1               5                  10                  15
   Ser Ala Leu Gly Gly Gly Pro Val Gln Ala Ala Val Leu Cys Thr Val
               20                  25                  30
   Val Ala Asp Ala Ala Asp Gly Arg Ile Leu Phe Gln Gln Gly Thr Gln
           35                  40                  45
   Gln Ala Cys Ala Glu Arg Tyr Thr Pro Ala Ser Thr Phe Lys Leu Ala
       50                  55                  60
   Ile Ala Leu Met Gly Ala Asp Ala Gly Ile Leu Gln Gly Pro His Glu
   65                  70                  75                  80
   Pro Val Trp Asn Tyr Gln Pro Ala Tyr Pro Asp Trp Gly Gly Asp Ala
                   85                  90                  95
   Trp Arg Gln Pro Thr Asp Pro Ala Arg Trp Ile Lys Tyr Ser Val Val
               100                 105                 110
   Trp Tyr Ser Gln Leu Thr Ala Lys Ala Leu Gly Gln Asp Arg Phe Gln
           115                 120                 125
   Arg Tyr Thr Ser Ala Phe Gly Tyr Gly Asn Ala Asp Val Ser Gly Glu
       130                 135                 140
   Pro Gly Lys His Asn Gly Thr Asp Gly Ala Trp Ile Ile Ser Ser Leu
   145                 150                 155                 160
   Arg Ile Ser Pro Leu Glu Gln Leu Ala Phe Leu Arg Lys Leu Val Asn
                   165                 170                 175
   Arg Gln Leu Pro Val Lys Ala Ala Ala Tyr Glu Leu Ala Glu Asn Leu
               180                 185                 190
   Phe Glu Ala Gly Gln Ala Asp Gly Trp Arg Leu Tyr Gly Lys Thr Gly
           195                 200                 205
   Thr Gly Ser Pro Gly Ser Asn Gly Val Tyr Thr Ala Ala Asn Ala Tyr
       210                 215                 220
   Gly Trp Phe Val Gly Trp Ala Arg Lys Asp Gly Arg Gln Leu Val Tyr
   225                 230                 235                 240
   Ala Arg Leu Leu Gln Asp Glu Arg Ala Thr Arg Pro Asn Ala Gly Leu
                   245                 250                 255
   Arg Ala Arg Asp Glu Leu Val Arg Asp Trp Pro Ala Met Ala Gly Ala
               260                 265                 270
   Trp Arg Pro
           275
<210> 434
   <211> 274
   <212> PRT
   <213> RESISTANCE GENE
<400> 434
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Val Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Glu Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 435
   <211> 266
   <212> PRT
   <213> RESISTANCE GENE
<400> 435
   Met Lys Thr Phe Ala Ala Tyr Val Ile Ile Ala Cys Leu Ser Ser Thr
    1               5                  10                  15
   Ala Leu Ala Gly Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asp Leu Ala Arg Ala Ser Lys Glu Tyr Leu
       50                  55                  60
   Pro Ala Ser Thr Phe Lys Ile Pro Ser Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Thr Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Asp Gln Leu Arg
   145                 150                 155                 160
   Ile Ser Ala Val Asn Gln Val Glu Phe Leu Glu Ser Leu Tyr Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Glu Thr Glu Val Tyr Phe Phe Ala Phe Asn Met Asp
   225                 230                 235                 240
   Ile Asp Asn Glu Ser Lys Leu Pro Leu Arg Lys Ser Ile Pro Thr Lys
                   245                 250                 255
   Ile Met Glu Ser Glu Gly Ile Ile Gly Gly
               260                 265
<210> 436
   <211> 274
   <212> PRT
   <213> RESISTANCE GENE
<400> 436
   Met Asn Ile Lys Thr Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 437
   <211> 274
   <212> PRT
   <213> RESISTANCE GENE
<400> 437
   Met Asn Ile Lys Ala Leu Leu Leu Ile Thr Ser Ala Ile Phe Ile Ser
    1              5                   10                  15
   Ala Cys Ser Pro Tyr Ile Val Thr Thr Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Thr Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 438
   <211> 274
   <212> PRT
   <213> RESISTANCE GENE
<400> 438
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Val Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Gln Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 439
   <211> 274
   <212> PRT
   <213> RESISTANCE GENE
<400> 439
   Met Asn Ile Lys Thr Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Val
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Met Thr Tyr Lys Ser Leu Glu Gln Leu Gly
               260                 265                 270
   Ile Leu
<210> 440
   <211> 273
   <212> PRT
   <213> RESISTANCE GENE
<400> 440
   Met Asn Lys Tyr Phe Thr Cys Tyr Val Val Ala Ser Leu Phe Phe Ser
    1               5                  10                  15
   Gly Cys Thr Val Gln His Asn Leu Ile Asn Glu Thr Gln Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Thr
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Glu Arg Ile Asp Phe Gly Asn Ala Glu Ile Gly Gln Gln
   145                 150                 155                 160
   Val Asp Asn Phe Trp Leu Ile Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Asn
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 441
   <211> 274
   <212> PRT
   <213> RESISTANCE GENE
<400> 441
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
    65                 70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Ala Met Asp Ile
       210                 215                 220
   Lys Pro Gln Val Gly Trp Leu Thr Gly Trp Val Glu Gln Pro Asp Gly
   225                 230                 235                 240
   Lys Ile Val Ala Phe Ala Leu Asn Met Glu Met Arg Ser Glu Met Pro
                   245                 250                 255
   Ala Ser Ile Arg Asn Glu Leu Leu Met Lys Ser Leu Lys Gln Leu Asn
               260                 265                 270
   Ile Ile
<210> 442
   <211> 266
   <212> PRT
   <213> RESISTANCE GENE
<400> 442
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
    65                 70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Leu Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 443
   <211> 275
   <212> PRT
   <213> RESISTANCE GENE
<400> 443
   Met Ala Ile Arg Ile Phe Ala Ile Leu Phe Ser Ile Phe Ser Leu Ala
    1               5                  10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
       50                  55                  60
   Lys Lys Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe Gln Ile Phe Arg
                   85                  90                  95
   Trp Asp Gly Val Asn Arg Gly Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Glu Ile Gly Asp Asp Lys Ala Arg Arg Tyr Leu Lys Lys Ile
       130                 135                 140
   Asp Tyr Gly Asp Ala Asp Pro Ser Thr Ser Asn Gly Asp Tyr Trp Ile
   145                 150                 155                 160
   Glu Gly Ser Leu Ala Ile Ser Ala Gln Glu Gln Ile Ala Phe Leu Arg
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Ser Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 444
   <211> 274
   <212> PRT
   <213> RESISTANCE GENE
<400> 444
   Met Asn Ile Lys Ala Leu Leu Leu Ile Thr Ser Ala Ile Phe Ile Ser
    1               5                   10                  15
   Ala Cys Ser Pro Tyr Ile Val Thr Ala Asn Pro Asn His Ser Ala Ser
               20                  25                  30
   Lys Ser Asp Lys Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Ser Thr Thr Glu Val Phe Lys Trp Asn Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Lys Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 445
   <211> 274
   <212> PRT
   <213> RESISTANCE GENE
<400> 445
   Met Asn Ile Lys Ala Leu Leu Leu Ile Thr Ser Ala Ile Phe Ile Ser
    1               5                   10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
                   115                     120                     125
   Ile Pro Val Tyr Gln Asp Leu Ala Arg Arg Ile Gly Leu Glu Leu Met
       130                     135                     140
   Ser Asn Glu Val Lys His Val Gly Tyr Gly Asn Ala Asp Ile Gly Thr
   145                 150                     155                 160
   Gln Val Asp Asn Phe Trp Leu Val Gly Pro Leu Lys Ile Thr Pro Gln
                   165                     170                 175
   Gln Glu Ala Gln Phe Ala Tyr Lys Leu Ala Asn Lys Thr Leu Pro Phe
               180                     185                 190
   Ser Pro Lys Val Gln Asp Glu Val Gln Ser Met Leu Phe Ile Glu Glu
           195                     200                     205
   Lys Asn Gly Asn Lys Ile Tyr Ala Lys Ser Gly Trp Gly Trp Asp Val
       210                     215                     220
   Asn Pro Gln Val Gly Trp Leu Thr Gly Trp Val Val Gln Pro Gln Gly
   225                 230                     235                 240
   Asn Ile Val Ala Phe Ser Leu Asn Leu Glu Met Lys Lys Gly Ile Pro
                   245                     250                 255
   Ser Ser Val Arg Lys Glu Ile Thr Tyr Lys Ser Leu Glu Gln Leu Gly
               260                     265                     270
   Ile Leu
<210> 446
   <211> 274
   <212> PRT
   <213> RESISTANCE GENE
<400> 446
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys His Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 447
   <211> 275
   <212> PRT
   <213> RESISTANCE GENE
<400> 447
   Met Lys Lys Phe Ile Leu Pro Ile Leu Ser Ile Ser Thr Leu Leu Ser
    1               5                  10                  15
   Val Ser Ala Cys Ser Ser Ile Gln Thr Lys Phe Glu Asp Thr Phe His
               20                  25                  30
   Thr Ser Asn Gln Gln His Glu Lys Ala Ile Lys Ser Tyr Phe Asp Glu
           35                  40                  45
   Ala Gln Thr Gln Gly Val Ile Ile Ile Lys Lys Gly Lys Asn Ile Ser
       50                  55                  60
   Thr Tyr Gly Asn Asn Leu Thr Arg Ala His Thr Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Thr Glu Ile Phe Lys Trp Asp Gly Lys Lys Arg Ser Tyr
               100                 105                 110
   Pro Met Trp Glu Lys Asp Met Thr Leu Gly Asp Ala Met Ala Leu Ser
           115                 120                 125
   Ala Val Pro Val Tyr Gln Glu Leu Ala Arg Arg Thr Gly Leu Asp Leu
       130                 135                 140
   Met Gln Lys Glu Val Lys Arg Val Gly Phe Gly Asn Met Asn Ile Gly
   145                 150                 155                 160
   Thr Gln Val Asp Asn Phe Trp Leu Val Gly Pro Leu Lys Ile Thr Pro
                   165                 170                 175
   Ile Gln Glu Val Asn Phe Ala Asp Asp Phe Ala Asn Asn Arg Leu Pro
               180                 185                 190
   Phe Lys Leu Glu Thr Gln Glu Glu Val Lys Lys Met Leu Leu Ile Lys
           195                 200                 205
   Glu Phe Asn Gly Ser Lys Ile Tyr Ala Lys Ser Gly Trp Gly Met Asp
       210                 215                 220
   Val Thr Pro Gln Val Gly Trp Leu Thr Gly Trp Val Glu Lys Ser Asn
   225                 230                 235                 240
   Gly Glu Lys Val Ala Phe Ser Leu Asn Ile Glu Met Lys Gln Gly Met
                   245                 250                 255
   Pro Gly Ser Ile Arg Asn Glu Ile Thr Tyr Lys Ser Leu Glu Asn Leu
               260                 265                 270
   Gly Ile Ile
           275
<210> 448
   <211> 274
   <212> PRT
   <213> RESISTANCE GENE
<400> 448
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Glu Val Gln Asp Glu Val Gln Ser Ile Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 449
   <211> 275
   <212> PRT
   <213> RESISTANCE GENE
<400> 449
   Met Lys Lys Phe Ile Leu Pro Ile Phe Ser Ile Ser Ile Leu Val Ser
    1               5                  10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Thr Tyr
               100                 105                 110
   Pro Met Trp Glu Lys Asp Met Thr Leu Gly Glu Ala Met Ala Leu Ser
           115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Gly
       210                 215                 220
   Val Thr Ser Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Ser Gly Ser Ile Arg Asn Glu Ile Thr Tyr Lys Ser Leu Glu Asn Leu
               260                 265                 270
   Gly Ile Ile
           275
<210> 450
   <211> 265
   <212> PRT
   <213> RESISTANCE GENE
<400> 450
   Met Arg Val Leu Ala Leu Ser Ala Val Phe Leu Val Ala Ser Ile Ile
   1               5                   10                  15
   Gly Met Pro Ala Val Ala Lys Glu Trp Gln Glu Asn Lys Ser Trp Asn
               20                  25                  30
   Ala His Phe Thr Glu His Lys Ser Gln Gly Val Val Val Leu Trp Asn
           35                  40                  45
   Glu Asn Lys Gln Gln Gly Phe Thr Asn Asn Leu Lys Arg Ala Asn Gln
       50                  55                  60
   Ala Phe Leu Pro Ala Ser Thr Phe Lys Ile Pro Asn Ser Leu Ile Ala
   65                  70                  75                  80
   Leu Asp Leu Gly Val Val Lys Asp Glu His Gln Val Phe Lys Trp Asp
                   85                  90                  95
   Gly Gln Thr Arg Asp Ile Ala Thr Trp Asn Arg Asp His Asn Leu Ile
               100                 105                 110
   Thr Ala Met Lys Tyr Ser Val Val Pro Val Tyr Gln Glu Phe Ala Arg
           115                 120                 125
   Gln Ile Gly Glu Ala Arg Met Ser Lys Met Leu His Ala Phe Asp Tyr
       130                 135                 140
   Gly Asn Glu Asp Ile Ser Gly Asn Val Asp Ser Phe Trp Leu Asp Gly
   145                 150                 155                 160
   Gly Ile Arg Ile Ser Ala Thr Glu Gln Ile Ser Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Gly Asp Tyr Ile Ile Arg Ala Lys
           195                 200                 205
   Thr Gly Tyr Ser Ala Arg Ile Glu Pro Lys Ile Gly Trp Trp Val Gly
       210                 215                 220
   Trp Val Glu Leu Asp Asp Asn Val Trp Phe Phe Ala Met Asn Met Asp
   225                 230                 235                 240
   Met Pro Thr Ser Asp Gly Leu Gly Leu Arg Gln Ala Ile Thr Lys Glu
                   245                 250                 255
   Val Leu Lys Gln Glu Lys Ile Ile Pro
               260                 265
<210> 451
   <211> 274
   <212> PRT
   <213> RESISTANCE GENE
<400> 451
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                       85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Val Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Leu Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 452
   <211> 274
   <212> PRT
   <213> RESISTANCE GENE
<400> 452
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Val Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Leu Asp Val
       210                 215                 220
   Asn Leu Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 453
   <211> 274
   <212> PRT
   <213> RESISTANCE GENE
<400> 453
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Lys Leu Gly
               260                 265                 270
   Ile Leu
<210> 454
   <211> 274
   <212> PRT
   <213> RESISTANCE GENE
<400> 454
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Arg Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65 70 75 80
   Thr Phe Lys Met Leu Asn Ala Leu Ile Gly Leu Glu His His Lys Ala
   85 90 95
   Thr Thr Thr Glu Val Phe Lys Trp Asp Gly Lys Lys Arg Leu Phe Pro
   100 105 110
   Glu Trp Glu Lys Asp Met Thr Leu Gly Asp Ala Met Lys Ala Ser Ala
   115 120 125
   Ile Pro Val Tyr Gln Asp Leu Ala Arg Arg Ile Gly Leu Glu Leu Met
   130 135 140
   Ser Lys Glu Val Lys Arg Val Gly Tyr Gly Asn Ala Asp Ile Gly Thr
   145 150 155 160
   Gln Val Asp Asn Phe Trp Val Val Gly Pro Leu Lys Ile Thr Pro Gln
   165 170 175
   Gln Glu Ala Gln Phe Ala Tyr Lys Leu Ala Asn Lys Thr Leu Pro Phe
   180 185 190
   Ser Gln Lys Val Gln Asp Glu Val Gln Ser Met Leu Phe Ile Glu Glu
   195 200 205
   Lys Asn Gly Asn Lys Ile Tyr Ala Lys Ser Gly Trp Gly Trp Asp Val
   210 215 220
   Asn Pro Gln Val Gly Trp Leu Thr Gly Trp Val Val Gln Pro Gln Gly
   225 230 235 240
   Asn Ile Val Ala Phe Ser Leu Asn Leu Glu Met Lys Lys Gly Ile Pro
   245 250 255
   Ser Ser Val Arg Lys Glu Ile Thr Tyr Lys Ser Leu Glu Gln Leu Gly
   260 265 270
   Ile Leu
<210> 455
   <211> 274
   <212> PRT
   <213> RESISTANCE GENE
<400> 455
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Ser Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 456
   <211> 274
   <212> PRT
   <213> RESISTANCE GENE
<400> 456
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Val Lys Ala Glu Lys Ile Lys Asn Leu Phe Asn Glu Ala
            35                  40                  45
   His Thr Thr Gly Val Leu Val Ile Gln Gln Gly Gln Thr Gln Gln Ser
        50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                    85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Gly Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 457
   <211> 274
   **<212> PRT**
   **<213> RESISTANCE GENE**
**<400> 457**
   Met Asn Ile Lys Ala Leu Leu Leu Ile Thr Ser Ala Ile Phe Ile Ser
     1               5                  10                  15
   Ala Cys Ser Pro Tyr Ile Val Thr Ala Asn Pro Asn His Ser Ala Ser
                20                  25                  30
   Lys Ser Asp Val Lys Ala Glu Lys Ile Lys Asn Leu Phe Asn Glu Ala
                   35                  40                  45
   His Thr Thr Gly Val Leu Val Ile Gln Gln Gly Gln Thr Gln Gln Ser
               50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Ser Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Leu Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 458
   <211> 280
   <212> PRT
   <213> RESISTANCE GENE
<400> 458
   Met Lys Leu Leu Lys Ile Leu Ser Leu Val Cys Leu Ser Ile Ser Ile
    1               5                  10                  15
   Gly Ala Cys Ala Glu His Ser Met Ser Arg Ala Lys Thr Ser Thr Ile
               20                  25                  30
   Pro Gln Val Asn Asn Ser Ile Ile Asp Gln Asn Val Gln Ala Leu Phe
           35                  40                  45
   Asn Glu Ile Ser Ala Asp Ala Val Phe Val Thr Tyr Asp Gly Gln Asn
       50                  55                  60
   Ile Lys Lys Tyr Gly Thr His Leu Asp Arg Ala Lys Thr Ala Tyr Ile
   65                  70                  75                  80
   Pro Ala Ser Thr Phe Lys Ile Ala Asn Ala Leu Ile Gly Leu Glu Asn
                   85                  90                  95
   His Lys Ala Thr Ser Thr Glu Ile Phe Lys Trp Asp Gly Lys Pro Arg
               100                 105                 110
   Phe Leu Lys Ala Trp Asp Lys Asp Phe Thr Leu Gly Glu Ala Met Gln
           115                 120                 125
   Ala Ser Thr Val Pro Val Tyr Gln Glu Leu Ala Arg Arg Ile Gly Pro
       130                 135                 140
   Ser Leu Met Gln Ser Glu Leu Gln Arg Ile Gly Tyr Gly Asn Met Gln
   145                 150                 155                 160
   Ile Gly Thr Glu Val Asp Gln Phe Trp Leu Lys Gly Pro Leu Thr Ile
                   165                 170                 175
   Thr Pro Ile Gln Glu Val Lys Phe Val Tyr Asp Leu Ala Gln Gly Gln
               180                 185                 190
   Leu Pro Phe Lys Pro Glu Val Gln Gln Gln Val Lys Glu Met Leu Tyr
           195                 200                 205
   Val Glu Arg Arg Gly Glu Asn Arg Leu Tyr Ala Lys Ser Gly Trp Gly
       210                 215                 220
   Met Ala Val Asp Pro Gln Val Gly Trp Tyr Val Gly Phe Val Glu Lys
   225                 230                 235                 240
   Ala Asp Gly Gln Val Val Ala Phe Ala Leu Asn Met Gln Met Lys Ala
                   245                 250                 255
   Gly Asp Asp Ile Ala Leu Arg Lys Gln Leu Ser Leu Asp Val Leu Asp
               260                 265                 270
   Lys Leu Gly Val Phe His Tyr Leu
           275                 280
<210> 459
   <211> 273
   <212> PRT
   <213> RESISTANCE GENE
<400> 459
   Met Asn Lys Tyr Phe Thr Cys Tyr Val Val Ala Ser Leu Phe Leu Ser
   1 5 10 15
   Gly Cys Thr Val Gln His Asn Leu Ile Asn Glu Thr Pro Ser Gln Ile
   20 25 30
   Val Gln Gly His Asn Gln Val Ile His Gln Tyr Phe Asp Glu Lys Asn
   35 40 45
   Thr Ser Gly Val Leu Val Ile Gln Thr Asp Lys Lys Ile Asn Leu Tyr
   50 55 60
   Gly Asn Ala Leu Ser Arg Ala Asn Thr Glu Tyr Val Pro Ala Ser Thr
   65 70 75 80
   Phe Lys Met Leu Asn Ala Leu Ile Gly Leu Glu Asn Gln Lys Thr Asp
   85 90 95
   Ile Asn Glu Ile Phe Lys Trp Lys Gly Glu Lys Arg Ser Phe Thr Ala
   100 105 110
   Trp Glu Lys Asp Met Thr Leu Gly Glu Ala Met Lys Leu Ser Ala Val
   115 120 125
   Pro Val Tyr Gln Glu Leu Ala Arg Arg Ile Gly Leu Asp Leu Met Gln
   130 135 140
   Lys Glu Val Lys Arg Ile Gly Phe Gly Asn Ala Glu Ile Gly Gln Gln
   145 150 155 160
   Val Asp Asn Phe Trp Leu Val Gly Pro Leu Lys Val Thr Pro Ile Gln
   165 170 175
   Glu Val Glu Phe Val Ser Gln Leu Ala His Thr Gln Leu Pro Phe Ser
   180 185 190
   Glu Lys Val Gln Ala Asn Val Lys Asn Met Leu Leu Leu Glu Glu Ser
   195 200 205
   Asn Gly Tyr Lys Ile Phe Gly Lys Thr Gly Trp Ala Met Asp Val Lys
   210 215 220
   Pro Gln Val Gly Trp Leu Thr Gly Trp Val Glu Gln Pro Asp Gly Lys
   225 230 235 240
   Ile Val Ala Phe Ala Leu Asn Met Glu Met Arg Ser Glu Met Pro Ala
   245 250 255
   Ser Ile Arg Asn Glu Leu Leu Met Lys Ser Leu Lys Gln Leu Asn Ile
   260 265 270
   Ile
<210> 460
   <211> 273
   <212> PRT
   <213> RESISTANCE GENE
<400> 460
   Met Asn Lys Tyr Phe Thr Cys Tyr Val Val Ala Ser Leu Phe Leu Ser
   1 5 10 15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Ala Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 461
   <211> 273
   <212> PRT
   <213> RESISTANCE GENE
<400> 461
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Met Met Lys Ser Leu Lys Gln Leu Asn Ile
               260                 265                 270
   Ile
<210> 462
   <211> 273
   <212> PRT
   <213> RESISTANCE GENE
<400> 462
   Met Asn Lys Tyr Phe Thr Cys Tyr Val Val Ala Ser Leu Phe Leu Ser
   1 5 10 15
   Gly Cys Thr Val Gln His Asn Leu Ile Asn Glu Thr Pro Ser Gln Ile
   20 25 30
   Val Gln Gly His Asn Gln Val Ile His Gln Tyr Phe Asp Glu Lys Asn
   35 40 45
   Thr Ser Gly Val Leu Val Ile Gln Thr Asp Lys Lys Ile Asn Leu Tyr
   50 55 60
   Gly Asn Ala Leu Ser Arg Ala Asn Thr Glu Tyr Val Pro Ala Ser Thr
   65 70 75 80
   Phe Lys Met Leu Asn Ala Leu Ile Gly Leu Glu Asn Gln Lys Thr Asp
   85 90 95
   Ile Asn Glu Ile Phe Lys Trp Lys Gly Glu Lys Arg Ser Phe Thr Ala
   100 105 110
   Trp Glu Lys Asp Met Thr Leu Gly Glu Ala Met Lys Leu Ser Ala Val
   115 120 125
   Pro Val Tyr Gln Glu Leu Ala Arg Arg Ile Gly Leu Asp Leu Met Gln
   130 135 140
   Lys Glu Val Lys Arg Ile Gly Phe Gly Asn Ala Glu Ile Gly Gln Gln
   145 150 155 160
   Val Asp Asn Phe Trp Leu Val Gly Pro Leu Lys Val Thr Pro Ile Gln
   165 170 175
   Glu Val Glu Phe Val Ser Gln Leu Ala His Thr Gln Leu Pro Phe Ser
   180 185 190
   Glu Lys Val Gln Ala Asn Val Lys Asn Met Leu Leu Leu Glu Glu Ser
   195 200 205
   Asn Gly Tyr Lys Ile Phe Gly Lys Thr Gly Trp Ala Met Asp Ile Lys
   210 215 220
   Pro Gln Val Gly Trp Leu Ala Gly Trp Val Glu Gln Pro Asp Gly Lys
   225 230 235 240
   Ile Val Ala Phe Ala Leu Asn Met Glu Met Arg Ser Glu Met Pro Ala
   245 250 255
   Ser Ile Arg Asn Glu Leu Leu Met Lys Ser Leu Lys Gln Leu Asn Ile
   260 265 270
   Ile
<210> 463
   <211> 273
   <212> PRT
   <213> RESISTANCE GENE
<400> 463
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Arg Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
    65                 70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 464
   <211> 273
   <212> PRT
   <213> RESISTANCE GENE
<400> 464
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Lys Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 465
   <211> 273
   <212> PRT
   <213> RESISTANCE GENE
<400> 465
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Thr Arg Asn Glu Leu Leu Met Lys Ser Leu Lys Gln Leu Asn Ile
               260                 265                 270
   Ile
<210> 466
   <211> 275
   <212> PRT
   <213> RESISTANCE GENE
<400> 466
   Met Lys Lys Phe Ile Leu Pro Ile Phe Ser Ile Ser Ile Leu Leu Ser
    1               5                  10                  15
   Leu Ser Ala Cys Ser Ser Ile Gln Thr Lys Phe Glu Asp Thr Phe His
               20                  25                  30
   Ile Ser Asn Gln Lys His Glu Lys Ala Ile Lys Ser Tyr Phe Asp Glu
           35                  40                  45
   Ala Gln Thr Gln Gly Val Ile Ile Ile Lys Glu Gly Lys Asn Ile Ser
       50                  55                  60
   Ser Tyr Gly Asn Asn Leu Val Arg Ala His Thr Glu Tyr Val Pro Ala
   65                  70                  75                  80
   Ser Thr Phe Lys Met Leu Asn Ala Leu Ile Gly Leu Glu Asn His Lys
                   85                  90                  95
   Ala Thr Thr Asn Glu Ile Phe Lys Trp Asp Gly Lys Lys Arg Ser Tyr
               100                 105                 110
   Pro Met Trp Glu Lys Asp Met Thr Leu Gly Glu Ala Met Ala Leu Ser
           115                 120                 125
   Ala Val Pro Val Tyr Gln Asp Leu Ala Arg Arg Ile Gly Leu Asn Leu
       130                 135                 140
   Met Gln Lys Glu Val Lys Arg Val Gly Phe Gly Asn Met Asn Ile Gly
   145                 150                 155                 160
   Thr Gln Val Asp Asn Phe Trp Leu Ile Gly Pro Leu Lys Ile Thr Pro
                   165                 170                 175
   Ile Gln Glu Val Asn Phe Ala Asp Asp Leu Ala Asn Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Asp
       210                 215                 220
   Val Ser Pro Gln Val Gly Trp Leu Thr Gly Trp Val Glu Lys Ser Asn
   225                 230                 235                 240
   Gly Glu Lys Val Ser Phe Ser Leu Asn Ile Glu Met Lys Gln Gly Met
                   245                 250                 255
   Ser Gly Ser Ile Arg Asn Glu Ile Thr Tyr Lys Ser Leu Glu Asn Leu
               260                 265                 270
   Gly Ile Ile
           275
<210> 467
   <211> 274
   <212> PRT
   <213> RESISTANCE GENE
<400> 467
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Ala Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 468
   <211> 274
   <212> PRT
   <213> RESISTANCE GENE
<400> 468
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Ile Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 469
   <211> 273
   <212> PRT
   <213> RESISTANCE GENE
<400> 469
   Met Lys Ile Leu Ile Phe Leu Pro Leu Leu Ser Cys Leu Gly Leu Thr
    1               5                  10                  15
   Ala Cys Ser Leu Pro Val Ser Ser Leu Pro Ser Gln Ser Ile Ser Thr
               20                  25                  30
   Gln Ala Ile Ala Ser Leu Phe Asp Gln Ala Gln Ser Ser Gly Val Leu
           35                  40                  45
   Val Ile Gln Arg Asp Gln Gln Val Gln Val Tyr Gly Asn Asp Leu Asn
       50                  55                  60
   Arg Ala Asn Thr Glu Tyr Val Pro Ala Ser Thr Phe Lys Met Leu Asn
   65                  70                  75                  80
   Ala Leu Ile Gly Leu Gln His Gly Lys Ala Thr Thr Asn Glu Ile Phe
                   85                  90                  95
   Lys Trp Asp Gly Lys Lys Arg Ser Phe Thr Ala Trp Glu Lys Asp Met
               100                 105                 110
   Thr Leu Gly Gln Ala Met Gln Ala Ser Ala Val Pro Val Tyr Gln Glu
           115                 120                 125
   Leu Ala Arg Arg Ile Gly Leu Glu Leu Met Gln Gln Glu Val Gln Arg
       130                 135                 140
   Ile Gln Phe Gly Asn Gln Gln Ile Gly Gln Gln Val Asp Asn Phe Trp
   145                 150                 155                 160
   Leu Val Gly Pro Leu Lys Val Thr Pro Lys Gln Glu Val Gln Phe Val
                   165                 170                 175
   Ser Ala Leu Ala Arg Glu Gln Leu Ala Phe Asp Pro Gln Val Gln Gln
               180                 185                 190
   Gln Val Lys Ala Met Leu Phe Leu Gln Glu Arg Lys Ala Tyr Arg Leu
           195                 200                 205
   Tyr Val Lys Ser Gly Trp Gly Met Asp Val Glu Pro Gln Val Gly Trp
       210                 215                 220
   Leu Thr Gly Trp Val Glu Thr Pro Gln Ala Glu Ile Val Ala Phe Ser
   225                 230                 235                 240
   Leu Asn Met Gln Met Gln Asn Gly Ile Asp Pro Ala Ile Arg Leu Glu
                   245                 250                 255
   Ile Leu Gln Gln Ala Leu Ala Glu Leu Gly Leu Tyr Pro Lys Ala Glu
               260                 265                 270
   Gly
<210> 470
   <211> 262
   <212> PRT
   <213> RESISTANCE GENE
<400> 470
   Met His Lys His Met Ser Lys Leu Phe Ile Ala Phe Leu Ala Phe Leu
    1               5                  10                  15
   Leu Ser Val Pro Ala Ala Ala Glu Asp Gln Thr Leu Ala Glu Leu Phe
               20                  25                  30
   Ala Gln Gln Gly Ile Asp Gly Thr Ile Val Ile Ser Ser Leu His Asn
           35                  40                  45
   Gly Lys Thr Phe Ile His Asn Asp Pro Arg Ala Lys Gln Arg Phe Ser
       50                  55                  60
   Thr Ala Ser Thr Phe Lys Ile Leu Asn Thr Leu Ile Ser Leu Glu Glu
   65                  70                  75                  80
   Lys Ala Ile Ser Gly Lys Asp Asp Val Leu Lys Trp Asp Gly His Ile
                   85                  90                  95
   Tyr Asp Phe Pro Asp Trp Asn Arg Asp Gln Thr Leu Glu Ser Ala Phe
                       100                 105                 110
   Lys Val Ser Cys Val Trp Cys Tyr Gln Ala Leu Ala Arg Gln Val Gly
                   115                 120                 125
   Ala Glu Lys Tyr Arg Asn Tyr Leu Arg Lys Ser Val Tyr Gly Glu Leu
               130                 135                 140
   Arg Glu Pro Phe Glu Glu Thr Thr Phe Trp Leu Asp Gly Ser Leu Gln
   145                 150                 155                 160
   Ile Ser Ala Ile Glu Gln Val Asn Phe Leu Lys Lys Val His Leu Arg
                   165                 170                 175
   Thr Leu Pro Phe Ser Ala Ser Ser Tyr Glu Thr Leu Arg Gln Ile Met
               180                 185                 190
   Leu Ile Glu Gln Thr Pro Ala Phe Thr Leu Arg Ala Lys Thr Gly Trp
           195                 200                 205
   Ala Thr Arg Val Lys Pro Gln Val Gly Trp Tyr Val Gly His Val Glu
       210                 215                 220
   Thr Pro Thr Asp Val Trp Phe Phe Ala Thr Asn Ile Glu Val Arg Asp
   225                 230                 235                 240
   Glu Lys Asp Leu Pro Leu Arg Gln Lys Leu Thr Arg Lys Ala Leu Gln
                   245                 250                 255
   Ala Lys Gly Ile Ile Glu
               260
<210> 471
   <211> 266
   <212> PRT
   <213> RESISTANCE GENE
<400> 471
   Met Lys Thr Phe Ala Ala Tyr Val Ile Thr Ala Cys Leu Ser Ser Thr
   1               5                   10                  15
   Ala Leu Ala Ser Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Thr Asp Lys Phe Trp Leu Glu Asp Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 472
   <211> 274
   <212> PRT
   <213> RESISTANCE GENE
<400> 472
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Leu Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Leu Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 473
   <211> 274
   <212> PRT
   <213> RESISTANCE GENE
<400> 473
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Gln Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 474
   <211> 274
   <212> PRT
   <213> RESISTANCE GENE
<400> 474
   Met Asn Ile Lys Ala Leu Leu Leu Ile Thr Ser Ala Ile Phe Ile Ser
   1               5                   10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Met Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 475
   <211> 274
   <212> PRT
   <213> RESISTANCE GENE
<400> 475
   Met Asn Ile Lys Ala Leu Leu Leu Ile Thr Ser Ala Ile Phe Ile Ser
   1 5 10 15
   Ala Cys Ser Pro Tyr Ile Val Thr Ala Asn Pro Asn His Ser Ala Ser
   20 25 30
   Lys Ser Asp Glu Lys Ala Glu Lys Ile Lys Asn Leu Phe Asn Glu Val
   35 40 45
   His Thr Thr Gly Val Leu Val Ile Gln Gln Gly Gln Thr Gln Gln Ser
   50 55 60
   Tyr Gly Asn Asp Leu Ala Arg Ala Ser Thr Glu Tyr Val Pro Ala Ser
   65 70 75 80
   Thr Phe Lys Met Leu Asn Ala Leu Ile Gly Leu Glu His His Lys Ala
   85 90 95
   Thr Thr Thr Glu Val Phe Lys Trp Asp Gly Lys Lys Arg Leu Phe Pro
   100 105 110
   Glu Trp Glu Lys Asn Met Thr Leu Gly Asp Ala Met Lys Ala Ser Ala
   115 120 125
   Ile Pro Val Tyr Gln Asp Leu Ala Arg Arg Ile Gly Leu Glu Leu Met
   130 135 140
   Ser Asn Glu Val Lys Arg Val Gly Tyr Gly Asn Ala Asp Ile Gly Thr
   145 150 155 160
   Gln Val Asp Asn Phe Trp Leu Val Gly Pro Leu Lys Ile Thr Pro Gln
   165 170 175
   Gln Glu Ala Gln Phe Ala Tyr Lys Leu Ala Asn Lys Thr Leu Pro Phe
   180 185 190
   Ser Gln Lys Val Gln Asp Glu Val Gln Ser Met Leu Phe Ile Glu Glu
   195 200 205
   Lys Asn Gly Asn Lys Ile Tyr Ala Lys Ser Gly Trp Gly Trp Asp Val
   210 215 220
   Asp Pro Gln Val Gly Trp Leu Thr Gly Trp Val Val Gln Pro Gln Gly
   225 230 235 240
   Asn Ile Val Ala Phe Ser Leu Asn Leu Glu Met Lys Lys Gly Ile Pro
   245 250 255
   Ser Ser Val Arg Lys Glu Ile Thr Tyr Lys Ser Leu Glu Gln Leu Gly
   260 265 270
   Ile Leu
<210> 476
   <211> 261
   <212> PRT
   <213> RESISTANCE GENE
<400> 476
   Met Arg Val Leu Ala Leu Ser Ala Val Phe Leu Val Ala Ser Ile Ile
   1 5 10 15
   Gly Met Pro Ala Val Ala Lys Glu Trp Gln Glu Asn Lys Ser Trp Asn
   20 25 30
   Ala His Phe Thr Glu His Lys Ser Gln Gly Val Val Val Leu Trp Asn
   35 40 45
   Glu Asn Lys Gln Gln Gly Phe Thr Asn Asn Leu Lys Arg Ala Asn Gln
   50 55 60
   Ala Phe Leu Pro Ala Ser Thr Phe Lys Ile Pro Asn Ser Leu Ile Ala
   65 70 75 80
   Leu Asp Leu Gly Val Val Lys Asp Glu His Gln Val Phe Lys Trp Asp
                           85                  90                  95
   Gly Gln Thr Arg Asp Ile Ala Thr Trp Asn Arg Asp His Asn Leu Ile
               100                 105                 110
   Thr Ala Met Lys Tyr Ser Val Val Pro Val Tyr Gln Glu Phe Ala Arg
           115                 120                 125
   Gln Ile Gly Glu Ala Arg Met Ser Lys Met Leu His Ala Phe Asp Tyr
       130                 135                 140
   Gly Asn Glu Asp Ile Ser Gly Asn Val Asp Ser Phe Trp Leu Asp Gly
   145                 150                 155                 160
   Gly Ile Arg Ile Ser Ala Thr Glu Gln Ile Ser Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Gly Asp Tyr Ile Ile Arg Ala Lys
           195                 200                 205
   Thr Gly Tyr Asp Thr Lys Ile Gly Trp Trp Val Gly Trp Val Glu Leu
       210                 215                 220
   Asp Asp Asn Val Trp Phe Phe Ala Met Asn Met Asp Met Pro Thr Ser
   225                 230                 235                 240
   Asp Gly Leu Gly Leu Arg Gln Ala Ile Thr Lys Glu Val Leu Lys Gln
                   245                 250                 255
   Glu Lys Ile Ile Pro
               260
<210> 477
   <211> 269
   <212> PRT
   <213> RESISTANCE GENE
<400> 477
   Met Ser Lys Lys Asn Phe Ile Leu Ile Phe Ile Phe Val Ile Leu Ile
   1               5                   10                  15
   Ser Cys Lys Asn Thr Glu Lys Thr Ser Asn Glu Thr Thr Leu Ile Asp
               20                  25                  30
   Asn Ile Phe Thr Asn Ser Asn Ala Glu Gly Thr Leu Val Ile Tyr Asn
           35                  40                  45
   Leu Asn Asp Asp Lys Tyr Ile Ile His Asn Lys Glu Arg Ala Glu Gln
       50                  55                  60
   Arg Phe Tyr Pro Ala Ser Thr Phe Lys Ile Tyr Asn Ser Leu Ile Gly
   65                  70                  75                  80
   Leu Asn Glu Lys Ala Val Lys Asp Val Asp Glu Val Phe Tyr Lys Tyr
                   85                  90                  95
   Asn Gly Glu Lys Val Phe Leu Glu Ser Trp Ala Lys Asp Ser Asn Leu
               100                 105                 110
   Arg Tyr Ala Ile Lys Asn Ser Gln Val Pro Ala Tyr Lys Glu Leu Ala
           115                 120                 125
   Arg Arg Ile Gly Leu Glu Lys Met Lys Glu Asn Ile Glu Lys Leu Asp
       130                 135                 140
   Phe Gly Asn Lys Asn Ile Gly Asp Ser Val Asp Thr Phe Trp Leu Glu
   145                 150                 155                 160
   Gly Pro Leu Glu Ile Ser Ala Met Glu Gln Val Lys Leu Leu Thr Lys
                   165                 170                 175
   Leu Ala Gln Asn Glu Leu Pro Tyr Pro Ile Glu Ile Gln Lys Ala Val
               180                 185                 190
   Ser Asp Ile Thr Ile Leu Glu Gln Thr Asp Asn Tyr Thr Leu His Gly
           195                 200                 205
   Lys Thr Gly Leu Ala Asp Ser Glu Asn Met Thr Thr Glu Pro Ile Gly
       210                 215                 220
   Trp Leu Val Gly Trp Leu Glu Glu Asn Asn Asn Ile Tyr Val Phe Ala
   225                 230                 235                 240
   Leu Asn Ile Asp Asn Ile Asn Ser Asp Asp Leu Ala Lys Arg Ile Asn
                   245                 250                 255
   Ile Val Lys Glu Ser Leu Lys Ala Leu Asn Leu Leu Lys
                       260                 265
<210> 478
   <211> 274
   <212> PRT
   <213> RESISTANCE GENE
<400> 478
   Met Asn Ile Lys Ala Leu Phe Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 479
   <211> 274
   <212> PRT
   <213> RESISTANCE GENE
<400> 479
   Met Asn Ile Lys Ala Leu Phe Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Ser
               180                 185                 190
   Ser Gln Lys Val Gln Asp Glu Val Gln Ser Met Leu Phe Ile Glu Glu
           195                 200                 205
   Lys Asn Gly Asn Lys Met Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 480
   <211> 274
   <212> PRT
   <213> RESISTANCE GENE
<400> 480
   Met Asn Ile Lys Ala Leu Phe Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Ala Tyr Lys Ser Leu Glu Gln Leu Gly
               260                 265                 270
   Ile Leu
<210> 481
   <211> 275
   <212> PRT
   <213> RESISTANCE GENE
<400> 481
   Met Ala Ile Arg Ile Phe Ala Ile Leu Phe Ser Ile Phe Ser Leu Ala
    1               5                  10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
       50                  55                  60
   Lys Lys Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe Gln Ile Phe Arg
                   85                  90                  95
   Trp Asp Gly Val Asn Arg Gly Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Glu Ile Gly Asp Asp Lys Ala Arg Arg Tyr Leu Lys Lys Ile
       130                 135                 140
   Asp Tyr Gly Asn Ala Asp Pro Ser Thr Ser Asn Gly Asp Cys Trp Ile
   145                 150                 155                 160
   Glu Gly Ser Leu Ala Ile Ser Ala Gln Glu Gln Ile Ala Phe Leu Arg
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Ser Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 482
   <211> 274
   <212> PRT
   <213> RESISTANCE GENE
<400> 482
   Met Asn Ile Lys Thr Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Val
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
    65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Val Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 483
   <211> 274
   <212> PRT
   <213> RESISTANCE GENE
<400> 483
   Met Asn Ile Lys Thr Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Val
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu Tyr His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 484
   <211> 266
   <212> PRT
   <213> RESISTANCE GENE
<400> 484
   Met Ala Ile Arg Phe Leu Thr Ile Leu Leu Ser Thr Phe Phe Leu Thr
    1               5                  10                  15
   Ser Phe Val His Ala Gln Glu His Val Val Val Arg Ser Asp Trp Lys
               20                  25                  30
   Lys Phe Phe Ser Asp Leu Gln Ala Glu Gly Ala Ile Val Ile Ala Asp
           35                  40                  45
   Glu Arg Gln Ala Glu His Ala Leu Leu Val Phe Gly Gln Glu Arg Ala
       50                  55                  60
   Ala Lys Arg Tyr Ser Pro Ala Ser Thr Phe Lys Leu Pro His Thr Leu
   65                  70                  75                  80
   Phe Ala Leu Asp Ala Gly Ala Val Arg Asp Glu Phe Gln Val Phe Arg
                   85                  90                  95
   Trp Asp Gly Val Lys Arg Ser Phe Ala Gly His Asn Gln Asp Gln Asp
               100                 105                 110
   Leu Arg Ser Ala Met Arg Asn Ser Ala Val Trp Val Tyr Glu Leu Phe
           115                 120                 125
   Ala Lys Glu Ile Gly Glu Asp Asn Ala Arg Arg Tyr Leu Lys Gln Ile
       130                 135                 140
   Asp Tyr Gly Asn Ala Asp Pro Ser Thr Ile Lys Gly Asn Tyr Trp Ile
   145                 150                 155                 160
   Asp Gly Asn Leu Glu Ile Ser Ala His Glu Gln Ile Ser Phe Leu Arg
                   165                 170                 175
   Lys Leu Tyr Arg Asn Gln Leu Pro Phe Gln Val Glu His Gln Arg Leu
               180                 185                 190
   Val Lys Tyr Leu Met Ile Thr Glu Ala Gly Arg Asn Trp Ile Leu Arg
           195                 200                 205
   Ala Lys Thr Gly Trp Glu Gly Arg Phe Gly Trp Trp Ile Gly Trp Val
       210                 215                 220
   Glu Trp Pro Thr Gly Pro Val Phe Phe Ala Leu Asn Ile Asp Thr Pro
   225                 230                 235                 240
   Asn Arg Thr Asp Asp Leu Phe Lys Arg Glu Ala Ile Ala Arg Ala Ile
                   245                 250                 255
   Leu Arg Ser Ile Asp Ala Leu Pro Pro Asn
               260                 265
<210> 485
   <211> 265
   <212> PRT
   <213> RESISTANCE GENE
<400> 485
   Met Arg Val Leu Ala Leu Ser Ala Val Phe Leu Val Ala Ser Ile Ile
    1               5                  10                  15
   Gly Met Pro Ala Val Ala Lys Glu Trp Gln Glu Asn Lys Ser Trp Asn
               20                  25                  30
   Ala His Phe Thr Glu His Lys Ser Gln Gly Val Val Val Leu Trp Asn
           35                  40                  45
   Glu Asn Lys Gln Gln Gly Phe Thr Asn Asn Leu Lys Arg Ala Asn Gln
       50                  55                  60
   Ala Phe Leu Pro Ala Ser Thr Phe Lys Ile Pro Asn Ser Leu Ile Ala
    65                 70                  75                  80
   Leu Asp Leu Gly Val Val Lys Asp Glu His Gln Val Phe Lys Trp Asp
                    85                  90                  95
   Gly Gln Thr Arg Asp Ile Ala Ala Trp Asn Arg Asp His Asp Leu Ile
               100                 105                 110
   Thr Ala Met Lys Tyr Ser Val Val Pro Val Tyr Gln Glu Phe Ala Arg
           115                 120                 125
   Gln Ile Gly Glu Ala Arg Met Ser Lys Met Leu His Ala Phe Asp Tyr
       130                 135                 140
   Gly Asn Glu Asp Ile Ser Gly Asn Val Asp Ser Phe Trp Leu Asp Gly
   145                 150                 155                 160
   Gly Ile Arg Ile Ser Ala Thr Gln Gln Ile Ala Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Gly Asp Tyr Ile Ile Arg Ala Lys
           195                 200                 205
   Thr Gly Tyr Ser Thr Arg Ile Glu Pro Lys Ile Gly Trp Trp Val Gly
       210                 215                 220
   Trp Val Glu Leu Asp Asp Asn Val Trp Phe Phe Ala Met Asn Met Asp
   225                 230                 235                 240
   Met Pro Thr Ser Asp Gly Leu Gly Leu Arg Gln Ala Ile Thr Lys Glu
                   245                 250                 255
   Val Leu Lys Gln Glu Lys Ile Ile Pro
               260                 265
<210> 486
   <211> 273
   <212> PRT
   <213> RESISTANCE GENE
<400> 486
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Ser Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 487
   <211> 274
   <212> PRT
   <213> RESISTANCE GENE
<400> 487
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Val Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Ser Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 488
   <211> 275
   <212> PRT
   <213> RESISTANCE GENE
<400> 488
   Met Ala Ile Arg Ile Phe Ala Ile Leu Phe Ser Ile Phe Ser Leu Ala
    1               5                  10                  15
   Thr Phe Ala His Ala Gln Glu Gly Thr Leu Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Ala Asp Arg Ala Met Leu Val Phe Asp Pro Val Arg Ser
       50                  55                  60
   Lys Lys Arg Tyr Ser Pro Ala Ser Thr Phe Lys Ile Pro His Thr Leu
   65                  70                  75                  80
   Phe Ala Leu Asp Ala Gly Ala Val Arg Asp Glu Phe Gln Ile Phe Arg
                   85                  90                  95
   Trp Asp Gly Val Asn Arg Gly Phe Ala Gly His Asn Gln Asp Gln Asp
               100                 105                 110
   Leu Arg Ser Ala Met Arg Asn Ser Thr Val Trp Val Tyr Glu Leu Phe
           115                 120                 125
   Ala Lys Glu Ile Gly Asp Asp Lys Ala Arg Arg Tyr Leu Lys Lys Ile
       130                 135                 140
   Asp Tyr Gly Asn Ala Asp Pro Ser Thr Ser Asn Gly Asp Tyr Trp Ile
   145                 150                 155                 160
   Glu Gly Ser Leu Ala Ile Ser Ala Gln Glu Gln Ile Ala Phe Leu Arg
                   165                 170                 175
   Lys Leu Tyr Arg Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Ser Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 489
   <211> 267
   <212> PRT
   <213> RESISTANCE GENE
<400> 489
   Met Lys Thr Ile Ala Ala Tyr Leu Val Leu Val Phe Tyr Ala Ser Thr
    1               5                  10                  15
   Ala Leu Ser Glu Ser Ile Ser Glu Asn Leu Ala Trp Asn Lys Glu Phe
               20                  25                  30
   Ser Ser Glu Ser Val His Gly Val Phe Val Leu Cys Lys Ser Ser Ser
           35                  40                  45
   Asn Ser Cys Thr Thr Asn Asn Ala Ala Arg Ala Ser Thr Ala Tyr Ile
       50                  55                  60
   Pro Ala Ser Thr Phe Lys Ile Pro Asn Ala Leu Ile Gly Leu Glu Thr
   65                  70                  75                  80
   Gly Ala Ile Lys Asp Glu Arg Gln Val Phe Lys Trp Asp Gly Lys Pro
                   85                  90                  95
   Arg Ala Met Lys Gln Trp Glu Lys Asp Leu Lys Leu Arg Gly Ala Ile
               100                 105                 110
   Gln Val Ser Ala Val Pro Val Phe Gln Gln Ile Ala Arg Glu Val Gly
           115                 120                 125
   Glu Ile Arg Met Gln Lys Tyr Leu Asn Leu Phe Ser Tyr Gly Asn Ala
       130                 135                 140
   Asn Ile Gly Gly Gly Ile Asp Lys Phe Trp Leu Glu Gly Gln Leu Arg
   145                 150                 155                 160
   Ile Ser Ala Phe Asn Gln Val Lys Phe Leu Glu Ser Leu Tyr Leu Asn
                   165                 170                 175
   Asn Leu Pro Ala Ser Lys Ala Asn Gln Leu Ile Val Lys Glu Ala Ile
               180                 185                 190
   Val Thr Glu Ala Thr Pro Glu Tyr Ile Val His Ser Lys Thr Gly Tyr
           195                 200                 205
   Ser Gly Val Gly Thr Glu Ser Ser Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Ser Lys Leu Pro Ser Arg Lys Ser Ile Ser Thr Lys
                   245                 250                 255
   Ile Met Ala Ser Glu Gly Ile Ile Ile Gly Gly
               260                 265
<210> 490
   <211> 266
   <212> PRT
   <213> RESISTANCE GENE
<400> 490
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Phe Trp Asn Lys Glu Phe
               20                  25                  30
   Ser Ala Glu Ala Val Asn Gly Val Phe Val Leu Cys Lys Ser Ser Ser
           35                  40                  45
   Lys Leu Ala Cys Ala Thr Asn Asn Leu Ala Arg Ala Ser Lys Glu Tyr
       50                  55                  60
   Leu Pro Ala Ser Thr Phe Lys Ile Pro Asn Ala Ile Ile Gly Leu Glu
   65                  70                  75                  80
   Thr Gly Val Ile Lys Asn Glu His Gln Ile Phe Lys Trp Asp Gly Lys
                   85                  90                  95
   Pro Arg Ala Met Lys Gln Trp Glu Arg Asp Leu Ser Leu Arg Gly Ala
               100                 105                 110
   Ile Gln Val Ser Ala Val Pro Val Phe Gln Gln Ile Ala Arg Glu Val
           115                 120                 125
   Gly Glu Val Arg Met Gln Lys Tyr Leu Lys Lys Phe Ser Tyr Gly Asn
       130                 135                 140
   Gln Asn Ile Ser Gly Gly Ile Asp Lys Phe Trp Leu Glu Gly Gln Leu
   145                 150                 155                 160
   Arg Ile Ser Ala Val Asn Gln Val Glu Phe Leu Glu Ser Leu Phe Leu
                   165                 170                 175
   Asn Lys Leu Ser Ala Ser Lys Glu Asn Gln Leu Ile Val Lys Glu Ala
               180                 185                 190
   Leu Val Thr Glu Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Ala Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 491
   <211> 275
   <212> PRT
   <213> RESISTANCE GENE
<400> 491
   Met Ala Ile Arg Ile Phe Ala Ile Leu Phe Ser Thr Phe Val Phe Gly
    1               5                  10                  15
   Thr Phe Ala His Ala Gln Glu Gly Met Arg Glu Arg Ser Asp Trp Arg
               20                  25                  30
   Lys Phe Phe Ser Glu Phe Gln Ala Lys Gly Thr Ile Val Val Ala Asp
           35                  40                  45
   Glu Arg Gln Thr Asp Arg Val Ile Leu Val Phe Asp Gln Val Arg Ser
       50                  55                  60
   Glu Lys Arg Tyr Ser Pro Ala Ser Thr Phe Lys Ile Pro His Thr Leu
    65                  70                  75                  80
   Phe Ala Leu Asp Ala Gly Ala Ala Arg Asp Glu Phe Gln Val Phe Arg
                    85                  90                  95
   Trp Asp Gly Ile Lys Arg Ser Phe Ala Ala His Asn Gln Asp Gln Asp
               100                 105                 110
   Leu Arg Ser Ala Met Arg Asn Ser Thr Val Trp Ile Tyr Glu Leu Phe
           115                 120                 125
   Ala Lys Glu Ile Gly Glu Asp Lys Ala Arg Arg Tyr Leu Lys Gln Ile
       130                 135                 140
   Asp Tyr Gly Asn Ala Asp Pro Ser Thr Ser Asn Gly Asp Tyr Trp Ile
   145                 150                 155                 160
   Asp Gly Asn Leu Ala Ile Ala Ala Gln Glu Gln Ile Ala Phe Leu Arg
                   165                 170                 175
   Lys Leu Tyr His Asn Glu Leu Pro Phe Arg Val Glu His Gln Arg Leu
               180                 185                 190
   Val Lys Asp Leu Met Ile Val Glu Ala Gly Arg Asn Trp Ile Leu Arg
           195                 200                 205
   Ala Lys Thr Gly Trp Glu Gly Arg Met Gly Trp Trp Val Gly Trp Val
       210                 215                 220
   Glu Trp Pro Thr Gly Pro Val Phe Phe Ala Leu Asn Ile Asp Thr Pro
   225                 230                 235                 240
   Asn Arg Met Asp Asp Leu Phe Lys Arg Glu Ala Ile Val Arg Ala Ile
                   245                 250                 255
   Leu Arg Ser Ile Glu Ala Leu Pro Pro Asn Pro Ala Val Asn Ser Asp
               260                 265                 270
   Ala Ala Arg
           275
<210> 492
   <211> 266
   <212> PRT
   <213> RESISTANCE GENE
<400> 492
   Met Lys Thr Phe Ala Ala Tyr Val Ile Ile Ala Cys Leu Ser Ser Thr
   1               5                   10                  15
   Ala Leu Ala Gly Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asp Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Thr Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Ser Gln
       130                 135                 140
   Asn Ile Ser Gly Gly Ile Asp Lys Phe Trp Leu Glu Asp Gln Leu Arg
   145                 150                 155                 160
   Ile Ser Ala Val Asn Gln Val Glu Phe Leu Glu Ser Leu Tyr Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Glu Thr Glu Val Tyr Phe Phe Ala Phe Asn Met Asp
   225                 230                 235                 240
   Ile Asp Asn Glu Ser Lys Leu Pro Leu Arg Lys Ser Ile Pro Thr Lys
                   245                 250                 255
   Ile Met Glu Ser Glu Gly Ile Ile Gly Gly
               260                 265
<210> 493
   <211> 273
   <212> PRT
   <213> RESISTANCE GENE
<400> 493
   Met Asn Lys Tyr Phe Thr Cys Tyr Val Val Ala Ser Leu Phe Leu Ser
    1               5                  10                  15
   Gly Cys Thr Val Gln His Asn Leu Ile Asn Glu Thr Pro Ser Gln Ile
               20                  25                  30
   Val Gln Gly His Asn Gln Val Ile His Gln Tyr Phe Asp Glu Lys Asn
           35                  40                  45
   Thr Ser Gly Val Leu Val Ile Gln Thr Asp Lys Lys Ile Asn Leu Tyr
       50                  55                  60
   Gly Asn Ala Leu Ser Arg Ala Asn Thr Glu Tyr Val Pro Ala Ser Thr
   65                  70                  75                  80
   Phe Lys Met Leu Asn Ala Leu Ile Gly Leu Glu Asn Gln Lys Thr Asp
                   85                  90                  95
   Ile Asn Glu Ile Phe Lys Trp Lys Gly Glu Lys Arg Ser Phe Thr Ala
               100                 105                 110
   Trp Glu Lys Asp Met Thr Leu Gly Glu Ala Met Lys Leu Ser Ala Val
           115                 120                 125
   Pro Val Tyr Gln Glu Leu Ala Arg Arg Ile Gly Leu Asp Leu Met Gln
       130                 135                 140
   Lys Glu Val Lys Arg Ile Gly Phe Gly Asn Ala Glu Ile Gly Gln Gln
   145                 150                 155                 160
   Val Asp Asn Phe Trp Leu Val Gly Pro Leu Lys Val Thr Pro Ile Gln
                   165                 170                 175
   Glu Val Glu Phe Val Ser Gln Leu Ala His Thr Gln Leu Pro Phe Ser
               180                 185                 190
   Glu Lys Val Gln Ala Asn Val Lys Asn Met Leu Leu Leu Glu Glu Ser
           195                 200                 205
   Asn Gly Tyr Lys Ile Phe Gly Lys Thr Gly Trp Ala Met Asp Ile Lys
       210                 215                 220
   Pro Gln Val Gly Trp Leu Thr Gly Trp Val Glu Gln Pro Asp Gly Lys
   225                 230                 235                 240
   Ile Val Ala Phe Ala Leu Asn Met Glu Met Arg Ser Glu Met Pro Ala
                   245                 250                 255
   Ser Ile Arg Asn Glu Leu Leu Met Lys Ser Leu Lys Gln Leu Asn Ile
               260                 265                 270
   Ile
<210> 494
   <211> 266
   <212> PRT
   <213> RESISTANCE GENE
<400> 494
   Met Lys Lys Leu Ser Val Leu Leu Trp Leu Thr Leu Phe Tyr Cys Gly
    1               5                  10                  15
   Thr Ile Trp Ala Gln Ser Thr Cys Phe Leu Val Gln Glu Asn Gln Thr
               20                  25                  30
   Val Leu Lys His Glu Gly Lys Asp Cys Asn Lys Arg Phe Ala Pro Glu
           35                  40                  45
   Ser Thr Phe Lys Ile Ala Leu Ser Leu Met Gly Phe Asp Ser Gly Ile
           50                  55                  60
   Leu Lys Asp Thr Leu Asn Pro Glu Trp Pro Tyr Lys Lys Glu Tyr Glu
   65                  70                  75                  80
   Leu Tyr Leu Asn Val Trp Lys Tyr Pro His Asn Pro Arg Thr Trp Ile
                   85                  90                  95
   Arg Asp Ser Cys Val Trp Tyr Ser Gln Val Leu Thr Gln Gln Leu Gly
               100                 105                 110
   Met Thr Arg Phe Lys Asn Tyr Val Asp Ala Phe His Tyr Gly Asn Gln
           115                 120                 125
   Asp Ile Ser Gly Asp Lys Gly Gln Asn Asn Gly Leu Thr His Ser Trp
       130                 135                 140
   Leu Ser Ser Ser Leu Ala Ile Ser Pro Ser Glu Gln Ile Gln Phe Leu
   145                 150                 155                 160
   Gln Lys Ile Val Asn Lys Lys Leu Ser Val Asn Pro Lys Ala Phe Thr
                   165                 170                 175
   Met Thr Lys Asp Ile Leu Tyr Ile Gln Glu Leu Ala Gly Gly Trp Lys
               180                 185                 190
   Leu Tyr Gly Lys Thr Gly Asn Gly Arg Gln Leu Thr Lys Asp Lys Ser
           195                 200                 205
   Gln Lys Leu Ser Leu Gln His Gly Trp Phe Ile Gly Trp Ile Glu Lys
       210                 215                 220
   Asp Gly Arg Val Ile Thr Phe Thr Lys His Ile Ala Asp Ser Lys Lys
   225                 230                 235                 240
   His Val Thr Phe Ala Ser Phe Arg Ala Lys Asn Glu Thr Leu Asn Gln
                   245                 250                 255
   Leu Phe Tyr Leu Ile Asn Glu Leu Glu Lys
               260                 265
<210> 495
   <211> 274
   <212> PRT
   <213> RESISTANCE GENE
<400> 495
   Met Asn Ile Lys Thr Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Val
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Pro Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 496
   <211> 269
   <212> PRT
   <213> RESISTANCE GENE
<400> 496
   Met Lys Phe Arg His Ala Leu Ser Ser Ala Phe Val Leu Leu Gly Cys
    1               5                  10                  15
   Ile Ala Ala Ser Ala His Ala Lys Thr Ile Cys Thr Ala Ile Ala Asp
               20                  25                  30
   Ala Gly Thr Gly Lys Leu Leu Val Gln Asp Gly Asp Cys Gly Arg Arg
           35                  40                  45
   Ala Ser Pro Ala Ser Thr Phe Lys Ile Ala Ile Ser Leu Met Gly Tyr
       50                  55                  60
   Asp Ala Gly Phe Leu Arg Asn Glu His Asp Pro Val Leu Pro Tyr Arg
   65                  70                  75                  80
   Asp Ser Tyr Ile Ala Trp Gly Gly Glu Ala Trp Lys Gln Pro Thr Asp
                   85                  90                  95
   Pro Thr Arg Trp Leu Lys Tyr Ser Val Val Trp Tyr Ser Gln Gln Val
               100                 105                 110
   Ala His His Leu Gly Ala Gln Arg Phe Ala Gln Tyr Ala Lys Ala Phe
           115                 120                 125
   Gly Tyr Gly Asn Ala Asp Val Ser Gly Asp Pro Gly Gln Asn Asn Gly
       130                 135                 140
   Leu Asp Arg Ala Trp Ile Gly Ser Ser Leu Gln Ile Ser Pro Leu Glu
   145                 150                 155                 160
   Gln Leu Glu Phe Leu Gly Lys Met Leu Asn Arg Lys Leu Pro Val Ser
                   165                 170                 175
   Pro Thr Ala Val Asp Met Thr Glu Arg Ile Val Glu Ser Thr Thr Leu
               180                 185                 190
   Ala Asp Gly Thr Val Val His Gly Lys Thr Gly Val Ser Tyr Pro Leu
           195                 200                 205
   Leu Ala Asp Gly Thr Arg Asp Trp Ala Arg Gly Ser Gly Trp Phe Val
       210                 215                 220
   Gly Trp Ile Val Arg Gly Asn Gln Thr Leu Val Phe Ala Arg Leu Thr
   225                 230                 235                 240
   Gln Asp Glu Arg Lys Gln Pro Val Ser Ala Gly Ile Arg Thr Arg Glu
                   245                 250                 255
   Ala Phe Leu Arg Asp Leu Pro Arg Leu Leu Ala Ala Arg
               260                 265
<210> 497
   <211> 269
   <212> PRT
   <213> RESISTANCE GENE
<400> 497
   Met Lys Phe Arg His Ala Leu Ser Ser Ala Phe Val Leu Leu Gly Cys
    1               5                  10                  15
   Ile Ala Ala Ser Ala His Ala Lys Thr Ile Cys Thr Ala Ile Ala Asp
               20                  25                  30
   Ala Gly Thr Gly Lys Leu Leu Val Gln Asp Gly Asp Cys Gly Arg Arg
           35                  40                  45
   Ala Ser Pro Ala Ser Thr Phe Lys Ile Ala Ile Ser Leu Met Gly Tyr
       50                  55                  60
   Asp Ala Gly Phe Leu Arg Asn Glu His Asp Pro Val Leu Pro Tyr Arg
    65                  70                  75                  80
   Asp Ser Tyr Ile Ala Trp Gly Gly Glu Ala Trp Lys Gln Pro Thr Asp
                    85                  90                  95
   Pro Thr Arg Trp Leu Lys Tyr Pro Val Val Trp Tyr Ser Gln Gln Val
               100                 105                 110
   Ala His His Leu Gly Ala Gln Arg Phe Ala Gln Tyr Ala Lys Ala Phe
           115                 120                 125
   Gly Tyr Gly Asn Ala Asp Val Ser Gly Asp Pro Gly Gln Asn Asn Gly
       130                 135                 140
   Leu Asp Arg Ala Trp Ile Gly Ser Ser Leu Gln Ile Ser Pro Leu Glu
   145                 150                 155                 160
   Gln Leu Glu Phe Leu Gly Lys Met Leu Asn Arg Lys Leu Pro Val Ser
                   165                 170                 175
   Pro Thr Ala Val Asp Met Thr Glu Arg Ile Val Glu Ser Thr Thr Leu
               180                 185                 190
   Ala Asp Gly Thr Val Val His Gly Lys Thr Gly Val Ser Tyr Pro Leu
           195                 200                 205
   Leu Ala Asp Gly Thr Arg Asp Trp Ala Arg Gly Ser Gly Trp Phe Val
       210                 215                 220
   Gly Trp Ile Val Arg Gly Lys Gln Thr Leu Val Phe Ala Arg Leu Thr
   225                 230                 235                 240
   Gln Asp Glu Arg Lys Gln Pro Val Ser Ala Gly Ile Arg Thr Arg Glu
                   245                 250                 255
   Ala Phe Leu Arg Asp Leu Pro Arg Leu Leu Ala Ala Arg
               260                 265
<210> 498
   <211> 264
   <212> PRT
   <213> RESISTANCE GENE
<400> 498
   Met Arg Gly Lys His Thr Val Ile Leu Gly Ala Ala Leu Ser Ala Leu
   1               5                   10                  15
   Phe Ala Gly Ala Ala Gly Ala Gln Met Leu Glu Cys Thr Leu Val Ala
               20                  25                  30
   Asp Ala Ala Ser Gly Gln Glu Leu Tyr Arg Lys Gly Ala Cys Asp Lys
           35                  40                  45
   Ala Phe Ala Pro Met Ser Thr Phe Lys Val Pro Leu Ala Val Met Gly
       50                  55                  60
   Tyr Asp Ala Gly Ile Leu Val Asp Ala His Asn Pro Arg Trp Asp Tyr
   65                  70                  75                  80
   Lys Pro Glu Phe Asn Gly Tyr Lys Phe Gln Gln Lys Thr Thr Asp Pro
                   85                  90                  95
   Thr Ile Trp Glu Lys Asp Ser Ile Val Trp Tyr Ser Gln Gln Leu Thr
               100                 105                 110
   Arg Lys Met Gly Gln Lys Arg Phe Ala Ala Tyr Val Ala Gly Phe Gly
           115                 120                 125
   Tyr Gly Asn Gly Asp Ile Ser Gly Glu Pro Gly Lys Ser Asn Gly Leu
       130                 135                 140
   Thr His Ser Trp Leu Gly Ser Ser Leu Lys Ile Ser Pro Glu Gly Gln
   145                 150                 155                 160
   Val Arg Phe Val Arg Asp Leu Leu Ser Ala Lys Leu Pro Ala Ser Lys
                   165                 170                 175
   Asp Ala Gln Gln Met Thr Val Ser Ile Leu Pro His Phe Ala Ala Gly
               180                 185                 190
   Asp Trp Ala Val Gln Gly Lys Thr Gly Thr Gly Ser Phe Ile Asp Ala
           195                 200                 205
   Arg Gly Ala Lys Ala Pro Leu Gly Trp Phe Ile Gly Trp Ala Thr His
       210                 215                 220
   Glu Glu Arg Arg Val Val Phe Ala Arg Met Thr Ala Gly Gly Lys Lys
   225                 230                 235                 240
   Gly Glu Gln Pro Ala Gly Pro Ala Ala Arg Asp Ala Phe Leu Lys Ala
                   245                 250                 255
   Leu Pro Asp Leu Ala Lys Arg Phe
               260
<210> 499
   <211> 269
   <212> PRT
   <213> RESISTANCE GENE
<400> 499
   Met Lys Phe Arg His Ala Leu Ser Ser Ala Phe Val Leu Leu Gly Cys
    1               5                  10                  15
   Ile Ala Ala Ser Ala His Ala Lys Thr Ile Cys Thr Ala Ile Ala Asp
               20                  25                  30
   Ala Gly Thr Gly Lys Leu Leu Val Gln Asp Gly Asp Cys Gly Arg Arg
           35                  40                  45
   Ala Ser Pro Ala Ser Thr Phe Lys Ile Ala Ile Ser Leu Met Gly Tyr
       50                  55                  60
   Asp Ala Gly Phe Leu Arg Asn Glu His Asp Pro Val Leu Pro Tyr Arg
   65                  70                  75                  80
   Asp Ser Tyr Ile Ala Trp Gly Gly Glu Ala Trp Lys Gln Pro Thr Asp
                   85                  90                  95
   Pro Thr Arg Trp Leu Lys Tyr Ser Val Val Trp Tyr Ser Gln Gln Val
               100                 105                 110
   Ala His His Leu Gly Ala Gln Arg Phe Ala Gln Tyr Ala Lys Ala Phe
           115                 120                 125
   Gly Tyr Gly Asn Ala Asp Val Ser Gly Asp Pro Gly Gln Asn Asn Gly
       130                 135                 140
   Leu Asp Arg Ala Trp Ile Gly Ser Ser Leu Gln Ile Ser Pro Leu Glu
   145                 150                 155                 160
   Gln Leu Glu Phe Leu Gly Lys Met Leu Asp Arg Lys Leu Pro Val Ser
                   165                 170                 175
   Pro Thr Ala Val Asp Met Thr Glu Arg Ile Val Glu Ser Thr Thr Leu
               180                 185                 190
   Ala Asp Gly Thr Val Val His Gly Lys Thr Gly Val Ser Tyr Pro Leu
           195                 200                 205
   Leu Ala Asp Gly Thr Arg Asp Trp Ala Arg Gly Ser Gly Trp Phe Val
       210                 215                 220
   Gly Trp Ile Val Arg Gly Lys Gln Thr Leu Val Phe Ala Arg Leu Thr
   225                 230                 235                 240
   Gln Asp Glu Arg Lys Gln Pro Val Ser Ala Gly Ile Arg Thr Arg Glu
                   245                 250                 255
   Ala Phe Leu Arg Asp Leu Pro Arg Leu Leu Ala Ala Arg
               260                 265
<210> 500
   <211> 269
   <212> PRT
   <213> RESISTANCE GENE
<400> 500
   Met Lys Phe Arg His Ala Leu Ser Ser Ala Phe Val Leu Leu Gly Cys
    1               5                  10                  15
   Ile Ala Ala Ser Ala His Ala Lys Thr Ile Cys Thr Ala Ile Ala Asp
               20                  25                  30
   Ala Gly Thr Gly Lys Leu Leu Val Gln Asp Gly Asp Cys Gly Arg Arg
           35                  40                  45
   Ala Ser Pro Ala Ser Thr Phe Lys Ile Ala Ile Ser Leu Met Gly Tyr
       50                  55                  60
   Asp Ala Gly Phe Leu Arg Asn Glu His Asp Pro Val Leu Pro Tyr Arg
   65                  70                  75                  80
   Asp Ser Tyr Ile Ala Trp Gly Gly Glu Ala Trp Lys Gln Pro Thr Asp
                       85                  90                  95
   Pro Thr Arg Trp Leu Lys Tyr Ser Val Val Trp Tyr Ser Gln Gln Val
                   100                 105                 110
   Ala His His Leu Gly Ala Gln Arg Phe Ala Gln Tyr Ala Lys Ala Phe
               115                 120                 125
   Gly Tyr Gly Asn Ala Asp Val Ser Gly Asp Pro Gly Gln Asn Asn Gly
       130                 135                 140
   Leu Asp Arg Ala Trp Ile Gly Ser Ser Leu Gln Ile Ser Pro Leu Glu
   145                 150                 155                 160
   Gln Leu Glu Phe Leu Gly Lys Met Leu Asn Arg Lys Leu Pro Val Ser
                   165                 170                 175
   Pro Thr Ala Val Asp Met Thr Glu Arg Ile Val Glu Ser Thr Thr Leu
                   180                 185                 190
   Ala Asp Gly Thr Val Val His Gly Lys Thr Gly Val Ser Tyr Pro Leu
               195                 200                 205
   Leu Ala Asp Gly Thr Arg Asp Trp Ala Arg Gly Ser Gly Trp Phe Val
       210                 215                 220
   Gly Trp Ile Val Arg Gly Lys Gln Thr Leu Val Phe Ala Arg Leu Thr
   225                 230                 235                 240
   Gln Asp Glu Arg Lys Gln Pro Val Ser Ala Gly Ile Arg Thr Arg Glu
                   245                 250                 255
   Ala Phe Leu Arg Asp Leu Pro Arg Leu Leu Ala Ala Arg
                   260                 265
<210> 501
   <211> 266
   <212> PRT
   <213> RESISTANCE GENE
<400> 501
   Met Lys Thr Phe Ala Ala Tyr Val Ile Ile Ala Cys Leu Ser Ser Thr
   1               5                   10                  15
   Ala Leu Ala Gly Ser Ile Thr Glu Asn Thr Ser Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asp Leu Ala Arg Ala Ser Lys Glu Tyr Leu
       50                  55                  60
   Pro Val Ser Thr Phe Lys Ile Pro Ser Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Thr Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Tyr Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Glu Thr Glu Val Tyr Phe Phe Ala Phe Asn Met Asp
   225                 230                 235                 240
   Ile Asp Asn Glu Ser Lys Leu Pro Leu Arg Lys Ser Ile Pro Thr Lys
                   245                 250                 255
   Ile Met Glu Ser Glu Gly Ile Ile Gly Gly
                       260                 265
<210> 502
   <211> 274
   <212> PRT
   <213> RESISTANCE GENE
<400> 502
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Lys Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 503
   <211> 274
   <212> PRT
   <213> RESISTANCE GENE
<400> 503
   Met Asn Ile Lys Ala Leu Leu Leu Ile Thr Ser Ala Ile Phe Ile Ser
    1               5                  10                  15
   Ala Cys Ser Pro Tyr Ile Val Ser Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asn Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asn Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 504
   <211> 266
   <212> PRT
   <213> RESISTANCE GENE
<400> 504
   Met Lys Thr Phe Ala Ala Tyr Val Ile Thr Ala Cys Leu Ser Ser Thr
    1               5                  10                  15
   Ala Leu Ala Ser Ser Ile Thr Glu Asn Thr Phe Trp Asn Lys Glu Phe
               20                  25                  30
   Ser Ala Glu Ala Val Asn Gly Val Phe Val Leu Cys Lys Ser Ser Ser
           35                  40                  45
   Lys Ser Cys Ala Thr Asn Asn Leu Ala Arg Ala Ser Lys Glu Tyr Leu
       50                  55                  60
   Pro Ala Ser Thr Phe Lys Ile Pro Asn Ala Ile Ile Gly Leu Glu Thr
   65                  70                  75                  80
   Gly Val Ile Lys Asn Glu His Gln Val Phe Lys Trp Asp Gly Lys Pro
                   85                  90                  95
   Arg Ala Met Lys Gln Trp Glu Arg Asp Leu Ser Leu Arg Gly Ala Ile
               100                 105                 110
   Gln Val Ser Ala Val Pro Val Phe Gln Gln Ile Ala Arg Glu Val Gly
           115                 120                 125
   Glu Val Arg Met Gln Lys Tyr Leu Lys Lys Phe Ser Tyr Gly Asn Gln
       130                 135                 140
   Asn Ile Ser Gly Gly Ile Asp Lys Phe Trp Leu Glu Gly Gln Leu Arg
   145                 150                 155                 160
   Ile Ser Ala Val Asn Gln Val Glu Phe Leu Glu Ser Leu Phe Leu Asn
                   165                 170                 175
   Lys Leu Ser Ala Ser Lys Glu Asn Gln Leu Ile Val Lys Glu Ala Leu
               180                 185                 190
   Val Thr Glu Ala Ala Pro Glu Tyr Leu Val His Ser Lys Thr Gly Phe
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Asn Lys Leu Pro Leu Arg Lys Ser Ile Pro Thr Lys
                   245                 250                 255
   Ile Met Ala Ser Glu Gly Ile Ile Gly Gly
               260                 265
<210> 505
   <211> 267
   <212> PRT
   <213> RESISTANCE GENE
<400> 505
   Met Lys Thr Ile Ala Ala Tyr Leu Val Leu Val Phe Phe Ala Gly Thr
   1               5                   10                  15
   Ala Leu Ser Glu Ser Ile Ser Glu Asn Leu Ala Trp Asn Lys Glu Phe
               20                  25                  30
   Ser Ser Glu Ser Val His Gly Val Phe Val Leu Cys Lys Ser Ser Ser
           35                  40                  45
   Asn Ser Cys Thr Thr Asn Asn Ala Thr Arg Ala Ser Thr Ala Tyr Ile
       50                  55                  60
   Pro Ala Ser Thr Phe Lys Ile Pro Asn Ala Leu Ile Gly Leu Glu Thr
   65                  70                  75                  80
   Gly Ala Ile Lys Asp Ala Arg Gln Val Phe Lys Trp Asp Gly Lys Pro
                   85                  90                  95
   Arg Ala Met Lys Gln Trp Glu Lys Asp Leu Thr Leu Arg Gly Ala Ile
               100                 105                 110
   Gln Val Ser Ala Val Pro Val Phe Gln Gln Ile Ala Arg Asp Ile Gly
           115                 120                 125
   Lys Lys Arg Met Gln Lys Tyr Leu Asn Leu Phe Ser Tyr Gly Asn Ala
       130                 135                 140
   Asn Ile Gly Gly Gly Ile Asp Lys Phe Trp Leu Glu Gly Gln Leu Arg
   145                 150                 155                 160
   Ile Ser Ala Val Asn Gln Val Lys Phe Leu Glu Ser Leu Tyr Leu Asn
                   165                 170                 175
   Asn Leu Pro Ala Ser Lys Ala Asn Gln Leu Ile Val Lys Glu Ala Ile
               180                 185                 190
   Val Thr Glu Ala Thr Pro Glu Tyr Ile Val His Ser Lys Thr Gly Tyr
           195                 200                 205
   Ser Gly Val Gly Thr Glu Ser Asn Pro Gly Val Ala Trp Trp Val Gly
       210                 215                 220
   Trp Val Glu Lys Gly Thr Glu Val Tyr Phe Phe Ala Phe Asn Met Asp
   225                 230                 235                 240
   Ile Asp Asn Glu Ser Lys Leu Pro Ser Arg Lys Ser Ile Pro Thr Lys
                   245                 250                 255
   Ile Met Ala Ser Glu Gly Ile Ile Ile Gly Gly
               260                 265
<210> 506
   <211> 275
   <212> PRT
   <213> RESISTANCE GENE
<400> 506
   Met Lys Lys Phe Ile Leu Pro Ile Phe Ser Ile Ser Ile Leu Val Ser
   1               5                   10                  15
   Leu Ser Ala Cys Ser Ser Ile Lys Thr Lys Ser Glu Asp Asn Phe His
               20                  25                  30
   Ile Ser Ser Gln Gln His Glu Lys Ala Ile Lys Ser Tyr Phe Asp Glu
           35                  40                  45
   Ala Gln Thr Gln Gly Val Ile Ile Ile Lys Glu Gly Lys Asn Leu Ser
       50                  55                  60
   Thr Tyr Gly Asn Ala Leu Ala Arg Ala Asn Lys Glu Tyr Val Pro Ala
   65                  70                  75                  80
   Ser Thr Phe Lys Met Leu Ile Ala Leu Ile Gly Leu Glu Asn His Lys
                   85                  90                  95
   Ala Thr Thr Asn Glu Ile Phe Lys Trp Asp Gly Lys Lys Arg Thr Tyr
                   100                 105                 110
   Pro Met Trp Glu Lys Asp Met Thr Leu Gly Glu Ala Met Ala Leu Ser
               115                 120                 125
   Ala Val Pro Val Tyr Gln Glu Leu Ala Arg Arg Thr Gly Leu Glu Leu
       130                 135                 140
   Met Gln Lys Glu Val Lys Arg Val Asn Phe Gly Asn Thr Asn Ile Gly
   145                 150                 155                 160
   Thr Gln Val Asp Asn Phe Trp Leu Val Gly Pro Leu Lys Ile Thr Pro
                   165                 170                 175
   Val Gln Glu Val Asn Phe Ala Asp Asp Leu Ala His Asn Arg Leu Pro
               180                 185                 190
   Phe Lys Leu Glu Thr Gln Glu Glu Val Lys Lys Met Leu Leu Ile Lys
           195                 200                 205
   Glu Val Asn Gly Ser Lys Ile Tyr Ala Lys Ser Gly Trp Gly Met Gly
       210                 215                 220
   Val Thr Pro Gln Val Gly Trp Leu Thr Gly Trp Val Glu Gln Ala Asn
   225                 230                 235                 240
   Gly Lys Lys Ile Pro Phe Ser Leu Asn Leu Glu Met Lys Glu Gly Met
                   245                 250                 255
   Ser Gly Ser Ile Arg Asn Glu Ile Thr Tyr Lys Ser Leu Glu Asn Leu
               260                 265                 270
   Gly Ile Ile
           275
<210> 507
   <211> 274
   <212> PRT
   <213> RESISTANCE GENE
<400> 507
   Met Asn Ile Lys Ala Leu Leu Leu Ile Thr Ser Ala Ile Phe Ile Ser
    1               5                  10                  15
   Ala Cys Ser Pro Tyr Ile Val Thr Ala Asn Pro Asn His Ser Ala Ser
               20                  25                  30
   Lys Ser Asp Asp Lys Ala Glu Lys Ile Lys Asn Leu Phe Asn Glu Ala
           35                  40                  45
   His Thr Thr Gly Val Leu Val Ile His Gln Gly Gln Thr Gln Gln Ser
       50                  55                  60
   Tyr Gly Asn Asp Leu Ala Arg Ala Ser Thr Glu Tyr Val Pro Ala Ser
    65                 70                  75                  80
   Thr Phe Lys Met Leu Asn Ala Leu Ile Gly Leu Glu His His Lys Ala
                   85                  90                  95
   Thr Thr Thr Glu Val Phe Lys Trp Asn Gly Gln Lys Arg Leu Phe Pro
               100                 105                 110
   Glu Trp Glu Lys Asp Met Thr Leu Gly Asp Ala Met Lys Ala Ser Ala
           115                 120                 125
   Ile Pro Val Tyr Gln Asp Leu Ala Arg Arg Ile Gly Leu Glu Leu Met
       130                 135                 140
   Ser Asn Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
                   260                 265                 270
   Ile Leu
<210> 508
   <211> 274
   <212> PRT
   <213> RESISTANCE GENE
<400> 508
   Met Asn Ile Lys Ala Leu Leu Leu Ile Thr Ser Ala Ile Ser Ile Ser
    1               5                  10                  15
   Ala Cys Ser Pro Tyr Ile Val Thr Ala Asn Pro Asn His Ser Ala Ser
               20                  25                  30
   Lys Ser Asp Glu Lys Ala Glu Lys Ile Lys Asn Leu Phe Asn Glu Ala
           35                  40                  45
   His Thr Thr Gly Val Leu Val Ile Gln Gln Gly Gln Thr Gln Gln Ser
       50                  55                  60
   Tyr Gly Asn Asp Leu Ala Arg Ala Ser Thr Glu Tyr Val Pro Ala Ser
   65                  70                  75                  80
   Thr Phe Lys Met Leu Asn Ala Leu Ile Gly Leu Glu His His Lys Ala
                   85                  90                  95
   Thr Thr Thr Glu Val Phe Lys Trp Asp Gly Gln Lys Arg Leu Phe Pro
               100                 105                 110
   Glu Trp Glu Lys Asp Met Thr Leu Gly Asp Ala Ile Lys Ala Ser Ala
           115                 120                 125
   Ile Pro Val Tyr Gln Asp Leu Ala Arg Arg Ile Gly Leu Glu Leu Met
       130                 135                 140
   Ser Lys Glu Val Lys Arg Val Gly Tyr Gly Asn Ala Asp Ile Gly Thr
   145                 150                 155                 160
   Gln Val Asp Asn Phe Trp Leu Val Gly Pro Leu Lys Ile Thr Pro Gln
                   165                 170                 175
   Gln Glu Ala Gln Phe Ala Tyr Lys Leu Ala Asn Lys Thr Leu Pro Phe
               180                 185                 190
   Ser Gln Lys Val Gln Asp Glu Val Gln Ser Met Leu Phe Ile Glu Glu
           195                 200                 205
   Lys Asn Gly Asn Lys Ile Tyr Ala Lys Ser Gly Trp Gly Trp Asp Val
       210                 215                 220
   Asp Pro Gln Val Gly Trp Leu Thr Gly Trp Val Val Gln Pro Gln Gly
   225                 230                 235                 240
   Asn Ile Val Ala Phe Ser Leu Asn Leu Glu Met Lys Lys Gly Ile Pro
                   245                 250                 255
   Ser Ser Val Arg Lys Glu Ile Thr Tyr Lys Ser Leu Glu Gln Leu Gly
               260                 265                 270
   Ile Leu
<210> 509
   <211> 20
   <212> PRT
   <213> RESISTANCE GENE
<400> 509
   Ala Ala Ala Tyr Glu Leu Ala Glu Asn Leu Phe Glu Ala Gly Gln Ala
    1               5                  10                  15
   Asp Gly Trp Arg
               20
<210> 510
   <211> 12
   <212> PRT
   <213> RESISTANCE GENE
<400> 510
   Ala Ala Glu Gly Phe Ile Pro Ala Ser Thr Phe Lys
    1               5                  10
<210> 511
   <211> 5
   <212> PRT
   <213> RESISTANCE GENE
<400> 511
   Ala Ala Leu Gly Arg
   1 5
<210> 512
   <211> 15
   <212> PRT
   <213> RESISTANCE GENE
<400> 512
   Ala Asp Gly Gln Val Val Ala Phe Ala Leu Asn Met Gln Met Lys
   1 5 10 15
<210> 513
   <211> 8
   <212> PRT
   <213> RESISTANCE GENE
<400> 513
   Ala Asp Ile Asn Glu Ile Phe Lys
   1 5
<210> 514
   <211> 5
   <212> PRT
   <213> RESISTANCE GENE
<400> 514
   Ala Asp Trp Gly Lys
   1 5
<210> 515
   <211> 11
   <212> PRT
   <213> RESISTANCE GENE
<400> 515
   Ala Glu Gly Ala Ile Val Ile Ser Asp Glu Arg
   1 5 10
<210> 516
   <211> 10
   <212> PRT
   <213> RESISTANCE GENE
<400> 516
   Ala Phe Ala Leu Asn Leu Asp Ile Asp Lys
   1 5 10
<210> 517
   <211> 9
   <212> PRT
   <213> RESISTANCE GENE
<400> 517
   Ala Phe Ala Pro Met Ser Thr Phe Lys
   1 5
<210> 518
   <211> 21
   <212> PRT
   <213> RESISTANCE GENE
<400> 518
   Ala Phe Gly Tyr Gly Asn Ala Asp Val Ser Gly Asp Pro Gly Gln Asn
   1 5 10 15
   Asn Gly Leu Asp Arg
   20
<210> 519
   <211> 6
   <212> PRT
   <213> RESISTANCE GENE
<400> 519
   Ala Phe Thr Met Thr Lys
   1 5
<210> 520
   <211> 8
   <212> PRT
   <213> RESISTANCE GENE
<400> 520
   Ala Gly Asp Asp Ile Ala Leu Arg
   1 5
<210> 521
   <211> 15
   <212> PRT
   <213> RESISTANCE GENE
<400> 521
   Ala Gly His Val Tyr Ala Phe Ala Leu Asn Ile Asp Met Pro Arg
   1 5 10 15
<210> 522
   <211> 5
   <212> PRT
   <213> RESISTANCE GENE
<400> 522
   Ala Gly Leu Trp Arg
   1 5
<210> 523
   <211> 12
   <212> PRT
   <213> RESISTANCE GENE
<400> 523
   Ala His Thr Glu Tyr Val Pro Ala Ser Thr Phe Lys
   1 5 10
<210> 524
   <211> 8
   <212> PRT
   <213> RESISTANCE GENE
<400> 524
   Ala Ile Ile Pro Trp Asp Gly Lys
   1 5
<210> 525
   <211> 10
   <212> PRT
   <213> RESISTANCE GENE
<400> 525
   Ala Ile Ile Pro Trp Asp Gly Lys Pro Arg
   1 5 10
<210> 526
   <211> 9
   <212> PRT
   <213> RESISTANCE GENE
<400> 526
   Ala Ile Ser Asp Ile Thr Ile Thr Arg
   1 5
<210> 527
   <211> 5
   <212> PRT
   <213> RESISTANCE GENE
<400> 527
   Ala Ile Ser Gly Lys
   1 5
<210> 528
   <211> 6
   <212> PRT
   <213> RESISTANCE GENE
<400> 528
   Ala Leu Gly Gln Asp Arg
   1 5
<210> 529
   <211> 7
   <212> PRT
   <213> RESISTANCE GENE
<400> 529
   Ala Leu Pro Asp Leu Ala Lys
   1 5
<210> 530
   <211> 5
   <212> PRT
   <213> RESISTANCE GENE
<400> 530
   Ala Leu Gln Ala Lys
   1 5
<210> 531
   <211> 12
   <212> PRT
   <213> RESISTANCE GENE
<400> 531
   Ala Met Glu Thr Phe Ser Pro Ala Ser Thr Phe Lys
   1 5 10
<210> 532
   <211> 8
   <212> PRT
   <213> RESISTANCE GENE
<400> 532
   Ala Met Leu Phe Leu Gln Glu Arg
   1 5
<210> 533
   <211> 9
   <212> PRT
   <213> RESISTANCE GENE
<400> 533
   Ala Met Leu Val Phe Asp Pro Val Arg
   1 5
<210> 534
   <211> 16
   <212> PRT
   <213> RESISTANCE GENE
<400> 534
   Ala Met Thr Leu Leu Glu Ser Gly Pro Gly Trp Glu Leu His Gly Lys
   1               5                   10                  15
<210> 535
   <211> 10
   <212> PRT
   <213> RESISTANCE GENE
<400> 535
   Ala Asn Leu His Ile Thr Leu His Gly Lys
   1               5                   10
<210> 536
   <211> 7
   <212> PRT
   <213> RESISTANCE GENE
<400> 536
   Ala Asn Gln Leu Ile Val Lys
   1               5
<210> 537
   <211> 12
   <212> PRT
   <213> RESISTANCE GENE
<400> 537
   Ala Asn Thr Glu Tyr Val Pro Ala Ser Thr Phe Lys
   1               5                   10
<210> 538
   <211> 5
   <212> PRT
   <213> RESISTANCE GENE
<400> 538
   Ala Asn Val Ser Arg
   1               5
<210> 539
   <211> 12
   <212> PRT
   <213> RESISTANCE GENE
<400> 539
   Ala Pro Ile Gly Trp Phe Ile Gly Trp Ala Thr Arg
   1               5                   10
<210> 540
   <211> 15
   <212> PRT
   <213> RESISTANCE GENE
<400> 540
   Ala Pro Leu Gly Trp Phe Ile Gly Trp Ala Thr His Glu Glu Arg
   1 5 10 15
<210> 541
   <211> 14
   <212> PRT
   <213> RESISTANCE GENE
<400> 541
   Ala Gln Asp Glu Val Gln Ser Met Leu Phe Ile Glu Glu Lys
   1 5 10
<210> 542
   <211> 12
   <212> PRT
   <213> RESISTANCE GENE
<400> 542
   Ala Gln Gly Val Ile Val Leu Trp Asn Glu Asn Lys
   1 5 10
<210> 543
   <211> 12
   <212> PRT
   <213> RESISTANCE GENE
<400> 543
   Ala Ser Ala Ile Ala Val Tyr Gln Asp Leu Ala Arg
   1 5 10
<210> 544
   <211> 12
   <212> PRT
   <213> RESISTANCE GENE
<400> 544
   Ala Ser Ala Ile Leu Val Tyr Gln Asp Leu Ala Arg
   1 5 10
<210> 545
   <211> 12
   <212> PRT
   <213> RESISTANCE GENE
<400> 545
   Ala Ser Ala Ile Pro Val Tyr Gln Asp Leu Ala Arg
   1 5 10
<210> 546
   <211> 12
   <212> PRT
   <213> RESISTANCE GENE
<400> 546
   Ala Ser Ala Ile Pro Val Tyr Gln Asp Leu Pro Arg
   1 5 10
<210> 547
   <211> 12
   <212> PRT
   <213> RESISTANCE GENE
<400> 547
   Ala Ser Ala Ile Gln Val Tyr Gln Asp Leu Ala Arg
   1 5 10
<210> 548
   <211> 12
   <212> PRT
   <213> RESISTANCE GENE
<400> 548
   Ala Ser Ala Ile Ser Val Tyr Gln Asp Leu Ala Arg
   1 5 10
<210> 549
   <211> 12
   <212> PRT
   <213> RESISTANCE GENE
<400> 549
   Ala Ser Ala Leu Pro Val Tyr Gln Asp Leu Ala Arg
   1 5 10
<210> 550
   <211> 12
   <212> PRT
   <213> RESISTANCE GENE
<400> 550
   Ala Ser Ala Met Pro Val Tyr Gln Asp Leu Ala Arg
   1 5 10
<210> 551
   <211> 12
   <212> PRT
   <213> RESISTANCE GENE
<400> 551
   Ala Ser Ala Val Pro Val Tyr Gln Asp Leu Ala Arg
   1 5 10
<210> 552
   <211> 12
   <212> PRT
   <213> RESISTANCE GENE
<400> 552
   Ala Ser Ile Glu Tyr Val Pro Ala Ser Thr Phe Lys
   1 5 10
<210> 553
   <211> 12
   <212> PRT
   <213> RESISTANCE GENE
<400> 553
   Ala Ser Asn Val Pro Val Tyr Gln Glu Leu Ala Arg
   1 5 10
<210> 554
   <211> 8
   <212> PRT
   <213> RESISTANCE GENE
<400> 554
   Ala Ser Pro Ala Ser Thr Phe Lys
   1 5
<210> 555
   <211> 12
   <212> PRT
   <213> RESISTANCE GENE
<400> 555
   Ala Ser Thr Ala Tyr Ile Pro Ala Ser Thr Phe Lys
   1 5 10
<210> 556
   <211> 12
   <212> PRT
   <213> RESISTANCE GENE
<400> 556
   Ala Ser Thr Glu Tyr Val Pro Ala Ser Thr Phe Lys
   1 5 10
<210> 557
   <211> 8
   <212> PRT
   <213> RESISTANCE GENE
<400> 557
   Ala Ser Thr Thr Glu Val Phe Lys
   1 5
<210> 558
   <211> 8
   <212> PRT
   <213> RESISTANCE GENE
<400> 558
   Ala Thr Ser Thr Glu Ile Phe Lys
   1 5
<210> 559
   <211> 8
   <212> PRT
   <213> RESISTANCE GENE
<400> 559
   Ala Thr Thr Asn Glu Ile Phe Lys
   1 5
<210> 560
   <211> 8
   <212> PRT
   <213> RESISTANCE GENE
<400> 560
   Ala Thr Thr Thr Ala Val Phe Lys
   1 5
<210> 561
   <211> 8
   <212> PRT
   <213> RESISTANCE GENE
<400> 561
   Ala Thr Thr Thr Glu Ile Phe Lys
   1 5
<210> 562
   <211> 8
   <212> PRT
   <213> RESISTANCE GENE
<400> 562
   Ala Thr Thr Thr Glu Val Phe Lys
   1 5
<210> 563
   <211> 19
   <212> PRT
   <213> RESISTANCE GENE
<400> 563
   Ala Val Ser Asp Ile Thr Ile Leu Glu Gln Thr Asp Asn Tyr Thr Leu
    1               5                  10                  15
   His Gly Lys
<210> 564
   <211> 19
   <212> PRT
   <213> RESISTANCE GENE
<400> 564
   Ala Val Ser Asp Ile Thr Ile Leu Glu Gln Thr Tyr Asn Tyr Thr Leu
    1               5                  10                  15
   His Gly Lys
<210> 565
   <211> 16
   <212> PRT
   <213> RESISTANCE GENE
<400> 565
   Ala Val Val Pro His Phe Glu Ala Gly Asp Trp Asp Val Gln Gly Lys
    1               5                  10                  15
<210> 566
   <211> 9
   <212> PRT
   <213> RESISTANCE GENE
<400> 566
   Ala Trp Glu His Asp Met Ser Leu Arg
    1               5
<210> 567
   <211> 20
   <212> PRT
   <213> RESISTANCE GENE
<400> 567
   Ala Trp Ile Gly Ser Ser Leu Gln Ile Ser Pro Leu Glu Gln Leu Glu
    1               5                  10                  15
   Phe Leu Gly Lys
               20
<210> 568
   <211> 12
   <212> PRT
   <213> RESISTANCE GENE
<400> 568
   Cys Ala Ala Gln Met Ala Pro Asp Ser Thr Phe Lys
   1 5 10
<210> 569
   <211> 12
   <212> PRT
   <213> RESISTANCE GENE
<400> 569
   Cys Ala Thr Gln Met Ala Pro Asp Ser Thr Phe Lys
   1 5 10
<210> 570
   <211> 21
   <212> PRT
   <213> RESISTANCE GENE
<400> 570
   Cys Thr Ile Ile Ala Asp Ala Ile Thr Gly Asn Thr Leu Tyr Glu Thr
   1 5 10 15
   Gly Glu Cys Ala Arg
   20
<210> 571
   <211> 5
   <212> PRT
   <213> RESISTANCE GENE
<400> 571
   Asp Ala Phe Leu Lys
   1 5
<210> 572
   <211> 8
   <212> PRT
   <213> RESISTANCE GENE
<400> 572
   Asp Asp Phe Ile Leu His Gly Lys
   1 5
<210> 573
   <211> 11
   <212> PRT
   <213> RESISTANCE GENE
<400> 573
   Asp Asp Gln Glu Val Leu Pro Tyr Gly Gly Lys
   1 5 10
<210> 574
   <211> 5
   <212> PRT
   <213> RESISTANCE GENE
<400> 574
   Asp Asp Val Leu Lys
   1 5
<210> 575
   <211> 7
   <212> PRT
   <213> RESISTANCE GENE
<400> 575
   Asp Glu Phe His Val Phe Arg
   1 5
<210> 576
   <211> 7
   <212> PRT
   <213> RESISTANCE GENE
<400> 576
   Asp Glu Phe Gln Ile Phe Arg
   1 5
<210> 577
   <211> 7
   <212> PRT
   <213> RESISTANCE GENE
<400> 577
   Asp Glu Phe Gln Val Phe Arg
   1 5
<210> 578
   <211> 5
   <212> PRT
   <213> RESISTANCE GENE
<400> 578
   Asp Glu Leu Val Arg
   1 5
<210> 579
   <211> 5
   <212> PRT
   <213> RESISTANCE GENE
<400> 579
   Asp Glu Thr Ser Arg
   1 5
<210> 580
   <211> 13
   <212> PRT
   <213> RESISTANCE GENE
<400> 580
   Asp Phe Asp Tyr Gly Asn Gln Asp Phe Ser Gly Asp Lys
    1               5                   10
<210> 581
   <211> 21
   <212> PRT
   <213> RESISTANCE GENE
<400> 581
   Asp Phe Thr Leu Gly Glu Ala Met Gln Ala Ser Thr Val Pro Val Tyr
    1               5                   10                  15
   Gln Glu Leu Ala Arg
                20
<210> 582
   <211> 21
   <212> PRT
   <213> RESISTANCE GENE
<400> 582
   Asp Gly Asn Ile Thr Ser Val Ala Ile Asn Ile Asp Met Leu Ser Glu
    1               5                   10                  15
   Ala Asp Ala Pro Lys
                20
<210> 583
   <211> 9
   <212> PRT
   <213> RESISTANCE GENE
<400> 583
   Asp His Asp Leu Ile Thr Ala Met Lys
    1               5
<210> 584
   <211> 7
   <212> PRT
   <213> RESISTANCE GENE
<400> 584
   Asp Ile Ala Ala Trp Asn Arg
    1               5
<210> 585
   <211> 6
   <212> PRT
   <213> RESISTANCE GENE
<400> 585
   Asp Ile Gly Glu Asp Lys
   1 5
<210> 586
   <211> 13
   <212> PRT
   <213> RESISTANCE GENE
<400> 586
   Asp Ile Leu Tyr Ile Gln Glu Leu Ala Gly Gly Trp Lys
   1 5 10
<210> 587
   <211> 7
   <212> PRT
   <213> RESISTANCE GENE
<400> 587
   Asp Ile Thr Ile Leu Glu Lys
   1 5
<210> 588
   <211> 6
   <212> PRT
   <213> RESISTANCE GENE
<400> 588
   Asp Leu Leu Ser Ala Lys
   1 5
<210> 589
   <211> 9
   <212> PRT
   <213> RESISTANCE GENE
<400> 589
   Asp Leu Met Ile Thr Glu Ala Gly Arg
   1 5
<210> 590
   <211> 9
   <212> PRT
   <213> RESISTANCE GENE
<400> 590
   Asp Leu Met Ile Val Glu Ala Gly Arg
   1 5
<210> 591
   <211> 8
   <212> PRT
   <213> RESISTANCE GENE
<400> 591
   Asp Leu Met Ile Val Glu Ala Lys
   1 5
<210> 592
   <211> 5
   <212> PRT
   <213> RESISTANCE GENE
<400> 592
   Asp Leu Pro Leu Arg
   1 5
<210> 593
   <211> 8
   <212> PRT
   <213> RESISTANCE GENE
<400> 593
   Asp Leu Ser Gly Asn Pro Gly Lys
   1 5
<210> 594
   <211> 5
   <212> PRT
   <213> RESISTANCE GENE
<400> 594
   Asp Leu Ser Leu Arg
   1 5
<210> 595
   <211> 5
   <212> PRT
   <213> RESISTANCE GENE
<400> 595
   Asp Leu Thr Leu Arg
   1 5
<210> 596
   <211> 9
   <212> PRT
   <213> RESISTANCE GENE
<400> 596
   Asp Met Thr Leu Gly Asp Ala Ile Lys
   1 5
<210> 597
   <211> 21
   <212> PRT
   <213> RESISTANCE GENE
<400> 597
   Asp Met Thr Leu Gly Asp Ala Met Ala Leu Ser Ala Val Pro Val Tyr
    1               5                  10                  15
   Gln Glu Leu Ala Arg
                20
<210> 598
   <211> 9
   <212> PRT
   <213> RESISTANCE GENE
<400> 598
   Asp Met Thr Leu Gly Asp Ala Met Lys
    1               5
<210> 599
   <211> 21
   <212> PRT
   <213> RESISTANCE GENE
<400> 599
   Asp Met Thr Leu Gly Glu Ala Met Ala Leu Ser Ala Val Pro Val Tyr
    1               5                  10                  15
   Gln Asp Leu Ala Arg
                20
<210> 600
   <211> 21
   <212> PRT
   <213> RESISTANCE GENE
<400> 600
   Asp Met Thr Leu Gly Glu Ala Met Ala Leu Ser Ala Val Pro Val Tyr
    1               5                  10                  15
   Gln Glu Leu Ala Arg
                20
<210> 601
   <211> 9
   <212> PRT
   <213> RESISTANCE GENE
<400> 601
   Asp Met Thr Leu Gly Glu Ala Met Lys
    1               5
<210> 602
   <211> 21
   <212> PRT
   <213> RESISTANCE GENE
<400> 602
   Asp Met Thr Leu Gly Gln Ala Met Gln Ala Ser Ala Val Pro Val Tyr
   1 5 10 15
   Gln Glu Leu Ala Arg
   20
<210> 603
   <211> 5
   <212> PRT
   <213> RESISTANCE GENE
<400> 603
   Asp Asn Asn Gly Lys
   1 5
<210> 604
   <211> 5
   <212> PRT
   <213> RESISTANCE GENE
<400> 604
   Asp Gln Asp Leu Arg
   1 5
<210> 605
   <211> 13
   <212> PRT
   <213> RESISTANCE GENE
<400> 605
   Asp Gln Gln Ile Gly Trp Phe Val Gly Trp Ala Ser Lys
   1 5 10
<210> 606
   <211> 16
   <212> PRT
   <213> RESISTANCE GENE
<400> 606
   Asp Gln Gln Ile Gly Trp Phe Val Gly Trp Ala Ser Lys Pro Gly Lys
   1 5 10 15
<210> 607
   <211> 13
   <212> PRT
   <213> RESISTANCE GENE
<400> 607
   Asp Gln Gln Val Gln Val Tyr Gly Asn Asp Leu Asn Arg
   1 5 10
<210> 608
   <211> 5
   <212> PRT
   <213> RESISTANCE GENE
<400> 608
   Asp Gln Ser Phe Arg
   1 5
<210> 609
   <211> 9
   <212> PRT
   <213> RESISTANCE GENE
<400> 609
   Asp Gln Thr Leu Glu Ser Ala Phe Lys
   1 5
<210> 610
   <211> 18
   <212> PRT
   <213> RESISTANCE GENE
<400> 610
   Asp Ser Cys Val Trp Tyr Ser Gln Val Leu Thr Gln Gln Leu Gly Met
   1 5 10 15
   Thr Arg
<210> 611
   <211> 12
   <212> PRT
   <213> RESISTANCE GENE
<400> 611
   Asp Ser Ile Val Trp Tyr Ser Gln Glu Leu Thr Arg
   1 5 10
<210> 612
   <211> 12
   <212> PRT
   <213> RESISTANCE GENE
<400> 612
   Asp Ser Ile Val Trp Tyr Ser Gln Gln Leu Thr Arg
   1 5 10
<210> 613
   <211> 5
   <212> PRT
   <213> RESISTANCE GENE
<400> 613
   Asp Ser Asn Leu Arg
   1 5
<210> 614
   <211> 12
   <212> PRT
   <213> RESISTANCE GENE
<400> 614
   Asp Ser Tyr Ile Ala Trp Gly Gly Glu Ala Trp Lys
   1 5 10
<210> 615
   <211> 10
   <212> PRT
   <213> RESISTANCE GENE
<400> 615
   Asp Thr Leu Asn Pro Glu Trp Pro Tyr Lys
   1 5 10
<210> 616
   <211> 8
   <212> PRT
   <213> RESISTANCE GENE
<400> 616
   Asp Val Asp Glu Val Phe Tyr Lys
   1 5
<210> 617
   <211> 8
   <212> PRT
   <213> RESISTANCE GENE
<400> 617
   Asp Val Ser Gly Asp Pro Gly Lys
   1 5
<210> 618
   <211> 5
   <212> PRT
   <213> RESISTANCE GENE
<400> 618
   Asp Trp Ile Leu Arg
   1 5
<210> 619
   <211> 10
   <212> PRT
   <213> RESISTANCE GENE
<400> 619
   Asp Trp Pro Ala Met Ala Gly Ala Trp Arg
   1 5 10
<210> 620
   <211> 5
   <212> PRT
   <213> RESISTANCE GENE
<400> 620
   Glu Ala Phe Leu Arg
   1 5
<210> 621
   <211> 5
   <212> PRT
   <213> RESISTANCE GENE
<400> 621
   Glu Ala Ile Ala Arg
   1 5
<210> 622
   <211> 5
   <212> PRT
   <213> RESISTANCE GENE
<400> 622
   Glu Ala Ile Val Arg
   1 5
<210> 623
   <211> 16
   <212> PRT
   <213> RESISTANCE GENE
<400> 623
   Glu Ala Ile Val Thr Glu Ala Thr Pro Glu Tyr Ile Val His Ser Lys
   1 5 10 15
<210> 624
   <211> 16
   <212> PRT
   <213> RESISTANCE GENE
<400> 624
   Glu Ala Leu Val Thr Glu Ala Ala Pro Glu Tyr Leu Val His Ser Lys
   1 5 10 15
<210> 625
   <211> 15
   <212> PRT
   <213> RESISTANCE GENE
<400> 625
   Glu Ala Leu Val Thr Glu Ala Pro Glu Tyr Leu Val His Ser Lys
   1 5 10 15
<210> 626
   <211> 5
   <212> PRT
   <213> RESISTANCE GENE
<400> 626
   Glu Glu Ile Val Arg
   1 5
<210> 627
   <211> 12
   <212> PRT
   <213> RESISTANCE GENE
<400> 627
   Glu Glu Val Leu Ala Ala Leu Pro Ala Gln Leu Lys
   1 5 10
<210> 628
   <211> 6
   <212> PRT
   <213> RESISTANCE GENE
<400> 628
   Glu Phe Asn Gly Ser Lys
   1 5
<210> 629
   <211> 15
   <212> PRT
   <213> RESISTANCE GENE
<400> 629
   Glu Phe Ser Ala Glu Ala Val Asn Gly Val Phe Val Leu Cys Lys
   1 5 10 15
<210> 630
   <211> 15
   <212> PRT
   <213> RESISTANCE GENE
<400> 630
   Glu Phe Ser Ser Glu Ser Val His Gly Val Phe Val Leu Cys Lys
   1 5 10 15
<210> 631
   <211> 6
   <212> PRT
   <213> RESISTANCE GENE
<400> 631
   Glu Gly Asp Met Ala Lys
   1 5
<210> 632
   <211> 8
   <212> PRT
   <213> RESISTANCE GENE
<400> 632
   Glu Gly Met Ser Gly Ser Ile Arg
   1 5
<210> 633
   <211> 8
   <212> PRT
   <213> RESISTANCE GENE
<400> 633
   Glu Gly Met Thr Gly Ser Ile Arg
   1 5
<210> 634
   <211> 6
   <212> PRT
   <213> RESISTANCE GENE
<400> 634
   Glu Gly Ser Cys Asp Lys
   1 5
<210> 635
   <211> 7
   <212> PRT
   <213> RESISTANCE GENE
<400> 635
   Glu Ile Ala Val Trp Asn Arg
   1 5
<210> 636
   <211> 5
   <212> PRT
   <213> RESISTANCE GENE
<400> 636
   Glu Ile Ala Tyr Lys
   1 5
<210> 637
   <211> 5
   <212> PRT
   <213> RESISTANCE GENE
<400> 637
   Glu Ile Phe Glu Arg
   1 5
<210> 638
   <211> 7
   <212> PRT
   <213> RESISTANCE GENE
<400> 638
   Glu Ile Phe Tyr His Tyr Arg
   1 5
<210> 639
   <211> 6
   <212> PRT
   <213> RESISTANCE GENE
<400> 639
   Glu Ile Gly Asp Asp Lys
   1 5
<210> 640
   <211> 6
   <212> PRT
   <213> RESISTANCE GENE
<400> 640
   Glu Ile Gly Asp Gly Lys
   1 5
<210> 641
   <211> 6
   <212> PRT
   <213> RESISTANCE GENE
<400> 641
   Glu Ile Gly Glu Asp Lys
   1 5
<210> 642
   <211> 8
   <212> PRT
   <213> RESISTANCE GENE
<400> 642
   Glu Ile Gly Glu Asp Asn Ala Arg
   1 5
<210> 643
   <211> 6
   <212> PRT
   <213> RESISTANCE GENE
<400> 643
   Glu Ile Gly Glu Asn Lys
   1 5
<210> 644
   <211> 5
   <212> PRT
   <213> RESISTANCE GENE
<400> 644
   Glu Ile Gly Pro Lys
   1 5
<210> 645
   <211> 8
   <212> PRT
   <213> RESISTANCE GENE
<400> 645
   Glu Ile Gly Ser Glu Ile Asp Lys
   1 5
<210> 646
   <211> 5
   <212> PRT
   <213> RESISTANCE GENE
<400> 646
   Glu Ile Thr Tyr Lys
   1 5
<210> 647
   <211> 5
   <212> PRT
   <213> RESISTANCE GENE
<400> 647
   Glu Ile Thr Tyr Arg
   1 5
<210> 648
   <211> 6
   <212> PRT
   <213> RESISTANCE GENE
<400> 648
   Glu Met Ile Tyr Leu Lys
   1 5
<210> 649
   <211> 7
   <212> PRT
   <213> RESISTANCE GENE
<400> 649
   Glu Met Leu Tyr Val Glu Arg
   1 5
<210> 650
   <211> 5
   <212> PRT
   <213> RESISTANCE GENE
<400> 650
   Glu Met Thr Tyr Lys
   1 5
<210> 651
   <211> 5
   <212> PRT
   <213> RESISTANCE GENE
<400> 651
   Glu Asn Ile Glu Lys
   1 5
<210> 652
   <211> 7
   <212> PRT
   <213> RESISTANCE GENE
<400> 652
   Glu Asn Gln Leu Ile Val Lys
   1 5
<210> 653
   <211> 8
   <212> PRT
   <213> RESISTANCE GENE
<400> 653
   Glu Gln Ala Ile Leu Leu Phe Arg
   1 5
<210> 654
   <211> 8
   <212> PRT
   <213> RESISTANCE GENE
<400> 654
   Glu Gln Ile Gln Phe Leu Leu Arg
   1 5
<210> 655
   <211> 14
   <212> PRT
   <213> RESISTANCE GENE
<400> 655
   Glu Gln Leu Ala Phe Asp Pro Gln Val Gln Gln Gln Val Lys
   1 5 10
<210> 656
   <211> 8
   <212> PRT
   <213> RESISTANCE GENE
<400> 656
   Glu Gln Val Asp Phe Val Gln Arg
   1 5
<210> 657
   <211> 18
   <212> PRT
   <213> RESISTANCE GENE
<400> 657
   Glu Thr Glu Val Tyr Phe Phe Ala Phe Asn Met Asp Ile Asp Asn Glu
   1 5 10 15
   Ser Lys
<210> 658
   <211> 6
   <212> PRT
   <213> RESISTANCE GENE
<400> 658
   Glu Thr Thr Thr Pro Arg
   1 5
<210> 659
   <211> 6
   <212> PRT
   <213> RESISTANCE GENE
<400> 659
   Glu Val Gly Glu Ile Arg
   1 5
<210> 660
   <211> 6
   <212> PRT
   <213> RESISTANCE GENE
<400> 660
   Glu Val Gly Glu Val Arg
   1 5
<210> 661
   <211> 6
   <212> PRT
   <213> RESISTANCE GENE
<400> 661
   Glu Val Asn Gly Ser Lys
   1 5
<210> 662
   <211> 6
   <212> PRT
   <213> RESISTANCE GENE
<400> 662
   Glu Trp Gln Glu Asn Lys
   1 5
<210> 663
   <211> 10
   <212> PRT
   <213> RESISTANCE GENE
<400> 663
   Glu Tyr Glu Leu Tyr Leu Asn Val Trp Lys
   1 5 10
<210> 664
   <211> 9
   <212> PRT
   <213> RESISTANCE GENE
<400> 664
   Glu Tyr Leu Pro Ala Ser Thr Phe Lys
   1 5
<210> 665
   <211> 9
   <212> PRT
   <213> RESISTANCE GENE
<400> 665
   Glu Tyr Leu Pro Val Ser Thr Phe Lys
   1 5
<210> 666
   <211> 14
   <212> PRT
   <213> RESISTANCE GENE
<400> 666
   Glu Tyr Asn Thr Ser Gly Thr Phe Val Phe Tyr Asp Gly Lys
   1 5 10
<210> 667
   <211> 9
   <212> PRT
   <213> RESISTANCE GENE
<400> 667
   Glu Tyr Val Pro Ala Ser Thr Phe Lys
   1 5
<210> 668
   <211> 21
   <212> PRT
   <213> RESISTANCE GENE
<400> 668
   Phe Ala Ala Tyr Val Ala Gly Phe Gly Tyr Gly Asn Gly Asp Ile Ser
   1 5 10 15
   Gly Glu Pro Gly Lys
   20
<210> 669
   <211> 8
   <212> PRT
   <213> RESISTANCE GENE
<400> 669
   Phe Ala Pro Glu Ser Thr Phe Lys
   1 5
<210> 670
   <211> 6
   <212> PRT
   <213> RESISTANCE GENE
<400> 670
   Phe Ala Gln Tyr Ala Lys
   1 5
<210> 671
   <211> 6
   <212> PRT
   <213> RESISTANCE GENE
<400> 671
   Phe Asp Tyr Gly Asn Lys
   1 5
<210> 672
   <211> 6
   <212> PRT
   <213> RESISTANCE GENE
<400> 672
   Phe Asp Tyr Gly Asn Arg
   1 5
<210> 673
   <211> 6
   <212> PRT
   <213> RESISTANCE GENE
<400> 673
   Phe Glu Asp Leu Tyr Lys
   1 5
<210> 674
   <211> 11
   <212> PRT
   <213> RESISTANCE GENE
<400> 674
   Phe Glu Asp Thr Phe His Ile Ser Asn Gln Lys
   1 5 10
<210> 675
   <211> 14
   <212> PRT
   <213> RESISTANCE GENE
<400> 675
   Phe Glu Asp Thr Phe His Thr Ser Asn Gln Gln His Glu Lys
   1 5 10
<210> 676
   <211> 14
   <212> PRT
   <213> RESISTANCE GENE
<400> 676
   Phe Glu Tyr Gly Asn Gln Asp Val Ser Gly Asp Ser Gly Lys
   1 5 10
<210> 677
   <211> 8
   <212> PRT
   <213> RESISTANCE GENE
<400> 677
   Phe Phe Ser Asp Phe Gln Ala Lys
   1 5
<210> 678
   <211> 17
   <212> PRT
   <213> RESISTANCE GENE
<400> 678
   Phe Phe Ser Asp Leu Gln Ala Glu Gly Ala Ile Val Ile Ala Asp Glu
   1 5 10 15
   Arg
<210> 679
   <211> 6
   <212> PRT
   <213> RESISTANCE GENE
<400> 679
   Phe Phe Ser Asp Leu Arg
   1 5
<210> 680
   <211> 8
   <212> PRT
   <213> RESISTANCE GENE
<400> 680
   Phe Phe Ser Glu Phe Gln Ala Lys
   1 5
<210> 681
   <211> 8
   <212> PRT
   <213> RESISTANCE GENE
<400> 681
   Phe Gly Leu Glu Gly Gln Leu Arg
   1 5
<210> 682
   <211> 21
   <212> PRT
   <213> RESISTANCE GENE
<400> 682
   Phe Ile Leu Pro Ile Phe Ser Ile Ser Ile Leu Val Ser Leu Ser Ala
     1               5                  10                  15
   Cys Ser Ser Ile Lys
               20
<210> 683
   <211> 20
   <212> PRT
   <213> RESISTANCE GENE
<400> 683
   Phe Leu Ala Leu Leu Phe Ser Ala Val Val Leu Val Ser Leu Gly His
     1               5                  10                  15
   Ala Gln Asp Lys
               20
<210> 684
   <211> 20
   <212> PRT
   <213> RESISTANCE GENE
<400> 684
   Phe Leu Ala Leu Leu Phe Ser Ala Val Val Leu Val Ser Leu Gly His
     1               5                  10                  15
   Ala Gln Glu Lys
               20
<210> 685
   <211> 14
   <212> PRT
   <213> RESISTANCE GENE
<400> 685
   Phe Leu Glu Ser Leu Tyr Leu Asn Asn Leu Pro Ala Ser Lys
     1               5                  10
<210> 686
   <211> 8
   <212> PRT
   <213> RESISTANCE GENE
<400> 686
   Phe Leu Leu Glu Gly Gln Leu Arg
     1               5
<210> 687
   <211> 7
   <212> PRT
   <213> RESISTANCE GENE
<400> 687
   Phe Gln Gln Tyr Val Asp Arg
     1               5
<210> 688
   <211> 7
   <212> PRT
   <213> RESISTANCE GENE
<400> 688
   Phe Ser Asp Tyr Val Gln Arg
   1 5
<210> 689
   <211> 8
   <212> PRT
   <213> RESISTANCE GENE
<400> 689
   Phe Ser Thr Ala Ser Thr Phe Lys
   1 5
<210> 690
   <211> 6
   <212> PRT
   <213> RESISTANCE GENE
<400> 690
   Phe Ser Trp Asp Gly Lys
   1 5
<210> 691
   <211> 14
   <212> PRT
   <213> RESISTANCE GENE
<400> 691
   Phe Ser Tyr Gly Asn Gln Asn Ile Ser Gly Gly Ile Asp Lys
   1 5 10
<210> 692
   <211> 14
   <212> PRT
   <213> RESISTANCE GENE
<400> 692
   Phe Ser Tyr Gly Asn Gln Asn Ile Ser Gly Gly Thr Asp Lys
   1 5 10
<210> 693
   <211> 14
   <212> PRT
   <213> RESISTANCE GENE
<400> 693
   Phe Ser Tyr Gly Ser Gln Asn Ile Ser Gly Gly Ile Asp Lys
   1 5 10
<210> 694
   <211> 6
   <212> PRT
   <213> RESISTANCE GENE
<400> 694
   Phe Thr Glu Tyr Val Lys
   1 5
<210> 695
   <211> 5
   <212> PRT
   <213> RESISTANCE GENE
<400> 695
   Phe Val Ala His Lys
   1 5
<210> 696
   <211> 8
   <212> PRT
   <213> RESISTANCE GENE
<400> 696
   Phe Val Pro Ala Ser Thr Tyr Lys
   1 5
<210> 697
   <211> 13
   <212> PRT
   <213> RESISTANCE GENE
<400> 697
   Phe Val Tyr Asp Leu Ala Gln Gly Gln Leu Pro Phe Lys
   1 5 10
<210> 698
   <211> 21
   <212> PRT
   <213> RESISTANCE GENE
<400> 698
   Phe Val Tyr Asp Leu Ala Gln Gly Gln Leu Pro Phe Lys Pro Glu Val
   1 5 10 15
   Gln Gln Gln Val Lys
   20
<210> 699
   <211> 8
   <212> PRT
   <213> RESISTANCE GENE
<400> 699
   Phe Trp Leu Glu Asp Gln Leu Arg
   1 5
<210> 700
   <211> 8
   <212> PRT
   <213> RESISTANCE GENE
<400> 700
   Phe Trp Leu Glu Gly Pro Leu Lys
   1 5
<210> 701
   <211> 8
   <212> PRT
   <213> RESISTANCE GENE
<400> 701
   Phe Trp Leu Glu Gly Gln Leu Arg
   1 5
<210> 702
   <211> 8
   <212> PRT
   <213> RESISTANCE GENE
<400> 702
   Phe Tyr Pro Ala Ser Ser Phe Lys
   1 5
<210> 703
   <211> 8
   <212> PRT
   <213> RESISTANCE GENE
<400> 703
   Phe Tyr Pro Ala Ser Thr Phe Lys
   1 5
<210> 704
   <211> 5
   <212> PRT
   <213> RESISTANCE GENE
<400> 704
   Gly Ala Cys Asp Lys
   1 5
<210> 705
   <211> 18
   <212> PRT
   <213> RESISTANCE GENE
<400> 705
   Gly Ala Glu Val Tyr Phe Phe Ala Phe Asn Met Asp Ile Asp Asn Glu
    1               5                  10                  15
   Asn Lys
<210> 706
   <211> 16
   <212> PRT
   <213> RESISTANCE GENE
<400> 706
   Gly Ala Ile Gln Val Ser Ala Val Pro Val Phe Gln Gln Ile Ala Arg
    1               5                  10                  15
<210> 707
   <211> 16
   <212> PRT
   <213> RESISTANCE GENE
<400> 707
   Gly Ala Ile Gln Val Ser Ala Val Pro Val Phe Gln Gln Ile Thr Arg
    1               5                  10                  15
<210> 708
   <211> 21
   <212> PRT
   <213> RESISTANCE GENE
<400> 708
   Gly Asp Tyr Trp Ile Asp Gly Asn Leu Glu Ile Ser Ala His Glu Gln
    1               5                  10                  15
   Ile Ser Phe Leu Arg
                20
<210> 709
   <211> 10
   <212> PRT
   <213> RESISTANCE GENE
<400> 709
   Gly Asp Tyr Trp Ile Asp Gly Asn Leu Lys
    1               5                  10
<210> 710
   <211> 14
   <212> PRT
   <213> RESISTANCE GENE
<400> 710
   Gly Glu Leu Pro Val Ser Glu Asp Ala Leu Glu Met Thr Lys
   1 5 10
<210> 711
   <211> 10
   <212> PRT
   <213> RESISTANCE GENE
<400> 711
   Gly Glu Gln Pro Ala Gly Pro Ala Ala Arg
   1 5 10
<210> 712
   <211> 12
   <212> PRT
   <213> RESISTANCE GENE
<400> 712
   Gly Phe Ala Gly His Asn Gln Asp Gln Asp Leu Arg
   1 5 10
<210> 713
   <211> 7
   <212> PRT
   <213> RESISTANCE GENE
<400> 713
   Gly Ile Pro Ser Ser Val Arg
   1 5
<210> 714
   <211> 7
   <212> PRT
   <213> RESISTANCE GENE
<400> 714
   Gly Ile Ser Ser Ser Val Arg
   1 5
<210> 715
   <211> 12
   <212> PRT
   <213> RESISTANCE GENE
<400> 715
   Gly Leu Asn Gly Thr Phe Val Val Tyr Asp Leu Lys
   1 5 10
<210> 716
   <211> 12
   <212> PRT
   <213> RESISTANCE GENE
<400> 716
   Gly Met Glu Ile Trp Asn Ser Asn His Thr Pro Lys
   1 5 10
<210> 717
   <211> 9
   <212> PRT
   <213> RESISTANCE GENE
<400> 717
   Gly Asn Gln Thr Leu Val Phe Ala Arg
   1 5
<210> 718
   <211> 21
   <212> PRT
   <213> RESISTANCE GENE
<400> 718
   Gly Asn Tyr Trp Ile Asp Gly Asn Leu Glu Ile Ser Ala His Glu Gln
   1 5 10 15
   Ile Ser Phe Leu Arg
   20
<210> 719
   <211> 12
   <212> PRT
   <213> RESISTANCE GENE
<400> 719
   Gly Pro Leu Glu Ile Ser Ala Phe Glu Glu Ala Arg
   1 5 10
<210> 720
   <211> 12
   <212> PRT
   <213> RESISTANCE GENE
<400> 720
   Gly Pro Leu Thr Ile Thr Pro Ile Gln Glu Val Lys
   1 5 10
<210> 721
   <211> 11
   <212> PRT
   <213> RESISTANCE GENE
<400> 721
   Gly Ser Gly Trp Phe Val Gly Trp Ile Val Arg
   1 5 10
<210> 722
   <211> 9
   <212> PRT
   <213> RESISTANCE GENE
<400> 722
   Gly Ser Leu Leu Leu Trp Asp Gln Lys
   1               5
<210> 723
   <211> 18
   <212> PRT
   <213> RESISTANCE GENE
<400> 723
   Gly Thr Glu Val Tyr Phe Phe Ala Phe Asn Met Asp Ile Asp Asn Glu
   1               5                   10                  15
   Asn Lys
<210> 724
   <211> 18
   <212> PRT
   <213> RESISTANCE GENE
<400> 724
   Gly Thr Glu Val Tyr Phe Phe Ala Phe Asn Met Asp Ile Asp Asn Glu
   1               5                   10                  15
   Ser Lys
<210> 725
   <211> 10
   <212> PRT
   <213> RESISTANCE GENE
<400> 725
   Gly Thr Phe Val Leu Tyr Asp Val Gln Arg
   1               5                   10
<210> 726
   <211> 9
   <212> PRT
   <213> RESISTANCE GENE
<400> 726
   Gly Thr Ile Val Val Ala Asp Glu Arg
   1               5
<210> 727
   <211> 9
   <212> PRT
   <213> RESISTANCE GENE
<400> 727
   Gly Thr Ile Val Val Leu Asp Ala Arg
   1 5
<210> 728
   <211> 9
   <212> PRT
   <213> RESISTANCE GENE
<400> 728
   Gly Thr Ile Val Val Val Asp Glu Arg
   1 5
<210> 729
   <211> 8
   <212> PRT
   <213> RESISTANCE GENE
<400> 729
   Gly Thr Leu Pro Phe Ser Ala Arg
   1 5
<210> 730
   <211> 7
   <212> PRT
   <213> RESISTANCE GENE
<400> 730
   Gly Thr Pro Ser Ser Val Arg
   1 5
<210> 731
   <211> 20
   <212> PRT
   <213> RESISTANCE GENE
<400> 731
   His Ala Leu Ser Ser Ala Phe Val Leu Leu Gly Cys Ile Ala Ala Ser
   1 5 10 15
   Ala His Ala Lys
   20
<210> 732
   <211> 6
   <212> PRT
   <213> RESISTANCE GENE
<400> 732
   His Ile Ala Asp Ser Lys
   1 5
<210> 733
   <211> 14
   <212> PRT
   <213> RESISTANCE GENE
<400> 733
   His Asn Gly Leu Thr His Ala Trp Leu Ala Ser Ser Leu Lys
   1 5 10
<210> 734
   <211> 18
   <212> PRT
   <213> RESISTANCE GENE
<400> 734
   His Asn Gly Leu Thr Gln Ser Trp Leu Met Ser Ser Leu Thr Ile Ser
   1 5 10 15
   Pro Lys
<210> 735
   <211> 14
   <212> PRT
   <213> RESISTANCE GENE
<400> 735
   His Asn Gly Thr Asp Gly Ala Trp Ile Ile Ser Ser Leu Arg
   1 5 10
<210> 736
   <211> 10
   <212> PRT
   <213> RESISTANCE GENE
<400> 736
   His Thr Leu Ser Val Phe Asp Gln Glu Arg
   1 5 10
<210> 737
   <211> 8
   <212> PRT
   <213> RESISTANCE GENE
<400> 737
   His Val Thr Phe Ala Ser Phe Arg
   1 5
<210> 738
   <211> 14
   <212> PRT
   <213> RESISTANCE GENE
<400> 738
   Ile Ala Ile Ser Leu Met Gly Tyr Asp Ala Gly Phe Leu Arg
   1 5 10
<210> 739
   <211> 16
   <212> PRT
   <213> RESISTANCE GENE
<400> 739
   Ile Ala Leu Ser Leu Met Ala Phe Asp Ala Glu Ile Ile Asp Gln Lys
   1 5 10 15
<210> 740
   <211> 14
   <212> PRT
   <213> RESISTANCE GENE
<400> 740
   Ile Ala Leu Ser Leu Met Gly Phe Asp Ser Gly Ile Leu Lys
   1 5 10
<210> 741
   <211> 12
   <212> PRT
   <213> RESISTANCE GENE
<400> 741
   Ile Ala Asn Ala Leu Ile Gly Leu Glu Asn His Lys
   1 5 10
<210> 742
   <211> 11
   <212> PRT
   <213> RESISTANCE GENE
<400> 742
   Ile Ala Trp Ile Val Gly Phe Val Tyr Leu Lys
   1 5 10
<210> 743
   <211> 11
   <212> PRT
   <213> RESISTANCE GENE
<400> 743
   Ile Asp Thr Phe Trp Leu Asp Asn Ser Leu Lys
   1 5 10
<210> 744
   <211> 8
   <212> PRT
   <213> RESISTANCE GENE
<400> 744
   Ile Asp Tyr Tyr Asn Leu Asp Arg
   1               5
<210> 745
   <211> 14
   <212> PRT
   <213> RESISTANCE GENE
<400> 745
   Ile Phe Asn Ala Leu Ile Ala Leu Asp Ser Gly Val Ile Lys
   1               5                   10
<210> 746
   <211> 17
   <212> PRT
   <213> RESISTANCE GENE
<400> 746
   Ile Phe Asn Ser Leu Leu Ala Leu Asp Ser Gly Ala Leu Asp Asn Glu
   1               5                   10                  15
   Arg
<210> 747
   <211> 14
   <212> PRT
   <213> RESISTANCE GENE
<400> 747
   Ile Phe Asn Thr Leu Ile Gly Leu Glu Asn Gly Ile Val Lys
   1               5                   10
<210> 748
   <211> 8
   <212> PRT
   <213> RESISTANCE GENE
<400> 748
   Ile Gly Leu Asp Leu Met Gln Lys
   1               5
<210> 749
   <211> 5
   <212> PRT
   <213> RESISTANCE GENE
<400> 749
   Ile Gly Leu Glu Lys
   1 5
<210> 750
   <211> 12
   <212> PRT
   <213> RESISTANCE GENE
<400> 750
   Ile Gly Leu Glu Leu Met Gln Gln Glu Val Gln Arg
   1 5 10
<210> 751
   <211> 8
   <212> PRT
   <213> RESISTANCE GENE
<400> 751
   Ile Gly Leu Glu Leu Met Ser Lys
   1 5
<210> 752
   <211> 11
   <212> PRT
   <213> RESISTANCE GENE
<400> 752
   Ile Gly Leu Glu Leu Met Ser Asn Glu Val Lys
   1 5 10
<210> 753
   <211> 5
   <212> PRT
   <213> RESISTANCE GENE
<400> 753
   Ile Gly Leu Glu Arg
   1 5
<210> 754
   <211> 5
   <212> PRT
   <213> RESISTANCE GENE
<400> 754
   Ile Gly Leu Asn Lys
   1 5
<210> 755
   <211> 8
   <212> PRT
   <213> RESISTANCE GENE
<400> 755
   Ile Gly Leu Asn Leu Met Gln Lys
   1 5
<210> 756
   <211> 12
   <212> PRT
   <213> RESISTANCE GENE
<400> 756
   Ile Gly Pro Ser Leu Met Gln Ser Glu Leu Gln Arg
   1 5 10
<210> 757
   <211> 18
   <212> PRT
   <213> RESISTANCE GENE
<400> 757
   Ile Gly Tyr Gly Asn Met Gln Ile Gly Thr Glu Val Asp Gln Phe Trp
   1 5 10 15
   Leu Lys
<210> 758
   <211> 18
   <212> PRT
   <213> RESISTANCE GENE
<400> 758
   Ile Gly Tyr Gly Asn Met Gln Met Gly Thr Glu Val Asp Gln Phe Trp
   1 5 10 15
   Leu Lys
<210> 759
   <211> 9
   <212> PRT
   <213> RESISTANCE GENE
<400> 759
   Ile Ile Asn His Asn Leu Pro Val Lys
   1 5
<210> 760
   <211> 9
<400> 760
<210> 761
   <211> 14
   <212> PRT
   <213> RESISTANCE GENE
<400> 761
   Ile Leu Phe Gln Gln Gly Thr Gln Gln Ala Cys Ala Glu Arg
   1 5 10
<210> 762
   <211> 7
   <212> PRT
   <213> RESISTANCE GENE
<400> 762
   Ile Leu Asn Asn Trp Phe Lys
   1 5
<210> 763
   <211> 11
   <212> PRT
   <213> RESISTANCE GENE
<400> 763
   Ile Leu Asn Thr Leu Ile Ser Leu Glu Glu Lys
   1 5 10
<210> 764
   <211> 21
   <212> PRT
   <213> RESISTANCE GENE
<400> 764
   Ile Leu Ser Leu Val Cys Leu Ser Ile Ser Ile Gly Ala Cys Ala Glu
   1 5 10 15
   His Ser Met Ser Arg
   20
<210> 765
   <211> 5
   <212> PRT
   <213> RESISTANCE GENE
<400> 765
   Ile Asn Glu Ser Arg
   1 5
<210> 766
   <211> 5
   <212> PRT
   <213> RESISTANCE GENE
<400> 766
   Ile Asn Ile Val Lys
   1 5
<210> 767
   <211> 10
   <212> PRT
   <213> RESISTANCE GENE
<400> 767
   Ile Asn Leu Tyr Gly Asn Ala Leu Ser Arg
   1 5 10
<210> 768
   <211> 10
   <212> PRT
   <213> RESISTANCE GENE
<400> 768
   Ile Pro Phe Ser Leu Asn Leu Glu Met Lys
   1 5 10
<210> 769
   <211> 14
   <212> PRT
   <213> RESISTANCE GENE
<400> 769
   Ile Pro His Thr Leu Phe Ala Leu Asp Ala Asp Ala Val Arg
   1 5 10
<210> 770
   <211> 14
   <212> PRT
   <213> RESISTANCE GENE
<400> 770
   Ile Pro His Thr Leu Phe Ala Leu Asp Ala Gly Ala Ala Arg
   1 5 10
<210> 771
   <211> 14
   <212> PRT
   <213> RESISTANCE GENE
<400> 771
   Ile Pro His Thr Leu Phe Ala Leu Asp Ala Gly Ala Val Arg
   1 5 10
<210> 772
   <211> 5
   <212> PRT
   <213> RESISTANCE GENE
<400> 772
   Ile Pro Leu Gly Lys
   1 5
<210> 773
   <211> 14
   <212> PRT
   <213> RESISTANCE GENE
<400> 773
   Ile Pro Asn Ala Ile Ile Gly Leu Glu Thr Gly Val Ile Lys
   1 5 10
<210> 774
   <211> 14
   <212> PRT
   <213> RESISTANCE GENE
<400> 774
   Ile Pro Asn Ala Leu Ile Gly Leu Glu Thr Gly Ala Ile Lys
   1 5 10
<210> 775
   <211> 14
   <212> PRT
   <213> RESISTANCE GENE
<400> 775
   Ile Pro Asn Ser Leu Ile Ala Phe Asp Thr Gly Ala Val Arg
   1 5 10
<210> 776
   <211> 14
   <212> PRT
   <213> RESISTANCE GENE
<400> 776
   Ile Pro Ser Ala Ile Ile Gly Leu Glu Thr Gly Val Ile Lys
   1 5 10
<210> 777
   <211> 8
   <212> PRT
   <213> RESISTANCE GENE
<400> 777
   Ile Ser Ala Phe Asn Gln Val Lys
   1 5
<210> 778
   <211> 11
   <212> PRT
   <213> RESISTANCE GENE
<400> 778
   Ile Ser Ala His Glu Gln Ile Leu Phe Leu Arg
   1 5 10
<210> 779
   <211> 8
   <212> PRT
   <213> RESISTANCE GENE
<400> 779
   Ile Ser Ala Met Glu Gln Thr Arg
   1 5
<210> 780
   <211> 8
   <212> PRT
   <213> RESISTANCE GENE
<400> 780
   Ile Ser Ala Met Glu Gln Val Lys
   1 5
<210> 781
   <211> 11
   <212> PRT
   <213> RESISTANCE GENE
<400> 781
   Ile Ser Ala Thr Glu Gln Val Ala Phe Leu Arg
   1 5 10
<210> 782
   <211> 11
   <212> PRT
   <213> RESISTANCE GENE
<400> 782
   Ile Ser Ala Thr Gln Gln Ile Ala Phe Leu Arg
   1 5 10
<210> 783
   <211> 17
   <212> PRT
   <213> RESISTANCE GENE
<400> 783
   Ile Ser Ala Val Asn Gln Val Glu Phe Leu Glu Ser Leu Phe Leu Asn
   1 5 10 15
   Lys
<210> 784
   <211> 17
   <212> PRT
   <213> RESISTANCE GENE
<400> 784
   Ile Ser Ala Val Asn Gln Val Glu Phe Leu Glu Ser Leu Tyr Leu Asn
   1 5 10 15
   Lys
<210> 785
   <211> 8
   <212> PRT
   <213> RESISTANCE GENE
<400> 785
   Ile Ser Ala Val Asn Gln Val Lys
   1 5
<210> 786
   <211> 11
   <212> PRT
   <213> RESISTANCE GENE
<400> 786
   Ile Ser Pro Glu Glu Gln Ile Gln Phe Leu Arg
   1 5 10
<210> 787
   <211> 8
   <212> PRT
   <213> RESISTANCE GENE
<400> 787
   Ile Ser Pro Glu Glu Gln Val Arg
   1 5
<210> 788
   <211> 8
   <212> PRT
   <213> RESISTANCE GENE
<400> 788
   Ile Ser Pro Glu Gly Gln Val Arg
   1 5
<210> 789
   <211> 11
   <212> PRT
   <213> RESISTANCE GENE
<400> 789
   Ile Ser Pro Leu Glu Gln Leu Ala Phe Leu Arg
    1               5                   10
<210> 790
   <211> 11
   <212> PRT
   <213> RESISTANCE GENE
<400> 790
   Ile Thr Ala Phe Gln Gln Val Asp Phe Leu Arg
    1               5                   10
<210> 791
   <211> 16
   <212> PRT
   <213> RESISTANCE GENE
<400> 791
   Ile Thr Leu Phe Leu Leu Phe Leu Asn Leu Val Phe Gly Gln Asp Lys
    1               5                   10                  15
<210> 792
   <211> 17
   <212> PRT
   <213> RESISTANCE GENE
<400> 792
   Ile Thr Pro Ile Gln Glu Val Asn Phe Ala Asp Asp Phe Ala Asn Asn
    1               5                   10                  15
   Arg
<210> 793
   <211> 17
   <212> PRT
   <213> RESISTANCE GENE
<400> 793
   Ile Thr Pro Ile Gln Glu Val Asn Phe Ala Asp Asp Leu Ala Asn Asn
    1               5                   10                  15
   Arg
<210> 794
   <211> 12
   <212> PRT
   <213> RESISTANCE GENE
<400> 794
   Ile Thr Pro Gln Gln Glu Ala Gln Phe Ala Tyr Lys
   1 5 10
<210> 795
   <211> 12
   <212> PRT
   <213> RESISTANCE GENE
<400> 795
   Ile Thr Pro Gln Gln Glu Ala Gln Phe Thr Tyr Lys
   1 5 10
<210> 796
   <211> 17
   <212> PRT
   <213> RESISTANCE GENE
<400> 796
   Ile Thr Pro Val Gln Glu Val Asn Phe Ala Asp Asp Leu Ala His Asn
   1 5 10 15
   Arg
<210> 797
   <211> 7
   <212> PRT
   <213> RESISTANCE GENE
<400> 797
   Ile Val Ala Phe Ala Leu Lys
   1 5
<210> 798
   <211> 11
   <212> PRT
   <213> RESISTANCE GENE
<400> 798
   Ile Val Ala Phe Ala Leu Asn Met Glu Met Arg
   1 5 10
<210> 799
   <211> 16
   <212> PRT
   <213> RESISTANCE GENE
<400> 799
   Ile Val Glu Ser Thr Thr Leu Ala Asp Gly Thr Val Val His Gly Lys
   1 5 10 15
<210> 800
   <211> 11
   <212> PRT
   <213> RESISTANCE GENE
<400> 800
   Ile Tyr Asn Ser Leu Ile Gly Leu Asn Glu Lys
    1               5                   10
<210> 801
   <211> 13
   <212> PRT
   <213> RESISTANCE GENE
<400> 801
   Lys Pro Asp Ile Gly Trp Trp Val Gly Trp Ile Glu Arg
    1               5                   10
<210> 802
   <211> 10
   <212> PRT
   <213> RESISTANCE GENE
<400> 802
   Leu Ala Cys Ala Thr Asn Asn Leu Ala Arg
    1               5                   10
<210> 803
   <211> 17
   <212> PRT
   <213> RESISTANCE GENE
<400> 803
   Leu Ala Gln Gly Glu Leu Pro Phe Pro Ala Pro Val Gln Ser Thr Val
       1               5                   10                  15
   Arg
<210> 804
   <211> 14
   <212> PRT
   <213> RESISTANCE GENE
<400> 804
   Leu Ala Gln Asn Glu Leu Pro Tyr Pro Ile Glu Ile Gln Lys
    1               5                   10
<210> 805
   <211> 14
   <212> PRT
   <213> RESISTANCE GENE
<400> 805
   Leu Ala Gln Asn Glu Leu Gln Tyr Pro Ile Glu Ile Gln Lys
   1 5 10
<210> 806
   <211> 6
   <212> PRT
   <213> RESISTANCE GENE
<400> 806
   Leu Asp Phe Gly Asn Lys
   1 5
<210> 807
   <211> 7
   <212> PRT
   <213> RESISTANCE GENE
<400> 807
   Leu Asp Gly Ser Leu Asn Arg
   1 5
<210> 808
   <211> 5
   <212> **PRT**
   <213> **RESISTANCE GENE**
<400> 808
   Leu Glu Ile Gly Lys
   1 5
<210> 809
   <211> 16
   <212> PRT
   <213> RESISTANCE GENE
<400> 809
   Leu Glu Ile Leu Gln Gln Ala Leu Ala Glu Leu Gly Leu Tyr Pro Lys
   1 5 10 15
<210> 810
   <211> 8
   <212> PRT
   <213> RESISTANCE GENE
<400> 810
   Leu Glu Asn Gln Glu Gln Val Lys
   1 5
<210> 811
   <211> 9
   <212> **PRT**
   <213> **RESISTANCE GENE**
<400> 811
   Leu Glu Thr Gln Glu Glu Val Glu Lys
   1 5
<210> 812
   <211> 8
   <212> PRT
   <213> RESISTANCE GENE
<400> 812
   Leu Glu Thr Gln Glu Glu Val Lys
   1 5
<210> 813
   <211> 8
   <212> PRT
   <213> RESISTANCE GENE
<400> 813
   Leu Phe Ala Ala Glu Gly Val Lys
   1 5
<210> 814
   <211> 8
   <212> PRT
   <213> RESISTANCE GENE
<400> 814
   Leu Phe Glu Ser Ala Gly Val Lys
   1 5
<210> 815
   <211> 8
   <212> PRT
   <213> RESISTANCE GENE
<400> 815
   Leu Phe Gly Ala Ala Gly Val Lys
   1 5
<210> 816
   <211> 7
   <212> PRT
   <213> RESISTANCE GENE
<400> 816
   Leu Phe Pro Glu Trp Glu Lys
   1 5
<210> 817
   <211> 5
   <212> **PRT**
   <213> **RESISTANCE GENE**
<400> 817
   Leu Gly Glu Ser Arg
   1 5
<210> 818
   <211> 5
   <212> PRT
   <213> RESISTANCE GENE
<400> 818
   Leu Gly Val Asp Arg
   1 5
<210> 819
   <211> 6
   <212> PRT
   <213> RESISTANCE GENE
<400> 819
   Leu His Val Ser Glu Arg
   1 5
<210> 820
   <211> 7
   <212> PRT
   <213> RESISTANCE GENE
<400> 820
   Leu His Tyr Gly Asn Ala Lys
   1 5
<210> 821
   <211> 9
   <212> PRT
   <213> RESISTANCE GENE
<400> 821
   Leu Leu Asn Leu Leu Ser Gln Ser Lys
   1 5
<210> 822
   <211> 6
   <212> PRT
   <213> RESISTANCE GENE
<400> 822
   Leu Leu Gln Asp Glu Arg
   1 5
<210> 823
   <211> 10
   <212> PRT
   <213> RESISTANCE GENE
<400> 823
   Leu Leu Val Gln Asp Gly Asp Cys Gly Arg
   1 5 10
<210> 824
   <211> 9
   <212> PRT
   <213> RESISTANCE GENE
<400> 824
   Leu Asn Glu Val Gly Tyr Gly Asn Arg
   1 5
<210> 825
   <211> 11
   <212> PRT
   <213> RESISTANCE GENE
<400> 825
   Leu Asn Tyr Gly Asn Ala Asp Pro Ser Thr Lys
   1 5 10
<210> 826
   <211> 6
   <212> PRT
   <213> RESISTANCE GENE
<400> 826
   Leu Asn Tyr Gly Asn Lys
   1 5
<210> 827
   <211> 5
   <212> PRT
   <213> RESISTANCE GENE
<400> 827
   Leu Pro Ala Ser Lys
   1 5
<210> 828
   <211> 14
   <212> PRT
   <213> RESISTANCE GENE
<400> 828
   Leu Pro His Thr Leu Phe Ala Leu Asp Ala Asp Ala Val Arg
   1 5 10
<210> 829
   <211> 14
   <212> PRT
   <213> RESISTANCE GENE
<400> 829
   Leu Pro His Thr Leu Phe Ala Leu Asp Ala Gly Ala Val Arg
   1 5 10
<210> 830
   <211> 17
   <212> PRT
   <213> RESISTANCE GENE
<400> 830
   Leu Pro Leu Ala Ile Met Gly Phe Asp Ser Gly Ile Leu Gln Ser Pro
   1 5 10 15
   Lys
<210> 831
   <211> 20
   <212> PRT
   <213> RESISTANCE GENE
<400> 831
   Leu Pro Leu Ala Ile Met Gly Tyr Asp Ala Asp Ile Leu Leu Asp Ala
   1 5 10 15
   Thr Thr Pro Arg
   20
<210> 832
   <211> 14
   <212> PRT
   <213> RESISTANCE GENE
<400> 832
   Leu Pro Ser Ser Leu Ile Ala Leu Glu Thr Gly Ala Val Arg
   1 5 10
<210> 833
   <211> 16
   <212> PRT
   <213> RESISTANCE GENE
<400> 833
   Leu Pro Val Ser Ala Gln Thr Leu Gln Tyr Thr Ala Asn Ile Leu Lys
   1 5 10 15
<210> 834
   <211> 6
   <212> PRT
   <213> RESISTANCE GENE
<400> 834
   Leu Pro Val Ser Glu Arg
   1 5
<210> 835
   <211> 13
   <212> PRT
   <213> RESISTANCE GENE
<400> 835
   Leu Pro Val Ser Pro Thr Ala Val Asp Met Thr Glu Arg
   1 5 10
<210> 836
   <211> 5
   <212> PRT
   <213> RESISTANCE GENE
<400> 836
   Leu Ser Ala Ser Lys
   1 5
<210> 837
   <211> 12
   <212> PRT
   <213> RESISTANCE GENE
<400> 837
   Leu Ser Ala Val Pro Ile Tyr Gln Glu Val Ala Arg
   1 5 10
<210> 838
   <211> 12
   <212> PRT
   <213> RESISTANCE GENE
<400> 838
   Leu Ser Ala Val Pro Val Tyr Gln Glu Leu Ala Arg
   1 5 10
<210> 839
   <211> 17
   <212> PRT
   <213> RESISTANCE GENE
<400> 839
   Leu Ser Cys Thr Leu Val Ile Asp Glu Ala Ser Gly Asp Leu Leu His
   1               5                   10                  15
   Arg
<210> 840
   <211> 14
   <212> PRT
   <213> RESISTANCE GENE
<400> 840
   Leu Ser Leu Gln His Gly Trp Phe Ile Gly Trp Ile Glu Lys
   1               5                   10
<210> 841
   <211> 17
   <212> PRT
   <213> RESISTANCE GENE
<400> 841
   Leu Ser Gln Asn Ser Leu Pro Phe Ser Gln Glu Ala Met Asn Ser Val
   1               5                   10                  15
   Lys
<210> 842
   <211> 6
   <212> PRT
   <213> RESISTANCE GENE
<400> 842
   Leu Ser Val Asn Pro Lys
   1               5
<210> 843
   <211> 6
   <212> PRT
   <213> RESISTANCE GENE
<400> 843
   Leu Thr Gln Asp Glu Arg
   1               5
<210> 844
   <211> 6
   <212> PRT
   <213> RESISTANCE GENE
<400> 844
   Leu Thr Val Gly Ala Arg
   1               5
<210> 845
   <211> 19
   <212> PRT
   <213> RESISTANCE GENE
<400> 845
   Leu Tyr Gly Phe Ala Leu Asn Ile Asp Met Pro Gly Gly Glu Ala Asp
   1               5                   10                  15
   Ile Gly Lys
<210> 846
   <211> 9
   <212> PRT
   <213> RESISTANCE GENE
<400> 846
   Leu Tyr His Asn Glu Leu Pro Phe Arg
   1               5
<210> 847
   <211> 5
   <212> PRT
   <213> RESISTANCE GENE
<400> 847
   Leu Tyr His Asn Lys
   1               5
<210> 848
   <211> 9
   <212> PRT
   <213> RESISTANCE GENE
<400> 848
   Leu Tyr Gln Asn Asp Leu Pro Phe Arg
   1               5
<210> 849
   <211> 6
   <212> PRT
   <213> RESISTANCE GENE
<400> 849
   Met Asp Asp Leu Phe Lys
   1               5
<210> 850
   <211> 6
   <212> PRT
   <213> RESISTANCE GENE
<400> 850
   Met Glu Asp Leu His Lys
   1 5
<210> 851
   <211> 12
   <212> PRT
   <213> RESISTANCE GENE
<400> 851
   Met Leu Ile Ala Leu Ile Gly Leu Glu Asn His Lys
   1 5 10
<210> 852
   <211> 15
   <212> PRT
   <213> RESISTANCE GENE
<400> 852
   Met Leu Leu Ile Glu Gln Gln Gly Asp Ala Ala Leu Tyr Ala Lys
   1 5 10 15
<210> 853
   <211> 5
   <212> PRT
   <213> RESISTANCE GENE
<400> 853
   Met Leu Leu Ile Lys
   1 5
<210> 854
   <211> 12
   <212> PRT
   <213> RESISTANCE GENE
<400> 854
   Met Leu Asn Ala Leu Ile Gly Leu Glu His His Lys
   1 5 10
<210> 855
   <211> 12
   <212> PRT
   <213> RESISTANCE GENE
<400> 855
   Met Leu Asn Ala Leu Ile Gly Leu Glu Asn His Lys
   1 5 10
<210> 856
   <211> 12
   <212> PRT
   <213> RESISTANCE GENE
<400> 856
   Met Leu Asn Ala Leu Ile Gly Leu Glu Asn Gln Lys
   1 5 10
<210> 857
   <211> 12
   <212> PRT
   <213> RESISTANCE GENE
<400> 857
   Met Leu Asn Ala Leu Ile Gly Leu Glu Tyr His Lys
   1 5 10
<210> 858
   <211> 12
   <212> PRT
   <213> RESISTANCE GENE
<400> 858
   Met Leu Asn Ala Leu Ile Gly Leu Gln His Gly Lys
   1 5 10
<210> 859
   <211> 12
   <212> PRT
   <213> RESISTANCE GENE
<400> 859
   Met Leu Asn Ala Leu Ile Ser Leu Glu His His Lys
   1 5 10
<210> 860
   <211> 13
   <212> PRT
   <213> RESISTANCE GENE
<400> 860
   Met Gln Ala Tyr Val Asp Ala Phe Asp Tyr Gly Asn Arg
   1 5 10
<210> 861
   <211> 7
   <212> PRT
   <213> RESISTANCE GENE
<400> 861
   Met Gln Glu Gly Leu Asn Lys
   1 5
<210> 862
   <211> 8
   <212> PRT
   <213> RESISTANCE GENE
<400> 862
   Met Ser Pro Ala Ser Thr Tyr Lys
   1 5
<210> 863
   <211> 6
   <212> PRT
   <213> RESISTANCE GENE
<400> 863
   Met Thr Ala Gly Gly Lys
   1 5
<210> 864
   <211> 5
   <212> PRT
   <213> RESISTANCE GENE
<400> 864
   Met Val Ser Gly Lys
   1 5
<210> 865
   <211> 10
   <212> PRT
   <213> RESISTANCE GENE
<400> 865
   Asn Glu His Asp Pro Val Leu Pro Tyr Arg
   1 5 10
<210> 866
   <211> 7
   <212> PRT
   <213> RESISTANCE GENE
<400> 866
   Asn Glu His Gln Ile Phe Lys
   1 5
<210> 867
   <211> 7
   <212> PRT
   <213> RESISTANCE GENE
<400> 867
   Asn Glu His Gln Val Phe Lys
   1 5
<210> 868
   <211> 6
   <212> PRT
   <213> RESISTANCE GENE
<400> 868
   Asn Glu Ile Thr Tyr Lys
   1 5
<210> 869
   <211> 6
   <212> PRT
   <213> RESISTANCE GENE
<400> 869
   Asn Glu Leu Leu Met Lys
   1 5
<210> 870
   <211> 6
   <212> PRT
   <213> RESISTANCE GENE
<400> 870
   Asn Glu Leu Met Met Lys
   1 5
<210> 871
   <211> 6
   <212> PRT
   <213> RESISTANCE GENE
<400> 871
   Asn Glu Leu Pro Phe Arg
   1 5
<210> 872
   <211> 15
   <212> PRT
   <213> RESISTANCE GENE
<400> 872
   Asn Phe Ile Leu Ile Phe Ile Phe Val Ile Leu Ile Ser Cys Lys
   1 5 10 15
<210> 873
   <211> 15
   <212> PRT
   <213> RESISTANCE GENE
<400> 873
   Asn Phe Ile Leu Ile Phe Ile Phe Val Ile Leu Thr Ser Cys Lys
   1 5 10 15
<210> 874
   <211> 11
   <212> PRT
   <213> RESISTANCE GENE
<400> 874
   Asn Ile Ser Ser Tyr Gly Asn Asn Leu Val Arg
   1 5 10
<210> 875
   <211> 11
   <212> PRT
   <213> RESISTANCE GENE
<400> 875
   Asn Ile Ser Thr Tyr Gly Asn Asn Leu Thr Arg
   1 5 10
<210> 876
   <211> 15
   <212> PRT
   <213> RESISTANCE GENE
<400> 876
   Asn Leu Phe Asn Glu Val His Thr Thr Gly Val Leu Val Ile Arg
   1 5 10 15
<210> 877
   <211> 11
   <212> PRT
   <213> RESISTANCE GENE
<400> 877
   Asn Leu Ser Thr Tyr Gly Asn Ala Leu Ala Arg
   1 5 10
<210> 878
   <211> 10
   <212> PRT
   <213> RESISTANCE GENE
<400> 878
   Asn Met Glu Asn Leu Glu Leu Phe Gly Lys
   1 5 10
<210> 879
   <211> 12
   <212> PRT
   <213> RESISTANCE GENE
<400> 879
   Asn Met Leu Leu Leu Glu Glu Asn Asn Gly Tyr Lys
   1 5 10
<210> 880
   <211> 12
   <212> PRT
   <213> RESISTANCE GENE
<400> 880
   Asn Met Leu Leu Leu Glu Glu Ser Asn Gly Tyr Lys
   1 5 10
<210> 881
   <211> 7
   <212> PRT
   <213> RESISTANCE GENE
<400> 881
   Asn Met Leu Leu Leu Glu Lys
   1 5
<210> 882
   <211> 9
   <212> PRT
   <213> RESISTANCE GENE
<400> 882
   Asn Met Thr Leu Gly Asp Ala Met Lys
   1 5
<210> 883
   <211> 14
   <212> PRT
   <213> RESISTANCE GENE
<400> 883
   Asn Asn Gly Leu Thr Glu Ala Trp Leu Glu Ser Ser Leu Lys
   1 5 10
<210> 884
   <211> 6
   <212> PRT
   <213> RESISTANCE GENE
<400> 884
   Asn Gln Leu Pro Phe Lys
   1 5
<210> 885
   <211> 11
   <212> PRT
   <213> RESISTANCE GENE
<400> 885
   Asn Gln Leu Pro Phe Gln Val Glu His Gln Arg
   1 5 10
<210> 886
   <211> 21
   <212> PRT
   <213> RESISTANCE GENE
<400> 886
   Asn Ser Ala Ile Glu Asn Thr Ile Asp Asn Met Tyr Leu Gln Asp Leu
   1 5 10 15
   Glu Asn Ser Thr Lys
   20
<210> 887
   <211> 21
   <212> PRT
   <213> RESISTANCE GENE
<400> 887
   Asn Ser Ala Ile Glu Asn Thr Ile Glu Asn Met Tyr Leu Gln Asp Leu
   1 5 10 15
   Asp Asn Ser Thr Lys
   20
<210> 888
   <211> 21
   <212> PRT
   <213> RESISTANCE GENE
<400> 888
   Asn Ser Ala Ile Glu Asn Thr Ile Glu Asn Met Tyr Leu Gln Asp Leu
   1 5 10 15
   Glu Asn Ser Thr Lys
   20
<210> 889
   <211> 12
   <212> PRT
   <213> RESISTANCE GENE
<400> 889
   Asn Ser Ala Val Trp Val Tyr Glu Leu Phe Ala Lys
   1 5 10
<210> 890
   <211> 8
   <212> PRT
   <213> RESISTANCE GENE
<400> 890
   Asn Ser Gln Val Pro Ala Tyr Lys
   1 5
<210> 891
   <211> 12
   <212> PRT
   <213> RESISTANCE GENE
<400> 891
   Asn Ser Thr Val Trp Ile Tyr Glu Leu Phe Ala Lys
   1 5 10
<210> 892
   <211> 12
   <212> PRT
   <213> RESISTANCE GENE
<400> 892
   Asn Ser Thr Val Trp Val Tyr Glu Leu Phe Ala Lys
   1 5 10
<210> 893
   <211> 12
   <212> PRT
   <213> RESISTANCE GENE
<400> 893
   Asn Ser Thr Val Trp Val Tyr Gln Leu Phe Ala Lys
   1 5 10
<210> 894
   <211> 12
   <212> PRT
   <213> RESISTANCE GENE
<400> 894
   Asn Thr Ser Gly Ala Leu Val Ile Gln Thr Asp Lys
   1 5 10
<210> 895
   <211> 12
   <212> PRT
   <213> RESISTANCE GENE
<400> 895
   Asn Thr Ser Gly Val Leu Val Ile Gln Thr Asp Lys
   1 5 10
<210> 896
   <211> 13
   <212> PRT
   <213> RESISTANCE GENE
<400> 896
   Asn Val Asp Glu Met Phe Tyr Tyr Tyr Asp Gly Ser Lys
   1 5 10
<210> 897
   <211> 5
   <212> PRT
   <213> RESISTANCE GENE
<400> 897
   Asn Trp Ile Leu Arg
   1 5
<210> 898
   <211> 9
   <212> PRT
   <213> RESISTANCE GENE
<400> 898
   Asn Trp Asn Ala Ala Met Asp Leu Arg
   1 5
<210> 899
   <211> 17
   <212> PRT
   <213> RESISTANCE GENE
<400> 899
   Asn Tyr Val Asp Ala Phe His Tyr Gly Asn Gln Asp Ile Ser Gly Asp
   1 5 10 15
   Lys
<210> 900
   <211> 13
   <212> PRT
   <213> RESISTANCE GENE
<400> 900
   Gln Ala Asp His Ala Ile Leu Val Phe Asp Gln Ala Arg
   1 5 10
<210> 901
   <211> 13
   <212> PRT
   <213> RESISTANCE GENE
<400> 901
   Gln Ala Glu His Ala Leu Leu Val Phe Gly Gln Glu Arg
   1 5 10
<210> 902
   <211> 5
   <212> PRT
   <213> RESISTANCE GENE
<400> 902
   Gln Ala Ile Thr Lys
   1 5
<210> 903
   <211> 14
   <212> PRT
   <213> RESISTANCE GENE
<400> 903
   Gln Ala Met Leu Thr Glu Ala Asn Ser Asp Tyr Ile Ile Arg
   1 5 10
<210> 904
   <211> 11
   <212> PRT
   <213> RESISTANCE GENE
<400> 904
   Gln Glu Val Gln Phe Val Ser Ala Leu Ala Arg
   1 5 10
<210> 905
   <211> 6
   <212> PRT
   <213> RESISTANCE GENE
<400> 905
   Gln Phe Ala Ser Ile Lys
   1 5
<210> 906
   <211> 8
   <212> PRT
   <213> RESISTANCE GENE
<400> 906
   Gln Gly Met Pro Gly Ser Ile Arg
   1 5
<210> 907
   <211> 8
   <212> PRT
   <213> RESISTANCE GENE
<400> 907
   Gln Gly Met Ser Gly Ser Ile Arg
   1 5
<210> 908
   <211> 14
   <212> PRT
   <213> RESISTANCE GENE
<400> 908
   Gln Gly Gln Thr Gln Gln Ser Tyr Gly Asn Asp Leu Ala Arg
   1 5 10
<210> 909
   <211> 13
   <212> PRT
   <213> RESISTANCE GENE
<400> 909
   Gln Ile Asp Tyr Gly Asn Ala Asp Pro Ser Thr Ile Lys
   1 5 10
<210> 910
   <211> 13
   <212> PRT
   <213> RESISTANCE GENE
<400> 910
   Gln Ile Asp Tyr Gly Asn Val Asp Pro Ser Thr Ile Lys
   1 5 10
<210> 911
   <211> 6
   <212> PRT
   <213> RESISTANCE GENE
<400> 911
   Gln Ile Gly Glu Ala Arg
   1 5
<210> 912
   <211> 6
   <212> PRT
   <213> RESISTANCE GENE
<400> 912
   Gln Ile Gly Gln Ala Arg
   1 5
<210> 913
   <211> 14
   <212> PRT
   <213> RESISTANCE GENE
<400> 913
   Gln Ile Met Leu Ile Glu Gln Thr Pro Ala Phe Thr Leu Arg
   1 5 10
<210> 914
   <211> 12
   <212> PRT
   <213> RESISTANCE GENE
<400> 914
   Gln Leu Gly Ser Ala Ile Asp Gln Phe Trp Leu Arg
   1 5 10
<210> 915
   <211> 6
   <212> PRT
   <213> RESISTANCE GENE
<400> 915
   Gln Leu His Asp Asn Lys
   1 5
<210> 916
   <211> 11
   <212> PRT
   <213> RESISTANCE GENE
<400> 916
   Gln Leu Ile Phe Val His Thr Val Val Gln Lys
   1 5 10
<210> 917
   <211> 14
   <212> PRT
   <213> RESISTANCE GENE
<400> 917
   Gln Leu Ile Phe Val His Thr Val Val Gln Lys Pro Gly Lys
   1 5 10
<210> 918
   <211> 5
   <212> PRT
   <213> RESISTANCE GENE
<400> 918
   Gln Leu Pro Val Lys
   1 5
<210> 919
   <211> 7
   <212> PRT
   <213> RESISTANCE GENE
<400> 919
   Gln Leu Pro Val Lys Pro Arg
   1 5
<210> 920
   <211> 9
   <212> PRT
   <213> RESISTANCE GENE
<400> 920
   Gln Leu Ser Leu Asp Val Leu Asp Lys
   1 5
<210> 921
   <211> 6
   <212> PRT
   <213> RESISTANCE GENE
<400> 921
   Gln Leu Val Tyr Ala Arg
   1 5
<210> 922
   <211> 14
   <212> PRT
   <213> RESISTANCE GENE
<400> 922
   Gln Met Met Leu Thr Glu Ala Ser Thr Asp Tyr Ile Ile Arg
   1 5 10
<210> 923
   <211> 16
   <212> PRT
   <213> RESISTANCE GENE
<400> 923
   Gln Met Ser Ile Val Glu Ala Thr Pro Asp Tyr Val Leu His Gly Lys
   1 5 10 15
<210> 924
   <211> 7
   <212> PRT
   <213> RESISTANCE GENE
<400> 924
   Gln Pro Thr Asp Pro Ala Arg
   1 5
<210> 925
   <211> 7
   <212> PRT
   <213> RESISTANCE GENE
<400> 925
   Gln Pro Thr Asp Pro Thr Arg
   1 5
<210> 926
   <211> 8
   <212> PRT
   <213> RESISTANCE GENE
<400> 926
   Gln Pro Val Ser Ala Gly Ile Arg
   1 5
<210> 927
   <211> 5
   <212> PRT
   <213> RESISTANCE GENE
<400> 927
   Gln Gln Leu Val Lys
   1 5
<210> 928
   <211> 7
   <212> PRT
   <213> RESISTANCE GENE
<400> 928
   Gln Thr Leu Val Phe Ala Arg
   1 5
<210> 929
   <211> 6
   <212> PRT
   <213> RESISTANCE GENE
<400> 929
   Gln Val Gly Ala Glu Lys
   1 5
<210> 930
   <211> 6
   <212> PRT
   <213> RESISTANCE GENE
<400> 930
   Gln Val Val Phe Ala Arg
   1 5
<210> 931
   <211> 11
   <212> PRT
   <213> RESISTANCE GENE
<400> 931
   Ser Ala Asp Glu Val Leu Pro Tyr Gly Gly Lys
   1 5 10
<210> 932
   <211> 14
   <212> PRT
   <213> RESISTANCE GENE
<400> 932
   Ser Ala Asp Glu Val Leu Pro Tyr Gly Gly Lys Pro Gln Arg
   1 5 10
<210> 933
   <211> 9
   <212> PRT
   <213> RESISTANCE GENE
<400> 933
   Ser Cys Ala Thr Asn Asp Leu Ala Arg
   1 5
<210> 934
   <211> 9
   <212> PRT
   <213> RESISTANCE GENE
<400> 934
   Ser Cys Ala Thr Asn Asn Leu Ala Arg
   1 5
<210> 935
   <211> 8
   <212> PRT
   <213> RESISTANCE GENE
<400> 935
   Ser Asp Ile Pro Gly Gly Ser Lys
   1 5
<210> 936
   <211> 5
   <212> PRT
   <213> RESISTANCE GENE
<400> 936
   Ser Asp Trp Gly Lys
   1 5
<210> 937
   <211> 14
   <212> PRT
   <213> RESISTANCE GENE
<400> 937
   Ser Glu Asp Asn Phe His Ile Ser Ser Gln Gln His Glu Lys
   1 5 10
<210> 938
   <211> 8
   <212> PRT
   <213> RESISTANCE GENE
<400> 938
   Ser Glu Met Pro Ala Ser Ile Arg
   1 5
<210> 939
   <211> 8
   <212> PRT
   <213> RESISTANCE GENE
<400> 939
   Ser Glu Met Pro Ala Ser Thr Arg
   1 5
<210> 940
   <211> 12
   <212> PRT
   <213> RESISTANCE GENE
<400> 940
   Ser Phe Ala Ala His Asn Gln Asp Gln Asp Leu Arg
   1 5 10
<210> 941
   <211> 7
   <212> PRT
   <213> RESISTANCE GENE
<400> 941
   Ser Phe Ala Gly His Asn Lys
   1 5
<210> 942
   <211> 12
   <212> PRT
   <213> RESISTANCE GENE
<400> 942
   Ser Phe Ala Gly His Asn Gln Asp Gln Asp Leu Arg
   1 5 10
<210> 943
   <211> 12
   <212> PRT
   <213> RESISTANCE GENE
<400> 943
   Ser Phe Ala Gly His Asn Gln Asp Gln Asn Leu Arg
   1 5 10
<210> 944
   <211> 8
   <212> PRT
   <213> RESISTANCE GENE
<400> 944
   Ser Phe Leu Glu Ser Trp Ala Lys
   1 5
<210> 945
   <211> 7
   <212> PRT
   <213> RESISTANCE GENE
<400> 945
   Ser Phe Thr Ala Trp Glu Lys
   1 5
<210> 946
   <211> 7
   <212> PRT
   <213> RESISTANCE GENE
<400> 946
   Ser Phe Thr Thr Trp Glu Lys
   1 5
<210> 947
   <211> 7
   <212> PRT
   <213> RESISTANCE GENE
<400> 947
   Ser Gly Ser Gly Trp Leu Arg
   1 5
<210> 948
   <211> 20
   <212> PRT
   <213> RESISTANCE GENE
<400> 948
   Ser Gly Trp Gly Met Ala Val Asp Pro Gln Val Gly Trp Tyr Val Gly
    1               5                  10                  15
   Phe Val Glu Lys
               20
<210> 949
   <211> 20
   <212> PRT
   <213> RESISTANCE GENE
<400> 949
   Ser Gly Trp Gly Met Asp Val Ser Pro Gln Val Gly Trp Leu Thr Gly
    1               5                  10                  15
   Trp Val Glu Lys
               20
<210> 950
   <211> 20
   <212> PRT
   <213> RESISTANCE GENE
<400> 950
   Ser Gly Trp Gly Met Asp Val Thr Pro Gln Val Gly Trp Leu Thr Gly
    1               5                  10                  15
   Trp Val Glu Lys
               20
<210> 951
   <211> 9
   <212> PRT
   <213> RESISTANCE GENE
<400> 951
   Ser Ile His Pro Ala Ser Thr Phe Lys
    1               5
<210> 952
   <211> 5
   <212> PRT
   <213> RESISTANCE GENE
<400> 952
   Ser Ile Pro Thr Lys
    1               5
<210> 953
   <211> 5
   <212> PRT
   <213> RESISTANCE GENE
<400> 953
   Ser Ile Ser Thr Lys
   1 5
<210> 954
   <211> 10
   <212> PRT
   <213> RESISTANCE GENE
<400> 954
   Ser Leu Gly Leu Ser Asn Asn Leu Ser Arg
   1 5 10
<210> 955
   <211> 7
   <212> PRT
   <213> RESISTANCE GENE
<400> 955
   Ser Leu Ser Met Ser Gly Lys
   1 5
<210> 956
   <211> 8
   <212> PRT
   <213> RESISTANCE GENE
<400> 956
   Ser Met Leu Phe Ile Glu Glu Lys
   1 5
<210> 957
   <211> 5
   <212> PRT
   <213> RESISTANCE GENE
<400> 957
   Ser Asn Gly Glu Lys
   1 5
<210> 958
   <211> 14
   <212> PRT
   <213> RESISTANCE GENE
<400> 958
   Ser Asn Gly Leu Thr His Ser Trp Leu Gly Ser Ser Leu Lys
   1 5 10
<210> 959
   <211> 5
   <212> PRT
   <213> RESISTANCE GENE
<400> 959
   Ser Asn Gly Tyr Lys
   1 5
<210> 960
   <211> 7
   <212> PRT
   <213> RESISTANCE GENE
<400> 960
   Ser Pro Thr Trp Glu Leu Lys
   1 5
<210> 961
   <211> 15
   <212> PRT
   <213> RESISTANCE GENE
<400> 961
   Ser Pro Thr Trp Glu Leu Lys Pro Glu Tyr Asn Pro Ser Pro Arg
   1 5 10 15
<210> 962
   <211> 6
   <212> PRT
   <213> RESISTANCE GENE
<400> 962
   Ser Gln Asp Ile Val Arg
   1 5
<210> 963
   <211> 13
   <212> PRT
   <213> RESISTANCE GENE
<400> 963
   Ser Gln Gln Lys Pro Thr Asp Pro Thr Ile Trp Leu Lys
   1 5 10
<210> 964
   <211> 16
   <212> PRT
   <213> RESISTANCE GENE
<400> 964
   Ser Gln Val Gly Trp Leu Thr Gly Trp Val Glu Gln Pro Asp Gly Lys
   1 5 10 15
<210> 965
   <211> 13
   <212> PRT
   <213> RESISTANCE GENE
<400> 965
   Ser Ser Ser Asn Ser Cys Thr Thr Asn Asn Ala Ala Arg
   1 5 10
<210> 966
   <211> 13
   <212> PRT
   <213> RESISTANCE GENE
<400> 966
   Ser Ser Ser Asn Ser Cys Thr Thr Asn Asn Ala Thr Arg
   1 5 10
<210> 967
   <211> 7
   <212> PRT
   <213> RESISTANCE GENE
<400> 967
   Ser Val Tyr Gly Glu Leu Arg
   1 5
<210> 968
   <211> 5
   <212> PRT
   <213> RESISTANCE GENE
<400> 968
   Ser Trp Ile Leu Arg
   1 5
<210> 969
   <211> 15
   <212> PRT
   <213> RESISTANCE GENE
<400> 969
   Ser Tyr Phe Asp Glu Ala Gln Thr Gln Gly Val Ile Ile Ile Lys
   1 5 10 15
<210> 970
   <211> 5
   <212> PRT
   <213> RESISTANCE GENE
<400> 970
   Ser Tyr Leu Glu Lys
   1 5
<210> 971
   <211> 7
   <212> PRT
   <213> RESISTANCE GENE
<400> 971
   Ser Tyr Pro Met Trp Glu Lys
   1 5
<210> 972
   <211> 10
   <212> PRT
   <213> RESISTANCE GENE
<400> 972
   Thr Ala Tyr Ile Pro Ala Ser Thr Phe Lys
   1 5 10
<210> 973
   <211> 6
   <212> PRT
   <213> RESISTANCE GENE
<400> 973
   Thr Asp Asp Leu Phe Lys
   1 5
<210> 974
   <211> 8
   <212> PRT
   <213> RESISTANCE GENE
<400> 974
   Thr Asp Ile Asn Glu Ile Phe Lys
   1 5
<210> 975
   <211> 8
   <212> PRT
   <213> RESISTANCE GENE
<400> 975
   Thr Phe Ile His Asn Asp Pro Arg
   1 5
<210> 976
   <211> 9
   <212> PRT
   <213> RESISTANCE GENE
<400> 976
   Thr Gly Ala Gly Phe Thr Ala Asn Arg
   1 5
<210> 977
   <211> 8
   <212> PRT
   <213> RESISTANCE GENE
<400> 977
   Thr Gly Phe Asn Asp Gly Gln Lys
   1 5
<210> 978
   <211> 7
   <212> PRT
   <213> RESISTANCE GENE
<400> 978
   Thr Gly Leu Ala Asp Ser Lys
   1 5
<210> 979
   <211> 8
   <212> PRT
   <213> RESISTANCE GENE
<400> 979
   Thr Gly Leu Asp Leu Met Gln Lys
   1 5
<210> 980
   <211> 8
   <212> PRT
   <213> RESISTANCE GENE
<400> 980
   Thr Gly Leu Glu Leu Met Gln Lys
   1 5
<210> 981
   <211> 7
   <212> PRT
   <213> RESISTANCE GENE
<400> 981
   Thr Gly Met Gly Tyr Pro Lys
   1 5
<210> 982
   <211> 5
   <212> PRT
   <213> RESISTANCE GENE
<400> 982
   Thr Gly Asn Gly Arg
   1 5
<210> 983
   <211> 10
   <212> PRT
   <213> RESISTANCE GENE
<400> 983
   Thr Gly Thr Gly Ser Phe Ile Asp Ala Arg
   1 5 10
<210> 984
   <211> 10
   <212> PRT
   <213> RESISTANCE GENE
<400> 984
   Thr Gly Thr Gly Ser Leu Ser Asp Ala Lys
   1 5 10
<210> 985
   <211> 20
   <212> PRT
   <213> RESISTANCE GENE
<400> 985
   Thr Gly Val Ala Thr Glu Tyr Gln Pro Glu Ile Gly Trp Trp Ala Gly
   1 5 10 15
   Trp Val Glu Arg
   20
<210> 986
   <211> 13
   <212> PRT
   <213> RESISTANCE GENE
<400> 986
   Thr Gly Val Ser Tyr Pro Leu Leu Ala Asp Gly Thr Arg
   1 5 10
<210> 987
   <211> 9
   <212> PRT
   <213> RESISTANCE GENE
<400> 987
   Thr Gly Trp Ala Ala Met Asp Ile Lys
   1 5
<210> 988
   <211> 8
   <212> PRT
   <213> RESISTANCE GENE
<400> 988
   Thr Gly Trp Ala Met Asp Ile Lys
   1 5
<210> 989
   <211> 8
   <212> PRT
   <213> RESISTANCE GENE
<400> 989
   Thr Gly Trp Ala Met Asp Val Lys
   1 5
<210> 990
   <211> 6
   <212> PRT
   <213> RESISTANCE GENE
<400> 990
   Thr Gly Trp Ala Thr Arg
   1 5
<210> 991
   <211> 19
   <212> PRT
   <213> RESISTANCE GENE
<400> 991
   Thr Gly Trp Cys Phe Asp Cys Thr Pro Glu Leu Gly Trp Trp Val Gly
   1 5 10 15
   Trp Val Lys
<210> 992
   <211> 6
   <212> PRT
   <213> RESISTANCE GENE
<400> 992
   Thr Gly Trp Glu Gly Arg
   1 5
<210> 993
   <211> 7
   <212> PRT
   <213> RESISTANCE GENE
<400> 993
   Thr Gly Trp Phe Val Asp Lys
   1 5
<210> 994
   <211> 6
   <212> PRT
   <213> RESISTANCE GENE
<400> 994
   Thr Gly Tyr Asp Thr Lys
   1 5
<210> 995
   <211> 6
   <212> PRT
   <213> RESISTANCE GENE
<400> 995
   Thr Gly Tyr Gly Val Arg
   1 5
<210> 996
   <211> 6
   <212> PRT
   <213> RESISTANCE GENE
<400> 996
   Thr Gly Tyr Ser Ala Arg
   1 5
<210> 997
   <211> 6
   <212> PRT
   <213> RESISTANCE GENE
<400> 997
   Thr Gly Tyr Ser Thr Arg
   1 5
<210> 998
   <211> 9
   <212> PRT
   <213> RESISTANCE GENE
<400> 998
   Thr His Glu Ser Ser Asn Trp Gly Lys
   1 5
<210> 999
   <211> 13
   <212> PRT
   <213> RESISTANCE GENE
<400> 999
   Thr Ile Cys Thr Ala Ile Ala Asp Ala Gly Thr Gly Lys
   1 5 10
<210> 1000
   <211> 20
   <212> PRT
   <213> RESISTANCE GENE
<400> 1000
   Thr Ile Gly Gly Ala Pro Asp Ala Tyr Trp Val Asp Asp Ser Leu Gln
   1 5 10 15
   Ile Ser Ala Arg
   20
<210> 1001
   <211> 13
   <212> PRT
   <213> RESISTANCE GENE
<400> 1001
   Thr Leu Pro Phe Ser Ala Ser Ser Tyr Glu Thr Leu Arg
   1 5 10
<210> 1002
   <211> 7
   <212> PRT
   <213> RESISTANCE GENE
<400> 1002
   Thr Leu Pro Phe Ser Leu Lys
   1 5
<210> 1003
   <211> 7
   <212> PRT
   <213> RESISTANCE GENE
<400> 1003
   Thr Leu Pro Phe Ser Pro Lys
   1 5
<210> 1004
   <211> 21
   <212> PRT
   <213> RESISTANCE GENE
<400> 1004
   Thr Leu Pro Phe Ser Gln Glu Val Gln Asp Glu Val Gln Ser Ile Leu
   1 5 10 15
   Phe Ile Glu Glu Lys
   20
<210> 1005
   <211> 21
   <212> PRT
   <213> RESISTANCE GENE
<400> 1005
   Thr Leu Pro Phe Ser Gln Glu Val Gln Asp Glu Val Gln Ser Met Leu
    1               5                  10                  15
   Phe Ile Glu Glu Lys
               20
<210> 1006
   <211> 7
   <212> PRT
   <213> RESISTANCE GENE
<400> 1006
   Thr Leu Pro Phe Ser Gln Lys
    1               5
<210> 1007
   <211> 7
   <212> PRT
   <213> RESISTANCE GENE
<400> 1007
   Thr Leu Pro Ser Ser Gln Lys
    1               5
<210> 1008
   <211> 14
   <212> PRT
   <213> RESISTANCE GENE
<400> 1008
   Thr Leu Gln Asn Gly Trp Phe Glu Gly Phe Ile Ile Ser Lys
    1               5                  10
<210> 1009
   <211> 8
   <212> PRT
   <213> RESISTANCE GENE
<400> 1009
   Thr Met Gln Glu Tyr Leu Asn Lys
    1               5
<210> 1010
   <211> 13
   <212> PRT
   <213> RESISTANCE GENE
<400> 1010
   Thr Asn Gly Asn Ser Thr Ser Val Tyr Asn Glu Ser Arg
   1 5 10
<210> 1011
   <211> 11
   <212> PRT
   <213> RESISTANCE GENE
<400> 1011
   Thr Gln Thr Tyr Gln Ala Tyr Asp Ala Ala Arg
   1 5 10
<210> 1012
   <211> 9
   <212> PRT
   <213> RESISTANCE GENE
<400> 1012
   Thr Thr Asp Pro Thr Ile Trp Glu Lys
   1 5
<210> 1013
   <211> 8
   <212> PRT
   <213> RESISTANCE GENE
<400> 1013
   Thr Thr Thr Thr Glu Val Phe Lys
   1 5
<210> 1014
   <211> 9
   <212> PRT
   <213> RESISTANCE GENE
<400> 1014
   Thr Trp Ala Ser Asn Asp Phe Ser Arg
   1 5
<210> 1015
   <211> 7
   <212> PRT
   <213> RESISTANCE GENE
<400> 1015
   Thr Trp Asp Met Val Gln Arg
   1 5
<210> 1016
   <211> 17
   <212> PRT
   <213> RESISTANCE GENE
<400> 1016
   Thr Trp Met Gln Phe Ser Val Val Trp Val Ser Gln Glu Ile Thr Gln
   1 5 10 15
   Lys
<210> 1017
   <211> 7
   <212> PRT
   <213> RESISTANCE GENE
<400> 1017
   Thr Tyr Pro Met Trp Glu Lys
   1 5
<210> 1018
   <211> 8
   <212> PRT
   <213> RESISTANCE GENE
<400> 1018
   Thr Tyr Val Val Asp Pro Ala Arg
   1 5
<210> 1019
   <211> 10
   <212> PRT
   <213> RESISTANCE GENE
<400> 1019
   Val Ala Phe Ser Leu Asn Ile Glu Met Lys
   1 5 10
<210> 1020
   <211> 14
   <212> PRT
   <213> RESISTANCE GENE
<400> 1020
   Val Ala Asn Ser Leu Ile Gly Leu Ser Thr Gly Ala Val Arg
   1 5 10
<210> 1021
   <211> 5
   <212> PRT
   <213> RESISTANCE GENE
<400> 1021
   Val Glu His Gln Arg
   1 5
<210> 1022
   <211> 5
   <212> PRT
   <213> RESISTANCE GENE
<400> 1022
   Val Glu Leu Gly Lys
   1 5
<210> 1023
   <211> 20
   <212> PRT
   <213> RESISTANCE GENE
<400> 1023
   Val Phe Asp Asp Ala Gly Val Ser Gly Thr Phe Val Leu Met Asp Ile
   1 5 10 15
   Thr Ala Asp Arg
   20
<210> 1024
   <211> 8
   <212> PRT
   <213> RESISTANCE GENE
<400> 1024
   Val Phe Leu Asp Ser Trp Ala Lys
   1 5
<210> 1025
   <211> 8
   <212> PRT
   <213> RESISTANCE GENE
<400> 1025
   Val Phe Leu Glu Ser Trp Ala Lys
   1 5
<210> 1026
   <211> 17
   <212> PRT
   <213> RESISTANCE GENE
<400> 1026
   Val Phe Leu Ser Ser Trp Ala Gln Asp Met Asn Leu Ser Ser Ala Ile
   1 5 10 15
   Lys
<210> 1027
   <211> 5
   <212> PRT
   <213> RESISTANCE GENE
<400> 1027
   Val Gly Phe Glu Arg
   1 5
<210> 1028
   <211> 9
   <212> PRT
   <213> RESISTANCE GENE
<400> 1028
   Val Ile Leu Val Phe Asp Gln Val Arg
   1 5
<210> 1029
   <211> 6
   <212> PRT
   <213> RESISTANCE GENE
<400> 1029
   Val Ile Thr Phe Thr Lys
   1 5
<210> 1030
   <211> 7
   <212> PRT
   <213> RESISTANCE GENE
<400> 1030
   Val Met Ala Ala Met Val Arg
   1 5
<210> 1031
   <211> 20
   <212> PRT
   <213> RESISTANCE GENE
<400> 1031
   Val Pro Leu Ala Val Met Gly Tyr Asp Ala Gly Ile Leu Val Asp Ala
   1 5 10 15
   His Asn Pro Arg
   20
<210> 1032
   <211> 6
   <212> PRT
   <213> RESISTANCE GENE
<400> 1032
   Val Gln Ala Asn Val Lys
   1 5
<210> 1033
   <211> 6
   <212> PRT
   <213> RESISTANCE GENE
<400> 1033
   Val Gln Asp Glu Val Lys
   1 5
<210> 1034
   <211> 14
   <212> PRT
   <213> RESISTANCE GENE
<400> 1034
   Val Gln Asp Glu Val Gln Ser Met Leu Phe Ile Glu Glu Lys
   1 5 10
<210> 1035
   <211> 18
   <212> PRT
   <213> RESISTANCE GENE
<400> 1035
   Val Gln Asp Glu Val Gln Ser Met Leu Phe Ile Glu Glu Met Asn Gly
   1 5 10 15
   Asn Lys
<210> 1036
   <211> 14
   <212> PRT
   <213> RESISTANCE GENE
<400> 1036
   Val Gln Asp Gly Val Gln Ser Met Leu Phe Ile Glu Glu Lys
   1 5 10
<210> 1037
   <211> 14
   <212> PRT
   <213> RESISTANCE GENE
<400> 1037
   Val Gln His Glu Val Gln Ser Met Leu Phe Ile Glu Glu Lys
   1 5 10
<210> 1038
   <211> 13
   <212> PRT
   <213> RESISTANCE GENE
<400> 1038
   Val Ser Cys Leu Pro Cys Tyr Gln Val Val Ser His Lys
   1 5 10
<210> 1039
   <211> 12
   <212> PRT
   <213> RESISTANCE GENE
<400> 1039
   Val Ser Cys Val Trp Cys Tyr Gln Ala Leu Ala Arg
   1 5 10
<210> 1040
   <211> 17
   <212> PRT
   <213> RESISTANCE GENE
<400> 1040
   Val Ser Asp Val Cys Ser Glu Val Thr Ala Glu Gly Trp Gln Glu Val
   1 5 10 15
   Arg
<210> 1041
   <211> 12
   <212> PRT
   <213> RESISTANCE GENE
<400> 1041
   Val Ser Glu Val Glu Gly Trp Gln Ile His Gly Lys
   1 5 10
<210> 1042
   <211> 10
   <212> PRT
   <213> RESISTANCE GENE
<400> 1042
   Val Ser Phe Ser Leu Asn Ile Glu Met Lys
   1 5 10
<210> 1043
   <211> 8
   <212> PRT
   <213> RESISTANCE GENE
<400> 1043
   Val Ser Pro Cys Ser Ser Phe Lys
   1 5
<210> 1044
   <211> 8
   <212> PRT
   <213> RESISTANCE GENE
<400> 1044
   Val Ser Gln Val Pro Ala Tyr Lys
   1 5
<210> 1045
   <211> 5
   <212> PRT
   <213> RESISTANCE GENE
<400> 1045
   Val Val Phe Ala Arg
   1 5
<210> 1046
   <211> 5
   <212> PRT
   <213> RESISTANCE GENE
<400> 1046
   Trp Asp Gly Ala Lys
   1 5
<210> 1047
   <211> 5
   <212> PRT
   <213> RESISTANCE GENE
<400> 1047
   Trp Asp Gly Glu Lys
   1 5
<210> 1048
   <211> 13
   <212> PRT
   <213> RESISTANCE GENE
<400> 1048
   Trp Asp Gly His Ile Tyr Asp Phe Pro Asp Trp Asn Arg
   1 5 10
<210> 1049
   <211> 5
   <212> PRT
   <213> RESISTANCE GENE
<400> 1049
   Trp Asp Gly Ile Lys
   1 5
<210> 1050
   <211> 6
   <212> PRT
   <213> RESISTANCE GENE
<400> 1050
   Trp Asp Gly Lys Pro Arg
   1 5
<210> 1051
   <211> 5
   <212> PRT
   <213> RESISTANCE GENE
<400> 1051
   Trp Asp Gly Gln Lys
   1 5
<210> 1052
   <211> 6
   <212> PRT
   <213> RESISTANCE GENE
<400> 1052
   Trp Asp Gly Gln Thr Arg
   1 5
<210> 1053
   <211> 5
   <212> PRT
   <213> RESISTANCE GENE
<400> 1053
   Trp Asp Gly Val Lys
   1 5
<210> 1054
   <211> 6
   <212> PRT
   <213> RESISTANCE GENE
<400> 1054
   Trp Asp Gly Val Asn Arg
   1 5
<210> 1055
   <211> 11
   <212> PRT
   <213> RESISTANCE GENE
<400> 1055
   Trp Asp Tyr Lys Pro Glu Phe Asn Gly Tyr Lys
   1 5 10
<210> 1056
   <211> 21
   <212> PRT
   <213> RESISTANCE GENE
<400> 1056
   Trp Glu Thr Tyr Ser Val Val Trp Phe Ser Gln Gln Ile Thr Glu Trp
   1 5 10 15
   Leu Gly Met Glu Arg
   20
<210> 1057
   <211> 5
   <212> PRT
   <213> RESISTANCE GENE
<400> 1057
   Trp Asn Gly Gln Lys
   1 5
<210> 1058
   <211> 5
   <212> PRT
   <213> RESISTANCE GENE
<400> 1058
   Tyr Ala Gln Ala Lys
   1 5
<210> 1059
   <211> 8
   <212> PRT
   <213> RESISTANCE GENE
<400> 1059
   Tyr Phe Ser Asp Phe Asn Ala Lys
   1 5
<210> 1060
   <211> 7
   <212> PRT
   <213> RESISTANCE GENE
<400> 1060
   Tyr Gly Thr His Leu Asp Arg
   1 5
<210> 1061
   <211> 6
   <212> PRT
   <213> RESISTANCE GENE
<400> 1061
   Tyr Ile Ile His Asn Lys
   1 5
<210> 1062
   <211> 7
   <212> PRT
   <213> RESISTANCE GENE
<400> 1062
   Tyr Leu Asp Glu Leu Val Lys
   1 5
<210> 1063
   <211> 9
   <212> PRT
   <213> RESISTANCE GENE
<400> 1063
   Tyr Leu Met Ile Thr Glu Ala Gly Arg
   1 5
<210> 1064
   <211> 18
   <212> PRT
   <213> RESISTANCE GENE
<400> 1064
   Tyr Leu Asn Leu Phe Ser Tyr Gly Asn Ala Asn Ile Gly Gly Gly Ile
   1 5 10 15
   Asp Lys
<210> 1065
   <211> 5
   <212> PRT
   <213> RESISTANCE GENE
<400> 1065
   Tyr Asn Gly Glu Lys
   1 5
<210> 1066
   <211> 6
   <212> PRT
   <213> RESISTANCE GENE
<400> 1066
   Tyr Pro His Asn Pro Arg
   1 5
<210> 1067
   <211> 18
   <212> PRT
   <213> RESISTANCE GENE
<400> 1067
   Tyr Pro Val Val Trp Tyr Ser Gln Gln Val Ala His His Leu Gly Ala
   1 5 10 15
   Gln Arg
<210> 1068
   <211> 8
   <212> PRT
   <213> RESISTANCE GENE
<400> 1068
   Tyr Ser Asn Val Leu Ala Phe Lys
   1 5
<210> 1069
   <211> 8
   <212> PRT
   <213> RESISTANCE GENE
<400> 1069
   Tyr Ser Pro Ala Ser Thr Phe Lys
   1 5
<210> 1070
   <211> 12
   <212> PRT
   <213> RESISTANCE GENE
<400> 1070
   Tyr Ser Val Val Pro Val Tyr Gln Gln Leu Ala Arg
   1 5 10
<210> 1071
   <211> 12
   <212> PRT
   <213> RESISTANCE GENE
<400> 1071
   Tyr Ser Val Val Trp Tyr Ser Gln Leu Thr Ala Lys
   1 5 10
<210> 1072
   <211> 18
   <212> PRT
   <213> RESISTANCE GENE
<400> 1072
   Tyr Ser Val Val Trp Tyr Ser Gln Gln Val Ala His His Leu Gly Ala
   1 5 10 15
   Gln Arg
<210> 1073
   <211> 8
   <212> PRT
   <213> RESISTANCE GENE
<400> 1073
   Tyr Thr Pro Ala Ser Thr Phe Lys
   1 5
<210> 1074
   <211> 18
   <212> PRT
   <213> RESISTANCE GENE
<400> 1074
   Tyr Thr Ser Ala Phe Gly Tyr Gly Asn Ala Asp Val Ser Gly Glu Pro
   1 5 10 15
   Gly Lys
<210> 1075
   <211> 20
   <212> PRT
   <213> RESISTANCE GENE
<400> 1075
   Tyr Val Phe Val Ser Ala Leu Thr Gly Asn Leu Gly Ser Asn Leu Thr
   1 5 10 15
   Ser Ser Ile Lys
   20
<210> 1076
   <211> 20
   <212> PRT
   <213> RESISTANCE GENE
<400> 1076
   Tyr Val Phe Val Ser Ala Leu Thr Gly Ser Leu Gly Ser Asn Leu Thr
   1 5 10 15
   Ser Ser Ile Lys
   20
<210> 1077
   <211> 6
   <212> PRT
   <213> RESISTANCE GENE
<400> 1077
   Tyr Val Gly His Asp Arg
   1               5
<210> 1078
   <211> 270
   <212> PRT
   <213> RESISTANCE GENE
<400> 1078
   Met Glu Leu Pro Asn Ile Met His Pro Val Ala Lys Leu Ser Thr Ala
   1               5                   10                  15
   Leu Ala Ala Ala Leu Met Leu Ser Gly Cys Met Ala Gly Glu Ile Arg
               20                  25                  30
   Pro Thr Ile Gly Gln Gln Met Glu Thr Gly Asp Gln Arg Phe Gly Asp
           35                  40                  45
   Leu Val Phe Arg Gln Leu Ala Pro Asn Val Trp Gln His Thr Ser Tyr
       50                  55                  60
   Leu Asp Met Pro Gly Phe Gly Ala Val Ala Ser Asn Gly Leu Ile Val
   65                  70                  75                  80
   Arg Asp Gly Gly Arg Val Leu Val Val Asp Thr Ala Trp Thr Asp Asp
                   85                  90                  95
   Gln Thr Ala Gln Ile Leu Asn Trp Ile Lys Gln Glu Ile Asn Leu Pro
               100                 105                 110
   Val Ala Leu Ala Val Val Thr His Ala His Gln Asp Lys Met Gly Gly
           115                 120                 125
   Met Asp Ala Leu His Ala Ala Gly Ile Ala Thr Tyr Ala Asn Ala Leu
       130                 135                 140
   Ser Asn Gln Leu Ala Pro Gln Glu Gly Met Val Ala Ala Gln His Ser
   145                 150                 155                 160
   Leu Thr Phe Ala Ala Asn Gly Trp Val Glu Pro Ala Thr Ala Pro Asn
                   165                 170                 175
   Phe Gly Pro Leu Lys Val Phe Tyr Pro Gly Pro Gly His Thr Ser Asp
               180                 185                 190
   Asn Ile Thr Val Gly Ile Asp Gly Thr Asp Ile Ala Phe Gly Gly Cys
           195                 200                 205
   Leu Ile Lys Asp Ser Lys Ala Lys Ser Leu Gly Asn Leu Gly Asp Ala
       210                 215                 220
   Asp Thr Glu His Tyr Ala Ala Ser Ala Arg Ala Phe Gly Ala Ala Phe
   225                 230                 235                 240
   Pro Lys Ala Ser Met Ile Val Met Ser His Ser Ala Pro Asp Ser Arg
                   245                 250                 255
   Ala Ala Ile Thr His Thr Ala Arg Met Ala Asp Lys Leu Arg
               260                 265                 270
<210> 1079
   <211> 270
   <212> PRT
   <213> RESISTANCE GENE
<400> 1079
   Met Glu Leu Pro Asn Ile Met His Pro Val Ala Lys Leu Ser Thr Ala
   1               5                   10                  15
   Leu Ala Ala Ala Leu Met Leu Ser Gly Cys Met Pro Gly Glu Ile Arg
               20                  25                  30
   Pro Thr Ile Gly Gln Gln Met Glu Thr Gly Asp Gln Arg Phe Gly Asp
           35                  40                  45
   Leu Val Phe Arg Gln Leu Ala Pro Asn Val Trp Gln His Thr Ser Tyr
       50                  55                  60
   Leu Asp Met Pro Gly Phe Gly Ala Val Ala Ser Asn Gly Leu Ile Val
   65                  70                  75                  80
   Arg Asp Gly Gly Arg Val Leu Leu Val Asp Thr Ala Trp Thr Asp Asp
                   85                  90                  95
   Gln Thr Ala Gln Ile Leu Asn Trp Ile Lys Gln Glu Ile Asn Leu Pro
               100                 105                 110
   Val Ala Leu Ala Val Val Thr His Ala His Gln Asp Lys Met Gly Gly
           115                 120                 125
   Met Asp Ala Leu His Ala Ala Gly Ile Ala Thr Tyr Ala Asn Ala Leu
       130                 135                 140
   Ser Asn Gln Leu Ala Pro Gln Glu Gly Leu Val Ala Ala Gln His Ser
   145                 150                 155                 160
   Leu Thr Phe Ala Ala Asn Gly Trp Val Glu Pro Ala Thr Ala Pro Asn
                   165                 170                 175
   Phe Gly Pro Leu Lys Val Phe Tyr Pro Gly Pro Gly His Thr Ser Asp
               180                 185                 190
   Asn Ile Thr Val Gly Ile Asp Gly Thr Asp Ile Ala Phe Gly Gly Cys
           195                 200                 205
   Leu Ile Lys Asp Ser Lys Ala Lys Ser Leu Gly Asn Leu Gly Asp Ala
       210                 215                 220
   Asp Thr Glu His Tyr Ala Ala Ser Ala Arg Ala Phe Gly Ala Ala Phe
   225                 230                 235                 240
   Pro Lys Ala Ser Met Ile Val Met Ser His Ser Ala Pro Asp Ser Arg
                   245                 250                 255
   Ala Ala Ile Thr His Thr Ala Arg Met Ala Asp Lys Leu Arg
               260                 265                 270
<210> 1080
   <211> 270
   <212> PRT
   <213> RESISTANCE GENE
<400> 1080
   Met Glu Leu Pro Asn Ile Met His Pro Val Ala Lys Leu Ser Thr Ala
    1               5                  10                  15
   Leu Ala Ala Ala Leu Met Leu Ser Gly Cys Met Pro Gly Glu Ile Arg
               20                  25                  30
   Pro Thr Ile Gly Gln Gln Met Glu Thr Gly Asp Gln Arg Phe Gly Asp
           35                  40                  45
   Leu Val Phe Arg Gln Leu Ala Pro Asn Val Trp Gln His Thr Ser Tyr
       50                  55                  60
   Leu Asp Met Pro Gly Phe Gly Ala Val Ala Ser Asn Gly Leu Ile Val
   65                  70                  75                  80
   Arg Asp Gly Gly Arg Val Leu Val Val Asp Thr Ala Trp Thr Asp Asp
                   85                  90                  95
   Gln Thr Ala Gln Ile Leu Asn Trp Ile Lys Gln Glu Ile Asn Leu Pro
               100                 105                 110
   Val Ala Leu Ala Val Val Thr His Ala His Gln Asp Lys Met Gly Gly
           115                 120                 125
   Met Asp Ala Leu His Ala Ala Gly Ile Ala Thr Tyr Ala Asn Ala Leu
       130                 135                 140
   Ser Asn Gln Leu Ala Pro Gln Glu Gly Met Val Ala Ala Gln His Ser
   145                 150                 155                 160
   Leu Thr Phe Ala Ala Asn Gly Trp Val Glu Pro Ala Thr Ala Pro Asn
                   165                 170                 175
   Phe Gly Pro Leu Lys Val Phe Tyr Pro Gly Pro Gly His Thr Ser Asp
               180                 185                 190
   Asn Ile Thr Val Gly Ile Asp Gly Thr Asp Ile Ala Phe Gly Gly Cys
           195                 200                 205
   Leu Ile Lys Asp Ser Lys Ala Lys Ser Leu Gly Asn Leu Gly Asp Ala
       210                 215                 220
   Asp Thr Glu His Tyr Ala Ala Ser Ala Arg Ala Phe Gly Ala Ala Phe
   225                 230                 235                 240
   Pro Lys Ala Ser Met Ile Val Met Ser His Ser Ala Pro Asp Ser Arg
                   245                 250                 255
   Ala Ala Ile Thr His Thr Ala Arg Met Ala Asp Lys Leu Arg
               260                 265                 270
<210> 1081
   <211> 20
   <212> PRT
   <213> RESISTANCE GENE
<400> 1081
   Leu Ser Thr Ala Leu Ala Ala Ala Leu Met Leu Ser Gly Cys Met Ala
    1               5                  10                  15
   Gly Glu Ile Arg
               20
<210> 1082
   <211> 20
   <212> PRT
   <213> RESISTANCE GENE
<400> 1082
   Leu Ser Thr Ala Leu Ala Ala Ala Leu Met Leu Ser Gly Cys Met Pro
    1               5                  10                  15
   Gly Glu Ile Arg
               20
<210> 1083
   <211> 21
   <212> PRT
   <213> RESISTANCE GENE
<400> 1083
   Val Leu Leu Val Asp Thr Ala Trp Thr Asp Asp Gln Thr Ala Gln Ile
    1               5                  10                  15
   Leu Asn Trp Ile Lys
               20
<210> 1084
   <211> 293
   <212> PRT
   <213> RESISTANCE GENE
<400> 1084
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Gly Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1085
   <211> 293
   <212> PRT
   <213> RESISTANCE GENE
<400> 1085
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Gly Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
               275                 280                 285
   Gly Val Asn Gly Gln
       290
<210> 1086
   <211> 293
   <212> PRT
   <213> RESISTANCE GENE
<400> 1086
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1087
   <211> 293
   <212> PRT
   <213> RESISTANCE GENE
<400> 1087
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
    65                 70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1088
   <211> 293
   <212> PRT
   <213> RESISTANCE GENE
<400> 1088
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Ser Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1089
   <211> 293
   <212> PRT
   <213> RESISTANCE GENE
<400> 1089
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Met Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1090
   <211> 293
   <212> PRT
   <213> RESISTANCE GENE
<400> 1090
   Met Ser Leu Tyr Arg Arg Leu Val Leu Leu Ser Cys Leu Ser Trp Pro
   1               5                   10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Arg Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys His
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1091
   <211> 293
   <212> PRT
   <213> RESISTANCE GENE
<400> 1091
   Met Ser Leu Tyr Arg Arg Leu Val Leu Leu Ser Cys Leu Ser Trp Pro
    1               5                  10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Arg Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 1092
   <211> 284
   <212> PRT
   <213> RESISTANCE GENE
<400> 1092
   Arg Leu Val Leu Leu Ser Cys Leu Ser Trp Pro Leu Ala Gly Phe Ser
    1               5                  10                  15
   Ala Thr Ala Leu Thr Asn Leu Val Ala Glu Pro Phe Ala Lys Leu Glu
               20                  25                  30
   Gln Asp Phe Gly Gly Ser Ile Gly Val Tyr Ala Met Asp Thr Gly Ser
           35                  40                  45
   Gly Ala Thr Val Ser Tyr Arg Ala Glu Glu Arg Phe Pro Leu Cys Ser
       50                  55                  60
   Ser Phe Lys Gly Phe Leu Ala Ala Ala Val Leu Ala Arg Ser Gln Gln
   65                  70                  75                  80
   Gln Ala Gly Leu Leu Asp Thr Pro Ile Arg Tyr Gly Lys Asn Ala Leu
                   85                  90                  95
   Val Pro Trp Ser Pro Ile Ser Glu Lys Tyr Leu Thr Thr Gly Met Thr
               100                 105                 110
   Val Ala Glu Leu Ser Ala Ala Ala Val Gln Tyr Ser Asp Asn Ala Ala
           115                 120                 125
   Ala Asn Leu Leu Leu Lys Glu Leu Gly Gly Pro Ala Gly Leu Thr Ala
       130                 135                 140
   Phe Met Arg Ser Ile Gly Asp Thr Thr Phe Arg Leu Asp Arg Trp Glu
   145                 150                 155                 160
   Leu Glu Leu Asn Ser Ala Ile Pro Gly Asp Ala Arg Asp Thr Ser Ser
                   165                 170                 175
   Pro Arg Ala Val Thr Glu Ser Leu Gln Lys Leu Thr Leu Gly Ser Ala
               180                 185                 190
   Leu Ala Ala Pro Gln Arg Gln Gln Phe Val Asp Trp Leu Lys Gly Asn
           195                 200                 205
   Thr Thr Gly Asn His Arg Ile Arg Ala Ala Val Pro Ala Asp Trp Ala
       210                 215                 220
   Val Gly Asp Lys Thr Gly Thr Cys Gly Ala Tyr Gly Thr Ala Asn Asp
   225                 230                 235                 240
   Tyr Ala Val Val Trp Pro Thr Gly Arg Ala Pro Ile Val Leu Ala Val
                   245                 250                 255
   Tyr Thr Arg Ala Pro Asn Lys Asp Asp Lys Tyr Ser Glu Ala Val Ile
               260                 265                 270
   Ala Ala Ala Ala Arg Leu Ala Leu Glu Gly Leu Gly
                       275                 280
<210> 1093
   <211> 292
   <212> PRT
   <213> RESISTANCE GENE
<400> 1093
   Ser Leu Tyr Arg Arg Leu Val Leu Leu Ser Cys Leu Ser Trp Pro Leu
   1 5 10 15
   Ala Gly Phe Ser Ala Thr Ala Leu Thr Asn Leu Val Ala Glu Pro Phe
   20 25 30
   Ala Lys Leu Glu Gln Asp Phe Gly Gly Ser Ile Gly Val Tyr Ala Met
   35 40 45
   Asp Thr Gly Ser Gly Ala Thr Val Ser Tyr Arg Ala Glu Glu Arg Phe
   50 55 60
   Pro Leu Cys Ser Ser Phe Lys Gly Phe Leu Ala Ala Ala Val Leu Ala
   65 70 75 80
   Arg Ser Gln Gln Gln Ala Gly Leu Leu Asp Thr Pro Ile Arg Tyr Gly
   85 90 95
   Lys Asn Ala Leu Val Arg Trp Ser Pro Ile Ser Glu Lys Tyr Leu Thr
   100 105 110
   Thr Gly Met Thr Val Ala Glu Leu Ser Ala Ala Ala Val Gln Tyr Ser
   115 120 125
   Asp Asn Ala Ala Ala Asn Leu Leu Leu Lys Glu Leu Gly Gly Pro Ala
   130 135 140
   Gly Leu Thr Ala Phe Met Arg Ser Ile Gly Asp Thr Thr Phe Arg Leu
   145 150 155 160
   Asp Arg Trp Glu Leu Glu Leu Asn Ser Ala Ile Pro Gly Asp Ala Arg
   165 170 175
   Asp Thr Ser Ser Pro Arg Ala Val Thr Glu Ser Leu Gln Lys Leu Thr
   180 185 190
   Leu Gly Ser Ala Leu Ala Ala Pro Gln Arg Gln Gln Phe Val Asp Trp
   195 200 205
   Leu Lys Gly Asn Thr Thr Gly Asn His Arg Ile Arg Ala Ala Val Pro
   210 215 220
   Ala Asp Trp Ala Val Gly Asp Lys Thr Gly Thr Cys Gly Gly Tyr Gly
   225 230 235 240
   Thr Ala Asn Asp Tyr Ala Val Val Trp Pro Thr Gly Arg Ala Pro Ile
   245 250 255
   Val Leu Ala Val Tyr Thr Arg Ala Pro Asn Lys Asp Asp Lys His Ser
   260 265 270
   Glu Ala Val Ile Ala Ala Ala Ala Arg Leu Ala Leu Glu Gly Leu Gly
   275 280 285
   Val Asn Gly Gln
   290
<210> 1094
   <211> 5
   <212> PRT
   <213> RESISTANCE GENE
<400> 1094
   Asn Ala Leu Val Arg
   1 5
<210> 1095
   <211> 21
   <212> PRT
   <213> RESISTANCE GENE
<400> 1095
   Thr Gly Thr Cys Gly Ala Tyr Gly Thr Ala Asn Asp Tyr Ala Val Val
   1 5 10 15
   Trp Pro Thr Gly Arg
   20
<210> 1096
   <211> 14
   <212> PRT
   <213> RESISTANCE GENE
<400> 1096
   Trp Glu Leu Glu Leu Asn Ser Ala Ile Pro Ser Asp Ala Arg
   1 5 10
<210> 1097
   <211> 14
   <212> PRT
   <213> RESISTANCE GENE
<400> 1097
   Trp Glu Leu Glu Met Asn Ser Ala Ile Pro Gly Asp Ala Arg
   1 5 10
<210> 1098
   <211> 12
   <212> PRT
   <213> RESISTANCE GENE
<400> 1098
   Ala Asn Gln Ala Phe Leu Pro Ala Ser Thr Phe Lys
   1 5 10
<210> 1099
   <211> 7
   <212> PRT
   <213> RESISTANCE GENE
<400> 1099
   Asp Glu His Gln Val Phe Lys
   1 5
<210> 1100
   <211> 9
   <212> PRT
   <213> RESISTANCE GENE
<400> 1100
   Asp His Asn Leu Ile Thr Ala Met Lys
   1 5
<210> 1101
   <211> 7
   <212> PRT
   <213> RESISTANCE GENE
<400> 1101
   Asp Ile Ala Thr Trp Asn Arg
   1 5
<210> 1102
   <211> 14
   <212> PRT
   <213> RESISTANCE GENE
<400> 1102
   Ile Pro Asn Ser Leu Ile Ala Leu Asp Leu Gly Val Val Lys
   1 5 10
<210> 1103
   <211> 11
   <212> PRT
   <213> RESISTANCE GENE
<400> 1103
   Ile Ser Ala Thr Glu Gln Ile Ser Phe Leu Arg
   1 5 10
<210> 1104
   <211> 14
   <212> PRT
   <213> RESISTANCE GENE
<400> 1104
   Gln Ala Met Leu Thr Glu Ala Asn Gly Asp Tyr Ile Ile Arg
   1 5 10
<210> 1105
   <211> 9
   <212> PRT
   <213> RESISTANCE GENE
<400> 1105
   Gln Gln Gly Phe Thr Asn Asn Leu Lys
   1 5
<210> 1106
   <211> 12
   <212> PRT
   <213> RESISTANCE GENE
<400> 1106
   Ser Gln Gly Val Val Val Leu Trp Asn Glu Asn Lys
   1 5 10
<210> 1107
   <211> 10
   <212> PRT
   <213> RESISTANCE GENE
<400> 1107
   Ser Trp Asn Ala His Phe Thr Glu His Lys
   1 5 10
<210> 1108
   <211> 21
   <212> PRT
   <213> RESISTANCE GENE
<400> 1108
   Val Leu Ala Leu Ser Ala Val Phe Leu Val Ala Ser Ile Ile Gly Met
   1 5 10 15
   Pro Ala Val Ala Lys
   20
<210> 1109
   <211> 12
   <212> PRT
   <213> RESISTANCE GENE
<400> 1109
   Tyr Ser Val Val Pro Val Tyr Gln Glu Phe Ala Arg
   1 5 10

## Description

La présente invention concerne le domaine de la microbiologie. Plus précisément, l'invention concerne la détection d'au moins un mécanisme de résistance aux carbapénèmes d'au moins un microorganisme issu d'un échantillon en utilisant la spectrométrie de masse.

Depuis la découverte des microbes par Pasteur, les microorganismes sont étudiés par microscopie et analyses biochimiques. Ces méthodes traditionnelles sont souvent longues et fastidieuses et des alternatives analytiques ont très tôt été recherchées. C'est ainsi que l'analyse de bactéries par spectrométrie de masse a été initiée dès 1975 par J. Anhalt et C. Fenselau [1].

Ces travaux préliminaires ont été suivis par l'étude en chromatographie en phase gazeuse couplée à la spectrométrie de masse (GC-MS) d'acides gras de la paroi des microorganismes [2]. Cette méthode a été popularisée sous l'appellation anglo-saxonne de FAME pour Fatty Acid Methyl Ester. Elle constitue actuellement une méthode de référence pour les études taxonomiques. Son utilisation reste cependant limitée à certains laboratoires spécialisés maîtrisant le traitement de l'échantillon par saponification, hydrolyse et dérivation.

En 1996, les travaux de M. Claydon et al [3] ainsi que de T. Krishnamurthy et P. Ross [4] ont montré la possibilité d'identifier différentes espèces bactériennes avec un spectromètre de masse de type MALDI-TOF (acronyme de l'anglais Matrix Assisted Laser Desorption lonization - Time Of Flight). L'analyse associe l'acquisition d'un spectre de masse et l'interprétation d'un logiciel expert. Elle est extrêmement simple et peut être effectuée en quelques minutes. Cependant elle ne se diffuse que depuis peu de temps dans les laboratoires d'analyses médicales [5]. Son utilisation clinique est actuellement limitée à l'identification d'espèces bactériennes et de levures. Elle n'est pas utilisée en routine pour l'identification des résistances aux antimicrobiens.

Or l'identification de résistances aux antimicrobiens tels que les antibiotiques est un élément essentiel pour assurer une prise en charge optimale des patients.

D'autres procédés de spectrométrie de masse, notamment en tandem, ont été proposés pour répondre à ces besoins. A titre d'exemple, il est possible de citer les travaux de C. Fenselau et al. pour l'identification de β-Lactamase avec un quadripôle-TOF (Q-TOF) [6].

Cependant ces résultats de recherche ne sont pas applicables à une utilisation clinique de routine. Ils ont été obtenus avec des instruments de recherche nécessitant un personnel hautement qualifié. Les temps d'analyse, souvent supérieurs à une heure par échantillon, sont incompatibles avec la charge de travail d'un laboratoire d'analyse microbiologique.

Plus récemment S. Hofstadler et al. [7] ont proposé un procédé associant une amplification du génome microbien par PCR à une détection des produits de PCR par électrospray-TOF (ESI-TOF). Ce procédé est maintenant totalement automatisé [8]. Toutefois, il nécessite une amplification par PCR avec les défauts inhérents à la biologie moléculaire, à savoir rendement d'extraction, coût des sondes, etc.

Dans ce contexte, l'objectif de la présente invention est de proposer un procédé de détection de mécanismes de résistance aux carbapénèmes, qui permette de palier les inconvénients des procédés de l'art antérieur, à savoir fournir un procédé peu coûteux, sans réactifs spécifiques à chaque espèce, notamment par rapport aux procédés de biologie moléculaire, donnant un résultat en un temps court, inférieur à une heure, et utilisable en clinique de routine, sans nécessiter un personnel hautement qualifié.

A cette fin, l'invention propose un nouveau procédé de détection, par spectrométrie de masse de type MS/MS en mode MRM, pour au moins un microorganisme compris dans un échantillon, d'au moins un marqueur de résistance à au moins un antimicrobien, qui est un carbapénème, lesdites marqueurs de résistance sont des marqueurs de type KPC, choisis parmi les peptides de séquence SEQ ID N°20 à SEQ ID N°33, SEQ ID N°1094, SEQ ID N°1096 et SEQ ID N°1097. Avantageusement, des marqueurs de résistance à plusieurs antimicrobiens différents peuvent être détectés simultanément.

Comme indiqué dans la demande PCT/FR2010/052181, des marqueurs de typage et/ou de virulence desdits microorganismes peuvent être détectés, de la même façon, par spectrométrie de masse, préalablement ou simultanément à la détection des marqueurs de mécanisme de résistance.

Par marqueurs de la résistance à au moins un antimicrobien de la classe des carbapénèmes, on entend des molécules d'origine protéique qui sont caractéristiques desdites propriétés.

Les carbapénèmes sont des antibiotiques appartenant à la famille des béta-lactamines et ont pour principaux représentants l'imipénème, le méropénème, l'ertapénème et le doripénème. Ces molécules sont dégradées par les béta-lactamases 2df, 2f et 3a de la classification de Bush et Jacoby ([9] , Antimicrobial Agents and Chemotherapy, 2010 ; 54 (3) : 969-976).

Par détermination de la résistance à au moins un antimicrobien, on entend la détermination de la susceptibilité d'un microorganisme à être détruit par un antimicrobien. Les protéines impliquées dans les mécanismes de résistance vont différer selon la famille et l'espèce.

La nomenclature des béta-lactamases, enzymes bactériennes de résistance aux beta-lactamines, n'est pas standardisée. Elles sont soit classées en quatre classes moléculaires (A à D) sur la base de leur structure primaire, soit en groupes fonctionnels sur la base des substrats ciblés et de leur résistance aux inhibiteurs (pour un revue, voir [9] Bush and Jacoby, *supra*). Pour la classification moléculaire, les techniques de séquençage ont permis une classification plus précise : par exemple, 183 variants de la protéine TEM ont été décrits (notés TEM-i, i étant compris entre 1 et 183). Pour la classification fonctionnelle, Bush et Jacoby (*supra*) ont proposé de nouveaux sous-groupes fonctionnels :
- les enzymes du groupe 1 sont des céphalosporinases appartenant à la classe moléculaire C. CMY et FOX sont des enzymes, portées par des plasmides, appartenant à ce sous-groupe.
- les enzymes du groupe 2 appartiennent aux classes moléculaires A et D. Ce groupe est lui-même subdivisé en sous-groupes, 2b, 2be, 2br, 2ber, 2d, 2de, 2df, 2f, etc. CTX-M (2be) et TEM (dont 2be, 2br) sont des enzymes appartenant à ce sous-groupe. Le sous-groupe 2b correspond à des béta-lactamases à large spectre, inhibées par l'acide clavulanique, le sulfobactame ou le tazobactame. Le sous-groupe 2be correspond à des béta-lactamases à spectre étendu (BLSE), également inhibées par l'acide clavulanique, le sulfobactame ou le tazobactame. Le sous-groupe 2br correspond aux beta-lactamases du sous-groupe 2b qui sont insensibles à l'inhibition par l'acide clavulanique, le sulfobactame ou le tazobactame. Le sous-groupe 2df inclut les OXA ayant un spectre étendu aux carbapénèmes. Le groupe 2f correspond aux béta-lactamases de type carbapénémases telles que KPC.
- le groupe 3 regroupe les métallo-beta-lactamases qui hydrolysent les carbapénèmes, telles que IMP, VIM, SPM, GIM, SIM, AIM, KHM, DIM ou NDM.

La beta-lactamase de type NDM-1 a été décrite en 2010 (Kumarasamy et al., 2010, Lancet Infect. Dis., 10 :597-602). Elle correspond à une métallo-béta-lactamase qui confère une résistance à toutes les béta-lactamines excepté l'aztreonam.

Les béta-lactamases de type KPC ont été décrites à partir de 2001 aux Etats-Unis (Yigit et al., 2001, Antimicrobio. Agents Chemother., 45:1151-1161) puis dans l'ensemble du monde. Elles correspondent à des béta-lactamases de classe A qui confèrent une résistance aux céphalosporines et aux carbapénèmes, notamment à l'imipénème et au méropénème.

Les beta-lactamases de type IMP ont été décrites à partir de 1994 au Japon (Osano et al., 1994, Antimicrobio. Agents Chemother., 38:71-78) puis dansl'ensemble du monde. Elles correspondent à des métallo-béta-lactamases qui confèrent une résistance aux céphalosporines et aux carbapénèmes mais ne confèrent pas de résistance à la Temocilline et à l'aztreonam.

Les béta-lactamases de type VIM ont été décrites à partir de 1999 en Europe (Lauretti et al., 1999, Antimicrobio. Agents Chemother., 43:1584-1590) puis dansl'ensemble du monde. Elles correspondent à des métallo-béta-lactamases qui confèrent une résistance aux céphalosporines et aux carbapénèmes mais ne confèrent pas de résistance à l'aztreonam.

La première béta-lactamase de type GES a été isolée en 1998 en Guyane française (Poirel et al., 2000, Antimicrobio. Agents Chemother., 43:622-632). Cette enzyme (GES-1) conférait une résistance de type BLSE (ESBL). Le deuxième isolat d'une bactérie portant une béta-lactamase de type GES a été réalisée en 2000 en Afrique du Sud (Poirel et al., 2001, Antimicrobio. Agents Chemother., 45 :2598-2603). Cette enzyme (GES-2) confèrait une résistance aux céphalosporines et aux carbapénèmes tels que l'imipénème.

Les béta-lactamases de type IND ont été décrites pour la première fois en 1999 (Bellais et al., 1999, FEMS Microbio. Lett., 171:127-132). Elles correspondent à des métallo-béta-lactamases qui confèrent une résistance aux céphalosporines et aux carbapénèmes.

Les béta-lactamases de type SME ont été décrites pour la première fois en 1994 (Naas et al., 1994, Antimicrobio. Agents Chemother., 38:1262-1270). Elles correspondent à des béta-lactamases de classe A qui confèrent une résistance aux céphalosporines et aux carbapénèmes.

Les béta-lactamases de type OXA (ou oxacillinases) correspondent à des béta-lactamases de classe D. Selon leur séquence primaire, elles peuvent conférer des résistances aux céphalosporines ou aux céphalosporines et aux carbapénèmes (Poirel et al, 2010, Antimicrobio. Agents Chemother., 54 :24-38).

Le procédé de l'invention peut être mis en oeuvre pour détecter des mécanismes de résistance aux carbapénèmes chez des bactéries. Ainsi, par exemple, à titre de bactéries chez lesquelles il est possible de rechercher un mécanisme de résistance aux carbapénèmes selon le procédé de l'invention, on peut citer, de façon non exhaustive : *Escherichia coli, Klebsiella pneumoniae, Pseudomonas aeruginosa, Acinetobacter baumannii, Bacillus* spp, *Stenotrophomonas maltophilia, Aeromonas* spp, *Bacteroides fragilis, Pseudomonas otitidis* et *Enterobacter cloacae,* et de façon plus générale, les Enterobacteriaceae, qui portent le gène de résistance *bla*_{NDM-1} ou *bla*_{KPC}. Il est à noter par ailleurs que les souches connues comme résistantes aux carbapénèmes sont également résistantes aux céphalosporines et aux pénicillines. Ainsi, le procédé selon l'invention permet également de détecter un mécanisme de résistance auxdits antibiotiques.

L'échantillon sur lequel le procédé de l'invention peut être mis en oeuvre est tout échantillon susceptible de contenir un microorganisme cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel dans la mesure où les marqueurs des mécanismes de résistance des bactéries aux beta-lactamines sont disponibles dans l'échantillon testé, ou bien il peut subir préalablement à l'analyse, une préparation de type enrichissement, extraction, concentration, purification, culture, selon des méthodes connues de l'homme du métier.

L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages), de viande, de poisson, d'oeuf, de fruit, de légume, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

En amont de la détection par spectrométrie de masse, l'échantillon à analyser est préférentiellement traité au préalable pour générer des peptides à partir de l'ensemble des protéines présentes dans l'échantillon pour fragmenter ces protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré par rapport au traitement physico-chimique car il préserve davantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de *Staphylococcus aureus* qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

La génération de peptides à l'aide d'un réactif chimique ou d'une protéase, peut être obtenu par simple réaction en solution. Elle peut également être mise en oeuvre avec un four à micro-ondes [10], ou sous pression [11], ou bien encore avec un dispositif à ultrasons [12]. Dans ces trois derniers cas, le protocole sera beaucoup plus rapide. Parmi les peptides ainsi obtenus, les peptides spécifiques de la protéine, sont nommés peptides protéotypiques. Ce sont eux qui seront dosés par spectrométrie de masse.

Selon l'invention, les marqueurs des mécanismes de résistance des bactéries aux carbapénèmes sont des protéines de la bactérie chez laquelle les mécanismes de résistance aux carbapénèmes sont à rechercher. En particulier, lesdites protéines sont digérées en peptides, de préférence par une enzyme, de préférence encore par la trypsine.

De même, l'échantillon contenant des marqueurs protéiques de caractérisation des mécanismes de résistance des bactéries aux carbapénèmes peut également être préalablement traité à des fins de purification. Ce traitement préalable de purification peut être mis en oeuvre avant ou après l'étape de génération de peptides tels que décrits précédemment.

Le traitement préalable de purification d'échantillon est largement connu de l'homme du métier et pourra notamment mettre en oeuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [13], ou H. Keshishian et al. [14]. Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([15] et demande de brevet WO 2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

La spectrométrie de masse à mettre en oeuvre dans le procédé de l'invention est largement connue de l'homme du métier comme un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelque soit la méthode de spectrométrie de masse utilisée pour la détection, cette dernière comprend une étape d'ionisation de la molécule cible en ions dits moléculaires, dans le cas présent une étape d'ionisation des marqueurs de caractérisation, et une étape de séparation des ions moléculaires obtenus en fonction de leur masse.

Tous les spectromètres de masse comportent donc :
- une source d'ionisation destinée à ioniser les marqueurs présents dans l'échantillon à analyser, c'est-à-dire à conférer une charge positive ou négative à ces marqueurs;
- un analyseur de masse destiné à séparer les marqueurs ionisés, ou ions moléculaires, en fonction de leur ratio masse sur charge (m /z) ;
- un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être mise en oeuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice (MALDI), activée par une surface (SELDI) ou sur silicium (DIOS)
- l'ionisation-désorption par interaction avec espèces métastables (DART) Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [16]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF), la résonance cyclotronique ionique à transformé de fourier (FT-ICR), l'orbitrappe.

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS². Lorsque trois séparations MS successives sont réalisées, l'analyse est appelée MS/MS/MS ou MS³ et plus généralement, lorsque n séparations MS successives sont réalisées, l'analyse est appelée MSⁿ.

Parmi les techniques mettant en oeuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS². De même le mode MRM³ est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées, par rapport à un dosage MS qui sont :
- une fragmentation des ions moléculaires, alors appelés ions précurseurs, pour donner des ions dit ions fragments de 1^{ère} génération, et
- une séparation des ions dit ions fragments de 1^{ère} génération en fonction de leur masse (m/z)₂, le rapport (m/z)₁ correspondant au rapport (m/z) des ions précurseurs.

C'est alors le rapport masse/charge des ions fragments de 1^{ère} génération ainsi obtenus qui est corrélé à la molécule cible à détecter. Par ion fragment de première génération, on entend un ion issu de l'ion précurseur, suite à une étape de fragmentation et dont le rapport masse sur charge m/z est différent de l'ion précurseur.

Les couples (m/z)₁ et (m/z)₂ sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [17]. Des logiciels commerciaux, tels que les logiciels MIDAS et MRM Pilote d'Applied Biosytems ou encore MRMaid [18] pourront être utilisés par l'homme de l'art pour lui permettre de prédire tous les couples de transitions possibles. Il pourra également être fait appel à une base de données nommée PeptideAtlas, construite par F. Desiere et al. [19] pour compiler l'ensemble des transitions MRM de peptides décrites par la communauté scientifique. Cette base PeptideAtlas est disponible en accès libre sur internet. Pour des molécules non protéiques, il est également possible d'utiliser des bases de données, telles que par exemple celle accessible au travers du logiciel Cliquid de la société Applied Biosytems (Etats Unis d'Amérique). Une approche alternative pour sélectionner les peptides protéotypiques, (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunions utilisateurs [18]. Elle permet de générer une liste de transitions candidates à partir des peptides identifiés expérimentalement par le logiciel SIEVE (Thermo Scientific). Certains critères ont été détaillés par J. Mead et al. [18] pour le choix des ions (m/z)₁ et (m/z)₂ et sont détaillés ci-après :
- les peptides avec des sites de clivage interne, c'est-à-dire avec de la Lysine ou de l'Arginine interne, doivent être évités, sauf si la Lysine ou l'Arginine est suivie par de la Proline,
- les peptides avec de l'Asparagine ou de la Glutamine doivent être évités car ils peuvent se désaminer,
- les peptides avec de la Glutamine ou de l'Acide Glutamique en N-terminal doivent être évités car ils peuvent se cycliser spontanément,
- les peptides avec de la Méthionine doivent être évités car ils peuvent être oxydés,
- les peptides avec de la Cystéine doivent être évités car ils peuvent être modifiés de façon non reproductible lors d'une éventuelle étape de dénaturation, réduction et blocage des fonctions thiols,
- les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- la sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en oeuvre dans une cellule de fragmentation telle que les modèles de type triple quadripôle [20], ou de type trappe ionique [21], ou encore de type temps de vol (TOF) [22], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e⁻V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Q1q2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être détectés, voire quantifiés. Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

Lorsque la spectrométrie de masse mise en oeuvre dans le procédé de l'invention est une spectrométrie de masse en tandem (MS², MS³, MS⁴ ou MS⁵), plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Selon des modes de réalisation préférés de l'invention, le procédé de l'invention comprend une ou plusieurs des caractéristiques suivantes :
- la spectrométrie de masse, mise en oeuvre pour les propriétés de résistance potentielle à au moins un antimicrobien, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est de la MRM, ce qui a pour avantage d'utiliser un temps de cycle d'analyse dans le spectromètre de masse de quelques dizaines de millisecondes, ce qui permet de détecter ou de quantifier avec une grande sensibilité, et de façon multiplexée, un grand nombre de molécules différentes ;
- le cas échéant, la détermination des propriétés de typage, et du facteur de virulence est mise en oeuvre dans le même appareil de spectrométrie de masse que la détermination des marqueurs de résistance à au moins un antimicrobien, de préférence simultanément, ce qui a pour avantage de réduire le temps d'analyse et le coût de l'instrument, cela facilite également le traitement et le rendu des résultats.

Outre la détermination de la résistance à un antibiotique, il convient d'identifier le ou les microorganismes présents dans l'échantillon à tester.

Les procédés d'identification de microorganismes sont largement connus de l'homme du métier, comme décrit par exemple par Murray P. R. el al. dans Manuel of Clinical Microbiology, 2007, 9ème édition, et en particulier dans le Vol. I, Section III, chapitres 15 et 16 pour les bactéries et levures, Vol. II, Section VI, chapitre 82 pour les virus, et Vol. II, Section X, chapitre 135 pour les protozoaires. A titre d'exemple de procédés classiques d'identification, on peut citer la détermination du profil biologique, en utilisant par exemple les cartes d'identification du Vitek 2 (bioMérieux), ou bien encore en utilisant des techniques de biologie moléculaire avec des critères d'identification basés sur l'étude de la présence de certains gènes, sur l'étude de leur séquence.

L'identification peut être mise en oeuvre directement à partir de l'échantillon dans lequel on effectue l'identification, ou bien les microorganismes contenus dans l'échantillon peuvent être cultivés par des méthodes bien connues de l'homme du métier avec des milieux de culture et des conditions de culture optimales adaptées en fonction des espèces de microorganismes à rechercher, comme décrit par Murray P.R. et al. dans Manuel of Clinical Microbiology, 2007, 9ème édition, Vol. I, Section III, chapitre 14, et en particulier dans le Vol. I, Section IV, chapitre 21 pour les bactéries, Vol. II, Section VI, chapitre 81 pour les virus, Vol. II, Section VIII, chapitre 117 pour les levures, et Vol. II, Section X, chapitre 134 pour les protozoaires.

Ainsi, de façon générale, dans le cas d'une identification par une méthode biochimique d'une bactérie dans un prélèvement, il faut d'abord l'obtenir en culture pure, par exemple après ensemencement sur gélose. La biologie moléculaire (PCR) peut dans certains cas s'appliquer directement à l'échantillon à analyser.

Au lieu de cultiver les microorganismes, ces derniers peuvent être concentrés par capture directement dans l'échantillon au moyen de surfaces actives. Un tel procédé a été décrit par W.-J. Chen et al. [10] qui ont capturé différentes espèces bactériennes à l'aide de billes magnétiques avec une surface activée au Fe₃O₄/TiO₂. Une capture par d'autres moyens est également possible, telle qu'une capture par des lectines [23], ou par des anticorps [24], ou encore par de la Vancomycine [25]. La capture permet de concentrer les microorganismes et ainsi de réduire ou même de supprimer l'étape de culture. Il s'en suit un gain de temps considérable. L'identification peut également être mise en oeuvre par spectrométrie de masse, selon les techniques décrites précédemment, de préférence par MS, par MS/MS, ou bien par MS suivie d'une spectrométrie de type MS/MS, ce qui constitue un mode de réalisation de l'invention. Dans ce cas également, l'échantillon peut être soumis préalablement à une étape de culture telle qu'un ensemencement sur gélose. L'utilisation d'un procédé d'identification par MS est avantageux en ce qu'il peut être réalisé en quelques minutes et en ce qu'il nécessite un spectromètre de masse avec un seul analyseur, c'est à dire un instrument moins complexe qu'un spectromètre de masse en tandem utilisé en MS/MS.

L'utilisation d'un procédé d'identification par MS suivi d'une spectrométrie de type MS/MS est également avantageuse. Elle permet de s'assurer de l'identité des ions observée en MS, ce qui augmente la spécificité de l'analyse.

L'utilisation d'un procédé d'identification par MS/MS de type MRM présente l'avantage d'être plus sensible et plus simple que les approches MS puis MS/MS traditionnelle. Ce procédé ne nécessite ni logiciel performant pour traiter l'information entre l'acquisition du spectre MS et du spectre MS/MS, ni changement du réglage des paramètres machines pour enchaîner des spectres MS puis MS/MS.

Le procédé d'identification par MS peut être mis en oeuvre avec une source électrospray sur échantillon brut, comme décrit par S. Vaidyanathan et al. [26] ou encore par R. Everley et al. [27] après séparation chromatographique. Différentes gammes de m/z permettent alors d'identifier les microorganismes. S. Vaidyanathan et al. ont utilisé une fenêtre entre 200 et 2000 Th, et R. Everley et al. une fenêtre entre 620 et 2450 Th. Les spectres de masse peuvent également être déconvolués pour accéder à la masse des protéines indépendamment de leur état de charge. R. Everley et al. ont ainsi exploité les masses entre environ 5 000 et 50 000 Da. De façon alternative, le procédé d'identification par MS peut être également mis en oeuvre à l'aide d'un MALDI-TOF, comme décrit par Claydon et al [3] et T. Krishnamurthy et P. Ross [4]. L'analyse associe l'acquisition d'un spectre de masse et l'interprétation d'un logiciel expert. Elle est extrêmement simple et peut être effectuée en quelques minutes. Ce procédé d'identification se répand actuellement dans les laboratoires d'analyse médicale [28].

L'identification de bactéries par MS puis MS/MS via leurs protéines présentes dans l'échantillon, a été largement appliquée par de nombreuses équipes. A titre d'exemple, il est possible de citer les travaux récents de Manes N. et al. [29] qui ont étudié le peptidome de *Salmonella enterica*, ou les travaux de R. Nandakumar et al. [30] ou de L. Hernychova et al. [31] qui ont étudié le protéome de bactéries après digestion des protéines avec de la trypsine. L'approche classique consiste à i) acquérir un spectre MS, ii) sélectionner successivement chaque ion précurseur observé sur le spectre MS avec un signal intense, iii) fragmenter successivement chaque ion précurseur et acquérir son spectre MS/MS, iv) interroger des bases de données protéiques telles que SWISS-PROT ou NCBI, aux travers de logiciels tels que Mascot (Matrix Science, Londres, Royaume-Uni) ou SEQUEST (Thermo Scientific, Waltham, Etats-Unis d'Amérique), pour identifier le peptide ayant une forte probabilité de correspondre au spectre MS/MS observé. Cette méthode peut conduire à l'identification d'un microorganisme si une protéine ou un peptide caractéristique de l'espèce est identifié.

Un des avantages de l'utilisation de la spectrométrie de masse réside en ce qu'elle est particulièrement utile pour quantifier des molécules, dans le cas présent les marqueurs des mécanismes de résistance des bactéries aux beta-lactamines. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Un calibrage est néanmoins nécessaire pour pouvoir corréler l'aire du pic mesurée, correspondant à l'intensité de courant induit par les ions détectés, à la quantité de molécule cible à doser. Pour cela, les calibrages classiquement utilisés en spectrométrie de masse pourront être mis en oeuvre, dans le cadre de l'invention. Les dosages MRM sont classiquement calibrés à l'aide de standards externes ou, de préférence, à l'aide de standards internes tels que décrits par T. Fortin et al. [13]. Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N).

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [32] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. [33] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [33] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.
L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage, dans le cadre de l'invention.

De façon préférentielle, la spectrométrie de masse utilisée dans le procédé de détection selon l'invention est de type MS/MS. Plus préférentiellement, la spectrométrie de masse est la MRM.

Le procédé de l'invention permet la détection de résistances aux carbapénèmes, caractérisée par la détection d'au moins un peptide en tant que marqueur de résistance. De façon préférentielle, ledit peptide marqueur de résistance appartient aux protéines NDM, KPC, GES, IMP, IND, SME, VIM ou OXA.

En particulier, la détection d'un mécanisme de résistance aux carbapénèmes, induit par l'expression d'une protéine NDM, est caractérisée par la détection d'au moins un peptide appartenant à une protéine NDM et à ses différents variants de séquences SEQ ID N° 1 et SEQ ID N° 1078 à SEQ ID N°1080. lesdits peptides étant choisis, de préférence, parmi les peptides de séquence **SEQ ID N°2** à **SEQ ID N°9** et **SEQ ID N°1083** tels que définis ci-après :

| **Peptide SEQ ID N°** | **Séquence d'acides aminés** | **Position du peptide dans la ou les protéines NDM** |
|---|---|---|
| **SEQ ID N°2** | AAIYHTAR | 257-264 pour les protéines de SEQ N°1, 1078, 1079, 1080 |
| **SEQ ID N°3** | AFGAAFPK | 235-242 pour les protéines de SEQ N°1, 1078, 1079, 1080 |
| **SEQ ID N°4** | ASMIVMSHSAPDSR | 243-256 pour les protéines de SEQ N°1, 1078, 1079, 1080 |
| **SEQ ID N°5** | FGDLVFR | 46-52 pour les protéines de SEQ N°1, 1078, 1079, 1080 |
| **SEQ ID N°6** | MELPNIMHPVAK | 1-12 pour les protéines de SEQ N°1, 1078, 1079, 1080 |
| **SEQ ID N°7** | QEINLPVALAVVTHAHQDK | 107-125 pour les protéines de SEQ N°1, 1078, 1079, 1080 |
| **SEQ ID N°8** | SLGNLGDADTEHYAASAR | 217-234 pour les protéines de SEQ N°1, 1078, 1079, 1080 |
| **SEQ ID N°9** | VLVVDTAWTDDQTAQILNWIK | 86-106 pour les protéines de SEQ N°1, 1078, 1080 |
| **SEQ ID N°1081** | LSTALAAALMLSGCMAGEIR | 13-32 pour la protéine de SEQ N°1078 |
| **SEQ ID N°1082** | LSTALAAALMLSGCMPGEIR | 13-32 pour la protéine de SEQ N°1, 1079, 1080 |
| **SEQ ID N°1083** | VLLVDTAWTDDQTAQILNWIK | 86-106 pour la protéine de SEQ N°1079 |

De façon préférentielle, les marqueurs de résistance sont des marqueurs de type NDM, choisis parmi les peptides de séquence SEQ ID N°2, SEQ ID N°3, SEQ ID N°5, ou SEQ ID N°7.

La détection d'un mécanisme de résistance aux carbapénèmes, induit par l'expression d'une protéine KPC, est caractérisée par la détection d'au moins un peptide appartenant une protéine KPC et à ses différents variants de séquences **SEQ ID N° 10** à **SEQ ID N°19** et **SEQ ID N°1084** à **SEQ ID N°1093.** lesdits peptides étant choisis, de préférence, parmi les peptides de séquence **SEQ ID N°20** à **SEQ ID N°33** et **SEQ ID N°1094** à **SEQ ID N°1097** tels que définis ci-après :

| **Peptide SEQ ID N°** | **Séquence d'acides aminés** | **Position du peptide dans la ou les protéines KPC** |
|---|---|---|
| **SEQ ID N°20** | AAVPADWAVGDK | 221-232 pour la protéine de SEQ N°1093; 222-233 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°21** | APIVLAVYTR | 254-263 pour la protéine de SEQ N°1093; 255-264 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°22** | AVTESLQK | 183-190 pour la protéine de SEQ N° 1093; 184-191 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°23** | ELGGPAGLTAFMR | 139-151 pour la protéine de SEQ N°1093; 140-152 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°24** | FPLCSSFK | 64-71 pour la protéine de SEQ N° 1093; 65-72 pour les protéines de séquence SEQ ID N°10, 11, 12, 13, 14, 15, 16, 17, 18, 19, 1084, 1085, 1086, 1087, 1088, 1089,1090, 1091, |
| | | 1092 |
| **SEQ ID N°25** | GFLAAAVLAR | 72-81 pour la protéine de SEQ N°1093; 73-82 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°26** | GNTTGNHR | 211-218 pour la protéine de SEQ N°1093; 212-219 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°27** | LALEGLGVNGQ | 282-292 pour la protéine de SEQ N°1093; 283-293 pour les protéines de séquence SEQ ID N°10, 11, 12, 13, 14, 15, 16, 18, 19, 1084, 1085, 1086, 1087, 1088, 1089,1090, 1091 |
| **SEQ ID N°28** | LTLGSALAAPQR | 191-202 pour la protéine de SEQ N°1093; 192-203 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°29** | NALVPWSPISEK | 94-105 pour la protéine de SEQ N° 1092; 99-110 pour les protéines de séquence SEQ ID N°10, 11, 14, 15, 16, 17, 1084, 1085, 1086, 1087, 1088, 1089 |
| **SEQ ID N° 30** | QQFVDWLK | 203-210 pour la protéine de SEQ N°1093; 204-211 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°31** | SIGDTTFR | 152-159 pour la protéine de SEQ N°1093; 153-160 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°32** | SQQQAGLLDTPIR | 82-94 pour la protéine de SEQ N°1093; 83-95 pour les protéines de séquence SEQ ID N°10, 11, 12, 13, 14, 15, 16, 17, 18, 19, 1084, 1085, 1086, 1087, 1088, 1089,1090, 1091, 1092 |
| **SEQ ID N°33** | WELELNSAIPGDAR | 163-176 pour la protéine de SEQ N° 1093; 164-177 pour les protéines de séquence SEQ ID N°10, 11, 12, 13, 14, 15, 16, 17, 18, 19, 1084, 1085, 1086, 1087, 1090, 1091, 1092 |
| **SEQ ID N°1094** | NALVR | 98-102 pour les protéines de SEQ N°1093; 99-103 pour les protéines de séquence SEQ ID N°12, 13, 18, 1090, 1091 |
| **SEQ ID N°1095** | TGTCGAYGTANDYAVVWPTGR | 229-249 pour la protéine de SEQ N°1092; 234-254 pour la protéine de séquence SEQ ID N°17 |
| **SEQ ID N°1096** | WELELNSAIPSDAR | 164-177 pour la protéine de SEQ N°1088 |
| **SEQ ID N°1097** | WELEMNSAIPGDAR | 164-177 pour la protéine de SEQ N°1089 |

De façon préférentielle, les marqueurs de résistance sont des marqueurs de type KPC, choisis parmi les peptides de séquence SEQ ID N°20, SEQ ID N°21, SEQ ID N°23, SEQ ID N°25, SEQ ID N°28, SEQ ID N°29, SEQ ID N°31, ou SEQ ID N°32.

La détection d'un mécanisme de résistance aux carbapénèmes et/ou aux céphalosporines, induit par l'expression d'une protéine GES, est caractérisée par la détection d'au moins un peptide appartenant à une protéine GES et à ses différents variants de séquences **SEQ ID N° 34** à **SEQ ID N°50.** lesdits peptides étant choisis, de préférence, parmi les peptides de séquence **SEQ ID N°51** à **SEQ ID N°79** tels que définis ci-après :

| **Peptide SEQ ID N°** | **Séquence d'acides aminés** | **Position du peptide dans la ou les protéines GES** | **Intérêt clinique** |
|---|---|---|---|
| **SEQ ID N°51** | | 36-55 pour la protéine de SEQ N°46 | carba |
| **SEQ ID N°52** | | 36-55 pour les protéines de SEQ N°34, 35, 36, 37, 38, 39, 40, 41, 42, 43, 44, 45, 47, 48, 49, 50 | BLSE |
| **SEQ ID N°53** | AGFPK | 218-222 pour les protéines de SEQ N°34, 35, 36, 37, 38, 39, 40, 41, 42, 43, 44, 45, 46, 47, 48, 49, 50 | BLSE |
| **SEQ ID N°54** | DTTTPIAMAR | 174-183 pour les protéines de SEQ N°34, 35, 36, 37, 38, 39, 40, 41, 42, 43, 44, 45, 46, 47, 48, 49, 50 | BLSE |
| **SEQ ID N°55** | DWVVGEK | 223-229 pour les protéines de SEQ N°34, 35, 36, 37, 38, 39, 40, 41, 42, 43, 44, 45, 46, 47, 48, 49, 50 | BLSE |
| **SEQ ID N°56** | DYAVAVYTTAPK | 250-261 pour les protéines de SEQ N°34, 35, 36, 37, 38, 39, 40, 41, 42, 43, 44, 45, 46, 47, 48, 49, 50 | BLSE |
| **SEQ ID N°57** | EIGGPAAMTQYFR | 136-148 pour les protéines de SEQ N°34, 35, 36, 37, 38, 39, 40, 41, 42, 43, 44, 45, 46, 47, 48, 49, 50 | BLSE |
| **SEQ ID N°58** | EPEMGDNTPGDLR | 161-173 pour les protéines de SEQ N°34, 35, 36, 40, 41, 42, 47, 48, 50 | BLSE |
| **SEQ ID N°59** | EPEMNDNTPGDLR | 161-173 pour les protéines de SEQ N°37, 43 | carba |
| **SEQ ID N°60** | EPEMSDNTPGDLR | 161-173 pour les protéines de SEQ N°38, 39, 45, 46, 49 | carba |
| **SEQ ID N°61** | ESEMSDNTPGDLR | 161-173 pour la protéine de SEQ N°44 | carba |
| **SEQ ID N°62** | FAMCSTFK | 60-67 pour les protéines de SEQ N°34, 35, 36, 37, 38, 39, 40, 41, 42, 43, 44, 45, 46, 47, 48, 49, 50 | BLSE |
| **SEQ ID N°63** | FIHALLLAGIAHSAYASEK | 3-21 pour les protéines de SEQ N°34, 35, 36, 37, 38, 39, 40, 41, 43, 44, 45, 46, 47, 48, 49, 50 | BLSE |
| **SEQ ID N°64** | FIHALLLAGTAHSAYASEK | 3-21 pour la protéine de SEQ N°42 | carba |
| **SEQ ID N°65** | FPLAALVFER | 68-77 pour les protéines de SEQ N°34, 35, 36, 37, 38, 39, 40, 41, 42, 43, 44, 45, 46, 47, 48, 49, 50 | BLSE |
| **SEQ ID N°66** | IDSGTER | 78-84 pour les protéines de SEQ N°34, 35, 36, 37, 38, 39, 40, 41, 42, 43, 44, 45, 46, 47, 48, 49, 50 | BLSE |
| **SEQ ID N°67** | IGDSVSR | 150-156 pour les protéines de SEQ N°34, 35, 36, 37, 38, 39, 40, 41, 42, 43, 44, 45, 46, 47, 48, 49, 50 | BLSE |
| **SEQ ID N°68** | LSAVER | 262-267 pour les protéines de SEQ N°34, 35, 36, 37, 38, 39, 40, 41, 42, 43, 44, 45, 46, 47, 48, 49, 50 | BLSE |
| **SEQ ID N°69** | LSYGPDMIVEWSPATER | 89-105 pour les protéines de SEQ N°34, 36, 37, 38, 40, 41, 42, 44, 45, 46, 50 | BLSE |
| **SEQ ID N°70** | LSYGPDMIVK | 89-98 pour les protéines de SEQ N°35, 39, 43, 47, 48, 49 | carba |
| **SEQ ID N°71** | NDIGFFK | 239-245 pour les protéines de SEQ N°34, 35, 36, 37, 38, 39, 40, 41, 42, 43, 44, 45, 46, 47, 48, 49, 50 | BLSE |
| **SEQ ID N°72** | TDLEK | 26-30 pour les protéines de SEQ N°34, 35, 36, 37, 38, 39, 40, 41, 42, 43, 44, 45, 46, 47, 48, 49, 50 | BLSE |
| **SEQ ID N°73** | TGACANGAR | 230-238 pour la protéine de SEQ N°50 | carba |
| **SEQ ID N°74** | TGTCANGAR | 230-238 pour les protéines de SEQ N°41, 45, 47 | carba |
| **SEQ ID N°75** | TGTCANGGR | 230-238 pour les protéines de SEQ N°34, 35, 36, 37, 38, 39, 42, 43, 44, 46, 48, 49 | BLSE |
| **SEQ ID N°76** | TGTCANGSR | 230-238 pour la protéine de SEQ N°40 | carba |
| **SEQ ID N°77** | VLYGGALTSTSTHTIER | 188-204 pour les protéines de SEQ N°34, 35, 36, 37, 38, 39, 40, 41, 42, 43, 44, 45, 46, 47, 48, 49, 50 | BLSE |
| **SEQ ID N°78** | WLIGNQTGDATLR | 205-217 pour les protéines de SEQ N°34, 35, 36, 37, 38, 39, 40, 41, 42, 43, 44, 45, 46, 47, 48, 49, 50 | BLSE |
| **SEQ ID N°79** | WSPATER | 99-105 pour les protéines de SEQ N°34, 35, 36, 37, 38, 39, 40, 41, 42, 43, 44, 45, 46, 47, 48, 49, 50 | carba |

Dans la colonne intérêt clinique, les mentions BLSE et carba correspondent aux activités beta-lactamases GES que permet de détecter le peptide correspondant. Ainsi la détection d'un peptide carba signera la présence d'une béta-lactamase de type carbapénèmase capable d'hydrolyser les carbapénèmes.

Si aucun peptide noté carba n'est détecté, la détection d'un peptide noté BLSE signera la présence d'une beta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La détection d'un mécanisme de résistance aux carbapénèmes induit par une protéine GES, est caractérisée par la détection d'au moins un peptide marqueur de résistance, de type carba, choisi parmi les séquences **SEQ ID N° 51, 59, 60, 61, 64, 70, 73, 74, 76, 79.**

La détection d'un mécanisme de résistance aux carbapénèmes, induit par l'expression d'une protéine IMP, est caractérisée par la détection d'au moins un peptide appartenant à une protéine IMP et à ses différents variants de séquences **SEQ ID N° 80** à **SEQ ID N°105.** lesdits peptides étant choisis, de préférence, parmi les peptides de séquence SEQ ID N°106, SEQ ID N°108 à SEQ ID N°130, SEQ ID N°133 à SEQ ID N°173, SEQ ID N°175 à SEQ ID N°180, tels que définis ci-après :

| **Peptide SEQ ID N°** | **Séquence d'acides aminés** | **Position du peptide dans la ou les protéines IMP** |
|---|---|---|
| **SEQ ID N°106** | DTENLVNWFVER | 73-84 pour la protéine de SEQ N°85 |
| **SEQ ID N°107** | EILFGGCFVK | 170-179 pour la protéine de SEQ N°95 |
| **SEQ ID N°108** | EVNGWGVVPK | 42-51 pour les protéines de SEQ N°80, 81, 82, 84, 87, 90, 93, 96, 97 |
| **SEQ ID N°109** | GDASLMK | 219-225 pour la protéine de SEQ N°104 |
| **SEQ ID N°110** | GFNESK | 234-239 pour la protéine de SEQ N°85 |
| **SEQ ID N°111** | GFTIK | 85-89 pour la protéine de SEQ N°103 |
| **SEQ ID N°112** | GLNESK | 234-239 pour les protéines de SEQ N°80, 81, 82, 83, 84, 86, 88, 90, 91, 93, 94, 96, 97, 98, 99, 100, 101, 102 |
| **SEQ ID N°113** | GLNESR | 234-239 pour la protéine de SEQ N°92 |
| **SEQ ID N°114** | GSISSHFHSDSTGGIEWLNSR | 90-110 pour les protéines de SEQ N°80, 90, 93, 97, 98 |
| **SEQ ID N°115** | GSISSHFHSDSTGGIGWLNSR | 90-110 pour la protéine de SEQ N°96 |
| **SEQ ID N°116** | GVYVHTSFEEVK | 33-44 pour les protéines de SEQ N°86, 88 |
| **SEQ ID N°117** | GWGVVTK | 45-51 pour les protéines de SEQ N°88, 95, 103, 104, 105 |
| **SEQ ID N°118** | GWSWTK | 45-51 pour la protéine de SEQ N°86 |
| **SEQ ID N°119** | GYEIK | 85-89 pour la protéine de SEQ N°104 |
| **SEQ ID N°120** | HGLVFLVNTDAYLIDTPFAAK | 52-72 pour la protéine de SEQ N°105 |
| **SEQ ID N°121** | HGLVILVNTDAYLIDTPFTAK | 52-72 pour la protéine de SEQ N°89 |
| **SEQ ID N°122** | HGLVVLIGADAYLIDTPFTAK | 52-72 pour la protéine de SEQ N°95 |
| **SEQ ID N°123** | HGLVVLVDAEAYLIDTPFTAK | 52-72 pour les protéines de SEQ N°82, 94 |
| **SEQ ID N°124** | HGLVVLVK | 52-59 pour les protéines de SEQ N°86, 88 |
| **SEQ ID N°125** | HGLVVLVNAEAYLIDTPFTAK | 52-72 pour les protéines de SEQ N°80, 90, 93, 96, 97, 98 |
| **SEQ ID N°126** | HGLVVLVNNDAYLIDTPFTNK | 52-72 pour la protéine de SEQ N°103 |
| **SEQ ID N°127** | HGLVVLVNTDAYLIDTPFTAK | 52-72 pour les protéines de SEQ N°81, 85, 99, 101 |
| **SEQ ID N°128** | HGLVVLVNTEAYLIDTPFTAK | 52-72 pour les protéines de SEQ N°84, 87 |
| **SEQ ID N°129** | HSFNGVSYSLIK | 134-145 pour la protéine de SEQ N°86 |
| **SEQ ID N°130** | IEVFYPGPGHTQDNVVVWLPK | 148-168 pour les protéines de SEQ N°99, 101 |
| **SEQ ID N°131** | ILFGGCFIK | 171-179 pour les protéines de SEQ N°80, 82, 90, 93, 94, 96, 97, 98, 104 |
| **SEQ ID N°132** | ILFGGCFVK | 171-179 pour les protéines de SEQ N°83, 86, 88, 89, 91, 92, 95, 99, 100, 101, 102, 103, 105 |
| **SEQ ID N°133** | ILMEK | 200-204 pour la protéine de SEQ N°95 |
| **SEQ ID N°134** | ILMSK | 200-204 pour les protéines de SEQ N°83, 86, 87, 88, 91, 92, 100, 102, 103, 104 |
| **SEQ ID N°135** | LDEGVYVHTSFK | 30-41 pour la protéine de SEQ N°90 |
| **SEQ ID N°136** | LEEGVYVHTSFEEVK | 30-44 pour la protéine de SEQ N°86 |
| **SEQ ID N°137** | LEEGVYVHTSYEEVK | 30-44 pour la protéine de SEQ N°95 |
| **SEQ ID N°138** | LFVLCVCFLCSIT AAGAR | 4-21 pour la protéine de SEQ N°102 |
| **SEQ ID N°139** | LLISK | 200-204 pour les protéines de SEQ N°82, 94 |
| **SEQ ID N°140** | LLMSK | 200-204 pour les protéines de SEQ N°84, 85 |
| **SEQ ID N°141** | LLVSK | 200-204 pour la protéine de SEQ N°81 |
| **SEQ ID N°142** | LPDLK | 22-26 pour les protéines de SEQ N°80, 81, 82, 83, 84, 85, 86, 87, 88, 89, 90, 91, 92, 93, 94, 96, 97, 98, 99, 100, 101, 102, 103, 104, 105 |
| **SEQ ID N°143** | LTLEQAVK | 226-233 pour les protéines de SEQ N°80, 82, 90, 93, 94, 96, 97, 98 |
| **SEQ ID N°144** | LTWEQAVK | 226-233 pour les protéines de SEQ N°87, 89, 99, 101, 103, 105 |
| **SEQ ID N°145** | LTWEQTVK | 226-233 pour la protéine de SEQ N°85 |
| **SEQ ID N°146** | LVAWFVGR | 77-84 pour la protéine de SEQ N°103 |
| **SEQ ID N°147** | LVNWFIEHGYR | 77-87 pour la protéine de SEQ N°88 |
| **SEQ ID N°148** | LVNWFVER | 77-84 pour les protéines de SEQ N°83, 85, 86, 91, 92, 95, 99, 100, 101, 102, 104, 105 |
| **SEQ ID N°149** | LVTWFVER | 77-84 pour les protéines de SEQ N°80, 81, 82, 84, 87, 89, 90, 93, 94, 96, 97, 98 |
| **SEQ ID N°150** | LVVPGHSEVGDASLLK | 210-225 pour les protéines de SEQ N°93, 96, 97 |
| **SEQ ID N°151** | LVVPSHSDIGDASLLK | 210-225 pour les protéines de SEQ N°89, 99, 101 |
| **SEQ ID N°152** | LVVPSHSDIGDSSLLK | 210-225 pour la protéine de SEQ N°85 |
| **SEQ ID N°153** | LVVPSHSDVGDASLLK | 210-225 pour la protéine de SEQ N°105 |
| **SEQ ID N°154** | LVVPSHSEAGDASLLK | 210-225 pour les protéines de SEQ N°82, 94 |
| **SEQ ID N°155** | LVVPSHSEVGDASLLK | 210-225 pour les protéines de SEQ N°80, 81, 84, 90, 98 |
| **SEQ ID N°156** | LVVSGHSEIGNASLLK | 210-225 pour la protéine de SEQ N°103 |
| **SEQ ID N°157** | LVVSGHSETGDATHLK | 210-225 pour la protéine de SEQ N°95 |
| **SEQ ID N°158** | LVVSSHSDIGDVSLLK | 210-225 pour la protéine de SEQ N°86 |
| **SEQ ID N°159** | LVVSSHSEIGDASLLK | 210-225 pour les protéines de SEQ N°83, 91, 92, 100, 102 |
| **SEQ ID N°160** | LVVSSHSEIGNASLLQR | 210-226 pour la protéine de SEQ N°88 |
| **SEQ ID N°161** | LVVSSHSEK | 210-218 pour la protéine de SEQ N°104 |
| **SEQ ID N°162** | LVVSSHSETGNASLLK | 210-225 pour la protéine de SEQ N°87 |
| **SEQ ID N°163** | NDAYLIDTPITAK | 60-72 pour les protéines de SEQ N°86, 88 |
| **SEQ ID N°164** | NSFDGVSYWLAK | 134-145 pour la protéine de SEQ N°95 |
| **SEQ ID N°165** | NSFGGVNYWLVK | 134-145 pour les protéines de SEQ N°82, 94 |
| **SEQ ID N°166** | NSFSGASYWLVK | 134-145 pour les protéines de SEQ N°81, 84 |
| **SEQ ID N°167** | NSFSGGSYWLVNNK | 134-147 pour la protéine de SEQ N°87 |
| **SEQ ID N°168** | NSFSGVSYWLLK | 134-145 pour la protéine de SEQ N°105 |
| **SEQ ID N°169** | NSFSGVSYWLVK | 134-145 pour les protéines de SEQ N°83, 89, 91, 92, 99, 100, 101, 102, 103 |
| **SEQ ID N°170** | SIPTYASELTNELLK | 111-125 pour les protéines de SEQ N°80, 81, 82, 83, 84, 85, 86, 87, 90, 91, 92, 93, 94, 95, 96, 97, 98, 100, 102, 103, 104, 105 |
| **SEQ ID N°171** | TLEQAVK | 227-233 pour les protéines de SEQ N°80, 81, 82, 84, 90, 93, 94, 96, 97, 98 |
| **SEQ ID N°172** | TWEQALK | 227-233 pour la protéine de SEQ N°104 |
| **SEQ ID N°173** | TWEQAVK | 227-233 pour les protéines de SEQ N°83, 86, 87, 88, 89, 91, 92, 95, 99, 100, 101, 102, 103, 105 |
| **SEQ ID N°174** | VLFGGCFVK | 171-179 pour les protéines de SEQ N°81, 84, 85, 87 |
| **SEQ ID N°175** | VQATNSFSGVNYWLVK | 130-145 pour les protéines de SEQ N°80, 90, 93, 96, 97, 98 |
| **SEQ ID N°176** | VQATNSFSGVSYSLIK | 130-145 pour la protéine de SEQ N°88 |
| **SEQ ID N°177** | VQATNSFSGVSYWLVK | 130-145 pour la protéine de SEQ N°103 |
| **SEQ ID N°178** | YGNAK | 205-209 pour les protéines de SEQ N°89, 99, 101, 105 |
| **SEQ ID N°179** | YSFSEVSYWLVK | 134-145 pour la protéine de SEQ N°104 |
| **SEQ ID N°180** | YSFSGVSYWLVK | 134-145 pour la protéine de SEQ N°85 |

La détection d'un mécanisme de résistance aux carbapénèmes, induit par l'expression de la protéine IND, est caractérisée par la détection d'au moins un peptide appartenant à la protéine IND et à ses différents variants de séquences **SEQ ID N° 181** à **SEQ ID N°187.**

lesdits peptides étant choisis, de préférence, parmi les peptides de séquence SEQ ID N°188 à SEQ ID N°197, SEQ ID N°200, SEQ ID N°201, SEQ ID N°203 à SEQ ID N°262, tels que définis ci-après :

| **Peptide SEQ ID N°** | **Séquence d'acides aminés** | **Position du peptide dans la ou les protéines IND** |
|---|---|---|
| **SEQ ID N°188** | AATDLGYIK | 184-192 pour la protéine de SEQ N°184 |
| **SEQ ID N°189** | AGDLSFFNNK | 102-111 pour les protéines de SEQ N°181, 184, 186, 187 |
| **SEQ ID N°190** | AGDLSFYNK | 103-111 pour la protéine de SEQ N°185 |
| **SEQ ID N°191** | AGDLSFYNQK | 102-111 pour les protéines de SEQ N°182, 183 |
| **SEQ ID N°192** | AQYQSLMDTIK | 72-82 pour les protéines de SEQ N°186, 187 |
| **SEQ ID N°193** | ASLVIPGHDEWK | 213-224 pour la protéine de SEQ N°184 |
| **SEQ ID N°194** | ATLIIPGHDDWK | 213-224 pour la protéine de SEQ N°186 |
| **SEQ ID N°195** | ATLIIPGHDEWK | 213-224 pour la protéine de SEQ N°181 |
| **SEQ ID N°196** | ATSSK | 132-136 pour la protéine de SEQ N°185 |
| **SEQ ID N°197** | ATSTEIIK | 131-138 pour les protéines de SEQ N°181, 182, 183, 184, 186 |
| **SEQ ID N°198** | ATSTELIK | 131-138 pour la protéine de SEQ N°187 |
| **SEQ ID N°199** | ATSTELIKPGK | 131-141 pour la protéine de SEQ N°187 |
| **SEQ ID N°200** | A TSTELIKPGKPYR | 131-144 pour la protéine de SEQ N°187 |
| **SEQ ID N°201** | DFVIEPPIK | 24-32 pour les protéines de SEQ N°181, 184, 186, 187 |
| **SEQ ID N°202** | DFVIEPPVK | 24-32 pour les protéines de SEQ N°182, 183 |
| **SEQ ID N°203** | DFVIEPPVKPNLYLYK | 24-39 pour les protéines de SEQ N°182, 183 |
| **SEQ ID N°204** | DFVIEQPFGK | 25-34 pour la protéine de SEQ N°185 |
| **SEQ ID N°205** | EANLEQWPK | 193-201 pour la protéine de SEQ N°187 |
| **SEQ ID N°206** | EANVEQWPITIDK | 193-205 pour la protéine de SEQ N°186 |
| **SEQ ID N°207** | EANVEQWPK | 193-201 pour les protéines de SEQ N°181, 184 |
| **SEQ ID N°208** | EQYQTLMDTIQK | 72-83 pour les protéines de SEQ N°182, 183 |
| **SEQ ID N°209** | EYSANAVYLTTK | 48-59 pour les protéines de SEQ N°182, 183 |
| **SEQ ID N°210** | EYSANSMYLVTK | 48-59 pour la protéine de SEQ N°181 |
| **SEQ ID N°211** | EYSANSVYLVTK | 48-59 pour les protéines de SEQ N°186, 187 |
| **SEQ ID N°212** | EYSANSVYLVTQK | 48-60 pour la protéine de SEQ N°184 |
| **SEQ ID N°213** | EYSTNALYLVTK | 49-60 pour la protéine de SEQ N°185 |
| **SEQ ID N°214** | FFIVSILLSPFASAQVK | 7-23 pour la protéine de SEQ N°181 |
| **SEQ ID N°215** | GGGHVEHTLELLDK | 225-238 pour la protéine de SEQ N°187 |
| **SEQ ID N°216** | GGGHVEHTLELLNK | 225-238 pour les protéines de SEQ N°181, 186 |
| **SEQ ID N°217** | GGGHVK | 225-230 pour la protéine de SEQ N°184 |
| **SEQ ID N°218** | GGGHVQHTLDLLDK | 225-238 pour les protéines de SEQ N°182, 183 |
| **SEQ ID N°219** | GIPTYATAK | 113-121 pour la protéine de SEQ N°185 |
| **SEQ ID N°220** | GNDHVK | 226-231 pour la protéine de SEQ N°185 |
| **SEQ ID N°221** | GWLFDVPWEK | 61-71 pour les protéines de SEQ N°181, 184, 186, 187 |
| **SEQ ID N°222** | GWLFDVPWQK | 62-72 pour la protéine de SEQ N°185; 61-71 pour la protéine de séquence SEQ ID N°182; 61-71 pour la protéine de séquence SEQ ID N°183 |
| **SEQ ID N°223** | HHLPVIAVFATHSHDDR | 85-101 pour les protéines de SEQ N°182, 183 |
| **SEQ ID N°224** | HNLPVIAVFA THSHDDR | 85-101 pour les protéines de SEQ N°184, 186 |
| **SEQ ID N°225** | HNLPVIAVFATHSHSDR | 86-102 pour la protéine de SEQ N°185 |
| **SEQ ID N°226** | HNLPVVAVFATHSHDDR | 85-101 pour les protéines de SEQ N°181, 187 |
| **SEQ ID N°227** | HTLELLDQQK | 232-241 pour la protéine de SEQ N°185 |
| **SEQ ID N°228** | HTLELLNK | 231-238 pour les protéines de SEQ N°181, 184, 186 |
| **SEQ ID N°229** | IFTVLSLFLINFFNAQAR | 7-24 pour la protéine de SEQ N°185 |
| **SEQ ID N°230** | IQFFMVSMMLAPMFNAQVK | 5-23 pour la protéine de SEQ N°186 |
| **SEQ ID N°231** | IQFFMVSMMLSSLFSAQVK | 5-23 pour la protéine de SEQ N°184 |
| **SEQ ID N°232** | IQFFMVSMMLTPLFSAQVK | 5-23 pour la protéine de SEQ N°187 |
| **SEQ ID N°233** | IQYQSLMDTIK | 72-82 pour la protéine de SEQ N°181 |
| **SEQ ID N°234** | NLHIYK | 34-39 pour les protéines de SEQ N°181, 184, 186 |
| **SEQ ID N°235** | NLYIYK | 34-39 pour la protéine de SEQ N°187 |
| **SEQ ID N°236** | NNLHIYK | 33-39 pour les protéines de SEQ N°181, 186 |
| **SEQ ID N°237** | QLYLYK | 35-40 pour la protéine de SEQ N°185 |
| **SEQ ID N°238** | QWPETMR | 198-204 pour les protéines de SEQ N°182, 183 |
| **SEQ ID N°239** | SFGVFGGK | 40-47 pour les protéines de SEQ N°182, 183 |
| **SEQ ID N°240** | SIQLLMMSMFLSPLINAQVK | 4-23 pour les protéines de SEQ N°182, 183 |
| **SEQ ID N°241** | SNSATDLGYIK | 182-192 pour les protéines de SEQ N°181, 186, 187 |
| **SEQ ID N°242** | TATDLGYTGEANVK | 184-197 pour les protéines de SEQ N°182, 183 |
| **SEQ ID N°243** | TFGVFDGK | 41-48 pour la protéine de SEQ N°185 |
| **SEQ ID N°244** | TFGVFGGK | 40-47 pour les protéines de SEQ N°181, 184, 186, 187 |
| **SEQ ID N°245** | TGKPYK | 139-144 pour les protéines de SEQ N°182, 183 |
| **SEQ ID N°246** | TGKPYR | 139-144 pour les protéines de SEQ N°181, 184, 186 |
| **SEQ ID N°247** | TGVVLFDVPWEK | 60-71 pour la protéine de SEQ N°187 |
| **SEQ ID N°248** | TNEFLK | 121-126 pour les protéines de SEQ N°181, 184, 186 |
| **SEQ ID N°249** | TNELLK | 122-127 pour la protéine de SEQ N°185; 121-126 pour les protéines de séquence SEQ ID N°182, 183 |
| **SEQ ID N°250** | TNQFLK | 121-126 pour la protéine de SEQ N°187 |
| **SEQ ID N°251** | TQYQSLMDTIK | 73-83 pour la protéine de SEQ N°185 |
| **SEQ ID N°252** | TYATAK | 116-121 pour la protéine de SEQ N°185; 115-120 pour les protéines de séquence SEQ ID N°181, 182, 183 |
| **SEQ ID N°253** | TYATPK | 115-120 pour la protéine de SEQ N°187 |
| **SEQ ID N°254** | TYATSK | 115-120 pour les protéines de SEQ N°184, 186 |
| **SEQ ID _{N°255}** | VIPGHDEWK | 217-225 pour la protéine de SEQ N°185; 216-224 pour la protéine de séquence SEQ ID N°182; 216-224 pour la protéine de séquence SEQ ID N°184 |
| **SEQ ID N°256** | VLDGGCLVK | 173-181 pour les protéines de SEQ N°181, 182, 183, 184, 186, 187; 174-182 pour la protéine de séquence SEQ ID N°185 |
| **SEQ ID N°257** | VQYQSLMDTIQK | 72-83 pour la protéine de SEQ N°184 |
| **SEQ ID N°258** | YAQATLVIPGHDEWK | 210-224 pour la protéine de SEQ N°182 |
| **SEQ ID N°259** | YAQATLVIPGHEEWK | 210-224 pour la protéine de SEQ N°183 |
| **SEQ ID N°260** | YNVLDGGCLVK | 171-181 pour les protéines de SEQ N°181, 184, 186; 172-182 pour la protéine de séquence SEQ ID N°185 |
| **SEQ ID N°261** | YPSTAK | 211-216 pour la protéine de SEQ N°185 |
| **SEQ ID N°262** | YSEAVLIIPGHDEWK | 210-224 pour la protéine de SEQ N°187 |

La détection d'un mécanisme de résistance aux carbapénèmes, induit par l'expression de la protéine SME, est caractérisée par la détection d'au moins un peptide appartenant à la protéine SME et à ses différents variants de séquences **SEQ ID N° 263** à **SEQ ID N°265.** lesdits peptides étant choisis, de préférence, parmi les peptides de séquence **SEQ ID N°266** à **SEQ ID N°287** tels que définis ci-après :

| **Peptide SEQ ID N°** | **Séquence d'acides aminés** | **Position du peptide dans la ou les protéines SME** |
|---|---|---|
| **SEQ ID N°266** | AIYQNWLK | 209-216 pour les protéines de SEQ N°263,264,265 |
| **SEQ ID N°267** | APLIVSIYTTR | 260-270 pour les protéines de SEQ N°263,264,265 |
| **SEQ ID N°268** | ASVPADWVVGDK | 227-238 pour les protéines de SEQ N°263,264,265 |
| **SEQ ID N°269** | AVANSLNK | 189-196 pour les protéines de SEQ N°263, 264, 265 |
| **SEQ ID N°270** | DLEYHSPITTK | 104-114 pour les protéines de SEQ N°263, 265 |
| **SEQ ID N°271** | DLEYYSPITTK | 104-114 pour la protéine de SEQ N°264 |
| **SEQ ID N°272** | DTSTPK | 183-188 pour les protéines de SEQ N°263, 264, 265 |
| **SEQ ID N°273** | FLGGPEGMTK | 145-154 pour les protéines de SEQ N°263, 264, 265 |
| **SEQ ID N°274** | ...... GFLAAAVLER | 77-86 pour les protéines de SEQ N°263, 264, 265 |
| **SEQ ID N°275** | GNTTGDAR | 217-224 pour les protéines de SEQ N°263,264,265 |
| **SEQ ID N°276** | IGVFAIDTGSGNTFGYR | 48-64 pour les protéines de SEQ N°263, 264,265 |
| **SEQ ID N°277** | LALGNVLNAK | 197-206 pour les protéines de SEQ N°263,264,265 |
| **SEQ ID N°278** | LDINQK | 92-97 pour les protéines de SEQ N°263, 264,265 |
| **SEQ ID N°279** | LEEDFDGR | 40-47 pour les protéines de SEQ N°263, 264,265 |
| **SEQ ID N°280** | SDAAAK | 30-35 pour les protéines de SEQ N°263, 264,265 |
| **SEQ ID N°281** | SIGDNEFR | 158-165 pour les protéines de SEQ N°263,264,265 |
| **SEQ ID N°282** | TASFLFSVCLALSAFNAHANK | 9-29 pour les protéines de SEQ N°263, 264,265 |
| **SEQ ID N°283** | TGSCGAIGTANDYAVIWPK | 239-257 pour la protéine de SEQ N°265 |
| **SEQ ID N°284** | TGSCGAYGTANDYAVIWPK | 239-257 pour les protéines de SEQ N°263, 264 |
| **SEQ ID N°285** | TIAEASR | 281-287 pour les protéines de SEQ N°263, 264, 265 |
| **SEQ ID N°286** | WELELNTAIPGDK | 169-181 pour les protéines de SEQ N°263,264,265 |
| **SEQ ID N°287** | FPLCSSFK | 69-76 pour les protéines de SEQ N°263, 264,265 |

La détection d'un mécanisme de résistance aux carbapénèmes, induit par l'expression d'une protéine VIM, est caractérisée par la détection d'au moins un peptide appartenant à une protéine VIM et à ses différents variants de séquences **SEQ ID N° 288** à **SEQ ID N°313.** lesdits peptides étant choisis, de préférence, parmi les peptides de séquence **SEQ ID N°314** à **SEQ ID N°346** tels que définis ci-après :

| **Peptide SEQ ID N°** | **Séquence d'acides aminés** | **Position du peptide dans la ou les protéines VIM** |
|---|---|---|
| **SEQ ID N°314** | AAGVATYASPSAR | 128-140 pour la protéine de SEQ N°293 |
| **SEQ ID N°315** | AAGVATYASPSIR | 128-140 pour la protéine de SEQ N°294 |
| **SEQ ID N°316** | AAGVATYASPSTR | 128-140 pour les protéines de SEQ N°288, 289, 290, 292, 295, 296, 297, 298, 299, 300, 301, 302, 303, 304, 305, 307, 308, 309, 310, 312, 313 |
| **SEQ ID N°317** | AAGVATYTSPLTR | 127-139 pour la protéine de SEQ N°311 |
| **SEQ ID N°318** | AGVATYASPSTR | 129-140 pour les protéines de SEQ N°288, 289, 290, 291, 292, 295, 296, 297, 298, 299, 300, 301, 302, 303, 304, 305, 306, 307, 308, 309, 310,312,313 |
| **SEQ ID N°319** | AHTNR | 254-258 pour la protéine de SEQ N°312; 258-262 pour les protéines de séquence SEQ ID N°288, 289, 292, 293, 294, 295, 296, 298, 299, 300,301,302,303,305,306,309 |
| **SEQ ID N°320** | ALSSSGDVVR | 156-165 pour la protéine de SEQ N°311 |
| **SEQ ID N°321** | AVSTHFHDDR | 110-119 pour les protéines de SEQ N°288, 289, 290, 291, 292, 293, 294, 295, 296, 297, 298, 300, 301, 302, 303, 304, 305, 306, 307, 308, 309,310,312,313 |
| **SEQ ID N°322** | DADELLLIDTAWGAK | 75-89 pour la protéine de SEQ N°311 |
| **SEQ ID N°323** | DGDELLLIDTAWGAK | 76-90 pour les protéines de SEQ N°288, 289, 290, 291, 292, 293, 294, 295, 296, 297, 298, 300, 301, 302, 303, 304, 305, 306, 307, 308, 309,310,312,313 |
| **SEQ ID N°324** | DGDELLLIDTAWGTK | 76-90 pour la protéine de SEQ N°299 |
| **SEQ ID N°325** | ESAGNVADANLAEWPATIK | 205-223 pour la protéine de SEQ N°311 |
| **SEQ ID N°326** | GEYPTVSEIPVGEVR | 31-45 pour les protéines de SEQ N°288, 289, 292,293,294,295,296,299,300,301,302, 303, 305, 312 |
| **SEQ ID N°327** | HTTNVVK | 247-253 pour la protéine de SEQ N°312; 251-257 pour les protéines de séquence SEQ ID N°288, 289, 292, 293, 294, 295, 296, 298, 300, 301, 302, 303, 305, 306, 309; 250-256 pour la protéine de séquence SEQ ID N°311 |
| **SEQ ID N°328** | IGDGVWSHIATQK | 48-60 pour la protéine de SEQ N°311 |
| **SEQ ID N°329** | LANEIPTHSLEGLSSSGDAVR | 142-162 pour la protéine de SEQ N°312 |
| **SEQ ID N°330** | LGDTVYSSNGLIVR | 61-74 pour la protéine de SEQ N°311 |
| **SEQ ID N°331** | LYQIADGVWSHIATK | 46-60 pour la protéine de SEQ N°289 |
| **SEQ ID N°332** | LYQIADGVWSHIATR | 46-60 pour les protéines de SEQ N°292, 312 |
| **SEQ ID N°333** | NTAALLAEIEK | 91-101 pour les protéines de SEQ N°288, 289, 290, 291, 292, 293, 294, 295, 296, 297, 298, 300, 301, 302, 303, 304, 305, 306, 307, 308, 309,310,312,313 |
| **SEQ ID N°334** | NTVALLAEIEK | 90-100 pour la protéine de SEQ N°311; 91-101 pour la protéine de séquence SEQ ID N°299 |
| **SEQ ID N°335** | QIGLPVTR | 102-109 pour les protéines de SEQ N°288, 289, 290, 291, 292, 293, 294, 295, 296, 297, 298, 299,300,301,302,303,304,305,306,307, 308, 309, 310, 312, 313; 101-108 pour la protéine de séquence SEQ ID N°311 |
| **SEQ ID N°336** | QLAEAAGNEVPAHSLK | 140-155 pour la protéine de SEQ N°311 |
| **SEQ ID N°337** | SFDGAVYPSNGLIVR | 61-75 pour les protéines de SEQ N°288, 289, 290, 291, 292, 293, 294, 295, 296, 297, 298, 300, 301, 302, 303, 304, 305, 306, 307, 308, 309,310,312,313 |
| **SEQ ID N°338** | SISTHFHDDR | 109-118 pour la protéine de SEQ N°311 |
| **SEQ ID N°339** | SVSTHFHDDR | 110-119 pour la protéine de SEQ N°299 |
| **SEQ ID N°340** | TSAGNVADADLAEWPGSVER | 206-225 pour la protéine de SEQ N°299 |
| **SEQ ID N°341** | TSAGNVADADLAEWPTSIER | 202-221 pour la protéine de SEQ N°312; 206-N°225 pour la protéine de séquence SEQ ID N°288, 289, 292, 293, 294, 295, 296, 298, 300, 301,302,303,305,309 |
| **SEQ ID N°342** | TSAGNVADADLAEWPTSVER | 206-225 pour les Protéines de SEQ N°290, 291, 297, 304, 306, 307, 308, 310, 313 |
| **SEQ ID N°343** | VGGVDALR | 120-127 pour la protéine de SEQ N°299 |
| **SEQ ID N°344** | VGGVDVLR | 120-127 pour les protéines de SEQ N°288, 289, 290, 291, 292, 293, 294, 295, 296, 297, 298, 300,301,302,303,304,305,306,307,308, 309, 310, 312, 313; 119-126 pour la protéine de séquence SEQ ID N°311 |
| **SEQ ID N°345** | VLFGGCAVHEASR | 192-204 pour la protéine de SEQ N°311 |
| **SEQ ID N°346** | VLYGGCAVHELSR | 193-205 pour les protéines de SEQ N°290, 297, 304 |

La détection d'un mécanisme de résistance aux carbapénèmes et/ou aux céphalosporines, induit par l'expression d'une protéine OXA, est caractérisée par la détection d'au moins un peptide appartenant à une protéine OXA et à ses différents variants de séquences **SEQ ID N° 347** à **SEQ ID N°508.** lesdits peptides étant choisis, de préférence, parmi les peptides de séquence SEQ ID N°509 à SEQ ID N°523, SEQ ID N°525 à SEQ ID N°572, SEQ ID N°574 à SEQ ID N°604, SEQ ID N°606 à SEQ ID N°618, SEQ ID N°620 à SEQ ID N°696, SEQ ID N°698 à SEQ ID N°1077 et SEQ ID N°1098 à SEQ ID N°1109, tels que définis ci-après :

| **Peptide SEQ ID N°** | **Séquence d'acides aminés** | **Position du peptide dans la ou les protéines OXA** | **Intérêt clinique** |
|---|---|---|---|
| **SEQ ID N°509** | AAAYELAENLFEAGQADGWR | 183-202 pour la protéine de SEQ N°433 | 2d |
| **SEQ ID N°510** | AAEGFIPASTFK | 86-97 pour la protéine de SEQ N°381 | 2df |
| **SEQ ID N°511** | AALGR | 41-45 pour la protéine de SEQ N°382 | 2df |
| **SEQ ID N°512** | ADGQVVAFALNMQMK | 241-255 pour les protéines de SEQ N°410, 413, 458 | 2df |
| **SEQ ID N°513** | ADINEIFK | 95-102 pour la protéine de SEQ N°366 | 2df |
| **SEQ ID N°514** | ADWGK | 50-54 pour la protéine de SEQ N°382 | 2df |
| **SEQ ID N°515** | AEGAIVISDER | 40-50 pour les protéines de SEQ N°369, 372 | OXA |
| **SEQ ID N°516** | AFALNLDIDK | 222-231 pour la protéine de SEQ N°385 | 2d |
| **SEQ ID N°517** | AFAPMSTFK | 49-57 pour la protéine de SEQ N°498; 60-68 pour la protéine de séquence SEQ ID N°351 | OXA |
| **SEQ ID N°518** | AFGYGNADVSGDPGQNNGLDR | 127-147 pour les protéines de SEQ N°496, 497, 499, 500 | 2d |
| **SEQ ID N°519** | AFTMTK | 174-179 pour la protéine de SEQ N°494 | 2de |
| **SEQ ID N°520** | AGDDIALR | 256-263 pour les protéines de SEQ N°410, 413, 458 | 2df |
| **SEQ ID N°521** | AGHVYAFALNIDMPR | 233-247 pour la protéine de SEQ N°379 | 2df |
| **SEQ ID N°522** | AGLWR | 11-15 pour la protéine de SEQ N°382 | 2df |
| **SEQ ID N°523** | AHTEYVPASTFK | 73-84 pour les protéines de SEQ N°447, 466 | 2df |
| **SEQ ID N°524** | AIIPWDGK | 112-119 pour la protéine de SEQ N°382 | 2df |
| **SEQ ID N°525** | AIIPWDGKPR | 112-121 pour la protéine de SEQ N°382 | 2df |
| **SEQ ID N°526** | AISDITITR | 190-198 pour la protéine de SEQ N°386 | 2d |
| **SEQ ID N°527** | AISGK | 82-86 pour la protéine de SEQ N°470 | 2df |
| **SEQ ID N°528** | ALGQDR | 121-126 pour la protéine de SEQ N°433 | 2d |
| **SEQ ID N°529** | ALPDLAK | 256-262 pour la protéine de SEQ N°498 | 2d |
| **SEQ ID N°530** | ALQAK | 254-258 pour la protéine de SEQ N°470 | 2df |
| **SEQ ID N°531** | AMETFSPASTFK | 50-61 pour la protéine de SEQ N°385 | 2d |
| **SEQ ID N°532** | AMLFLQER | 196-203 pour la protéine de SEQ N°469 | 2df |
| **SEQ ID N°533** | AMLVFDPVR | 55-63 pour les protéines de SEQ N°350, 367, 370, 415, 443, 481, 488; 44-52 pour la protéine de séquence SEQ ID N°362 | OXA |
| **SEQ ID N°534** | AMTLLESGPGWELHGK | 189-204 pour la protéine de SEQ N°380 | 2d |
| **SEQ ID N°535** | ANLHITLHGK | 199-208 pour la protéine de SEQ N°386 | 2d |
| **SEQ ID N°536** | ANQLIVK | 183-189 pour les protéines de SEQ N°489, 505 | OXA |
| **SEQ ID N°537** | ANTEYVPASTFK | 71-82 pour les protéines de SEQ N°366, 374, 387, 440, 441, 459, 460, 461, 462, 463, 464, 465, 486, 493; 66-77 pour la protéine de séquence SEQ ID N°469 | 2df |
| **SEQ ID N°538** | ANVSR | 133-137 pour la protéine de SEQ N°380 | 2d |
| **SEQ ID N°539** | APIGWFIGWATR | 224-235 pour la protéine de SEQ N°351 | 2de |
| **SEQ ID N°540** | APLGWFIGWATHEER | 213-227 pour la protéine de SEQ N°498 | 2d |
| **SEQ ID N°541** | AQDEVQSMLFIEEK | 196-209 pour la protéine de SEQ N°403 | 2df |
| **SEQ ID N°542** | AQGVIVLWNENK | 40-51 pour la protéine de SEQ N°384 | 2df |
| **SEQ ID N°543** | ASAIAVYQDLAR | 126-137 pour la protéine de SEQ N°467 | 2df |
| **SEQ ID N°544** | ASAILVYQDLAR | 126-137 pour les protéines de SEQ N°417, 426, 457, 472 | 2df |
| **SEQ ID N°545** | ASAIPVYQDLAR | 126-137 pour les protéines de SEQ N°388, 389, 390, 391, 392, 393, 394, 395, 396, 398, 399, 402, 403, 404, 405, 406, 407, 408, 409, 411, 412, 416, 418, 419, 420, 423, 424, 425, 427, 428, 434, 436, 437, 439, 444, 445, 446, 448, 453, 454, 455, 456, 468, 475, 478, 479, 480, 482, 483, 495, 502, 503, 507, 508; 120-131 pour les protéines de séquence SEQ ID N°431, 432 | 2df |
| **SEQ ID N°546** | ASAIPVYQDLPR | 126-137 pour la protéine de SEQ N°397 | 2df |
| **SEQ ID N°547** | ASAIQVYQDLAR | 126-137 pour les protéines de SEQ N°421, 473 | 2df |
| **SEQ ID N°548** | ASAISVYQDLAR | 126-137 pour les protéines de SEQ N°400, 487 | 2df |
| **SEQ ID N°549** | ASALPVYQDLAR | 126-137 pour les protéines de SEQ N°401, 422 | 2df |
| **SEQ ID N°550** | ASAMPVYQDLAR | 126-137 pour la protéine de SEQ N°474 | 2df |
| **SEQ ID N°551** | ASAVPVYQDLAR | 126-137 pour les protéines de SEQ N°438, 451, 452 | 2df |
| **SEQ ID N°552** | ASIEYVPASTFK | 72-83 pour les protéines de SEQ N°399, 403 | 2df |
| **SEQ ID N°553** | ASNVPVYQELAR | 113-124 pour la protéine de SEQ N°380 | 2d |
| **SEQ ID N°554** | ASPASTFK | 49-56 pour les protéines de SEQ N°496, 497, 499, 500 | 2d |
| **SEQ ID N°555** | ASTAYIPASTFK | 59-70 pour les protéines de SEQ N°489, 505 | OXA |
| **SEQ ID N°556** | ASTEYVPASTFK | 72-83 pour les protéines de SEQ N°388, 389, 390, 391, 392, 393, 394, 395, 396, 397, 398, 400, 401, 402, 404, 405, 406, 407, 408, 409, 411, 412, 416, 417, 418, 419, 420, 421, 422, 423, 424, 425, 426, 427, 428, 434, 436, 437, 438, 439, 444, 445, 446, 448, 451, 452, 453, 454, 455, 456, 457, 467, 468, 472, 473, 474, 475, 478, 479, 480, 482, 483, 487, 495, 502, 503, 507, 508; 66-77 pour les protéines de séquence SEQ ID N°431, 432 | 2df |
| **SEQ ID N°557** | ASTTEVFK | 96-103 pour la protéine de SEQ N°444 | 2df |
| **SEQ ID N°558** | ATSTEIFK | 99-106 pour les protéines de SEQ N°410, 413, 458 | 2df |
| **SEQ ID N°559** | A TTNEIFK | 97-104 pour les protéines de SEQ N°364, 365, 371, 414, 449, 466, 506; 90-97 pour la protéine de séquence SEQ ID N°469 | 2df |
| **SEQ ID N°560** | ATTTAVFK | 96-103 pour la protéine de SEQ N°396 | 2df |
| **SEQ ID N°561** | A TTTEIFK | 97-104 pour la protéine de SEQ N°447; 96-103 pour les protéines de séquence SEQ ID N°399, 403, 411 | 2df |
| **SEQ ID N°562** | ATTTEVFK | 96-103 pour les protéines de SEQ N°388, 389, 390, 391, 392, 393, 394, 397, 398, 400, 401, 402, 404, 405, 406, 407, 408, 409, 412, 416, 417, 418, 419, 420, 421, 422, 423, 424, 425, 426, 427, 434, 436, 437, 438, 439, 445, 446, 448, 451, 452, 453, 454, 455, 456, 457, 467, 468, 472, 473, 474, 475, 478, 479, 480, 482, 483, 487, 495, 502, 503, 507, 508; 90-97 pour les protéines de séquence SEQ ID N°431, 432 | 2df |
| **SEQ ID N°563** | AVSDITILEQTDNYTLHGK | 191-209 pour la protéine de SEQ N°477 | OXA |
| **SEQ ID N°564** | AVSDITILEQTYNYTLHGK | 191-209 pour les protéines de SEQ N°429, 430 | 2d |
| **SEQ ID N°565** | AVVPHFEAGDWDVQGK | 195-210 pour la protéine de SEQ N°351 | 2de |
| **SEQ ID N°566** | A WEHDMSLR | 100-108 pour la protéine de SEQ N°380 | 2d |
| **SEQ ID N°567** | AWIGSSLQISPLEQLEFLGK | 148-167 pour les protéines de SEQ N°496, 497, 499, 500 | 2d |
| **SEQ ID N°568** | CAAQMAPDSTFK | 63-74 pour la protéine de SEQ N°376 | 2d |
| **SEQ ID N°569** | CATQMAPDSTFK | 48-59 pour la protéine de SEQ N°361; 63-74 pour la protéine de séquence SEQ ID N°360 | 2d |
| **SEQ ID N°570** | CTIIADAITGNTLYETGECAR | 32-52 pour la protéine de SEQ N°349 | 2d |
| **SEQ ID N°571** | DAFLK | 251-255 pour la protéine de SEQ N°498 | 2d |
| **SEQ ID N°572** | DDFILHGK | 189-196 pour la protéine de SEQ N°377 | 2d |
| **SEQ ID N°573** | DDQEVLPYGGK | 92-102 pour la protéine de SEQ N°379 | 2df |
| **SEQ ID N°574** | DDVLK | 87-91 pour la protéine de SEQ N°470 | 2df |
| **SEQ ID N°575** | DEFHVFR | 90-96 pour les protéines de SEQ N°355, 363 | 2d |
| **SEQ ID N°576** | DEFQIFR | 90-96 pour les protéines de SEQ N°350, 367, 370, 375, 415, 443, 481, 488; 79-85 pour la protéine de séquence SEQ ID N°362 | OXA |
| **SEQ ID N°577** | DEFQVFR | 90-96 pour les protéines de SEQ N°356, 369, 372, 373, 484, 491 | 2d |
| **SEQ ID N°578** | DELVR | 260-264 pour la protéine de SEQ N°43 3 | 2d |
| **SEQ ID N°579** | DETSR | 112-116 pour la protéine de SEQ N°3 81 | 2df |
| **SEQ ID N°580** | DFDYGNQDFSGDK | 141-153 pour les protéines de SEQ N°360, 376; 126-13 8 pour la protéine de séquence SEQ ID N°361 | 2d |
| **SEQ ID N°581** | DFTLGEAMQASTVPVYQELAR | 120-140 pour les protéines de SEQ N°410, 413, 458 | 2df |
| **SEQ ID N°582** | DGNITS VAINIDMLSEADAPK | 250-270 pour la protéine de SEQ N°382 | 2df |
| **SEQ ID N°583** | DHDLITAMK | 108-116 pour les protéines de SEQ N°384, 485 | 2df |
| **SEQ ID N°584** | DIAAWNR | 101-107 pour les protéines de SEQ N°384, 485 | 2df |
| **SEQ ID N°585** | DIGEDK | 131-136 pour la protéine de SEQ N°373 | 2d |
| **SEQ ID N°586** | DILYIQELAGGWK | 180-192 pour la protéine de SEQ N°494 | 2de |
| **SEQ ID N°587** | DITILEK | 181-187 pour la protéine de SEQ N°377 | 2d |
| **SEQ ID N°588** | DLLSAK | 166-171 pour la protéine de SEQ N°498 | 2d |
| **SEQ ID N°589** | DLMITEAGR | 195-203 pour les protéines de SEQ N°369, 372, 373 | 2d |
| **SEQ ID N°590** | DLMIVEAGR | 195-203 pour les protéines de SEQ N°350, 356, 367, 370, 375, 415, 443, 481, 488, 491; 184-192 pour la protéine de séquence SEQ ID N°362 | OXA |
| **SEQ ID N°591** | DLMIVEAK | 195-202 pour les protéines de SEQ N°355, 363 | 2d |
| **SEQ ID N°592** | DLPLR | 243-247 pour la protéine de SEQ N°470 | 2df |
| **SEQ ID N°593** | DLSGNPGK | 131-138 pour la protéine de SEQ N°347 | 2d |
| **SEQ ID N°594** | DLSLR | 105-109 pour les protéines de SEQ N°357, 358, 359, 368, 383, 442, 471, 504; 106-110 pour la protéine de séquence SEQ ID N°490 | OXA |
| **SEQ ID N°595** | DLTLR | 105-109 pour les protéines de SEQ N°352, 435, 492, 501, 505; 96-100 pour les protéines de séquence SEQ ID N°348, 353, 354 | OXA |
| **SEQ ID N°596** | DMTLGDAIK | 117-125 pour les protéines de SEQ N°468, 508 | 2df |
| **SEQ ID N°597** | | 118-13 8 pour la protéine de SEQ N°447 | 2df |
| **SEQ ID N°598** | DMTLGDAMK | 117-125 pour les protéines de SEQ N°389, 390, 391, 394, 395, 398, 399, 400, 401, 403, 404, 407, 408, 409, 411, 412, 417, 418, 420, 421, 423, 425, 426, 427, 428, 436, 437, 438, 439, 444, 451, 452, 453, 454, 455, 456, 457, 467, 472, 473, 474, 478, 479, 480, 482, 483, 487, 495, 502, 507 | 2df |
| **SEQ ID N°599** | | 118-13 8 pour la protéine de SEQ N°466 | 2df |
| **SEQ ID N°600** | DMTLGEAMALSAVPVYQELAR | 118-138 pour les protéines de SEQ N°364, 365, 371, 414, 449, 506 | 2df |
| **SEQ ID N°601** | DMTLGEAMK | 116-124 pour les protéines de SEQ N°366, 374, 387, 440, 441, 459, 460, 461, 462, 463, 464, 465, 486, 493 | 2df |
| **SEQ ID N°602** | | 111-131 pour la protéine de SEQ N°469 | 2df |
| **SEQ ID N°603** | DNNGK | 214-218 pour la protéine de SEQ N°349 | 2d |
| **SEQ ID N°604** | DQDLR | 110-114 pour les protéines de SEQ N°350, 356, 367, 369, 370, 372, 373, 375, 415, 443, 481, 484, 488, 491; 99-103 pour la protéine de séquence SEQ ID N°362 | 2d |
| **SEQ ID N°605** | DQQIGWFVGWASK | 213-225 pour la protéine de SEQ N°347 | 2d |
| **SEQ ID N°606** | DQQIGWFVGWASKPGK | 213-228 pour la protéine de SEQ N°347 | 2d |
| **SEQ ID N°607** | DQQVQVYGNDLNR | 53-65 pour la protéine de SEQ N°469 | 2df |
| **SEQ ID N°608** | DQSFR | 132-136 pour la protéine de SEQ N°381 | 2df |
| **SEQ ID N°609** | DQTLESAFK | 105-113 pour la protéine de SEQ N°470 | 2df |
| **SEQ ID N°610** | DSCVWYSQVLTQQLGMTR | 98-115 pour la protéine de SEQ N°494 | 2de |
| **SEQ ID N°611** | DSIVWYSQELTR | 113-124 pour la protéine de SEQ N°351 | 2de |
| **SEQ ID N°612** | DSIVWYSQQLTR | 102-113 pour la protéine de SEQ N°498 | 2d |
| **SEQ ID N°613** | DSNLR | 109-113 pour les protéines de SEQ N°429, 430, 477; 96-100 pour les protéines de séquence SEQ ID N°377, 386 | 2d |
| **SEQ ID N°614** | DSYIAWGGEAWK | 81-92 pour les protéines de SEQ N°496, 497, 499, 500 | 2d |
| **SEQ ID N°615** | DTLNPEWPYK | 67-76 pour la protéine de SEQ N°494 | 2de |
| **SEQ ID N°616** | DVDEVFYK | 88-95 pour les protéines de SEQ N°386, 429, 430, 477 | 2d |
| **SEQ ID N°617** | DVSGDPGK | 144-151 pour la protéine de SEQ N°351 | 2de |
| **SEQ ID N°618** | DWILR | 204-208 pour les protéines de SEQ N°355, 363 | 2d |
| **SEQ ID N°619** | DWPAMAGAWR | 265-274 pour la protéine de SEQ N°433 | 2d |
| **SEQ ID N°620** | EAFLR | 256-260 pour les protéines de SEQ N°496, 497, 499, 500 | 2d |
| **SEQ ID N°621** | EAIAR | 250-254 pour les protéines de SEQ N°355, 363, 369, 372, 373, 375, 484 | 2d |
| **SEQ ID N°622** | EAIVR | 250-254 pour les protéines de SEQ N°350, 356, 367, 370, 415, 443, 481, 488, 491; 239-243 pour la protéine de séquence SEQ ID N°362 | OXA |
| **SEQ ID N°623** | EAIVTEATPEYIVHSK | 190-205 pour les protéines de SEQ N°489, 505 | OXA |
| **SEQ ID N°624** | EALVTEAAPEYLVHSK | 190-205 pour les protéines de SEQ N°352, 357, 358, 359, 368, 383, 435, 442, 471, 492, 501, 504; 181-196 pour les protéines de séquence SEQ ID N°348, 353, 354 | OXA |
| **SEQ ID N°625** | EALVTEAPEYLVHSK | 191-205 pour la protéine de SEQ N°490 | 2d |
| **SEQ ID N°626** | EEIVR | 240-244 pour la protéine de SEQ N°377 | 2d |
| **SEQ ID N°627** | EEVLAALPAQLK | 251-262 pour la protéine de SEQ N°347 | 2d |
| **SEQ ID N°628** | EFNGSK | 209-214 pour la protéine de SEQ N°447 | 2df |
| **SEQ ID N°629** | EFSAEAVNGVFVLCK | 31-45 pour les protéines de SEQ N°352, 357, 358, 359, 368, 383, 435, 442, 471, 490, 492, 501, 504; 22-36 pour les protéines de séquence SEQ ID N°348, 353, 354 | OXA |
| **SEQ ID N°630** | EFSSESVHGVFVLCK | 31-45 pour les protéines de SEQ N°489, 505 | OXA |
| **SEQ ID N°631** | EGDMAK | 248-253 pour la protéine de SEQ N°379 | 2df |
| **SEQ ID N°632** | EGMSGSIR | 254-261 pour les protéines de SEQ N°364, 371, 414, 449, 506 | 2df |
| **SEQ ID N°633** | EGMTGSIR | 254-261 pour la protéine de SEQ N°365 | 2df |
| **SEQ ID N°634** | EGSCDK | 54-59 pour la protéine de SEQ N°351 | 2de |
| **SEQ ID N°635** | EIAVWNR | 125-131 pour la protéine de SEQ N°381 | 2df |
| **SEQ ID N°636** | EIAYK | 262-266 pour la protéine de SEQ N°480 | 2df |
| **SEQ ID N°637** | EIFER | 20-24 pour la protéine de SEQ N°377 | 2d |
| **SEQ ID N°638** | EIFYHYR | 79-85 pour la protéine de SEQ N°385 | 2d |
| **SEQ ID N°639** | EIGDDK | 131-136 pour les protéines de SEQ N°350, 367, 370, 415, 443, 481, 488; 120-125 pour la protéine de séquence SEQ ID N°362 | OXA |
| **SEQ ID N°640** | EIGDGK | 131-136 pour la protéine de SEQ N°375 | 2d |
| **SEQ ID N°641** | EIGEDK | 131-136 pour les protéines de SEQ N°356, 372, 491 | 2d |
| **SEQ ID N°642** | EIGEDNAR | 131-138 pour la protéine de SEQ N°484 | OXA |
| **SEQ ID N°643** | EIGENK | 131-136 pour les protéines de SEQ N°355, 363 | 2d |
| **SEQ ID N°644** | EIGPK | 153-157 pour la protéine de SEQ N°381 | 2df |
| **SEQ ID N°645** | EIGSEIDK | 136-143 pour la protéine de SEQ N°377 | 2d |
| **SEQ ID N°646** | EITYK | 262-266 pour les protéines de SEQ N°389, 390, 391, 392, 393, 394, 395, 396, 397, 398, 399, 400, 401, 402, 403, 404, 405, 406, 407, 408, 409, 411, 412, 416, 417, 418, 419, 420, 421, 422, 423, 424, 425, 426, 427, 428, 434, 436, 437, 438, 444, 445, 446, 448, 451, 452, 453, 454, 455, 456, 457, 467, 468, 472, 473, 474, 475, 478, 479, 482, 483, 487, 495, 502, 503, 507, 508; 263-267 pour les protéines de séquence SEQ ID N°364, 365, 371, 414, 447, 449, 466, 506; 256-260 pour les protéines de séquence SEQ ID N°431, 432 | 2df |
| **SEQ ID N°647** | EITYR | 262-266 pour la protéine de SEQ N°388 | 2df |
| **SEQ ID N°648** | EMIYLK | 181-186 pour la protéine de SEQ N°385 | 2d |
| **SEQ ID N°649** | EMLYVER | 205-211 pour les protéines de SEQ N°410, 413, 458 | 2df |
| **SEQ ID N°650** | EMTYK | 262-266 pour la protéine de SEQ N°439 | 2df |
| **SEQ ID N°651** | ENIEK | 138-142 pour les protéines de SEQ N°429, 430, 477; 137-141 pour la protéine de séquence SEQ ID N°386 | 2d |
| **SEQ ID N°652** | ENQLIVK | 183-189 pour les protéines de SEQ N°352, 357, 358, 359, 368, 383, 435, 442, 471, 492, 501, 504; 174-180 pour les protéines de séquence SEQ ID N°348, 353, 354; 184-190 pour la protéine de séquence SEQ ID N°490 | OXA |
| **SEQ ID N°653** | EQAILLFR | 156-163 pour la protéine de SEQ N°385 | 2d |
| **SEQ ID N°654** | EQIQFLLR | 165-172 pour la protéine de SEQ N°349 | 2d |
| **SEQ ID N°655** | EQLAFDPQVQQQVK | 182-195 pour la protéine de SEQ N°469 | 2df |
| **SEQ ID N°656** | EQVDFVQR | 189-196 pour la protéine de SEQ N°382 | 2df |
| **SEQ ID N°657** | ETEVYFFAFNMDIDNESK | 229-246 pour les protéines de SEQ N°352, 435, 492, 501; 220-237 pour les protéines de séquence SEQ ID N°348, 353, 354 | OXA |
| **SEQ ID N°658** | ETTTPR | 90-95 pour la protéine de SEQ N°347 | 2d |
| **SEQ ID N°659** | EVGEIR | 126-131 pour la protéine de SEQ N°489 | 2d |
| **SEQ ID N°660** | EVGEVR | 126-131 pour les protéines de SEQ N°352, 357, 358, 359, 368, 383, 435, 442, 471, 492, 501, 504; 117-122 pour les protéines de séquence SEQ ID N°348, 353, 354; 127-132 pour la protéine de séquence SEQ ID N°490 | OXA |
| **SEQ ID N°661** | EVNGSK | 209-214 pour les protéines de SEQ N°364, 365, 371, 414, 449, 466, 506 | 2df |
| **SEQ ID N°662** | EWQENK | 24-29 pour les protéines de SEQ N°378, 450, 476, 485 | 2df |
| **SEQ ID N°663** | EYELYLNVWK | 78-87 pour la protéine de SEQ N°494 | 2de |
| **SEQ ID N°664** | EYLPASTFK | 62-70 pour les protéines de SEQ N°352, 357, 358, 359, 368, 383, 435, 442, 471, 492, 504; 53-61 pour les protéines de séquence SEQ ID N°348, 353, 354; 63-71 pour la protéine de séquence SEQ ID N°490 | OXA |
| **SEQ ID N°665** | EYLPVSTFK | 62-70 pour la protéine de SEQ N°501 | 2de |
| **SEQ ID N°666** | EYNTSGTFVFYDGK | 27-40 pour la protéine de SEQ N°385 | 2d |
| **SEQ ID N°667** | EYVPASTFK | 75-83 pour les protéines de SEQ N°388, 389, 390, 391, 392, 393, 394, 395, 396, 397, 398, 399, 400, 401, 402, 403, 404, 405, 406, 407, 408, 409, 411, 412, 416, 417, 418, 419, 420, 421, 422, 423, 424, 425, 426, 427, 428, 434, 436, 437, 438, 439, 444, 445, 446, 448, 451, | 2df |
| | | 452, 453, 454, 455, 456, 457, 467, 468, 472, 473, 474, 475, 478, 479, 480, 482, 483, 487, 495, 502, 503, 507, 508; 69-77 pour les protéines de séquence SEQ ID N°431, 432, 469; 74-82 pour les protéines de séquence SEQ ID N°366, 374, 387, 440, 441, 459, 460, 461, 462, 463, 464, 465, 486, 493; 76-84 pour les protéines de séquence SEQ ID N°364, 365, 371, 414, 447, 449, 466, 506 | |
| **SEQ ID N°668** | FAAYVAGFGYGNGDISGEPGK | 120-140 pour la protéine de SEQ N°498 | 2d |
| **SEQ ID N°669** | FAPESTFK | 45-52 pour la protéine de SEQ N°494 | 2de |
| **SEQ ID N°670** | FAQYAK | 121-126 pour les protéines de SEQ N°496, 497, 499, 500 | 2d |
| **SEQ ID N°671** | FDYGNK | 138-143 pour la protéine de SEQ N°351 | 2de |
| **SEQ ID N°672** | FDYGNR | 146-151 pour la protéine de SEQ N°379; 125-130 pour la protéine de séquence SEQ ID N°347 | 2d |
| **SEQ ID N°673** | FEDLYK | 232-237 pour la protéine de SEQ N°385 | 2d |
| **SEQ ID N°674** | FEDTFHISNQK | 27-37 pour la protéine de SEQ N°466 | 2df |
| **SEQ ID N°675** | FEDTFHTSNQQHEK | 27-40 pour la protéine de SEQ N°447 | 2df |
| **SEQ ID N°676** | FEYGNQDVSGDSGK | 133-146 pour la protéine de SEQ N°349 | 2d |
| **SEQ ID N°677** | FFSDFQAK | 34-41 pour la protéine de SEQ N°375 | 2d |
| **SEQ ID N°678** | FFSDLQAEGAIVIADER | 34-50 pour les protéines de SEQ N°373, 484 | 2d |
| **SEQ ID N°679** | FFSDLR | 34-39 pour les protéines de SEQ N°369, 372 | OXA |
| **SEQ ID N°680** | FFSEFQAK | 34-41 pour les protéines de SEQ N°350, 356, 367, 370, 415, 443, 481, 488, 491; 23-30 pour la protéine de séquence SEQ ID N°362 | OXA |
| **SEQ ID N°681** | FGLEGQLR | 153-160 pour la protéine de SEQ N°359 | 2de |
| **SEQ ID N°682** | FILPIFSISILVSLSACSSIK | 4-24 pour les protéines de SEQ N°364, 365, 371, 414, 449, 506 | 2df |
| **SEQ ID N°683** | FLALLFSAVVLVSLGHAQDK | 5-24 pour la protéine de SEQ N°363 | 2d |
| **SEQ ID N°684** | FLALLFSAVVLVSLGHAQEK | 5-24 pour la protéine de SEQ N°355 | 2d |
| **SEQ ID N°685** | FLESLYLNNLPASK | 169-182 pour les protéines de SEQ N°489, 505 | OXA |
| **SEQ ID N°686** | FLLEGQLR | 153-160 pour la protéine de SEQ N°442 | 2de |
| **SEQ ID N°687** | FQQYVDR | 118-124 pour la protéine de SEQ N°347 | 2d |
| **SEQ ID N°688** | FSDYVQR | 131-137 pour la protéine de SEQ N°351 | 2de |
| **SEQ ID N°689** | FSTASTFK | 63-70 pour la protéine de SEQ N°470 | 2df |
| **SEQ ID N°690** | FSWDGK | 117-122 pour la protéine de SEQ N°3 81 | 2df |
| **SEQ ID N°691** | FSYGNQNISGGIDK | 139-152 pour les protéines de SEQ N°352, 357, 358, 359, 368, 383, 435, 442, 501, 504; 130-143 pour les protéines de séquence SEQ ID N°348, 353, 354; 140-153 pour la protéine de séquence SEQ ID N°490 | OXA |
| **SEQ ID N°692** | FSYGNQNISGGTDK | 139-152 pour la protéine de SEQ N°471 | 2de |
| **SEQ ID N°693** | FSYGSQNISGGIDK | 139-152 pour la protéine de SEQ N°492 | 2de |
| **SEQ ID N°694** | FTEYVK | 126-131 pour la protéine de SEQ N°349 | 2d |
| **SEQ ID N°695** | FVAHK | 173-177 pour la protéine de SEQ N°349 | 2d |
| **SEQ ID N°696** | FVPASTYK | 62-69 pour la protéine de SEQ N°380 | 2d |
| **SEQ ID N°697** | FVYDLAQGQLPFK | 184-196 pour les protéines de SEQ N°410, 413, 458 | 2df |
| **SEQ ID N°698** | FVYDLAQGQLPFKPEVQQQVK | 184-204 pour les protéines de SEQ N°410, 413, 458 | 2df |
| **SEQ ID N°699** | FWLEDQLR | 153-160 pour les protéines de SEQ N°358, 435, 471, 492; 144-151 pour les protéines de séquence SEQ ID N°348, 353 | 2de |
| **SEQ ID N°700** | FWLEGPLK | 144-151 pour la protéine de SEQ N°377 | 2d |
| **SEQ ID N°701** | FWLEGQLR | 153-160 pour les protéines de SEQ N°352, 357, 368, 383, 489, 501, 504, 505; 144-151 pour la protéine de séquence SEQ ID N°354; 154-161 pour la protéine de séquence SEQ **ID** N°490 | OXA |
| **SEQ ID N°702** | FYPASSFK | 53-60 pour la protéine de SEQ N°377 | 2d |
| **SEQ ID N°703** | FYPASTFK | 66-73 pour les protéines de SEQ N°386, 429, 430, 477 | 2d |
| **SEQ ID N°704** | GACDK | 44-48 pour la protéine de SEQ N°498 | 2d |
| **SEQ ID N°705** | GAEVYFF AFNMDIDNENK | 229-246 pour les protéines de SEQ N°383, 490 | 2d |
| **SEQ ID N°706** | GAIQVSAVPVFQQIAR | 110-125 pour les protéines de SEQ N°352, 357, 358, 359, 368, 383, 435, 442, 471, 489, 492, 501, 504, 505; 101-116 pour les protéines de séquence SEQ ID N°348, 354; 111-126 pour la protéine de séquence SEQ ID N°490 | OXA |
| **SEQ ID N°707** | GAIQVSAVPVFQQITR | 101-116 pour la protéine de SEQ N°353 | 2de |
| **SEQ ID N°708** | GDYWIDGNLEISAHEQISFLR | 156-176 pour les protéines de SEQ N°369, 372 | OXA |
| **SEQ ID N°709** | GDYWIDGNLK | 156-165 pour la protéine de SEQ N°373 | 2d |
| **SEQ ID N°710** | GELPVSEDALEMTK | 181-194 pour la protéine de SEQ N°351 | 2de |
| **SEQ ID N°711** | GEQPAGPAAR | 241-250 pour la protéine de SEQ N°498; 252-261 pour la protéine de séquence SEQ ID N°351 | OXA |
| **SEQ ID N°712** | GFAGHNQDQDLR | 103-114 pour les protéines de SEQ N°350, 367, 370, 415, 443, 481, 488; 92-103 pour la protéine de séquence SEQ ID N°362 | OXA |
| **SEQ ID N°713** | GIPSSVR | 254-260 pour les protéines de SEQ N°389, 390, 391, 392, 393, 394, 395, 397, 398, 399, 400, 401, 402, 403, 404, 405, 406, 407, 408, 409, 411, 412, 416, 417, 418, 419, 420, 421, 422, 423, 424, 425, 426, 427, 428, 434, 436, 437, 438, 439, 444, 445, 446, 448, 451, 452, 453, 454, 455, 456, 457, 467, 468, 472, 473, 474, 475, 478, 479, 480, 482, 483, 487, 495, 502, 503, 507, 508; 248-254 pour les protéines de séquence SEQ ID N°431, 432 | 2df |
| **SEQ ID N°714** | GISSSVR | 254-260 pour la protéine de SEQ N°388 | 2df |
| **SEQ ID N°715** | GLNGTFVVYDLK | 26-37 pour la protéine de SEQ N°377 | 2d |
| **SEQ ID N°716** | GMEIWNSNHTPK | 101-112 pour les protéines de SEQ N°360, 376; 86-97 pour la protéine de séquence SEQ ID N°361 | 2d |
| **SEQ ID N°717** | GNQTLVFAR | 230-238 pour la protéine de SEQ N°496 | 2d |
| **SEQ ID N°718** | GNYWIDGNLEISAHEQISFLR | 156-176 pour la protéine de SEQ N°484 | OXA |
| **SEQ ID N°719** | GPLEISAFEEAR | 164-175 pour la protéine de SEQ N°379 | 2df |
| **SEQ ID N°720** | GPLTITPIQEVK | 172-183 pour les protéines de SEQ N°410, 413, 458 | 2df |
| **SEQ ID N°721** | GSGWFVGWIVR | 219-229 pour les protéines de SEQ N°496, 497, 499, 500 | 2d |
| **SEQ ID N°722** | GSLLLWDQK | 66-74 pour la protéine de SEQ N°381 | 2df |
| **SEQ ID N°723** | GTEVYFFAFNMDIDNENK | 229-246 pour les protéines de SEQ N°357, 358, 359, 368, 442, 471, 504 | OXA |
| **SEQ ID N°724** | GTEVYFFAFNMDIDNESK | 229-246 pour les protéines de SEQ N°489, 505 | OXA |
| **SEQ ID N°725** | GTFVLYDVQR | 38-47 pour la protéine de SEQ N°380 | 2d |
| **SEQ ID N°726** | GTIVVADER | 42-50 pour les protéines de SEQ N°350, 356, 367, 370, 375, 415, 443, 481, 488, 491; 31-39 pour la protéine de séquence SEQ ID N°362 | OXA |
| **SEQ ID N°727** | GTIVVLDAR | 63-71 pour la protéine de SEQ N°382 | 2df |
| **SEQ ID N°728** | GTIVVVDER | 42-50 pour les protéines de SEQ N°355, 363 | 2d |
| **SEQ ID N°729** | GTLPFSAR | 200-207 pour la protéine de SEQ N°382 | 2df |
| **SEQ ID N°730** | GTPSSVR | 254-260 pour la protéine de SEQ N°396 | 2df |
| **SEQ ID N°731** | HALSSAFVLLGCIAASAHAK | 5-24 pour les protéines de SEQ N°496, 497, 499, 500 | 2d |
| **SEQ ID N°732** | HIADSK | 234-239 pour la protéine de SEQ N°494 | 2de |
| **SEQ ID N°733** | HNGLTHAWLASSLK | 152-165 pour la protéine de SEQ N°351 | 2de |
| **SEQ ID N°734** | HNGLTQSWLMSSLTISPK | 147-164 pour la protéine de SEQ N°349 | 2d |
| **SEQ ID N°735** | HNGTDGAWIISSLR | 148-161 pour la protéine de SEQ N°433 | 2d |
| **SEQ ID N°736** | HTLSVFDQER | 54-63 pour la protéine de SEQ N°373 | 2d |
| **SEQ ID N°737** | HVTFASFR | 241-248 pour la protéine de SEQ N°494 | 2de |
| **SEQ ID N°738** | IAISLMGYDAGFLR | 57-70 pour les protéines de SEQ N°496, 497, 499, 500 | 2d |
| **SEQ ID N°739** | IALSLMAFDAEIIDQK | 75-90 pour les protéines de SEQ N°360, 376; 60-75 pour la protéine de séquence SEQ ID N°361 | 2d |
| **SEQ ID N°740** | IALSLMGFDSGILK | 53-66 pour la protéine de SEQ N°494 | 2de |
| **SEQ ID N°741** | IANALIGLENHK | 87-98 pour les protéines de SEQ N°410, 413, 458 | 2df |
| **SEQ ID N°742** | IAWIVGFVYLK | 205-215 pour la protéine de SEQ N°385 | 2d |
| **SEQ ID N°743** | IDTFWLDNSLK | 141-151 pour la protéine de SEQ N°385 | 2d |
| **SEQ ID N°744** | IDYYNLDR | 41-48 pour la protéine de SEQ N°377 | 2d |
| **SEQ ID N°745** | IFNALIALDSGVIK | 62-75 pour la protéine de SEQ N°385 | 2d |
| **SEQ ID N°746** | IFNSLLALDSGALDNER | 95-111 pour la protéine de SEQ N°382 | 2df |
| **SEQ ID N°747** | IFNTLIGLENGIVK | 61-74 pour la protéine de SEQ N°377 | 2d |
| **SEQ ID N°748** | IGLDLMQK | 138-145 pour les protéines de SEQ N°366, 374, 387, 440, 441, 459, 460, 461, 462, 463, 464, 465, 486, 493 | 2df |
| **SEQ ID N°749** | IGLEK | 131-135 pour la protéine de SEQ N°477 | OXA |
| **SEQ ID N°750** | IGLELMQQEVQR | 133-144 pour la protéine de SEQ N°469 | 2df |
| **SEQ ID N°751** | IGLELMSK | 139-146 pour les protéines de SEQ N°389, 390, 391, 393, 395, 398, 399, 400, 401, 402, 403, 404, 408, 409, 411, 412, 417, 419, 420, 421, 422, 423, 424, 425, 426, 427, 428, 436, 437, 438, 439, 451, 452, 453, 454, 455, 456, 457, 467, 468, 472, 473, 474, 478, 479, 480, 482,483, 487,495, 502, 508 | 2df |
| **SEQ ID N°752** | IGLELMSNEVK | 139-149 pour les protéines de SEQ N°388, 392, 394, 396, 397, 405, 406, 407, 416, 418, 434, 444, 445, 446, 448, 475, 503, 507; 133-143 pour les protéines de séquence SEQ ID N°431, 432 | 2df |
| **SEQ ID N°753** | IGLER | 126-130 pour la protéine de SEQ N°380 | 2d |
| **SEQ ID N°754** | IGLNK | 130-134 pour les protéines de SEQ N°360, 376; 115-119 pour la protéine de séquence SEQ ID N°361 | 2d |
| **SEQ ID N°755** | IGLNLMQK | 140-147 pour la protéine de SEQ N°466 | 2df |
| **SEQ ID N°756** | IGPSLMQSELQR | 142-153 pour les protéines de SEQ N°410, 413, 458 | 2df |
| **SEQ ID N°757** | IGYGNMQIGTEVDQFWLK | 154-171 pour les protéines de SEQ N°413, 458 | 2df |
| **SEQ ID N°758** | IGYGNMQMGTEVDQFWLK | 154-171 pour la protéine de SEQ N°410 | 2df |
| **SEQ ID N°759** | IINHNLPVK | 167-175 pour la protéine de SEQ N°361; 182-190 pour la protéine de séquence SEQ ID N°360 | 2d |
| **SEQ ID N°760** | IINHNLPVR | 182-190 pour la protéine de SEQ N°376 | 2d |
| **SEQ ID N°761** | ILFQQGTQQACAER | 41-54 pour la protéine de SEQ N°433 | 2d |
| **SEQ ID N°762** | ILNNWFK | 20-26 pour la protéine de SEQ N°385 | 2d |
| **SEQ ID N°763** | ILNTLISLEEK | 71-81 pour la protéine de SEQ N°470 | 2df |
| **SEQ ID N°764** | ILSLVCLSISIGACAEHSMSR | 6-26 pour les protéines de SEQ N°410, 413, 458 | 2df |
| **SEQ ID N°765** | INESR | 219-223 pour la protéine de SEQ N°349 | 2d |
| **SEQ ID N°766** | INIVK | 255-259 pour les protéines de SEQ N°429, 430, 477; 254-258 pour la protéine de séquence SEQ ID N°386 | 2d |
| **SEQ ID N°767** | INLYGNALSR | 61-70 pour les protéines de SEQ N°366, 374, 387, 440, 441, 459, 460, 461, 462, 463, 464, 465, 486, 493 | 2df |
| **SEQ ID N°768** | IPFSLNLEMK | 244-253 pour les protéines de SEQ N°364, 365, 371, 414, 449, 506 | 2df |
| **SEQ ID N°769** | IPHTLF ALDADAVR | 76-89 pour la protéine de SEQ N°373 | 2d |
| **SEQ ID N°770** | IPHTLF ALDAGAAR | 76-89 pour les protéines de SEQ N°356, 491 | 2d |
| **SEQ ID N°771** | IPHTLF ALDAGAVR | 76-89 pour les protéines de SEQ N°350, 355, 363, 367, 370, 375, 415, 443, 481, 488; 65-78 pour la protéine de séquence SEQ ID N°362 | OXA |
| **SEQ ID N°772** | IPLGK | 255-259 pour la protéine de SEQ N°379 | 2df |
| **SEQ ID N°773** | IPNAIIGLETGVIK | 71-84 pour les protéines de SEQ N°352, 358, 359, 368, 383, 442, 471, 492, 504; 62-75 pour les protéines de séquence SEQ ID N°348, 353; 72-85 pour la protéine de séquence SEQ ID N°490 | OXA |
| **SEQ ID N°774** | IPNALIGLETGAIK | 71-84 pour les protéines de SEQ N°489, 505 | OXA |
| **SEQ ID N°775** | IPNSLIAFDTGAVR | 78-91 pour la protéine de SEQ N°379 | 2df |
| **SEQ ID N°776** | IPSAIIGLETGVIK | 71-84 pour les protéines de SEQ N°357, 435, 501; 62-75 pour la protéine de séquence SEQ ID N°354 | 2de |
| **SEQ ID N°777** | ISAFNQVK | 161-168 pour la protéine de SEQ N°489 | 2d |
| **SEQ ID N°778** | ISAHEQILFLR | 166-176 pour la protéine de SEQ N°373 | 2d |
| **SEQ ID N°779** | ISAMEQTR | 160-167 pour la protéine de SEQ N°380 | 2d |
| **SEQ ID N°780** | ISAMEQVK | 165-172 pour les protéines de SEQ N°429, 477; 152-159 pour la protéine de séquence SEQ ID N°377; 164-171 pour la protéine de séquence SEQ ID N°386 | 2d |
| **SEQ ID N°781** | ISATEQVAFLR | 164-174 pour la protéine de SEQ N°384 | 2df |
| **SEQ ID N°782** | ISATQQIAFLR | 164-174 pour la protéine de SEQ N°485 | 2df |
| **SEQ ID N°783** | ISAVNQVEFLESLFLNK | 161-177 pour les protéines de SEQ N°357, 358, 359, 368, 383, 442, 471, 504; 162-178 pour la protéine de séquence SEQ ID N°490 | OXA |
| **SEQ ID N°784** | ISAVNQVEFLESL YLNK | 161-177 pour les protéines de SEQ N°352, 435, 492, 501; 152-168 pour les protéines de séquence SEQ ID N°348, 353, 354 | OXA |
| **SEQ ID N°785** | ISAVNQVK | 161-168 pour la protéine de SEQ N°505 | 2de |
| **SEQ ID N°786** | ISPEEQIQFLR | 170-180 pour les protéines de SEQ N°360, 376; 155-165 pour la protéine de séquence SEQ ID N°361 | 2d |
| **SEQ ID N°787** | ISPEEQVR | 166-173 pour la protéine de SEQ N°351 | 2de |
| **SEQ ID N°788** | ISPEGQVR | 155-162 pour la protéine de SEQ N°498 | 2d |
| **SEQ ID N°789** | ISPLEQLAFLR | 162-172 pour la protéine de SEQ N°433 | 2d |
| **SEQ ID N°790** | ITAFQQVDFLR | 188-198 pour la protéine de SEQ N°381 | 2df |
| **SEQ ID N°791** | ITLFLLFLNLVFGQDK | 4-19 pour la protéine de SEQ N°385 | 2d |
| **SEQ ID N°792** | ITPIQEVNFADDFANNR | 174-190 pour la protéine de SEQ N°447 | 2df |
| **SEQ ID N°793** | ITPIQEVNFADDLANNR | 174-190 pour la protéine de SEQ N°466 | 2df |
| **SEQ ID N°794** | ITPQQEAQFAYK | 173-184 pour les protéines de SEQ N°388, 389, 390, 391, 392, 393, 394, 395, 396, 397, 398, 399, 400, 401, 402, 403, 404, 406, 407, 408, 409, 411, 412, 416, 417, 418, 419, 420, 421, 422, 423, 424, 425, 426, 427, 428, 434, 436, 437, 438, 439, 444, 445, 446, 448, 451, 452, 453, 454, 455, 456, 457, 467, 468, 472, 473, 474, 475, 478, 479, 480, 482, 483, 487, 495, 502, 503, 507, 508; 167-178 pour les protéines de séquence SEQ ID N°431, 432 | 2df |
| **SEQ ID N°795** | ITPQQEAQFTYK | 173-184 pour la protéine de SEQ N°405 | 2df |
| **SEQ ID N°796** | ITPVQEVNFADDLAHNR | 174-190 pour les protéines de SEQ N°364, 365, 371, 414, 449, 506 | 2df |
| **SEQ ID N°797** | IVAFALK | 241-247 pour les protéines de SEQ N°366, 387 | 2df |
| **SEQ ID N°798** | IVAFALNMEMR | 241-251 pour les protéines de SEQ N°440, 459, 460, 461, 462, 463, 464, 465, 486, 493; 242-252 pour les protéines de séquence SEQ ID N°374, 441 | 2df |
| **SEQ ID N°799** | IVESTTLADGTVVHGK | 186-201 pour les protéines de SEQ N°496, 497, 499, 500 | 2d |
| **SEQ ID N°800** | IYNSLIGLNEK | 74-84 pour les protéines de SEQ N°386, 429, 430, 477 | 2d |
| **SEQ ID N°801** | KPDIGWWVGWIER | 23 7-249 pour la protéine de SEQ N°382 | 2df |
| **SEQ ID N°802** | LACATNNLAR | 50-59 pour la protéine de SEQ N°490 | 2d |
| **SEQ ID N°803** | LAQGELPFPAPVQSTVR | 172-188 pour la protéine de SEQ N°380 | 2d |
| **SEQ ID N°804** | LAQNELPYPIEIQK | 177-190 pour les protéines de SEQ N°429, 430, 477 | 2d |
| **SEQ ID N°805** | LAQNELQYPIEIQK | 176-189 pour la protéine de SEQ N°3 86 | 2d |
| **SEQ ID N°806** | LDFGNK | 143-148 pour les protéines de SEQ N°429, 430, 477; 142-147 pour la protéine de séquence SEQ ID N°386 | 2d |
| **SEQ ID N°807** | LDGSLNR | 206-212 pour la protéine de SEQ N°347 | 2d |
| **SEQ ID N°808** | LEIGK | 244-248 pour la protéine de SEQ N°349 | 2d |
| **SEQ ID N°809** | LEILQQALAELGL YPK | 255-270 pour la protéine de SEQ N°469 | 2df |
| **SEQ ID N°810** | LENQEQVK | 173-180 pour la protéine de SEQ N°377 | 2d |
| **SEQ ID N°811** | LETQEEVEK | 195-203 pour la protéine de SEQ N°364 | 2df |
| **SEQ ID N°812** | LETQEEVK | 195-202 pour les protéines de SEQ N°365, 371, 414, 447, 449, 466, 506 | 2df |
| **SEQ ID N°813** | LFAAEGVK | 55-62 pour la protéine de SEQ N°382 | 2df |
| **SEQ ID N°814** | LFESAGVK | 58-65 pour la protéine de SEQ N°381 | 2df |
| **SEQ ID N°815** | LFGAAGVK | 30-37 pour la protéine de SEQ N°380 | 2d |
| **SEQ ID N°816** | LFPEWEK | 110-116 pour les protéines de SEQ N°388, 389, 390, 391, 392, 393, 394, 395, 396, 397, 398, 399, 400, 401, 402, 403, 404, 405, 406, 407, 408, 409, 411, 412, 416, 417, 418, 419, 420, 421, 422, 423, 424, 425, 426, 427, 428, 434, 436, 437, 438, 439, 444, 445, 446, 448, 451, 452, 453, 454, 455, 456, 457, 467, 468, 472, 473, 474, 475, 478, 479, 480, 482, 483, 487, 495, 502, 503, 507, 508; 104-110 pour les protéines de séquence SEQ ID N°431, 432 | 2df |
| **SEQ ID N°817** | LGESR | 126-130 pour la protéine de SEQ N°351 | 2de |
| **SEQ ID N°818** | LGVDR | 121-125 pour la protéine de SEQ N°349 | 2d |
| **SEQ ID N°819** | LHVSER | 181-186 pour les protéines de SEQ N°378, 384, 450, 476, 485 | 2df |
| **SEQ ID N°820** | LHYGNAK | 131-137 pour la protéine de SEQ N°385 | 2d |
| **SEQ ID N°821** | LLNLLSQSK | 160-168 pour la protéine de SEQ N°377 | 2d |
| **SEQ ID N°822** | LLQDER | 243-248 pour la protéine de SEQ N°433 | 2d |
| **SEQ ID N°823** | LLVQDGDCGR | 38-47 pour les protéines de SEQ N°496, 497, 499, 500 | 2d |
| **SEQ ID N°824** | LNEVGYGNR | 160-168 pour la protéine de SEQ N°382 | 2df |
| **SEQ ID N°825** | LNYGNADPSTK | 144-154 pour les protéines de SEQ N°355, 363 | 2d |
| **SEQ ID N°826** | LNYGNK | 130-135 pour la protéine de SEQ N°377 | 2d |
| **SEQ ID N°827** | LPASK | 178-182 pour les protéines de SEQ N°489, 505; 172-176 pour la protéine de séquence SEQ ID N°498 | 2d |
| **SEQ ID N°828** | LPHTLFALDADAVR | 76-89 pour les protéines de SEQ N°369, 372 | OXA |
| **SEQ ID N°829** | LPHTLFALDAGAVR | 76-89 pour la protéine de SEQ N°484 | OXA |
| **SEQ ID N°830** | LPLAIMGFDSGILQSPK | 62-78 pour la protéine de SEQ N°349 | 2d |
| **SEQ ID N°831** | LPLAIMGYDADILLDATTPR | 69-88 pour la protéine de SEQ N°351 | 2de |
| **SEQ ID N°832** | LPSSLIALETGAVR | 98-111 pour la protéine de SEQ N°381 | 2df |
| **SEQ ID N°833** | LPVSAQTLQYTANILK | 170-185 pour la protéine de SEQ N°347 | 2d |
| **SEQ ID N°834** | LPVSER | 205-210 pour la protéine de SEQ N°381 | 2df |
| **SEQ ID N°835** | LPVSPTAVDMTER | 173-185 pour les protéines de SEQ N°496, 497, 499, 500 | 2d |
| **SEQ ID N°836** | LSASK | 178-182 pour les protéines de SEQ N°352, 357, 358, 359, 368, 383, 435, 442, 471, 492, 501, 504; 169-173 pour les protéines de séquence SEQ ID N°348, 353, 354; 179-183 pour la protéine de séquence SEQ ID N°490 | OXA |
| **SEQ ID N°837** | LSAVPIYQEVAR | 121-132 pour la protéine de SEQ N°379 | 2df |
| **SEQ ID N°838** | LS AVPVYQELAR | 127-138 pour les protéines de SEQ N°364, 365, 371, 414, 447, 449, 506; 125-136 pour les protéines de séquence SEQ ID N°366, 374, 387, 440, 441, 459, 460, 461, 462, 463, 464, 465, 486, 493 | 2df |
| **SEQ ID N°839** | LSCTLVIDEASGDLLHR | 37-53 pour la protéine de SEQ N°351 | 2de |
| **SEQ ID N°840** | LSLQHGWFIGWIEK | 211-224 pour la protéine de SEQ N°494 | 2de |
| **SEQ ID N°841** | LSQNSLPFSQEAMNSVK | 164-180 pour la protéine de SEQ N°3 85 | 2d |
| **SEQ ID N°842** | LSVNPK | 168-173 pour la protéine de SEQ N°494 | 2de |
| **SEQ ID N°843** | LTQDER | 239-244 pour les protéines de SEQ N°496, 497, 499, 500 | 2d |
| **SEQ ID N°844** | LTVGAR | 245-250 pour la protéine de SEQ N°351 | 2de |
| **SEQ ID N°845** | LYGFALNIDMPGGEADIGK | 228-246 pour la protéine de SEQ N°380 | 2d |
| **SEQ ID N°846** | LYHNELPFR | 178-186 pour les protéines de SEQ N°356, 491 | 2d |
| **SEQ ID N°847** | LYHNK | 176-180 pour les protéines de SEQ N°378, 384, 450, 476, 485 | 2df |
| **SEQ ID N°848** | LYQNDLPFR | 178-186 pour la protéine de SEQ N°375 | 2d |
| **SEQ ID N°849** | MDDLFK | 243-248 pour les protéines de SEQ N°350, 356, 367, 370, 375, 415, 443, 481, 488, 491; 232-237 pour la protéine de séquence SEQ ID N°362 | OXA |
| **SEQ ID N°850** | MEDLHK | 243-248 pour les protéines de SEQ N°355, 363 | 2d |
| **SEQ ID N°851** | MLIALIGLENHK | 85-96 pour la protéine de SEQ N°506 | 2df |
| **SEQ ID N°852** | MLLIEQQGDAALYAK | 198-212 pour la protéine de SEQ N°379 | 2df |
| **SEQ ID N°853** | MLLIK | 204-208 pour les protéines de SEQ N°364, 365, 371, 414, 447, 449, 466, 506 | 2df |
| **SEQ ID N°854** | MLNALIGLEHHK | 84-95 pour les protéines de SEQ N°388, 389, 390, 391, 392, 393, 394, 395, 396, 397, 398, 399, 400, 401, 402, 403, 404, 405, 406, 408, 409, 411, 412, 416, 417, 418, 419, 420, 421, 422, 423, 424, 425, 426, 427, 428, 434, 436, 437, 438, 439, 444, 445, 446, 448, 451, 452, 453, 454, 455, 456, 457, 467, 468, 472, 473, 474, 475, 478, 479, 480, 482, 487, 495, 502, 503, 507, 508; 78-89 pour les protéines de séquence SEQ ID N°431,432 | 2df |
| **SEQ ID N°855** | MLNALIGLENHK | 85-96 pour les protéines de SEQ N°364, 365, 371, 414, 447, 449, 466 | 2df |
| **SEQ ID N°856** | MLNALIGLENQK | 83-94 pour les protéines de SEQ N°366, 374, 387, 440, 441, 459, 460, 461, 462, 463, 464, 465, 486, 493 | 2df |
| **SEQ ID N°857** | MLNALIGLEYHK | 84-95 pour la protéine de SEQ N°483 | 2df |
| **SEQ ID N°858** | MLNALIGLQHGK | 78-89 pour la protéine de SEQ N°469 | 2df |
| **SEQ ID N°859** | MLNALISLEHHK | 84-95 pour la protéine de SEQ N°407 | 2df |
| **SEQ ID N°860** | MQAYVDAFDYGNR | 139-151 pour la protéine de SEQ N°379 | 2df |
| **SEQ ID N°861** | MQEGLNK | 123-129 pour la protéine de SEQ N°377 | 2d |
| **SEQ ID N°862** | MSPASTYK | 87-94 pour la protéine de SEQ N°382 | 2df |
| **SEQ ID N°863** | MTAGGK | 234-239 pour la protéine de SEQ N°498 | 2d |
| **SEQ ID N°864** | MVSGK | 165-169 pour la protéine de SEQ N°347 | 2d |
| **SEQ ID N°865** | NEHDPVLPYR | 71-80 pour les protéines de SEQ N°496, 497, 499, 500 | 2d |
| **SEQ ID N°866** | NEHQIFK | 86-92 pour la protéine de SEQ N°490; 85-91 pour la protéine de séquence SEQ ID N°383 | 2d |
| **SEQ ID N°867** | NEHQVFK | 85-91 pour les protéines de SEQ N°352, 357, 358, 359, 368, 435, 442, 471, 492, 501, 504; 76-82 pour les protéines de séquence SEQ ID N°348, 353, 354 | OXA |
| **SEQ ID N°868** | NEITYK | 262-267 pour les protéines de SEQ N°364, 365, 371, 414, 447, 449, 466, 506 | 2df |
| **SEQ ID N°869** | NELLMK | 260-265 pour les protéines de SEQ N°366, 387, 440, 459, 460, 462, 463, 464, 465, 486, 493; 261-266 pour les protéines de séquence SEQ ID N°374, 441 | 2df |
| **SEQ ID N°870** | NELMMK | 260-265 pour la protéine de SEQ N°461 | 2df |
| **SEQ ID N°871** | NELPFR | 181-186 pour les protéines de SEQ N°350, 355, 356, 363, 367, 370, 415, 443, 481, 488, 491; 170-175 pour la protéine de séquence SEQ ID N°362 | OXA |
| **SEQ ID N°872** | NFILIFIFVILISCK | 5-19 pour les protéines de SEQ N°386, 429, 477 | 2d |
| **SEQ ID N°873** | NFILIFIFVILTSCK | 5-19 pour la protéine de SEQ N°430 | 2d |
| **SEQ ID N°874** | NISSYGNNL VR | 62-72 pour la protéine de SEQ N°466 | 2df |
| **SEQ ID N°875** | NISTYGNNLTR | 62-72 pour la protéine de SEQ N°447 | 2df |
| **SEQ ID N°876** | NLFNEVHTTGVLVIR | 43-57 pour la protéine de SEQ N°412 | 2df |
| **SEQ ID N°877** | NLSTYGNALAR | 62-72 pour les protéines de SEQ N°364, 365, 371, 414, 449, 506 | 2df |
| **SEQ ID N°878** | NMENLELFGK | 187-196 pour la protéine de SEQ N°385 | 2d |
| **SEQ ID N°879** | NMLLLEENNGYK | 201-212 pour la protéine de SEQ N°440 | 2df |
| **SEQ ID N°880** | NMLLLEESNGYK | 201-212 pour les protéines de SEQ N°366, 374, 387, 441, 459, 460, 461, 462, 463, 465, 486, 493 | 2df |
| **SEQ ID N°881** | NMLLLEK | 201-207 pour la protéine de SEQ N°464 | 2df |
| **SEQ ID N°882** | NMTLGDAMK | 117-125 pour les protéines de SEQ N°388, 392, 393, 396, 397, 402, 405, 406, 416, 419, 422, 424, 434, 445, 446, 448, 475, 503; 111-119 pour les protéines de séquence SEQ ID N°431, 432 | 2df |
| **SEQ ID N°883** | NNGLTEAWLES SLK | 156-169 pour les protéines de SEQ N°360, 376; 141-154 pour la protéine de séquence SEQ ID N°361 | 2d |
| **SEQ ID N°884** | NQLPFK | 181-186 pour la protéine de SEQ N°373 | 2d |
| **SEQ ID N°885** | NQLPFQVEHQR | 181-191 pour les protéines de SEQ N°369, 372, 484 | OXA |
| **SEQ ID N°886** | NSAIENTIDNMYLQDLENSTK | 191-211 pour la protéine de SEQ N°376 | 2d |
| **SEQ ID N°887** | NSAIENTIENMYLQDLDNSTK | 191-211 pour la protéine de SEQ N°360 | 2d |
| **SEQ ID N°888** | NSAIENTIENMYLQDLENSTK | 176-196 pour la protéine de SEQ N°361 | 2d |
| **SEQ ID N°889** | NSAVWVYELFAK | 119-130 pour les protéines de SEQ N°369, 372, 484 | OXA |
| **SEQ ID N°890** | NSQVPAYK | 118-125 pour les protéines de SEQ N°429, 430, 477; 117-124 pour la protéine de séquence SEQ ID N°386 | 2d |
| **SEQ ID N°891** | NSTVWIYELFAK | 119-130 pour les protéines de SEQ N°356, 491 | 2d |
| **SEQ ID N°892** | NSTVWVYELFAK | 119-130 pour les protéines de SEQ N°350, 367, 370, 373, 375, 415, 443, 481, 488; 108-119 pour la protéine de séquence SEQ ID N°362 | OXA |
| **SEQ ID N°893** | NSTVWVYQLFAK | 119-130 pour les protéines de SEQ N°355, 363 | 2d |
| **SEQ ID N°894** | NTSGALVIQTDK | 48-59 pour la protéine de SEQ N°460 | 2df |
| **SEQ ID N°895** | NTSGVLVIQTDK | 48-59 pour les protéines de SEQ N°366, 374, 387, 440, 441, 459, 461, 462, 463, 464, 465, 486, 493 | 2df |
| **SEQ ID N°896** | NVDEMFYYYDGSK | 75-87 pour la protéine de SEQ N°377 | 2d |
| **SEQ ID N°897** | NWILR | 204-208 pour les protéines de SEQ N°350, 356, 367, 369, 370, 372, 375,415, 443, 481, 484, 488, 491; 193-197 pour la protéine de séquence SEQ ID N°362 | OXA |
| **SEQ ID N°898** | NWNAAMDLR | 125-133 pour la protéine de SEQ N°382 | 2df |
| **SEQ ID N°899** | NYVDAFHYGNQDISGDK | 118-134 pour la protéine de SEQ N°494 | 2de |
| **SEQ ID N°900** | QADHAILVFDQAR | 51-63 pour la protéine de SEQ N°375 | 2d |
| **SEQ ID N°901** | QAEHALLVFGQER | 51-63 pour les protéines de SEQ N°369, 372, 484 | OXA |
| **SEQ ID N°902** | QAITK | 251-255 pour les protéines de SEQ N°378, 384, 450, 485; 247-251 pour la protéine de séquence SEQ ID N°476 | 2df |
| **SEQ ID N°903** | QAMLTEANSDYIIR | 193-206 pour la protéine de SEQ N°384 | 2df |
| **SEQ ID N°904** | QEVQFVSALAR | 171-181 pour la protéine de SEQ N°469 | 2df |
| **SEQ ID N°905** | QFASIK | 243-248 pour la protéine de SEQ N°347 | 2d |
| **SEQ ID N°906** | QGMPGSIR | 254-261 pour la protéine de SEQ N°447 | 2df |
| **SEQ ID N°907** | QGMSGSIR | 254-261 pour la protéine de SEQ N°466 | 2df |
| **SEQ ID N°908** | QGQTQQSYGNDLAR | 58-71 pour les protéines de SEQ N°388, 389, 390, 391, 392, 393, 394, 395, 396, 397, 398, 399, 400, 401, 402, 403, 404, 405, 406, 407, 408, 409, 411, 412, 416, 417, 418, 419, 420, 421, 422, 423, 424, 425, 426, 427, 428, 436, 437, 438, 439, 444, 445, 446, 448, 451, 452, 453, 454, 455, 456, 457, 467, 468, 472, 473, 474, 475, 478, 479, 480, 482, 483, 487, 495, 502, 503, 507, 508; 52-65 pour les protéines de séquence SEQ ID N°431, 432 | 2df |
| **SEQ ID N°909** | QIDYGNADPSTIK | 143-155 pour les protéines de SEQ N°369, 372, 484 | OXA |
| **SEQ ID N°910** | QIDYGNVDPSTIK | 143-155 pour la protéine de SEQ N°373 | 2d |
| **SEQ ID N°911** | QIGEAR | 129-134 pour les protéines de SEQ N°378, 450, 476, 485 | 2df |
| **SEQ ID N°912** | QIGQAR | 129-134 pour la protéine de SEQ N°384 | 2df |
| **SEQ ID N°913** | QIMLIEQTPAFTLR | 190-203 pour la protéine de SEQ N°470 | 2df |
| **SEQ ID N°914** | QLGSAIDQFWLR | 152-163 pour la protéine de SEQ N°379 | 2df |
| **SEQ ID N°915** | QLHDNK | 199-204 pour la protéine de SEQ N°381 | 2df |
| **SEQ ID N°916** | QLIFVHTVVQK | 229-239 pour la protéine de SEQ N°347 | 2d |
| **SEQ ID N°917** | QLIFVHTVVQKPGK | 229-242 pour la protéine de SEQ N°347 | 2d |
| **SEQ ID N°918** | QLPVK | 178-182 pour la protéine de SEQ N°433; 184-188 pour la protéine de séquence SEQ ID N°379 | OXA |
| **SEQ ID N°919** | QLPVKPR | 184-190 pour la protéine de SEQ N°379 | 2df |
| **SEQ ID N°920** | QLSLDVLDK | 265-273 pour les protéines de SEQ N°410, 413, 458 | 2df |
| **SEQ ID N°921** | QLVYAR | 237-242 pour la protéine de SEQ N°433 | 2d |
| **SEQ ID N°922** | QMMLTEASTDYIIR | 217-230 pour la protéine de SEQ N°381 | 2df |
| **SEQ ID N°923** | QMSIVEATPDYVLHGK | 214-229 pour la protéine de SEQ N°382 | 2df |
| **SEQ ID N°924** | QPTDPAR | 99-105 pour la protéine de SEQ N°433 | 2d |
| **SEQ ID N°925** | QPTDPTR | 93-99 pour les protéines de SEQ N°496, 497, 499, 500 | 2d |
| **SEQ ID N°926** | QPVSAGIR | 246-253 pour les protéines de SEQ N°496, 497, 499, 500 | 2d |
| **SEQ ID N°927** | QQLVK | 275-279 pour la protéine de SEQ N°381 | 2df |
| **SEQ ID N°928** | QTLVFAR | 232-238 pour les protéines de SEQ N°496, 497, 499, 500 | 2d |
| **SEQ ID N°929** | QVGAEK | 126-131 pour la protéine de SEQ N°470 | 2df |
| **SEQ ID N°930** | QWFAR | 23 8-243 pour la protéine de SEQ N°349 | 2d |
| **SEQ ID N°931** | SADEVLPYGGK | 84-94 pour la protéine de SEQ N°380 | 2d |
| **SEQ ID N°932** | SADEVLPYGGKPQR | 84-97 pour la protéine de SEQ N°380 | 2d |
| **SEQ ID N°933** | SCATNDLAR | 50-58 pour les protéines de SEQ N°352, 435, 492, 501; 41-49 pour les protéines de séquence SEQ ID N°348, 353, 354 | OXA |
| **SEQ ID N°934** | SCATNNLAR | 50-58 pour les protéines de SEQ N°357, 358, 359, 368, 383, 442, 471, 504 | OXA |
| **SEQ ID N°935** | SDIPGGSK | 251-258 pour la protéine de SEQ N°349 | 2d |
| **SEQ ID N°936** | SDWGK | 29-33 pour la protéine de SEQ N°375 | 2d |
| **SEQ ID N°937** | SEDNFHISSQQHEK | 27-40 pour les protéines de SEQ N°364, 365, 371, 414, 449, 506 | 2df |
| **SEQ ID N°938** | SEMPASIR | 252-259 pour les protéines de SEQ N°366, 387, 440, 459, 460, 461, 462, 463, 464, 486, 493; 253-260 pour les protéines de séquence SEQ ID N°374, 441 | 2df |
| **SEQ ID N°939** | SEMPASTR | 252-259 pour la protéine de SEQ N°465 | 2df |
| **SEQ ID N°940** | SFAAHNQDQDLR | 103-114 pour les protéines de SEQ N°356, 491 | 2d |
| **SEQ ID N°941** | SFAGHNK | 103-109 pour la protéine de SEQ N°375 | 2d |
| **SEQ ID N°942** | SFAGHNQDQDLR | 103-114 pour les protéines de SEQ N°369, 372, 373, 484 | 2d |
| **SEQ ID N°943** | SFAGHNQDQNLR | 103-114 pour les protéines de SEQ N°355, 363 | 2d |
| **SEQ ID N°944** | SFLESWAK | 100-107 pour la protéine de SEQ N°386 | 2d |
| **SEQ ID N°945** | SFTAWEK | 109-115 pour les protéines de SEQ N°366, 374, 387, 441, 459, 460, 461, 462, 463, 464, 465, 486, 493; 104-110 pour la protéine de séquence SEQ ID N°469 | 2df |
| **SEQ ID N°946** | SFTTWEK | 109-115 pour la protéine de SEQ N°440 | 2df |
| **SEQ ID N°947** | SGSGWLR | 207-213 pour la protéine de SEQ N°349 | 2d |
| **SEQ ID N°948** | SGWGMAVDPQVGWYVGFVEK | 221-240 pour les protéines de SEQ N°410, 413, 458 | 2df |
| **SEQ ID N°949** | SGWGMDVSPQVGWLTGWVEK | 219-238 pour la protéine de SEQ N°466 | 2df |
| **SEQ ID N°950** | SGWGMDVTPQVGWLTGWVEK | 219-238 pour la protéine de SEQ N°447 | 2df |
| **SEQ ID N°951** | SIHPASTFK | 69-77 pour la protéine de SEQ N°379 | 2df |
| **SEQ ID N°952** | SIPTK | 252-256 pour les protéines de SEQ N°352, 357, 358, 359, 368, 383, 435, 442, 471, 490, 492, 501, 504, 505; 243-247 pour les protéines de séquence SEQ ID N°348, 353, 354 | OXA |
| **SEQ ID N°953** | SISTK | 252-256 pour la protéine de SEQ N°489 | 2d |
| **SEQ ID N°954** | SLGLSNNLSR | 76-85 pour la protéine de SEQ N°381 | 2df |
| **SEQ ID N°955** | SLSMSGK | 4-10 pour la protéine de SEQ N°351 | 2de |
| **SEQ ID N°956** | SMLFIEEK | 202-209 pour les protéines de SEQ N°388, 389, 390, 391, 392, 393, 394, 395, 396, 397, 398, 399, 400, 401, 402, 403, 404, 405, 406, 407, 408, 409, 411, 416, 417, 418, 419, 420, 421, 422, 424, 425, 426, 427, 428, 434, 436, 437, 438, 439, 444, 445, 446, 451, 452, 453, 454, 455, 456, 457, 467, 468, 472, 473, 474, 475, 478, 479, 480, 482, 483, 487, 495, 502, 503, 507, 508; 196-203 pour les protéines de séquence SEQ ID N°431, 432 | 2df |
| **SEQ ID N°957** | SNGEK | 239-243 pour les protéines de SEQ N°447, 466 | 2df |
| **SEQ ID N°958** | SNGL THSWLGSSLK | 141-154 pour la protéine de SEQ N°498 | 2d |
| **SEQ ID N°959** | SNGYK | 208-212 pour les protéines de SEQ N°366, 374, 387, 441, 459, 460, 461, 462, 463, 464, 465, 486, 493 | 2df |
| **SEQ ID N°960** | SPTWELK | 79-85 pour la protéine de SEQ N°349 | 2d |
| **SEQ ID N°961** | SPTWELKPEYNPSPR | 79-93 pour la protéine de SEQ N°349 | 2d |
| **SEQ ID N°962** | SQDIVR | 208-213 pour la protéine de SEQ N°382 | 2df |
| **SEQ ID N°963** | SQQKPTDPTIWLK | 100-112 pour la protéine de SEQ N°351 | 2de |
| **SEQ ID N°964** | SQVGWLTGWVEQPDGK | 225-240 pour la protéine de SEQ N°486 | 2df |
| **SEQ ID N°965** | SSSNSCTTNNAAR | 46-58 pour la protéine de SEQ N°489 | 2d |
| **SEQ ID N°966** | SSSNSCTTNNATR | 46-58 pour la protéine de SEQ N°505 | 2de |
| **SEQ ID N°967** | SVYGELR | 139-145 pour la protéine de SEQ N°470 | 2df |
| **SEQ ID N°968** | SWILR | 204-208 pour la protéine de SEQ N°373 | 2d |
| **SEQ ID N°969** | SYFDEAQTQGVIIIK | 44-58 pour les protéines de SEQ N°364, 365, 371, 414, 447, 449, 466, 506 | 2df |
| **SEQ ID N°970** | SYLEK | 139-143 pour les protéines de SEQ N°355, 363 | 2d |
| **SEQ ID N°971** | SYPMWEK | 111-117 pour les protéines de SEQ N°447, 466 | 2df |
| **SEQ ID N°972** | TAYIPASTFK | 61-70 pour les protéines de SEQ N°489, 505; 77-86 pour les protéines de séquence SEQ ID N°410, 413, 458 | 2df |
| **SEQ ID N°973** | TDDLFK | 243-248 pour les protéines de SEQ N°369, 372, 373, 484 | 2d |
| **SEQ ID N°974** | TDINEIFK | 95-102 pour les protéines de SEQ N°374, 387, 440, 441, 459, 460, 461, 462, 463, 464, | 2df |
| | | 465, 486, 493 | |
| **SEQ ID N°975** | TFIHNDPR | 51-58 pour la protéine de SEQ N°470 | 2df |
| **SEQ ID N°976** | TGAGFTANR | 216-224 pour les protéines de SEQ N°360, 376; 201-209 pour la protéine de séquence SEQ ID N°361 | 2d |
| **SEQ ID N°977** | TGFNDGQK | 197-204 pour la protéine de SEQ N°385 | 2d |
| **SEQ ID N°978** | TGLADSK | 210-216 pour les protéines de SEQ N°429, 430; 209-215 pour la protéine de séquence SEQ ID N°386 | 2d |
| **SEQ ID N°979** | TGLDLMQK | 140-147 pour la protéine de SEQ N°447 | 2df |
| **SEQ ID N°980** | TGLELMQK | 140-147 pour les protéines de SEQ N°364, 365, 371, 414, 449, 506 | 2df |
| **SEQ ID N°981** | TGMGYPK | 198-204 pour la protéine de SEQ N°347 | 2d |
| **SEQ ID N°982** | TGNGR | 197-201 pour la protéine de SEQ N°494 | 2de |
| **SEQ ID N°983** | TGTGSFIDAR | 200-209 pour la protéine de SEQ N°498 | 2d |
| **SEQ ID N°984** | TGTGSLSDAK | 211-220 pour la protéine de SEQ N°351 | 2de |
| **SEQ ID N°985** | TGVATEYQPEIGWWAGWVER | 213-232 pour la protéine de SEQ N°379 | 2df |
| **SEQ ID N°986** | TGVSYPLLADGTR | 202-214 pour les protéines de SEQ N°496, 497, 499, 500 | 2d |
| **SEQ ID N°987** | TGWAAMDIK | 217-225 pour les protéines de SEQ N°374, 441 | 2df |
| **SEQ ID N°988** | TGWAMDIK | 217-224 pour les protéines de SEQ N°366, 387, 440, 460, 461, 462, 463, 464, 465, 486, 493 | 2df |
| **SEQ ID N°989** | TGWAMDVK | 217-224 pour la protéine de SEQ N°459 | 2df |
| **SEQ ID N°990** | TGWATR | 206-211 pour la protéine de SEQ N°470 | 2df |
| **SEQ ID N°991** | TGWCFDCTPELGWWVGWVK | 205-223 pour la protéine de SEQ N°380 | 2d |
| **SEQ ID N°992** | TGWEGR | 211-216 pour les protéines de SEQ N°350, 356, 367, 369, 370, 372, 373, 415, 443, 481, 484, 488, 491; 200-205 pour la protéine de séquence SEQ ID N°362 | OXA |
| **SEQ ID N°993** | TGWFVDK | 230-236 pour la protéine de SEQ N°382 | 2df |
| **SEQ ID N°994** | TGYDTK | 209-214 pour la protéine de SEQ N°476 | 2df |
| **SEQ ID N°995** | TGYGVR | 233-238 pour la protéine de SEQ N°381 | 2df |
| **SEQ ID N°996** | TGYSAR | 209-214 pour la protéine de SEQ N°450 | 2df |
| **SEQ ID N°997** | TGYSTR | 209-214 pour les protéines de SEQ N°378, 384, 485 | 2df |
| **SEQ ID N°998** | THESSNWGK | 25-33 pour les protéines de SEQ N°355, 363 | 2d |
| **SEQ ID N°999** | TICT AIADAGTGK | 25-37 pour les protéines de SEQ N°496, 497, 499, 500 | 2d |
| **SEQ ID N°1000** | TIGGAPDAYWVDDSLQISAR | 169-188 pour la protéine de SEQ N°382 | 2df |
| **SEQ ID N°1001** | TLPFSASSYETLR | 177-189 pour la protéine de SEQ N°470 | 2df |
| **SEQ ID N°1002** | TLPFSLK | 189-195 pour les protéines de SEQ N°399, 403, 411 | 2df |
| **SEQ ID N°1003** | TLPFSPK | 189-195 pour les protéines de SEQ N°389, 395, 412, 423, 428, 439, 445, 467, 482, 483, 495 | 2df |
| **SEQ ID N°1004** | TLPFSQEVQDEVQSILFIEEK | 189-209 pour la protéine de SEQ N°448 | 2df |
| **SEQ ID N°1005** | TLPFSQEVQDEVQSMLFIEEK | 189-209 pour les protéines de SEQ N°392, 434 | 2df |
| **SEQ ID N°1006** | TLPFSQK | 189-195 pour les protéines de SEQ N°388, 390, 391, 393, 394, 396, 397, 398, 400, 401, 402, 404, 405, 406, 407, 408, 409, 416, 417, 418, 419, 420, 421, 422, 424, 425, 426, 427, 436, 437, 438, 444, 446, 451, 452, 453, 454, 455, 456, 457, 468, 472, 473, 474, 475, 478, 480, 487, 502, 503, 507, 508; 183-189 pour les protéines de séquence SEQ ID N°431, 432 | 2df |
| **SEQ ID N°1007** | TLPSSQK | 189-195 pour la protéine de SEQ N°479 | 2df |
| **SEQ ID N°1008** | TLQNGWFEGFIISK | 225-238 pour les protéines de SEQ N°360, 376; 210-223 pour la protéine de séquence SEQ ID N°361 | 2d |
| **SEQ ID N°1009** | TMQEYLNK | 123-130 pour la protéine de SEQ N°385 | 2d |
| **SEQ ID N°1010** | TNGNSTSVYNESR | 51-63 pour les protéines de SEQ N°355, 363 | 2d |
| **SEQ ID N°1011** | TQTYQAYDAAR | 72-82 pour la protéine de SEQ N°382 | 2df |
| **SEQ ID N°1012** | TTDPTIWEK | 93-101 pour la protéine de SEQ N°498 | 2d |
| **SEQ ID N°1013** | TTTTEVFK | 96-103 pour les protéines de SEQ N°395, 428 | 2df |
| **SEQ ID N°1014** | TWASNDFSR | 41-49 pour la protéine de SEQ N°385 | 2d |
| **SEQ ID N°1015** | TWDMVQR | 191-197 pour la protéine de SEQ N°379 | 2df |
| **SEQ ID N°1016** | TWMQFSVVWVSQEITQK | 113-129 pour les protéines de SEQ N°360, 376; 98-114 pour la protéine de séquence SEQ ID N°361 | 2d |
| **SEQ ID N°1017** | TYPMWEK | 111-117 pour les protéines de SEQ N°364, 365,371,414,449,506 | 2df |
| **SEQ ID N°1018** | TYVVDPAR | 58-65 pour la protéine de SEQ N°379 | 2df |
| **SEQ ID N°1019** | VAFSLNIEMK | 244-253 pour la protéine de SEQ N°447 | 2df |
| **SEQ ID N°1020** | VANSLIGLSTGAVR | 70-83 pour la protéine de SEQ N°380 | 2d |
| **SEQ ID N°1021** | VEHQR | 187-191 pour les protéines de SEQ N°350, 355, 356, 363, 367, 369, 370, 372, 373, 375, 415, 443, 481, 484, 488, 491; 176-180 pour la protéine de séquence SEQ ID N°362 | OXA |
| **SEQ ID N°1022** | VELGK | 248-252 pour la protéine de SEQ N°380 | 2d |
| **SEQ ID N°1023** | VFDDAGVSGTFVLMDITADR | 38-57 pour la protéine de SEQ N°379 | 2df |
| **SEQ ID N°1024** | VFLDSWAK | 88-95 pour la protéine de SEQ N°377 | 2d |
| **SEQ ID N°1025** | VFLESWAK | 101-108 pour les protéines de SEQ N°429, 430, 477 | 2d |
| **SEQ ID N°1026** | VFLSSWAQDMNLSSAIK | 89-105 pour la protéine de SEQ N°385 | 2d |
| **SEQ ID N°1027** | VGFER | 134-138 pour la protéine de SEQ N°379 | 2df |
| **SEQ ID N°1028** | VILVFDQVR | 55-63 pour les protéines de SEQ N°356, 491 | 2d |
| **SEQ ID N°1029** | VITFTK | 228-233 pour la protéine de SEQ N°494 | 2de |
| **SEQ ID N°1030** | VMAAMVR | 158-164 pour la protéine de SEQ N°381 | 2df |
| **SEQ ID N°1031** | VPLAVMGYDAGILVDAHNPR | 58-77 pour la protéine de SEQ N°498 | 2d |
| **SEQ ID N°1032** | VQANVK | 195-200 pour les protéines de SEQ N°366, 374, 387, 440, 441, 459, 460, 461, 462, 463, 464, 465, 486, 493 | 2df |
| **SEQ ID N°1033** | VQDEVK | 196-201 pour la protéine de SEQ N°444 | 2df |
| **SEQ ID N°1034** | VQDEVQSMLFIEEK | 196-209 pour les protéines de SEQ N°388, 389, 390, 391, 392, 393, 395, 396, 397, 398, 399, 400, 401, 402, 404, 405, 406, 407, 408, | 2df |
| | | 409, 411, 416, 417, 418, 419, 420, 421, 422, 424, 425, 426, 427, 428, 434, 436, 437, 438, 439, 445, 446, 451, 452, 453, 454, 455, 457, 467, 468, 472, 473, 474, 475, 478, 479, 480, 482, 483, 487, 495, 502, 503, 507, 508; 190-203 pour les protéines de séquence SEQ ID N°431, 432 | |
| **SEQ ID N°1035** | VQDEVQSMLFIEEMNGNK | 196-213 pour les protéines de SEQ N°412, 423 | 2df |
| **SEQ ID N°1036** | VQDGVQSMLFIEEK | 196-209 pour la protéine de SEQ N°456 | 2df |
| **SEQ ID N°1037** | VQHEVQSMLFIEEK | 196-209 pour la protéine de SEQ N°394 | 2df |
| **SEQ ID N°1038** | VSCLPCYQVVSHK | 138-150 pour la protéine de SEQ N°382 | 2df |
| **SEQ ID N°1039** | VSCVWCYQALAR | 114-125 pour la protéine de SEQ N°470 | 2df |
| **SEQ ID N°1040** | VSDVCSEVTAEGWQEVR | 37-53 pour la protéine de SEQ N°381 | 2df |
| **SEQ ID N°1041** | VSEVEGWQIHGK | 186-197 pour la protéine de SEQ N°347 | 2d |
| **SEQ ID N°1042** | VSFSLNIEMK | 244-253 pour la protéine de SEQ N°466 | 2df |
| **SEQ ID N°1043** | VSPCSSFK | 54-61 pour la protéine de SEQ N°349 | 2d |
| **SEQ ID N°1044** | VSQVPAYK | 105-112 pour la protéine de SEQ N°377 | 2d |
| **SEQ ID N°1045** | VVFAR | 229-233 pour la protéine de SEQ N°498; 239-243 pour les protéines de séquence SEQ ID N°349,351 | OXA |
| **SEQ ID N°1046** | WDGAK | 97-101 pour les protéines de SEQ N°355, 363 | 2d |
| **SEQ ID N°1047** | WDGEK | 104-108 pour les protéines de SEQ N°393, 402, 419, 422, 424 | 2df |
| **SEQ ID N°1048** | WDGHIYDFPDWNR | 92-104 pour la protéine de SEQ N°470 | 2df |
| **SEQ ID N°1049** | WDGIK | 97-101 pour les protéines de SEQ N°356, 491 | 2d |
| **SEQ ID N°1050** | WDGKPR | 92-97 pour les protéines de SEQ N°352, 357, 358, 359, 368, 383, 435, 442, 471, 489, 492, 501, 504, 505; 83-88 pour les protéines de séquence SEQ ID N°348, 353, 354; 107-112 pour les protéines de séquence SEQ ID N°410, 413, 458; 93-98 pour la protéine de séquence SEQ ID N°490; 116-121 pour la protéine de séquence SEQ ID N°382; | OXA |
| **SEQ ID N°1051** | WDGQK | 104-108 pour les protéines de SEQ N°388, 389, 392, 395, 396, 397, 399, 403, 405, 406, 407, 411, 412, 423, 428, 434, 439, 445, 446, | 2df |
| | | 448, 467, 468, 482, 483, 495, 503, 508; 98-102 pour les protéines de séquence SEQ ID N°431, 432 | |
| **SEQ ID N°1052** | WDGQTR | 95-100 pour les protéines de SEQ N°378, 384, 450, 476, 485 | 2df |
| **SEQ ID N°1053** | WDGVK | 97-101 pour les protéines de SEQ N°369, 372, 375, 484 | 2d |
| **SEQ ID N°1054** | WDGVNR | 97-102 pour les protéines de SEQ N°350, 367, 370, 373, 415, 443, 481, 488; 86-91 pour la protéine de séquence SEQ ID N°362 | OXA |
| **SEQ ID N°1055** | WDYKPEFNGYK | 78-88 pour la protéine de SEQ N°498; 89-99 pour la protéine de séquence SEQ ID N°351 | OXA |
| **SEQ ID N°1056** | WETYSVVWFSQQITEWLGMER | 97-117 pour la protéine de SEQ N°347 | 2d |
| **SEQ ID N°1057** | WNGQK | 104-108 pour les protéines de SEQ N°394, 418,444,507 | 2df |
| **SEQ ID N°1058** | YAQAK | 155-159 pour la protéine de SEQ N°382 | 2df |
| **SEQ ID N°1059** | YFSDFNAK | 34-41 pour les protéines de SEQ N°355, 363 | 2d |
| **SEQ ID N°1060** | YGTHLDR | 68-74 pour les protéines de SEQ N°410, 413, 458 | 2df |
| **SEQ ID N°1061** | YIIHNK | 54-59 pour les protéines de SEQ N°386, 429, 430, 477 | 2d |
| **SEQ ID N°1062** | YLDELVK | 245-251 pour la protéine de SEQ N°385 | 2d |
| **SEQ ID N°1063** | YLMITEAGR | 195-203 pour la protéine de SEQ N°484 | OXA |
| **SEQ ID N°1064** | YLNLFSYGNANIGGGIDK | 135-152 pour les protéines de SEQ N°489, 505 | OXA |
| **SEQ ID N°1065** | YNGEK | 96-100 pour les protéines de SEQ N°429, 430, 477 | 2d |
| **SEQ ID N°1066** | YPHNPR | 88-93 pour la protéine de SEQ N°494 | 2de |
| **SEQ ID N°1067** | YPVVWYSQQVAHHLGAQR | 103-120 pour la protéine de SEQ N°497 | 2d |
| **SEQ ID N°1068** | YSNVLAFK | 106-113 pour la protéine de SEQ N°385 | 2d |
| **SEQ ID N°1069** | YSPASTFK | 68-75 pour les protéines de SEQ N°350, 355, 356, 363, 367, 369, 370, 372, 373, 375, 415, 443, 481, 484, 488, 491; 57-64 pour la protéine de séquence SEQ ID N°362 | OXA |
| **SEQ ID N°1070** | YSVVPVYQQLAR | 141-152 pour la protéine de SEQ N°381 | 2df |
| **SEQ ID N°1071** | YSVVWYSQLTAK | 109-120 pour la protéine de SEQ N°433 | 2d |
| **SEQ ID N°1072** | YSVVWYSQQVAHHLGAQR | 103-120 pour les protéines de SEQ N°496, 499, 500 | 2d |
| **SEQ ID N°1073** | YTPASTFK | 55-62 pour la protéine de SEQ N°433 | 2d |
| **SEQ ID N°1074** | YTSAFGYGNADVSGEPGK | 130-147 pour la protéine de SEQ N°433 | 2d |
| **SEQ ID N°1075** | YVFVSALTGNLGSNLTSSIK | 228-247 pour la protéine de SEQ N°361; 243-262 pour la protéine de séquence SEQ ID N°360 | 2d |
| **SEQ ID N°1076** | YVFVSALTGSLGSNLTSSIK | 243-262 pour la protéine de SEQ N°376 | 2d |
| **SEQ ID N°1077** | YVGHDR | 50-55 pour la protéine de SEQ N°380 | 2d |
| **SEQ ID N°1098** | ANQAFLP ASTFK | 62-73 pour les protéines de SEQ N°378, 384, 450, 476, 485 | 2df |
| **SEQ ID N°1099** | DEHQVFK | 88-94 pour les protéines de SEQ N°378, 384, 450, 476, 485 | 2df |
| **SEQ ID N°1100** | DHNLITAMK | 108-116 pour les protéines de SEQ N°378, 450, 476 | 2df |
| **SEQ ID N°1101** | DIATWNR | 101-107 pour les protéines de SEQ N°378, 450, 476 | 2df |
| **SEQ ID N°1102** | IPNSLIALDLGVVK | 74-87 pour les protéines de SEQ N°378, 384, 450, 476, 485 | 2df |
| **SEQ ID N°1103** | ISATEQISFLR | 164-174 pour les protéines de SEQ N°378, 450, 476 | 2df |
| **SEQ ID N°1104** | QAMLTEANGDYIIR | 193-206 pour les protéines de SEQ N°378, 450, 476, 485 | 2df |
| **SEQ ID N°1105** | QQGFTNNLK | 52-60 pour les protéines de SEQ N°378, 384, 450, 476, 485 | 2df |
| **SEQ ID N°1106** | SQGVVVLWNENK | 40-51 pour les protéines de SEQ N°378, 450, 476, 485 | 2df |
| **SEQ ID N°1107** | SWNAHFTEHK | 30-39 pour les protéines de SEQ N°378, 450, 476, 485 | 2df |
| **SEQ ID N°1108** | VLALSAVFLVASIIGMPAVAK | 3-23 pour les protéines de SEQ N°378, 450, 476, 485 | 2df |
| **SEQ ID N°1109** | YSVVPVYQEFAR | 117-128 pour les protéines de SEQ N°378, 384, 450, 476, 485 | 2df |

Dans la colonne intérêt clinique, les mentions 2d, 2de, 2df correspondent aux sous-groupes fonctionnels des beta-lactamases OXA que permet de détecter le peptide correspondant. Ainsi la détection d'un peptide 2df signera la présence d'une beta-lactamase de type carbapénèmase capable d'hydrolyser les carbapénèmes.

La mention 2de signera la présence d'une beta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La mention OXA indique un peptide commun entre au moins deux des sous-groupes 2d, 2de et 2df. Le peptide correspondant signe la présence d'une beta-lactamase de type OXA et la présence d'un mécanisme de résistance au moins aux pénicillines et aux céphalosporines de première génération.
La détection d'un mécanisme de résistance aux carbapénèmes induit par une protéine OXA, est caractérisée par la détection d'au moins un peptide marqueur de résistance, de type carba, choisi parmi les séquences **SEQ ID N° 510, 511, 512, 513, 514, 520, 521, 522, 523, 525, 527, 530, 532, 537, 541, 542, 543, 544, 545, 546, 547, 548, 549, 550, 551, 552, 556, 557, 558, 559, 560, 561, 562, 574, 579, 581, 582, 583, 584, 592, 596, 597, 598, 599, 600, 601, 602, 607, 608, 609, 628, 631, 632, 633, 635, 636, 644, 646, 647, 649, 650, 655, 656, 661, 662, 667, 674, 675, 682, 689, 690, 698, 713, 714, 719, 720, 722, 727, 729, 730, 741, 746, 748, 750, 751, 752, 755, 756, 757, 758, 763, 764, 767, 768, 772, 775, 781, 782, 790, 792, 793, 794, 795, 796, 797, 798, 801, 809, 811, 812, 813, 814, 816, 819, 824, 832, 834, 837, 838, 847, 851, 852, 853, 854, 855, 856, 857, 858, 859, 860, 862, 868, 869, 870, 874, 875, 876, 877, 879, 880, 881, 882, 894, 895, 898, 902, 903, 904, 906, 907, 908, 911, 912, 913, 914, 915, 919, 920, 922, 923, 927, 929, 937, 938, 939, 945, 946, 948, 949, 950, 951, 954, 956, 957, 959, 962, 964, 967, 969, 971, 972, 974, 975, 979, 980, 985, 988, 990, 993, 994, 995, 996, 997, 1000, 1001, 1002, 1003, 1004, 1005, 1006, 1007, 1011, 1013, 1015, 1017, 1018, 1019, 1023, 1027, 1030, 1032, 1033, 1034, 1035, 1036, 1037, 1038, 1039, 1040, 1042, 1047, 1048, 1051, 1052, 1057, 1058, 1060, 1070, 1098, 1099, 1100, 1101, 1102, 1103, 1104, 1105, 1106, 1107, 1108, 1109**

Certaines séquences peptidiques peuvent être communes à plusieurs mécanismes de résistance. Ainsi les séquences suivantes sont identiques :
SEQ ID N°24 et SEQ ID N°287

Dans tous les cas, les séquences ci-dessus signent l'expression d'un mécanisme de résistance aux pénicillines, aux céphalosporines y compris celles de troisième génération telles que céfotaxime/ceftazidime, aux monobactames et aux carbapénèmes.

Le procédé de l'invention et ses avantages ressortiront de la suite de la présente description qui présente plusieurs exemples, non limitatifs, de mise en oeuvre dudit procédé.

### Exemple 1 : Identification de microorganismes à partir d'un échantillon par profil biochimique

### 1. Mise-en en culture de l'échantillon sur milieu de culture

Les milieux de culture optimaux et les conditions de culture optimales sont différents selon les espèces de microorganisme. Par défaut l'échantillon est ensemencé sur différents milieux :
∘ gélose Columbia au sang de mouton (réf bioMérieux 43041) pendant 18 à 24 h à 35°C, en atmosphère aérobie ou anaérobie ;
∘ gélose TSA (référence bioMérieux 43011) pendant 18 à 24 h à 37°C.

### 2. Identification des microorganismes

L'identification est mise en oeuvre comme suit :
1. Sélection de colonies isolées
2. En respectant les conditions d'asepsie, transfert de 3,0 mL de solution saline stérile aqueuse (à 0,45-0,50 % de NaCl, de pH 4,5 à 7,0) dans un tube à essai en plastique transparent (polystyrène)
3. A l'aide d'un bâtonnet ou d'un écouvillon stérile, transfert d'un nombre suffisant de colonies identiques dans le tube de solution saline préparé à l'étape 2 et ajustement de la suspension bactérienne entre 0,50 et 0,63 McFarland avec un DENSICHEK étalonné du VITEK®
4. Positionnement du tube de suspension bactérienne et d'une carte d'identification VITEK® sur une cassette VITEK®
5. Chargement de la cassette dans l'instrument VITEK®
6. Les opérations de remplissage, scellage, incubation et lecture sont automatiques
7. Acquisition d'un profil biochimique
8. Identification avec le système VITEK® réalisée par comparaison à des profils biochimiques de souches connues

### Exemple 2 : Préparation d'un échantillon primaire urinaire par enrichissement en microorganismes:

Le protocole suivant est mis en oeuvre en 16 étapes (les étapes 5 à 12 sont optionnelles et pourraient être omises si l'échantillon enrichi est ultérieurement traité selon les exemples 4 et suivants) :
1. Centrifugation de 5 mL d'urine contaminée, à 2000g pendant 30 secondes
2. Récupération du surnageant
3. Centrifugation à 15000g pendant 5 minutes
4. Elimination du surnageant
5. Lavage du culot avec 3 mL d'eau distillée par remise en suspension
6. Centrifugation à 15000g pendant 5 minutes
7. Elimination du surnageant
8. Mettre le culot en présence de solvant (8 volumes acétone pour 1 volume de méthanol) en dilution au 1/10
9. Laisser 1 heure à -20°C
10. Centrifugation à 15000g pendant 5 minutes
11. Elimination du surnageant
12. Mettre le culot en présence de solvant (8 volumes acétone pour 1 volume de méthanol) en dilution au 1/10
13. Laisser 1 heure à -20°C
14. Centrifugation à 15000g pendant 5 minutes
15. Elimination du surnageant
16. Le culot constitue l'échantillon enrichi en microorganisme

### Exemple 3 : Identification de microorganismes à partir d'un échantillon par MALDI-TOF

L'identification est mise en oeuvre comme suit :
1. Transfert, à l'aide d'une oese de 1µl, d'une portion de colonie de microorganisme obtenue selon l'exemple 1, ou d'un échantillon enrichi selon l'exemple 2, et dépôt uniforme sur une plaque pour spectrométrie de masse par MALDI-TOF
2. Recouvrement du dépôt avec 1 µl de matrice. La matrice utilisée est une solution saturée d'HCCA (acide alpha-cyano-4-hydroxycinnamique) en solvant organique (50% d'acétonitrile et 2,5% d'acide trifluoroacétique)
3. Séchage à température ambiante
4. Introduction de la plaque dans le spectromètre de masse
5. Acquisition d'un spectre de masse
6. Comparaison du spectre obtenu avec les spectres contenus dans une base de connaissance
7. Identification du microorganisme par comparaison des pics obtenus à ceux de la base de connaissance

### Exemple 4 : Identification de microorganismes à partir d'un échantillon par ESI-MS

L'identification est mise en oeuvre comme suit :
1. Prélèvement d'une colonie de microorganisme, obtenue selon l'exemple 1, ou d'un échantillon enrichi selon l'exemple 2, et mise en suspension dans 100µl d'eau déminéralisée.
2. Centrifugation à 3000g pendant 5 minutes.
3. Elimination du surnageant.
4. Remise en suspension dans 100µl d'eau déminéralisée.
5. Centrifugation à 3000g pendant 5 minutes.
6. Elimination du surnageant.
7. Remise en suspension dans 100µl d'un mélange Acétonitrile, eau déminéralisée, acide formique (50/50/0.1%).
8. Filtration avec un filtre de porosité 0.45 µm.
9. Injection dans un spectromètre de masse en mode MS simple.
10. Acquisition d'un spectre de masse.
11. Comparaison du spectre obtenu avec les spectres contenus dans une base de connaissance.
12. Identification du microorganisme par référence à des spectres de référence.

### Exemple 5 : Obtention de protéines digérées à partir de microorganismes

Classiquement le protocole suivant est mis en oeuvre en 17 étapes :
1. Prélèvement d'une colonie de microorganisme, obtenue selon l'exemple 1, ou d'un échantillon enrichi selon l'exemple 2, et mise en suspension dans 10 à 100µl d'une solution d'hydrochlorure de guanidine 6M, 50 mM Tris-HCl, pH=8,0.
2. Ajout de dithiothréitol (DTT) pour obtenir une concentration finale de 5mM.
3. Réduction pendant 20 minutes à 95°C dans un bain-marie.
4. Refroidissement des tubes à température ambiante.
5. Ajout d'iodoacétamide pour obtenir une concentration finale de 12.5 mM.
6. Alkylation pendant 40 minutes à température ambiante et à l'obscurité.
7. Dilution d'un facteur 6 avec une solution de NH₄HCO₃ 50 mM, pH=8.0 pour obtenir une concentration finale en hydrochlorure de guanidine de 1M.
8. Ajout de 1 µg de trypsine.
9. Digestion à 37°C pendant 6 heures jusqu'à une nuit.
10. Ajout d'acide formique jusqu'à un pH inférieur à 4 pour stopper la réaction.
11. Le volume d'échantillon est complété à 1mL avec eau/acide formique 0,5% (v/v)
12. Equilibrage des colonnes HLB Oasis Waters avec 1ml de méthanol puis 1 ml H₂O/acide formique 0,1% (v/v)
13. Dépôt de l'échantillon qui s'écoule par gravité
14. Lavage avec 1 ml H₂O/acide formique 0,1% (v/v)
15. Elution avec 1ml d'un mélange 80% de méthanol et 20% d'eau/acide formique 0,1% (v/v)
16. L'éluat est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.
17. L'éluat est ensuite repris dans une solution eau/acide formique 0,5% (v/v) quantité suffisante pour (QSP) 250µl

### Exemple 6 : Identification d'une résistance aux beta-lactamines de type NDM-1:

Les échantillons Ech1 à Ech9 sont identifiés selon l'une des méthodes décrites dans les exemples 1, 3 ou 4. L'identification des espèces est reportée dans le **TABLEAU 1.**

**TABLEAU 1 :**

| Noms | Espèces |
|---|---|
| Ech1 | *K. pneumoniae* |
| Ech2 | *C. freundii* |
| Ech3 | *A. baumannii* |
| Ech4 | *A. caviae* |
| Ech5 | *C. braakii* |
| Ech6 | *E. cloacae* |
| Ech7 | *P. rettgeri* |
| Ech8 | *E. coli* |
| Ech9 | *K. pneumoniae* |

Les échantillons Ech1 à Ech9 correspondent à une espèce pouvant comporter un mécanisme de résistance de type NDM-1 (Enterobacteriaceae, *Pseudomonas* species, *Acinetobacter* species...). La méthode suivante est alors mise en oeuvre pour rechercher un tel mécanisme.

Chaque échantillon est traité selon l'exemple 5, puis un volume de 50µl de protéines digérées est injecté et analysé selon les conditions suivantes :
- Chaîne chromatographique Dionex Ultimate 3000 de la société Dionex Corporation (Sunnyvale, Etats Unis d'Amerique).
- Colonne Waters BEH130 C18, 2,1mm de diamètre interne, 100mm de long, taille de particule de 3,5µm (Waters, Saint-Quentin En Yvelines, France).
- Solvant A : H₂O + 0,1% acide formique.
- Solvant B : ACN + 0,1% acide formique.

Gradient HPLC défini dans le **TABLEAU 2** ci-après.

**TABLEAU 2 :**

| Temps (min) | Débit (µl) | Solvant A (%) | Solvant B (%) |
|---|---|---|---|
| 0 | 300 | 98 | 2 |
| 3 | 300 | 98 | 2 |
| 34 | 300 | 54.6 | 45.4 |
| 35 | 300 | 0 | 100 |
| 55 | 300 | 0 | 100 |
| 55.1 | 300 | 98 | 2 |
| 74 | 300 | 98 | 2 |

- L'éluat en sortie de colonne chromatographique est directement injecté dans la source d'ionisation du spectromètre de masse de type QTRAP® 5500 de la société Applied Biosystems (Foster City, Etats Unis d'Amérique).
- Les peptides, issus de la digestion des protéines du microorganisme, sont analysés par le spectromètre de masse en mode MRM. Seuls les peptides indiqués dans le **TABLEAU 3** sont détectés. Pour cela, le ou les fragments indiqués dans le **TABLEAU 3** sont détectés.

**TABLEAU 3 :**

| Transition numéro | Peptide | Etat de charge du précurseur | Ion fragment | Temps de rétention (minute) | (m/z) filtré en Q1 | (m/z) filtré en Q3 | Energie de collision (eV) |
|---|---|---|---|---|---|---|---|
| 1 | AAITHTAR | 2 | y4 monochargé | 5,61 | 420,74 | 484,26 | 24 |
| 2 | AAITHTAR | 2 | y5 monochargé | 5,61 | 420,74 | 585,31 | 24 |
| 3 | AAITHTAR | 2 | y6 monochargé | 5,61 | 420,74 | 698,39 | 24 |
| 4 | AFGAAFPK | 2 | y6 monochargé | 16,03 | 404,72 | 590,33 | 23 |
| 5 | AFGAAFPK | 2 | y7 monochargé | 16,03 | 404,72 | 737,4 | 23 |
| 6 | AFGAAFPK | 2 | y7 dichargé | 16,03 | 404,72 | 369,2 | 23 |
| 7 | ASMIVMSHSAPDSR | 2 | y7 monochargé | 13,65 | 744,85 | 769,36 | 38 |
| 8 | ASMIVMSHSAPDSR | 2 | y8 monochargé | 13,65 | 744,85 | 856,39 | 38 |
| 9 | ASMIVMSHSAPDSR | 2 | y9 monochargé | 13,65 | 744,85 | 987,43 | 38 |
| 10 | FGDLVFR | 2 | y4 monochargé | 19,14 | 427,23 | 534,34 | 24 |
| 11 | FGDLVFR | 2 | y5 monochargé | 19,14 | 427,23 | 649,37 | 24 |
| 12 | FGDLVFR | 2 | y6 monochargé | 19,14 | 427,23 | 706,39 | 24 |
| 13 | MELPNIMHPVAK | 2 | y10 dichargé | 19,09 | 690,36 | 560,32 | 35 |
| 14 | MELPNIMHPVAK | 2 | y9 monochargé | 19,09 | 690,36 | 1006,55 | 35 |
| 15 | MELPNIMHPVAK | 2 | y9 dichargé | 19,09 | 690,36 | 503,78 | 35 |
| 16 | QEINLPVALAVVTHAHQDK | 3 | y14 dichargé | 21,34 | 695,05 | 743,41 | 39 |
| 17 | QEINLPVALAVVTHAHQDK | 3 | y7 monochargé | 21,34 | 695,05 | 836,4 | 39 |
| 18 | QEINLPVALAVVTHAHQDK | 3 | y8 monochargé | 21,34 | 695,05 | 935,47 | 39 |
| 19 | SLGNLGDADTEHYAASAR | 2 | y14 dichargé | 14,64 | 924,43 | 738,84 | 46 |
| 20 | SLGNLGDADTEHYAASAR | 2 | y16 dichargé | 14,64 | 924,43 | 824,37 | 46 |
| 21 | SLGNLGDADTEHYAASAR | 2 | y7 monochargé | 14,64 | 924,43 | 775,38 | 46 |
| 22 | VLVVDTAWTDDQTAQILNWIK | 3 | y5 monochargé | 27,16 | 810,43 | 673,4 | 45 |
| 23 | VLVVDTAWTDDQTAQILNWIK | 3 | y6 monochargé | 27,16 | 810,43 | 786,49 | 45 |
| 24 | VLVVDTAWTDDQTAQILNWIK | 3 | y7 monochargé | 27,16 | 810,43 | 914,55 | 45 |

L'état de charge du peptide précurseur, son temps de rétention, le type d'ion fragment et les transitions, c'est-à-dire les rapports (m/z)₁ en Q1 et (m/z)₂ en Q3, sont indiqués dans le **TABLEAU 3**. L'énergie de collision utilisée pour fragmenter l'ion précurseur est également indiquée dans le **TABLEAU 3.**
• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 500,00 °C |
| Gaz de nébulisation: | 50,00 psi |
| Gaz chauffant: | 40,00 psi |
| Gaz de collision induisant dissociation : | 9,00 psi |
| Remplissage dynamique: | activé |
| Potentiel d'orifice : | 80,00 V |
| (en anglais declustering potential (DP)) | |
| Potentiel d'entrée avant Q0 (EP): | 10,00 V |
| Potentiel en sortie de cellule de collision : | 35 V |
| (en anglais cell exit potential (CXP)) | |
| Temps de cycle total: | 1.2 sec |
| Fenêtre de détection : | 90 sec |

Les aires obtenues pour chacune des transitions et pour chacun des microorganismes étudiés ont été mesurées. Toutes les transitions dont l'aire est supérieure ou égale à 2500 (unité arbitraire) sont considérées comme positives et ont été notées « 1 » dans le **TABLEAU 4**. Toutes les transitions dont l'aire est inférieure à 2500, sont considérées comme négatives et ont été notées 0 dans le **TABLEAU 4**. Lorsqu'on n'a observé aucun pic de signal, on a noté la transition comme négative.

**TABLEAU 4 :**

| Numéro transition | Ech1 | Ech2 | Ech3 | Ech4 | Ech5 | Ech6 | Ech7 | Ech8 | Ech9 |
|---|---|---|---|---|---|---|---|---|---|
| 1 | 1 | 1 | 1 | 1 | 1 | 1 | 0 | 1 | 1 |
| 2 | 1 | 1 | 1 | 1 | 1 | 1 | 1 | 1 | 1 |
| 3 | 1 | 1 | 0 | 1 | 1 | 1 | 0 | 1 | 1 |
| 4 | 1 | 1 | 1 | 1 | 1 | 1 | 1 | 1 | 1 |
| 5 | 1 | 1 | 1 | 1 | 1 | 1 | 1 | 1 | 1 |
| 6 | 1 | 1 | 0 | 1 | 1 | 1 | 1 | 1 | 1 |
| 7 | 0 | 0 | 0 | 0 | 0 | 0 | 0 | 0 | 0 |
| 8 | 0 | 0 | 0 | 0 | 0 | 0 | 0 | 0 | 0 |
| 9 | 0 | 0 | 0 | 0 | 0 | 0 | 0 | 0 | 0 |
| 10 | 1 | 1 | 1 | 1 | 1 | 1 | 1 | 1 | 1 |
| 11 | 1 | 1 | 1 | 1 | 1 | 1 | 1 | 1 | 1 |
| 12 | 1 | 1 | 1 | 1 | 1 | 1 | 1 | 1 | 1 |
| 13 | 0 | 0 | 0 | 0 | 0 | 0 | 0 | 0 | 0 |
| 14 | 0 | 0 | 0 | 0 | 0 | 0 | 0 | 0 | 0 |
| 15 | 0 | 0 | 0 | 0 | 0 | 0 | 0 | 0 | 0 |
| 16 | 1 | 1 | 0 | 1 | 1 | 0 | 0 | 0 | 1 |
| 17 | 1 | 1 | 0 | 1 | 1 | 1 | 1 | 0 | 1 |
| 18 | 1 | 1 | 0 | 1 | 1 | 0 | 1 | 0 | 1 |
| 19 | 0 | 0 | 0 | 0 | 0 | 0 | 0 | 0 | 0 |
| 20 | 0 | 0 | 0 | 0 | 0 | 0 | 0 | 0 | 0 |
| 21 | 0 | 0 | 0 | 0 | 0 | 0 | 0 | 0 | 0 |
| 22 | 0 | 0 | 0 | 0 | 0 | 0 | 0 | 0 | 0 |
| 23 | 0 | 0 | 0 | 0 | 0 | 0 | 0 | 0 | 0 |
| 24 | 0 | 0 | 0 | 0 | 0 | 0 | 0 | 0 | 0 |

Le nombre de transitions positives est ensuite sommé et reporté dans le **TABLEAU 5 :**

**TABLEAU 5 :**

| Noms | Espèces | Nombre de transitions positives |
|---|---|---|
| Ech1 | *K. pneumoniae* | 12 |
| Ech2 | *C. freundii* | 12 |
| Ech3 | *A. baumannii* | 7 |
| Ech4 | *A. caviae* | 12 |
| Ech5 | *C. braakii* | 12 |
| Ech6 | *E. cloacae* | 10 |
| Ech7 | *P. rettgeri* | 9 |
| Ech8 | *E. coli* | 9 |
| Ech9 | *K. pneumoniae* | 12 |

Les échantillons Ech1 à Ech9 comportent plus de 6 transitions positives, ils contiennent donc des bactéries qui expriment la protéine NDM-1. Les bactéries des Ech1 à Ech9 sont donc résistantes aux pénicillines, aux céphalosporines et aux carbapénèmes.

### Exemple 7 : Identification d'une résistance aux béta-lactamines de type KPC:

Les échantillons Ech62 à Ech73 sont identifiés selon l'une des méthodes décrites dans les exemples 1, 3 ou 4. L'identification des espèces est reportée dans le **TABLEAU 6.**

**TABLEAU 6 :**

| Noms | Espèces |
|---|---|
| Ech62 | *K. pneumoniae* |
| Ech63 | *K. pneumoniae* |
| Ech64 | *K. pneumoniae* |
| Ech65 | *K. pneumoniae* |
| Ech66 | *K. pneumoniae* |
| Ech67 | *K. pneumoniae* |
| Ech68 | *K. pneumoniae* |
| Ech69 | *K. pneumoniae* |
| Ech70 | *K. pneumoniae* |
| Ech71 | *K. pneumoniae* |
| Ech72 | *K. pneumoniae* |
| Ech73 | *K. pneumoniae* |

Les échantillons Ech62 à Ech73 correspondent à une espèce pouvant comporter un mécanisme de résistance de type KPC. La méthode suivante est alors mise en oeuvre pour détecter un tel mécanisme.

Chaque échantillon est traité selon l'exemple 5, puis analysé selon l'exemple 6 en détectant les peptides du **TABLEAU 7** en lieu et place des peptides du **TABLEAU 3.**

**TABLEAU 7 :**

| Transition numéro | Peptide | Oxydation méthionine | Etat de charge du précurseur | Ion fragment | Intérêt clinique |
|---|---|---|---|---|---|
| 1 | AAVPADWAVGDK | non | 2 | y9 dichargé | 2f |
| 2 | AAVPADWAVGDK | non | 2 | y10 dichargé | 2f |
| 3 | AAVPADWAVGDK | non | 2 | y9 monochargé | 2f |
| 4 | APIVLAVYTR | non | 2 | y7 monochargé | 2f |
| 5 | APIVLAVYTR | non | 2 | y5 monochargé | 2f |
| 6 | APIVLAVYTR | non | 2 | y6 monochargé | 2f |
| 7 | AVTESLQK | non | 2 | y5 monochargé | 2f |
| 8 | AVTESLQK | non | 2 | y6 monochargé | 2f |
| 9 | AVTESLQK | non | 2 | y4 monochargé | 2f |
| 10 | ELGGPAGLTAFMR | oui | 2 | y7 monochargé | 2f |
| 11 | ELGGPAGL TAFMR | oui | 2 | y5 monochargé | 2f |
| 12 | ELGGPAGL TAFMR | oui | 2 | y9 dichargé | 2f |
| 13 | ELGGPAGLTAFMR | non | 2 | y7 monochargé | 2f |
| 14 | ELGGPAGL TAFMR | non | 2 | y5 monochargé | 2f |
| 15 | ELGGPAGLTAFMR | non | 2 | y9 dichargé | 2f |
| 16 | FPLCSSFK | non | 2 | y6 monochargé | 2f |
| 17 | FPLCSSFK | non | 2 | y7 monochargé | 2f |
| 18 | FPLCSSFK | non | 2 | y5 monochargé | 2f |
| 19 | GFLAAAVLAR | non | 2 | y6 monochargé | 2f |
| 20 | GFLAAAVLAR | non | 2 | y7 monochargé | 2f |
| 21 | GFLAAAVLAR | non | 2 | y5 monochargé | 2f |
| 22 | GNTTGNHR | non | 2 | y5 monochargé | 2f |
| 23 | GNTTGNHR | non | 2 | y6 monochargé | 2f |
| 24 | GNTTGNHR | non | 2 | y4 monochargé | 2f |
| 25 | LALEGLGVNGQ | non | 3 | y8 monochargé | 2f |
| 26 | LALEGLGVNGQ | non | 3 | y7 monochargé | 2f |
| 27 | LALEGLGVNGQ | non | 3 | y6 monochargé | 2f |
| 28 | LTLGSALAAPQR | non | 3 | y9 monochargé | 2f |
| 29 | LTLGSALAAPQR | non | 3 | y5 monochargé | 2f |
| 30 | LTLGSALAAPQR | non | 3 | y6 monochargé | 2f |
| 31 | NALVPWSPISEK | non | 2 | y8 monochargé | 2f |
| 32 | NALVPWSPISEK | non | 2 | y8 dichargé | 2f |
| 33 | NALVPWSPISEK | non | 2 | y5 monochargé | 2f |
| 34 | QQFVDWLK | non | 2 | y5 monochargé | 2f |
| 35 | QQFVDWLK | non | 2 | y6 monochargé | 2f |
| 36 | QQFVDWLK | non | 2 | y4 monochargé | 2f |
| 37 | SIGDTTFR | non | 2 | y5 monochargé | 2f |
| 38 | SIGDTTFR | non | 2 | y6 monochargé | 2f |
| 39 | SIGDTTFR | non | 2 | y4 monochargé | 2f |
| 40 | SQQQAGLLDTPIR | non | 2 | y8 monochargé | 2f |
| 41 | SQQQAGLLDTPIR | non | 2 | y9 monochargé | 2f |
| 42 | SQQQAGLLDTPIR | non | 2 | y10 monochargé | 2f |
| 43 | WELELNSAIPGDAR | non | 2 | y5 monochargé | 2f |
| 44 | WELELNSAIPGDAR | non | 2 | y8 monochargé | 2f |
| 45 | WELELNSAIPGDAR | non | 2 | y9 monochargé | 2f |

Les transitions mentionnées dans le **TABLEAU 7** sont détectés en utilisant les paramètres figurant dans le **TABLEAU 8.**

| Transition numéro | Temps de rétention (minutes) | (m/z) filtré en Q1 | (m/z) filtré en Q3 | Energie de collision (eV) | Seuil de positivité |
|---|---|---|---|---|---|
| 1 | 16.29 | 600.31 | 479.73 | 31 | 2000 |
| 2 | 16.29 | 600.31 | 529.27 | 31 | 2000 |
| 3 | 16.29 | 600.31 | 958.46 | 31 | 2000 |
| 4 | 19.07 | 551.83 | 821.49 | 29 | 13000 |
| 5 | 19.07 | 551.83 | 609.33 | 29 | 13000 |
| 6 | 19.07 | 551.83 | 722.42 | 29 | 13000 |
| 7 | 10.38 | 438.25 | 604.33 | 24 | 2000 |
| 8 | 10.38 | 438.25 | 705.38 | 24 | 2000 |
| 9 | 10.38 | 438.25 | 475.29 | 24 | 2000 |
| 10 | 18.55 | 668.34 | 811.41 | 34 | 2000 |
| 11 | 18.55 | 668.34 | 641.31 | 34 | 2000 |
| 12 | 18.55 | 668.34 | 490.26 | 34 | 2000 |
| 13 | 21.72 | 660.34 | 795.42 | 34 | 2000 |
| 14 | 21.72 | 660.34 | 625.31 | 34 | 2000 |
| 15 | 21.72 | 660.34 | 482.26 | 34 | 2000 |
| 16 | 17.56 | 493.24 | 741.36 | 27 | 2000 |
| 17 | 17.56 | 493.24 | 838.41 | 27 | 2000 |
| 18 | 17.56 | 493.24 | 628.28 | 27 | 2000 |
| 19 | 20.67 | 494.8 | 600.38 | 27 | 14000 |
| 20 | 20.67 | 494.8 | 671.42 | 27 | 14000 |
| 21 | 20.67 | 494.8 | 529.35 | 27 | 14000 |
| 22 | 1.19 | 428.7 | 584.29 | 24 | 2000 |
| 23 | 1.19 | 428.7 | 685.34 | 24 | 2000 |
| 24 | 1.19 | 428.7 | 483.24 | 24 | 2000 |
| 25 | 18.89 | 535.8 | 773.38 | 42 | 2000 |
| 26 | 18.89 | 535.8 | 644.34 | 42 | 2000 |
| 27 | 18.89 | 535.8 | 587.31 | 42 | 2000 |
| 28 | 17.37 | 599.35 | 870.48 | 42 | 2000 |
| 29 | 17.37 | 599.35 | 542.3 | 42 | 2000 |
| 30 | 17.37 | 599.35 | 655.39 | 42 | 2000 |
| 31 | 20 | 670.86 | 943.49 | 35 | 2000 |
| 32 | 20 | 670.86 | 472.25 | 35 | 2000 |
| 33 | 20 | 670.86 | 573.32 | 35 | 2000 |
| 34 | 20.48 | 532.28 | 660.37 | 28 | 2000 |
| 35 | 20.48 | 532.28 | 807.44 | 28 | 2000 |
| 36 | 20.48 | 532.28 | 561.3 | 28 | 2000 |
| 37 | 13.42 | 448.73 | 639.31 | 25 | 2000 |
| 38 | 13.42 | 448.73 | 696.33 | 25 | 2000 |
| 39 | 13.42 | 448.73 | 524.28 | 25 | 2000 |
| 40 | 17.6 | 713.89 | 884.52 | 36 | 2000 |
| 41 | 17.6 | 713.89 | 955.56 | 36 | 2000 |
| 42 | 17.6 | 713.89 | 1083.62 | 36 | 2000 |
| 43 | 21.1 | 785.9 | 515.26 | 40 | 2000 |
| 44 | 21.1 | 785.9 | 786.41 | 40 | 2000 |
| 45 | 21.1 | 785.9 | 900.45 | 40 | 2000 |

Les aires obtenues pour chacune des transitions et pour chacun des microorganismes étudiés ont été mesurées. Lorsque les aires des 3 transitions d'un même peptide sont supérieures ou égales au seuil de positivité décrit dans le **TABLEAU 8,** la détection du peptide est considérée comme positive et est notée « 1 » dans le **TABLEAU 9.** Lorsque au moins une transition comporte une aire inférieure au seuil de positivité décrit dans le **TABLEAU 8,** le peptide correspondant est considéré comme non détecté et est noté « 0 » dans le **TABLEAU 9.**

**TABLEAU 9 :**

| Numéro transition | Ech62 | Ech63 | Ech64 | Ech65 | Ech66 | Ech67 | Ech68 | Ech69 | Ech70 | Ech71 | Ech72 | Ech73 |
|---|---|---|---|---|---|---|---|---|---|---|---|---|
| 1 | 0 | 0 | 0 | 0 | 0 | 0 | 1 | 1 | 1 | 1 | 1 | 1 |
| 2 | 0 | 0 | 0 | 0 | 0 | 0 | 1 | 1 | 1 | 1 | 1 | 1 |
| 3 | 0 | 0 | 0 | 0 | 0 | 0 | 1 | 1 | 1 | 1 | 1 | 1 |
| 4 | 0 | 0 | 0 | 0 | 0 | 0 | 1 | 1 | 1 | 1 | 1 | 1 |
| 5 | 0 | 0 | 0 | 0 | 0 | 0 | 1 | 1 | 1 | 1 | 1 | 1 |
| 6 | 0 | 0 | 0 | 0 | 0 | 0 | 1 | 1 | 1 | 1 | 1 | 1 |
| 7 | 0 | 0 | 0 | 0 | 0 | 0 | 0 | 0 | 0 | 0 | 0 | 0 |
| 8 | 0 | 0 | 0 | 0 | 0 | 0 | 0 | 0 | 0 | 0 | 0 | 0 |
| 9 | 0 | 0 | 0 | 0 | 0 | 0 | 0 | 0 | 0 | 0 | 0 | 0 |
| 10 | 0 | 0 | 0 | 0 | 0 | 0 | 0 | 0 | 0 | 0 | 0 | 0 |
| 11 | 0 | 0 | 0 | 0 | 0 | 0 | 0 | 0 | 0 | 0 | 0 | 0 |
| 12 | 0 | 0 | 0 | 0 | 0 | 0 | 0 | 0 | 0 | 0 | 0 | 0 |
| 13 | 0 | 0 | 0 | 0 | 0 | 0 | 1 | 1 | 1 | 1 | 1 | 1 |
| 14 | 0 | 0 | 0 | 0 | 0 | 0 | 1 | 1 | 1 | 1 | 1 | 1 |
| 15 | 0 | 0 | 0 | 0 | 0 | 0 | 1 | 1 | 1 | 1 | 1 | 1 |
| 16 | 0 | 0 | 0 | 0 | 0 | 0 | 0 | 0 | 0 | 0 | 0 | 0 |
| 17 | 0 | 0 | 0 | 0 | 0 | 0 | 0 | 0 | 0 | 0 | 0 | 0 |
| 18 | 0 | 0 | 0 | 0 | 0 | 0 | 0 | 0 | 0 | 0 | 0 | 0 |
| 19 | 0 | 0 | 0 | 0 | 0 | 0 | 1 | 1 | 1 | 1 | 1 | 1 |
| 20 | 0 | 0 | 0 | 0 | 0 | 0 | 1 | 1 | 1 | 1 | 1 | 1 |
| 21 | 0 | 0 | 0 | 0 | 0 | 0 | 1 | 1 | 1 | 1 | 1 | 1 |
| 22 | 0 | 0 | 0 | 0 | 0 | 0 | 0 | 0 | 0 | 0 | 0 | 0 |
| 23 | 0 | 0 | 0 | 0 | 0 | 0 | 0 | 0 | 0 | 0 | 0 | 0 |
| 24 | 0 | 0 | 0 | 0 | 0 | 0 | 0 | 0 | 0 | 0 | 0 | 0 |
| 25 | 0 | 0 | 0 | 0 | 0 | 0 | 0 | 0 | 0 | 0 | 0 | 0 |
| 26 | 0 | 0 | 0 | 0 | 0 | 0 | 0 | 0 | 0 | 0 | 0 | 0 |
| 27 | 0 | 0 | 0 | 0 | 0 | 0 | 0 | 0 | 0 | 0 | 0 | 0 |
| 28 | 0 | 0 | 0 | 0 | 0 | 0 | 1 | 1 | 1 | 1 | 1 | 1 |
| 29 | 0 | 0 | 0 | 0 | 0 | 0 | 1 | 1 | 1 | 1 | 1 | 1 |
| 30 | 0 | 0 | 0 | 0 | 0 | 0 | 1 | 1 | 1 | 1 | 1 | 1 |
| 31 | 0 | 0 | 0 | 0 | 0 | 0 | 1 | 1 | 1 | 1 | 1 | 1 |
| 32 | 0 | 0 | 0 | 0 | 0 | 0 | 1 | 1 | 1 | 1 | 1 | 1 |
| 33 | 0 | 0 | 0 | 0 | 0 | 0 | 1 | 1 | 1 | 1 | 1 | 1 |
| 34 | 0 | 0 | 0 | 0 | 0 | 0 | 0 | 0 | 0 | 0 | 0 | 0 |
| 35 | 0 | 0 | 0 | 0 | 0 | 0 | 0 | 0 | 0 | 0 | 0 | 0 |
| 36 | 0 | 0 | 0 | 0 | 0 | 0 | 0 | 0 | 0 | 0 | 0 | 0 |
| 37 | 0 | 0 | 0 | 0 | 0 | 0 | 1 | 1 | 1 | 1 | 1 | 1 |
| 38 | 0 | 0 | 0 | 0 | 0 | 0 | 1 | 1 | 1 | 1 | 1 | 1 |
| 39 | 0 | 0 | 0 | 0 | 0 | 0 | 0 | 0 | 0 | 0 | 0 | 0 |
| 40 | 0 | 0 | 0 | 0 | 0 | 0 | 1 | 1 | 1 | 1 | 1 | 1 |
| 41 | 0 | 0 | 0 | 0 | 0 | 0 | 1 | 1 | 1 | 1 | 1 | 1 |
| 42 | 0 | 0 | 0 | 0 | 0 | 0 | 1 | 1 | 1 | 1 | 1 | 1 |
| 43 | 0 | 0 | 0 | 0 | 0 | 0 | 0 | 0 | 0 | 0 | 0 | 0 |
| 44 | 0 | 0 | 0 | 0 | 0 | 0 | 0 | 0 | 0 | 0 | 0 | 0 |
| 45 | 0 | 0 | 0 | 0 | 0 | 0 | 0 | 0 | 0 | 0 | 0 | 0 |
| Somme des transitions | 0 | 0 | 0 | 0 | 0 | 0 | 23 | 23 | 23 | 23 | 23 | 23 |

Les échantillons Ech68 à Ech73 comportent au moins une transition caractéristique des KPC . Les bactéries présentes dans les échantillons Ech68 à Ech73 expriment donc une beta-lactamase qui leur confère une résistance aux pénicillines, aux céphalosporines y compris celles de troisième génération telles que céfotaxime/ceftazidime, aux monobactames et aux carbapénèmes.

Les échantillons Ech62 à Ech67 ne comportent aucune transition caractéristique des KPC . Les bactéries présentes dans les échantillons Ech62 à Ech67 n'expriment donc pas de béta-lactamase de type KPC et peuvent être sensibles aux antibiotiques de type carbapénème.

### Exemple 8 : Identification d'une résistance aux béta-lactamines de type NDM-1 ou KPC:

Les échantillons correspondant à une espèce pouvant comporter un mécanisme de résistance de type NDM-1 ou KPC peuvent être détectés en mettant en oeuvre la méthode suivante.

Chaque échantillon est traité selon l'exemple 5, puis analysé selon l'exemple 6 en détectant les peptides du **TABLEAU 10** en lieu et place des peptides du **TABLEAU 3.**

**TABLEAU 10 :**

| Protéines | Transition numéro | Peptide | ion fragment de première génération | Etat de charge du précurseur | Intérêt clinique |
|---|---|---|---|---|---|
| NDM-1 | 1 | AAITHTAR | y4 monochargé | 2 | 3a |
| NDM-1 | 2 | AAITHTAR | y5 monochargé | 2 | 3a |
| NDM-1 | 3 | AAITHTAR | y6 monochargé | 2 | 3a |
| NDM-1 | 4 | AFGAAFPK | y6 monochargé | 2 | 3a |
| NDM-1 | 5 | AFGAAFPK | y7 monochargé | 2 | 3a |
| NDM-1 | 6 | AFGAAFPK | y7 dichargé | 2 | 3a |
| NDM-1 | 7 | FGDLVFR | y4 monochargé | 2 | 3a |
| NDM-1 | 8 | FGDLVFR | y5 monochargé | 2 | 3a |
| NDM-1 | 9 | FGDLVFR | y6 monochargé | 2 | 3a |
| NDM-1 | 10 | QEINLPVALAVVTHAHQDK | y14 dichargé | 3 | 3a |
| NDM-1 | 11 | QEINLPVALAVVTHAHQDK | y7 monochargé | 3 | 3a |
| NDM-1 | 12 | QEINLPVALAVVTHAHQDK | y8 monochargé | 3 | 3a |
| KPC | 13 | AAVPADWAVGDK | y9 dichargé | 2 | 2f |
| KPC | 14 | AAVPADWAVGDK | y10 dichargé | 2 | 2f |
| KPC | 15 | AAVPADWAVGDK | y9 monochargé | 2 | 2f |
| KPC | 16 | APIVLAVYTR | y7 monochargé | 2 | 2f |
| KPC | 17 | APIVLAVYTR | y5 monochargé | 2 | 2f |
| KPC | 18 | APIVLAVYTR | y6 monochargé | 2 | 2f |
| KPC | 19 | ELGGPAGLTAFMR | y7 monochargé | 2 | 2f |
| KPC | 20 | ELGGPAGL TAFMR | y5 monochargé | 2 | 2f |
| KPC | 21 | ELGGPAGL TAFMR | y9 dichargé | 2 | 2f |
| KPC | 22 | GFLAAAVLAR | y6 monochargé | 2 | 2f |
| KPC | 23 | GFLAAAVLAR | y7 monochargé | 2 | 2f |
| KPC | 24 | GFLAAAVLAR | y5 monochargé | 2 | 2f |
| KPC | 25 | LTLGSALAAPQR | y9 monochargé | 3 | 2f |
| KPC | 26 | LTLGSALAAPQR | y5 monochargé | 3 | 2f |
| KPC | 27 | LTLGSALAAPQR | y6 monochargé | 3 | 2f |
| KPC | 28 | NALVPWSPISEK | y8 monochargé | 2 | 2f |
| KPC | 29 | NALVPWSPISEK | y8 dichargé | 2 | 2f |
| KPC | 30 | NALVPWSPISEK | y5 monochargé | 2 | 2f |
| KPC | 31 | SQQQAGLLDTPIR | y8 monochargé | 2 | 2f |
| KPC | 32 | SQQQAGLLDTPIR | y9 monochargé | 2 | 2f |
| KPC | 33 | SQQQAGLLDTPIR | y10 monochargé | 2 | 2f |

La mention 2f indique la présence d'une béta-lactamase de type carbapénémase, du sous-groupe 2f selon la classification de Bush et Jacoby [Antimicrob Agents Chemother. 2010 Mar;54(3):969-76. Epub 2009 Dec 7. Updated functional classification of beta-lactamases.], capable d'hydrolyser les carbapénèmes.

La mention 3a indique la présence d'une métallo-beta-lactamase, du sous-groupe 3a selon la classification de Bush et Jacoby [9], *supra,* capable d'hydrolyser les pénicillines, les céphalosporines et les carbapénèmes.

Les transitions mentionnées dans le **TABLEAU 10** sont détectées en utilisant les paramètres figurant dans le **TABLEAU 11.**

**TABLEAU 11 :**

| Transition numéro | Temps de rétention (minutes) | (m/z) filtré en Q1 | (m/z) filtré en Q3 | Energie de collision (eV) | Seuil de positivité |
|---|---|---|---|---|---|
| 1 | 5.61 | 420.74 | 484.26 | 24 | 2500 |
| 2 | 5.61 | 420.74 | 585.31 | 24 | 2500 |
| 3 | 5.61 | 420.74 | 698.39 | 24 | 2500 |
| 4 | 16.03 | 404.72 | 590.33 | 23 | 2500 |
| 5 | 16.03 | 404.72 | 737.4 | 23 | 2500 |
| 6 | 16.03 | 404.72 | 369.2 | 23 | 2500 |
| 7 | 19.14 | 427.23 | 534.34 | 24 | 2500 |
| 8 | 19.14 | 427.23 | 649.37 | 24 | 2500 |
| 9 | 19.14 | 427.23 | 706.39 | 24 | 2500 |
| 10 | 21.34 | 695.05 | 743.41 | 39 | 2500 |
| 11 | 21.34 | 695.05 | 836.4 | 39 | 2500 |
| 12 | 21.34 | 695.05 | 935.47 | 39 | 2500 |
| 13 | 16.29 | 600.31 | 479.73 | 31 | 2000 |
| 14 | 16.29 | 600.31 | 529.27 | 31 | 2000 |
| 15 | 16.29 | 600.31 | 958.46 | 31 | 2000 |
| 16 | 19.07 | 551.83 | 821.49 | 29 | 13000 |
| 17 | 19.07 | 551.83 | 609.33 | 29 | 13000 |
| 18 | 19.07 | 551.83 | 722.42 | 29 | 13000 |
| 19 | 21.72 | 660.34 | 795.42 | 34 | 2000 |
| 20 | 21.72 | 660.34 | 625.31 | 34 | 2000 |
| 21 | 21.72 | 660.34 | 482.26 | 34 | 2000 |
| 22 | 20.67 | 494.8 | 600.38 | 27 | 14000 |
| 23 | 20.67 | 494.8 | 671.42 | 27 | 14000 |
| 24 | 20.67 | 494.8 | 529.35 | 27 | 14000 |
| 25 | 17.37 | 599.35 | 870.48 | 42 | 2000 |
| 26 | 17.37 | 599.35 | 542.3 | 42 | 2000 |
| 27 | 17.37 | 599.35 | 655.39 | 42 | 2000 |
| 28 | 20 | 670.86 | 943.49 | 35 | 2000 |
| 29 | 20 | 670.86 | 472.25 | 35 | 2000 |
| 30 | 20 | 670.86 | 573.32 | 35 | 2000 |
| 31 | 17.6 | 713.89 | 884.52 | 36 | 2000 |
| 32 | 17.6 | 713.89 | 955.56 | 36 | 2000 |
| 33 | 17.6 | 713.89 | 1083.62 | 36 | 2000 |

Lorsque les aires d'au moins deux transitions d'un même peptide sont supérieures ou égales au seuil de positivité décrit dans le **TABLEAU 11**, la détection du peptide est considérée comme positive. Lorsque plus de deux transitions d'un même peptide comportent une aire inférieure au seuil de positivité décrit dans le **TABLEAU 11**, le peptide correspondant est considéré comme non détecté.

Un échantillon contient des bactéries qui expriment la protéine NDM-1, lorsque qu'au moins un peptide, correspondant au mécanisme de résistance NDM-1, est détecté. Ces bactéries sont résistantes aux pénicillines, aux céphalosporines et aux carbapénèmes.

Un échantillon contient des bactéries qui expriment la protéine KPC, lorsque qu'au moins un peptide, correspondant au mécanisme de résistance KPC, est détecté. Ces bactéries sont résistantes aux pénicillines, aux céphalosporines y compris celles de troisième génération telles que céfotaxime/ceftazidime, aux monobactames et aux carbapénèmes.

### Exemple 9 : Identification d'une résistance aux béta-lactamines de type IND:

Les échantillons Ech84 à Ech88 sont identifiés selon l'une des méthodes décrites dans les exemples 1, 3 ou 4. L'identification des espèces est reportée dans le **TABLEAU 12.**

**TABLEAU 12 :**

| Noms | Espèces |
|---|---|
| Ech84 | *C. indologenes* |
| Ech85 | *C. indologenes* |
| Ech86 | *C. indologenes* |
| Ech87 | *C. indologenes* |
| Ech88 | *C. indologenes* |

Les échantillons Ech84 à Ech88 correspondent à une espèce pouvant comporter un mécanisme de résistance de type IND. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 13** en lieu et place des peptides du **TABLEAU 3.**

**TABLEAU 13 :**

| Transition numéro | Peptide | Temps de rétention (minutes) | (m/z) filtré en Q1 | (m/z) filtré en Q3 | Potentiel d'orifice (eV) | Energie de collision (eV) | Potentiel en sortie de cellule de collision (eV) | seuil de positivité |
|---|---|---|---|---|---|---|---|---|
| 1 | AATDLGYIK | 14,66 | 476,26 | 593,37 | 65,8 | 26 | 15 | 2000 |
| 2 | AATDLGYIK | 14,66 | 476,26 | 708,39 | 65,8 | 26 | 15 | 2000 |
| 3 | AATDLGYIK | 14,66 | 476,26 | 809,44 | 65,8 | 26 | 15 | 2000 |
| 4 | AGDLSFFNNK | 18,08 | 556,77 | 522,27 | 71,7 | 29,5 | 15 | 2000 |
| 5 | AGDLSFFNNK | 18,08 | 556,77 | 669,34 | 71,7 | 29,5 | 15 | 2000 |
| 6 | AGDLSFFNNK | 18,08 | 556,77 | 756,37 | 71,7 | 29,5 | 15 | 2000 |
| 7 | AGDLSFYNK | 14,82 | 507,75 | 424,22 | 68,1 | 27,3 | 15 | 2000 |
| 8 | AGDLSFYNK | 14,84 | 507,75 | 571,29 | 68,1 | 27,3 | 15 | 2000 |
| 9 | AGDLSFYNK | 14,84 | 507,75 | 658,32 | 68,1 | 27,3 | 15 | 2000 |
| 10 | AGDLSFYNQK | 14,91 | 571,78 | 552,28 | 72,8 | 30,2 | 15 | 2000 |
| 11 | AGDLSFYNQK | 14,93 | 571,78 | 699,35 | 72,8 | 30,2 | 15 | 2000 |
| 12 | AGDLSFYNQK | 14,93 | 571,78 | 786,38 | 72,8 | 30,2 | 15 | 2000 |
| 13 | AQYQSLMDTIK | 18,01 | 649,33 | 1098,55 | 78,5 | 33,6 | 15 | 2000 |
| 14 | AQYQSLMDTIK | 18,01 | 649,33 | 607,31 | 78,5 | 33,6 | 15 | 2000 |
| 15 | AQYQSLMDTIK | 18,01 | 649,33 | 807,43 | 78,5 | 33,6 | 15 | 2000 |
| 16 | ASLVIPGHDEWK | 16,84 | 676,35 | 434,70 | 80,4 | 34,8 | 15 | 2000 |
| 17 | ASLVIPGHDEWK | 16,8 | 676,35 | 868,40 | 80,4 | 34,8 | 15 | 2000 |
| 18 | ASLVIPGHDEWK | 16,82 | 676,35 | 981,48 | 80,4 | 34,8 | 15 | 2000 |
| 19 | ATLIIPGHDDWK | 17,47 | 683,36 | 427,69 | 80,9 | 35,1 | 15 | 2000 |
| 20 | ATLIIPGHDDWK | 17,47 | 683,36 | 854,38 | 80,9 | 35,1 | 15 | 2000 |
| 21 | ATLIIPGHDDWK | 17,47 | 683,36 | 967,46 | 80,9 | 35,1 | 15 | 2000 |
| 22 | ATLIIPGHDEWK | 17,54 | 690,37 | 1094,56 | 81,4 | 35,4 | 10 | 2000 |
| 23 | ATLIIPGHDEWK | 17,54 | 690,37 | 868,40 | 81,4 | 35,4 | 10 | 2000 |
| 24 | ATLIIPGHDEWK | 17,54 | 690,37 | 981,48 | 81,4 | 35,4 | 10 | 2000 |
| 25 | ATSTELIKPGK | 11,63 | 572,83 | 301,19 | 72,9 | 30,2 | 15 | 2000 |
| 26 | ATSTELIKPGK | 11,67 | 572,83 | 486,79 | 72,9 | 30,2 | 15 | 2000 |
| 27 | ATSTELIKPGK | 11,67 | 572,83 | 655,45 | 72,9 | 30,2 | 15 | 2000 |
| 28 | DFVIEPPIK | 19,93 | 529,30 | 454,30 | 69,7 | 28,3 | 15 | 2000 |
| 29 | DFVIEPPIK | 19,93 | 529,30 | 696,43 | 69,7 | 28,3 | 15 | 2000 |
| 30 | DFVIEPPIK | 19,93 | 529,30 | 795,50 | 69,7 | 28,3 | 15 | 2000 |
| 31 | DFVIEPPVKPNLYLYK | 22,03 | 645,69 | 730,91 | 78,2 | 36,3 | 15 | 2000 |
| 32 | DFVIEPPVKPNLYLYK | 22,03 | 645,69 | 787,45 | 78,2 | 36,3 | 15 | 2000 |
| 33 | DFVIEPPVKPNLYLYK | 22,08 | 645,69 | 836,99 | 78,2 | 36,3 | 15 | 2000 |
| 34 | DFVIEQPFGK | 19,77 | 590,31 | 448,26 | 74,2 | 31 | 15 | 2000 |
| 35 | DFVIEQPFGK | 19,75 | 590,31 | 705,36 | 74,2 | 31 | 15 | 2000 |
| 36 | DFVIEQPFGK | 19,75 | 590,31 | 818,44 | 74,2 | 31 | 15 | 2000 |
| 37 | EANLEQWPK | 15,53 | 557,78 | 430,25 | 71,8 | 29,5 | 15 | 2000 |
| 38 | EANLEQWPK | 15,55 | 557,78 | 558,30 | 71,8 | 29,5 | 15 | 2000 |
| 39 | EANLEQWPK | 15,55 | 557,78 | 687,35 | 71,8 | 29,5 | 15 | 2000 |
| 40 | EANVEQWPITIDK | 19,5 | 514,93 | 343,71 | 68,7 | 29,7 | 10 | 2000 |
| 41 | EANVEQWPITIDK | 19,5 | 514,93 | 686,41 | 68,7 | 29,7 | 10 | 2000 |
| 42 | EANVEQWPITIDK | 19,48 | 514,93 | 872,49 | 68,7 | 29,7 | 10 | 2000 |
| 43 | EANVEQWPK | 13,84 | 550,77 | 430,25 | 71,3 | 29,2 | 15 | 2000 |
| 44 | EANVEQWPK | 13,86 | 550,77 | 558,30 | 71,3 | 29,2 | 15 | 2000 |
| 45 | EANVEQWPK | 13,86 | 550,77 | 687,35 | 71,3 | 29,2 | 15 | 2000 |
| 46 | EQYQTLMDTIQK | 17,9 | 749,37 | 735,37 | 85,7 | 38 | 10 | 2000 |
| 47 | EQYQTLMDTIQK | 17,9 | 749,37 | 848,46 | 85,7 | 38 | 10 | 2000 |
| 48 | EQYQTLMDTIQK | 17,9 | 749,37 | 949,50 | 85,7 | 38 | 10 | 2000 |
| 49 | EYSANAVYLTTK | 15,26 | 680,34 | 1067,57 | 80,7 | 34,9 | 10 | 2000 |
| 50 | EYSANAVYLTTK | 15,28 | 680,34 | 625,36 | 80,7 | 34,9 | 10 | 2000 |
| 51 | EYSANAVYLTTK | 15,26 | 680,34 | 795,46 | 80,7 | 34,9 | 10 | 2000 |
| 52 | EYSANSMYLVTK | 16,5 | 703,34 | 1113,56 | 82,4 | 35,9 | 10 | 2000 |
| 53 | EYSANSMYLVTK | 16,5 | 703,34 | 841,45 | 82,4 | 35,9 | 10 | 2000 |
| 54 | EYSANSMYLVTK | 16,5 | 703,34 | 955,49 | 82,4 | 35,9 | 10 | 2000 |
| 55 | EYSANSVYLVTK | 16,19 | 687,35 | 1081,59 | 81,2 | 35,2 | 10 | 2000 |
| 56 | EYSANSVYLVTK | 16,14 | 687,35 | 623,38 | 81,2 | 35,2 | 10 | 2000 |
| 57 | EYSANSVYLVTK | 16,12 | 687,35 | 923,52 | 81,2 | 35,2 | 10 | 2000 |
| 58 | EYSANSVYLVTQK | 16,19 | 501,26 | 376,22 | 67,7 | 29,1 | 10 | 2000 |
| 59 | EYSANSVYLVTQK | 16,19 | 501,26 | 475,29 | 67,7 | 29,1 | 10 | 2000 |
| 60 | EYSANSVYLVTQK | 16,21 | 501,26 | 751,44 | 67,7 | 29,1 | 10 | 2000 |
| 61 | EYSTNALYLVTK | 18,83 | 701,37 | 1109,62 | 82,2 | 35,9 | 10 | 2000 |
| 62 | EYSTNALYLVTK | 18,83 | 701,37 | 460,31 | 82,2 | 35,9 | 10 | 2000 |
| 63 | EYSTNALYLVTK | 18,81 | 701,37 | 623,38 | 82,2 | 35,9 | 10 | 2000 |
| 64 | GGGHVEHTLELLDK | 15,6 | 502,26 | 730,44 | 67,7 | 29,1 | 10 | 2000 |
| 65 | GGGHVEHTLELLDK | 15,6 | 502,26 | 831,48 | 67,7 | 29,1 | 10 | 2000 |
| 66 | GGGHVEHTLELLDK | 15,6 | 502,26 | 968,54 | 67,7 | 29,1 | 10 | 2000 |
| 67 | GGGHVEHTLELLNK | 15 | 501,94 | 616,37 | 67,7 | 29,1 | 10 | 2000 |
| 68 | GGGHVEHTLELLNK | 15 | 501,94 | 729,45 | 67,7 | 29,1 | 10 | 2000 |
| 69 | GGGHVEHTLELLNK | 15 | 501,94 | 830,50 | 67,7 | 29,1 | 10 | 2000 |
| 70 | GGGHVQHTLDLLDK | 15,35 | 745,39 | 1082,58 | 85,5 | 37,8 | 10 | 2000 |
| 71 | GGGHVQHTLDLLDK | 15,35 | 745,39 | 1181,65 | 85,5 | 37,8 | 10 | 2000 |
| 72 | GGGHVQHTLDLLDK | 15,35 | 745,39 | 488,31 | 85,5 | 37,8 | 10 | 2000 |
| 73 | GIPTYATAK | 12,63 | 461,26 | 376,20 | 64,7 | 25,3 | 15 | 2000 |
| 74 | GIPTYATAK | 12,63 | 461,26 | 654,35 | 64,7 | 25,3 | 15 | 2000 |
| 75 | GIPTYATAK | 12,63 | 461,26 | 751,40 | 64,7 | 25,3 | 15 | 2000 |
| 76 | GNDHVK | 1,3 | 335,17 | 383,24 | 55,6 | 19,7 | 15 | 2000 |
| 77 | GNDHVK | 1,3 | 335,17 | 498,27 | 55,6 | 19,7 | 15 | 2000 |
| 78 | GNDHVK | 1,3 | 335,17 | 612,31 | 55,6 | 19,7 | 15 | 2000 |
| 79 | GVVLFDVPWEK | 23,79 | 644,86 | 559,29 | 78,1 | 33,4 | 15 | 2000 |
| 80 | GVVLFDVPWEK | 23,82 | 644,86 | 658,36 | 78,1 | 33,4 | 15 | 2000 |
| 81 | GVVLFDVPWEK | 23,82 | 644,86 | 920,45 | 78,1 | 33,4 | 15 | 2000 |
| 82 | GVVLFDVPWQK | 23,32 | 644,36 | 558,30 | 78,1 | 33,4 | 15 | 2000 |
| 83 | GVVLFDVPWQK | 23,32 | 644,36 | 772,40 | 78,1 | 33,4 | 15 | 2000 |
| 84 | GVVLFDVPWQK | 23,32 | 644,36 | 919,47 | 78,1 | 33,4 | 15 | 2000 |
| 85 | HNLPVIAVFATHSHSDR | 17,94 | 634,33 | 768,90 | 77,4 | 35,7 | 15 | 2000 |
| 86 | HNLPVIAVFATHSHSDR | 17,95 | 634,33 | 825,44 | 77,4 | 35,7 | 15 | 2000 |
| 87 | HNLPVIAVFATHSHSDR | 17,93 | 634,33 | 882,46 | 77,4 | 35,7 | 15 | 2000 |
| 88 | HNLPVVAVFATHSHDDR | 17,17 | 638,99 | 775,89 | 77,7 | 35,9 | 15 | 2000 |
| 89 | HNLPVVAVFATHSHDDR | 17,17 | 638,99 | 832,43 | 77,7 | 35,9 | 15 | 2000 |
| 90 | HNLPVVAVFATHSHDDR | 17,17 | 638,99 | 889,45 | 77,7 | 35,9 | 15 | 2000 |
| 91 | HTLELLDQQK | 15,02 | 612,83 | 403,23 | 75,8 | 32 | 15 | 2000 |
| 92 | HTLELLDQQK | 15,02 | 612,83 | 518,26 | 75,8 | 32 | 15 | 2000 |
| 93 | HTLELLDQQK | 15,02 | 612,83 | 986,55 | 75,8 | 32 | 15 | 2000 |
| 94 | HTLELLNK | 14,44 | 484,28 | 616,37 | 66,4 | 26,3 | 15 | 2000 |
| 95 | HTLELLNK | 14,44 | 484,28 | 729,45 | 66,4 | 26,3 | 15 | 2000 |
| 96 | HTLELLNK | 14,44 | 484,28 | 830,50 | 66,4 | 26,3 | 15 | 2000 |
| 97 | IQYQSLMDTIK | 19,41 | 670,34 | 1098,55 | 80 | 34,5 | 15 | 2000 |
| 98 | IQYQSLMDTIK | 19,38 | 670,34 | 607,31 | 80 | 34,5 | 15 | 2000 |
| 99 | IQYQSLMDTIK | 19,41 | 670,34 | 807,43 | 80 | 34,5 | 15 | 2000 |
| 100 | NLHIYK | 11,54 | 394,23 | 337,21 | 59,9 | 22,3 | 15 | 2000 |
| 101 | NLHIYK | 11,54 | 394,23 | 423,26 | 59,9 | 22,3 | 15 | 2000 |
| 102 | NLHIYK | 11,54 | 394,23 | 560,32 | 59,9 | 22,3 | 15 | 2000 |
| 103 | NLYIYK | 14,93 | 407,23 | 423,26 | 60,8 | 22,9 | 15 | 2000 |
| 104 | NLYIYK | 14,91 | 407,23 | 586,32 | 60,8 | 22,9 | 15 | 2000 |
| 105 | NLYIYK | 14,93 | 407,23 | 699,41 | 60,8 | 22,9 | 15 | 2000 |
| 106 | NNLHIYK | 11,29 | 451,25 | 423,26 | 64 | 24,9 | 15 | 2000 |
| 107 | NNLHIYK | 11,29 | 451,25 | 560,32 | 64 | 24,9 | 15 | 2000 |
| 108 | NNLHIYK | 11,27 | 451,25 | 673,40 | 64 | 24,9 | 15 | 2000 |
| 109 | QLYLYK | 15,22 | 414,24 | 423,26 | 61,3 | 23,2 | 15 | 2000 |
| 110 | QLYLYK | 15,2 | 414,24 | 586,32 | 61,3 | 23,2 | 15 | 2000 |
| 111 | QLYLYK | 15,22 | 414,24 | 699,41 | 61,3 | 23,2 | 15 | 2000 |
| 112 | QWPETMR | 14,84 | 474,22 | 317,16 | 65,7 | 25,9 | 15 | 2000 |
| 113 | QWPETMR | 14,75 | 474,22 | 407,21 | 65,7 | 25,9 | 15 | 2000 |
| 114 | QWPETMR | 14,73 | 474,22 | 633,30 | 65,7 | 25,9 | 15 | 2000 |
| 115 | SFGVFGGK | 16,69 | 399,71 | 356,20 | 60,3 | 22,6 | 15 | 2000 |
| 116 | SFGVFGGK | 16,69 | 399,71 | 408,22 | 60,3 | 22,6 | 15 | 2000 |
| 117 | SFGVFGGK | 16,69 | 399,71 | 564,31 | 60,3 | 22,6 | 15 | 2000 |
| 118 | SIQLLMMSMFLSPLINAQVK | 32,4 | 755,41 | 441,77 | 86,2 | 41,8 | 15 | 2000 |
| 119 | SIQLLMMSMFLSPLINAQVK | 32,4 | 755,41 | 882,54 | 86,2 | 41,8 | 15 | 2000 |
| 120 | SIQLLMMSMFLSPLINAQVK | 32,4 | 755,41 | 969,57 | 86,2 | 41,8 | 15 | 2000 |
| 121 | SNSATDLGYIK | 14,71 | 584,80 | 593,37 | 73,7 | 30,7 | 15 | 2000 |
| 122 | SNSATDLGYIK | 14,71 | 584,80 | 809,44 | 73,7 | 30,7 | 15 | 2000 |
| 123 | SNSATDLGYIK | 14,71 | 584,80 | 967,51 | 73,7 | 30,7 | 15 | 2000 |
| 124 | TATDLGYTGEANVK | 13,61 | 720,35 | 718,37 | 83,6 | 36,7 | 10 | 2000 |
| 125 | TATDLGYTGEANVK | 13,61 | 720,35 | 881,44 | 83,6 | 36,7 | 10 | 2000 |
| 126 | TATDLGYTGEANVK | 13,61 | 720,35 | 938,46 | 83,6 | 36,7 | 10 | 2000 |
| 127 | TFGVFDGK | 16,56 | 435,72 | 466,23 | 62,9 | 24,2 | 15 | 2000 |
| 128 | TFGVFDGK | 16,58 | 435,72 | 622,32 | 62,9 | 24,2 | 15 | 2000 |
| 129 | TFGVFDGK | 16,58 | 435,72 | 769,39 | 62,9 | 24,2 | 15 | 2000 |
| 130 | TFGVFGGK | 16,78 | 406,72 | 408,22 | 60,8 | 22,9 | 15 | 2000 |
| 131 | TFGVFGGK | 16,76 | 406,72 | 564,31 | 60,8 | 22,9 | 15 | 2000 |
| 132 | TFGVFGGK | 16,78 | 406,72 | 711,38 | 60,8 | 22,9 | 15 | 2000 |
| 133 | TGKPYK | 1,41 | 347,20 | 407,23 | 56,4 | 20,3 | 15 | 2000 |
| 134 | TGKPYK | 1,41 | 347,20 | 535,32 | 56,4 | 20,3 | 15 | 2000 |
| 135 | TGKPYK | 1,41 | 347,20 | 592,35 | 56,4 | 20,3 | 15 | 2000 |
| 136 | TGKPYR | 1,41 | 361,20 | 435,24 | 57,4 | 20,9 | 15 | 2000 |
| 137 | TGKPYR | 1,41 | 361,20 | 563,33 | 57,4 | 20,9 | 15 | 2000 |
| 138 | TGKPYR | 1,41 | 361,20 | 620,35 | 57,4 | 20,9 | 15 | 2000 |
| 139 | TGVVLFDVPWEK | 24,03 | 695,37 | 1033,54 | 81,8 | 35,6 | 10 | 2000 |
| 140 | TGVVLFDVPWEK | 23,97 | 695,37 | 559,29 | 81,8 | 35,6 | 10 | 2000 |
| 141 | TGVVLFDVPWEK | 23,97 | 695,37 | 920,45 | 81,8 | 35,6 | 10 | 2000 |
| 142 | TNEFLK | 12,85 | 376,20 | 407,27 | 58,5 | 21,6 | 15 | 2000 |
| 143 | TNEFLK | 12,88 | 376,20 | 536,31 | 58,5 | 21,6 | 15 | 2000 |
| 144 | TNEFLK | 12,85 | 376,20 | 650,35 | 58,5 | 21,6 | 15 | 2000 |
| 145 | TNELLK | 11,69 | 359,21 | 373,28 | 57,3 | 20,8 | 15 | 2000 |
| 146 | TNELLK | 11,72 | 359,21 | 502,32 | 57,3 | 20,8 | 15 | 2000 |
| 147 | TNELLK | 11,69 | 359,21 | 616,37 | 57,3 | 20,8 | 15 | 2000 |
| 148 | TNQFLK | 12,3 | 375,71 | 407,27 | 58,5 | 21,5 | 15 | 2000 |
| 149 | TNQFLK | 12,27 | 375,71 | 535,32 | 58,5 | 21,5 | 15 | 2000 |
| 150 | TNQFLK | 12,27 | 375,71 | 649,37 | 58,5 | 21,5 | 15 | 2000 |
| 151 | TQYQSLMDTIK | 18,12 | 664,33 | 1098,55 | 79,5 | 34,2 | 15 | 2000 |
| 152 | TQYQSLMDTIK | 18,1 | 664,33 | 607,31 | 79,5 | 34,2 | 15 | 2000 |
| 153 | TQYQSLMDTIK | 18,12 | 664,33 | 807,43 | 79,5 | 34,2 | 15 | 2000 |
| 154 | TYATAK | 1,9 | 327,68 | 319,20 | 55 | 19,4 | 15 | 2000 |
| 155 | TYATAK | 1,85 | 327,68 | 390,24 | 55 | 19,4 | 15 | 2000 |
| 156 | TYATAK | 1,9 | 327,68 | 553,30 | 55 | 19,4 | 15 | 2000 |
| 157 | TYATPK | 7,79 | 340,68 | 345,21 | 56 | 20 | 15 | 2000 |
| 158 | TYATPK | 7,77 | 340,68 | 416,25 | 56 | 20 | 15 | 2000 |
| 159 | TYATPK | 7,79 | 340,68 | 579,31 | 56 | 20 | 15 | 2000 |
| 160 | TYATSK | 1,45 | 335,67 | 335,19 | 55,6 | 19,8 | 15 | 2000 |
| 161 | TYATSK | 1,45 | 335,67 | 406,23 | 55,6 | 19,8 | 15 | 2000 |
| 162 | TYATSK | 1,47 | 335,67 | 569,29 | 55,6 | 19,8 | 15 | 2000 |
| 163 | VIPGHDEWK | 12,43 | 540,78 | 434,70 | 70,5 | 28,8 | 15 | 2000 |
| 164 | VIPGHDEWK | 12,45 | 540,78 | 771,34 | 70,5 | 28,8 | 15 | 2000 |
| 165 | VIPGHDEWK | 12,43 | 540,78 | 868,40 | 70,5 | 28,8 | 15 | 2000 |
| 166 | VLDGGCLVK | 14,44 | 480,76 | 633,34 | 66,2 | 26,2 | 15 | 2000 |
| 167 | VLDGGCLVK | 14,44 | 480,76 | 748,37 | 66,2 | 26,2 | 15 | 2000 |
| 168 | VLDGGCLVK | 14,46 | 480,76 | 861,45 | 66,2 | 26,2 | 15 | 2000 |
| 169 | VQYQSLMDTIQK | 18,24 | 727,37 | 1063,55 | 84,1 | 37 | 10 | 2000 |
| 170 | VQYQSLMDTIQK | 18,24 | 727,37 | 1226,61 | 84,1 | 37 | 10 | 2000 |
| 171 | VQYQSLMDTIQK | 18,24 | 727,37 | 935,49 | 84,1 | 37 | 10 | 2000 |
| 172 | YAQATLVIPGHDEWK | 18,03 | 576,63 | 577,26 | 73,2 | 32,8 | 10 | 2000 |
| 173 | YAQATLVIPGHDEWK | 18,03 | 576,63 | 747,39 | 73,2 | 32,8 | 10 | 2000 |
| 174 | YAQATLVIPGHDEWK | 18,05 | 576,63 | 868,40 | 73,2 | 32,8 | 10 | 2000 |
| 175 | YAQATLVIPGHEEWK | 17,99 | 581,30 | 690,37 | 73,5 | 33,1 | 10 | 2000 |
| 176 | YAQATLVIPGHEEWK | 17,95 | 581,30 | 754,40 | 73,5 | 33,1 | 10 | 2000 |
| 177 | YAQATLVIPGHEEWK | 17,97 | 581,30 | 882,41 | 73,5 | 33,1 | 10 | 2000 |
| 178 | YNVLDGGCLVK | 17,86 | 619,32 | 633,34 | 76,3 | 32,2 | 15 | 2000 |
| 179 | YNVLDGGCLVK | 17,86 | 619,32 | 748,37 | 76,3 | 32,2 | 15 | 2000 |
| 180 | YNVLDGGCLVK | 17,86 | 619,32 | 861,45 | 76,3 | 32,2 | 15 | 2000 |
| 181 | YPSTAK | 4,3 | 333,68 | 319,20 | 55,4 | 19,7 | 15 | 2000 |
| 182 | YPSTAK | 4,44 | 333,68 | 406,23 | 55,4 | 19,7 | 15 | 2000 |
| 183 | YPSTAK | 4,28 | 333,68 | 503,28 | 55,4 | 19,7 | 15 | 2000 |
| 184 | YSEAVLIIPGHDEWK | 19,76 | 586,30 | 753,90 | 73,9 | 33,3 | 15 | 2000 |
| 185 | YSEAVLIIPGHDEWK | 19,72 | 586,30 | 797,42 | 73,9 | 33,3 | 15 | 2000 |
| 186 | YSEAVLIIPGHDEWK | 19,72 | 586,30 | 868,40 | 73,9 | 33,3 | 15 | 2000 |

• Les autres paramètres machine utilisés sont les suivants :

| | |
|---|---|
| Type de balayage : | MRM |
| MRM planifié : | non |
| Polarité : | Positive |
| Source d'ionisation : | Turbo V™ (Applied BioSystems) |
| Réglage Q1 : | Filtrage avec résolution unitaire |
| Réglage Q3 : | Filtrage avec résolution unitaire |
| Pause inter-scan : | 5.00 msec |
| Vitesse de balayage : | 10 Da/s |
| Gaz rideau : | 40,00 psi |
| Tension de cône : | 5500,00 V |
| Température de source : | 500,00 °C |
| Gaz de nébulisation : | 50,00 psi |
| Gaz chauffant : | 50,00 psi |
| Gaz de collision induisant dissociation | : 9,00 psi |
| Remplissage dynamique : | activé |
| Potentiel d'entrée avant Q0 (EP) : | 10, 00V |

Les aires obtenues pour chacune des transitions et pour chacun des microorganismes étudiés ont été mesurées. Lorsque les aires des transitions sont supérieures ou égales au seuil de positivité décrit dans le **TABLEAU 13,** la détection de la transition est considérée comme positive et est notée « 1 » dans le **TABLEAU 14.** Lorsqu'une transition a une aire inférieure au seuil de positivité décrit dans le **TABLEAU 13,** la transition est considérée comme non détectée et est notée « 0 » dans le **TABLEAU 14.**

Pour un peptide donné, lorsque au moins 3 transitions sont notées « 1 », le peptide est considéré comme détecté.

**TABLEAU 14 :**

| Transition numéro | Ech84 | Ech85 | Ech86 | Ech87 | Ech88 |
|---|---|---|---|---|---|
| 1 | 0 | 1 | 1 | 1 | 1 |
| 2 | 0 | 0 | 1 | 1 | 0 |
| 3 | 0 | 0 | 0 | 1 | 0 |
| 4 | 0 | 0 | 0 | 0 | 0 |
| 5 | 0 | 0 | 0 | 0 | 0 |
| 6 | 0 | 0 | 0 | 0 | 0 |
| 7 | 0 | 0 | 0 | 0 | 0 |
| 8 | 0 | 0 | 0 | 1 | 0 |
| 9 | 0 | 0 | 0 | 0 | 0 |
| 10 | 0 | 1 | 1 | 0 | 1 |
| 11 | 0 | 0 | 0 | 0 | 0 |
| 12 | 0 | 0 | 1 | 0 | 0 |
| 13 | 0 | 0 | 0 | 0 | 0 |
| 14 | 0 | 0 | 0 | 0 | 0 |
| 15 | 0 | 0 | 0 | 0 | 0 |
| 16 | 0 | 0 | 0 | 1 | 0 |
| 17 | 0 | 0 | 0 | 1 | 0 |
| 18 | 0 | 1 | 0 | 1 | 1 |
| 19 | 0 | 0 | 0 | 0 | 0 |
| 20 | 0 | 0 | 0 | 0 | 0 |
| 21 | 1 | 1 | 1 | 1 | 0 |
| 22 | 0 | 0 | 0 | 0 | 0 |
| 23 | 0 | 0 | 0 | 0 | 0 |
| 24 | 0 | 0 | 0 | 0 | 0 |
| 25 | 1 | 1 | 1 | 1 | 1 |
| 26 | 1 | 1 | 0 | 1 | 1 |
| 27 | 1 | 1 | 0 | 1 | 1 |
| 28 | 0 | 1 | 0 | 1 | 1 |
| 29 | 0 | 1 | 0 | 1 | 1 |
| 30 | 0 | 1 | 0 | 1 | 1 |
| 31 | 0 | 0 | 0 | 0 | 0 |
| 32 | 0 | 0 | 0 | 0 | 0 |
| 33 | 0 | 0 | 0 | 0 | 0 |
| 34 | 0 | 0 | 0 | 0 | 0 |
| 35 | 0 | 0 | 0 | 0 | 0 |
| 36 | 0 | 0 | 0 | 0 | 0 |
| 37 | 0 | 0 | 0 | 0 | 0 |
| 38 | 0 | 0 | 0 | 0 | 0 |
| 39 | 0 | 0 | 0 | 0 | 0 |
| 40 | 0 | 0 | 0 | 0 | 0 |
| 41 | 0 | 0 | 0 | 0 | 0 |
| 42 | 0 | 0 | 0 | 0 | 0 |
| 43 | 0 | 0 | 0 | 0 | 0 |
| 44 | 0 | 0 | 0 | 0 | 0 |
| 45 | 0 | 0 | 0 | 0 | 0 |
| 46 | 0 | 0 | 0 | 0 | 0 |
| 47 | 0 | 0 | 0 | 0 | 0 |
| 48 | 0 | 0 | 0 | 0 | 0 |
| 49 | 0 | 0 | 0 | 0 | 0 |
| 50 | 0 | 0 | 0 | 0 | 0 |
| 51 | 0 | 0 | 0 | 0 | 0 |
| 52 | 0 | 0 | 0 | 1 | 0 |
| 53 | 0 | 0 | 0 | 1 | 0 |
| 54 | 0 | 0 | 0 | 1 | 0 |
| 55 | 0 | 0 | 0 | 0 | 0 |
| 56 | 0 | 0 | 0 | 0 | 0 |
| 57 | 0 | 0 | 0 | 0 | 0 |
| 58 | 0 | 0 | 0 | 0 | 0 |
| 59 | 0 | 0 | 0 | 0 | 0 |
| 60 | 0 | 0 | 0 | 0 | 0 |
| 61 | 0 | 0 | 0 | 0 | 0 |
| 62 | 0 | 0 | 0 | 0 | 0 |
| 63 | 0 | 0 | 0 | 0 | 0 |
| 64 | 0 | 0 | 0 | 0 | 0 |
| 65 | 0 | 0 | 0 | 0 | 0 |
| 66 | 0 | 0 | 0 | 0 | 0 |
| 67 | 0 | 0 | 0 | 0 | 0 |
| 68 | 0 | 0 | 0 | 0 | 0 |
| 69 | 0 | 0 | 0 | 0 | 0 |
| 70 | 0 | 0 | 0 | 0 | 0 |
| 71 | 0 | 0 | 0 | 0 | 0 |
| 72 | 0 | 0 | 0 | 0 | 0 |
| 73 | 0 | 0 | 0 | 0 | 0 |
| 74 | 1 | 0 | 0 | 0 | 0 |
| 75 | 0 | 0 | 0 | 0 | 0 |
| 76 | 0 | 0 | 0 | 0 | 0 |
| 77 | 0 | 0 | 0 | 0 | 0 |
| 78 | 1 | 0 | 0 | 0 | 0 |
| 79 | 0 | 0 | 0 | 0 | 0 |
| 80 | 0 | 0 | 0 | 0 | 0 |
| 81 | 0 | 0 | 0 | 0 | 0 |
| 82 | 1 | 0 | 0 | 1 | 0 |
| 83 | 0 | 0 | 0 | 0 | 0 |
| 84 | 0 | 0 | 0 | 0 | 0 |
| 85 | 0 | 0 | 0 | 0 | 0 |
| 86 | 0 | 0 | 0 | 0 | 0 |
| 87 | 0 | 0 | 0 | 0 | 0 |
| 88 | 0 | 0 | 0 | 0 | 0 |
| 89 | 0 | 0 | 0 | 0 | 0 |
| 90 | 0 | 0 | 0 | 0 | 0 |
| 91 | 0 | 0 | 0 | 0 | 0 |
| 92 | 0 | 0 | 0 | 0 | 0 |
| 93 | 0 | 0 | 0 | 0 | 0 |
| 94 | 0 | 0 | 0 | 0 | 0 |
| 95 | 0 | 0 | 0 | 0 | 0 |
| 96 | 0 | 0 | 0 | 0 | 0 |
| 97 | 0 | 0 | 0 | 0 | 0 |
| 98 | 0 | 0 | 0 | 0 | 0 |
| 99 | 0 | 0 | 0 | 0 | 0 |
| 100 | 0 | 0 | 0 | 0 | 0 |
| 101 | 0 | 0 | 0 | 0 | 0 |
| 102 | 0 | 0 | 0 | 0 | 0 |
| 103 | 0 | 0 | 0 | 0 | 0 |
| 104 | 0 | 0 | 0 | 0 | 0 |
| 105 | 0 | 0 | 0 | 0 | 0 |
| 106 | 0 | 0 | 0 | 0 | 0 |
| 107 | 0 | 0 | 0 | 0 | 0 |
| 108 | 0 | 0 | 0 | 0 | 0 |
| 109 | 0 | 0 | 0 | 0 | 0 |
| 110 | 0 | 0 | 0 | 0 | 0 |
| 111 | 0 | 0 | 0 | 0 | 0 |
| 112 | 0 | 0 | 0 | 0 | 0 |
| 113 | 0 | 0 | 0 | 0 | 0 |
| 114 | 0 | 0 | 0 | 0 | 0 |
| 115 | 0 | 0 | 0 | 0 | 0 |
| 116 | 0 | 0 | 0 | 0 | 0 |
| 117 | 0 | 0 | 0 | 0 | 0 |
| 118 | 0 | 0 | 0 | 0 | 0 |
| 119 | 0 | 0 | 0 | 0 | 0 |
| 120 | 0 | 0 | 0 | 0 | 0 |
| 121 | 0 | 0 | 0 | 0 | 0 |
| 122 | 0 | 0 | 0 | 0 | 0 |
| 123 | 0 | 0 | 0 | 0 | 0 |
| 124 | 0 | 0 | 0 | 0 | 0 |
| 125 | 0 | 0 | 0 | 0 | 0 |
| 126 | 0 | 0 | 0 | 0 | 0 |
| 127 | 0 | 0 | 0 | 0 | 0 |
| 128 | 0 | 0 | 0 | 0 | 0 |
| 129 | 0 | 0 | 0 | 0 | 0 |
| 130 | 0 | 0 | 0 | 0 | 0 |
| 131 | 0 | 0 | 0 | 0 | 0 |
| 132 | 0 | 0 | 0 | 0 | 0 |
| 133 | 0 | 1 | 0 | 0 | 1 |
| 134 | 0 | 1 | 0 | 0 | 1 |
| 135 | 0 | 1 | 0 | 0 | 1 |
| 136 | 1 | 0 | 0 | 0 | 0 |
| 137 | 1 | 0 | 0 | 1 | 0 |
| 138 | 1 | 0 | 0 | 0 | 1 |
| 139 | 0 | 0 | 0 | 0 | 0 |
| 140 | 0 | 0 | 0 | 0 | 0 |
| 141 | 0 | 0 | 0 | 0 | 0 |
| 142 | 0 | 0 | 0 | 0 | 0 |
| 143 | 0 | 0 | 0 | 0 | 0 |
| 144 | 0 | 0 | 0 | 0 | 0 |
| 145 | 1 | 0 | 0 | 0 | 0 |
| 146 | 1 | 0 | 0 | 1 | 0 |
| 147 | 1 | 0 | 0 | 0 | 0 |
| 148 | 0 | 0 | 0 | 0 | 0 |
| 149 | 0 | 0 | 0 | 0 | 0 |
| 150 | 0 | 0 | 0 | 0 | 0 |
| 151 | 0 | 0 | 0 | 0 | 0 |
| 152 | 0 | 0 | 0 | 0 | 0 |
| 153 | 0 | 0 | 0 | 0 | 0 |
| 154 | 0 | 0 | 0 | 0 | 0 |
| 155 | 0 | 0 | 0 | 0 | 0 |
| 156 | 0 | 0 | 0 | 0 | 0 |
| 157 | 0 | 0 | 0 | 0 | 0 |
| 158 | 0 | 0 | 0 | 0 | 0 |
| 159 | 0 | 0 | 0 | 0 | 0 |
| 160 | 0 | 0 | 0 | 0 | 0 |
| 161 | 0 | 0 | 0 | 0 | 0 |
| 162 | 0 | 0 | 0 | 0 | 0 |
| 163 | 0 | 0 | 0 | 0 | 0 |
| 164 | 0 | 0 | 0 | 0 | 0 |
| 165 | 0 | 0 | 0 | 0 | 0 |
| 166 | 0 | 0 | 0 | 0 | 0 |
| 167 | 0 | 0 | 0 | 0 | 0 |
| 168 | 0 | 0 | 0 | 0 | 0 |
| 169 | 0 | 0 | 0 | 0 | 0 |
| 170 | 0 | 0 | 0 | 0 | 0 |
| 171 | 0 | 0 | 0 | 0 | 0 |
| 172 | 0 | 0 | 0 | 0 | 0 |
| 173 | 0 | 0 | 0 | 0 | 0 |
| 174 | 0 | 0 | 0 | 0 | 0 |
| 175 | 0 | 1 | 0 | 1 | 1 |
| 176 | 0 | 1 | 0 | 1 | 1 |
| 177 | 1 | 1 | 0 | 1 | 1 |
| 178 | 0 | 0 | 0 | 0 | 0 |
| 179 | 0 | 0 | 0 | 0 | 0 |
| 180 | 0 | 0 | 0 | 0 | 0 |
| 181 | 0 | 0 | 0 | 0 | 0 |
| 182 | 0 | 0 | 0 | 0 | 0 |
| 183 | 0 | 0 | 0 | 0 | 0 |
| 184 | 0 | 0 | 0 | 0 | 0 |
| 185 | 0 | 0 | 0 | 0 | 0 |
| 186 | 0 | 0 | 0 | 0 | 0 |
| 187 | 0 | 0 | 0 | 1 | 0 |
| 188 | 0 | 0 | 0 | 1 | 0 |
| 189 | 0 | 1 | 0 | 1 | 0 |
| 190 | 0 | 0 | 0 | 0 | 0 |
| 191 | 0 | 0 | 0 | 0 | 0 |
| 192 | 0 | 0 | 0 | 0 | 0 |
| 193 | 0 | 0 | 0 | 0 | 0 |
| 194 | 0 | 0 | 0 | 0 | 0 |
| 195 | 0 | 0 | 0 | 0 | 0 |

Les échantillons Ech84 à Ech88 comportent au moins un peptide caractéristique des IND. Les bactéries présentes dans les échantillons Ech84 à Ech88 expriment donc une beta-lactamase qui leur confère une résistance aux pénicillines, aux céphalosporines et aux carbapénèmes.

### Exemple 10 : Identification d'une résistance aux béta-lactamines de type GES:

Les échantillons, Ech89 et Ech90, sont identifiés selon l'une des méthodes décrites dans les exemples 1, 3 ou 4. L'identification des espèces est reportée dans le **TABLEAU 15.**

**TABLEAU 15 :**

| Noms | Espèces |
|---|---|
| Ech89 | *E. coli* |
| Ech90 | *P. aeruginosa* |

Les échantillons Ech89 et Ech90 correspondent à une espèce pouvant comporter un mécanisme de résistance de type GES. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 16** en lieu et place des peptides du **TABLEAU 3.**

**TABLEAU 16 :**

| Transition numéro | Peptide | Temps de rétention (minutes) | (m/z) filtré en Q1 | (m/z) filtré en Q3 | Potentiel d'orifice (eV) | Energie de collision (eV) |
|---|---|---|---|---|---|---|
| 1 | AAEIGVAIVDPQGEIVAGHR | 19,11 | 668,03 | 695,88 | 79,8 | 37,4 |
| 2 | AAEIGVAIVDPQGEIVAGHR | 19,13 | 668,03 | 731,39 | 79,8 | 37,4 |
| 3 | AAEIGVAIVDPQGEIVAGHR | 19,11 | 668,03 | 809,44 | 79,8 | 37,4 |
| 4 | AAQIGVAIVDPQGEIVAGHR | 18,76 | 667,70 | 695,88 | 79,8 | 37,4 |
| 5 | AAQIGVAIVDPQGEIVAGHR | 18,76 | 667,70 | 731,39 | 79,8 | 37,4 |
| 6 | AAQIGVAIVDPQGEIVAGHR | 18,76 | 667,70 | 809,44 | 79,8 | 37,4 |
| 7 | DTTTPIAMAR | 14,23 | 538,77 | 658,37 | 70,4 | 28,7 |
| 8 | DTTTPIAMAR | 14,23 | 538,77 | 759,42 | 70,4 | 28,7 |
| 9 | DTTTPIAMAR | 14,23 | 538,77 | 860,47 | 70,4 | 28,7 |
| 10 | DWVVGEK | 14,41 | 416,71 | 432,25 | 61,5 | 23,3 |
| 11 | DWVVGEK | 14,43 | 416,71 | 531,31 | 61,5 | 23,3 |
| 12 | DWVVGEK | 14,45 | 416,71 | 717,39 | 61,5 | 23,3 |
| 13 | DYAVAVYTTAPK | 15,83 | 649,84 | 680,36 | 78,5 | 33,6 |
| 14 | DYAVAVYTTAPK | 15,83 | 649,84 | 779,43 | 78,5 | 33,6 |
| 15 | DYAVAVYTTAPK | 15,85 | 649,84 | 850,4 7 | 78,5 | 33,6 |
| 16 | EIGGPAAMTQYFR | 20,03 | 720,85 | 1198,5 7 | 83,7 | 36,7 |
| 17 | EIGGPAAMTQYFR | 20,03 | 720,85 | 845,40 | 83,7 | 36,7 |
| 18 | EIGGPAAMTQYFR | 20,03 | 720,85 | 916,44 | 83,7 | 36,7 |
| 19 | EPEMGDNTPGDLR | 13,53 | 715,81 | 557,30 | 83,3 | 36,5 |
| 20 | EPEMGDNTPGDLR | 13,53 | 715,81 | 772,40 | 83,3 | 36,5 |
| 21 | EPEMGDNTPGDLR | 13,53 | 715,81 | 944,44 | 83,3 | 36,5 |
| 22 | ESEMSDNTPGDLR | 12,72 | 725,81 | 557,30 | 84 | 36,9 |
| 23 | ESEMSDNTPGDLR | 12,7 | 725,81 | 887,42 | 84 | 36,9 |
| 24 | ESEMSDNTPGDLR | 12,71 | 725,81 | 974,45 | 84 | 36,9 |
| 25 | FAMCSTFK | 16,14 | 496,22 | 642,29 | 67,3 | 26,8 |
| 26 | FAMCSTFK | 16,12 | 496,22 | 773,33 | 67,3 | 26,8 |
| 27 | FAMCSTFK | 16,12 | 496,22 | 844,37 | 67,3 | 26,8 |
| 28 | FIHALLLAGIAHSAYASEK | 20,93 | 671,37 | 1204,60 | 80,1 | 37,6 |
| 29 | FIHALLLAGIAHSAYASEK | 20,92 | 671,37 | 807,95 | 80,1 | 37,6 |
| 30 | FIHALLLAGIAHSAYASEK | 20,93 | 671,37 | 876,48 | 80,1 | 37,6 |
| 31 | FIHALLLAGTAHSAYASEK | 18,21 | 667,36 | 766,41 | 79,8 | 37,4 |
| 32 | FIHALLLAGTAHSAYASEK | 18,21 | 667,36 | 801,93 | 79,8 | 37,4 |
| 33 | FIHALLLAGTAHSAYASEK | 18,21 | 667,36 | 870,46 | 79,8 | 37,4 |
| 34 | FPLAALVFER | 24,46 | 581,84 | 734,42 | 73,5 | 30,6 |
| 35 | FPLAALVFER | 24,46 | 581,84 | 805,46 | 73,5 | 30,6 |
| 36 | FPLAALVFER | 24,44 | 581,84 | 918,54 | 73,5 | 30,6 |
| 37 | IDSGTER | 1,66 | 389,19 | 462,23 | 59,5 | 22,1 |
| 38 | IDSGTER | 1,84 | 389,19 | 549,26 | 59,5 | 22,1 |
| 39 | IDSGTER | 1,75 | 389,19 | 664,29 | 59,5 | 22,1 |
| 40 | IGDSVSR | 8,48 | 367,20 | 448,25 | 57,9 | 21,2 |
| 41 | IGDSVSR | 8,46 | 367,20 | 563,28 | 57,9 | 21,2 |
| 42 | IGDSVSR | 8,44 | 367,20 | 620,30 | 57,9 | 21,2 |
| 43 | LSAVER | 9,1 | 337,70 | 403,23 | 55,7 | 19,9 |
| 44 | LSAVER | 9,08 | 337,70 | 474,27 | 55,7 | 19,9 |
| 45 | LSAVER | 9,1 | 337,70 | 561,30 | 55,7 | 19,9 |
| 46 | LSYGPDMIVEWSPATER | 22,31 | 650,98 | 573,30 | 78,6 | 36,5 |
| 47 | LSYGPDMIVEWSPATER | 22,29 | 650,98 | 660,33 | 78,6 | 36,5 |
| 48 | LSYGPDMIVEWSPATER | 22,26 | 650,98 | 846,41 | 78,6 | 36,5 |
| 49 | LSYGPDMIVK | 17,71 | 561,80 | 1009,5 0 | 72,1 | 29,7 |
| 50 | LSYGPDMIVK | 17,69 | 561,80 | 759,41 | 72,1 | 29,7 |
| 51 | LSYGPDMIVK | 17,69 | 561,80 | 922,47 | 72,1 | 29,7 |
| 52 | NDIGFFK | 17,71 | 420,72 | 498,27 | 61,8 | 23,5 |
| 53 | NDIGFFK | 17,69 | 420,72 | 611,36 | 61,8 | 23,5 |
| 54 | NDIGFFK | 17,74 | 420,72 | 726,38 | 61,8 | 23,5 |
| 55 | TDLEK | 3,66 | 303,16 | 389,24 | 53,2 | 18,3 |
| 56 | TDLEK | 3,73 | 303,16 | 459,21 | 53,2 | 18,3 |
| 57 | TDLEK | 3,6 | 303,16 | 504,27 | 53,2 | 18,3 |
| 58 | TGACANGAR | 1,48 | 439,20 | 648,29 | 63,1 | 24,3 |
| 59 | TGACANGAR | 1,48 | 439,20 | 719,33 | 63,1 | 24,3 |
| 60 | TGACANGAR | 1,48 | 439,20 | 776,35 | 63,1 | 24,3 |
| 61 | TGTCANGAR | 1,48 | 454,21 | 648,29 | 64,2 | 25 |
| 62 | TGTCANGAR | 1,48 | 454,21 | 749,34 | 64,2 | 25 |
| 63 | TGTCANGAR | 1,48 | 454,21 | 806,36 | 64,2 | 25 |
| 64 | TGTCANGGR | 1,48 | 447,20 | 474,24 | 63,7 | 24,7 |
| 65 | TGTCANGGR | 1,48 | 447,20 | 634,27 | 63,7 | 24,7 |
| 66 | TGTCANGGR | 1,48 | 447,20 | 735,32 | 63,7 | 24,7 |
| 67 | VLYGGALTSTSTHTIER | 15,87 | 602,65 | 1245,6 4 | 75,1 | 34,1 |
| 68 | VLYGGALTSTSTHTIER | 15,85 | 602,65 | 715,87 | 75,1 | 34,1 |
| 69 | VLYGGALTSTSTHTIER | 15,87 | 602,65 | 797,40 | 75,1 | 34,1 |
| 70 | WLIGNQTGDATLR | 18,93 | 722,88 | 1032,5 1 | 83,8 | 36,8 |
| 71 | WLIGNQTGDATLR | 19,02 | 722,88 | 1145,5 9 | 83,8 | 36,8 |
| 72 | WLIGNQTGDATLR | 18,96 | 722,88 | 733,38 | 83,8 | 36,8 |
| 73 | WSPATER | 11,37 | 423,71 | 476,25 | 62 | 23,6 |
| 74 | WSPATER | 11,37 | 423,71 | 573,30 | 62 | 23,6 |
| 75 | WSPATER | 11,34 | 423,71 | 660,33 | 62 | 23,6 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 40,00 psi |
| Tension de cône: | 5500,00 V |
| Température de source: | 500,00 °C |
| Gaz de nébulisation: | 50,00 psi |
| Gaz chauffant: | 50,00 psi |
| Gaz de collision induisant dissociation: | 9,00 psi |
| Remplissage dynamique: | activé |
| Potentiel d'entrée avant Q0 (EP) : | 10, 00V |
| Potentiel en sortie de cellule de collision (CXP) : | 15, 00V |

Les aires obtenues pour chacune des transitions et pour chacun des microorganismes étudiés ont été mesurées. Lorsque les aires des transitions sont supérieures ou égales au seuil de positivité décrit dans le **TABLEAU 16,** la détection de la transition est considérée comme positive et est notée « 1 » dans le **TABLEAU 17.** Lorsqu'une transition a une aire inférieure au seuil de positivité décrit dans le **TABLEAU 16,** la transition est considérée comme non détectée et est notée « 0 » dans le **TABLEAU 17**.

Pour un peptide donné, lorsque au moins 3 transitions sont notées « 1 », le peptide est considéré comme détecté.

**TABLEAU 17 :**

| Transition numéro | Ech89 | Ech90 |
|---|---|---|
| 1 | 0 | 0 |
| 2 | 0 | 0 |
| 3 | 0 | 0 |
| 4 | 0 | 0 |
| 5 | 1 | 1 |
| 6 | 0 | 0 |
| 7 | 1 | 1 |
| 8 | 1 | 1 |
| 9 | 1 | 1 |
| 10 | 1 | 1 |
| 11 | 1 | 1 |
| 12 | 1 | 1 |
| 13 | 0 | 0 |
| 14 | 0 | 0 |
| 15 | 0 | 0 |
| 16 | 0 | 0 |
| 17 | 0 | 0 |
| 18 | 0 | 0 |
| 19 | 0 | 0 |
| 20 | 0 | 0 |
| 21 | 0 | 0 |
| 22 | 0 | 0 |
| 23 | 0 | 0 |
| 24 | 0 | 0 |
| 25 | 0 | 0 |
| 26 | 0 | 0 |
| 27 | 0 | 0 |
| 28 | 0 | 0 |
| 29 | 0 | 0 |
| 30 | 0 | 0 |
| 31 | 0 | 0 |
| 32 | 0 | 0 |
| 33 | 0 | 0 |
| 34 | 0 | 0 |
| 35 | 0 | 0 |
| 36 | 0 | 0 |
| 37 | 1 | 1 |
| 38 | 1 | 1 |
| 39 | 1 | 1 |
| 40 | 1 | 1 |
| 41 | 1 | 1 |
| 42 | 1 | 1 |
| 43 | 1 | 1 |
| 44 | 1 | 1 |
| 45 | 1 | 1 |
| 46 | 1 | 1 |
| 47 | 1 | 1 |
| 48 | 1 | 1 |
| 49 | 0 | 0 |
| 50 | 0 | 0 |
| 51 | 0 | 0 |
| 52 | 1 | 1 |
| 53 | 1 | 1 |
| 54 | 1 | 1 |
| 55 | 0 | 0 |
| 56 | 0 | 0 |
| 57 | 0 | 0 |
| 58 | 0 | 0 |
| 59 | 0 | 0 |
| 60 | 0 | 0 |
| 61 | 0 | 0 |
| 62 | 0 | 0 |
| 63 | 0 | 0 |
| 64 | 1 | 0 |
| 65 | 0 | 1 |
| 66 | 1 | 1 |
| 67 | 1 | 1 |
| 68 | 1 | 1 |
| 69 | 1 | 1 |
| 70 | 1 | 1 |
| 71 | 1 | 1 |
| 72 | 1 | 1 |
| 73 | 0 | 0 |
| 74 | 0 | 0 |
| 75 | 0 | 0 |

Les échantillons Ech89 et Ech90 comportent au moins un peptide caractéristique du phénotype carbapénémase. Les bactéries présentes dans les échantillons Ech89 et Ech90 expriment donc une béta-lactamase qui leur confère une résistance aux pénicillines, aux céphalosporines et aux carbapénèmes.

### Exemple 11 : Identification d'une résistance aux béta-lactamines de type SME:

Les échantillons Ech91 à Ech95 sont identifiés selon l'une des méthodes décrites dans les exemples 1, 3 ou 4. L'identification des espèces est reportée dans le **TABLEAU 18.**

**TABLEAU 18 :**

| Noms | Espèces |
|---|---|
| Ech91 | *S. marcescens* |
| Ech92 | *S. marcescens* |
| Ech93 | *S. marcescens* |
| Ech94 | *S. marcescens* |
| Ech95 | *S. marcescens* |

Les échantillons Ech91 à Ech95 correspondent à une espèce pouvant comporter un mécanisme de résistance de type SME. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 19** en lieu et place des peptides du **TABLEAU 3.**

**TABLEAU 19 :**

| Transition numéro | Peptide | Temps de rétention (minutes) | (m/z) filtré en Q1 | (m/z) filtré en Q3 | Energie de collision (eV) | seuil de positivité |
|---|---|---|---|---|---|---|
| 1 | AIYQNWLK | 18,69 | 518,29 | 426,22 | 25,3 | 2500 |
| 2 | AIYQNWLK | 18,69 | 518,29 | 688,38 | 25,3 | 2500 |
| 3 | AIYQNWLK | 18,69 | 518,29 | 851,44 | 25,3 | 2500 |
| 4 | APLIVSIYTTR | 20,21 | 617,36 | 740,39 | 30,9 | 2500 |
| 5 | APLIVSIYTTR | 20,21 | 617,36 | 839,46 | 30,9 | 2500 |
| 6 | APLIVSIYTTR | 20,21 | 617,36 | 952,55 | 30,9 | 2500 |
| 7 | ASVPADWVVGDK | 17,56 | 622,32 | 493,75 | 31,2 | 2500 |
| 8 | ASVPADWVVGDK | 17,56 | 622,32 | 543,29 | 31,2 | 2500 |
| 9 | ASVPADWVVGDK | 17,56 | 622,32 | 986,49 | 31,2 | 2500 |
| 10 | AVANSLNK | 8,75 | 408,73 | 461,27 | 19 | 2500 |
| 11 | AVANSLNK | 8,75 | 408,73 | 575,32 | 19 | 2500 |
| 12 | AVANSLNK | 8,75 | 408,73 | 646,35 | 19 | 2500 |
| 13 | DLEYHSPITTK | 14,48 | 435,22 | 473,75 | 20,6 | 2500 |
| 14 | DLEYHSPITTK | 14,48 | 435,22 | 538,28 | 20,6 | 2500 |
| 15 | DLEYHSPITTK | 14,48 | 435,22 | 646,38 | 20,6 | 2500 |
| 16 | DLEYYSPITTK | 17,4 | 665,33 | 559,35 | 33,7 | 2500 |
| 17 | DLEYYSPITTK | 17,4 | 665,33 | 646,38 | 33,7 | 2500 |
| 18 | DLEYYSPITTK | 17,4 | 665,33 | 809,44 | 33,7 | 2500 |
| 19 | DTSTPK | 1,45 | 324,66 | 345,21 | 14,2 | 2500 |
| 20 | DTSTPK | 1,45 | 324,66 | 432,25 | 14,2 | 2500 |
| 21 | DTSTPK | 1,45 | 324,66 | 533,29 | 14,2 | 2500 |
| 22 | FLGGPEGMTK | 14,94 | 518,76 | 662,32 | 25,3 | 2500 |
| 23 | FLGGPEGMTK | 14,94 | 518,76 | 719,34 | 25,3 | 2500 |
| 24 | FLGGPEGMTK | 14,94 | 518,76 | 776,36 | 25,3 | 2500 |
| 25 | GFLAAAVLER | 20,49 | 523,80 | 587,35 | 25,6 | 2500 |
| 26 | GFLAAAVLER | 20,49 | 523,80 | 658,39 | 25,6 | 2500 |
| 27 | GFLAAAVLER | 20,49 | 523,80 | 729,43 | 25,6 | 2500 |
| 28 | GNTTGDAR | 6,45 | 396,19 | 418,20 | 18,3 | 2500 |
| 29 | GNTTGDAR | 6,45 | 396,19 | 519,25 | 18,3 | 2500 |
| 30 | GNTTGDAR | 6,45 | 396,19 | 620,30 | 18,3 | 2500 |
| 31 | IGVFAIDTGSGNTFGYR | 21,45 | 592,30 | 542,27 | 25,4 | 2500 |
| 32 | IGVFAIDTGSGNTFGYR | 21,45 | 887,94 | 1174,51 | 46,3 | 2500 |
| 33 | IGVFAIDTGSGNTFGYR | 21,45 | 887,94 | 958,44 | 46,3 | 2500 |
| 34 | LALGNVLNAK | 18,56 | 506,81 | 414,75 | 24,6 | 2500 |
| 35 | LALGNVLNAK | 18,56 | 506,81 | 715,41 | 24,6 | 2500 |
| 36 | LALGNVLNAK | 18,56 | 506,81 | 828,49 | 24,6 | 2500 |
| 37 | LDINQK | 10,3 | 365,71 | 389,21 | 16,6 | 2500 |
| 38 | LDINQK | 10,3 | 365,71 | 502,30 | 16,6 | 2500 |
| 39 | LDINQK | 10,3 | 365,71 | 617,33 | 16,6 | 2500 |
| 40 | LEEDFDGR | 12,51 | 490,72 | 609,26 | 23,7 | 2500 |
| 41 | LEEDFDGR | 12,51 | 490,72 | 738,31 | 23,7 | 2500 |
| 42 | LEEDFDGR | 12,51 | 490,72 | 867,35 | 23,7 | 2500 |
| 43 | SDAAAK | 7,06 | 281,65 | 289,19 | 11,8 | 2500 |
| 44 | SDAAAK | 7,06 | 281,65 | 360,22 | 11,8 | 2500 |
| 45 | SDAAAK | 7,06 | 281,65 | 475,25 | 11,8 | 2500 |
| 46 | SIGDNEFR | 12,81 | 469,22 | 565,27 | 22,5 | 2500 |
| 47 | SIGDNEFR | 12,81 | 469,22 | 680,30 | 22,5 | 2500 |
| 48 | SIGDNEFR | 12,81 | 469,22 | 737,32 | 22,5 | 2500 |
| 49 | TGSCGAIGTANDYAVIWPK | 20,29 | 660,99 | 430,25 | 27,6 | 2500 |
| 50 | TGSCGAIGTANDYAVIWPK | 20,29 | 660,99 | 713,43 | 27,6 | 2500 |
| 51 | TGSCGAIGTANDYAVIWPK | 20,29 | 990,98 | 430,25 | 52,2 | 2500 |
| 52 | TGSCGAYGTANDYAVIWPK | 19,78 | 1015,97 | 430,25 | 53,6 | 2500 |
| 53 | TGSCGAYGTANDYAVIWPK | 19,78 | 677,65 | 642,40 | 28,1 | 2500 |
| 54 | TGSCGAYGTANDYAVIWPK | 19,78 | 677,65 | 713,43 | 28,1 | 2500 |
| 55 | TIAEASR | 6,98 | 374,20 | 333,19 | 17,1 | 2500 |
| 56 | TIAEASR | 6,98 | 374,20 | 462,23 | 17,1 | 2500 |
| 57 | TIAEASR | 6,98 | 374,20 | 646,35 | 17,1 | 2500 |
| 58 | WELELNTAIPGDK | 21,06 | 495,92 | 416,21 | 22,5 | 2500 |
| 59 | WELELNTAIPGDK | 21,06 | 743,38 | 1170,64 | 38,1 | 2500 |
| 60 | WELELNTAIPGDK | 21,06 | 743,38 | 416,21 | 38,1 | 2500 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 40,00 psi |
| Tension de cône: | 5500,00 V |
| Température de source: | 500,00 °C |
| Gaz de nébulisation: | 50,00 psi |
| Gaz chauffant: | 50,00 psi |
| Gaz de collision induisant dissociation : | 9,00 psi |
| Remplissage dynamique: | activé |
| Potentiel d'orifice (DP) : | 100,00 V |
| Potentiel d'entrée avant Q0 (EP) : | 10,00V |
| Potentiel en sortie de cellule de collision (CXP) : 1 | 5,00V |

Les aires obtenues pour chacune des transitions et pour chacun des microorganismes étudiés ont été mesurées. Lorsque les aires des transitions sont supérieures ou égales au seuil de positivité décrit dans le **TABLEAU 19,** la détection de la transition est considérée comme positive et est notée « 1 » dans le **TABLEAU 20.** Lorsqu'une transition a une aire inférieure au seuil de positivité décrit dans le **TABLEAU 19,** la transition est considérée comme non détectée et est notée « 0 » dans le **TABLEAU 20.**

Pour un peptide donné, lorsque au moins 3 transitions sont notées « 1 », le peptide est considéré comme détecté.

**TABLEAU 20 :**

| Transition numéro | Ech91 | Ech92 | Ech93 | Ech94 | Ech95 |
|---|---|---|---|---|---|
| 1 | 0 | 1 | 0 | 0 | 0 |
| 2 | 1 | 1 | 0 | 0 | 1 |
| 3 | 0 | 1 | 0 | 0 | 0 |
| 4 | 0 | 0 | 0 | 0 | 0 |
| 5 | 0 | 0 | 0 | 0 | 0 |
| 6 | 0 | 0 | 0 | 0 | 0 |
| 7 | 1 | 1 | 1 | 1 | 1 |
| 8 | 1 | 1 | 1 | 1 | 1 |
| 9 | 1 | 1 | 1 | 1 | 1 |
| 10 | 1 | 1 | 1 | 1 | 1 |
| 11 | 1 | 1 | 1 | 1 | 1 |
| 12 | 1 | 1 | 1 | 1 | 1 |
| 13 | 1 | 1 | 1 | 1 | 1 |
| 14 | 1 | 1 | 1 | 1 | 1 |
| 15 | 1 | 1 | 1 | 1 | 1 |
| 16 | 0 | 0 | 0 | 0 | 0 |
| 17 | 0 | 0 | 0 | 0 | 0 |
| 18 | 0 | 0 | 0 | 0 | 0 |
| 19 | 0 | 0 | 0 | 0 | 0 |
| 20 | 0 | 0 | 0 | 0 | 0 |
| 21 | 0 | 0 | 0 | 0 | 0 |
| 22 | 1 | 1 | 1 | 0 | 1 |
| 23 | 1 | 1 | 1 | 0 | 1 |
| 24 | 1 | 1 | 1 | 0 | 1 |
| 25 | 1 | 1 | 1 | 1 | 1 |
| 26 | 1 | 1 | 1 | 1 | 1 |
| 27 | 1 | 1 | 1 | 1 | 1 |
| 28 | 0 | 0 | 0 | 0 | 0 |
| 29 | 0 | 0 | 0 | 0 | 0 |
| 30 | 0 | 0 | 0 | 0 | 0 |
| 31 | 0 | 0 | 0 | 0 | 0 |
| 32 | 0 | 0 | 0 | 0 | 0 |
| 33 | 0 | 0 | 0 | 0 | 0 |
| 34 | 1 | 1 | 1 | 1 | 1 |
| 35 | 1 | 1 | 1 | 1 | 1 |
| 36 | 1 | 1 | 1 | 1 | 1 |
| 37 | 0 | 0 | 0 | 0 | 0 |
| 38 | 0 | 0 | 0 | 0 | 0 |
| 39 | 0 | 0 | 0 | 0 | 0 |
| 40 | 1 | 1 | 1 | 0 | 1 |
| 41 | 1 | 1 | 1 | 0 | 1 |
| 42 | 1 | 1 | 1 | 0 | 1 |
| 43 | 0 | 0 | 0 | 0 | 0 |
| 44 | 0 | 0 | 0 | 0 | 0 |
| 45 | 0 | 0 | 0 | 0 | 0 |
| 46 | 0 | 0 | 1 | 0 | 0 |
| 47 | 0 | 0 | 1 | 0 | 0 |
| 48 | 0 | 0 | 1 | 0 | 0 |
| 49 | 0 | 0 | 0 | 0 | 0 |
| 50 | 0 | 0 | 0 | 0 | 0 |
| 51 | 0 | 0 | 0 | 0 | 0 |
| 52 | 0 | 0 | 0 | 0 | 0 |
| 53 | 0 | 0 | 0 | 0 | 0 |
| 54 | 0 | 0 | 0 | 0 | 0 |
| 55 | 0 | 0 | 0 | 0 | 0 |
| 56 | 0 | 0 | 0 | 0 | 0 |
| 57 | 0 | 0 | 0 | 0 | 0 |
| 58 | 0 | 0 | 0 | 0 | 0 |
| 59 | 0 | 0 | 0 | 0 | 0 |
| 60 | 0 | 0 | 0 | 0 | 0 |

Les échantillons Ech91 à Ech95 comportent au moins un peptide caractéristique des SME. Les bactéries présentes dans les échantillons Ech91 à Ech95 expriment donc une béta-lactamase qui leur confère une résistance aux pénicillines, aux céphalosporines et aux carbapénèmes.

### Exemple 12 : Identification d'une résistance aux béta-lactamines de type IMP:

Les échantillons correspondant à une espèce pouvant comporter un mécanisme de résistance de type IMP peuvent être détectés en mettant en oeuvre la méthode suivante.

Chaque échantillon est traité selon l'exemple 5, puis analysé selon l'exemple 6 en détectant les peptides du **TABLEAU 21** en lieu et place des peptides du **TABLEAU 3.**

**TABLEAU 21 :**

| Transition numéro | Peptide | Temps de rétention (minutes) | (m/z) filtré en Q1 | (m/z) filtré en Q3 | Energie de collision (eV) |
|---|---|---|---|---|---|
| 1 | EVNGWGVVPK | 16,02 | 542,79 | 742,35 | 29 |
| 2 | EVNGWGVVPK | 16,02 | 542,79 | 856,47 | 29 |
| 3 | EVNGWGVVPK | 16,02 | 542,79 | 955,54 | 29 |
| 4 | GSISSHFHSDSTGGIGWLNSR | 16,97 | 551,26 | 675,36 | 31 |
| 5 | GSISSHFHSDSTGGIGWLNSR | 16,97 | 551,26 | 732,38 | 31 |
| 6 | GSISSHFHSDSTGGIGWLNSR | 16,97 | 734,68 | 959,51 | 41 |
| 7 | HGLVILVNTDAYLIDTPFTAK | 24,53 | 767,75 | 892,48 | 42 |
| 8 | HGLVILVNTDAYLIDTPFTAK | 24,53 | 767,75 | 1005,56 | 42 |
| 9 | HGLVILVNTDAYLIDTPFTAK | 24,53 | 767,75 | 1133,63 | 42 |
| 10 | HGLVVLVNNDAYLIDTPFTNK | 22,75 | 781,75 | 822,4 | 43 |
| 11 | HGLVVLVNNDAYLIDTPFTNK | 22,75 | 781,75 | 935,48 | 43 |
| 12 | HGLVVLVNNDAYLIDTPFTNK | 22,75 | 781,75 | 1132,61 | 43 |
| 13 | HGLVVLVNTDAYLIDTPFTAK | 23,91 | 763,08 | 779,39 | 42 |
| 14 | HGLVVLVNTDAYLIDTPFTAK | 23,91 | 763,08 | 892,48 | 42 |
| 15 | HGLVVLVNTDAYLIDTPFTAK | 23,91 | 763,08 | 1119,62 | 42 |
| 16 | HGLVVLVNTEAYLIDTPFTAK | 24,53 | 767,75 | 779,39 | 42 |
| 17 | HGLVVLVNTEAYLIDTPFTAK | 24,53 | 767,75 | 892,48 | 42 |
| 18 | HGLVVLVNTEAYLIDTPFTAK | 24,53 | 767,75 | 1133,63 | 42 |
| 19 | IEVFYPGPGHTQDNVVVWLPK | 22,25 | 599,57 | 642,4 | 33 |
| 20 | IEVFYPGPGHTQDNVVVWLPK | 22,25 | 599,57 | 741,47 | 33 |
| 21 | IEVFYPGPGHTQDNVVVWLPK | 22,25 | 799,09 | 872,46 | 44 |
| 22 | ILMEK | 11,28 | 317,19 | 407,2 | 19 |
| 23 | ILMEK | 11,28 | 317,19 | 487,26 | 19 |
| 24 | ILMEK | 11,28 | 317,19 | 520,28 | 19 |
| 25 | ILMSK | 10,48 | 296,18 | 365,19 | 18 |
| 26 | ILMSK | 10,48 | 296,18 | 445,25 | 18 |
| 27 | ILMSK | 10,48 | 296,18 | 478,27 | 18 |
| 28 | LDEGVYVHTSFK | 15,03 | 465,57 | 482,26 | 27 |
| 29 | LDEGVYVHTSFK | 15,03 | 465,57 | 619,32 | 27 |
| 30 | LDEGVYVHTSFK | 15,03 | 465,57 | 881,45 | 27 |
| 31 | LEEGVYVHTSYEEVK | 14,55 | 594,62 | 855,41 | 34 |
| 32 | LEEGVYVHTSYEEVK | 14,55 | 594,62 | 992,47 | 34 |
| 33 | LEEGVYVHTSYEEVK | 14,55 | 891,43 | 992,47 | 44 |
| 34 | LLISK | 12,19 | 287,2 | 347,23 | 18 |
| 35 | LLISK | 12,19 | 287,2 | 427,29 | 18 |
| 36 | LLISK | 12,19 | 287,2 | 460,31 | 18 |
| 37 | LLMSK | 11,18 | 296,18 | 365,19 | 18 |
| 38 | LLMSK | 11,18 | 296,18 | 445,25 | 18 |
| 39 | LLMSK | 11,18 | 296,18 | 478,27 | 18 |
| 40 | LLVSK | 10,48 | 280,19 | 333,21 | 17 |
| 41 | LLVSK | 10,48 | 280,19 | 413,28 | 17 |
| 42 | LLVSK | 10,48 | 280,19 | 446,3 | 17 |
| 43 | LPDLK | 12,56 | 293,18 | 375,22 | 18 |
| 44 | LPDLK | 12,56 | 293,18 | 439,26 | 18 |
| 45 | LPDLK | 12,56 | 293,18 | 472,28 | 18 |
| 46 | LVVSGHSETGDATHLK | 11,41 | 413,47 | 569,34 | 24 |
| 47 | LVVSGHSETGDATHLK | 11,41 | 550,95 | 719,85 | 32 |
| 48 | LVVSGHSETGDATHLK | 11,41 | 550,95 | 1058,51 | 32 |
| 49 | NSFDGVSYWLAK | 20,75 | 693,84 | 767,41 | 36 |
| 50 | NSFDGVSYWLAK | 20,75 | 693,84 | 1038,53 | 36 |
| 51 | NSFDGVSYWLAK | 20,75 | 693,84 | 1185,59 | 36 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 13 : Identification d'une résistance aux béta-lactamines de type KPC:

Les échantillons correspondant à une espèce pouvant comporter un mécanisme de résistance de type KPC peuvent être détectés en mettant en oeuvre la méthode suivante.

Chaque échantillon est traité selon l'exemple 5, puis analysé selon l'exemple 6 en détectant les peptides du **TABLEAU 22** en lieu et place des peptides du **TABLEAU 3.**

**TABLEAU 22 :**

| Transition numéro | Peptide | Temps de rétention (minutes) | (m/z) filtré en Q1 | (m/z) filtré en Q3 | Energie de collision (eV) |
|---|---|---|---|---|---|
| 1 | NALVR | 8,14 | 286,68 | 387,27 | 18 |
| 2 | NALVR | 8,14 | 286,68 | 398,24 | 18 |
| 3 | NALVR | 8,14 | 286,68 | 458,31 | 18 |
| 4 | TGTC[CAM]GAYGTANDYAVVWPTGR | 18,76 | 739,67 | 1169,45 | 41 |
| 5 | TGTC[CAM]GAYGTANDYAVVWPTGR | 18,76 | 1109,01 | 1163,58 | 54 |
| 6 | TGTC[CAM]GAYGTANDYAVVWPTGR | 18,76 | 1109,01 | 1169,45 | 54 |
| 7 | WELELNSAIPSDAR | 20,43 | 534,27 | 545,27 | 31 |
| 8 | WELELNSAIPSDAR | 20,43 | 800,9 | 930,46 | 40 |
| 9 | WELELNSAIPSDAR | 20,43 | 800,9 | 1043,55 | 40 |
| 10 | WELEMNSAIPGDAR | 19,35 | 794,87 | 900,45 | 40 |
| 11 | WELEMNSAIPGDAR | 19,35 | 794,87 | 1031,49 | 40 |
| 12 | WELEMNSAIPGDAR | 19,35 | 794,87 | 1074,49 | 40 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 14 : Identification d'une résistance aux béta-lactamines de type NDM:

Les échantillons correspondant à une espèce pouvant comporter un mécanisme de résistance de type NDM peuvent être détectés en mettant en oeuvre la méthode suivante.

Chaque échantillon est traité selon l'exemple 5, puis analysé selon l'exemple 6 en détectant les peptides du **TABLEAU 23** en lieu et place des peptides du **TABLEAU 3.**

**TABLEAU 23 :**

| Transition numéro | Peptide | Temps de rétention (minutes) | (m/z) filtré en Q1 | (m/z) filtré en Q3 | Energie de collision (eV) |
|---|---|---|---|---|---|
| 1 | VLLVDTAWTDDQTAQILNWIK | 27,87 | 815,1 | 914,55 | 45 |
| 2 | VLLVDTAWTDDQTAQILNWIK | 27,86 | 815,1 | 985,58 | 45 |
| 3 | VLLVDTAWTDDQTAQILNWIK | 27,85 | 815,1 | 1086,63 | 45 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 15 : Identification d'une résistance aux béta-lactamines de type VIM:

Les échantillons correspondant à une espèce pouvant comporter un mécanisme de résistance de type VIM peuvent être détectés en mettant en oeuvre la méthode suivante.

Chaque échantillon est traité selon l'exemple 5, puis analysé selon l'exemple 6 en détectant les peptides du **TABLEAU 24** en lieu et place des peptides du **TABLEAU 3.**

**TABLEAU 24 :**

| Transition numéro | Peptide | Temps de rétention (minutes) | (m/z) filtré en Q1 | (m/z) filtré en Q3 | Energie de collision (eV) |
|---|---|---|---|---|---|
| 1 | LANEIPTHSLEGLSSSGDAVR | 16,72 | 718,37 | 778,37 | 40 |
| 2 | LANEIPTHSLEGLSSSGDAVR | 16,72 | 718,37 | 948,47 | 40 |
| 3 | LANEIPTHSLEGLSSSGDAVR | 16,72 | 718,37 | 1077,52 | 40 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 16 : Identification d'une résistance aux béta-lactamines de type OXA:

Les échantillons correspondant à une espèce pouvant comporter un mécanisme de résistance de type OXA peuvent être détectés en mettant en oeuvre la méthode suivante.

Chaque échantillon est traité selon l'exemple 5, puis analysé selon l'exemple 6 en détectant les peptides du **TABLEAU 25** en lieu et place des peptides du **TABLEAU 3.**

**TABLEAU 25 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AAAYELAENLFEAGQADGWR | 24,48 | 728,01 | 1249,6 | 40 |
| 2 | AAAYELAENLFEAGQADGWR | 24,48 | 1091,51 | 1193,58 | 53 |
| 3 | AAAYELAENLFEAGQADGWR | 24,48 | 1091,51 | 1249,6 | 53 |
| 4 | AAEGFIPASTFK | 17,74 | 619,82 | 763,43 | 32 |
| 5 | AAEGFIPASTFK | 17,74 | 619,82 | 910,5 | 32 |
| 6 | AAEGFIPASTFK | 17,74 | 619,82 | 967,52 | 32 |
| 7 | ADGQVVAFALNMQMK | 21,27 | 811,91 | 982,48 | 41 |
| 8 | ADGQVVAFALNMQMK | 21,29 | 811,91 | 1053,52 | 41 |
| 9 | ADGQVVAFALNMQMK | 21,27 | 811,91 | 1152,59 | 41 |
| 10 | ADINEIFK | 17,3 | 475,25 | 650,35 | 26 |
| 11 | ADINEIFK | 17,3 | 475,25 | 763,43 | 26 |
| 12 | ADINEIFK | 17,3 | 475,25 | 878,46 | 26 |
| 13 | ADWGK | 6,9 | 288,64 | 390,21 | 18 |
| 14 | ADWGK | 6,91 | 288,64 | 430,17 | 18 |
| 15 | ADWGK | 6,89 | 288,64 | 505,24 | 18 |
| 16 | AEGAIVISDER | 13,52 | 387,2 | 419,19 | 23 |
| 17 | AEGAIVISDER | 13,53 | 387,2 | 506,22 | 23 |
| 18 | AEGAIVISDER | 13,52 | 387,2 | 619,3 | 23 |
| 19 | AFALNLDIDK | 20,16 | 560,31 | 717,38 | 30 |
| 20 | AFALNLDIDK | 20,16 | 560,31 | 830,46 | 30 |
| 21 | AFALNLDIDK | 20,16 | 560,31 | 901,5 | 30 |
| 22 | AFAPMSTFK | 16,96 | 500,25 | 710,35 | 27 |
| 23 | AFAPMSTFK | 16,96 | 500,25 | 781,39 | 27 |
| 24 | AFAPMSTFK | 16,96 | 500,25 | 928,46 | 27 |
| 25 | AFGYGNADVSGDPGQNNGLDR | 15,12 | 708,65 | 873,42 | 39 |
| 26 | AFGYGNADVSGDPGQNNGLDR | 15,12 | 708,65 | 970,47 | 39 |
| 27 | AFGYGNADVSGDPGQNNGLDR | 15,12 | 708,65 | 1154,47 | 39 |
| 28 | AFTMTK | 11,32 | 349,68 | 480,25 | 20 |
| 29 | AFTMTK | 11,33 | 349,68 | 552,25 | 20 |
| 30 | AFTMTK | 11,33 | 349,68 | 627,32 | 20 |
| 31 | AGDDIALR | 12,23 | 415,72 | 587,35 | 23 |
| 32 | AGDDIALR | 12,23 | 415,72 | 702,38 | 23 |
| 33 | AGDDIALR | 12,23 | 415,72 | 759,4 | 23 |
| 34 | AGHVYAFALNIDMPR | 20,63 | 558,95 | 631,32 | 32 |
| 35 | AGHVYAFALNIDMPR | 20,63 | 558,95 | 745,37 | 32 |
| 36 | AGHVYAFALNIDMPR | 20,63 | 558,95 | 817,4 | 32 |
| 37 | AGLWR | 13,44 | 301,67 | 361,2 | 18 |
| 38 | AGLWR | 13,44 | 301,67 | 474,28 | 18 |
| 39 | AGLWR | 13,44 | 301,67 | 531,3 | 18 |
| 40 | AHTEYVPASTFK | 13,18 | 450,89 | 553,3 | 27 |
| 41 | AHTEYVPASTFK | 13,18 | 450,89 | 602,26 | 27 |
| 42 | AHTEYVPASTFK | 13,18 | 450,89 | 650,35 | 27 |
| 43 | AIIPWDGKPR | 15,84 | 384,89 | 428,23 | 23 |
| 44 | AIIPWDGKPR | 15,84 | 384,89 | 457,29 | 23 |
| 45 | AIIPWDGKPR | 15,84 | 384,89 | 572,32 | 23 |
| 46 | AISDITITR | 14,8 | 495,28 | 603,38 | 27 |
| 47 | AISDITITR | 14,8 | 495,28 | 718,41 | 27 |
| 48 | AISDITITR | 14,8 | 495,28 | 805,44 | 27 |
| 49 | ALGQDR | 11,25 | 330,18 | 475,23 | 20 |
| 50 | ALGQDR | 11,25 | 330,18 | 485,24 | 20 |
| 51 | ALGQDR | 11,25 | 330,18 | 588,31 | 20 |
| 52 | ALQAK | 1,86 | 265,67 | 346,21 | 17 |
| 53 | ALQAK | 1,87 | 265,67 | 384,22 | 17 |
| 54 | ALQAK | 1,87 | 265,67 | 459,29 | 17 |
| 55 | AMETFSPASTFK | 17,06 | 658,81 | 737,38 | 34 |
| 56 | AMETFSPASTFK | 17,05 | 658,81 | 985,5 | 34 |
| 57 | AMETFSPASTFK | 17,06 | 658,81 | 1114,54 | 34 |
| 58 | AMLFLQER | 18,48 | 504,27 | 545,3 | 27 |
| 59 | AMLFLQER | 18,48 | 504,27 | 692,37 | 27 |
| 60 | AMLFLQER | 18,48 | 504,27 | 805,46 | 27 |
| 61 | AMLVFDPVR | 19,87 | 524,29 | 732,4 | 28 |
| 62 | AMLVFDPVR | 19,87 | 524,29 | 845,49 | 28 |
| 63 | AMLVFDPVR | 19,87 | 524,29 | 976,53 | 28 |
| 64 | AMTLLESGPGWELHGK | 19,32 | 575,96 | 923,47 | 33 |
| 65 | AMTLLESGPGWELHGK | 19,32 | 575,96 | 980,49 | 33 |
| 66 | AMTLLESGPGWELHGK | 19,32 | 575,96 | 1067,53 | 33 |
| 67 | ANLHITLHGK | 12,18 | 368,55 | 403,24 | 22 |
| 68 | ANLHITLHGK | 12,18 | 368,55 | 555,32 | 22 |
| 69 | ANLHITLHGK | 12,18 | 368,55 | 668,41 | 22 |
| 70 | ANQLIVK | 11,87 | 393,25 | 600,41 | 22 |
| 71 | ANQLIVK | 11,86 | 393,25 | 639,38 | 22 |
| 72 | ANQLIVK | 11,86 | 393,25 | 714,45 | 22 |
| 73 | ANTEYVPASTFK | 14,54 | 664,33 | 912,48 | 34 |
| 74 | ANTEYVPASTFK | 14,54 | 664,33 | 1041,53 | 34 |
| 75 | ANTEYVPASTFK | 14,54 | 664,33 | 1142,57 | 34 |
| 76 | ANVSR | 9,57 | 273,65 | 361,22 | 17 |
| 77 | ANVSR | 9,57 | 273,65 | 372,19 | 17 |
| 78 | ANVSR | 9,57 | 273,65 | 475,26 | 17 |
| 79 | APIGWFIGWATR | 25,58 | 687,87 | 850,46 | 35 |
| 80 | APIGWFIGWATR | 25,58 | 687,87 | 1093,56 | 35 |
| 81 | APIGWFIGWATR | 25,58 | 687,87 | 1206,64 | 35 |
| 82 | APLGWFIGWATHEER | 24,69 | 590,63 | 742,35 | 34 |
| 83 | APLGWFIGWATHEER | 24,69 | 590,63 | 985,45 | 34 |
| 84 | APLGWFIGWATHEER | 24,69 | 590,63 | 1098,53 | 34 |
| 85 | AQDEVQSMLFIEEK | 20,15 | 833,9 | 996,51 | 42 |
| 86 | AQDEVQSMLFIEEK | 20,14 | 833,9 | 1124,57 | 42 |
| 87 | AQDEVQSMLFIEEK | 20,15 | 833,9 | 1223,63 | 42 |
| 88 | AQGVIVLWNENK | 18,95 | 685,87 | 902,47 | 35 |
| 89 | AQGVIVLWNENK | 18,95 | 685,87 | 1015,56 | 35 |
| 90 | AQGVIVLWNENK | 18,95 | 685,87 | 1171,65 | 35 |
| 91 | ASAIAVYQDLAR | 18,05 | 639,35 | 765,39 | 33 |
| 92 | ASAIAVYQDLAR | 18,05 | 639,35 | 864,46 | 33 |
| 93 | ASAIAVYQDLAR | 18,05 | 639,35 | 935,49 | 33 |
| 94 | ASAILVYQDLAR | 19,08 | 660,37 | 765,39 | 34 |
| 95 | ASAILVYQDLAR | 19,08 | 660,37 | 864,46 | 34 |
| 96 | ASAILVYQDLAR | 19,08 | 660,37 | 977,54 | 34 |
| 97 | ASAIPVYQDLAR | 17,45 | 652,35 | 765,39 | 34 |
| 98 | ASAIPVYQDLAR | 17,45 | 652,35 | 864,46 | 34 |
| 99 | ASAIPVYQDLAR | 17,45 | 652,35 | 961,51 | 34 |
| 100 | ASAIPVYQDLPR | 17,59 | 665,36 | 791,4 | 34 |
| 101 | ASAIPVYQDLPR | 17,59 | 665,36 | 890,47 | 34 |
| 102 | ASAIPVYQDLPR | 17,6 | 665,36 | 987,53 | 34 |
| 103 | ASAIQVYQDLAR | 18,37 | 667,86 | 765,39 | 34 |
| 104 | ASAIQVYQDLAR | 18,37 | 667,86 | 864,46 | 34 |
| 105 | ASAIQVYQDLAR | 18,37 | 667,86 | 992,52 | 34 |
| 106 | ASAISVYQDLAR | 17,93 | 647,34 | 765,39 | 33 |
| 107 | ASAISVYQDLAR | 17,93 | 647,34 | 864,46 | 33 |
| 108 | ASAISVYQDLAR | 17,93 | 647,34 | 951,49 | 33 |
| 109 | ASALPVYQDLAR | 17,77 | 652,35 | 864,46 | 34 |
| 110 | ASALPVYQDLAR | 17,77 | 652,35 | 961,51 | 34 |
| 111 | ASALPVYQDLAR | 17,77 | 652,35 | 1074,59 | 34 |
| 112 | ASAMPVYQDLAR | 16,64 | 661,33 | 765,39 | 34 |
| 113 | ASAMPVYQDLAR | 16,64 | 661,33 | 864,46 | 34 |
| 114 | ASAMPVYQDLAR | 16,64 | 661,33 | 961,51 | 34 |
| 115 | ASAVPVYQDLAR | 16,29 | 645,35 | 765,39 | 33 |
| 116 | ASAVPVYQDLAR | 16,29 | 645,35 | 864,46 | 33 |
| 117 | ASAVPVYQDLAR | 16,29 | 645,35 | 961,51 | 33 |
| 118 | ASIEYVPASTFK | 16,7 | 656,84 | 749,42 | 34 |
| 119 | ASIEYVPASTFK | 16,7 | 656,84 | 912,48 | 34 |
| 120 | ASIEYVPASTFK | 16,7 | 656,84 | 1041,53 | 34 |
| 121 | ASNVPVYQELAR | 18,48 | 673,86 | 779,4 | 35 |
| 122 | ASNVPVYQELAR | 18,48 | 673,86 | 878,47 | 35 |
| 123 | ASNVPVYQELAR | 18,48 | 673,86 | 975,53 | 35 |
| 124 | ASPASTFK | 10,29 | 404,71 | 553,3 | 23 |
| 125 | ASPASTFK | 10,29 | 404,71 | 650,35 | 23 |
| 126 | ASPASTFK | 10,28 | 404,71 | 737,38 | 23 |
| 127 | ASTAYIPASTFK | 15,69 | 628,83 | 763,43 | 33 |
| 128 | ASTAYIPASTFK | 15,69 | 628,83 | 926,5 | 33 |
| 129 | ASTAYIPASTFK | 15,69 | 628,83 | 997,54 | 33 |
| 130 | ASTEYVPASTFK | 14,59 | 650,82 | 749,42 | 34 |
| 131 | ASTEYVPASTFK | 14,59 | 650,82 | 912,48 | 34 |
| 132 | ASTEYVPASTFK | 14,6 | 650,82 | 1041,53 | 34 |
| 133 | ASTTEVFK | 11,78 | 441,73 | 623,34 | 24 |
| 134 | ASTTEVFK | 11,78 | 441,73 | 724,39 | 24 |
| 135 | ASTTEVFK | 11,78 | 441,73 | 811,42 | 24 |
| 136 | ATSTEIFK | 13,15 | 448,74 | 637,36 | 25 |
| 137 | ATSTEIFK | 13,15 | 448,74 | 724,39 | 25 |
| 138 | ATSTEIFK | 13,15 | 448,74 | 825,44 | 25 |
| 139 | ATTNEIFK | 13,21 | 462,25 | 650,35 | 25 |
| 140 | ATTNEIFK | 13,21 | 462,25 | 751,4 | 25 |
| 141 | ATTNEIFK | 13,21 | 462,25 | 852,45 | 25 |
| 142 | ATTTAVFK | 11,9 | 419,74 | 464,29 | 23 |
| 143 | ATTTAVFK | 11,9 | 419,74 | 565,33 | 23 |
| 144 | ATTTAVFK | 11,9 | 419,74 | 666,38 | 23 |
| 145 | ATTTEIFK | 13,64 | 455,75 | 637,36 | 25 |
| 146 | ATTTEIFK | 13,65 | 455,75 | 738,4 | 25 |
| 147 | ATTTEIFK | 13,65 | 455,75 | 839,45 | 25 |
| 148 | ATTTEVFK | 11,98 | 448,74 | 623,34 | 25 |
| 149 | ATTTEVFK | 11,98 | 448,74 | 724,39 | 25 |
| 150 | ATTTEVFK | 11,98 | 448,74 | 825,44 | 25 |
| 151 | AVSDITILEQTDNYTLHGK | 19,19 | 706,7 | 974,49 | 39 |
| 152 | AVSDITILEQTDNYTLHGK | 19,19 | 706,7 | 1048,51 | 39 |
| 153 | AVSDITILEQTDNYTLHGK | 19,18 | 706,7 | 1176,56 | 39 |
| 154 | AVSDITILEQTYNYTLHGK | 22,29 | 722,71 | 995,49 | 40 |
| 155 | AVSDITILEQTYNYTLHGK | 22,29 | 722,71 | 998,5 | 40 |
| 156 | AVSDITILEQTYNYTLHGK | 22,28 | 722,71 | 1224,6 | 40 |
| 157 | AVVPHFEAGDWDVQGK | 17,81 | 585,62 | 743,34 | 33 |
| 158 | AVVPHFEAGDWDVQGK | 17,81 | 585,62 | 792,88 | 33 |
| 159 | AVVPHFEAGDWDVQGK | 17,81 | 585,62 | 904,42 | 33 |
| 160 | AWEHDMSLR | 13,99 | 572,76 | 758,36 | 30 |
| 161 | AWEHDMSLR | 13,99 | 572,76 | 887,4 | 30 |
| 162 | AWEHDMSLR | 13,99 | 572,76 | 1073,48 | 30 |
| 163 | AWIGSSLQISPLEQLEFLGK | 26,98 | 739,4 | 963,51 | 41 |
| 164 | AWIGSSLQISPLEQLEFLGK | 26,99 | 739,4 | 1173,65 | 41 |
| 165 | AWIGSSLQISPLEQLEFLGK | 26,98 | 1108,6 | 1173,65 | 54 |
| 166 | DAFLK | 12,42 | 297,17 | 407,27 | 18 |
| 167 | DAFLK | 12,43 | 297,17 | 447,22 | 18 |
| 168 | DAFLK | 12,42 | 297,17 | 478,3 | 18 |
| 169 | DDFILHGK | 13,99 | 472,75 | 714,43 | 26 |
| 170 | DDFILHGK | 13,99 | 472,75 | 798,38 | 26 |
| 171 | DDFILHGK | 13,99 | 472,75 | 829,46 | 26 |
| 172 | DDVLK | 8,62 | 295,16 | 359,27 | 18 |
| 173 | DDVLK | 8,63 | 295,16 | 443,21 | 18 |
| 174 | DDVLK | 8,62 | 295,16 | 474,29 | 18 |
| 175 | DEFHVFR | 15,39 | 475,23 | 705,38 | 26 |
| 176 | DEFHVFR | 15,39 | 475,23 | 775,34 | 26 |
| 177 | DEFHVFR | 15,39 | 475,23 | 834,43 | 26 |
| 178 | DEFQIFR | 19,02 | 477,74 | 520,2 | 26 |
| 179 | DEFQIFR | 19,02 | 477,74 | 563,33 | 26 |
| 180 | DEFQIFR | 19,02 | 477,74 | 710,4 | 26 |
| 181 | DEFQVFR | 17,29 | 470,73 | 549,31 | 26 |
| 182 | DEFQVFR | 17,28 | 470,73 | 619,27 | 26 |
| 183 | DEFQVFR | 17,29 | 470,73 | 696,38 | 26 |
| 184 | DELVR | 9,33 | 316,17 | 387,27 | 19 |
| 185 | DELVR | 9,35 | 316,17 | 457,23 | 19 |
| 186 | DELVR | 9,33 | 316,17 | 516,31 | 19 |
| 187 | DFDYGNQDFSGDK | 14,72 | 754,3 | 967,41 | 38 |
| 188 | DFDYGNQDFSGDK | 14,72 | 754,3 | 1130,47 | 38 |
| 189 | DFDYGNQDFSGDK | 14,72 | 754,3 | 1245,5 | 38 |
| 190 | DFTLGEAMQASTVPVYQELAR | 24,19 | 776,05 | 975,53 | 43 |
| 191 | DFTLGEAMQASTVPVYQELAR | 24,19 | 776,05 | 1074,59 | 43 |
| 192 | DFTLGEAMQASTVPVYQELAR | 24,19 | 1163,57 | 1175,64 | 56 |
| 193 | DHDLITAMK | 14,23 | 522,26 | 563,32 | 28 |
| 194 | DHDLITAMK | 14,23 | 522,26 | 695,34 | 28 |
| 195 | DHDLITAMK | 14,23 | 522,26 | 791,43 | 28 |
| 196 | DIAAWNR | 13,63 | 423,22 | 546,28 | 24 |
| 197 | DIAAWNR | 13,63 | 423,22 | 617,32 | 24 |
| 198 | DIAAWNR | 13,62 | 423,22 | 730,4 | 24 |
| 199 | DILYIQELAGGWK | 24,49 | 753,4 | 888,46 | 38 |
| 200 | DILYIQELAGGWK | 24,48 | 753,4 | 1001,54 | 38 |
| 201 | DILYIQELAGGWK | 24,49 | 753,4 | 1164,6 | 38 |
| 202 | DITILEK | 15,9 | 416,24 | 603,37 | 23 |
| 203 | DITILEK | 15,91 | 416,24 | 685,38 | 23 |
| 204 | DITILEK | 15,91 | 416,24 | 716,46 | 23 |
| 205 | DLLSAK | 12,45 | 323,69 | 429,23 | 19 |
| 206 | DLLSAK | 12,44 | 323,69 | 500,27 | 19 |
| 207 | DLLSAK | 12,45 | 323,69 | 531,35 | 19 |
| 208 | DLMITEAGR | 15,07 | 503,26 | 533,27 | 27 |
| 209 | DLMITEAGR | 15,07 | 503,26 | 646,35 | 27 |
| 210 | DLMITEAGR | 15,07 | 503,26 | 777,39 | 27 |
| 211 | DLMIVEAGR | 16,68 | 502,27 | 531,29 | 27 |
| 212 | DLMIVEAGR | 16,68 | 502,27 | 644,37 | 27 |
| 213 | DLMIVEAGR | 16,68 | 502,27 | 775,41 | 27 |
| 214 | DLMIVEAK | 16,23 | 459,75 | 473,24 | 25 |
| 215 | DLMIVEAK | 16,23 | 459,75 | 559,34 | 25 |
| 216 | DLMIVEAK | 16,23 | 459,75 | 690,39 | 25 |
| 217 | DLSGNPGK | 6,69 | 394,2 | 472,25 | 22 |
| 218 | DLSGNPGK | 6,69 | 394,2 | 559,28 | 22 |
| 219 | DLSGNPGK | 6,7 | 394,2 | 672,37 | 22 |
| 220 | DLSLR | 12,37 | 302,18 | 375,24 | 18 |
| 221 | DLSLR | 12,35 | 302,18 | 429,23 | 18 |
| 222 | DLSLR | 12,36 | 302,18 | 488,32 | 18 |
| 223 | DLTLR | 12,48 | 309,18 | 389,25 | 19 |
| 224 | DLTLR | 12,47 | 309,18 | 443,25 | 19 |
| 225 | DLTLR | 12,47 | 309,18 | 502,33 | 19 |
| 226 | DMTLGDAIK | 15,97 | 482,24 | 503,28 | 26 |
| 227 | DMTLGDAIK | 15,97 | 482,24 | 616,37 | 26 |
| 228 | DMTLGDAIK | 15,97 | 482,24 | 717,41 | 26 |
| 229 | DMTLGDAMALSAVPVYQELAR | 25,76 | 751,04 | 975,53 | 42 |
| 230 | DMTLGDAMALSAVPVYQELAR | 25,76 | 1126,06 | 1145,63 | 55 |
| 231 | DMTLGDAMALSAVPVYQELAR | 25,75 | 1126,06 | 1232,66 | 55 |
| 232 | DMTLGDAMK | 14,46 | 491,22 | 634,32 | 27 |
| 233 | DMTLGDAMK | 14,46 | 491,22 | 735,37 | 27 |
| 234 | DMTLGDAMK | 14,46 | 491,22 | 866,41 | 27 |
| 235 | DMTLGEAMALSAVPVYQDLAR | 25,92 | 751,04 | 961,51 | 42 |
| 236 | DMTLGEAMALSAVPVYQDLAR | 25,92 | 1126,06 | 1131,62 | 55 |
| 237 | DMTLGEAMALSAVPVYQDLAR | 25,92 | 1126,06 | 1218,65 | 55 |
| 238 | DMTLGEAMALSAVPVYQELAR | 26,48 | 755,71 | 779,4 | 42 |
| 239 | DMTLGEAMALSAVPVYQELAR | 26,48 | 755,71 | 975,53 | 42 |
| 240 | DMTLGEAMALSAVPVYQELAR | 26,47 | 1133,07 | 1232,66 | 55 |
| 241 | DMTLGEAMK | 15,09 | 498,23 | 535,25 | 27 |
| 242 | DMTLGEAMK | 15,09 | 498,23 | 648,34 | 27 |
| 243 | DMTLGEAMK | 15,09 | 498,23 | 749,39 | 27 |
| 244 | DMTLGQAMQASAVPVYQELAR | 23,29 | 760,38 | 779,4 | 42 |
| 245 | DMTLGQAMQASAVPVYQELAR | 23,29 | 760,38 | 975,53 | 42 |
| 246 | DMTLGQAMQASAVPVYQELAR | 23,29 | 760,38 | 976,42 | 42 |
| 247 | DQDLR | 2,54 | 323,66 | 403,23 | 19 |
| 248 | DQDLR | 2,55 | 323,66 | 472,2 | 19 |
| 249 | DQDLR | 2,55 | 323,66 | 531,29 | 19 |
| 250 | DQQIGWFVGWASKPGK | 21,64 | 601,98 | 830,45 | 34 |
| 251 | DQQIGWFVGWASKPGK | 21,64 | 902,46 | 929,52 | 45 |
| 252 | DQQIGWFVGWASKPGK | 21,64 | 902,46 | 1076,59 | 45 |
| 253 | DQQVQVYGNDLNR | 13,59 | 774,87 | 851,4 | 39 |
| 254 | DQQVQVYGNDLNR | 13,58 | 774,87 | 950,47 | 39 |
| 255 | DQQVQVYGNDLNR | 13,59 | 774,87 | 1078,53 | 39 |
| 256 | DQTLESAFK | 15,21 | 519,76 | 581,29 | 28 |
| 257 | DQTLESAFK | 15,21 | 519,76 | 694,38 | 28 |
| 258 | DQTLESAFK | 15,21 | 519,76 | 795,42 | 28 |
| 259 | DSIVWYSQELTR | 19,61 | 748,87 | 896,45 | 38 |
| 260 | DSIVWYSQELTR | 19,61 | 748,87 | 1082,53 | 38 |
| 261 | DSIVWYSQELTR | 19,61 | 748,87 | 1181,59 | 38 |
| 262 | DSIVWYSQQLTR | 19,1 | 748,38 | 895,46 | 38 |
| 263 | DSIVWYSQQLTR | 19,11 | 748,38 | 1081,54 | 38 |
| 264 | DSIVWYSQQLTR | 19,1 | 748,38 | 1180,61 | 38 |
| 265 | DSNLR | 1,77 | 302,66 | 402,25 | 18 |
| 266 | DSNLR | 1,77 | 302,66 | 430,19 | 18 |
| 267 | DSNLR | 1,77 | 302,66 | 489,28 | 18 |
| 268 | DSYIAWGGEAWK | 19,67 | 691,82 | 833,39 | 35 |
| 269 | DSYIAWGGEAWK | 19,67 | 691,82 | 904,43 | 35 |
| 270 | DSYIAWGGEAWK | 19,66 | 691,82 | 1017,52 | 35 |
| 271 | DTLNPEWPYK | 17,3 | 631,81 | 819,4 | 33 |
| 272 | DTLNPEWPYK | 17,3 | 631,81 | 933,45 | 33 |
| 273 | DTLNPEWPYK | 17,3 | 631,81 | 1046,53 | 33 |
| 274 | DVDEVFYK | 15,62 | 507,74 | 685,36 | 27 |
| 275 | DVDEVFYK | 15,62 | 507,74 | 800,38 | 27 |
| 276 | DVDEVFYK | 15,62 | 507,74 | 899,45 | 27 |
| 277 | DWILR | 17,44 | 351,7 | 415,2 | 20 |
| 278 | DWILR | 17,44 | 351,7 | 528,28 | 20 |
| 279 | DWILR | 17,44 | 351,7 | 587,37 | 20 |
| 280 | EAFLR | 12,51 | 318,18 | 435,27 | 19 |
| 281 | EAFLR | 12,51 | 318,18 | 461,24 | 19 |
| 282 | EAFLR | 12,51 | 318,18 | 506,31 | 19 |
| 283 | EAIVR | 7,84 | 294,18 | 387,27 | 18 |
| 284 | EAIVR | 7,84 | 294,18 | 413,24 | 18 |
| 285 | EAIVR | 7,84 | 294,18 | 458,31 | 18 |
| 286 | EAIVTEATPEYIVHSK | 16,43 | 596,31 | 746,42 | 34 |
| 287 | EAIVTEATPEYIVHSK | 16,43 | 596,31 | 972,51 | 34 |
| 288 | EAIVTEATPEYIVHSK | 16,42 | 596,31 | 1073,56 | 34 |
| 289 | EALVTEAAPEYLVHSK | 17,3 | 586,31 | 875,46 | 33 |
| 290 | EALVTEAAPEYLVHSK | 17,3 | 586,31 | 972,51 | 33 |
| 291 | EALVTEAAPEYLVHSK | 17,3 | 586,31 | 1114,59 | 33 |
| 292 | EALVTEAPEYLVHSK | 17,58 | 562,63 | 637,32 | 32 |
| 293 | EAL VTEAPEYL VHSK | 17,58 | 562,63 | 972,51 | 32 |
| 294 | EALVTEAPEYLVHSK | 17,58 | 562,63 | 1043,55 | 32 |
| 295 | EEIVR | 8,41 | 323,18 | 387,27 | 19 |
| 296 | EEIVR | 8,4 | 323,18 | 471,24 | 19 |
| 297 | EEIVR | 8,4 | 323,18 | 516,31 | 19 |
| 298 | EEVLAALPAQLK | 19,48 | 641,37 | 740,47 | 33 |
| 299 | EEVLAALPAQLK | 19,47 | 641,37 | 811,5 | 33 |
| 300 | EEVLAALPAQLK | 19,47 | 641,37 | 924,59 | 33 |
| 301 | EFSAEAVNGVFVLC[CAM]K | 21,1 | 835,42 | 936,5 | 42 |
| 302 | EFSAEAVNGVFVLC[CAM]K | 21,1 | 835,42 | 1106,6 | 42 |
| 303 | EFSAEAVNGVFVLC[CAM]K | 21,1 | 835,42 | 1235,65 | 42 |
| 304 | EFSSESVHGVFVLC[CAM]K | 18,26 | 575,62 | 666,36 | 33 |
| 305 | EFSSESVHGVFVLC[CAM]K | 18,26 | 575,62 | 822,45 | 33 |
| 306 | EFSSESVHGVFVLC[CAM]K | 18,26 | 575,62 | 959,51 | 33 |
| 307 | EGMSGSIR | 9,88 | 418,7 | 432,26 | 23 |
| 308 | EGMSGSIR | 9,88 | 418,7 | 519,29 | 23 |
| 309 | EGMSGSIR | 9,88 | 418,7 | 707,35 | 23 |
| 310 | EGMTGSIR | 10,63 | 425,71 | 432,26 | 24 |
| 311 | EGMTGSIR | 10,63 | 425,71 | 533,3 | 24 |
| 312 | EGMTGSIR | 10,63 | 425,71 | 664,34 | 24 |
| 313 | EIAVWNR | 14,78 | 444,24 | 475,24 | 25 |
| 314 | EIAVWNR | 14,77 | 444,24 | 574,31 | 25 |
| 315 | EIAVWNR | 14,77 | 444,24 | 645,35 | 25 |
| 316 | EIAYK | 8,46 | 312,17 | 381,21 | 19 |
| 317 | EIAYK | 8,46 | 312,17 | 477,23 | 19 |
| 318 | EIAYK | 8,46 | 312,17 | 494,3 | 19 |
| 319 | EIFER | 11,7 | 347,18 | 451,23 | 20 |
| 320 | EIFER | 11,7 | 347,18 | 519,24 | 20 |
| 321 | EIFER | 11,7 | 347,18 | 564,31 | 20 |
| 322 | EIFYHYR | 13,31 | 514,25 | 785,37 | 28 |
| 323 | EIFYHYR | 13,31 | 514,25 | 853,39 | 28 |
| 324 | EIFYHYR | 13,32 | 514,25 | 898,46 | 28 |
| 325 | EIGDDK | 1,99 | 338,66 | 434,19 | 20 |
| 326 | EIGDDK | 1,99 | 338,66 | 530,21 | 20 |
| 327 | EIGDDK | 1,99 | 338,66 | 547,27 | 20 |
| 328 | EIGDGK | 1,76 | 309,66 | 376,18 | 19 |
| 329 | EIGDGK | 1,75 | 309,66 | 472,2 | 19 |
| 330 | EIGDGK | 1,75 | 309,66 | 489,27 | 19 |
| 331 | EIGEDK | 2,32 | 345,67 | 448,2 | 20 |
| 332 | EIGEDK | 2,33 | 345,67 | 544,22 | 20 |
| 333 | EIGEDK | 2,33 | 345,67 | 561,29 | 20 |
| 334 | EIGEDNAR | 10,05 | 452,21 | 604,27 | 25 |
| 335 | EIGEDNAR | 10,05 | 452,21 | 661,29 | 25 |
| 336 | EIGEDNAR | 10,06 | 452,21 | 774,37 | 25 |
| 337 | EIGENK | 1,86 | 345,18 | 447,22 | 20 |
| 338 | EIGENK | 1,86 | 345,18 | 543,24 | 20 |
| 339 | EIGENK | 1,86 | 345,18 | 560,3 | 20 |
| 340 | EIGSEIDK | 11,04 | 445,73 | 591,3 | 25 |
| 341 | EIGSEIDK | 11,04 | 445,73 | 648,32 | 25 |
| 342 | EIGSEIDK | 11,04 | 445,73 | 761,4 | 25 |
| 343 | EMIYLK | 15,11 | 398,72 | 536,34 | 23 |
| 344 | EMIYLK | 15,11 | 398,72 | 650,32 | 23 |
| 345 | EMIYLK | 15,11 | 398,72 | 667,38 | 23 |
| 346 | EMLYVER | 14,12 | 470,23 | 566,29 | 26 |
| 347 | EMLYVER | 14,12 | 470,23 | 679,38 | 26 |
| 348 | EMLYVER | 14,12 | 470,23 | 810,42 | 26 |
| 349 | ENIEK | 11,07 | 316,67 | 389,24 | 19 |
| 350 | ENIEK | 11,07 | 316,67 | 486,22 | 19 |
| 351 | ENIEK | 11,07 | 316,67 | 503,28 | 19 |
| 352 | ENQLIVK | 12,15 | 422,25 | 472,35 | 24 |
| 353 | ENQLIVK | 12,15 | 422,25 | 600,41 | 24 |
| 354 | ENQLIVK | 12,15 | 422,25 | 714,45 | 24 |
| 355 | EQAILLFR | 19,88 | 495,29 | 548,36 | 27 |
| 356 | EQAILLFR | 19,88 | 495,29 | 661,44 | 27 |
| 357 | EQAILLFR | 19,88 | 495,29 | 732,48 | 27 |
| 358 | EQIQFLLR | 19,45 | 523,8 | 548,36 | 28 |
| 359 | EQIQFLLR | 19,45 | 523,8 | 676,41 | 28 |
| 360 | EQIQFLLR | 19,45 | 523,8 | 789,5 | 28 |
| 361 | EQLAFDPQVQQQVK | 16,43 | 829,43 | 954,54 | 41 |
| 362 | EQLAFDPQVQQQVK | 16,42 | 829,43 | 1069,56 | 41 |
| 363 | EQLAFDPQVQQQVK | 16,42 | 829,43 | 1216,63 | 41 |
| 364 | EQVDFVQR | 13,09 | 510,76 | 549,31 | 27 |
| 365 | EQVDFVQR | 13,09 | 510,76 | 664,34 | 27 |
| 366 | EQVDFVQR | 13,09 | 510,76 | 763,41 | 27 |
| 367 | EVGEIR | 9,35 | 351,69 | 474,27 | 20 |
| 368 | EVGEIR | 9,35 | 351,69 | 528,27 | 20 |
| 369 | EVGEIR | 9,35 | 351,69 | 573,34 | 20 |
| 370 | EVGEVR | 6,91 | 344,68 | 460,25 | 20 |
| 371 | EVGEVR | 6,91 | 344,68 | 514,25 | 20 |
| 372 | EVGEVR | 6,91 | 344,68 | 559,32 | 20 |
| 373 | EYLPASTFK | 15,41 | 528,27 | 553,3 | 28 |
| 374 | EYLPASTFK | 15,41 | 528,27 | 650,35 | 28 |
| 375 | EYLPASTFK | 15,41 | 528,27 | 763,43 | 28 |
| 376 | EYLPVSTFK | 17,16 | 542,29 | 581,33 | 29 |
| 377 | EYLPVSTFK | 17,16 | 542,29 | 678,38 | 29 |
| 378 | EYLPVSTFK | 17,16 | 542,29 | 791,47 | 29 |
| 379 | EYNTSGTFVFYDGK | 18,2 | 814,37 | 1033,5 | 41 |
| 380 | EYNTSGTFVFYDGK | 18,2 | 814,37 | 1120,53 | 41 |
| 381 | EYNTSGTFVFYDGK | 18,2 | 814,37 | 1221,58 | 41 |
| 382 | EYVPASTFK | 13,89 | 521,27 | 553,3 | 28 |
| 383 | EYVPASTFK | 13,89 | 521,27 | 650,35 | 28 |
| 384 | EYVPASTFK | 13,89 | 521,27 | 749,42 | 28 |
| 385 | FAPESTFK | 13,67 | 463,73 | 482,26 | 25 |
| 386 | FAPESTFK | 13,67 | 463,73 | 611,3 | 25 |
| 387 | FAPESTFK | 13,67 | 463,73 | 708,36 | 25 |
| 388 | FAQYAK | 9,39 | 364,19 | 509,27 | 21 |
| 389 | FAQYAK | 9,39 | 364,19 | 580,31 | 21 |
| 390 | FAQYAK | 9,39 | 364,19 | 581,27 | 21 |
| 391 | FDYGNR | 10,1 | 386,17 | 509,25 | 22 |
| 392 | FDYGNR | 10,1 | 386,17 | 597,23 | 22 |
| 393 | FDYGNR | 10,09 | 386,17 | 624,27 | 22 |
| 394 | FEDLYK | 13,52 | 407,7 | 423,26 | 23 |
| 395 | FEDLYK | 13,52 | 407,7 | 538,29 | 23 |
| 396 | FEDLYK | 13,52 | 407,7 | 667,33 | 23 |
| 397 | FEDTFHISNQK | 14,33 | 455,89 | 476,25 | 27 |
| 398 | FEDTFHISNQK | 14,33 | 455,89 | 589,33 | 27 |
| 399 | FEDTFHISNQK | 14,33 | 455,89 | 726,39 | 27 |
| 400 | FEDTFHTSNQQHEK | 10,66 | 583,26 | 870,41 | 33 |
| 401 | FEDTFHTSNQQHEK | 10,66 | 583,26 | 971,45 | 33 |
| 402 | FEDTFHTSNQQHEK | 10,66 | 583,26 | 1108,51 | 33 |
| 403 | FEYGNQDVSGDSGK | 11,95 | 751,82 | 764,34 | 38 |
| 404 | FEYGNQDVSGDSGK | 11,95 | 751,82 | 1063,47 | 38 |
| 405 | FEYGNQDVSGDSGK | 11,95 | 751,82 | 1226,53 | 38 |
| 406 | FFSDFQAK | 16 | 495,24 | 608,3 | 27 |
| 407 | FFSDFQAK | 16 | 495,24 | 695,34 | 27 |
| 408 | FFSDFQAK | 16 | 495,24 | 842,4 | 27 |
| 409 | FFSDLQAEGAIVIADER | 20,44 | 627,65 | 1143,6 | 35 |
| 410 | FFSDLQAEGAIVIADER | 20,43 | 940,97 | 1143,6 | 46 |
| 411 | FFSDLQAEGAIVIADER | 20,44 | 940,97 | 1179,57 | 46 |
| 412 | FFSDLR | 15,38 | 392,7 | 490,26 | 22 |
| 413 | FFSDLR | 15,38 | 392,7 | 610,29 | 22 |
| 414 | FFSDLR | 15,38 | 392,7 | 637,33 | 22 |
| 415 | FFSEFQAK | 16,13 | 502,25 | 622,32 | 27 |
| 416 | FFSEFQAK | 16,13 | 502,25 | 709,35 | 27 |
| 417 | FFSEFQAK | 16,13 | 502,25 | 856,42 | 27 |
| 418 | FGLEGQLR | 15,8 | 460,25 | 473,28 | 25 |
| 419 | FGLEGQLR | 15,8 | 460,25 | 602,33 | 25 |
| 420 | FGLEGQLR | 15,8 | 460,25 | 772,43 | 25 |
| 421 | FLESLYLNNLPASK | 20,75 | 804,94 | 856,49 | 40 |
| 422 | FLESLYLNNLPASK | 20,75 | 804,94 | 1019,55 | 40 |
| 423 | FLESLYLNNLPASK | 20,75 | 804,94 | 1219,67 | 40 |
| 424 | FLLEGQLR | 18,06 | 488,28 | 602,33 | 26 |
| 425 | FLLEGQLR | 18,06 | 488,28 | 715,41 | 26 |
| 426 | FLLEGQLR | 18,06 | 488,28 | 828,49 | 26 |
| 427 | FQQYVDR | 11,19 | 478,24 | 552,28 | 26 |
| 428 | FQQYVDR | 11,19 | 478,24 | 680,34 | 26 |
| 429 | FQQYVDR | 11,19 | 478,24 | 808,39 | 26 |
| 430 | FSDYVQR | 11,83 | 457,72 | 565,31 | 25 |
| 431 | FSDYVQR | 11,83 | 457,72 | 680,34 | 25 |
| 432 | FSDYVQR | 11,83 | 457,72 | 767,37 | 25 |
| 433 | FSTASTFK | 12,71 | 444,73 | 553,3 | 25 |
| 434 | FSTASTFK | 12,7 | 444,73 | 654,35 | 25 |
| 435 | FSTASTFK | 12,7 | 444,73 | 741,38 | 25 |
| 436 | FSWDGK | 14,32 | 370,17 | 505,24 | 21 |
| 437 | FSWDGK | 14,32 | 370,17 | 592,27 | 21 |
| 438 | FSWDGK | 14,32 | 370,17 | 593,24 | 21 |
| 439 | FSYGNQNISGGIDK | 14,61 | 750,36 | 803,43 | 38 |
| 440 | FSYGNQNISGGIDK | 14,61 | 750,36 | 1045,53 | 38 |
| 441 | FSYGNQNISGGIDK | 14,61 | 750,36 | 1102,55 | 38 |
| 442 | FSYGNQNISGGTDK | 12,74 | 744,34 | 791,39 | 38 |
| 443 | FSYGNQNISGGTDK | 12,74 | 744,34 | 1033,49 | 38 |
| 444 | FSYGNQNISGGTDK | 12,74 | 744,34 | 1090,51 | 38 |
| 445 | FSYGSQNISGGIDK | 14,74 | 736,85 | 803,43 | 37 |
| 446 | FSYGSQNISGGIDK | 14,74 | 736,85 | 1075,54 | 37 |
| 447 | FSYGSQNISGGIDK | 14,75 | 736,85 | 1238,6 | 37 |
| 448 | FTEYVK | 11,81 | 393,71 | 538,29 | 22 |
| 449 | FTEYVK | 11,81 | 393,71 | 639,33 | 22 |
| 450 | FTEYVK | 11,81 | 393,71 | 640,3 | 22 |
| 451 | FVPASTYK | 11,76 | 456,74 | 498,26 | 25 |
| 452 | FVPASTYK | 11,76 | 456,74 | 569,29 | 25 |
| 453 | FVPASTYK | 11,77 | 456,74 | 666,35 | 25 |
| 454 | FVYDLAQGQLPFKPEVQQQVK | 20,48 | 821,44 | 955,52 | 45 |
| 455 | FVYDLAQGQLPFKPEVQQQVK | 20,48 | 821,44 | 1108,59 | 45 |
| 456 | FVYDLAQGQLPFKPEVQQQVK | 20,49 | 821,44 | 1109,07 | 45 |
| 457 | FWLEDQLR | 20,39 | 553,79 | 660,33 | 29 |
| 458 | FWLEDQLR | 20,39 | 553,79 | 773,42 | 29 |
| 459 | FWLEDQLR | 20,38 | 553,79 | 959,49 | 29 |
| 460 | FWLEGPLK | 20,63 | 495,28 | 543,31 | 27 |
| 461 | FWLEGPLK | 20,63 | 495,28 | 656,4 | 27 |
| 462 | FWLEGPLK | 20,63 | 495,28 | 842,48 | 27 |
| 463 | FWLEGQLR | 19,49 | 524,78 | 602,33 | 28 |
| 464 | FWLEGQLR | 19,49 | 524,78 | 715,41 | 28 |
| 465 | FWLEGQLR | 19,48 | 524,78 | 901,49 | 28 |
| 466 | FYPASSFK | 14,74 | 473,74 | 636,34 | 26 |
| 467 | FYPASSFK | 14,74 | 473,74 | 799,4 | 26 |
| 468 | FYPASSFK | 14,74 | 473,74 | 800,36 | 26 |
| 469 | FYPASTFK | 14,98 | 480,74 | 553,3 | 26 |
| 470 | FYPASTFK | 14,99 | 480,74 | 650,35 | 26 |
| 471 | FYPASTFK | 14,98 | 480,74 | 813,41 | 26 |
| 472 | GAIQVSAVPVFQQIAR | 21,6 | 842,48 | 958,55 | 42 |
| 473 | GAIQVSAVPVFQQIAR | 21,6 | 842,48 | 1057,62 | 42 |
| 474 | GAIQVSAVPVFQQIAR | 21,59 | 842,48 | 1128,65 | 42 |
| 475 | GAIQVSAVPVFQQITR | 21,52 | 857,49 | 988,56 | 43 |
| 476 | GAIQVSAVPVFQQITR | 21,51 | 857,49 | 1087,63 | 43 |
| 477 | GAIQVSAVPVFQQITR | 21,52 | 857,49 | 1158,66 | 43 |
| 478 | GELPVSEDALEMTK | 18,1 | 759,87 | 936,43 | 38 |
| 479 | GELPVSEDALEMTK | 18,11 | 759,87 | 1023,47 | 38 |
| 480 | GELPVSEDALEMTK | 18,11 | 759,87 | 1122,53 | 38 |
| 481 | GISSSVR | 8,65 | 353,2 | 448,25 | 21 |
| 482 | GISSSVR | 8,65 | 353,2 | 535,28 | 21 |
| 483 | GISSSVR | 8,67 | 353,2 | 648,37 | 21 |
| 484 | GNQTLVFAR | 14,83 | 503,28 | 605,38 | 27 |
| 485 | GNQTLVFAR | 14,83 | 503,28 | 706,42 | 27 |
| 486 | GNQTLVFAR | 14,83 | 503,28 | 834,48 | 27 |
| 487 | GPLEISAFEEAR | 18,95 | 659,84 | 809,38 | 34 |
| 488 | GPLEISAFEEAR | 18,94 | 659,84 | 922,46 | 34 |
| 489 | GPLEISAFEEAR | 18,94 | 659,84 | 1051,51 | 34 |
| 490 | GPL TITPIQEVK | 18,14 | 648,38 | 814,47 | 34 |
| 491 | GPLTITPIQEVK | 18,15 | 648,38 | 927,55 | 34 |
| 492 | GPLTITPIQEVK | 18,14 | 648,38 | 1028,6 | 34 |
| 493 | GSLLLWDQK | 19,61 | 530,3 | 576,28 | 28 |
| 494 | GSLLLWDQK | 19,61 | 530,3 | 689,36 | 28 |
| 495 | GSLLLWDQK | 19,61 | 530,3 | 802,45 | 28 |
| 496 | GTFVLYDVQR | 17,93 | 599,32 | 680,34 | 31 |
| 497 | GTFVLYDVQR | 17,93 | 599,32 | 793,42 | 31 |
| 498 | GTFVLYDVQR | 17,93 | 599,32 | 892,49 | 31 |
| 499 | GTIVVADER | 11,82 | 480,26 | 490,23 | 26 |
| 500 | GTIVVADER | 11,82 | 480,26 | 589,29 | 26 |
| 501 | GTIVVADER | 11,82 | 480,26 | 688,36 | 26 |
| 502 | GTIVVLDAR | 15,77 | 472,28 | 573,34 | 26 |
| 503 | GTIVVLDAR | 15,77 | 472,28 | 672,4 | 26 |
| 504 | GTIVVLDAR | 15,77 | 472,28 | 785,49 | 26 |
| 505 | GTIVVVDER | 13,6 | 494,28 | 518,26 | 27 |
| 506 | GTIVVVDER | 13,6 | 494,28 | 617,33 | 27 |
| 507 | GTIVVVDER | 13,6 | 494,28 | 716,39 | 27 |
| 508 | GTLPFSAR | 14,96 | 424,73 | 577,31 | 24 |
| 509 | GTLPFSAR | 14,96 | 424,73 | 690,39 | 24 |
| 510 | GTLPFSAR | 14,97 | 424,73 | 791,44 | 24 |
| 511 | HIADSK | 11,91 | 335,68 | 420,21 | 20 |
| 512 | HIADSK | 11,9 | 335,68 | 524,25 | 20 |
| 513 | HIADSK | 11,91 | 335,68 | 533,29 | 20 |
| 514 | HNGTDGAWIISSLR | 19,36 | 509,6 | 575,35 | 29 |
| 515 | HNGTDGAWIISSLR | 19,35 | 509,6 | 653,26 | 29 |
| 516 | HNGTDGAWIISSLR | 19,36 | 509,6 | 688,44 | 29 |
| 517 | HTLSVFDQER | 14,25 | 411,21 | 432,22 | 25 |
| 518 | HTLSVFDQER | 14,25 | 411,21 | 547,25 | 25 |
| 519 | HTLSVFDQER | 14,25 | 411,21 | 694,32 | 25 |
| 520 | HVTFASFR | 14,36 | 322,17 | 338,18 | 20 |
| 521 | HVTFASFR | 14,36 | 322,17 | 409,22 | 20 |
| 522 | HVTFASFR | 14,36 | 322,17 | 485,25 | 20 |
| 523 | IAISLMGYDAGFLR | 23,93 | 763,91 | 898,44 | 39 |
| 524 | IAISLMGYDAGFLR | 23,93 | 763,91 | 1029,48 | 39 |
| 525 | IAISLMGYDAGFLR | 23,94 | 763,91 | 1229,6 | 39 |
| 526 | IALSLMGFDSGILK | 24,91 | 732,91 | 836,45 | 37 |
| 527 | IALSLMGFDSGILK | 24,91 | 732,91 | 967,49 | 37 |
| 528 | IALSLMGFDSGILK | 24,91 | 732,91 | 1167,61 | 37 |
| 529 | IANALIGLENHK | 15,95 | 431,58 | 697,36 | 26 |
| 530 | IANALIGLENHK | 15,95 | 646,87 | 697,36 | 33 |
| 531 | IANALIGLENHK | 15,95 | 646,87 | 810,45 | 33 |
| 532 | IDTFWLDNSLK | 21,79 | 676,35 | 689,38 | 35 |
| 533 | IDTFWLDNSLK | 21,79 | 676,35 | 875,46 | 35 |
| 534 | IDTFWLDNSLK | 21,79 | 676,35 | 1123,58 | 35 |
| 535 | IDYYNLDR | 14,85 | 536,26 | 680,34 | 29 |
| 536 | IDYYNLDR | 14,85 | 536,26 | 843,4 | 29 |
| 537 | IDYYNLDR | 14,85 | 536,26 | 958,43 | 29 |
| 538 | IFNALIALDSGVIK | 24,74 | 737,44 | 802,47 | 37 |
| 539 | IFNALIALDSGVIK | 24,74 | 737,44 | 915,55 | 37 |
| 540 | IFNALIALDSGVIK | 24,74 | 737,44 | 1028,64 | 37 |
| 541 | IFNSLLALDSGALDNER | 22,76 | 924,48 | 976,43 | 46 |
| 542 | IFNSLLALDSGALDNER | 22,77 | 924,48 | 1089,52 | 46 |
| 543 | IFNSLLALDSGALDNER | 22,76 | 924,48 | 1160,55 | 46 |
| 544 | IFNTLIGLENGIVK | 23,29 | 765,95 | 829,48 | 39 |
| 545 | IFNTLIGLENGIVK | 23,3 | 765,95 | 942,56 | 39 |
| 546 | IFNTLIGLENGIVK | 23,3 | 765,95 | 1055,65 | 39 |
| 547 | IGLDLMQK | 17,7 | 459,26 | 634,32 | 25 |
| 548 | IGLDLMQK | 17,7 | 459,26 | 747,41 | 25 |
| 549 | IGLDLMQK | 17,7 | 459,26 | 804,43 | 25 |
| 550 | IGLEK | 8,54 | 280,18 | 389,24 | 17 |
| 551 | IGLEK | 8,55 | 280,18 | 413,24 | 17 |
| 552 | IGLEK | 8,54 | 280,18 | 446,26 | 17 |
| 553 | IGLELMQQEVQR | 18,73 | 722,38 | 787,41 | 37 |
| 554 | IGLELMQQEVQR | 18,73 | 722,38 | 918,45 | 37 |
| 555 | IGLELMQQEVQR | 18,73 | 722,38 | 1031,53 | 37 |
| 556 | IGLELMSK | 17,52 | 445,75 | 478,27 | 25 |
| 557 | IGLELMSK | 17,52 | 445,75 | 720,4 | 25 |
| 558 | IGLELMSK | 17,52 | 445,75 | 777,42 | 25 |
| 559 | IGLELMSNEVK | 18,73 | 616,83 | 707,34 | 32 |
| 560 | IGLELMSNEVK | 18,73 | 616,83 | 820,42 | 32 |
| 561 | IGLELMSNEVK | 18,73 | 616,83 | 949,47 | 32 |
| 562 | IGLER | 10,96 | 294,18 | 304,16 | 18 |
| 563 | IGLER | 10,96 | 294,18 | 417,25 | 18 |
| 564 | IGLER | 10,96 | 294,18 | 474,27 | 18 |
| 565 | IGLNK | 9,59 | 272,68 | 374,24 | 17 |
| 566 | IGLNK | 9,59 | 272,68 | 398,24 | 17 |
| 567 | IGLNK | 9,59 | 272,68 | 431,26 | 17 |
| 568 | IGLNLMQK | 17,1 | 458,77 | 633,34 | 25 |
| 569 | IGLNLMQK | 17,09 | 458,77 | 746,42 | 25 |
| 570 | IGLNLMQK | 17,11 | 458,77 | 803,44 | 25 |
| 571 | IGPSLMQSELQR | 17,02 | 679,86 | 760,39 | 35 |
| 572 | IGPSLMQSELQR | 17,02 | 679,86 | 891,44 | 35 |
| 573 | IGPSLMQSELQR | 17,02 | 679,86 | 1188,6 | 35 |
| 574 | IGYGNMQIGTEVDQFWLK | 24,31 | 700,35 | 935,5 | 39 |
| 575 | IGYGNMQIGTEVDQFWLK | 24,32 | 1050,02 | 1164,54 | 51 |
| 576 | IGYGNMQIGTEVDQFWLK | 24,3 | 1050,02 | 1222,61 | 51 |
| 577 | IINHNLPVK | 11,88 | 349,88 | 456,32 | 21 |
| 578 | IINHNLPVK | 11,88 | 349,88 | 570,36 | 21 |
| 579 | IINHNLPVK | 11,88 | 349,88 | 592,32 | 21 |
| 580 | IINHNLPVR | 12,04 | 359,22 | 598,37 | 22 |
| 581 | IINHNLPVR | 12,04 | 538,32 | 598,37 | 29 |
| 582 | IINHNLPVR | 12,04 | 538,32 | 849,47 | 29 |
| 583 | I LFQQGTQQAC[CAM]AER | 14,51 | 550,61 | 606,27 | 32 |
| 584 | I LFQQGTQQAC[CAM]AER | 14,51 | 825,41 | 1020,45 | 41 |
| 585 | I LFQQGTQQAC[CAM]AER | 14,51 | 825,41 | 1148,51 | 41 |
| 586 | ILNNWFK | 18,98 | 467,76 | 594,3 | 26 |
| 587 | ILNNWFK | 18,98 | 467,76 | 708,35 | 26 |
| 588 | ILNNWFK | 18,97 | 467,76 | 821,43 | 26 |
| 589 | ILNTLISLEEK | 19,98 | 636,87 | 718,4 | 33 |
| 590 | ILNTLISLEEK | 19,98 | 636,87 | 1046,57 | 33 |
| 591 | ILNTLISLEEK | 19,98 | 636,87 | 1159,66 | 33 |
| 592 | INIVK | 11,43 | 293,7 | 359,27 | 18 |
| 593 | INIVK | 11,43 | 293,7 | 440,29 | 18 |
| 594 | INIVK | 11,43 | 293,7 | 473,31 | 18 |
| 595 | INLYGNALSR | 16,05 | 560,81 | 617,34 | 30 |
| 596 | INLYGNALSR | 16,05 | 560,81 | 780,4 | 30 |
| 597 | INLYGNALSR | 16,05 | 560,81 | 893,48 | 30 |
| 598 | IPFSLNLEMK | 21,68 | 596,33 | 834,44 | 31 |
| 599 | IPFSLNLEMK | 21,67 | 596,33 | 981,51 | 31 |
| 600 | IPFSLNLEMK | 21,67 | 596,33 | 1078,56 | 31 |
| 601 | I PHTLFALDADAVR | 20 | 513,62 | 531,29 | 30 |
| 602 | I PHTLFALDADAVR | 20 | 513,62 | 646,32 | 30 |
| 603 | I PHTLFALDADAVR | 20 | 769,92 | 1191,64 | 39 |
| 604 | IPHTLFALDAGAAR | 18,58 | 726,9 | 744,4 | 37 |
| 605 | I PHTLFALDAGAAR | 18,58 | 726,9 | 891,47 | 37 |
| 606 | I PHTLFALDAGAAR | 18,58 | 726,9 | 1004,55 | 37 |
| 607 | I PHTLFALDAGAVR | 19,72 | 494,28 | 588,31 | 29 |
| 608 | I PHTLFALDAGAVR | 19,71 | 494,28 | 780,44 | 29 |
| 609 | I PHTLFALDAGAVR | 19,72 | 740,92 | 1133,63 | 38 |
| 610 | IPNAIIGLETGVIK | 21,75 | 719,44 | 816,48 | 37 |
| 611 | IPNAIIGLETGVIK | 21,75 | 719,44 | 929,57 | 37 |
| 612 | IPNAIIGLETGVIK | 21,75 | 719,44 | 1227,73 | 37 |
| 613 | IPNALIGLETGAIK | 20,96 | 705,42 | 788,45 | 36 |
| 614 | IPNALIGLETGAIK | 20,96 | 705,42 | 901,54 | 36 |
| 615 | IPNALIGLETGAIK | 20,96 | 705,42 | 1014,62 | 36 |
| 616 | IPNSLIAFDTGAVR | 20,24 | 737,41 | 765,39 | 37 |
| 617 | IPNSLIAFDTGAVR | 20,24 | 737,41 | 836,43 | 37 |
| 618 | IPNSLIAFDTGAVR | 20,24 | 737,41 | 949,51 | 37 |
| 619 | IPSAIIGLETGVIK | 21,66 | 705,93 | 816,48 | 36 |
| 620 | IPSAIIGLETGVIK | 21,67 | 705,93 | 929,57 | 36 |
| 621 | IPSAIIGLETGVIK | 21,66 | 705,93 | 1200,72 | 36 |
| 622 | ISAFNQVK | 13,02 | 453,76 | 488,28 | 25 |
| 623 | ISAFNQVK | 13,02 | 453,76 | 706,39 | 25 |
| 624 | ISAFNQVK | 13,02 | 453,76 | 793,42 | 25 |
| 625 | ISAHEQILFLR | 18,28 | 442,92 | 548,36 | 26 |
| 626 | ISAHEQILFLR | 18,28 | 442,92 | 789,5 | 26 |
| 627 | ISAHEQILFLR | 18,28 | 663,88 | 918,54 | 34 |
| 628 | ISAMEQTR | 9,84 | 468,23 | 664,31 | 26 |
| 629 | ISAMEQTR | 9,84 | 468,23 | 735,35 | 26 |
| 630 | ISAMEQTR | 9,84 | 468,23 | 822,38 | 26 |
| 631 | ISAMEQVK | 11,65 | 453,24 | 634,32 | 25 |
| 632 | ISAMEQVK | 11,65 | 453,24 | 705,36 | 25 |
| 633 | ISAMEQVK | 11,65 | 453,24 | 792,39 | 25 |
| 634 | ISATEQVAFLR | 17,7 | 412,23 | 435,27 | 25 |
| 635 | ISATEQVAFLR | 17,71 | 412,23 | 506,31 | 25 |
| 636 | ISATEQVAFLR | 17,7 | 412,23 | 605,38 | 25 |
| 637 | ISATQQIAFLR | 18,58 | 624,36 | 747,45 | 32 |
| 638 | ISATQQIAFLR | 18,58 | 624,36 | 1047,59 | 32 |
| 639 | ISATQQIAFLR | 18,58 | 624,36 | 1134,63 | 32 |
| 640 | ISAVNQVEFLESLFLNK | 28,77 | 976,03 | 988,51 | 48 |
| 641 | ISAVNQVEFLESLFLNK | 28,77 | 976,03 | 1110,62 | 48 |
| 642 | ISAVNQVEFLESLFLNK | 28,77 | 976,03 | 1239,66 | 48 |
| 643 | ISAVNQVK | 10,32 | 429,76 | 488,28 | 24 |
| 644 | ISAVNQVK | 10,32 | 429,76 | 658,39 | 24 |
| 645 | ISAVNQVK | 10,32 | 429,76 | 745,42 | 24 |
| 646 | ISPEEQIQFLR | 18,87 | 680,37 | 933,52 | 35 |
| 647 | ISPEEQIQFLR | 18,87 | 680,37 | 1062,56 | 35 |
| 648 | ISPEEQIQFLR | 18,87 | 680,37 | 1159,61 | 35 |
| 649 | ISPEEQVR | 10,49 | 479,25 | 531,29 | 26 |
| 650 | ISPEEQVR | 10,49 | 479,25 | 660,33 | 26 |
| 651 | ISPEEQVR | 10,49 | 479,25 | 757,38 | 26 |
| 652 | ISPEGQVR | 9,86 | 443,24 | 459,27 | 25 |
| 653 | ISPEGQVR | 9,86 | 443,24 | 588,31 | 25 |
| 654 | ISPEGQVR | 9,86 | 443,24 | 685,36 | 25 |
| 655 | ISPLEQLAFLR | 24,02 | 643,88 | 876,49 | 33 |
| 656 | ISPLEQLAFLR | 24,01 | 643,88 | 989,58 | 33 |
| 657 | ISPLEQLAFLR | 24,02 | 643,88 | 1086,63 | 33 |
| 658 | ITAFQQVDFLR | 21,11 | 669,36 | 777,43 | 34 |
| 659 | ITAFQQVDFLR | 21,12 | 669,36 | 905,48 | 34 |
| 660 | ITAFQQVDFLR | 21,12 | 669,36 | 1123,59 | 34 |
| 661 | ITPIQEVNFADDFANNR | 21,25 | 655,32 | 736,34 | 37 |
| 662 | ITPIQEVNFADDFANNR | 21,25 | 655,32 | 851,36 | 37 |
| 663 | ITPIQEVNFADDFANNR | 21,25 | 655,32 | 922,4 | 37 |
| 664 | ITPIQEVNFADDLANNR | 20,95 | 643,99 | 817,38 | 36 |
| 665 | ITPIQEVNFADDLANNR | 20,95 | 965,49 | 1149,53 | 47 |
| 666 | ITPIQEVNFADDLANNR | 20,96 | 965,49 | 1248,6 | 47 |
| 667 | ITPQQEAQFAYK | 14,52 | 712,36 | 856,42 | 36 |
| 668 | ITPQQEAQFAYK | 14,52 | 712,36 | 984,48 | 36 |
| 669 | ITPQQEAQFAYK | 14,52 | 712,36 | 1209,59 | 36 |
| 670 | ITPQQEAQFTYK | 14,33 | 485,25 | 558,29 | 28 |
| 671 | ITPQQEAQFTYK | 14,33 | 727,37 | 1014,49 | 37 |
| 672 | ITPQQEAQFTYK | 14,33 | 727,37 | 1239,6 | 37 |
| 673 | ITPVQEVNFADDLAHNR | 18,98 | 646,99 | 840,4 | 36 |
| 674 | ITPVQEVNFADDLAHNR | 18,98 | 646,99 | 862,92 | 36 |
| 675 | ITPVQEVNFADDLAHNR | 18,98 | 646,99 | 911,43 | 36 |
| 676 | IVAFALK | 17,21 | 381,25 | 478,3 | 22 |
| 677 | IVAFALK | 17,22 | 381,25 | 549,34 | 22 |
| 678 | IVAFALK | 17,21 | 381,25 | 648,41 | 22 |
| 679 | IVAFALNMEMR | 17,95 | 647,84 | 864,41 | 34 |
| 680 | IVAFALNMEMR | 17,95 | 647,84 | 1011,48 | 34 |
| 681 | IVAFALNMEMR | 17,97 | 647,84 | 1082,51 | 34 |
| 682 | IVESTTLADGTVVHGK | 13,69 | 542,96 | 697,4 | 31 |
| 683 | IVESTTLADGTVVHGK | 13,69 | 542,96 | 812,43 | 31 |
| 684 | IVESTTLADGTVVHGK | 13,68 | 542,96 | 883,46 | 31 |
| 685 | IYNSLIGLNEK | 17,37 | 632,35 | 673,39 | 33 |
| 686 | IYNSLIGLNEK | 17,37 | 632,35 | 786,47 | 33 |
| 687 | IYNSLIGLNEK | 17,37 | 632,35 | 987,55 | 33 |
| 688 | KPDIGWWVGWIER | 24,47 | 547,96 | 660,35 | 31 |
| 689 | KPDIGWWVGWIER | 24,47 | 547,96 | 883,45 | 31 |
| 690 | KPDIGWWVGWIER | 24,46 | 821,43 | 1188,59 | 41 |
| 691 | LAC[CAM]ATNNLAR | 11,22 | 552,28 | 688,37 | 29 |
| 692 | LAC[CAM]ATNNLAR | 11,22 | 552,28 | 759,41 | 29 |
| 693 | LAC[CAM]ATNNLAR | 11,22 | 552,28 | 919,44 | 29 |
| 694 | LAQGELPFPAPVQSTVR | 19,84 | 905,5 | 954,54 | 45 |
| 695 | LAQGELPFPAPVQSTVR | 19,84 | 905,5 | 1101,61 | 45 |
| 696 | LAQGELPFPAPVQSTVR | 19,84 | 905,5 | 1198,66 | 45 |
| 697 | LAQNELPYPIEIQK | 19,09 | 828,45 | 929,47 | 41 |
| 698 | LAQNELPYPIEIQK | 19,09 | 828,45 | 987,55 | 41 |
| 699 | LAQNELPYPIEIQK | 19,08 | 828,45 | 1100,64 | 41 |
| 700 | LAQNELQYPIEIQK | 17,98 | 843,96 | 890,5 | 42 |
| 701 | LAQNELQYPIEIQK | 17,98 | 843,96 | 1018,56 | 42 |
| 702 | LAQNELQYPIEIQK | 17,98 | 843,96 | 1131,64 | 42 |
| 703 | LDFGNK | 11,75 | 347,18 | 465,25 | 20 |
| 704 | LDFGNK | 11,74 | 347,18 | 547,25 | 20 |
| 705 | LDFGNK | 11,75 | 347,18 | 580,27 | 20 |
| 706 | LDGSLNR | 9,48 | 387,71 | 402,25 | 22 |
| 707 | LDGSLNR | 9,48 | 387,71 | 546,3 | 22 |
| 708 | LDGSLNR | 9,48 | 387,71 | 661,33 | 22 |
| 709 | LEILQQALAELGLYPK | 29,81 | 900,02 | 1003,58 | 45 |
| 710 | LEILQQALAELGLYPK | 29,81 | 900,02 | 1074,62 | 45 |
| 711 | LEILQQALAELGLYPK | 29,81 | 900,02 | 1202,68 | 45 |
| 712 | LENQEQVK | 7,6 | 494,26 | 631,34 | 27 |
| 713 | LENQEQVK | 7,59 | 494,26 | 745,38 | 27 |
| 714 | LENQEQVK | 7,59 | 494,26 | 874,43 | 27 |
| 715 | LETQEEVEK | 9,88 | 552,77 | 633,31 | 29 |
| 716 | LETQEEVEK | 9,88 | 552,77 | 862,42 | 29 |
| 717 | LETQEEVEK | 9,88 | 552,77 | 991,46 | 29 |
| 718 | LETQEEVK | 9,5 | 488,25 | 504,27 | 26 |
| 719 | LETQEEVK | 9,49 | 488,25 | 733,37 | 26 |
| 720 | LETQEEVK | 9,49 | 488,25 | 862,42 | 26 |
| 721 | LFAAEGVK | 13,53 | 417,74 | 503,28 | 23 |
| 722 | LFAAEGVK | 13,53 | 417,74 | 574,32 | 23 |
| 723 | LFAAEGVK | 13,53 | 417,74 | 721,39 | 23 |
| 724 | LFESAGVK | 12,99 | 425,74 | 461,27 | 24 |
| 725 | LFESAGVK | 12,99 | 425,74 | 590,31 | 24 |
| 726 | LFESAGVK | 12,99 | 425,74 | 737,38 | 24 |
| 727 | LFGAAGVK | 13,94 | 381,73 | 445,28 | 22 |
| 728 | LFGAAGVK | 13,94 | 381,73 | 502,3 | 22 |
| 729 | LFGAAGVK | 13,94 | 381,73 | 649,37 | 22 |
| 730 | LGVDR | 8,51 | 280,16 | 290,15 | 17 |
| 731 | LGVDR | 8,51 | 280,16 | 389,21 | 17 |
| 732 | LGVDR | 8,5 | 280,16 | 446,24 | 17 |
| 733 | LLNLLSQSK | 17,97 | 508,31 | 562,32 | 27 |
| 734 | LLNLLSQSK | 17,97 | 508,31 | 789,45 | 27 |
| 735 | LLNLLSQSK | 17,97 | 508,31 | 902,53 | 27 |
| 736 | LLQDER | 9,34 | 387,21 | 547,25 | 22 |
| 737 | LLQDER | 9,31 | 387,21 | 599,3 | 22 |
| 738 | LLQDER | 9,34 | 387,21 | 660,33 | 22 |
| 739 | LLVQDGDC[CAM]GR | 11,92 | 566,77 | 679,25 | 30 |
| 740 | LLVQDGDC[CAM]GR | 11,92 | 566,77 | 807,3 | 30 |
| 741 | LLVQDGDC[CAM]GR | 11,92 | 566,77 | 906,37 | 30 |
| 742 | LNEVGYGNR | 10,74 | 511,26 | 566,27 | 27 |
| 743 | LNEVGYGNR | 10,74 | 511,26 | 665,34 | 27 |
| 744 | LNEVGYGNR | 10,73 | 511,26 | 794,38 | 27 |
| 745 | LNYGNADPSTK | 10,76 | 590,29 | 732,35 | 31 |
| 746 | LNYGNADPSTK | 10,76 | 590,29 | 789,37 | 31 |
| 747 | LNYGNADPSTK | 10,76 | 590,29 | 952,44 | 31 |
| 748 | LNYGNK | 7,21 | 354,69 | 481,24 | 21 |
| 749 | LNYGNK | 7,24 | 354,69 | 562,26 | 21 |
| 750 | LNYGNK | 7,22 | 354,69 | 595,28 | 21 |
| 751 | LPASK | 1,93 | 258,16 | 305,18 | 16 |
| 752 | LPASK | 1,93 | 258,16 | 369,21 | 16 |
| 753 | LPASK | 1,93 | 258,16 | 402,23 | 16 |
| 754 | LPHTLFALDADAVR | 19,98 | 769,92 | 977,51 | 39 |
| 755 | LPHTLFALDADAVR | 19,98 | 769,92 | 1090,59 | 39 |
| 756 | LPHTLFALDADAVR | 19,98 | 769,92 | 1191,64 | 39 |
| 757 | LPHTLFALDAGAVR | 19,7 | 740,92 | 919,5 | 38 |
| 758 | LPHTLFALDAGAVR | 19,67 | 740,92 | 1032,58 | 38 |
| 759 | LPHTLFALDAGAVR | 19,7 | 740,92 | 1133,63 | 38 |
| 760 | LPLAIMGFDSGILQSPK | 25,08 | 893,99 | 944,5 | 44 |
| 761 | LPLAIMGFDSGILQSPK | 25,08 | 893,99 | 1091,57 | 44 |
| 762 | LPLAIMGFDSGILQSPK | 25,08 | 893,99 | 1148,59 | 44 |
| 763 | LPLAIMGYDADILLDATTPR | 27,86 | 720,39 | 773,42 | 40 |
| 764 | LPLAIMGYDADILLDATTPR | 27,87 | 720,39 | 886,5 | 40 |
| 765 | LPLAIMGYDADILLDATTPR | 27,87 | 720,39 | 1160,57 | 40 |
| 766 | LPSSLIALETGAVR | 20,6 | 713,92 | 816,46 | 36 |
| 767 | LPSSLIALETGAVR | 20,6 | 713,92 | 929,54 | 36 |
| 768 | LPSSLIALETGAVR | 20,6 | 713,92 | 1216,69 | 36 |
| 769 | LPVSAQTLQYTANILK | 21,84 | 880,5 | 950,53 | 44 |
| 770 | LPVSAQTLQYTANILK | 21,84 | 880,5 | 1063,61 | 44 |
| 771 | LPVSAQTLQYTANILK | 21,85 | 880,5 | 1164,66 | 44 |
| 772 | LPVSER | 9,57 | 350,7 | 490,26 | 20 |
| 773 | LPVSER | 9,57 | 350,7 | 526,29 | 20 |
| 774 | LPVSER | 9,57 | 350,7 | 587,31 | 20 |
| 775 | LPVSPTAVDMTER | 16,21 | 708,36 | 1019,48 | 36 |
| 776 | LPVSPTAVDMTER | 16,21 | 708,36 | 1106,51 | 36 |
| 777 | LPVSPTAVDMTER | 16,21 | 708,36 | 1205,58 | 36 |
| 778 | LSASK | 10,72 | 253,15 | 305,18 | 16 |
| 779 | LSASK | 10,71 | 253,15 | 359,19 | 16 |
| 780 | LSASK | 10,71 | 253,15 | 392,21 | 16 |
| 781 | LSAVPIYQEVAR | 17,96 | 673,38 | 765,39 | 35 |
| 782 | LSAVPIYQEVAR | 17,96 | 673,38 | 975,53 | 35 |
| 783 | LSAVPIYQEVAR | 17,95 | 673,38 | 1074,59 | 35 |
| 784 | LSAVPVYQELAR | 18,45 | 449,25 | 616,34 | 26 |
| 785 | LSAVPVYQELAR | 18,44 | 673,38 | 779,4 | 35 |
| 786 | LSAVPVYQELAR | 18,44 | 673,38 | 975,53 | 35 |
| 787 | LSC[CAM]TLVIDEASGDLLHR | 20,38 | 633,66 | 797,43 | 36 |
| 788 | LSC[CAM]TLVIDEASGDLLHR | 20,38 | 633,66 | 868,46 | 36 |
| 789 | LSC[CAM]TLVIDEASGDLLHR | 20,38 | 633,66 | 1112,53 | 36 |
| 790 | LSLQHGWFIGWIEK | 23,95 | 571,98 | 632,34 | 33 |
| 791 | LSLQHGWFIGWIEK | 23,95 | 571,98 | 892,49 | 33 |
| 792 | LSLQHGWFIGWIEK | 23,95 | 571,98 | 969,49 | 33 |
| 793 | LSQNSLPFSQEAMNSVK | 18,64 | 627,31 | 1140,54 | 35 |
| 794 | LSQNSLPFSQEAMNSVK | 18,63 | 940,46 | 1140,54 | 46 |
| 795 | LSQNSLPFSQEAMNSVK | 18,64 | 940,46 | 1237,59 | 46 |
| 796 | LSVNPK | 9,8 | 329,2 | 457,28 | 19 |
| 797 | LSVNPK | 9,79 | 329,2 | 511,29 | 19 |
| 798 | LSVNPK | 9,8 | 329,2 | 544,31 | 19 |
| 799 | LTVGAR | 9,51 | 308,69 | 402,25 | 19 |
| 800 | LTVGAR | 9,51 | 308,69 | 442,27 | 19 |
| 801 | LTVGAR | 9,51 | 308,69 | 503,29 | 19 |
| 802 | LYGFALNIDMPGGEADIGK | 23,35 | 661 | 843,42 | 37 |
| 803 | LYGFALNIDMPGGEADIGK | 23,35 | 990,99 | 1089,49 | 49 |
| 804 | LYGFALNIDMPGGEADIGK | 23,35 | 990,99 | 1202,57 | 49 |
| 805 | LYHNELPFR | 15,29 | 396,88 | 414,21 | 24 |
| 806 | LYHNELPFR | 15,29 | 396,88 | 419,24 | 24 |
| 807 | LYHNELPFR | 15,29 | 396,88 | 657,3 | 24 |
| 808 | LYHNK | 8,54 | 337,68 | 414,21 | 20 |
| 809 | LYHNK | 8,53 | 337,68 | 528,26 | 20 |
| 810 | LYHNK | 8,53 | 337,68 | 561,28 | 20 |
| 811 | LYQNDLPFR | 17,2 | 583,3 | 761,39 | 31 |
| 812 | LYQNDLPFR | 17,2 | 583,3 | 889,45 | 31 |
| 813 | LYQNDLPFR | 17,2 | 583,3 | 1052,52 | 31 |
| 814 | MDDLFK | 15,5 | 384,68 | 522,29 | 22 |
| 815 | MDDLFK | 15,5 | 384,68 | 622,25 | 22 |
| 816 | MDDLFK | 15,5 | 384,68 | 637,32 | 22 |
| 817 | MEDLHK | 6,66 | 386,69 | 512,28 | 22 |
| 818 | MEDLHK | 6,65 | 386,69 | 626,26 | 22 |
| 819 | MEDLHK | 6,66 | 386,69 | 641,33 | 22 |
| 820 | MLIALIGLENHK | 21,33 | 451,26 | 527,26 | 27 |
| 821 | MLIALIGLENHK | 21,33 | 451,26 | 697,36 | 27 |
| 822 | MLIALIGLENHK | 21,33 | 451,26 | 810,45 | 27 |
| 823 | MLLIK | 15,81 | 309,21 | 373,28 | 19 |
| 824 | MLLIK | 15,81 | 309,21 | 471,3 | 19 |
| 825 | MLLIK | 15,81 | 309,21 | 486,36 | 19 |
| 826 | MLNALIGLEHHK | 16,89 | 459,26 | 550,27 | 27 |
| 827 | MLNALIGLEHHK | 16,89 | 459,26 | 720,38 | 27 |
| 828 | MLNALIGLEHHK | 16,89 | 459,26 | 833,46 | 27 |
| 829 | MLNALIGLENHK | 18,39 | 451,58 | 697,36 | 27 |
| 830 | MLNALIGLENHK | 18,38 | 676,87 | 697,36 | 35 |
| 831 | MLNALIGLENHK | 18,39 | 676,87 | 810,45 | 35 |
| 832 | MLNALIGLENQK | 19,71 | 672,37 | 688,36 | 35 |
| 833 | MLNALIGLENQK | 19,71 | 672,37 | 801,45 | 35 |
| 834 | MLNALIGLENQK | 19,71 | 672,37 | 914,53 | 35 |
| 835 | MLNALIGLEYHK | 19,6 | 701,38 | 746,38 | 36 |
| 836 | MLNALIGLEYHK | 19,6 | 701,38 | 859,47 | 36 |
| 837 | MLNALIGLEYHK | 19,6 | 701,38 | 1157,63 | 36 |
| 838 | MLNALIGLQHGK | 17,5 | 432,25 | 582,34 | 26 |
| 839 | MLNALIGLQHGK | 17,5 | 432,25 | 639,36 | 26 |
| 840 | MLNALIGLQHGK | 17,5 | 432,25 | 752,44 | 26 |
| 841 | MLNALISLEHHK | 17,2 | 352,2 | 359,17 | 21 |
| 842 | MLNALISLEHHK | 17,21 | 469,26 | 750,39 | 27 |
| 843 | MLNALISLEHHK | 17,2 | 469,26 | 863,47 | 27 |
| 844 | MQAYVDAFDYGNR | 17,56 | 775,34 | 957,41 | 39 |
| 845 | MQA YVDAFDYGNR | 17,56 | 775,34 | 1056,47 | 39 |
| 846 | MQA YVDAFDYGNR | 17,56 | 775,34 | 1219,54 | 39 |
| 847 | MQEGLNK | 8,68 | 410,21 | 560,3 | 23 |
| 848 | MQEGLNK | 8,66 | 410,21 | 673,3 | 23 |
| 849 | MQEGLNK | 8,68 | 410,21 | 688,36 | 23 |
| 850 | MSPASTYK | 9,49 | 442,71 | 569,29 | 24 |
| 851 | MSPASTYK | 9,49 | 442,71 | 666,35 | 24 |
| 852 | MSPASTYK | 9,49 | 442,71 | 753,38 | 24 |
| 853 | NEHDPVLPYR | 13,09 | 413,88 | 435,24 | 25 |
| 854 | NEHDPVLPYR | 13,09 | 620,31 | 744,44 | 32 |
| 855 | NEHDPVLPYR | 13,09 | 620,31 | 859,47 | 32 |
| 856 | NEHQIFK | 9,91 | 458,24 | 509,21 | 25 |
| 857 | NEHQIFK | 9,91 | 458,24 | 622,29 | 25 |
| 858 | NEHQIFK | 9,91 | 458,24 | 672,38 | 25 |
| 859 | NEHQVFK | 7,74 | 451,23 | 658,37 | 25 |
| 860 | NEHQVFK | 7,74 | 451,23 | 755,35 | 25 |
| 861 | NEHQVFK | 7,74 | 451,23 | 787,41 | 25 |
| 862 | NEITYK | 9,35 | 384,2 | 524,31 | 22 |
| 863 | NEITYK | 9,35 | 384,2 | 621,29 | 22 |
| 864 | NEITYK | 9,35 | 384,2 | 653,35 | 22 |
| 865 | NELLMK | 13,08 | 374,21 | 504,32 | 21 |
| 866 | NELLMK | 13,09 | 374,21 | 601,3 | 21 |
| 867 | NELLMK | 13,09 | 374,21 | 633,36 | 21 |
| 868 | NELPFR | 14,39 | 388,21 | 419,24 | 22 |
| 869 | NELPFR | 14,39 | 388,21 | 532,32 | 22 |
| 870 | NELPFR | 14,4 | 388,21 | 661,37 | 22 |
| 871 | NISSYGNNLVR | 14,36 | 618,82 | 835,44 | 32 |
| 872 | NISSYGNNLVR | 14,36 | 618,82 | 922,47 | 32 |
| 873 | NISSYGNNLVR | 14,36 | 618,82 | 1009,51 | 32 |
| 874 | NISTYGNNLTR | 13,1 | 626,82 | 674,36 | 33 |
| 875 | NISTYGNNLTR | 13,09 | 626,82 | 837,42 | 33 |
| 876 | NISTYGNNLTR | 13,1 | 626,82 | 1025,5 | 33 |
| 877 | NLFNEVHTTGVLVIR | 20,69 | 571,32 | 757,49 | 33 |
| 878 | NLFNEVHTTGVLVIR | 20,7 | 571,32 | 858,54 | 33 |
| 879 | NLFNEVHTTGVLVIR | 20,7 | 571,32 | 995,6 | 33 |
| 880 | NLSTYGNALAR | 14,34 | 590,31 | 764,4 | 31 |
| 881 | NLSTYGNALAR | 14,35 | 590,31 | 865,45 | 31 |
| 882 | NLSTYGNALAR | 14,35 | 590,31 | 952,48 | 31 |
| 883 | NMENLELFGK | 19,08 | 597,79 | 820,46 | 31 |
| 884 | NMENLELFGK | 19,08 | 597,79 | 949,5 | 31 |
| 885 | NMENLELFGK | 19,08 | 597,79 | 1080,54 | 31 |
| 886 | NMLLLEENNGYK | 16,71 | 719,36 | 853,37 | 37 |
| 887 | NMLLLEENNGYK | 16,69 | 719,36 | 966,45 | 37 |
| 888 | NMLLLEENNGYK | 16,68 | 719,36 | 1079,54 | 37 |
| 889 | NMLLLEESNGYK | 18,12 | 705,85 | 939,44 | 36 |
| 890 | NMLLLEESNGYK | 18,13 | 705,85 | 1052,53 | 36 |
| 891 | NMLLLEESNGYK | 18,11 | 705,85 | 1165,61 | 36 |
| 892 | NMLLLEK | 16,99 | 430,75 | 502,32 | 24 |
| 893 | NMLLLEK | 16,98 | 430,75 | 615,41 | 24 |
| 894 | NMLLLEK | 16,98 | 430,75 | 746,45 | 24 |
| 895 | NMTLGDAMK | 14,42 | 490,73 | 521,24 | 27 |
| 896 | NMTLGDAMK | 14,42 | 490,73 | 634,32 | 27 |
| 897 | NMTLGDAMK | 14,42 | 490,73 | 735,37 | 27 |
| 898 | NNGL TEAWLESSLK | 20,61 | 781,4 | 862,47 | 39 |
| 899 | NNGL TEAWLESSLK | 20,6 | 781,4 | 933,5 | 39 |
| 900 | NNGL TEAWLESSLK | 20,62 | 781,4 | 1163,59 | 39 |
| 901 | NQLPFK | 13,49 | 373,71 | 391,23 | 21 |
| 902 | NQLPFK | 13,49 | 373,71 | 504,32 | 21 |
| 903 | NQLPFK | 13,49 | 373,71 | 632,38 | 21 |
| 904 | NQLPFQVEHQR | 14,33 | 698,36 | 796,41 | 36 |
| 905 | NQLPFQVEHQR | 14,33 | 698,36 | 1040,53 | 36 |
| 906 | NQLPFQVEHQR | 14,33 | 698,36 | 1153,61 | 36 |
| 907 | NSAIENTIDNMYLQDLENSTK | 22,77 | 805,04 | 934,45 | 44 |
| 908 | NSAIENTIDNMYLQDLENSTK | 22,77 | 805,04 | 1047,53 | 44 |
| 909 | NSAIENTIDNMYLQDLENSTK | 22,77 | 805,04 | 1210,6 | 44 |
| 910 | NSAIENTIENMYLQDLDNSTK | 23,13 | 805,04 | 920,43 | 44 |
| 911 | NSAIENTIENMYLQDLDNSTK | 23,13 | 805,04 | 1033,52 | 44 |
| 912 | NSAIENTIENMYLQDLDNSTK | 23,14 | 805,04 | 1196,58 | 44 |
| 913 | NSAIENTIENMYLQDLENSTK | 23,7 | 809,72 | 934,45 | 44 |
| 914 | NSAIENTIENMYLQDLENSTK | 23,7 | 809,72 | 1047,53 | 44 |
| 915 | NSAIENTIENMYLQDLENSTK | 23,7 | 809,72 | 1217,55 | 44 |
| 916 | NSAVWVYELFAK | 24,66 | 713,87 | 869,48 | 36 |
| 917 | NSAVWVYELFAK | 24,66 | 713,87 | 1055,56 | 36 |
| 918 | NSAVWVYELFAK | 24,65 | 713,87 | 1154,62 | 36 |
| 919 | NSQVPAYK | 9,78 | 453,74 | 478,27 | 25 |
| 920 | NSQVPAYK | 9,78 | 453,74 | 577,33 | 25 |
| 921 | NSQVPAYK | 9,78 | 453,74 | 705,39 | 25 |
| 922 | NSTVWIYELFAK | 25,64 | 735,88 | 883,49 | 37 |
| 923 | NSTVWIYELFAK | 25,64 | 735,88 | 1069,57 | 37 |
| 924 | NSTVWIYELFAK | 25,64 | 735,88 | 1168,64 | 37 |
| 925 | NSTVWVYELFAK | 24,42 | 728,88 | 770,41 | 37 |
| 926 | NSTVWVYELFAK | 24,43 | 728,88 | 869,48 | 37 |
| 927 | NSTVWVYELFAK | 24,42 | 728,88 | 1055,56 | 37 |
| 928 | NSTVWVYQLFAK | 23,9 | 728,39 | 769,42 | 37 |
| 929 | NSTVWVYQLFAK | 23,91 | 728,39 | 1054,57 | 37 |
| 930 | NSTVWVYQLFAK | 23,91 | 728,39 | 1153,64 | 37 |
| 931 | NTSGALVIQTDK | 13,34 | 623,84 | 816,48 | 32 |
| 932 | NTSGALVIQTDK | 13,34 | 623,84 | 944,54 | 32 |
| 933 | NTSGALVIQTDK | 13,34 | 623,84 | 1031,57 | 32 |
| 934 | NTSGVLVIQTDK | 14,9 | 637,85 | 816,48 | 33 |
| 935 | NTSGVLVIQTDK | 14,9 | 637,85 | 972,57 | 33 |
| 936 | NTSGVLVIQTDK | 14,91 | 637,85 | 1059,6 | 33 |
| 937 | NVDEMFYYYDGSK | 18,86 | 815,84 | 895,38 | 41 |
| 938 | NVDEMFYYYDGSK | 18,86 | 815,84 | 1042,45 | 41 |
| 939 | NVDEMFYYYDGSK | 18,85 | 815,84 | 1173,49 | 41 |
| 940 | NWILR | 16,3 | 351,21 | 414,21 | 20 |
| 941 | NWILR | 16,29 | 351,21 | 527,3 | 20 |
| 942 | NWILR | 16,3 | 351,21 | 587,37 | 20 |
| 943 | NWNAAMDLR | 16,54 | 545,76 | 605,31 | 29 |
| 944 | NWNAAMDLR | 16,55 | 545,76 | 676,34 | 29 |
| 945 | NWNAAMDLR | 16,54 | 545,76 | 790,39 | 29 |
| 946 | NYVDAFHYGNQDISGDK | 15,76 | 648,29 | 933,43 | 36 |
| 947 | NYVDAFHYGNQDISGDK | 15,77 | 648,29 | 1096,49 | 36 |
| 948 | NYVDAFHYGNQDISGDK | 15,76 | 971,93 | 1233,55 | 48 |
| 949 | QADHAILVFDQAR | 16,58 | 495,26 | 523,23 | 29 |
| 950 | QADHAILVFDQAR | 16,61 | 495,26 | 636,31 | 29 |
| 951 | QADHAILVFDQAR | 16,58 | 495,26 | 735,38 | 29 |
| 952 | QAEHALLVFGQER | 16,86 | 499,93 | 636,31 | 29 |
| 953 | QAEHALLVFGQER | 16,85 | 499,93 | 735,38 | 29 |
| 954 | QAEHALLVFGQER | 16,87 | 499,93 | 763,41 | 29 |
| 955 | QAITK | 11 | 280,67 | 361,24 | 17 |
| 956 | QAITK | 11 | 280,67 | 414,23 | 17 |
| 957 | QAITK | 11,01 | 280,67 | 432,28 | 17 |
| 958 | QAMLTEANSDYIIR | 18,26 | 812,9 | 951,49 | 41 |
| 959 | QAMLTEANSDYIIR | 18,25 | 812,9 | 1080,53 | 41 |
| 960 | QAMLTEANSDYIIR | 18,26 | 812,9 | 1181,58 | 41 |
| 961 | QEVQFVSALAR | 17,69 | 624,34 | 763,45 | 32 |
| 962 | QEVQFVSALAR | 17,68 | 624,34 | 891,5 | 32 |
| 963 | QEVQFVSALAR | 17,68 | 624,34 | 990,57 | 32 |
| 964 | QFASIK | 11,66 | 347,2 | 434,2 | 20 |
| 965 | QFASIK | 11,66 | 347,2 | 547,29 | 20 |
| 966 | QFASIK | 11,68 | 347,2 | 565,33 | 20 |
| 967 | QGMPGSIR | 11,4 | 423,22 | 529,31 | 24 |
| 968 | QGMPGSIR | 11,43 | 423,22 | 660,35 | 24 |
| 969 | QGMPGSIR | 11,4 | 423,22 | 717,37 | 24 |
| 970 | QGMSGSIR | 9,44 | 418,21 | 519,29 | 23 |
| 971 | QGMSGSIR | 9,45 | 418,21 | 650,33 | 23 |
| 972 | QGMSGSIR | 9,44 | 418,21 | 707,35 | 23 |
| 973 | QGQTQQSYGNDLAR | 11,16 | 783,37 | 895,43 | 39 |
| 974 | QGQTQQSYGNDLAR | 11,17 | 783,37 | 1023,49 | 39 |
| 975 | QGQTQQSYGNDLAR | 11,16 | 783,37 | 1151,54 | 39 |
| 976 | QIDYGNADPSTIK | 13,41 | 711,35 | 845,44 | 36 |
| 977 | QIDYGNADPSTIK | 13,42 | 711,35 | 902,46 | 36 |
| 978 | QIDYGNADPSTIK | 13,42 | 711,35 | 1065,52 | 36 |
| 979 | QIDYGNVDPSTIK | 15,08 | 725,36 | 873,47 | 37 |
| 980 | QIDYGNVDPSTIK | 15,07 | 725,36 | 930,49 | 37 |
| 981 | QIDYGNVDPSTIK | 15,07 | 725,36 | 1093,55 | 37 |
| 982 | QIGQAR | 2,3 | 336,69 | 431,24 | 20 |
| 983 | QIGQAR | 2,33 | 336,69 | 498,27 | 20 |
| 984 | QIGQAR | 2,32 | 336,69 | 544,32 | 20 |
| 985 | QIMLIEQTPAFTLR | 24,42 | 830,96 | 933,52 | 42 |
| 986 | QIMLIEQTPAFTLR | 24,42 | 830,96 | 1062,56 | 42 |
| 987 | QIMLIEQTPAFTLR | 24,42 | 830,96 | 1175,64 | 42 |
| 988 | QLGSAIDQFWLR | 22,67 | 717,38 | 864,44 | 37 |
| 989 | QLGSAIDQFWLR | 22,68 | 717,38 | 977,52 | 37 |
| 990 | QLGSAIDQFWLR | 22,67 | 717,38 | 1192,61 | 37 |
| 991 | QLPVK | 9,57 | 292,69 | 343,23 | 18 |
| 992 | QLPVK | 9,58 | 292,69 | 438,27 | 18 |
| 993 | QLPVK | 9,57 | 292,69 | 456,32 | 18 |
| 994 | QLSLDVLDK | 18,63 | 515,79 | 589,32 | 28 |
| 995 | QLSLDVLDK | 18,62 | 515,79 | 789,44 | 28 |
| 996 | QLSLDVLDK | 18,63 | 515,79 | 902,52 | 28 |
| 997 | QLVYAR | 11,04 | 375,22 | 508,29 | 22 |
| 998 | QLVYAR | 11,04 | 375,22 | 575,32 | 22 |
| 999 | QLVYAR | 11,04 | 375,22 | 621,37 | 22 |
| 1000 | QMMLTEASTDYIIR | 19,82 | 836,41 | 867,46 | 42 |
| 1001 | QMMLTEASTDYIIR | 19,82 | 836,41 | 1067,54 | 42 |
| 1002 | QMMLTEASTDYIIR | 19,82 | 836,41 | 1168,58 | 42 |
| 1003 | QMSIVEATPDYVLHGK | 18,77 | 894,45 | 1029,54 | 44 |
| 1004 | QMSIVEATPDYVLHGK | 18,77 | 894,45 | 1100,57 | 44 |
| 1005 | QMSIVEATPDYVLHGK | 18,77 | 894,45 | 1229,62 | 44 |
| 1006 | QTLVFAR | 14,65 | 417,75 | 492,29 | 23 |
| 1007 | QTLVFAR | 14,65 | 417,75 | 605,38 | 23 |
| 1008 | QTLVFAR | 14,65 | 417,75 | 706,42 | 23 |
| 1009 | QVVFAR | 12,06 | 360,21 | 492,29 | 21 |
| 1010 | QVVFAR | 12,04 | 360,21 | 545,31 | 21 |
| 1011 | QVVFAR | 12,06 | 360,21 | 591,36 | 21 |
| 1012 | SADEVLPYGGKPQR | 12,96 | 506,26 | 642,37 | 29 |
| 1013 | SADEVLPYGGKPQR | 12,96 | 506,26 | 805,43 | 29 |
| 1014 | SADEVLPYGGKPQR | 12,96 | 506,26 | 902,48 | 29 |
| 1015 | SC[CAM]ATNDLAR | 9,37 | 504,23 | 689,36 | 27 |
| 1016 | SC[CAM]ATNDLAR | 9,37 | 504,23 | 760,39 | 27 |
| 1017 | SC[CAM]ATNDLAR | 9,37 | 504,23 | 920,43 | 27 |
| 1018 | SC[CAM]ATNNLAR | 8,66 | 503,74 | 688,37 | 27 |
| 1019 | SC[CAM]ATNNLAR | 8,66 | 503,74 | 759,41 | 27 |
| 1020 | SC[CAM]ATNNLAR | 8,67 | 503,74 | 919,44 | 27 |
| 1021 | SDIPGGSK | 7,63 | 380,7 | 558,32 | 22 |
| 1022 | SDIPGGSK | 7,63 | 380,7 | 614,28 | 22 |
| 1023 | SDIPGGSK | 7,63 | 380,7 | 673,35 | 22 |
| 1024 | SDWGK | 5,75 | 296,64 | 390,21 | 18 |
| 1025 | SDWGK | 5,75 | 296,64 | 446,17 | 18 |
| 1026 | SDWGK | 5,75 | 296,64 | 505,24 | 18 |
| 1027 | SEDNFHISSQQHEK | 10,36 | 422,19 | 541,27 | 24 |
| 1028 | SEDNFHISSQQHEK | 10,36 | 422,19 | 730,28 | 24 |
| 1029 | SEDNFHISSQQHEK | 10,36 | 422,19 | 756,36 | 24 |
| 1030 | SEMPASIR | 12,02 | 445,72 | 674,37 | 25 |
| 1031 | SEMPASIR | 12,02 | 445,72 | 716,33 | 25 |
| 1032 | SEMPASIR | 12,02 | 445,72 | 803,41 | 25 |
| 1033 | SEMPASTR | 8,2 | 439,71 | 662,33 | 24 |
| 1034 | SEMPASTR | 8,19 | 439,71 | 704,29 | 24 |
| 1035 | SEMPASTR | 8,19 | 439,71 | 791,37 | 24 |
| 1036 | SFAAHNQDQDLR | 10,35 | 467,89 | 531,29 | 27 |
| 1037 | SFAAHNQDQDLR | 10,35 | 467,89 | 871,37 | 27 |
| 1038 | SFAAHNQDQDLR | 10,35 | 467,89 | 888,42 | 27 |
| 1039 | SFAGHNK | 9,4 | 380,69 | 455,24 | 22 |
| 1040 | SFAGHNK | 9,4 | 380,69 | 526,27 | 22 |
| 1041 | SFAGHNK | 9,38 | 380,69 | 673,34 | 22 |
| 1042 | SFAGHNQDQDLR | 10,18 | 694,32 | 888,42 | 36 |
| 1043 | SFAGHNQDQDLR | 10,18 | 694,32 | 1025,48 | 36 |
| 1044 | SFAGHNQDQDLR | 10,18 | 694,32 | 1082,5 | 36 |
| 1045 | SFAGHNQDQNLR | 9,8 | 462,89 | 530,3 | 27 |
| 1046 | SFAGHNQDQNLR | 9,8 | 462,89 | 773,39 | 27 |
| 1047 | SFAGHNQDQNLR | 9,8 | 462,89 | 887,43 | 27 |
| 1048 | SFLESWAK | 18,27 | 484,25 | 491,26 | 26 |
| 1049 | SFLESWAK | 18,27 | 484,25 | 620,3 | 26 |
| 1050 | SFLESWAK | 18,27 | 484,25 | 733,39 | 26 |
| 1051 | SFTAWEK | 14,44 | 434,71 | 462,23 | 24 |
| 1052 | SFTAWEK | 14,44 | 434,71 | 533,27 | 24 |
| 1053 | SFTAWEK | 14,44 | 434,71 | 634,32 | 24 |
| 1054 | SFTTWEK | 14,1 | 449,72 | 462,23 | 25 |
| 1055 | SFTTWEK | 14,1 | 449,72 | 563,28 | 25 |
| 1056 | SFTTWEK | 14,1 | 449,72 | 664,33 | 25 |
| 1057 | SGSGWLR | 13,25 | 381,7 | 531,3 | 22 |
| 1058 | SGSGWLR | 13,25 | 381,7 | 618,34 | 22 |
| 1059 | SGSGWLR | 13,25 | 381,7 | 675,36 | 22 |
| 1060 | SGWGMAVDPQVGWYVGFVEK | 24,65 | 738,02 | 841,45 | 41 |
| 1061 | SGWGMAVDPQVGWYVGFVEK | 24,65 | 738,02 | 1029,45 | 41 |
| 1062 | SGWGMAVDPQVGWYVGFVEK | 24,68 | 1106,53 | 1128,51 | 54 |
| 1063 | SGWGMDVSPQVGWLTGWVEK | 26,32 | 1110,03 | 1144,51 | 54 |
| 1064 | SGWGMDVSPQVGWLTGWVEK | 26,32 | 1110,03 | 1174,63 | 54 |
| 1065 | SGWGMDVSPQVGWLTGWVEK | 26,32 | 1110,03 | 1201,53 | 54 |
| 1066 | SGWGMDVTPQVGWLTGWVEK | 26,61 | 745,03 | 832,46 | 41 |
| 1067 | SGWGMDVTPQVGWLTGWVEK | 26,61 | 745,03 | 1018,54 | 41 |
| 1068 | SGWGMDVTPQVGWLTGWVEK | 26,61 | 745,03 | 1075,56 | 41 |
| 1069 | SIHPASTFK | 10,74 | 494,27 | 650,35 | 27 |
| 1070 | SIHPASTFK | 10,73 | 494,27 | 787,41 | 27 |
| 1071 | SIHPASTFK | 10,73 | 494,27 | 900,49 | 27 |
| 1072 | SISTK | 10,41 | 268,16 | 335,19 | 17 |
| 1073 | SISTK | 10,42 | 268,16 | 389,2 | 17 |
| 1074 | SISTK | 10,42 | 268,16 | 448,28 | 17 |
| 1075 | SLGLSNNLSR | 14,23 | 530,79 | 690,35 | 28 |
| 1076 | SLGLSNNLSR | 14,23 | 530,79 | 803,44 | 28 |
| 1077 | SLGLSNNLSR | 14,23 | 530,79 | 860,46 | 28 |
| 1078 | SLSMSGK | 9,31 | 355,18 | 509,24 | 21 |
| 1079 | SLSMSGK | 9,32 | 355,18 | 563,25 | 21 |
| 1080 | SLSMSGK | 9,32 | 355,18 | 622,32 | 21 |
| 1081 | SMLFIEEK | 17,82 | 498,76 | 518,28 | 27 |
| 1082 | SMLFIEEK | 17,82 | 498,76 | 665,35 | 27 |
| 1083 | SMLFIEEK | 17,82 | 498,76 | 778,43 | 27 |
| 1084 | SNGLTHSWLGSSLK | 16,78 | 743,89 | 877,48 | 38 |
| 1085 | SNGLTHSWLGSSLK | 16,78 | 743,89 | 1014,54 | 38 |
| 1086 | SNGLTHSWLGSSLK | 16,78 | 743,89 | 1115,58 | 38 |
| 1087 | SPTWELKPEYNPSPR | 16,02 | 600,97 | 733,36 | 34 |
| 1088 | SPTWELKPEYNPSPR | 16,02 | 600,97 | 808,91 | 34 |
| 1089 | SPTWELKPEYNPSPR | 16,02 | 600,97 | 959,46 | 34 |
| 1090 | SQDIVR | 8,4 | 359,2 | 502,3 | 21 |
| 1091 | SQDIVR | 8,38 | 359,2 | 543,28 | 21 |
| 1092 | SQDIVR | 8,4 | 359,2 | 630,36 | 21 |
| 1093 | SQQKPTDPTIWLK | 16,6 | 514,62 | 660,41 | 30 |
| 1094 | SQQKPTDPTIWLK | 16,6 | 514,62 | 757,46 | 30 |
| 1095 | SQQKPTDPTIWLK | 16,6 | 514,62 | 785,38 | 30 |
| 1096 | SQVGWLTGWVEQPDGK | 22,27 | 893,94 | 1015,5 | 44 |
| 1097 | SQVGWLTGWVEQPDGK | 22,28 | 893,94 | 1116,53 | 44 |
| 1098 | SQVGWLTGWVEQPDGK | 22,28 | 893,94 | 1229,62 | 44 |
| 1099 | SSSNSC[CAM]TTNNAAR | 16,84 | 685,29 | 907,41 | 35 |
| 1100 | SSSNSC[CAM]TTNNAAR | 16,85 | 685,29 | 994,44 | 35 |
| 1101 | SSSNSC[CAM]TTNNAAR | 16,84 | 685,29 | 1108,48 | 35 |
| 1102 | SVYGELR | 12,65 | 412,22 | 417,25 | 23 |
| 1103 | SVYGELR | 12,65 | 412,22 | 474,27 | 23 |
| 1104 | SVYGELR | 12,65 | 412,22 | 637,33 | 23 |
| 1105 | SWILR | 16,33 | 337,7 | 401,29 | 20 |
| 1106 | SWILR | 16,32 | 337,7 | 500,29 | 20 |
| 1107 | SWILR | 16,33 | 337,7 | 587,37 | 20 |
| 1108 | SYLEK | 9,09 | 320,17 | 389,24 | 19 |
| 1109 | SYLEK | 9,09 | 320,17 | 493,23 | 19 |
| 1110 | SYLEK | 9,1 | 320,17 | 552,3 | 19 |
| 1111 | TAYIPASTFK | 15,43 | 549,8 | 650,35 | 29 |
| 1112 | TAYIPASTFK | 15,43 | 549,8 | 763,43 | 29 |
| 1113 | TAYIPASTFK | 15,43 | 549,8 | 926,5 | 29 |
| 1114 | TDDLFK | 13,48 | 369,69 | 407,27 | 21 |
| 1115 | TDDLFK | 13,48 | 369,69 | 522,29 | 21 |
| 1116 | TDDLFK | 13,48 | 369,69 | 637,32 | 21 |
| 1117 | TDINEIFK | 17,44 | 490,26 | 650,35 | 27 |
| 1118 | TDINEIFK | 17,44 | 490,26 | 763,43 | 27 |
| 1119 | TDINEIFK | 17,44 | 490,26 | 878,46 | 27 |
| 1120 | TFIHNDPR | 18,92 | 500,25 | 751,38 | 27 |
| 1121 | TFIHNDPR | 18,92 | 500,25 | 825,39 | 27 |
| 1122 | TFIHNDPR | 18,92 | 500,25 | 898,45 | 27 |
| 1123 | TGAGFTANR | 9,64 | 447,72 | 461,25 | 25 |
| 1124 | TGAGFTANR | 9,64 | 447,72 | 665,34 | 25 |
| 1125 | TGAGFTANR | 9,64 | 447,72 | 793,4 | 25 |
| 1126 | TGFNDGQK | 7,5 | 433,7 | 561,26 | 24 |
| 1127 | TGFNDGQK | 7,5 | 433,7 | 708,33 | 24 |
| 1128 | TGFNDGQK | 7,5 | 433,7 | 765,35 | 24 |
| 1129 | TGLADSK | 9,7 | 346,18 | 533,29 | 20 |
| 1130 | TGLADSK | 9,67 | 346,18 | 545,26 | 20 |
| 1131 | TGLADSK | 9,7 | 346,18 | 590,31 | 20 |
| 1132 | TGLDLMQK | 15,32 | 453,24 | 634,32 | 25 |
| 1133 | TGLDLMQK | 15,32 | 453,24 | 747,41 | 25 |
| 1134 | TGLDLMQK | 15,32 | 453,24 | 804,43 | 25 |
| 1135 | TGLELMQK | 15,03 | 460,25 | 648,34 | 25 |
| 1136 | TGLELMQK | 15,03 | 460,25 | 761,42 | 25 |
| 1137 | TGLELMQK | 15,03 | 460,25 | 818,44 | 25 |
| 1138 | TGMGYPK | 10,28 | 377,18 | 464,25 | 22 |
| 1139 | TGMGYPK | 10,28 | 377,18 | 595,29 | 22 |
| 1140 | TGMGYPK | 10,28 | 377,18 | 652,31 | 22 |
| 1141 | TGNGR | 0,8 | 252,63 | 330,14 | 16 |
| 1142 | TGNGR | 0,8 | 252,63 | 346,18 | 16 |
| 1143 | TGNGR | 0,81 | 252,63 | 403,2 | 16 |
| 1144 | TGTGSFIDAR | 13,35 | 512,76 | 708,37 | 28 |
| 1145 | TGTGSFIDAR | 13,35 | 512,76 | 765,39 | 28 |
| 1146 | TGTGSFIDAR | 13,35 | 512,76 | 866,44 | 28 |
| 1147 | TGTGSLSDAK | 8,32 | 468,74 | 620,32 | 26 |
| 1148 | TGTGSLSDAK | 8,32 | 468,74 | 677,35 | 26 |
| 1149 | TGTGSLSDAK | 8,32 | 468,74 | 778,39 | 26 |
| 1150 | TGVATEYQPEIGWWAGWVER | 25,49 | 779,04 | 903,45 | 43 |
| 1151 | TGVATEYQPEIGWWAGWVER | 25,5 | 779,04 | 1146,55 | 43 |
| 1152 | TGVATEYQPEIGWWAGWVER | 25,52 | 1168,06 | 1189,57 | 56 |
| 1153 | TGVSYPLLADGTR | 17,4 | 675,36 | 842,47 | 35 |
| 1154 | TGVSYPLLADGTR | 17,41 | 675,36 | 1005,54 | 35 |
| 1155 | TGVSYPLLADGTR | 17,4 | 675,36 | 1092,57 | 35 |
| 1156 | TGWAMDIK | 16,71 | 461,23 | 577,3 | 25 |
| 1157 | TGWAMDIK | 16,71 | 461,23 | 763,38 | 25 |
| 1158 | TGWAMDIK | 16,72 | 461,23 | 820,4 | 25 |
| 1159 | TGWATR | 9,71 | 346,18 | 517,24 | 20 |
| 1160 | TGWATR | 9,69 | 346,18 | 533,28 | 20 |
| 1161 | TGWATR | 9,69 | 346,18 | 590,3 | 20 |
| 1162 | TGWC[CAM]FDC[CAM]TPELGWWVGWVK | 28,39 | 795,36 | 960,51 | 44 |
| 1163 | TGWC[CAM]FDC[CAM]TPELGWWVGWVK | 28,39 | 795,36 | 1017,53 | 44 |
| 1164 | TGWC[CAM]FDC[CAM]TPELGWWVGWVK | 28,38 | 795,36 | 1028,36 | 44 |
| 1165 | TGWEGR | 9,1 | 353,17 | 531,22 | 21 |
| 1166 | TGWEGR | 9,09 | 353,17 | 547,26 | 21 |
| 1167 | TGWEGR | 9,09 | 353,17 | 604,28 | 21 |
| 1168 | TGWFVDK | 16,08 | 426,72 | 694,36 | 24 |
| 1169 | TGWFVDK | 16,1 | 426,72 | 706,32 | 24 |
| 1170 | TGWFVDK | 16,08 | 426,72 | 751,38 | 24 |
| 1171 | TGYDTK | 2,09 | 342,66 | 526,25 | 20 |
| 1172 | TGYDTK | 2,09 | 342,66 | 538,21 | 20 |
| 1173 | TGYDTK | 2,08 | 342,66 | 583,27 | 20 |
| 1174 | TGYGVR | 8,09 | 326,67 | 478,23 | 19 |
| 1175 | TGYGVR | 8,1 | 326,67 | 494,27 | 19 |
| 1176 | TGYGVR | 8,1 | 326,67 | 551,29 | 19 |
| 1177 | TGYSAR | 2,24 | 327,66 | 480,21 | 19 |
| 1178 | TGYSAR | 2,24 | 327,66 | 496,25 | 19 |
| 1179 | TGYSAR | 2,24 | 327,66 | 553,27 | 19 |
| 1180 | TGYSTR | 2,08 | 342,67 | 510,22 | 20 |
| 1181 | TGYSTR | 2,08 | 342,67 | 526,26 | 20 |
| 1182 | TGYSTR | 2,08 | 342,67 | 583,28 | 20 |
| 1183 | THESSNWGK | 5,36 | 523,24 | 678,32 | 28 |
| 1184 | THESSNWGK | 5,37 | 523,24 | 807,36 | 28 |
| 1185 | THESSNWGK | 5,37 | 523,24 | 944,42 | 28 |
| 1186 | TIC[CAM]TAIADAGTGK | 14,35 | 639,82 | 732,39 | 33 |
| 1187 | TIC[CAM]TAIADAGTGK | 14,35 | 639,82 | 904,47 | 33 |
| 1188 | TIC[CAM]TAIADAGTGK | 14,35 | 639,82 | 1064,5 | 33 |
| 1189 | TIGGAPDAYWVDDSLQISAR | 21,22 | 712,35 | 1004,5 | 40 |
| 1190 | TIGGAPDAYWVDDSLQISAR | 21,22 | 712,35 | 1103,57 | 40 |
| 1191 | TIGGAPDAYWVDDSLQISAR | 21,21 | 1068,02 | 1103,57 | 52 |
| 1192 | TLPFSASSYETLR | 18,73 | 736,37 | 855,42 | 37 |
| 1193 | TLPFSASSYETLR | 18,73 | 736,37 | 1013,49 | 37 |
| 1194 | TLPFSASSYETLR | 18,73 | 736,37 | 1160,56 | 37 |
| 1195 | TLPFSPK | 15 | 395,23 | 478,27 | 22 |
| 1196 | TLPFSPK | 15 | 395,23 | 575,32 | 22 |
| 1197 | TLPFSPK | 15 | 395,23 | 688,4 | 22 |
| 1198 | TLPFSQEVQDEVQSILFIEEK | 28,55 | 827,09 | 891,52 | 45 |
| 1199 | TLPFSQEVQDEVQSILFIEEK | 28,56 | 827,09 | 978,55 | 45 |
| 1200 | TLPFSQEVQDEVQSILFIEEK | 28,56 | 827,09 | 1106,61 | 45 |
| 1201 | TLPFSQEVQDEVQSMLFIEEK | 27,7 | 833,08 | 996,51 | 46 |
| 1202 | TLPFSQEVQDEVQSMLFIEEK | 27,69 | 833,08 | 1124,57 | 46 |
| 1203 | TLPFSQEVQDEVQSMLFIEEK | 27,7 | 833,08 | 1223,63 | 46 |
| 1204 | TLQNGWFEGFIISK | 24,12 | 820,43 | 940,51 | 41 |
| 1205 | TLQNGWFEGFIISK | 24,11 | 820,43 | 1126,59 | 41 |
| 1206 | TLQNGWFEGFIISK | 24,11 | 820,43 | 1183,61 | 41 |
| 1207 | TMQEYLNK | 12,6 | 513,75 | 666,35 | 28 |
| 1208 | TMQEYLNK | 12,6 | 513,75 | 794,4 | 28 |
| 1209 | TMQEYLNK | 12,6 | 513,75 | 925,44 | 28 |
| 1210 | TQTYQAYDAAR | 11,2 | 644,3 | 666,32 | 33 |
| 1211 | TQTYQAYDAAR | 11,2 | 644,3 | 957,44 | 33 |
| 1212 | TQTYQAYDAAR | 11,2 | 644,3 | 1058,49 | 33 |
| 1213 | TTDPTIWEK | 14,39 | 545,77 | 676,37 | 29 |
| 1214 | TTDPTIWEK | 14,39 | 545,77 | 773,42 | 29 |
| 1215 | TTDPTIWEK | 14,39 | 545,77 | 888,45 | 29 |
| 1216 | TTTTEVFK | 12,06 | 463,75 | 522,29 | 25 |
| 1217 | TTTTEVFK | 12,06 | 463,75 | 623,34 | 25 |
| 1218 | TTTTEVFK | 12,06 | 463,75 | 724,39 | 25 |
| 1219 | TWASNDFSR | 13,73 | 542,25 | 638,29 | 29 |
| 1220 | TWASNDFSR | 13,73 | 542,25 | 725,32 | 29 |
| 1221 | TWASNDFSR | 13,73 | 542,25 | 796,36 | 29 |
| 1222 | TWDMVQR | 14,28 | 468,22 | 648,31 | 26 |
| 1223 | TWDMVQR | 14,28 | 468,22 | 761,33 | 26 |
| 1224 | TWDMVQR | 14,28 | 468,22 | 834,39 | 26 |
| 1225 | TYVVDPAR | 12,15 | 460,75 | 557,3 | 25 |
| 1226 | TYVVDPAR | 12,14 | 460,75 | 656,37 | 25 |
| 1227 | TYVVDPAR | 12,15 | 460,75 | 819,44 | 25 |
| 1228 | VAFSLNIEMK | 20,65 | 576,31 | 747,41 | 30 |
| 1229 | VAFSLNIEMK | 20,65 | 576,31 | 834,44 | 30 |
| 1230 | VAFSLNIEMK | 20,65 | 576,31 | 981,51 | 30 |
| 1231 | VANSLIGLSTGAVR | 17,97 | 679,39 | 760,43 | 35 |
| 1232 | VANSLIGLSTGAVR | 17,97 | 679,39 | 873,52 | 35 |
| 1233 | VANSLIGLSTGAVR | 17,97 | 679,39 | 986,6 | 35 |
| 1234 | VELGK | 7,74 | 273,17 | 342,2 | 17 |
| 1235 | VELGK | 7,75 | 273,17 | 399,22 | 17 |
| 1236 | VELGK | 7,74 | 273,17 | 446,26 | 17 |
| 1237 | VFLDSWAK | 18,2 | 483,26 | 606,29 | 26 |
| 1238 | VFLDSWAK | 18,2 | 483,26 | 719,37 | 26 |
| 1239 | VFLDSWAK | 18,2 | 483,26 | 866,44 | 26 |
| 1240 | VFLESWAK | 18,11 | 490,27 | 620,3 | 27 |
| 1241 | VFLESWAK | 18,11 | 490,27 | 733,39 | 27 |
| 1242 | VFLESWAK | 18,11 | 490,27 | 880,46 | 27 |
| 1243 | VFLSSWAQDMNLSSAIK | 23,66 | 948,98 | 978,49 | 47 |
| 1244 | VFLSSWAQDMNLSSAIK | 23,66 | 948,98 | 1106,55 | 47 |
| 1245 | VFLSSWAQDMNLSSAIK | 23,66 | 948,98 | 1177,59 | 47 |
| 1246 | VGFER | 10,32 | 304,16 | 433,21 | 18 |
| 1247 | VGFER | 10,32 | 304,16 | 451,23 | 18 |
| 1248 | VGFER | 10,32 | 304,16 | 508,25 | 18 |
| 1249 | VILVFDQVR | 19,69 | 544,83 | 664,34 | 29 |
| 1250 | VILVFDQVR | 19,69 | 544,83 | 763,41 | 29 |
| 1251 | VILVFDQVR | 19,69 | 544,83 | 876,49 | 29 |
| 1252 | VMAAMVR | 12,3 | 389,21 | 476,26 | 22 |
| 1253 | VMAAMVR | 12,3 | 389,21 | 547,3 | 22 |
| 1254 | VMAAMVR | 12,3 | 389,21 | 678,34 | 22 |
| 1255 | VPLAVMGYDAGILVDAHNPR | 21,61 | 703,37 | 709,34 | 39 |
| 1256 | VPLAVMGYDAGILVDAHNPR | 21,61 | 703,37 | 808,41 | 39 |
| 1257 | VPLAVMGYDAGILVDAHNPR | 21,61 | 703,37 | 921,49 | 39 |
| 1258 | VQDEVQSMLFIEEK | 20,48 | 847,92 | 996,51 | 42 |
| 1259 | VQDEVQSMLFIEEK | 20,48 | 847,92 | 1124,57 | 42 |
| 1260 | VQDEVQSMLFIEEK | 20,47 | 847,92 | 1223,63 | 42 |
| 1261 | VQDGVQSMLFIEEK | 20,26 | 811,91 | 996,51 | 41 |
| 1262 | VQDGVQSMLFIEEK | 20,27 | 811,91 | 1124,57 | 41 |
| 1263 | VQDGVQSMLFIEEK | 20,25 | 811,91 | 1223,63 | 41 |
| 1264 | VSC[CAM]LPC[CAM]YQVVSHK | 14,32 | 526,26 | 569,34 | 30 |
| 1265 | VSC[CAM]LPC[CAM]YQVVSHK | 14,32 | 526,26 | 860,46 | 30 |
| 1266 | VSC[CAM]LPC[CAM]YQVVSHK | 14,31 | 526,26 | 1020,49 | 30 |
| 1267 | VSC[CAM]VWC[CAM]YQALAR | 18,41 | 756,86 | 881,43 | 38 |
| 1268 | VSC[CAM]VWC[CAM]YQALAR | 18,41 | 756,86 | 1067,51 | 38 |
| 1269 | VSC[CAM]VWC[CAM]YQALAR | 18,41 | 756,86 | 1166,58 | 38 |
| 1270 | VSDVC[CAM]SEVTAEGWQEVR | 17,33 | 650,97 | 774,39 | 37 |
| 1271 | VSDVC[CAM]SEVTAEGWQEVR | 17,34 | 975,95 | 1075,52 | 48 |
| 1272 | VSDVC[CAM]SEVTAEGWQEVR | 17,34 | 975,95 | 1174,59 | 48 |
| 1273 | VSEVEGWQIHGK | 13,92 | 456,9 | 582,34 | 27 |
| 1274 | VSEVEGWQIHGK | 13,92 | 456,9 | 768,42 | 27 |
| 1275 | VSEVEGWQIHGK | 13,92 | 456,9 | 825,44 | 27 |
| 1276 | VSFSLNIEMK | 20,65 | 584,31 | 834,44 | 31 |
| 1277 | VSFSLNIEMK | 20,64 | 584,31 | 981,51 | 31 |
| 1278 | VSFSLNIEMK | 20,65 | 584,31 | 1068,54 | 31 |
| 1279 | VSPC[CAM]SSFK | 11,04 | 456,22 | 468,25 | 25 |
| 1280 | VSPC[CAM]SSFK | 11,04 | 456,22 | 628,28 | 25 |
| 1281 | VSPC[CAM]SSFK | 11,04 | 456,22 | 725,33 | 25 |
| 1282 | VSQVPAYK | 10,68 | 446,25 | 478,27 | 25 |
| 1283 | VSQVPAYK | 10,68 | 446,25 | 577,33 | 25 |
| 1284 | VSQVPAYK | 10,68 | 446,25 | 705,39 | 25 |
| 1285 | VVFAR | 11,17 | 296,18 | 393,22 | 18 |
| 1286 | VVFAR | 11,17 | 296,18 | 417,25 | 18 |
| 1287 | VVFAR | 11,17 | 296,18 | 492,29 | 18 |
| 1288 | WDGAK | 4,9 | 288,64 | 302,11 | 18 |
| 1289 | WDGAK | 4,9 | 288,64 | 390,2 | 18 |
| 1290 | WDGAK | 4,9 | 288,64 | 430,17 | 18 |
| 1291 | WDGHIYDFPDWNR | 20,52 | 574,25 | 590,27 | 33 |
| 1292 | WDGHIYDFPDWNR | 20,52 | 574,25 | 687,32 | 33 |
| 1293 | WDGHIYDFPDWNR | 20,52 | 574,25 | 887,37 | 33 |
| 1294 | WDGIK | 12,03 | 309,67 | 359,13 | 19 |
| 1295 | WDGIK | 12,03 | 309,67 | 432,25 | 19 |
| 1296 | WDGIK | 12,03 | 309,67 | 472,22 | 19 |
| 1297 | WDGKPR | 6,36 | 379,7 | 457,29 | 22 |
| 1298 | WDGKPR | 6,35 | 379,7 | 572,32 | 22 |
| 1299 | WDGKPR | 6,36 | 379,7 | 584,28 | 22 |
| 1300 | WDGQTR | 7,41 | 381,68 | 461,25 | 22 |
| 1301 | WDGQTR | 7,41 | 381,68 | 576,27 | 22 |
| 1302 | WDGQTR | 7,41 | 381,68 | 588,24 | 22 |
| 1303 | WDGVK | 10,1 | 302,66 | 359,13 | 18 |
| 1304 | WDGVK | 10,1 | 302,66 | 418,23 | 18 |
| 1305 | WDGVK | 10,1 | 302,66 | 458,2 | 18 |
| 1306 | WDGVNR | 10,39 | 373,68 | 445,25 | 21 |
| 1307 | WDGVNR | 10,39 | 373,68 | 560,28 | 21 |
| 1308 | WDGVNR | 10,42 | 373,68 | 572,25 | 21 |
| 1309 | YAQAK | 12,58 | 290,66 | 363,17 | 18 |
| 1310 | YAQAK | 12,58 | 290,66 | 417,25 | 18 |
| 1311 | YAQAK | 12,58 | 290,66 | 434,2 | 18 |
| 1312 | YFSDFNAK | 14,21 | 496,23 | 681,32 | 27 |
| 1313 | YFSDFNAK | 14,21 | 496,23 | 828,39 | 27 |
| 1314 | YFSDFNAK | 14,21 | 496,23 | 828,39 | 27 |
| 1315 | YGTHLDR | 8,51 | 431,21 | 641,34 | 24 |
| 1316 | YGTHLDR | 8,52 | 431,21 | 687,31 | 24 |
| 1317 | YGTHLDR | 8,51 | 431,21 | 698,36 | 24 |
| 1318 | YLDELVK | 15,52 | 440,24 | 488,31 | 24 |
| 1319 | YLDELVK | 15,53 | 440,24 | 603,33 | 24 |
| 1320 | YLDELVK | 15,52 | 440,24 | 716,42 | 24 |
| 1321 | YLMITEAGR | 15,86 | 527,27 | 533,27 | 28 |
| 1322 | YLMITEAGR | 15,86 | 527,27 | 646,35 | 28 |
| 1323 | YLMITEAGR | 15,86 | 527,27 | 777,39 | 28 |
| 1324 | YLNLFSYGNANIGGGIDK | 22,16 | 639,32 | 773,42 | 36 |
| 1325 | YLNLFSYGNANIGGGIDK | 22,16 | 958,48 | 1015,52 | 47 |
| 1326 | YLNLFSYGNANIGGGIDK | 22,16 | 958,48 | 1178,58 | 47 |
| 1327 | YPWWYSQQVAHHLGAQR | 18,11 | 535,53 | 544,32 | 30 |
| 1328 | YPVVWYSQQVAHHLGAQR | 18,11 | 535,53 | 681,38 | 30 |
| 1329 | YPVVWYSQQVAHHLGAQR | 18,11 | 535,53 | 889,48 | 30 |
| 1330 | YSNVLAFK | 16,44 | 471,26 | 478,3 | 26 |
| 1331 | YSNVLAFK | 16,44 | 471,26 | 691,41 | 26 |
| 1332 | YSNVLAFK | 16,44 | 471,26 | 778,45 | 26 |
| 1333 | YSPASTFK | 12,22 | 450,73 | 553,3 | 25 |
| 1334 | YSPASTFK | 12,22 | 450,73 | 650,35 | 25 |
| 1335 | YSPASTFK | 12,22 | 450,73 | 737,38 | 25 |
| 1336 | YSVVPVYQQLAR | 18,42 | 711,89 | 778,42 | 36 |
| 1337 | YSVVPVYQQLAR | 18,42 | 711,89 | 974,54 | 36 |
| 1338 | YSVVPVYQQLAR | 18,43 | 711,89 | 1073,61 | 36 |
| 1339 | YSVVWYSQLTAK | 19,75 | 722,88 | 810,44 | 37 |
| 1340 | YSVVWYSQLTAK | 19,76 | 722,88 | 996,51 | 37 |
| 1341 | YSVVWYSQL TAK | 19,76 | 722,88 | 1095,58 | 37 |
| 1342 | YSVVWYSQQVAHHLGAQR | 18,61 | 533,02 | 544,32 | 30 |
| 1343 | YSVVWYSQQVAHHLGAQR | 18,61 | 533,02 | 681,38 | 30 |
| 1344 | YSVVWYSQQVAHHLGAQR | 18,61 | 533,02 | 889,48 | 30 |
| 1345 | YTPASTFK | 11,95 | 305,49 | 553,3 | 19 |
| 1346 | YTPASTFK | 11,98 | 457,73 | 553,3 | 25 |
| 1347 | YTPASTFK | 11,98 | 457,73 | 650,35 | 25 |
| 1348 | YTSAFGYGNADVSGEPGK | 15,03 | 607,28 | 673,35 | 34 |
| 1349 | YTSAFGYGNADVSGEPGK | 15,02 | 607,28 | 788,38 | 34 |
| 1350 | YTSAFGYGNADVSGEPGK | 15,02 | 910,41 | 1030,48 | 45 |
| 1351 | YVFVSALTGNLGSNLTSSIK | 23,66 | 691,04 | 906,49 | 39 |
| 1352 | YVFVSALTGNLGSNLTSSIK | 23,66 | 1036,06 | 1165,63 | 51 |
| 1353 | YVFVSALTGNLGSNLTSSIK | 23,67 | 1036,06 | 1190,64 | 51 |
| 1354 | YVFVSALTGSLGSNLTSSIK | 24,04 | 682,04 | 906,49 | 38 |
| 1355 | YVFVSALTGSLGSNLTSSIK | 24,04 | 1022,55 | 1106,61 | 50 |
| 1356 | YVFVSALTGSLGSNLTSSIK | 24,04 | 1022,55 | 1163,63 | 50 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 17 : Identification d'une résistance aux béta-lactamines de type IMP:

Les échantillons, Ech145 à Ech154, sont identifiés selon l'une des méthodes décrites dans les exemples 1, 3 ou 4. L'identification des espèces est reportée dans le **TABLEAU 26.**

**TABLEAU 26 :**

| Noms | Espèces |
|---|---|
| Ech145 | *A. baumannii* |
| Ech146 | *A. baumannii* |
| Ech147 | *E. coli* |
| Ech148 | *K. pneumoniae* |
| Ech149 | *K. pneumoniae* |
| Ech150 | *P. aeruginosa* |
| Ech151 | *P. aeruginosa* |
| Ech152 | *P. aeruginosa* |
| Ech153 | *P. aeruginosa* |
| Ech154 | *P. putida* |

Les échantillons Ech145 à Ech154 correspondent à une espèce pouvant comporter un mécanisme de résistance de type IMP. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 27** en lieu et place des peptides du **TABLEAU 3.**

**TABLEAU 27 :**

| Transition numéro | Peptide | Temps de rétention (minutes) | (m/z) filtré en Q1 | (m/z) filtré en Q3 | Energie de collision (eV) | Seuil de positivité |
|---|---|---|---|---|---|---|
| 1 | DTENLVNWFVER | 24,3 | 761,37 | 550,3 | 39,1 | 2000 |
| 2 | DTENLVNWFVER | 24,3 | 761,37 | 850,42 | 39,1 | 2000 |
| 3 | DTENLVNWFVER | 24,3 | 761,37 | 949,49 | 39,1 | 2000 |
| 4 | GDASLMK | 10,6 | 361,18 | 391,24 | 16,3 | 2000 |
| 5 | GDASLMK | 10,6 | 361,18 | 478,27 | 16,3 | 2000 |
| 6 | GDASLMK | 10,6 | 361,18 | 549,31 | 16,3 | 2000 |
| 7 | GFNESK | 2 | 341,16 | 312,65 | 15,2 | 2000 |
| 8 | GFNESK | 2 | 341,16 | 363,19 | 15,2 | 2000 |
| 9 | GFNESK | 2 | 341,16 | 477,23 | 15,2 | 2000 |
| 10 | GLNESK | 1,1 | 324,17 | 363,19 | 14,2 | 2000 |
| 11 | GLNESK | 1,1 | 324,17 | 477,23 | 14,2 | 2000 |
| 12 | GLNESK | 1,1 | 324,17 | 590,31 | 14,2 | 2000 |
| 13 | GLNESR | 2,2 | 338,18 | 309,66 | 15 | 2000 |
| 14 | GLNESR | 2,2 | 338,18 | 391,19 | 15 | 2000 |
| 15 | GLNESR | 2,1 | 338,18 | 505,24 | 15 | 2000 |
| 16 | GVYVHTSFEEVK | 15,1 | 465,57 | 488,74 | 21,5 | 2000 |
| 17 | GVYVHTSFEEVK | 15,1 | 465,57 | 538,28 | 21,5 | 2000 |
| 18 | GVYVHTSFEEVK | 15,1 | 465,57 | 619,81 | 21,5 | 2000 |
| 19 | GWGVVTK | 14,3 | 373,71 | 345,2 | 17 | 2000 |
| 20 | GWGVVTK | 14,3 | 373,71 | 347,23 | 17 | 2000 |
| 21 | GWGVVTK | 14,3 | 373,71 | 503,32 | 17 | 2000 |
| 22 | GWSVVTK | 14,2 | 388,72 | 347,23 | 17,9 | 2000 |
| 23 | GWSVVTK | 14,2 | 388,72 | 446,3 | 17,9 | 2000 |
| 24 | GWSVVTK | 14,2 | 388,72 | 533,33 | 17,9 | 2000 |
| 25 | HGLVVLVK | 15,4 | 432,79 | 557,4 | 20,4 | 2000 |
| 26 | HGLVVLVK | 15,5 | 432,79 | 670,49 | 20,4 | 2000 |
| 27 | HGLVVLVK | 15,4 | 432,79 | 727,51 | 20,4 | 2000 |
| 28 | HSFNGVSYSLIK | 17 | 451,24 | 460,31 | 21,1 | 2000 |
| 29 | HSFNGVSYSLIK | 17 | 451,24 | 623,38 | 21,1 | 2000 |
| 30 | HSFNGVSYSLIK | 17 | 451,24 | 710,41 | 21,1 | 2000 |
| 31 | LEEGVYVHTSFEEVK | 16,9 | 589,29 | 697,85 | 25,4 | 2000 |
| 32 | LEEGVYVHTSFEEVK | 16,9 | 589,29 | 762,37 | 25,4 | 2000 |
| 33 | LEEGVYVHTSFEEVK | 16,9 | 589,29 | 826,89 | 25,4 | 2000 |
| 34 | LFVLCVCFLCSITAAGAR | 19,5 | 686,68 | 659,38 | 28,4 | 2000 |
| 35 | LFVLCVCFLCSITAAGAR | 19,6 | 686,68 | 906,45 | 28,4 | 2000 |
| 36 | LFVLCVCFLCSITAAGAR | 19,5 | 1029,52 | 374,22 | 54,4 | 2000 |
| 37 | LFVLCVCFLCSITAAGAR | 19,5 | 1029,52 | 659,38 | 54,4 | 2000 |
| 38 | LTLEQAVK | 15,2 | 451,27 | 574,32 | 21,5 | 2000 |
| 39 | LTLEQAVK | 15,2 | 451,27 | 687,4 | 21,5 | 2000 |
| 40 | LTLEQAVK | 15,2 | 451,27 | 788,45 | 21,5 | 2000 |
| 41 | LTWEQAVK | 16,3 | 487,77 | 574,32 | 23,5 | 2000 |
| 42 | LTWEQAVK | 16,3 | 487,77 | 760,4 | 23,5 | 2000 |
| 43 | LTWEQAVK | 16,3 | 487,77 | 861,45 | 23,5 | 2000 |
| 44 | LTWEQTVK | 15,4 | 502,77 | 395,71 | 24,4 | 2000 |
| 45 | LTWEQTVK | 15,4 | 502,77 | 604,33 | 24,4 | 2000 |
| 46 | LTWEQTVK | 15,4 | 502,77 | 790,41 | 24,4 | 2000 |
| 47 | LVAWFVGR | 21,3 | 474,28 | 478,28 | 22,8 | 2000 |
| 48 | LVAWFVGR | 21,3 | 474,28 | 664,36 | 22,8 | 2000 |
| 49 | LVAWFVGR | 21,3 | 474,28 | 735,39 | 22,8 | 2000 |
| 50 | LVNWFIEHGYR | 20,1 | 478,58 | 611,29 | 21,9 | 2000 |
| 51 | LVNWFIEHGYR | 20,1 | 478,58 | 660,83 | 21,9 | 2000 |
| 52 | LVNWFIEHGYR | 20,1 | 478,58 | 661,31 | 21,9 | 2000 |
| 53 | LVNWFVER | 20,9 | 531,79 | 550,3 | 26 | 2000 |
| 54 | LVNWFVER | 20,9 | 531,79 | 736,38 | 26 | 2000 |
| 55 | LVNWFVER | 20,9 | 531,79 | 850,42 | 26 | 2000 |
| 56 | LVTWFVER | 20,6 | 525,29 | 550,3 | 25,7 | 2000 |
| 57 | LVTWFVER | 20,6 | 525,29 | 736,38 | 25,7 | 2000 |
| 58 | LVTWFVER | 20,6 | 525,29 | 837,43 | 25,7 | 2000 |
| 59 | LVVPGHSEVGDASLLK | 17,6 | 540,97 | 655,34 | 23,9 | 2000 |
| 60 | LVVPGHSEVGDASLLK | 17,6 | 540,97 | 704,88 | 23,9 | 2000 |
| 61 | LVVPGHSEVGDASLLK | 17,6 | 810,95 | 655,34 | 42 | 2000 |
| 62 | LVVPSHSDIGDASLLK | 18 | 550,97 | 670,35 | 24,2 | 2000 |
| 63 | LVVPSHSDIGDASLLK | 18 | 550,97 | 719,88 | 24,2 | 2000 |
| 64 | LVVPSHSDIGDASLLK | 18 | 825,96 | 670,35 | 42,8 | 2000 |
| 65 | LVVPSHSDIGDSSLLK | 17,7 | 556,31 | 678,34 | 24,3 | 2000 |
| 66 | LVVPSHSDIGDSSLLK | 17,7 | 556,31 | 719,39 | 24,3 | 2000 |
| 67 | LVVPSHSDIGDSSLLK | 17,7 | 556,31 | 727,88 | 24,3 | 2000 |
| 68 | LVVPSHSDVGDASLLK | 17,5 | 546,3 | 663,34 | 24 | 2000 |
| 69 | LVVPSHSDVGDASLLK | 17,5 | 546,3 | 712,87 | 24 | 2000 |
| 70 | LVVPSHSDVGDASLLK | 17,5 | 818,95 | 663,34 | 42,4 | 2000 |
| 71 | LVVPSHSEAGDASLLK | 16,1 | 541,63 | 656,33 | 23,9 | 2000 |
| 72 | LVVPSHSEAGDASLLK | 16,1 | 541,63 | 705,87 | 23,9 | 2000 |
| 73 | LVVPSHSEAGDASLLK | 16,1 | 541,63 | 755,4 | 23,9 | 2000 |
| 74 | LVVPSHSEVGDASLLK | 17,5 | 550,97 | 670,35 | 24,2 | 2000 |
| 75 | LVVPSHSEVGDASLLK | 17,5 | 550,97 | 719,88 | 24,2 | 2000 |
| 76 | LVVPSHSEVGDASLLK | 17,5 | 825,96 | 670,35 | 42,8 | 2000 |
| 77 | LVVSGHSEIGNASLLK | 16,8 | 541,97 | 656,85 | 23,9 | 2000 |
| 78 | LVVSGHSEIGNASLLK | 16,8 | 541,97 | 706,38 | 23,9 | 2000 |
| 79 | LVVSGHSEIGNASLLK | 16,8 | 541,97 | 755,92 | 23,9 | 2000 |
| 80 | LVVSSHSDIGDVSLLK | 18,9 | 556,98 | 679,35 | 24,3 | 2000 |
| 81 | LVVSSHSDIGDVSLLK | 18,9 | 556,98 | 728,89 | 24,3 | 2000 |
| 82 | LVVSSHSDIGDVSLLK | 18,9 | 556,98 | 778,42 | 24,3 | 2000 |
| 83 | LVVSSHSEIGDASLLK | 17,6 | 552,31 | 672,34 | 24,2 | 2000 |
| 84 | LVVSSHSEIGDASLLK | 17,6 | 552,31 | 721,88 | 24,2 | 2000 |
| 85 | LVVSSHSEIGDASLLK | 17,6 | 552,31 | 771,41 | 24,2 | 2000 |
| 86 | LVVSSHSEIGNASLLQR | 16,8 | 604 | 416,26 | 25,8 | 2000 |
| 87 | LVVSSHSEIGNASLLQR | 16,8 | 604 | 616,38 | 25,8 | 2000 |
| 88 | LVVSSHSEIGNASLLQR | 16,8 | 604 | 799,42 | 25,8 | 2000 |
| 89 | LVVSSHSEK | 8,1 | 329,18 | 387,19 | 17,3 | 2000 |
| 90 | LVVSSHSEK | 8,1 | 329,18 | 587,28 | 17,3 | 2000 |
| 91 | LVVSSHSEK | 8,1 | 493,27 | 773,38 | 23,9 | 2000 |
| 92 | LVVSSHSETGNASLLK | 14,7 | 547,97 | 665,83 | 24,1 | 2000 |
| 93 | LVVSSHSETGNASLLK | 14,7 | 547,97 | 715,37 | 24,1 | 2000 |
| 94 | LVVSSHSETGNASLLK | 14,7 | 547,97 | 764,9 | 24,1 | 2000 |
| 95 | NDAYLIDTPITAK | 18,8 | 717,88 | 745,41 | 36,7 | 2000 |
| 96 | NDAYLIDTPITAK | 18,8 | 717,88 | 858,49 | 36,7 | 2000 |
| 97 | NDAYLIDTPITAK | 18,8 | 717,88 | 971,58 | 36,7 | 2000 |
| 98 | NSFGGVNYWLVK | 21,4 | 692,36 | 822,45 | 35,2 | 2000 |
| 99 | NSFGGVNYWLVK | 21,4 | 692,36 | 1035,56 | 35,2 | 2000 |
| 100 | NSFGGVNYWLVK | 21,4 | 692,36 | 1182,63 | 35,2 | 2000 |
| 101 | NSFSGASYWLVK | 20,8 | 679,84 | 795,44 | 34,5 | 2000 |
| 102 | NSFSGASYWLVK | 20,8 | 679,84 | 923,5 | 34,5 | 2000 |
| 103 | NSFSGASYWLVK | 20,8 | 679,84 | 1010,53 | 34,5 | 2000 |
| 104 | NSFSGGSYWLVNNK | 18,8 | 786,88 | 375,2 | 40,6 | 2000 |
| 105 | NSFSGGSYWLVNNK | 18,8 | 786,88 | 474,27 | 40,6 | 2000 |
| 106 | NSFSGGSYWLVNNK | 18,8 | 786,88 | 1224,6 | 40,6 | 2000 |
| 107 | NSFSGVSYWLLK | 23,6 | 700,86 | 809,46 | 35,7 | 2000 |
| 108 | NSFSGVSYWLLK | 23,6 | 700,86 | 1052,58 | 35,7 | 2000 |
| 109 | NSFSGVSYWLLK | 23,6 | 700,86 | 1199,65 | 35,7 | 2000 |
| 110 | NSFSGVSYWLVK | 22,3 | 693,86 | 795,44 | 35,3 | 2000 |
| 111 | NSFSGVSYWLVK | 22,3 | 693,86 | 951,53 | 35,3 | 2000 |
| 112 | NSFSGVSYWLVK | 22,3 | 693,86 | 1038,56 | 35,3 | 2000 |
| 113 | SIPTYASELTNELLK | 23,8 | 560,3 | 717,41 | 24,5 | 2000 |
| 114 | SIPTYASELTNELLK | 23,8 | 560,3 | 739,89 | 24,5 | 2000 |
| 115 | SIPTYASELTNELLK | 23,8 | 839,95 | 739,89 | 43,6 | 2000 |
| 116 | TLEQAVK | 10,5 | 394,73 | 445,28 | 18,2 | 2000 |
| 117 | TLEQAVK | 10,5 | 394,73 | 574,32 | 18,2 | 2000 |
| 118 | TLEQAVK | 10,5 | 394,73 | 687,4 | 18,2 | 2000 |
| 119 | TWEQALK | 15,1 | 438,24 | 459,29 | 20,7 | 2000 |
| 120 | TWEQALK | 15,1 | 438,24 | 588,34 | 20,7 | 2000 |
| 121 | TWEQALK | 15,1 | 438,24 | 774,41 | 20,7 | 2000 |
| 122 | TWEQAVK | 12,8 | 431,23 | 445,28 | 20,3 | 2000 |
| 123 | TWEQAVK | 12,8 | 431,23 | 574,32 | 20,3 | 2000 |
| 124 | TWEQAVK | 12,8 | 431,23 | 760,4 | 20,3 | 2000 |
| 125 | VQATNSFSGVNYWLVK | 22,1 | 604,98 | 708,41 | 25,8 | 2000 |
| 126 | VQATNSFSGVNYWLVK | 22,1 | 604,98 | 822,45 | 25,8 | 2000 |
| 127 | VQATNSFSGVNYWLVK | 22,1 | 906,97 | 1212,64 | 47,4 | 2000 |
| 128 | VQATNSFSGVSYSLIK | 19,9 | 567,63 | 710,41 | 24,7 | 2000 |
| 129 | VQATNSFSGVSYSLIK | 19,9 | 567,63 | 953,53 | 24,7 | 2000 |
| 130 | VQATNSFSGVSYSLIK | 19,9 | 850,95 | 710,41 | 44,2 | 2000 |
| 131 | VQATNSFSGVSYWLVK | 22,5 | 595,98 | 708,41 | 25,6 | 2000 |
| 132 | VQATNSFSGVSYWLVK | 22,5 | 595,98 | 795,44 | 25,6 | 2000 |
| 133 | VQATNSFSGVSYWLVK | 22,5 | 893,46 | 1038,56 | 46,7 | 2000 |
| 134 | YSFSEVSYWLVK | 23,8 | 754,38 | 795,44 | 38,7 | 2000 |
| 135 | YSFSEVSYWLVK | 23,8 | 754,38 | 894,51 | 38,7 | 2000 |
| 136 | YSFSEVSYWLVK | 23,8 | 754,38 | 1110,58 | 38,7 | 2000 |
| 137 | YSFSGVSYWLVK | 23,4 | 718,37 | 795,44 | 36,7 | 2000 |
| 138 | YSFSGVSYWLVK | 23,4 | 718,37 | 951,53 | 36,7 | 2000 |
| 139 | YSFSGVSYWLVK | 23,4 | 718,37 | 1185,63 | 36,7 | 2000 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1 : | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 500,00 °C |
| Gaz de nébulisation: | 50,00 psi |
| Gaz chauffant: | 50,00 psi |
| Gaz de collision induisant dissociation | : 9,00 psi |
| Remplissage dynamique: | activé |
| Potentiel d'orifice : | 100, 00 V |
| (en anglais declustering potential (DP)) | |
| Potentiel d'entrée avant Q0 (EP): | 6,00 V |
| Potentiel en sortie de cellule de collision: | 15 V |
| (en anglais cell exit potential (CXP)) | |
| Temps de cycle total: | 1 sec |
| Fenêtre de détection: | 120 sec |

Les aires obtenues pour chacune des transitions et pour chacun des microorganismes étudiés ont été mesurées. Lorsque les aires des transitions sont supérieures ou égales au seuil de positivité décrit dans le **TABLEAU 27,** la détection de la transition est considérée comme positive et est notée « 1 » dans le **TABLEAU 28.** Lorsqu'une transition a une aire inférieure au seuil de positivité décrit dans le **TABLEAU 27,** la transition est considérée comme non détectée et est notée « 0 » dans le **TABLEAU 28.**

Pour un peptide donné, lorsque au moins 3 transitions sont notées « 1 », le peptide est considéré comme détecté.

**TABLEAU 28 :**

| Transition numéro | Ech145 | Ech146 | Ech147 | Ech148 | Ech149 | Ech150 | Ech151 | Ech152 | Ech153 | Ech154 |
|---|---|---|---|---|---|---|---|---|---|---|
| 1 | 0 | 0 | 1 | 0 | 0 | 0 | 0 | 0 | 0 | 0 |
| 2 | 0 | 0 | 1 | 0 | 0 | 0 | 0 | 0 | 0 | 0 |
| 3 | 0 | 0 | 0 | 0 | 0 | 0 | 0 | 0 | 0 | 0 |
| 4 | 0 | 0 | 0 | 0 | 0 | 0 | 0 | 0 | 1 | 0 |
| 5 | 0 | 0 | 0 | 0 | 0 | 0 | 0 | 0 | 1 | 0 |
| 6 | 0 | 0 | 0 | 0 | 0 | 0 | 0 | 0 | 1 | 0 |
| 7 | 0 | 0 | 0 | 0 | 0 | 0 | 0 | 0 | 0 | 0 |
| 8 | 0 | 0 | 0 | 0 | 0 | 0 | 0 | 0 | 0 | 0 |
| 9 | 0 | 0 | 0 | 0 | 0 | 0 | 0 | 0 | 0 | 0 |
| 10 | 1 | 1 | 0 | 0 | 0 | 0 | 0 | 0 | 0 | 0 |
| 11 | 1 | 1 | 0 | 0 | 0 | 0 | 0 | 0 | 0 | 0 |
| 12 | 0 | 0 | 0 | 0 | 0 | 0 | 0 | 0 | 0 | 0 |
| 13 | 0 | 0 | 0 | 0 | 0 | 0 | 0 | 0 | 0 | 0 |
| 14 | 0 | 0 | 0 | 0 | 0 | 0 | 0 | 0 | 0 | 0 |
| 15 | 0 | 0 | 0 | 0 | 0 | 0 | 0 | 0 | 0 | 0 |
| 16 | 0 | 0 | 0 | 0 | 0 | 0 | 0 | 0 | 0 | 0 |
| 17 | 0 | 0 | 0 | 0 | 0 | 1 | 1 | 0 | 1 | 0 |
| 18 | 0 | 0 | 0 | 0 | 0 | 0 | 0 | 0 | 0 | 0 |
| 19 | 0 | 1 | 0 | 0 | 0 | 0 | 0 | 0 | 0 | 0 |
| 20 | 0 | 0 | 0 | 0 | 0 | 1 | 0 | 1 | 1 | 0 |
| 21 | 0 | 0 | 0 | 0 | 0 | 0 | 0 | 1 | 0 | 0 |
| 22 | 0 | 0 | 0 | 0 | 1 | 0 | 0 | 0 | 0 | 0 |
| 23 | 0 | 0 | 1 | 1 | 1 | 0 | 0 | 0 | 1 | 0 |
| 24 | 0 | 0 | 0 | 0 | 0 | 0 | 0 | 0 | 0 | 0 |
| 25 | 0 | 0 | 0 | 0 | 0 | 0 | 0 | 0 | 0 | 0 |
| 26 | 0 | 0 | 0 | 0 | 0 | 0 | 0 | 0 | 0 | 0 |
| 27 | 0 | 0 | 0 | 0 | 0 | 0 | 0 | 0 | 0 | 0 |
| 28 | 0 | 0 | 0 | 0 | 0 | 0 | 0 | 0 | 0 | 0 |
| 29 | 0 | 0 | 0 | 0 | 0 | 0 | 0 | 0 | 0 | 0 |
| 30 | 0 | 0 | 0 | 0 | 0 | 0 | 0 | 0 | 0 | 0 |
| 31 | 0 | 0 | 0 | 0 | 0 | 0 | 0 | 0 | 0 | 0 |
| 32 | 0 | 0 | 0 | 0 | 0 | 0 | 0 | 0 | 0 | 0 |
| 33 | 1 | 0 | 0 | 0 | 0 | 0 | 0 | 0 | 0 | 0 |
| 34 | 0 | 0 | 0 | 0 | 0 | 0 | 0 | 0 | 0 | 0 |
| 35 | 0 | 0 | 0 | 0 | 0 | 0 | 0 | 0 | 0 | 0 |
| 36 | 0 | 0 | 0 | 0 | 0 | 0 | 0 | 0 | 0 | 0 |
| 37 | 0 | 0 | 0 | 0 | 0 | 0 | 0 | 0 | 0 | 0 |
| 38 | 0 | 1 | 1 | 1 | 0 | 0 | 0 | 1 | 0 | 0 |
| 39 | 0 | 1 | 1 | 1 | 0 | 0 | 0 | 1 | 0 | 0 |
| 40 | 0 | 1 | 1 | 1 | 0 | 0 | 0 | 1 | 0 | 0 |
| 41 | 0 | 0 | 1 | 0 | 0 | 0 | 0 | 0 | 0 | 1 |
| 42 | 0 | 0 | 0 | 0 | 0 | 0 | 0 | 0 | 0 | 1 |
| 43 | 0 | 0 | 0 | 0 | 0 | 0 | 0 | 0 | 0 | 1 |
| 44 | 0 | 0 | 0 | 0 | 0 | 0 | 0 | 0 | 0 | 0 |
| 45 | 0 | 0 | 0 | 0 | 0 | 0 | 0 | 0 | 0 | 0 |
| 46 | 0 | 0 | 0 | 0 | 0 | 0 | 0 | 0 | 0 | 0 |
| 47 | 0 | 0 | 0 | 0 | 0 | 0 | 0 | 0 | 0 | 0 |
| 48 | 0 | 0 | 0 | 0 | 0 | 0 | 0 | 0 | 0 | 1 |
| 49 | 0 | 0 | 0 | 0 | 0 | 0 | 0 | 0 | 0 | 0 |
| 50 | 0 | 0 | 0 | 0 | 0 | 0 | 0 | 0 | 0 | 0 |
| 51 | 0 | 0 | 0 | 0 | 0 | 0 | 0 | 0 | 0 | 0 |
| 52 | 0 | 0 | 0 | 0 | 0 | 1 | 0 | 0 | 0 | 0 |
| 53 | 1 | 0 | 0 | 0 | 1 | 1 | 0 | 0 | 1 | 0 |
| 54 | 1 | 0 | 1 | 0 | 0 | 1 | 0 | 0 | 1 | 0 |
| 55 | 1 | 1 | 1 | 0 | 1 | 1 | 0 | 0 | 1 | 0 |
| 56 | 0 | 1 | 1 | 1 | 1 | 0 | 1 | 1 | 0 | 0 |
| 57 | 0 | 1 | 1 | 1 | 1 | 0 | 1 | 1 | 0 | 0 |
| 58 | 0 | 1 | 1 | 1 | 1 | 0 | 1 | 1 | 0 | 0 |
| 59 | 0 | 0 | 0 | 0 | 0 | 1 | 1 | 0 | 0 | 0 |
| 60 | 0 | 0 | 0 | 0 | 0 | 1 | 1 | 0 | 0 | 0 |
| 61 | 0 | 0 | 0 | 0 | 0 | 1 | 1 | 0 | 0 | 0 |
| 62 | 0 | 0 | 0 | 1 | 0 | 0 | 1 | 1 | 0 | 0 |
| 63 | 0 | 0 | 0 | 1 | 0 | 0 | 1 | 1 | 0 | 0 |
| 64 | 0 | 0 | 0 | 1 | 0 | 0 | 1 | 1 | 0 | 0 |
| 65 | 0 | 0 | 0 | 0 | 0 | 1 | 1 | 1 | 1 | 0 |
| 66 | 0 | 0 | 0 | 0 | 0 | 0 | 0 | 0 | 0 | 0 |
| 67 | 0 | 0 | 0 | 0 | 0 | 0 | 0 | 0 | 0 | 0 |
| 68 | 0 | 0 | 0 | 0 | 0 | 0 | 0 | 0 | 0 | 0 |
| 69 | 0 | 0 | 0 | 0 | 0 | 0 | 0 | 0 | 0 | 0 |
| 70 | 0 | 1 | 0 | 0 | 0 | 0 | 0 | 0 | 0 | 0 |
| 71 | 1 | 1 | 1 | 0 | 0 | 0 | 0 | 0 | 0 | 0 |
| 72 | 0 | 1 | 1 | 0 | 0 | 0 | 0 | 0 | 0 | 0 |
| 73 | 0 | 1 | 1 | 0 | 0 | 0 | 0 | 0 | 0 | 0 |
| 74 | 0 | 0 | 0 | 1 | 0 | 0 | 1 | 1 | 0 | 0 |
| 75 | 0 | 0 | 0 | 1 | 0 | 0 | 1 | 1 | 0 | 0 |
| 76 | 0 | 0 | 0 | 1 | 0 | 0 | 1 | 1 | 0 | 0 |
| 77 | 0 | 0 | 0 | 0 | 0 | 0 | 0 | 0 | 0 | 1 |
| 78 | 0 | 0 | 0 | 0 | 0 | 0 | 0 | 0 | 0 | 1 |
| 79 | 0 | 1 | 1 | 0 | 0 | 0 | 0 | 0 | 0 | 1 |
| 80 | 0 | 0 | 0 | 0 | 0 | 0 | 0 | 0 | 0 | 0 |
| 81 | 0 | 0 | 0 | 0 | 0 | 0 | 0 | 0 | 0 | 0 |
| 82 | 0 | 1 | 0 | 0 | 0 | 0 | 0 | 0 | 0 | 0 |
| 83 | 1 | 0 | 0 | 0 | 1 | 1 | 0 | 0 | 0 | 1 |
| 84 | 1 | 0 | 0 | 0 | 1 | 1 | 0 | 0 | 1 | 0 |
| 85 | 1 | 0 | 1 | 1 | 1 | 1 | 1 | 1 | 1 | 0 |
| 86 | 0 | 0 | 1 | 0 | 0 | 0 | 0 | 0 | 0 | 0 |
| 87 | 0 | 0 | 1 | 0 | 0 | 0 | 0 | 0 | 0 | 0 |
| 88 | 0 | 0 | 0 | 0 | 0 | 0 | 0 | 0 | 0 | 0 |
| 89 | 0 | 0 | 0 | 0 | 0 | 0 | 0 | 0 | 0 | 0 |
| 90 | 0 | 0 | 0 | 0 | 0 | 0 | 0 | 0 | 1 | 0 |
| 91 | 0 | 0 | 0 | 0 | 0 | 0 | 0 | 0 | 1 | 0 |
| 92 | 1 | 0 | 0 | 0 | 0 | 0 | 0 | 0 | 0 | 0 |
| 93 | 0 | 0 | 0 | 0 | 0 | 0 | 0 | 0 | 0 | 0 |
| 94 | 0 | 0 | 0 | 0 | 0 | 0 | 0 | 0 | 0 | 0 |
| 95 | 0 | 1 | 0 | 0 | 0 | 0 | 0 | 0 | 0 | 0 |
| 96 | 0 | 0 | 0 | 0 | 0 | 0 | 0 | 0 | 0 | 0 |
| 97 | 0 | 0 | 0 | 0 | 0 | 0 | 0 | 0 | 0 | 0 |
| 98 | 0 | 1 | 1 | 0 | 0 | 0 | 0 | 0 | 0 | 0 |
| 99 | 0 | 1 | 1 | 0 | 0 | 0 | 0 | 0 | 0 | 0 |
| 100 | 0 | 1 | 1 | 1 | 0 | 0 | 0 | 0 | 0 | 0 |
| 101 | 0 | 0 | 0 | 0 | 0 | 0 | 0 | 0 | 0 | 0 |
| 102 | 0 | 0 | 0 | 0 | 0 | 0 | 0 | 0 | 0 | 0 |
| 103 | 0 | 0 | 0 | 0 | 0 | 0 | 1 | 1 | 0 | 0 |
| 104 | 0 | 0 | 0 | 1 | 0 | 0 | 0 | 0 | 0 | 0 |
| 105 | 0 | 0 | 0 | 1 | 1 | 0 | 0 | 0 | 0 | 0 |
| 106 | 0 | 0 | 0 | 0 | 0 | 0 | 0 | 0 | 0 | 0 |
| 107 | 0 | 0 | 0 | 0 | 0 | 0 | 0 | 0 | 0 | 0 |
| 108 | 0 | 0 | 0 | 0 | 0 | 0 | 0 | 0 | 0 | 0 |
| 109 | 0 | 0 | 0 | 0 | 0 | 0 | 0 | 0 | 0 | 0 |
| 110 | 1 | 1 | 0 | 0 | 0 | 0 | 0 | 0 | 0 | 0 |
| 111 | 1 | 1 | 0 | 0 | 0 | 0 | 0 | 0 | 1 | 0 |
| 112 | 1 | 0 | 0 | 0 | 0 | 0 | 0 | 0 | 0 | 0 |
| 113 | 0 | 0 | 0 | 1 | 0 | 0 | 0 | 0 | 0 | 0 |
| 114 | 0 | 0 | 0 | 1 | 0 | 0 | 0 | 1 | 0 | 0 |
| 115 | 0 | 0 | 0 | 1 | 0 | 0 | 0 | 0 | 0 | 0 |
| 116 | 0 | 1 | 0 | 1 | 0 | 0 | 1 | 0 | 0 | 0 |
| 117 | 0 | 1 | 1 | 1 | 0 | 0 | 1 | 0 | 0 | 0 |
| 118 | 0 | 1 | 0 | 1 | 0 | 0 | 1 | 0 | 0 | 0 |
| 119 | 0 | 0 | 0 | 0 | 0 | 0 | 0 | 0 | 1 | 0 |
| 120 | 0 | 0 | 0 | 0 | 0 | 0 | 0 | 0 | 1 | 0 |
| 121 | 0 | 0 | 0 | 0 | 0 | 0 | 0 | 0 | 1 | 0 |
| 122 | 1 | 0 | 0 | 0 | 1 | 1 | 0 | 0 | 0 | 0 |
| 123 | 1 | 0 | 0 | 0 | 1 | 1 | 0 | 0 | 0 | 0 |
| 124 | 1 | 0 | 0 | 0 | 1 | 1 | 0 | 0 | 0 | 0 |
| 125 | 0 | 0 | 0 | 0 | 0 | 0 | 0 | 0 | 0 | 0 |
| 126 | 0 | 0 | 0 | 0 | 0 | 0 | 0 | 1 | 0 | 0 |
| 127 | 0 | 0 | 0 | 1 | 1 | 0 | 0 | 0 | 0 | 0 |
| 128 | 0 | 0 | 0 | 0 | 0 | 0 | 0 | 0 | 0 | 1 |
| 129 | 0 | 0 | 0 | 0 | 0 | 0 | 0 | 0 | 0 | 0 |
| 130 | 0 | 0 | 0 | 0 | 0 | 0 | 0 | 0 | 0 | 0 |
| 131 | 0 | 0 | 0 | 0 | 0 | 0 | 0 | 0 | 0 | 0 |
| 132 | 0 | 0 | 0 | 0 | 0 | 0 | 0 | 0 | 0 | 0 |
| 133 | 0 | 0 | 0 | 0 | 0 | 0 | 0 | 0 | 0 | 0 |
| 134 | 0 | 0 | 0 | 0 | 0 | 0 | 0 | 0 | 0 | 0 |
| 135 | 0 | 0 | 0 | 0 | 0 | 0 | 1 | 0 | 0 | 0 |
| 136 | 0 | 0 | 0 | 0 | 0 | 0 | 0 | 0 | 0 | 0 |
| 137 | 1 | 0 | 0 | 0 | 0 | 0 | 0 | 0 | 0 | 0 |
| 138 | 0 | 0 | 0 | 0 | 0 | 0 | 0 | 0 | 0 | 0 |
| 139 | 0 | 0 | 0 | 0 | 0 | 0 | 0 | 0 | 0 | 0 |

Les échantillons Ech145 à Ech154 comportent au moins un peptide caractéristique des IMP. Les bactéries présentes dans les échantillons Ech145 à Ech154 expriment donc une béta-lactamase qui leur confère une résistance aux pénicillines, aux céphalosporines et aux carbapénèmes.

### Exemple 18 : Identification d'une résistance aux béta-lactamines de type OXA-48:

Les échantillons, Ech155 à Ech164, sont identifiés selon l'une des méthodes décrites dans les exemples 1, 3 ou 4. L'identification des espèces est reportée dans le **TABLEAU 29.**

**TABLEAU 29 :**

| Noms | Espèces |
|---|---|
| Ech155 | *K. pneumoniae* |
| Ech156 | *K. pneumoniae* |
| Ech157 | *K. pneumoniae* |
| Ech158 | *E. cloacae* |
| Ech159 | *E. cloacae* |
| Ech160 | *K. pneumoniae* |
| Ech161 | *K. pneumoniae* |
| Ech162 | *K. pneumoniae* |
| Ech163 | *K. pneumoniae* |
| Ech164 | *K. pneumoniae* |

Les échantillons Ech155 à Ech164 correspondent à une espèce pouvant comporter un mécanisme de résistance de type OXA-48. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 30** en lieu et place des peptides du **TABLEAU 3.**

**TABLEAU 30 :**

| Transition numéro | Peptide | Etat de charge du précurseur | Ion fragment | Temps de rétention (minutes) | (m/z) filtré en Q1 | (m/z) filtré en Q3 | Energie de collision (eV) | Seuil de positivité |
|---|---|---|---|---|---|---|---|---|
| 1 | ANQAFLPASTFK | 2 | y6 monochargé | 18,09 | 647,84 | 650,35 | 32,7 | 2000 |
| 2 | ANQAFLPASTFK | 2 | y7 monochargé | 18,11 | 647,84 | 763,44 | 32,7 | 2000 |
| 3 | ANQAFLPASTFK | 2 | y8 monochargé | 18,09 | 647,84 | 910,5 | 32,7 | 2000 |
| 4 | DEHQVFK | 3 | y5 dichargé | 9,89 | 301,48 | 329,69 | 16,4 | 2000 |
| 5 | DEHQVFK | 2 | y4 monochargé | 9,89 | 451,72 | 521,31 | 21,5 | 2000 |
| 6 | DEHQVFK | 2 | y5 monochargé | 9,91 | 451,72 | 658,37 | 21,5 | 2000 |
| 7 | DHNLITAMK | 3 | y3 monochargé | 14,57 | 348,18 | 349,19 | 17,9 | 2000 |
| 8 | DHNLITAMK | 3 | y4 monochargé | 14,57 | 348,18 | 450,24 | 17,9 | 2000 |
| 9 | DHNLITAMK | 2 | y7 monochargé | 14,57 | 521,77 | 790,45 | 25,5 | 2000 |
| 10 | DIATWNR | 2 | y3 monochargé | 13,79 | 438,22 | 475,24 | 20,7 | 2000 |
| 11 | DIATWNR | 2 | y4 monochargé | 13,79 | 438,22 | 576,29 | 20,7 | 2000 |
| 12 | DIATWNR | 2 | y5 monochargé | 13,79 | 438,22 | 647,33 | 20,7 | 2000 |
| 13 | IPNSLIALDLGVVK | 3 | y6 monochargé | 23,68 | 484,63 | 630,38 | 22,1 | 2000 |
| 14 | IPNSLIALDLGVVK | 2 | y13 dichargé | 23,68 | 726,45 | 669,9 | 37,1 | 2000 |
| 15 | IPNSLIALDLGVVK | 2 | y8 monochargé | 23,68 | 726,45 | 814,5 | 37,1 | 2000 |
| 16 | ISATEQISFLR | 2 | y4 monochargé | 19,17 | 632,85 | 522,3 | 31,8 | 2000 |
| 17 | ISATEQISFLR | 2 | y5 monochargé | 19,17 | 632,85 | 635,39 | 31,8 | 2000 |
| 18 | ISATEQISFLR | 2 | y6 monochargé | 19,17 | 632,85 | 763,45 | 31,8 | 2000 |
| 19 | QAMLTEANGDYIIR | 3 | y4 monochargé | 18,36 | 532,27 | 564,35 | 23,6 | 2000 |
| 20 | QAMLTEANGDYIIR | 3 | y6 monochargé | 18,36 | 532,27 | 736,4 | 23,6 | 2000 |
| 21 | QAMLTEANGDYIIR | 2 | y10 monochargé | 18,36 | 797,9 | 1151,57 | 41,2 | 2000 |
| 22 | QQGFTNNLK | 2 | y4 monochargé | 12,58 | 525,27 | 488,28 | 25,7 | 2000 |
| 23 | QQGFTNNLK | 2 | y5 monochargé | 12,58 | 525,27 | 589,33 | 25,7 | 2000 |
| 24 | QQGFTNNLK | 2 | y7 monochargé | 12,58 | 525,27 | 793,42 | 25,7 | 2000 |
| 25 | SQGVVVLWNENK | 2 | y5 monochargé | 18,54 | 686,87 | 690,32 | 34,9 | 2000 |
| 26 | SQGVVVLWNENK | 2 | y6 monochargé | 18,54 | 686,87 | 803,41 | 34,9 | 2000 |
| 27 | SQGVVVLWNENK | 2 | y7 monochargé | 18,52 | 686,87 | 902,47 | 34,9 | 2000 |
| 28 | SWNAHFTEHK | 3 | y8 dichargé | 12,23 | 419,53 | 492,24 | 20,1 | 2000 |
| 29 | SWNAHFTEHK | 3 | y9 dichargé | 12,23 | 419,53 | 585,28 | 20,1 | 2000 |
| 30 | SWNAHFTEHK | 3 | y5 monochargé | 12,23 | 419,53 | 661,33 | 20,1 | 2000 |
| 31 | VLALSAVFLVASIIGMPAVAK | 3 | y6 monochargé | 34,92 | 690,75 | 616,35 | 28,5 | 2000 |
| 32 | VLALSAVFLVASIIGMPAVAK | 3 | y7 monochargé | 34,94 | 690,75 | 673,37 | 28,5 | 2000 |
| 33 | VLALSAVFLVASIIGMPAVAK | 3 | y8 monochargé | 34,94 | 690,75 | 786,45 | 28,5 | 2000 |
| 34 | YSVVPVYQEFAR | 3 | y5 monochargé | 20,05 | 486,59 | 650,33 | 22,2 | 2000 |
| 35 | YSVVPVYQEFAR | 2 | y8 dichargé | 20,07 | 729,38 | 505,26 | 37,3 | 2000 |
| 36 | YSVVPVYQEFAR | 2 | y8 monochargé | 20,07 | 729,38 | 1009,51 | 37,3 | 2000 |

• Les autres paramètres machine utilisés sont les suivants :

| | |
|---|---|
| Type de balayage: | MRM |
| MRM planifié : | non |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 40,00 psi |
| Tension de cône: | 5500,00 V |
| Température de source: | 500,00 °C |
| Gaz de nébulisation: | 50,00 psi |
| Gaz chauffant: | 50,00 psi |
| Gaz de collision induisant dissociation: | 9,00 psi |
| Remplissage dynamique: | activé |
| Potentiel d'orifice : | 100, 00 V |
| (en anglais declustering potential (DP)) | |
| Potentiel d'entrée avant Q0 (EP): | 6,00 V |
| Potentiel en sortie de cellule de collision : | 15 V |
| (en anglais cell exit potential (CXP)) | |
| Temps de cycle total: | 1,1 sec |

Les aires obtenues pour chacune des transitions et pour chacun des microorganismes étudiés ont été mesurées. Lorsque les aires des transitions sont supérieures ou égales au seuil de positivité décrit dans le **TABLEAU 30,** la détection de la transition est considérée comme positive et est notée « 1 » dans le **TABLEAU 31.** Lorsqu'une transition a une aire inférieure au seuil de positivité décrit dans le **TABLEAU 30,** la transition est considérée comme non détectée et est notée « 0 » dans le **TABLEAU 31.**

Pour un peptide donné, lorsque au moins 3 transitions sont notées « 1 », le peptide est considéré comme détecté.

**TABLEAU 31 :**

| Transition numéro | Ech155 | Ech156 | Ech157 | Ech158 | Ech159 | Ech160 | Ech161 | Ech162 | Ech163 | Ech164 |
|---|---|---|---|---|---|---|---|---|---|---|
| 1 | 0 | 1 | 1 | 0 | 1 | 1 | 1 | 1 | 1 | 0 |
| 2 | 0 | 1 | 1 | 0 | 1 | 1 | 1 | 0 | 1 | 0 |
| 3 | 0 | 1 | 0 | 0 | 0 | 1 | 1 | 0 | 0 | 0 |
| 4 | 0 | 0 | 0 | 0 | 0 | 0 | 0 | 0 | 0 | 0 |
| 5 | 0 | 0 | 0 | 0 | 0 | 0 | 0 | 0 | 0 | 0 |
| 6 | 0 | 0 | 0 | 0 | 0 | 0 | 0 | 0 | 0 | 0 |
| 7 | 0 | 1 | 0 | 0 | 0 | 1 | 1 | 0 | 0 | 0 |
| 8 | 0 | 1 | 1 | 1 | 0 | 1 | 1 | 0 | 1 | 0 |
| 9 | 1 | 1 | 1 | 1 | 1 | 1 | 1 | 1 | 1 | 0 |
| 10 | 0 | 0 | 0 | 0 | 0 | 0 | 0 | 0 | 0 | 0 |
| 11 | 0 | 0 | 0 | 1 | 1 | 0 | 0 | 0 | 0 | 0 |
| 12 | 0 | 0 | 0 | 0 | 0 | 0 | 0 | 1 | 0 | 0 |
| 13 | 0 | 1 | 1 | 1 | 1 | 1 | 1 | 1 | 1 | 0 |
| 14 | 0 | 1 | 1 | 1 | 1 | 1 | 1 | 1 | 1 | 1 |
| 15 | 0 | 1 | 1 | 1 | 1 | 1 | 1 | 1 | 1 | 0 |
| 16 | 1 | 1 | 1 | 1 | 1 | 1 | 1 | 1 | 1 | 1 |
| 17 | 1 | 1 | 1 | 1 | 1 | 1 | 1 | 1 | 1 | 1 |
| 18 | 1 | 1 | 1 | 1 | 1 | 1 | 1 | 1 | 1 | 1 |
| 19 | 1 | 1 | 1 | 1 | 1 | 1 | 1 | 1 | 1 | 1 |
| 20 | 1 | 1 | 1 | 1 | 1 | 1 | 1 | 1 | 1 | 0 |
| 21 | 1 | 1 | 1 | 1 | 1 | 1 | 1 | 1 | 1 | 1 |
| 22 | 1 | 1 | 1 | 1 | 1 | 1 | 1 | 1 | 1 | 0 |
| 23 | 1 | 1 | 1 | 1 | 1 | 1 | 1 | 1 | 1 | 1 |
| 24 | 1 | 1 | 1 | 1 | 1 | 1 | 1 | 1 | 1 | 0 |
| 25 | 0 | 0 | 0 | 0 | 0 | 0 | 0 | 0 | 0 | 0 |
| 26 | 0 | 0 | 0 | 0 | 0 | 0 | 0 | 0 | 0 | 0 |
| 27 | 0 | 0 | 0 | 0 | 0 | 0 | 0 | 0 | 0 | 0 |
| 28 | 1 | 1 | 1 | 1 | 1 | 1 | 1 | 1 | 1 | 1 |
| 29 | 1 | 1 | 1 | 1 | 1 | 1 | 1 | 1 | 1 | 1 |
| 30 | 1 | 1 | 1 | 1 | 1 | 1 | 1 | 1 | 1 | 0 |
| 31 | 1 | 0 | 0 | 0 | 1 | 0 | 0 | 0 | 0 | 0 |
| 32 | 1 | 1 | 1 | 1 | 1 | 1 | 1 | 1 | 1 | 0 |
| 33 | 1 | 1 | 0 | 1 | 1 | 1 | 1 | 1 | 1 | 0 |
| 34 | 0 | 1 | 1 | 0 | 1 | 1 | 1 | 0 | 1 | 0 |
| 35 | 0 | 1 | 1 | 0 | 1 | 1 | 1 | 0 | 1 | 0 |
| 36 | 0 | 1 | 1 | 0 | 1 | 1 | 1 | 0 | 1 | 0 |

Les échantillons Ech155 à Ech164 comportent au moins un peptide caractéristique des OXA-48 groupe. Les bactéries présentes dans les échantillons Ech155 à Ech164 expriment donc une béta-lactamase qui leur confère une résistance aux pénicillines, aux céphalosporines de 1^{ère} et 2^{e} génération (mais pas aux céphalosporines à spectre étendu), et aux carbapénèmes.

### Exemple 19 : Identification d'une résistance aux béta-lactamines de type VIM:

Les échantillons, Ech165 à Ech170, sont identifiés selon l'une des méthodes décrites dans les exemples 1, 3 ou 4. L'identification des espèces est reportée dans le **TABLEAU 32.**

**TABLEAU32 :**

| Noms | Espèces |
|---|---|
| Ech165 | *P. aeruginosa* |
| Ech166 | *E. coli* |
| Ech167 | *A. baumannii complex* |
| Ech168 | *A. junii* |
| Ech169 | *E. coli* |
| Ech170 | *K. pneumoniae ssp pneumoniae* |

Les échantillons Ech165 à Ech170 correspondent à une espèce pouvant comporter un mécanisme de résistance de type VIM. La méthode suivante est alors mise en oeuvre pour détecter un tel mécanisme.

Chaque échantillon est traité selon l'exemple 5, puis analysé selon l'exemple 6 sauf mention contraire dans la suite de l'exemple, en détectant les peptides du **TABLEAU 33** en lieu et place des peptides du **TABLEAU 3.**

**TABLEAU 33 :**

| Transition numéro | Peptide | Temps de rétention (minutes) | (m/z) filtré en Q1 | (m/z) filtré en Q3 | Energie de collision (eV) | Seuil de positivité |
|---|---|---|---|---|---|---|
| 1 | AAGVATYASPSAR | 12,3 | 611,32 | 588,31 | 30,6 | 2500 |
| 2 | AAGVATYASPSAR | 12,3 | 611,32 | 852,42 | 30,6 | 2500 |
| 3 | AAGVATYASPSAR | 12,3 | 611,32 | 923,46 | 30,6 | 2500 |
| 4 | AAGVATYASPSIR | 14,5 | 632,34 | 630,36 | 31,8 | 2500 |
| 5 | AAGVATYASPSIR | 14,5 | 632,34 | 894,47 | 31,8 | 2500 |
| 6 | AAGVATYASPSIR | 14,5 | 632,34 | 965,51 | 31,8 | 2500 |
| 7 | AAGVATYASPSTR | 12 | 626,32 | 618,32 | 31,4 | 2500 |
| 8 | AAGVATYASPSTR | 12 | 626,32 | 882,43 | 31,4 | 2500 |
| 9 | AAGVATYASPSTR | 12 | 626,32 | 953,47 | 31,4 | 2500 |
| 10 | AAGVATYTSPLTR | 15,7 | 654,35 | 674,38 | 33 | 2500 |
| 11 | AAGVATYTSPLTR | 15,7 | 654,35 | 938,49 | 33 | 2500 |
| 12 | AAGVATYTSPLTR | 15,7 | 654,35 | 1009,53 | 33 | 2500 |
| 13 | AGVATYASPSTR | 11,8 | 590,8 | 547,28 | 29,4 | 2500 |
| 14 | AGVATYASPSTR | 11,8 | 590,8 | 618,32 | 29,4 | 2500 |
| 15 | AGVATYASPSTR | 11,8 | 590,8 | 781,38 | 29,4 | 2500 |
| 16 | ALSSSGDVVR | 11,3 | 495,76 | 632,34 | 24 | 2500 |
| 17 | ALSSSGDVVR | 11,3 | 495,76 | 719,37 | 24 | 2500 |
| 18 | ALSSSGDVVR | 11,3 | 495,76 | 806,4 | 24 | 2500 |
| 19 | AVSTHFHDDR | 9,2 | 395,52 | 413,68 | 19,3 | 2500 |
| 20 | AVSTHFHDDR | 9,2 | 395,52 | 507,72 | 19,3 | 2500 |
| 21 | AVSTHFHDDR | 9,2 | 395,52 | 689,3 | 19,3 | 2500 |
| 22 | DADELLLIDTAWGAK | 24,3 | 544,28 | 748,36 | 24 | 2500 |
| 23 | DADELLLIDTAWGAK | 24,3 | 815,92 | 544,31 | 42,2 | 2500 |
| 24 | DADELLLIDTAWGAK | 24,3 | 815,92 | 748,36 | 42,2 | 2500 |
| 25 | DADELLLIDTAWGAK | 24,3 | 815,92 | 861,45 | 42,2 | 2500 |
| 26 | DGDELLLIDTAWGAK | 24 | 539,61 | 748,36 | 23,8 | 2500 |
| 27 | DGDELLLIDTAWGAK | 24 | 808,91 | 748,36 | 41,8 | 2500 |
| 28 | DGDELLLIDTAWGAK | 24 | 808,91 | 861,45 | 41,8 | 2500 |
| 29 | DGDELLLIDTAWGTK | 24,1 | 549,61 | 778,37 | 24,1 | 2500 |
| 30 | DGDELLLIDTAWGTK | 24,1 | 823,92 | 778,37 | 42,7 | 2500 |
| 31 | DGDELLLIDTAWGTK | 24,1 | 823,92 | 891,46 | 42,7 | 2500 |
| 32 | ESAGNVADANLAEWPATIK | 20,2 | 652,99 | 529,33 | 27,3 | 2500 |
| 33 | ESAGNVADANLAEWPATIK | 20,2 | 652,99 | 715,41 | 27,3 | 2500 |
| 34 | ESAGNVADANLAEWPATIK | 20,2 | 978,99 | 529,33 | 51,5 | 2500 |
| 35 | GEYPTVSEIPVGEVR | 18,4 | 544,61 | 656,37 | 24 | 2500 |
| 36 | GEYPTVSEIPVGEVR | 18,4 | 816,42 | 641,85 | 42,3 | 2500 |
| 37 | GEYPTVSEIPVGEVR | 18,4 | 816,42 | 656,37 | 42,3 | 2500 |
| 38 | HTTNVVK | 1,3 | 399,73 | 345,25 | 18,5 | 2500 |
| 39 | HTTNVVK | 1,3 | 399,73 | 560,34 | 18,5 | 2500 |
| 40 | HTTNVVK | 1,3 | 399,73 | 661,39 | 18,5 | 2500 |
| 41 | IGDGVWSHIATQK | 17 | 471,25 | 563,8 | 21,7 | 2500 |
| 42 | IGDGVWSHIATQK | 17 | 471,25 | 621,32 | 21,7 | 2500 |
| 43 | IGDGVWSHIATQK | 17 | 471,25 | 649,83 | 21,7 | 2500 |
| 44 | LGDTVYSSNGLIVR | 17,9 | 747,4 | 387,27 | 38,3 | 2500 |
| 45 | LGDTVYSSNGLIVR | 17,9 | 747,4 | 845,48 | 38,3 | 2500 |
| 46 | LGDTVYSSNGLIVR | 17,9 | 747,4 | 1008,55 | 38,3 | 2500 |
| 47 | LYQIADGVWSHIATK | 20,8 | 567,97 | 592,81 | 24,7 | 2500 |
| 48 | LYQIADGVWSHIATK | 20,8 | 567,97 | 649,35 | 24,7 | 2500 |
| 49 | LYQIADGVWSHIATK | 20,8 | 567,97 | 713,38 | 24,7 | 2500 |
| 50 | LYQIADGVWSHIATR | 21 | 577,31 | 606,81 | 25 | 2500 |
| 51 | LYQIADGVWSHIATR | 21 | 577,31 | 663,35 | 25 | 2500 |
| 52 | LYQIADGVWSHIATR | 21 | 577,31 | 727,38 | 25 | 2500 |
| 53 | NTAALLAEIEK | 19,8 | 586,83 | 589,32 | 29,2 | 2500 |
| 54 | NTAALLAEIEK | 19,8 | 586,83 | 702,4 | 29,2 | 2500 |
| 55 | NTAALLAEIEK | 19,8 | 586,83 | 886,52 | 29,2 | 2500 |
| 56 | NTVALLAEIEK | 21,2 | 600,85 | 589,32 | 30 | 2500 |
| 57 | NTVALLAEIEK | 21,2 | 600,85 | 702,4 | 30 | 2500 |
| 58 | NTVALLAEIEK | 21,2 | 600,85 | 886,52 | 30 | 2500 |
| 59 | QIGLPVTR | 15,6 | 442,27 | 472,29 | 20,9 | 2500 |
| 60 | QIGLPVTR | 15,6 | 442,27 | 642,39 | 20,9 | 2500 |
| 61 | QIGLPVTR | 15,6 | 442,27 | 755,48 | 20,9 | 2500 |
| 62 | QLAEAAGNEVPAHSLK | 13,8 | 545,62 | 597,32 | 24 | 2500 |
| 63 | QLAEAAGNEVPAHSLK | 13,8 | 545,62 | 652,38 | 24 | 2500 |
| 64 | QLAEAAGNEVPAHSLK | 13,8 | 545,62 | 697,36 | 24 | 2500 |
| 65 | SFDGAVYPSNGLIVR | 19,2 | 797,92 | 559,32 | 41,2 | 2500 |
| 66 | SFDGAVYPSNGLIVR | 19,2 | 797,92 | 855,51 | 41,2 | 2500 |
| 67 | SFDGAVYPSNGLIVR | 19,2 | 797,92 | 1018,57 | 41,2 | 2500 |
| 68 | SISTHFHDDR | 10,6 | 405,52 | 413,68 | 19,7 | 2500 |
| 69 | SISTHFHDDR | 10,6 | 405,52 | 507,72 | 19,7 | 2500 |
| 70 | SISTHFHDDR | 10,6 | 405,52 | 689,3 | 19,7 | 2500 |
| 71 | SVSTHFHDDR | 9,2 | 400,85 | 413,68 | 19,5 | 2500 |
| 72 | SVSTHFHDDR | 9,2 | 400,85 | 507,72 | 19,5 | 2500 |
| 73 | SVSTHFHDDR | 9,2 | 400,85 | 689,3 | 19,5 | 2500 |
| 74 | TSAGNVADADLAEWPGSVER | 19,2 | 682,32 | 322,67 | 28,2 | 2500 |
| 75 | TSAGNVADADLAEWPGSVER | 19,2 | 682,32 | 644,34 | 28,2 | 2500 |
| 76 | TSAGNVADADLAEWPGSVER | 19,2 | 682,32 | 830,42 | 28,2 | 2500 |
| 77 | TSAGNVADADLAEWPGSVER | 19,2 | 1022,98 | 644,34 | 54 | 2500 |
| 78 | TSAGNVADADLAEWPTSIER | 20,7 | 701,67 | 351,69 | 28,8 | 2500 |
| 79 | TSAGNVADADLAEWPTSIER | 20,7 | 701,67 | 702,38 | 28,8 | 2500 |
| 80 | TSAGNVADADLAEWPTSIER | 20,7 | 701,67 | 888,46 | 28,8 | 2500 |
| 81 | TSAGNVADADLAEWPTSIER | 20,7 | 1052 | 702,38 | 55,7 | 2500 |
| 82 | TSAG NVADAD LAEWPTSVER | 19,6 | 697 | 344,69 | 28,7 | 2500 |
| 83 | TSAGNVADADLAEWPTSVER | 19,6 | 697 | 688,36 | 28,7 | 2500 |
| 84 | TSAGNVADADLAEWPTSVER | 19,6 | 697 | 874,44 | 28,7 | 2500 |
| 85 | TSAGNVADADLAEWPTSVER | 19,6 | 1045 | 688,36 | 55,3 | 2500 |
| 86 | VGGVDALR | 12,8 | 393,73 | 474,27 | 18,2 | 2500 |
| 87 | VGGVDALR | 12,8 | 393,73 | 630,36 | 18,2 | 2500 |
| 88 | VGGVDALR | 12,8 | 393,73 | 687,38 | 18,2 | 2500 |
| 89 | VGGVDVLR | 14,8 | 407,74 | 502,3 | 19 | 2500 |
| 90 | VGGVDVLR | 14,8 | 407,74 | 658,39 | 19 | 2500 |
| 91 | VGGVDVLR | 14,8 | 407,74 | 715,41 | 19 | 2500 |
| 92 | VLFGGCAVHEASR | 15,1 | 468,24 | 522,24 | 21,6 | 5100 |
| 93 | VLFGGCAVHEASR | 15,1 | 468,24 | 595,77 | 21,6 | 5100 |
| 94 | VLFGGCAVHEASR | 15,1 | 468,24 | 599,29 | 21,6 | 5100 |
| 95 | VLYGGCAVHELSR | 15,3 | 487,58 | 543,26 | 22,2 | 2500 |
| 96 | VLYGGCAVHELSR | 15,3 | 487,58 | 624,79 | 22,2 | 2500 |
| 97 | VLYGGCAVHELSR | 15,3 | 487,58 | 641,34 | 22,2 | 2500 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 500,00 °C |
| Gaz de nébulisation: | 50,00 psi |
| Gaz chauffant: | 50,00 psi |
| Gaz de collision induisant dissociation: | 9,00 psi |
| Remplissage dynamique: | activé |
| Potentiel d'orifice : 100, 00 V | |
| (en anglais declustering potential (DP)) | |
| Potentiel d'entrée avant Q0 (EP): | 6,00 V |
| Potentiel en sortie de cellule de collision : | 15 V |
| (en anglais cell exit potential (CXP)) | |
| Temps de cycle total: | 0,04 sec |
| Fenêtre de détection : | 120 sec |

Les aires obtenues pour chacune des transitions et pour chacun des microorganismes étudiés ont été mesurées. Lorsque les aires des transitions sont supérieures ou égales au seuil de positivité décrit dans le **TABLEAU 33,** la détection de la transition est considérée comme positive et est notée « 1 » dans le **TABLEAU 34.** Lorsqu'une transition a une aire inférieure au seuil de positivité décrit dans le **TABLEAU 33,** la transition est considérée comme non détectée et est notée « 0 » dans le **TABLEAU 34.**

Pour un peptide donné, lorsque au moins 3 transitions sont notées « 1 », le peptide est considéré comme détecté.

**TABLEAU 34 :**

| Transition numéro | Ech165 | Ech166 | Ech167 | Ech168 | Ech169 | Ech170 |
|---|---|---|---|---|---|---|
| 1 | 0 | 0 | 0 | 0 | 0 | 0 |
| 2 | 0 | 0 | 0 | 0 | 0 | 0 |
| 3 | 0 | 0 | 0 | 0 | 0 | 0 |
| 4 | 0 | 0 | 0 | 0 | 0 | 0 |
| 5 | 0 | 0 | 0 | 0 | 0 | 0 |
| 6 | 0 | 0 | 0 | 0 | 0 | 0 |
| 7 | 0 | 0 | 0 | 0 | 0 | 1 |
| 8 | 0 | 0 | 0 | 0 | 0 | 1 |
| 9 | 0 | 0 | 0 | 0 | 0 | 1 |
| 10 | 0 | 0 | 0 | 0 | 0 | 0 |
| 11 | 0 | 0 | 0 | 0 | 0 | 0 |
| 12 | 0 | 0 | 0 | 0 | 0 | 0 |
| 13 | 0 | 0 | 0 | 0 | 0 | 1 |
| 14 | 0 | 0 | 0 | 0 | 0 | 1 |
| 15 | 0 | 0 | 0 | 0 | 0 | 1 |
| 16 | 0 | 0 | 0 | 0 | 0 | 0 |
| 17 | 0 | 0 | 0 | 0 | 0 | 0 |
| 18 | 0 | 0 | 0 | 0 | 0 | 0 |
| 19 | 1 | 1 | 1 | 1 | 1 | 1 |
| 20 | 1 | 1 | 1 | 1 | 1 | 1 |
| 21 | 1 | 1 | 1 | 1 | 1 | 1 |
| 22 | 0 | 0 | 0 | 0 | 0 | 0 |
| 23 | 0 | 0 | 0 | 0 | 0 | 0 |
| 24 | 0 | 0 | 0 | 0 | 0 | 0 |
| 25 | 0 | 0 | 0 | 0 | 0 | 0 |
| 26 | 0 | 0 | 0 | 0 | 0 | 0 |
| 27 | 0 | 0 | 0 | 0 | 0 | 0 |
| 28 | 0 | 0 | 0 | 0 | 0 | 0 |
| 29 | 0 | 0 | 0 | 0 | 0 | 0 |
| 30 | 0 | 0 | 0 | 0 | 0 | 0 |
| 31 | 0 | 0 | 0 | 0 | 0 | 0 |
| 32 | 0 | 0 | 0 | 0 | 0 | 0 |
| 33 | 0 | 0 | 0 | 0 | 0 | 0 |
| 34 | 0 | 0 | 0 | 0 | 0 | 0 |
| 35 | 0 | 0 | 0 | 0 | 0 | 0 |
| 36 | 0 | 0 | 0 | 0 | 0 | 0 |
| 37 | 0 | 0 | 0 | 0 | 0 | 0 |
| 38 | 0 | 0 | 0 | 0 | 0 | 0 |
| 39 | 0 | 0 | 0 | 0 | 0 | 0 |
| 40 | 0 | 0 | 0 | 0 | 0 | 0 |
| 41 | 0 | 0 | 0 | 0 | 0 | 0 |
| 42 | 0 | 0 | 0 | 0 | 0 | 0 |
| 43 | 0 | 0 | 0 | 0 | 0 | 0 |
| 44 | 0 | 0 | 0 | 0 | 0 | 0 |
| 45 | 0 | 0 | 0 | 0 | 0 | 0 |
| 46 | 0 | 0 | 0 | 0 | 0 | 0 |
| 47 | 0 | 0 | 0 | 0 | 0 | 0 |
| 48 | 0 | 0 | 0 | 0 | 0 | 0 |
| 49 | 0 | 0 | 0 | 0 | 0 | 0 |
| 50 | 0 | 0 | 0 | 0 | 0 | 0 |
| 51 | 0 | 0 | 0 | 0 | 0 | 0 |
| 52 | 0 | 0 | 0 | 0 | 0 | 0 |
| 53 | 0 | 0 | 0 | 0 | 0 | 0 |
| 54 | 0 | 0 | 0 | 0 | 0 | 0 |
| 55 | 0 | 0 | 0 | 0 | 0 | 0 |
| 56 | 0 | 0 | 0 | 0 | 0 | 0 |
| 57 | 0 | 0 | 0 | 0 | 0 | 0 |
| 58 | 0 | 0 | 0 | 0 | 0 | 0 |
| 59 | 0 | 0 | 0 | 0 | 0 | 0 |
| 60 | 0 | 0 | 0 | 0 | 0 | 0 |
| 61 | 0 | 0 | 0 | 0 | 0 | 0 |
| 62 | 0 | 0 | 0 | 0 | 0 | 0 |
| 63 | 0 | 0 | 0 | 0 | 0 | 0 |
| 64 | 0 | 0 | 0 | 0 | 0 | 0 |
| 65 | 0 | 0 | 0 | 0 | 0 | 0 |
| 66 | 0 | 0 | 0 | 0 | 0 | 0 |
| 67 | 0 | 0 | 0 | 0 | 0 | 0 |
| 68 | 0 | 0 | 0 | 0 | 0 | 0 |
| 69 | 0 | 0 | 0 | 0 | 0 | 0 |
| 70 | 0 | 0 | 0 | 0 | 0 | 0 |
| 71 | 0 | 0 | 0 | 0 | 0 | 0 |
| 72 | 0 | 0 | 0 | 0 | 0 | 0 |
| 73 | 0 | 0 | 0 | 0 | 0 | 0 |
| 74 | 0 | 0 | 0 | 0 | 0 | 0 |
| 75 | 0 | 0 | 0 | 0 | 0 | 0 |
| 76 | 0 | 0 | 0 | 0 | 0 | 0 |
| 77 | 0 | 0 | 0 | 0 | 0 | 0 |
| 78 | 1 | 0 | 1 | 0 | 0 | 0 |
| 79 | 1 | 0 | 1 | 0 | 0 | 0 |
| 80 | 1 | 0 | 1 | 0 | 0 | 0 |
| 81 | 1 | 0 | 1 | 0 | 0 | 0 |
| 82 | 0 | 0 | 0 | 1 | 1 | 0 |
| 83 | 0 | 0 | 0 | 1 | 1 | 0 |
| 84 | 0 | 0 | 0 | 1 | 1 | 0 |
| 85 | 0 | 0 | 0 | 1 | 1 | 0 |
| 86 | 0 | 0 | 0 | 0 | 0 | 0 |
| 87 | 0 | 0 | 0 | 0 | 0 | 0 |
| 88 | 0 | 0 | 0 | 0 | 0 | 0 |
| 89 | 1 | 1 | 1 | 1 | 1 | 1 |
| 90 | 1 | 1 | 1 | 1 | 1 | 1 |
| 91 | 1 | 1 | 1 | 1 | 1 | 1 |
| 92 | 0 | 0 | 0 | 0 | 0 | 0 |
| 93 | 0 | 0 | 0 | 0 | 0 | 0 |
| 94 | 0 | 0 | 0 | 0 | 0 | 0 |
| 95 | 0 | 0 | 0 | 0 | 1 | 1 |
| 96 | 0 | 0 | 0 | 0 | 1 | 1 |
| 97 | 0 | 0 | 0 | 0 | 1 | 1 |

Les échantillons Ech165 à Ech170 comportent au moins un peptide caractéristique des VIM. Les bactéries présentes dans les échantillons Ech165 à Ech170 expriment donc une béta-lactamase qui leur confère une résistance aux pénicillines, aux céphalosporines et aux carbapénèmes.

Les méthodes de détection décrites dans les exemples 6 à 11 sont particulièrement avantageuses car elles permettent de doser un grand nombre de peptides et de détecter simultanément la présence d'un ou plusieurs mécanismes de résistance induit par une ou plusieurs carbapénèmases.

En outre, la détection est réalisée en un temps court, inférieur à une heure. En effet, seule la partie du gradient comprise entre 3 et 34 minutes est utile à l'analyse. Les temps de rétention des peptides dosés sont d'ailleurs tous inférieurs à 34 minutes.
De surcroit, les méthodes de détection décrites dans les exemples 6 à 11 sont plus avantageuses que les méthodes de biologie moléculaire car elles détectent le produit de l'expression des gènes et non les gènes eux-mêmes . La détection d'un gène de résistance peut ne pas avoir de sens clinique si ce gène n'est pas exprimé, ou s'il est trop faiblement exprimé pour conduire à une résistance effective. La détection d'un peptide caractérisant une protéine caractéristique d'un mécanisme de résistance n'a pas cet inconvénient.
De façon surprenante, les exemples ci-dessus montrent qu'il est possible d'atteindre par spectrométrie de masse la sensibilité nécessaire à la détection spécifique de l'existence d'un mécanisme de résistance à au moins un antimicrobien d'un microorganisme contenu dans un échantillon, et ce, sans mettre en oeuvre de méthode d'amplification comme c'est usuellement le cas lorsque des méthodes de biologie moléculaire sont utilisées.

### Références bibliographiques

[1] J. Anhalt & C. Fenselau, 1975, Anal. Chem., 47(2) :219-225.
[2] A. Fox et al, ed., 1990, Analytical microbiology methods : chromatography and mass spectrometry, Plenum Press, New York, N.Y.
[3] M. Claydon et al, 1996, Nature Biotech. 14 :1584-1586.
[4] T. Krishnamurthy & P. Ross, 1996, Rapid Com. Mass Spec., 10 :1992-1996.
[5] P. Seng et al. 2009, Clin. Infect. Dis., 49 :543-551.
[6] C. Fenselau et al., 2008, Appl. Environ. Microbiol., 904-906.
[7] S. Hofstadler et al., 2005, Int..J Mass Spectrom., 242:23-41.
[8] D. Ecker, 2008, Nat. Rev. Microbiol., 6(7):553-558.
[9] Bush and Jacoby, 2010, Antimicrobial Agents and Chemotherapy ; 54 (3) : 969-976
[10] W.-J. Chen et al., 2008, Anal. Chem., 80 : 9612-9621
[11] D. Lopez-Ferrer et al., 2008, Anal. Chem., 80 :8930-8936
[12] D. Lopez-Ferrer et al., 2005, J. Proteome res., 4(5) : 1569-1574
[13] T. Fortin et al., 2009, Mol. Cell Proteomics, 8(5) : 1006-1015.
[14] H. Keshishian et al., 2007, Mol. Cell Proteomics, 2212-2229.
[15] J. Stal-Zeng et al., 2007, Mol. Cell Proteomics, 1809-1817.
[16] Gaskell, Electrospray: principles and practise, 1997, J. Mass Spectrom., 32, 677-688).
[17] V. Fusaro et al., 2009, Nature Biotech. 27, 190-198.
[18] J. Mead et al., 15 nov 2008, Mol. Cell Proteomics, E-pub.
[19] F. Desiere et al., 2006, Nucleic Acids Res., 34(database issue) : D655-8).
[20] L. Anderson & C. Hunter, 2006, Mol. Cell Proteomics, 573-588).
[21] B. Han & R. Higgs, 2008, Brief Funct Genomic Proteomic.,7(5):340-54).
[22] K.-Y. Wang et al., 2008, Anal Chem, 80(16) 6159-6167).
[23] J. Bundy & C. Fenselau, 1999, Anal. Chem. 71 : 1460-1463.
[24] K-C Ho et al., 2004, Anal. Chem. 76 : 7162-7268.
[25] Y.S. Lin et al., 2005, Anal. Chem., 77 : 1753-1760.
[26] S. Vaidyanathan et al., 2001, Anal. Chem., 73 :4134-4144.
[27] R. Everley et al., 2009, J. Microbiol. Methods, 77:152-158.
[28] P. Seng et al., 2009, Clin. Infect. Dis., 49 :543-551.
[29] Manes N. et al., 2007, Mol. & Cell. Proteomics, 6(4): 717-727.
[30] R. Nandakumar et al., 2009, Oral Microbiology Immunology, 24 :347-352).
[31] L. Hernychova et al., 2008, Anal. Chem., 80 :7097-7104.
[32] J.-M. Pratt et al., 2006, Nat. Protoc., 1:1029-1043.
[33] V. Brun et al., 2007, Mol. Cell Proteomics, 2139-2149.

## Revendications

1. Procédé de détection, par spectrométrie de masse de type MS/MS en mode MRM, pour au moins un microorganisme compris dans un échantillon, d'au moins un marqueur de résistance à au moins un antimicrobien, qui est un carbapénème, lesdits marqueurs de résistance sont des marqueurs de type KPC, choisis parmi les peptides de séquence SEQ ID N°20 à SEQ ID N°33, SEQ ID N°1094, SEQ ID N°1096 et SEQ ID N°1097.

2. Procédé de détection selon la revendication 1, dans lequel les peptides sont obtenus par digestion de protéines dudit microorganismes.

3. Procédé de détection selon la revendication 2, dans lequel la digestion est réalisée par une enzyme.

4. Procédé de détection selon la revendication 3, dans lequel l'enzyme est la trypsine.

5. Procédé de détection selon la revendication 1, dans lequel les marqueurs de résistance sont des marqueurs de type KPC, choisis parmi les peptides de séquence SEQ ID N°20, SEQ ID N°21, SEQ ID N°23, SEQ ID N°25, SEQ ID N°28, SEQ ID N°29, SEQ ID N°31, ou SEQ ID N°32.

## Patentansprüche

1. Nachweisverfahren mittels MS/MS-Typ-Massenspektrometrie im MRM-Modus von mindestens einem Marker für Resistenz gegen mindestens ein antimikrobielles Mittel, bei dem es sich um ein Carbapenem handelt, bei mindestens einem in einer Probe befindlichen Mikroorganismus, wobei es sich bei den Resistenzmarkern um Marker des KPC-Typs, ausgewählt aus den Peptiden mit der Sequenz SEQ ID NR. 20 bis SEQ ID NR. 33, SEQ ID NR. 1094, SEQ ID NR. 1096 und SEQ ID NO: 1097, handelt.

2. Nachweisverfahren nach Anspruch 1, wobei die Peptide durch Verdau von Proteinen der Mikroorganismen erhalten werden.

3. Nachweisverfahren nach Anspruch 2, wobei der Verdau mit einem Enzym erfolgt.

4. Nachweisverfahren nach Anspruch 3, wobei es sich bei dem Enzym um Trypsin handelt.

5. Nachweisverfahren nach Anspruch 1, wobei es sich bei den Resistenzmarkern um Marker des KPC-Typs, ausgewählt aus den Peptiden der Sequenz SEQ ID NR. 20, SEQ ID NR. 21, SEQ ID NR. 23 SEQ ID NR. 25, SEQ ID NR. 28, SEQ ID NR. 29, SEQ ID NR. 31 oder SEQ ID NR. 32, handelt.

## Claims

1. A method of detection, by mass spectrometry of MS/MS type in MRM mode, for at least one microorganism contained in a sample, of at least one marker of resistance to at least one antimicrobial, which is a carbapenem, said markers of resistance being markers of KPC type, chosen from the peptides of sequence SEQ ID No. 20 to SEQ ID No. 33, SEQ ID No. 1094, SEQ ID No. 1096 and SEQ ID No. 1097.

2. The detection method according to claim 1, wherein the peptides are obtained by digestion of proteins of said microorganism.

3. The detection method according to claim 2, wherein the digestion is performed by an enzyme.

4. The detection method according to claim 3, wherein the enzyme is trypsin.

5. The detection method according to claim 1, wherein the resistance markers are KPC markers chosen from the peptides of sequence SEQ ID No. 20, SEQ ID No. 21, SEQ ID No. 23, SEQ ID No. 25, SEQ ID No. 28, SEQ ID No. 29, SEQ ID No. 31, or SEQ ID No. 32.
